# EUROPEAN PATENT APPLICATION

(11) **EP 2 351 776 A1**
(43) Date of publication of application: **03.08.2011**
(21) Application number: 10195787.6
(22) Date of filing: 13.06.2006
(51) Int. Cl.: C07K 14/605, A61K 38/26, A61P 3/04

(54) **Oxyntomodulin analogues and their effects on feeding behaviour**

(30) Priority: 13.06.2005 GB 0511986; 13.06.2005 GB 0511988; 13.06.2005 GB 0511990; 13.06.2005 GB 0511998; 08.02.2006 GB 0602567
(62) Divisional of application: 06744197.2
(71) Applicant: Imperial Innovations Limited, London SW7 2AZ (GB)
(72) Inventor: Bloom, Stephen Robert, London, SW7 2AZ (GB); Ghatei, Mohammad Ali, SW7 2AZ, London (GB)
(74) Representative: Dörries, Hans Ulrich

(57) **Abstract**

Compounds of the invention are novel peptide analogues of oxyntomodulin (oxm) in which one or more amino acids of the oxm sequence have been changed. Changing amino acids 15-24 of oxm to either amino acids 968 - 977 of the α-latrotoxin peptide (and variations thereof) or amino acids 15 - 24 of exendin-4 (and variations thereof), or combinations of sequences from these sources, and/or changing amino acids 27-33 of oxm to amino acids 27-33 of exendin-4, and/or the addition of amino acids to the C-terminus of the peptide, results in a series of analogues of oxm that demonstrate the oxm like activity of reducing food intake, and with certain embodiments a greater ability to decrease food intake.

## Description

### 1. FIELD OF THE INVENTION

This application relates to the use of agents to control appetite, feeding, food intake, energy expenditure and calorie intake, treat excess weight, obesity and to prevent and treat the comorbidities of obesity.

### 2. BACKGROUND OF THE INVENTION

According to the World Health Organisation (WHO), obesity represents a global epidemic in which more than one billion adults are overweight, of which at least 300 million are clinically obese. Furthermore, WHO estimate that 250,000 deaths per year in Europe, and more than 2.5 million deaths worldwide are weight related (World Health Organisation, Global Strategy on Diet, Physical Activity and Health, 2004).

The cause of obesity is complex and multi-factorial. Increasing evidence suggests that obesity is not a simple problem of self-control but is a complex disorder involving appetite regulation and energy metabolism. In addition, obesity is associated with a variety of conditions associated with increased morbidity and mortality in a population. Although the etiology of obesity is not definitively established, genetic, metabolic, biochemical, cultural and psychosocial factors are believed to contribute. In general, obesity has been described as a condition in which excess body fat puts an individual at a health risk.

There is strong evidence that obesity is associated with increased morbidity and mortality. Disease risk, such as cardiovascular disease risk and type 2 diabetes disease risk, increases independently with increased body mass index (BMI). Indeed, this risk has been quantified as a five percent increase in the risk of cardiac disease for females, and a seven percent increase in the risk of cardiac disease for males, for each point of a BMI greater than 24.9 (see Kenchaiah et al., N. Engl. J. Med. 347:305, 2002; Massie, N. Engl. J. Med. 347:358, 2002). In addition, there is substantial evidence that weight loss in obese or overweight persons reduces important disease risk factors. A weight loss of 10% of the initial body weight in both overweight and obese adults has been associated with a decrease in risk factors such as hypertension, hyperlipidemia, and hyperglycemia.

Although diet and exercise provide a simple process to decrease weight gain and promote weight loss, overweight and obese individuals often cannot sufficiently control these factors to lose weight effectively. Pharmacotherapy is available; several weight loss drugs have been approved by the US Food and Drug Administration that can be used as part of a comprehensive weight loss program. However, many of these drugs have serious adverse side effects. An example of a widely used appetite suppressant is sibutramine (reviewed by McNeely, W et al., Drugs, 1998, 56(6), 1093-1124). Sibutramine's primary and secondary metabolites are pharmacologically active and they are thought to induce enhancement of satiety and thermogenesis by inhibiting serotonin and noradrenaline reuptake. When less invasive methods have failed, and the patient is at high risk for obesity related morbidity or mortality, weight loss surgery is an option in carefully selected patients with clinically severe obesity. However, these treatments are high-risk, and suitable for use in only a limited number of patients (Wolfe and Morton, JAMA, 2005, 294,1960-1963). It is not only obese subjects who wish to lose weight. People with weight within the recommended range, for example, in the upper part of the recommended range, may wish to reduce their weight, to bring it closer to the ideal weight.

Oxyntomodulin (hereafter oxm) is a 37 amino acid peptide member of the glucagon superfamily (Sherwood et al, Endocrine Reviews, 2000, 21(6): 619-670) comprising the entire 29 amino acid sequence of glucagon, with an eight amino acid carboxy terminal extension, resulting from the tissue-specific processing of the pre-pro-glucagon precursor in the brain and gut (Holst, Ann Rev Physiol, 1997, 59:257-271). Administration of oxm to rats via intracerebroventricular and injection into the paraventricular and arcuate nuclei of the hypothalamus inhibits refeeding after a fast (Dakin et al, Endocrinology, 2001, 142:4244-4250; Dakin et al, Endocrinology, 2004, 145:2687-2695). Chronic central administration resulted in reduced weight gain consistent with a reduction in food intake (Dakin et al, Am J Physiol Endocrinol Metab, 2002, 283:E1173-E1177). Twice daily peripheral injections also resulted in reduced body weight gain and adiposity (Dakin et al, Endocrinology, 2004, 145:2687-2695).

WO 03/022304 discloses the use of oxm in its native form and analogues thereof as a medicament for use in control of appetite, feeding, food intake, energy expenditure and calorie intake, particularly in the field of obesity. Studies in humans have shown that intravenously infused oxm is an effective appetite suppressant (Cohen et al, J. Clin. Endocrinol Metab, 2003, 88(10): 4696-4701). In a study of the effects of oxm on weight loss in humans it was found that subcutaneous injections of 1.8 mg (approximately 400 nmol) of oxm to humans volunteers three times daily (30 mins before meals) for 28 days resulted in a significant reduction of body weight (Wynne et al, Diabetes, 2005, 54: 2390-2395).

Peptides are widely used in medical practice, although when native peptides or analogues thereof are used in therapy it is generally found that they have a high clearance rate and or are sensitive to degradation. In particular, a high clearance or rapid degradation of a therapeutic agent is inconvenient in cases where it is desired to maintain a high blood level over a prolonged period of time since repeat administrations will then be necessary, decreasing patient compliance and increasing the cost of the therapy accordingly.

The receptor for α-latrotoxin, a presynaptic neurotoxin isolated from the venom of the black widow spider *Latrodectus tredecimguttatus* shows marked primary amino acid sequence homology to the family of GTP-binding protein-coupled receptors that included receptors for glucagon superfamily, including glucagon, glucagon-like peptide-1 (GLP-1), GLP-2, glucose-dependent insulinotropic polypeptide (GIP), GH-releasing hormone (GRF), peptide histidine-methionine (PHM), PACAP, secretin, and vasoactive intestinal polypeptide (VIP). Comparison of the primary amino acid sequences of α-latrotoxin and exendin-4 (hereinafter exendin), a 39-amino acid peptide isolated from the salivary glands of the Gila monster *(Heloderma suspectum),* reveals conserved sequences that are also shown to be conserved in members of the glucagon superfamily of hormones (glucagon, GLP-1, VIP, oxm) and neuropeptides (PACAP). It has been proposed that these sequences may confer useful therapeutic properties (Holz and Habener, Comp Biochem Physiol, 1998, Part B 121: 177-184).

A need remains for agents that can be used to effect weight loss in overweight and obese subjects, and/or to treat patients with other conditions involving excess weight, for example diabetes and eating disorders. There is especially a need for agents structurally similar to oxm that show greater potency and/or a protracted or more therapeutically useful profile of action and/or a lower clearance rate than native oxm.

### 3. SUMMARY OF THE INVENTION

Compounds of the invention are novel peptide analogues of oxm (hereafter "oxm analogues") in which one or more amino acids or parts of the oxm sequence have been replaced by one or more particular substituent amino acids or sequences. Surprisingly, the inventors have found that changing amino acids 15 - 24 of oxm to either 968 - 977 of the α-latrotoxin peptide (and variations thereof) or 15 -24 of exendin-4 (and variations thereof), or combinations of sequences from these regions, and/or changing amino acids 27 - 33 of oxm to 27 - 33 of exendin-4 (and variations thereof), and/or the addition of amino acids to the C-terminus of the peptide, results in a series of analogues of oxm that demonstrate the oxm like activity of reducing food intake, and with certain embodiments a greater ability to decrease food intake. None of these regions of the forgoing peptide hormones have previously been associated with these properties. It is thought that changes to these regions of oxm beneficially alters the rigidity of the α-helical secondary structure of the peptide, thus conferring an enhanced stability upon the analogues and/or improving their biological function. The full length sequnce of α-latrotoxin is given in GenBank record 1616226A. It should, however be noted that the residue numbering used above, corresponds to the residue numbering used in Holz and Habener *(ibid)* rather than that used in the GenBank record.

It has also been surprisingly found that altering the N-terminal sequence of oxm, in particular to D-His Ala Asp or D-His Ala Glu, making the sequence more homologous to that of GLP-1 and thus potentially more sensitive to degradation by DPP IV, also increases the anorectic effects of oxm.

Further amino acid changes within these regions yielding beneficial effects are also described, along with analogue sequences derivatised to other chemical moieties such as acyl chains, albumin and PEG species.

It can be inferred from the exemplifying data that such enhanced anorectic effects are indicative of an increased potency, due to either (1) the markedly smaller doses required to achieve an equivalent (or greater) decrease in food intake; and (2) increased resistance to degredative activity, due to the anorectic effects of the analogues being prolonged significantly in comparison to that of native oxm.

The invention provides a compound of general Formula (I):

Z-X-S1 (I)

in which
X is oxm4-14 (meaning, according to the nomenclature used herein, residues 4 to 14 inclusive of an oxm sequence); and
Z is an amino acid sequence of three amino acid residues
wherein S1 is an amino acid sequence corresponding to line A or an amino acid sequence corresponding to line A in which m amino acids of said line A are substituted by m corresponding amino acids from residues 15 to 37 in line B, and t further amino acid residues of line A are substituted by t corresponding amino acid residues from residues 15 to 24 of line R, in which line A (SEQ ID NO: 1),line B (SEQ ID NO: 2) and line R (SEQ ID NO: 145) are as follows:

| | | | | | | | | | | |
|---|---|---|---|---|---|---|---|---|---|---|
| Line A | Asp | Ser | Arg | Arg | Ala | Gln | Asp | Phe | Val | Gln |
| | 15 | 16 | 17 | 18 | 19 | 20 | 21 | 22 | 23 | 24 |
| Line B | Glu | Glu | Glu | Ala | Val | Arg | Leu | Phe | Ile | Glu |
| Line R | Arg | Ile | Glu | Ile | Val | Lys | Tyr | Phe | Ile | Gly |
| | | | | | | | | | | |
| Line A | Trp | Leu | Met | Asn | Thr | Lys | Arg | Asn | Arg | Asn |
| | 25 | 26 | 27 | 28 | 29 | 30 | 31 | 32 | 33 | 34 |
| Line B | Trp | Leu | Lys | Asn | Gly | Gly | Pro | Ser | Ser | Gly |
| | | | | | | | | | | |
| Line A | Asn | Ile | Ala | | | | | | | |
| | 35 | 36 | 37 | | | | | | | |
| Line B | Ala | Pro | Pro | | | | | | | |

the compound optionally further including an extension moiety attached to the amino acid at position 37, the optional extension moiety comprising one or more amino acids;
m being an integer not less than 1;
and t being 0, 1, 2, 3, 4, 5, 6, 7, 8, 9 or 10
a variant or derivative thereof;
or a salt or solvate thereof
with the proviso that, if S 1 is identical to Line A, Z is not His Ser Gln.

In a first preferred embodiment, the invention provides a compound of general Formula (II):

Z-X-S2-Y (II)

in which
X and Z are as defined above with reference to formula (I);
Y is oxm25-37; and
wherein S2 is an amino acid sequence corresponding to line C (SEQ ID NO: 3) in which n amino acids of said line C are substituted by n corresponding amino acids from line D (SEQ ID NO: 4), and u further amino acids of line C are substituted by u corresponding amino acids from line S (SEQ ID NO: 145), in which line C, line D and line S are as follows::

| | | | | | | | | | | |
|---|---|---|---|---|---|---|---|---|---|---|
| Line C | Asp | Ser | Arg | Arg | Ala | Gln | Asp | Phe | Val | Gln |
| | 15 | 16 | 17 | 18 | 19 | 20 | 21 | 22 | 23 | 24 |
| Line D | Glu | Glu | Glu | Ala | Val | Arg | Leu | Phe | Ile | Glu |
| Line S | Arg | Ile | Glu | Ile | Val | Lys | Tyr | Phe | Ile | Gly |

n is an integer not less than 1;
and u is 0, 1, 2, 3, 4, 5, 6, 7, 8 or 9
a variant or derivative thereof;
or a salt or solvate thereof.

In a second preferred embodiment, the invention provides a compound of general Formula (III):

Z - X' - S3 - Y' (III)

in which
X' is oxm4-26;
Z is as defined above with reference to formula (I);
Y' is oxm34-37; and
wherein S3 is an amino acid sequence corresponding to line E in which p amino acids of said line E are substituted by p corresponding amino acids from line F, in which line E (SEQ ID NO: 5) and line F (SEQ ID NO: 6) are as follows:

| | | | | | | | |
|---|---|---|---|---|---|---|---|
| Line E | Met | Asn | Thr | Lys | Arg | Asn | Arg |
| | 27 | 28 | 29 | 30 | 31 | 32 | 33 |
| Line F | Lys | Asn | Gly | Gly | Pro | Ser | Ser |

and p is an integer not less than 1;
a variant or derivative thereof;
or a salt or solvate thereof.

According to a third preferred embodiment, the invention provides a compound according to Formula (I) above
in which:
X is defined above with reference to Formula (I),
S1 corresponds to at least a sequence of line A as defined above with reference to Formula (I), and having at least six amino acids; and
Z is A¹ - A³ -,
wherein:
A¹ is an amino acid other than L-histidine;
A² is L-alanine or L-serine; and
A³ is L-aspartate or L-glutamate or L-glutamine.

The invention also provides a compound having the general Formula (VI): 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Y Asn Asn Ile Ala-X (VI, SEQ ID NO: 7)
 wherein Y is Arg or Lys and X is at least one amino acid, a variant or derivate thereof; or a salt or solvate thereof.

Following the principles outlined above has yielded a series of more than 160 oxm analogues, of which the vast majority are more potent inhibitors of food intake than native oxm.

### 4. BRIEF DESCRIPTION OF THE FIGURES

**Figures 1a, 1b****,** **1c, 1d****,** **1e** **& If** show the results of experiments in which the appetite suppressing effects in mice of three compounds of the invention were compared with native human oxyntomodulin and saline over given time intervals following injection.
**Figures 2a, 2b** **&** **2c** show the cumulative food intake over time.
**Figures 3a****,** **3b****,** **3c****,** **3d and 3e** show the results of experiments in which appetite suppressing effects of three compounds of the invention were compared with exendin 4, and with saline over given time intervals following injection.
**Figures 4a, 4b****,** **4c and 4d** show the cumulative food intake over time, calculated from the data represented in Figs. 3a to 3c
**Figures 5a, 5b****,** **5c, 5d** **and** **5e** show the results of experiments in which the appetite suppressing effects in mice of two compounds of the invention were compared with the same quantities of human oxm and saline over given time intervals following injection.
**Figures 6a****,** **6b****,** **6c** **and** **6d** show the cumulative food intake over time as calculated from the data in Figs. 5a to 5e.
**Figures 7a****,** **7b & 7c** show the results of experiments in which the appetite suppressing effects in mice of a compound of the invention were compared with human oxm, porcine (pig) oxm and saline over given time intervals following injection.
**Figures 8a, 8b****,** **8c and 8d** show the cumulative food intake over time for the compound illustrated in Figs. 7a to 7c.
**Figures 9a, 9b** **and** **9c** show the results of experiments in which appetite suppressing effects of three compounds of the invention were compared with human oxm and with saline over given time intervals following injection.
**Figures 10a** **and** **10b** show the cumulative food intake over time, calculated from the data represented in Figs. 9a to 9c.
**Figures 11a****,** **11b****,** **11c** **and** **11d** show the results of experiments in which appetite suppressing effects of two compounds of the invention were compared with human oxm and saline over given time intervals following injection.
**Figures 12a****,** **12b and 12c** show cumulative food intake calculated from the data represented in Figs. 11a to 11d.
**Figures 13a, 13b****,** **13c, 13d** **and** **13e** show the results of experiments in which appetite suppressing effects of the two compounds of the invention represented in Figs. 11a to 11d at lower dosages were compared with human oxm, with exendin 4 and with saline over given time intervals following injection.
**Figures 14a****,** **14b****,** **14c** **and** **14d** show cumulative food intake over time, calculated from the data represented in Figs. 13a to 13e.
**Figures 15a****,** **15b****,** **15c** **and** **15d** show the results of experiments in which appetite suppressing effects of four compounds of the invention were compared with exendin 4 and with saline over given time intervals following injection.
**Figures 16a****,** **16b and 16c** show cumulative food intake over time, calculated from the data presented in Figs. 15a to 15d.
**Figures 17a, 17b****,** **17c, 17d** **and** **17e** show the results of experiments in which the appetite suppressing effects in mice administered with varying dosages of five compounds of the invention was compared with human oxm and saline.
**Figures 18a** **and** **18b** show the results of experiments in which the appetite suppressing effects of a compound of the invention was compared with human oxm and saline at given time intervals following injection.
**Figures 19a to 19e** show the results of experiments in which the appetite suppressing effects of four compounds of the invention were monitored over given time intervals following injection, and compared with a saline control.
**Figures 20 to 116** show the results of further experiments in which the appetite suppressing effects of compounds of the invention were monitored over given time intervals following injection, and compared with a saline control.

### 5. DEFINITIONS

In order to facilitate review of the various embodiments of this disclosure, the following explanations of specific terms are provided:
**Animal:** Living multi-cellular vertebrate organisms, a category that includes, for example, mammals and birds. The term mammal includes both human and non-human mammals. Similarly, the term "subject" includes both human and non-human subjects, including veterinary subjects.
**Appetite:** A natural desire, or longing for food. In one embodiment, appetite is measured by a survey to assess the desire for food. Increased appetite generally leads to increased feeding behavior.
**Appetite Suppressants:** Compounds that decrease the desire for food. Commercially available appetite suppressants include, but are not limited to, amfepramone (diethylpropion), phentermine, mazindol and phenylpropanolamine fenfluramine, dexfenfluramine, sibutramine, rimonabant and fluoxetine.
**Body Mass Index (BMI)**: A mathematical formula for measuring body mass, also sometimes called Quetelet's Index. BMI is calculated by dividing weight (in kg) by height² (in meters²). The recommended classifications for BMI in humans, adopted by the Expert Panel on the Identification, Evaluation and Treatment of Overweight and Obesity in Adults, and endorsed by leading organizations of health professionals, are as follows: Underweight <18.5 kg/m²; Normal weight 18.5-24.9 kg/m²; Overweight 25-29.9 kg/m²; Obesity (Class 1) 30-34.9 kg/m²; Obesity (Class 2) 35-39.9 kg/m²; Extreme obesity (Class 3) ≥40 kg/m² (Practical Guide to the Identification, Evaluation, and Treatment of Overweight and Obesity in Adults, The North American Association for the Study of Obesity (NAASO) and the National Heart, Lung, and Blood Institute (NHLBI) 2000). In one embodiment, a BMI of greater than 25 kg/m² can be used to identify a subject in need of a treatment for excess weight or obesity. Ideal body weight will vary among species and individuals based on height, body build, bone structure, and sex.
**Conservative substitutions:** The replacement of an amino acid residue by another similar residue in a polypeptide. Typical but not limiting conservative substitutions are the replacements, for one another, among the aliphatic amino acids Ala, Val, Leu and Ile; interchange of Ser and Thr containing hydroxy residues, interchange of the acidic residues Asp and Glu, interchange between the amide-containing residues Asn and Gln, interchange of the basic residues Lys and Arg, interchange of the aromatic residues Phe and Tyr, and interchange of the small-sized amino acids Ala, Ser, Thr, Met and Gly.
**Non-conservative substitutions:** The replacement, in a polypeptide, of an amino acid residue by another residue which is not biologically similar. For example, the replacement of an amino acid residue with another residue that has a substantially different charge, a substantially different hydrophobicity or a substantially different spatial configuration.

The phrase "alternative amino acid" as used in the items encompasses alternative amino acids that are the result of both conservative and non-conservative substitutions. In addition to the twenty commonly occurring amino acids that are typically found in naturally occurring polypeptides, rare amino acids, for example, canavanine, ornithine and 5-hydroxytryptophane, and artificial amino acids, that is to say amino acids not normally found *in vivo,* for example t-butylglycine, may be used as "alternative amino acids" in accordance with the invention. Any chiral form of an amino acid may be used.

**Diabetes:** A failure of cells to transport endogenous glucose across their membranes either because of an endogenous deficiency of insulin and/or a defect in insulin sensitivity. Diabetes is a chronic syndrome of impaired carbohydrate, protein, and fat metabolism owing to insufficient secretion of insulin or to target tissue insulin resistance. It occurs in two major forms: insulin-dependent diabetes mellitus (IDDM, type I) and non-insulin dependent diabetes mellitus (NIDDM, type II) which differ in etiology, pathology, genetics, age of onset, and treatment.

The two major forms of diabetes are both characterised by an inability to deliver insulin in an amount and with the precise timing that is needed for control of glucose homeostasis. Diabetes type I, or insulin dependent diabetes mellitus (IDDM) is caused by the destruction of β cells, which results in insufficient levels of endogenous insulin. Diabetes type II, or non-insulin dependent diabetes, results from a defect in both the body's sensitivity to insulin, and a relative deficiency in insulin production.

**Food intake**: The amount of food consumed by an individual subject. Food intake can be measured by volume or by weight. For example, food intake may be the total amount of food consumed by an individual subject. Or, food intake may be the amount of proteins, fat, carbohydrates, cholesterol, vitamins, minerals, or any other food component, of the individual subject. "Protein intake" refers to the amount of protein consumed by an individual. Similarly, "fat intake," "carbohydrate intake," "cholesterol intake," "vitamin intake," and "mineral intake" refer to the amount of fat, carbohydrates, cholesterol, vitamins, or minerals consumed by an individual subject respectively.

**Hyperpolarization:** A decrease in the membrane potential of a cell. Inhibitory neurotransmitters inhibit the transmission of nerve impulses via hyperpolarization. This hyperpolarization is called an inhibitory postsynaptic potential (IPSP). Although the threshold voltage of the cell is unchanged, a hyperpolarized cell requires a stronger excitatory stimulus to reach threshold.

**Normal Daily Diet:** The average food intake for an individual of a given species. A normal daily diet can be expressed in terms of caloric intake, protein intake, carbohydrate intake, and/or fat intake. A normal daily diet in humans generally comprises the following: about 2,000, about 2,400, or about 2,800 to significantly more calories. In addition, a normal daily diet in humans generally includes about 12 g to about 45 g of protein, about 120 g to about 610 g of carbohydrate, and about 11 g to about 90 g of fat. A low calorie diet would be no more than about 85%, and preferably no more than about 70%, of the normal caloric intake of a human individual.

In animals, the caloric and nutrient requirements vary depending on the species and size of the animal. For example, in cats, the total caloric intake per pound, as well as the percent distribution of protein, carbohydrate and fat varies with the age of the cat and the reproductive state. A general guideline for cats, however, is 40 cal/lb/day (18.2 cal/kg/day). About 30% to about 40% should be protein, about 7% to about 10% should be from carbohydrate, and about 50% to about 62.5% should be derived from fat intake. One of skill in the art can readily identify the normal daily diet of an individual of any species.

**Obesity:** A condition in which excess body fat may put a person at health risk (see Barlow and Dietz, Pediatrics 102:E29, 1998; National Institutes of Health, National Heart, Lung, and Blood Institute (NHLBI), Obes. Res. 6 (suppl. 2):51S-209S, 1998). Excess body fat is a result of an imbalance of energy intake and energy expenditure. For example, the Body Mass Index (BMI) may be used to assess obesity. In one commonly used convention, a BMI of 25.0 kg/m² to 29.9 kg/m² is overweight, while a BMI of 30 kg/m² or greater is obese.

In another convention, waist circumference is used to assess obesity. Excess abdominal fat is an important, independent assessment of the risks associated with obesity or being overweight. Waist circumference measurement is particularly useful in patients who are categorised as normal or overweight. It is not usually necessary to measure waist circumference in individuals with BMIs ≥ 35 kg/m² since it adds little to the predictive power of the disease risk classification of BMI. Men who have waist circumferences greater than 40 inches (102 cm), and women who have waist circumferences greater than 35 inches (90 cm), are at higher risk of diabetes, dyslipidemia, hypertension, and cardiovascular disease because of excess abdominal fat. Individuals with waist circumferences greater than these values should be considered one risk category above that defined by their BMI.

Strong evidence shows that obesity affects both the morbidity and mortality of individuals. For example, an overweight or obese individual is at increased risk for heart disease, non-insulin dependent (type 2) diabetes, hypertension, stroke, cancer (e.g. endometrial, breast, prostate, and colon cancer), dyslipidemia, gall bladder disease, sleep apnea, reduced fertility, and osteoarthritis, amongst others (see Lyznicki et al., Am. Fam. Phys. 63:2185, 2001).

**Overweight**: An individual who weighs more than their ideal body weight. An overweight individual can be obese, but is not necessarily obese. For example, an overweight individual is any individual who desires to decrease their weight. In one convention, an overweight individual is an individual with a BMI of 25.0 kg/m² to 29.9 kg/m².

**Pegylation:** the process of reacting a poly(alkylene glycol), preferably an activated poly(alkylene glycol) to form a covalent bond. A facilitator may be used, for example an amino acid, e.g. lysine. Although "pegylation" is often carried out using poly(ethylene glycol) or derivatives thereof, such as methoxy poly(ethylene glycol), the term is not limited herein to the use of methoxy poly(ethylene glycol) but also includes the use of any other useful poly(alkylene glycol), for example poly(propylene glycol). Pegylated shall be defined accordingly.

**Peripheral Administration**: Administration outside of the central nervous system. Peripheral administration does not include direct administration to the brain. Peripheral administration includes, but is not limited to intravascular, intramuscular, subcutaneous, inhalation, oral, rectal, transdermal, buccal, sub-lingual or intra-nasal administration

**Polypeptide**: A polymer in which the monomers are amino acid residues which are joined together through amide bonds. When the amino acids are alpha-amino acids, either the L-optical isomer or the D-optical isomer can be used, the L-isomers being preferred. The terms "polypeptide" or "protein" as used herein encompass any amino acid sequence and include modified sequences such as glycoproteins. The term "polypeptide" covers naturally occurring proteins, as well as those which are recombinantly or synthetically produced. The term "polypeptide fragment" refers to a portion of a polypeptide, for example such a fragment which exhibits at least one useful sequence in binding a receptor. The term "functional fragments of a polypeptide" refers to all fragments of a polypeptide that retain an activity of the polypeptide. Biologically functional peptides can also include fusion proteins, in which the peptide of interest is fused to another peptide(s).

**Therapeutically effective amount:** A dose sufficient to prevent advancement, or to cause regression of a disorder, or which is capable of relieving a sign or symptom of a disorder, or which is capable of achieving a desired result. In several embodiments, a therapeutically effective amount of a compound of the invention is an amount sufficient to inhibit or halt weight gain, or an amount sufficient to decrease appetite, or an amount sufficient to reduce caloric intake or food intake or increase energy expenditure, or an amount sufficient to reduce weight, or to reduce the risk of mortality or morbidity from conditions associated with the disorder.

**Note on Nomenclature:** The analogues of oxm refered to herein, are named in such a way that their amino acid sequence may be derived from their names. "oxm" refers to the wild type sequence of human oxm. Regions in which the sequence of oxm has been replaced by the corresponding exendin-4 sequence are identified in the manner "oxm(ex-15-27)" in which example residues 15 to 27 of oxm have been replaced by corresponding residues of exendin-4. Further amino acid substitutions or terminal extensions are refered to in the manner "Ser3-oxm" in which example the third amino acid of oxm has been replaced by Serine. The two conventions of nomenclature may be combined in the manner of "Leu18-oxm(ex15-27)". It should be noted that when both conventions are used together, the specifically named residue takes priority so that in the example "Leu18-oxm(ex15-27), residue 18 is Leu and not Ala which is exendin's 18^{th} residue.

### 6. DETAILED DESCRIPTION

The inventors have found that, surprisingly, oxm analogues of the invention are effective appetite suppressants and/or have a more sustained effect than native oxm on food intake, and/or have a more potent effect than native oxm on food intake. They also have a longer half-life or clearance time or improved resistance to degradation as compared with oxm. Increased duration of appetite suppression can be particularly important to avoid the effect known as "escape". A short duration appetite suppressant may reduce appetite for the time covered by one meal and, in that meal, the subject typically eats less food. If, however, the appetite suppressant has a short half-life or rapid clearance time or is then metabolized or otherwise removed from the circulation of the subject, then by the time of the next mealtime, the subject can regain its "normal" appetite. In view of the subject having eaten a small meal at the previous mealtime, the subject may in fact have an increased appetite by the time of the subsequent meal. If the subject satisfies that appetite, it is possible for the food intake over the two meals, in total, to be no lower than the food intake would have been without the appetite suppressant. That is to say that the subject may have "escaped" from the effects of the appetite suppressant. "Escape" can be reduced by using additional doses of appetite suppressant, or by using an appetite suppressant with a longer duration of action. If the subject has a reduced appetite for longer, then the degree to which it can make up the missed food from one meal in the next meal is reduced as there is a practical limit to the total capacity for food in a particular meal. Repeated or continuous administration of a compound over a period of time, for example over a period of days or weeks, will lead to extended appetite suppression and reduced potential for escape from the effects of the appetite suppression.

The improved activity and/or duration of action of the oxm analogues as compared with oxm offers various advantages. For example, effective suppression of appetite at lower dosages will be permitted (with the lower dosage and/or lower peak levels, offering the prospect of reduced side effects (including nausea) and reduction in the cost of treatment), or usage at relatively high dosages will be better tolerated by the patient enabling quicker and/or greater weight loss. It is thought that the appetite suppressing effect and the induced nausea of oxm will operate by virtue of different pathways, which may be separable. Based on that premise, by choice of appropriate amino acid substitutions in the oxm molecule, it is thought that it may be possible for the oxm analogues so obtained to have good appetite suppressant activity which is de-coupled wholly or in part from the nausea that is commonly experienced in the use of, for example, the highly nauseating appetite suppressant exendin, or that has recently been noted in the use of higher dosages of oxm by some subjects. Certain of the compounds of the invention used in the Examples herein exhibit a pattern of appetite suppression indicative of a 'flatter blood curve', that is to say they have a more gradual onset of the appetite suppressant activity than oxm and thereby potentially avoid an initial sharp peak (which may be associated with nausea) and a potentially longer duration of action.

Preferably, oxm, or oxm fragments referred to herein with reference to compounds of the invention, especially the fragments X, X', Y and Y' of Formulae (I), (II) and (III), are human oxm or human oxm fragments. According to another embodiment they may be porcine oxm or porcine oxm fragments.

The human (which is the same as the rat and hamster) full length oxm sequence is shown in SEQ ID NO: 7. Human oxm1-14 is shown in SEQ ID NO: 8, and corresponds to human full length oxm without residues 15 to 37. Human oxm1-26 is shown in SEQ ID NO: 9, and corresponds to human full length oxm without residues 27 to 37. Human oxm 15-37 (SEQ ID NO: 1) corresponds to human full length oxm without residues 1 to 14 at the N-terminal end. Human oxm 15-24 (SEQ ID NO: 3) corresponds to human full length oxm without residues 1 to 14 at the N-terminal end and residues 25 to 37 at the C-terminal end. Human oxm27-33 (SEQ ID NO: 5) corresponds to human full length oxm without residues 1 to 26 at the N-terminal end and 34 to 37 at the C-terminal end. Human oxm 34-37 is shown in SEQ ID NO: 10, and corresponds to human full length oxm without residues 1 to 33 at the N-terminal end. Human oxm3-37 is shown in SEQ ID NO: 11 and corresponds to human full length oxm without residues 1 and 2 at the N-terminal end. The numerals refer to the location on the full length oxm molecule, beginning at the N-terminus. Analogous numbering is used for other fragments and variants of oxm described herein.

**Table 1: Sequences of oxm and certain oxm fragments**

| | |
|---|---|
| Human oxm | His Ser Gln Gly Thr Phe Thr Ser Asp Tyr Ser Lys Tyr Leu Asp Ser Arg Arg Ala Gln Asp Phe Val Gln Trp Leu Met Asn Thr Lys Arg Asn Arg Asn Asn Ile Ala   SEQ ID NO: 7 |
| Human oxm1-14 | His Ser Gln Gly Thr Phe Thr Ser Asp Tyr Ser Lys Tyr Leu SEQ ID NO: 8 |
| Human oxm1-26 | His Ser Gln Gly Thr Phe Thr Ser Asp Tyr Ser Lys Tyr Leu Asp Ser Arg Arg Ala Gln Asp Phe Val Gln Trp Leu   SEQ ID NO: 9 |
| Human oxm34-37 | Asn Asn Ile Ala SEQ ID NO: 10 |
| Human oxm3-37 | Gln Gly Thr Phe Thr Ser Asp Tyr Ser Lys Tyr Leu Asp Ser Arg Arg Ala Gln Asp Phe Val Gln Trp Leu Met Asn Thr Lys Arg Asn Arg Asn Asn Ile Ala   SEQ ID NO: 11 |
| Human oxm15-37 | Asp Ser Arg Arg Ala Gln Asp Phe Val Gln Trp Leu Met Asn Thr Lys Arg Asn Arg Asn Asn Ile Ala   SEQ ID NO: 1 |
| Human oxm15-24 | Asp Ser Arg Arg Ala Gln Asp Phe Val Gln SEQ ID No: 3 |
| Human oxm27-33 | Met Asn Thr Lys Arg Asn Arg SEQ ID NO: 5 |
| Porcine oxm | His Ser Gln Gly Thr Phe Thr Ser Asp Tyr Ser Lys Tyr Leu Asp Ser Arg Arg Ala Gln Asp Phe Val Gln Trp Leu Met Asn Thr Lys Arg Asn Lys Asn Asn Ile Ala   SEQ ID NO: 12 |
| Eel oxm* | His Ser Gln Gly Thr Phe Thr Asn Asp Tyr Ser Lys Tyr Leu Glu Thr Arg |
| | Arg Ala Gln Asp Phe Val Gln Trp Leu Met Asn Ser Lys Arg Ser Gly Gly Pro Thr   SEQ ID NO:13 |

| | |
|---|---|
| *See Uesaka et al, "Glucagon-like peptide isolated from the eel intestine: effects on atrial beating", Journal of Experimental Biology, 204, 3019-3026 (2001). | |

The oxm fragments in a molecule of the invention may be, or may be related to, an oxm from a species other than human. The sequence of porcine (which is the same as the bovine) oxm and eel oxm are included in Table 1 (SEQ ID NO: 12 and SEQ ID NO: 13, respectively) by way of example. The oxm fragments in a molecule of the invention may also be taken from any of those sequences.

The invention includes in particular compounds of formula I in which residues 15 to 37 are as defined in line A except in so far that one or more residues 15 to 37 as defined in line A is substituted by the corresponding numbered residue in line B, and wherein optionally one or more further residues from position 15 to position 24 of line A are substituted by the corresponding numbered residues of line R. The extension moiety, where present, may comprise from 1 to 6 amino acids, preferably from 1 to 4 amino acids, and especially two amino acids.
In a first preferred embodiment, oxm analogues of the invention may have a first fragment corresponding to an N-terminal fragment of native oxm, a second fragment corresponding to a C-terminal fragment of native oxm and between and interconnecting the first and second fragments an intermediate fragment having the sequence of oxm15-24 (SEQ ID NO: 3) except for the substitution of one or more amino acids or groups of amino acids by a corresponding number of amino acids taken from the sequence below:

**(SEQ ID NO: 4)**

| Posn | 15 | 16 | 17 | 18 | 19 | 20 | 21 | 22 | 23 | 24 |
|---|---|---|---|---|---|---|---|---|---|---|
| AA | Glu | Glu | Glu | Ala | Val | Arg | Leu | Phe | Ile | Glu |

in which any said substitution is carried out at the correspondingly numbered position in the oxm15-24. Preferably the first fragment comprises oxm1-x in which x is an integer in the range of from 14 to 23, for example, from 14 to 21, preferably 14 to 20, more preferably 14 to 18 and especially 14. In an especially preferred embodiment, the oxm analogue is oxm1-14sub15-24oxm25-37, in which sub15-24 indicates substitution of all of the amino acids at positions 15 to 24 by the entire sequence SEQ ID NO: 4. Certain preferred compounds have the sequence of full-length oxm except for replacement of from 3 to 10 amino acids at or between positions 15 to 24 of the oxm molecule by 3 to 10 sequential residues commencing from the N-Terminus of SEQ ID NO: 4. Replacement of four residues, for example, replacement of oxm15-18 by residues 1 to 4 of SEQ ID NO: 4 may give increased and/or prolonged inhibition of food intake compared to a saline control and compared to oxm. Replacement of more than four residues, for example of five, advantageously six, and preferably seven residues gives compounds with still greater appetite suppressant activity. Especially advantageous properties in terms of appetite suppressant activity are observed when from seven to ten residues, commencing from oxm15 inclusive, are replaced by from seven to ten sequential residues of corresponding number from SEQ ID NO: 4.

With reference to the moiety S2 in Formula II, the full sequence in line D
(SEQ ID NO: 4) corresponds to residues 15-24 of exendin 4. As mentioned above, exendin 4 is known to have appetite suppressant activity but its usefulness is limited by the side effect of nausea encountered in its use (Buse et al., Diabetes Care, 27(11), 2628-2635, 2004). It is believed that the oxm analogues of the invention will have certain characteristics in common with oxm (including absence or reduced levels of nausea compared with exendin); whilst as demonstrated herein they may have prolonged activity as compared with native oxm. Furthermore, the regions of exendin that have an advantageous effect on the anorectic properties of oxm have not previously been associated with this effect.

Referring to the moiety Z in any of the Formulae herein, Z may be an amino acid sequence of any two amino acid residues followed by Gln, Asp or Glu, most preferably Z is an amino acid sequence of any two amino acid residues followed by Gln.

Referring to the moiety S2 in Formula (II), it is preferred for at least three amino acids, for example at least four amino acids, preferably at least six amino acids, and especially from seven to ten amino acids from line C to be substituted by correspondingly numbered amino acids from line D. Advantageously, the substituted amino acids comprise at least one sequential group of two or more amino acids, and preferably at least one sequential group of at least four amino acids. Preferably, the substituted amino acids comprise a sequential group of not more than ten amino acids. A substituted sequential group may have from 5 to 10 amino acids, for example, from 6 to 10 amino acids, preferably from 7 to 10 amino acids, most preferably from 8 to 10 amino acids, especially 9 or 10 amino acids.

Optionally, oxm analogues of the invention may comprise further amino acid residue substitutions wherein one or more amino acids or groups of amino acids from positions 15 to 24 may be substituted by a corresponding number of amino acids taken from the sequence below:

**(SEQ ID NO: 145)**

| Posn | 15 | 16 | 17 | 18 | 19 | 20 | 21 | 22 | 23 | 24 |
|---|---|---|---|---|---|---|---|---|---|---|
| AA | Arg | Ile | Glu | Ile | Val | Lys | Tyr | Phe | Ile | Gly |

Referring to the moiety S2 in Formula (II), it is preferred for at least three amino acids, for example at least four amino acids, preferably at least six amino acids, and especially from seven to ten amino acids from line A to be substituted by correspondingly numbered amino acids from line R. Advantageously, the substituted amino acids comprise at least one sequential group of two or more amino acids, and preferably at least one sequential group of at least four amino acids. Preferably, the substituted amino acids comprise a sequential group of not more than ten amino acids. A substituted sequential group may have from 5 to 10 amino acids, for example, from 6 to 10 amino acids, preferably from 7 to 10 amino acids, most preferably from 8 to 10 amino acids, especially 9 or 10 amino acids.

Preferred compounds of the said first preferred embodiment of the invention include :

His Ser Gln Gly Thr Phe Thr Ser Asp Tyr Ser Lys Tyr Leu Glu Glu Glu Ala Ala Gln Asp Phe Val
Gln Trp Leu Met Asn Thr Lys Arg Asn Arg Asn Asn Ile Ala (SEQ ID NO: 14)

His Ser Gln Gly Thr Phe Thr Ser Asp Tyr Ser Lys Tyr Leu Glu Glu Glu Ala Val Arg Leu Phe Val
Gln Trp Leu Met Asn Thr Lys Arg Asn Arg Asn Asn Ile Ala  (SEQ ID NO: 15)

His Ser Gln Gly Thr Phe Thr Ser Asp Tyr Ser Lys Tyr Leu Glu Glu Glu Ala Val Arg Leu Phe Ile
Glu Trp Leu Met Asn Thr Lys Arg Asn Arg Asn Asn Ile Ala    (SEQ ID NO: 16).

In a second preferred embodiment, oxm analogues of the invention may have a first fragment corresponding to an N-terminal fragment of native oxm, a second fragment corresponding to C-terminal fragment of native oxm and between and interconnecting the first and second fragments an intermediate fragment having the sequence of oxm27-33 (SEQ ID NO: 5) except for the substitution of one or more amino acids or groups of amino acids by a corresponding number of amino acids taken from the sequence below:

**(SEQ ID NO: 6)**

| Position | 27 | 28 | 29 | 30 | 31 | 32 | 33 |
|---|---|---|---|---|---|---|---|
| Acid | Lys | Asn | Gly | Gly | Pro | Ser | Ser |

in which any said substitution is carried out at the correspondingly numbered position in the oxm27-33. Preferably the first fragment comprises oxm1 -y in which y is an integer in the range of from 26 to 32, preferably 26 to 30, especially 26 or 30. In an especially preferred embodiment, the oxm analogue is oxm1-26sub27-33oxm34-37, in which sub27-33 indicates substitution of all of the amino acids at positions 27 to 33 by the correspondingly numbered amino acids given in SEQ ID NO: 6. Certain preferred compounds have the sequence of oxm except for replacement of from 4 to 7 amino acids at or between positions 27 to 33 of the oxm molecule by 4 to 7 sequential residues commencing from the N-Terminus of SEQ ID NO: 6. Replacement of four residues, for example, replacement of oxm27-30 by residues 27 to 30 of SEQ ID NO: 6 may give increased and/or prolonged inhibition of food intake compared to a saline control and compared to oxm. Replacement of more than four residues, for example of five, advantageously six, and preferably seven residues may give compounds with still greater appetite suppressant activity. Especially advantageous properties in terms of appetite suppressant activity are observed when seven sequential residues, commencing from oxm27 inclusive, are replaced by seven sequential residues of corresponding number from SEQ ID NO: 6.

With reference to the moiety S3 in Formula (III), the full sequence in line F corresponds to residues 27-33 of exendin 4. As mentioned above, exendin 4 is known to have appetite suppressant activity but its usefulness is limited by the side effect of nausea encountered in its use (Buse et al., Diabetes Care, 27(11), 2628-2635, 2004). Furthermore, the regions of exendin that have an advantageous effect on the anorectic properties of oxm have not previously been associated with this effect.

Thus, referring to the moiety S3 in Formula (III), it is preferred for at least three amino acids, for example at least four amino acids, preferably at least six amino acids and especially seven amino acids from line E to be substituted by correspondingly numbered amino acids from line F. Advantageously, the substituted amino acids comprise at least one sequential group of two or more amino acids, and preferably at least one sequential group of at least four amino acids. Preferably, the substituted amino acids comprise a sequential group of not more than seven amino acids.

A substituted sequential group may have from 3 to 7 amino acids, for example, from 4 to 7 amino acids, for example, four amino acids, five amino acids, six amino acids or, most preferably, seven amino acids.

Variants may be as defined above with reference to the first embodiment.

Preferred compounds of the said second aspect of the invention include compounds of sequences:

His Ser Gln Gly Thr Phe Thr Ser Asp Tyr Ser Lys Tyr Leu Asp Ser Arg Arg Ala Gln Asp Phe
Val Gln Trp Leu Met Asn Thr Gly Pro Ser Ser Asn Asn Ile Ala   (SEQ ID NO: 17)

His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Arg Asn Arg Asn Asn Ile Ala   (SEQ ID NO: 18)

His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Ser Ser Asn Asn Ile Ala   (SEQ ID NO: 19).

In the third preferred embodiment of the invention, a compound consists of the formula A¹-A³-oxm4-r in which r is from 15 to 37, and A¹ is an amino acid other than His (preferably D-Histidine), A² is Ala or Ser, and A³ is Glu, Asp or Gln. In this specification "His" is to be understood as referring to L-histidine unless it is explicitly indicated by the preface "D-" that D-histidine is intended. Preferably, A¹-A³- represents D-His-Ala-Asp-, D-His-Ser-Asp-, D-His-Ala-Glu-, D-His-Ser-Glu-, D-His-Ala-Gln-, or D-His-Ser-Gln.

In certain especially preferred embodiments of the invention, an oxm analogue may comprise two or more of the following:
(i) one or more substitutions at or between oxm positions 15 to 24 as defined with reference to Formula (II);
(ii) one or more substitutions at or between oxm positions 27 to 33 as defined with reference to Formula (III);
(iii) Z representing D-His-Ser-Asp-, D-His-Ala-Asp-, His-Ala,Asp-, D-His-Ala-Glu-, D-His-Ser-Glu-, D-His-Ala-Gln-, or D-His-Ser-Gln;
(iv) L-leucine at position 18; and
(v) an extension moiety comprising one to five, for example two amino acids at positions 38 onwards.

For example, a first preferred group of oxm analogues has (i) above in combination with (iii) above. A second preferred group of oxm analogues has (ii) above in combination with (iii) above. Further preferred groups of oxm analogues has (i) and (iii) or all of (i), (ii) and (iii) above. Thus, certain preferred analogues are defined by the formula (IV)

Z-X-S2-Trp-Leu-S3-Y' (IV)

in which Z and X are as defined with reference to formula (I), S2 is as defined with reference to formula (II) and S3 and Y' are as defined with reference to formula (III) with any of the preferred identities of Z, S2 and S3 defined with reference to formulae (I), (II) and (III) analogously being applicable to the above indicated formula.

Yet further especially preferred oxm analogues of the invention, and especially analogues of any of the preferred embodiments discussed above, including those especially preferred embodiments, may advantageously include an extension moiety of, preferably, two amino acids at position 38 and 39. In one advantageous embodiment the extension moiety is -Pro-Ser. In another advantageous embodiment the extension moiety is -Ala-Ala. Other preferred embodiments include -Ala-Ala-Lys; and -Ala-Ala-Glu-Glu-Lys. Compounds of the invention may feature extension moieties in combination with embodiments (i) and/or (ii) and/or (iii) and especially with all of (i) to (iii). It is thought that the presence of an extension moiety may protect against degradation, thus decreasing the rate of degradation and increasing the half-life of the analogue. In a fourth embodiment of the invention, an oxm analogue of Formula (I) comprises as N-terminal fragment oxm1-26, with oxm residues 27 to 37 being replaced the correspondingly numbered amino acids 27 to 37 of line B above.

For the avoidance of doubt, where substitution takes place at position 22 and/or position 28, at least one and preferably two or more further amino acids will also be substituted.

The invention also provides a compound of the general formula:

Z-X-S4-S5-E (V)

in which:
X is oxm 4-14;
Z is an amino acid sequence of three amino acid residues, for example having any of the preferred identities given above for Z in relation to general formula I;
S4 represents a ten amino acid sequence consisting of from 0 to 10 correspondingly positioned amino acids from the sequence Asp Ser Arg Arg Ala Gln Asp Phe Val Gln (SEQ ID NO: 35), from 1 to 10 correspondingly positioned amino acids from the sequence Glu Glu Glu Ala Val Arg Leu Phe Ile Glu (SEQ ID NO: 4), optionally from 0 to 9 correspondingly positioned amino acids from the sequence Arg Ile Glu Ile Val Lys Tyr Phe Ile Gly (SEQ ID NO: 145) and from 0 to 5 amino acids that differ in identity from the correspondingly positioned amino acids in SEQ ID NO: 4 and SEQ ID NO: 35,
S5 represents oxm25-37 or represents oxm25-37 in which at least one residue at positions 27 to 33 has been substituted by one or more correspondingly numbered residues from the sequence Lys(27) Asn(28) Gly(29) Gly(30) Pro(31) Ser(32) Ser(33) (SEQ ID NO: 24); and
E represents an optional extension moiety comprising one or more amino acid residues, for example any of the extension moieties indicated as preferred extension moieties with reference to general formula I;
a variant or derivative thereof;
or a salt or solvate thereof.

In the formula V, S4 advantageously contains at least three correspondingly positioned amino acids from SEQ ID NO: 4, and in one advantageous embodiment S4 includes at least one amino acid differing in identity from the correspondingly numbered amino acids in SEQ. ID NO: 4 and SEQ ID NO: 35. Preferably, S4 contains at least six correspondingly positioned amino acids from SEQ ID NO: 4 and at least one amino acid differing in identity to the correspondingly numbered amino acids in SEQ ID NO: 4 and SEQ ID NO: 35. More preferably, S4 is made up of nine correspondingly positioned amino acids from SEQ ID NO: 4 and at least one amino acid differing in identity to the correspondingly numbered amino acids in SEQ ID NO: 4 and SEQ ID NO: 35. The inventors have found that a number of such compounds exhibit a delayed onset of appetite suppressant activity as compared with other compounds of the invention (SEQ ID NO: 16 and SEQ ID NO: 7), which may in particular have the advantage that greater (and therefore longer-lasting) dosages of those compounds can be administered as compared with, for example, oxm itself, thereby reducing the likelihood of so-called "burst nausea", that is, an initial nausea associated with high blood concentration of the active peptides immediately following administration and/or allowing a greater effect with faster weight loss. Illustrative of compounds according to this aspect of the invention are:
His Ser Gln Gly Thr Phe Thr Ser Asp Tyr Ser Lys Tyr Leu Glu Glu Glu Val Val Arg Leu Phe Ile Glu Trp Leu Met Asn Thr Lys Arg Asn Arg Asn Asn Ile Ala (SEQ ID NO: 31)

His Ser Gln Gly Thr Phe Thr Ser Asp Tyr Ser Lys Tyr Leu Glu Glu Glu Ala Val Arg Ile Phe Ile
Glu Trp Leu Met Asn Thr Lys Arg Asn Arg Asn Asn Ile Ala (SEQ ID NO: 32)

His Ser Gln Gly Thr Phe Thr Ser Asp Tyr Ser Lys Tyr Leu Glu Glu Glu Ala Val Arg Leu Phe
Leu Glu Trp Leu Met Asn Thr Lys Arg Asn Arg Asn Asn Ile Ala (SEQ ID NO: 33)

and

His Ser Gln Gly Thr Phe Thr Ser Asp Tyr Ser Lys Tyr Leu Glu Glu Glu Ala Ile Arg Leu Phe Ile
Glu Trp Leu Met Asn Thr Lys Arg Asn Arg Asn Asn Ile Ala (SEQ ID NO: 34)

The peptides SEQ ID NO: 31 and SEQ ID NO: 34 in particular exhibit the potentially advantageous delayed onset mentioned above.

According to the embodiment of the invention shown in Formula VI, X may be 1, 2, 3, 4 or 5 amino acids or more than 5, for example, 5 to 10 or 5 to 20 amino acids. X may be Ala-Y wherein Y is any one or more amino acids or is absent.

Further examples of compounds of the invention include:

His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Ser Ser Gly Ala Pro Pro Pro Ser;

His Ser Gln Gly Thr Phe Thr Ser Asp Tyr Ser Lys Tyr Leu Glu Glu Glu Ala Val Arg Leu Phe Ile
Glu Trp Leu Lys Asn Gly Gly Pro Ser Ser Gly Ala Pro Pro Pro Ser;

D-His Ser Gln Gly Thr Phe Thr Ser Asp Tyr Ser Lys Tyr Leu Glu Glu Glu Ala Val Arg Leu Phe
Ile Glu Trp Leu Lys Asn Gly Gly Pro Ser Ser Gly Ala Pro Pro Pro Ser;

His Ser Gln Gly Thr Phe Thr Ser Asp Tyr Ser Lys Tyr Leu Asp Glu Glu Ala Val Arg Leu Phe Ile
Glu Trp Leu Met Asn Thr Lys Arg Asn Arg Asn Asn Ile Ala;

His Ser Gln Gly Thr Phe Thr Ser Asp Tyr Ser Lys Tyr Leu Glu Glu Glu Ala Val Arg Leu Phe Ile
Gln Trp Leu Met Asn Thr Lys Arg Asn Arg Asn Asn Ile Ala;

D-His Ala Gln Gly Thr Phe Thr Ser Asp Tyr Ser Lys Tyr Leu Glu Glu Glu Ala Val Arg Leu Phe
Val Gln Trp Leu Met Asn Thr Lys Arg Asn Arg Asn Asn Ile Ala;

His Ser Gln Gly Thr Phe Thr Ser Asp Tyr Ser Lys Tyr Leu Glu Glu Glu Ala Ala Arg Leu Phe Ile
Glu Trp Leu Met Asn Thr Lys Arg Asn Arg Asn Asn Ile Ala;

His Ser Gln Gly Thr Phe Thr Ser Asp Tyr Ser Lys Tyr Leu Glu Glu Glu Ala Val Arg Leu Phe Val
Glu Trp Leu Met Asn Thr Lys Arg Asn Arg Asn Asn Ile Ala;

His Ser Gln Gly Thr Phe Thr Ser Asp Tyr Ser Lys Tyr Leu Glu Glu Glu Ala Val Arg Leu Phe Ile
Glu Trp Leu Met Asn Thr Lys Arg Asn Arg Asn Asn Ile Ala;

His Ser Gln Gly Thr Phe Thr Ser Asp Tyr Ser Lys Tyr Leu Glu Glu Glu Ala Val Arg Leu Phe Val
Gln Trp Leu Met Asn Thr Lys Arg Asn Lys Asn Asn Ile Ala;

His Ser Gln Gly Thr Phe Thr Ser Asp Tyr Ser Lys Tyr Leu Glu Glu Glu Ala Val Lys Leu Phe Ile
Glu Trp Leu Met Asn Thr Lys Arg Asn Arg Asn Asn Ile Ala;

His Ser Gln Gly Thr Phe Thr Ser Asp Tyr Ser Lys Tyr Leu Glu Glu Asp Ala Val Arg Leu Phe Ile
Glu Trp Leu Met Asn Thr Lys Arg Asn Arg Asn Asn Ile Ala;

His Ser Gln Gly Thr Phe Thr Ser Asp Tyr Ser Lys Tyr Leu Glu Glu Glu Ala Val Arg Leu Phe Ile
Asp Trp Leu Met Asn Thr Lys Arg Asn Arg Asn Asn Ile Ala;

His Ser Gln Gly Thr Phe Thr Ser Asp Tyr Ser Lys Tyr Leu Glu Glu Glu Gln Val Arg Leu Phe Ile
Glu Trp Leu Met Asn Thr Lys Arg Asn Arg Asn Asn Ile Ala;

His Ser Gln Gly Thr Phe Thr Ser Asp Tyr Ser Lys Tyr Leu Glu Glu Glu Ala Ile Arg Leu Phe Ile
Glu Trp Leu Met Asn Thr Lys Arg Asn Arg Asn Asn Ile Ala;

His Ser Gln Gly Thr Phe Thr Ser Asp Tyr Ser Lys Tyr Leu Glu Glu Glu Ala Val Arg Leu Phe
Lys Gln Trp Leu Met Asn Thr Lys Arg Asn Arg Asn Asn Ile Ala;

His Ser Gln Gly Thr Phe Thr Ser Asp Tyr Ser Lys Tyr Leu Gln Glu Glu Ala Val Arg Leu Phe Val
Gln Trp Leu Met Asn Thr Lys Arg Asn Arg Asn Asn Ile Ala;

His Ser Gln Gly Thr Phe Thr Ser Asp Tyr Ser Lys Tyr Leu Glu Gln Glu Ala Val Arg Leu Phe Val
Gln Trp Leu Met Asn Thr Lys Arg Asn Arg Asn Asn Ile Ala;

His Ser Gln Gly Thr Phe Thr Ser Asp Tyr Ser Lys Tyr Leu Glu Glu Glu Val Val Arg Leu Phe Val
Gln Trp Leu Met Asn Thr Lys Arg Asn Arg Asn Asn Ile Ala;

His Ser Gln Gly Thr Phe Thr Ser Asp Tyr Ser Lys Tyr Leu Glu Glu Glu Val Ile Arg Leu Phe Val
Gln Trp Leu Met Asn Thr Lys Arg Asn Arg Asn Asn Ile Ala;

His Ser Gln Gly Thr Phe Thr Ser Asp Tyr Ser Lys Tyr Leu Glu Glu Glu Val Ile Arg Leu Phe Val
Gln Trp Leu Met Asn Thr Lys Arg Asn Arg Asn Asn Ile Ala;

D-His Ala Gln Gly Thr Phe Thr Ser Asp Tyr Ser Lys Tyr Leu Glu Glu Glu Val Ile Arg Ile Phe
Val Gln Trp Leu Met Asn Thr Lys Arg Asn Arg Asn Asn Ile Ala;

D-His Ala Gln Gly Thr Phe Thr Ser Asp Tyr Ser Lys Tyr Leu Glu Glu Glu Val Val Arg Leu Phe
Val Gln Trp Leu Met Asn Thr Lys Arg Asn Arg Asn Asn Ile Ala;

D-His Ala Gln Gly Thr Phe Thr Ser Asp Tyr Ser Lys Tyr Leu Glu Glu Glu Ala Val Arg Leu Phe
Ile Gln Trp Leu Met Asn Thr Lys Arg Asn Lys Asn Asn Ile Ala;

D-His Ala Gln Gly Thr Phe Thr Ser Asp Tyr Ser Lys Tyr Leu Glu Glu Glu Ala Val Arg Leu Phe
Val Gln Trp Leu Met Asn Thr Lys Arg Asn Lys Asn Asn Ile Ala Ala Ala;

His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Asn Ser Asn Asn Ile Ala;

His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Arg Asn Arg Asn Asn Ile Ala;

His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Asn Arg Asn Asn Ile Ala;

His Ser Gln Gly Thr Phe Thr Ser Asp Tyr Ser Lys Tyr Leu Asp Ser Arg Arg Ala Gln Asp Phe
Val Gln Trp Leu Met Asn Gly Gly Pro Ser Arg Asn Asn Ile Ala;

His Ser Gln Gly Thr Phe Thr Ser Asp Tyr Ser Lys Tyr Leu Asp Ser Arg Arg Ala Gln Asp Phe
Val Gln Trp Leu Met Asn Gly Gly Pro Ser Ser Asn Asn Ile Ala;

His Ser Gln Gly Thr Phe Thr Ser Asp Tyr Ser Lys Tyr Leu Asp Ser Arg Arg Ala Gln Asp Phe
Val Gln Trp Leu Arg Asn Gly Gly Pro Ser Ser Asn Asn Ile Ala;

His Ser Gln Gly Thr Phe Thr Ser Asp Tyr Ser Lys Tyr Leu Asp Ser Arg Arg Ala Gln Asp Phe
Val Gln Trp Leu Lys Asn Thr Gly Pro Ser Ser Asn Asn Ile Ala;

D-His Ala Gln Gly Thr Phe Thr Ser Asp Tyr Ser Lys Tyr Leu Glu Gln Glu Val Val Arg Leu Phe
Ile Gln Trp Leu Lys Asn Gly Gly Pro Ser Ser Asn Asn Ile Ala Ala Ala;

D-His Ala Gln Gly Thr Phe Thr Ser Asp Tyr Ser Lys Tyr Leu Glu Gln Glu Val Ile Arg Leu Phe
Ile Gln Trp Leu Lys Asn Gly Gly Pro Ser Ser Asn Asn Ile Ala Ala Ala;

D-His Ala Gln Gly Thr Phe Thr Ser Asp Tyr Ser Lys Tyr Leu Glu Gln Glu Val Ile Arg Leu Phe
Leu Gln Trp Leu Lys Asn Gly Gly Pro Ser Ser Asn Asn Ile Ala Ala Ala;

His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala Glu Glu Lys;

D-His Ala Gln Gly Thr Phe Thr Ser Asp Tyr Ser Lys Tyr Leu Gln Gln Gln Val Ile Arg Ile Phe Ile
Gln Trp Leu Lys Asn Gly Gly Pro Ser Lys Asn Asn Ile Ala Ala Ala;

D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala Lys;

D-His Ala Gln Gly Thr Phe Thr Ser Asp Tyr Ser Lys Tyr Leu Glu Glu Glu Ala Val Arg Leu Phe
Val Gln Trp Leu Lys Asn Gly Gly Pro Ser Lys Asn Asn Ile Ala Ala Ala Lys;

His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Lys Asn Asn Ile Ala Ala Ala Lys;

D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys Asn Asn Ile Ala Ala Ala;

D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys Asn Asn Ile Ala Ala Ala;

D-His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Ser Ser Gly Ala Pro Pro Pro Ser;

His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Ser Ser Gly Ala Pro Pro Pro Ser;

D-His Ala Gln Gly Thr Phe Thr Ser Asp Tyr Ser Lys Tyr Leu Glu Glu Glu Ala Val Arg Leu Phe
Ile Gln Trp Leu Met Asn Thr Lys Arg Asn Lys-lauroyl Asn Asn Ile Ala;

D-His Ala Gln Gly Thr Phe Thr Ser Asp Tyr Ser Lys Tyr Leu Glu Glu Glu Ala Val Arg Leu Phe
Ile Gln Trp Leu Met Asn Thr Lys Arg Asn Lys-palmitoyl Asn Asn Ile Ala;

D-His Ala Gln Gly Thr Phe Thr Ser Asp Tyr Ser Lys Tyr Leu Glu Glu Glu Ala Val Arg Leu Phe
Val Gln Trp Leu Met Asn Thr Lys Arg Asn Lys-palmitoyl Asn Asn Ile Ala;

D-His Ala Gln Gly Thr Phe Thr Ser Asp Tyr Ser Lys Tyr Leu Glu Glu Glu Val Val-palmitoyl
Arg Leu Phe Val Gln Trp Leu Met Asn Thr Lys Arg Asn Arg Asn Asn Ile Ala;

D-His Ala Gln Gly Thr Phe Thr Ser Asp Tyr Ser Lys Tyr Leu Glu Glu Glu Ala Val Arg Leu Phe
Val Gln Trp Leu Lys Asn Gly Gly Pro Ser Lys Asn Asn Ile Ala Ala Ala Lys-palmitoyl;

His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Lys Asn Asn Ile Ala Ala Ala Lys-palmitoyl;

D-His Ala Gln Gly Thr Phe Thr Ser Asp Tyr Ser Lys Tyr Leu Glu Glu Glu Ala Val Arg Leu Phe
Val Gln Trp Leu Lys Asn Gly Gly Pro Ser Lys Asn Asn Ile Ala Ala Ala Lys-lauroyl;

D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala Lys-palmitoyl;

D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala Lys-lauroyl;

D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys-palmitoyl Asn Asn Ile Ala Ala Ala;

His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala Glu Glu Lys-palmitoyl;

His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala Ala;

His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala Ala Ala Lys;

His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala Ala Ala Ala Ala Ala;

His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala Ala Ala Tyr;

His Ser Gln Gly Thr Phe Thr Ser Asp Tyr Ser Lys Tyr Leu Asp Ser Arg Arg Ala Gln Asp Phe
Val Gln Trp Leu Met Asn Thr Lys-dodecyl Arg Asn Arg Asn Asn Ile Ala;

His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Lys-dodecyl Asn Asn Ile Ala;

His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Lys-palmitoyl Asn Asn Ile Ala.

The invention further comprises embodiments incorporating the sequences disclosed in the attached examples and/or figures.

### Variants

Oxm referred to herein includes variants of oxm. The oxm analogues of the invention incorporate one or more oxm fragments, including in particular but not exclusively fragments selected from the group consisting of the fragments oxm1-14, oxm1-26, oxm3-37, oxm4-37, oxm34-37 and oxm25-37. The oxm or oxm fragment may be a variant of native oxm or of a native oxm fragment, for example a variant of native human oxm or a native human oxm fragment, or native porcine oxm or a native porcine oxm fragment. Variants include oxm molecules or fragments with deletions, insertions, inversions, repeats and substitutions, (e.g., conservative substitutions and non-conservative substitutions; see, e.g., Table 2 below) which retain at least some of the activity of a corresponding non-mutated oxm molecule or fragment when in a molecule of the invention. More than one amino acid (e.g., 2, 3 or 4) can be substituted with another amino acid. Preferably, at least 70%, for example, at least 80%, especially at least 90%, of amino acids of oxm fragments specified with reference to any of formulae (I), (II) and (III), in particular of X, X',Y or Y', correspond to amino acids of the native oxm fragment. Preferably, the C-terminal four amino acids of native oxm are all present in a molecule of the invention. 1, 2, 3, 4, 5, 6, 7, 8, 9, or 10 amino acids from positions 3 to 37 may be substituted by alternative amino acid(s).

Typically conservative substitutions are the replacements, for one another, among the aliphatic amino acids Ala, Val, Leu and Ile; interchange of Ser and Thr containing hydroxy residues, interchange of the acidic residues Asp and Glu, interchange between the amide residues Asn and Gln, interchange of the basic residues Lys and Arg, interchange of the aromatic residues Phe and Tyr, and interchange of the small-sized amino acids Ala, Ser, Thr, Met and Gly. Guidance concerning how to make phenotypically silent amino acid substitutions, i.e. substitutions that do not alter the expressed phenotype, is provided in Bowie et al., Science 247:1306-1310, 1990.

**Table 2: Non-limiting examples of conservative amino acid substitutions**

| Original Residue | Conservative Substitutions |
|---|---|
| | |
| Ala | Ser |
| Arg | Lys |
| Asn | Gln, His |
| Asp | Glu |
| Cys | Ser |
| Gln | Asn |
| Glu | Asp |
| His | Asn; Gln |
| Ile | Leu, Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

Variants of oxm or oxm fragments further include variants in which one or more amino acids (for example 2, 3, 4, 5, 6, 7 or 8) of oxm of one species are substituted by an amino acid present at the equivalent position in oxm derived from a different species. The sequence of human and porcine oxm are included in Table 1 above. In particular, variants of human or porcine oxm include variants in which one or more amino acids (for example 2, 3 or 4) of human or porcine oxm are substituted by an amino acid present at the equivalent position in oxm derived from a different species.

Variants have been described in this section with reference to native human oxm. Variants are also possible in a portion of a molecule that is not primarily derived from the sequence of oxm. Such variants may exist in an analogous fashion to the variants described for oxm.

Amino acids referred to herein are preferred to be naturally-occurring amino acids, but sequences or fragments referred to herein may if desired include one or more non-natural amino acids.

For the avoidance of doubt, reference hereafter in this description to Formula (I) includes reference to Formula (II) or Formula (III) or Formula (IV) or Formula (V) or Formula (VI), and also to compounds which are in accordance with any of the other preferred embodiments mentioned above.

A variant compound preferably retains at least some of the activity of the corresponding non-variant compound.

More preferably, a variant compound exhibits enhanced appetite suppression activity relative to the corresponding non-variant compound.

### Derivatives

A compound of the invention may comprise the structure of Formula (I) modified by well known processes including amidation, glycosylation, carbamylation, alkylation, acylation, for example acetylation, sulfation, phosphorylation, cyclization, lipidization, protein (for example albumin) conjugation and pegylation. The structure of Formula (I) may be modified at random positions within the molecule, or at predetermined positions within the molecule and may include one, two, three or more attached chemical moieties.

A compound of the invention may be a fusion protein, whereby the structure of Formula (I) is fused to another protein or polypeptide (the fusion partner) using recombinant methods known in the art. Alternatively, such a fusion protein may be synthetically synthesized by any known method. Such a fusion protein comprises the structure of Formula (I). Any suitable peptide or protein can be used as the fusion partner (e.g., serum albumin, carbonic anhydrase, glutathione-S-transferase, single chain antibodies, antibodies, antibody fragments or thioredoxin, etc.). For example, a compound of the invention could be fused to an immunoglobulin light chain variable domain that has binding specificity for serum albumin, as described in WO 05/118642. Preferred fusion partners will not have an adverse biological activity in vivo. Such fusion proteins may be made by linking the carboxy-terminus of the fusion partner to the amino-terminus of the structure of Formula (I) or vice versa. Optionally, a cleavable linker may be used to link the structure of Formula (I) to the fusion partner. A resulting cleavable fusion protein may be cleaved in vivo such that an active form of a compound of the invention is released. Examples of such cleavable linkers include, but are not limited to, the linkers D-D-D-D-Y, G-P-R, A-G-G and H-P-F-H-L, which can be cleaved by enterokinase, thrombin, ubiquitin cleaving enzyme and renin, respectively. See, e.g., U.S. Patent No. 6,410,707.

Alternatively a compound of the invention may be a fusion protein, whereby the structure of Formula (I) is attached to a fusion partner via disulphide bond(s) resulting in a covalent linkage between at least one Cys residue of the compound of the invention, and at least one Cys residue of the fusion partner.

When a protein is used as a fusion partner, it is preferably chosen so not to exhibit undesirable antigenicity. Undesirable antigenicity may be avoided by chosing a protein which is allogenic to the animal to which the compound is to be administered.

A compound of the invention may be a physiologically functional derivative of the structure of Formula (I). The term "physiologically functional derivative" is used herein to denote a chemical derivative of a compound of Formula (I) having the same physiological function as the corresponding unmodified compound of Formula (I). For example, a physiologically functional derivative may be convertible in the body to a compound of Formula (I). According to the present invention, examples of physiologically functional derivatives include esters, amides, and carbamates; preferably esters and amides.

Pharmaceutically acceptable esters and amides of the compounds of the invention may comprise a C₁₋₆ alkyl-, C₅₋₁₀ aryl-, C₅₋₁₀ ar-C₁₋₆ alkyl-, or amino acid-ester or -amide attached at an appropriate site, for example at an acid group.

Acyl side chains may be advantageous, for example, by their lipophilic nature causing the moiety to bind with albumin, thus causing a greatly reduced rate of clearance from a subject and so increasing half life and duration of effect. Whilst the acyl side chains may be lower acyl, for example C₁₋C₉acyl, especially C₁₋₆acyl, they are preferred to be C₄₋₄₀, in particular C₈₋₂₅acyl, especially C₁₆ or C₁₈ acyl. Palmitoyl is especially preferred as an acyl side chain as is lauroyl. Acyl side chains may be added at any position on the peptide back bone. An acyl substituent may be attached to an amino acid residue in such a way that a carboxyl group of the acyl substituent forms an amide bond with an amino group of the amino acid residue. Alternatively, an acyl substituent may be attached to an amino acid residue in such a way that an amino group of the acyl substituent forms an amide bond with a carboxyl group of the amino acid residue. In a further preferred embodiment, the present invention relates to an oxm derivative wherein an acyl substituent is attached to the parent peptide by means of a spacer. For example, the acyl substituent may be attached to the oxm moiety by means of a spacer in such a way that a carboxyl group of the spacer forms an amide bond with an amino group of the oxm moiety. It is especially preferred to add an acyl side chain (optionally via a spacer) at a position in the peptide back bone where a lysine residue is found. This is because lysine, having a four carbon atom side chain terminating at an epsilon-amino group, is particularly suitable for easily adding an acyl side chain. It may be necessary to introduce lysine residue into the sequence solely for the purpose of providing a convenient site at which to add an acyl side chain. Alternatively the acyl side chain may be added to the lysine residue in advance of the synthesis of the peptide, whereupon its incorporation at the relevant synthetic step will result directly in acylation. This methodology is advantageous if the peptide sequence contains more than one lysine residue as it avoids the necessity of using selective conditions that acylate only the particular lysine of interest. Preferably, the peptide derivatives have three, more preferably two, and most preferably one acyl side chain substituent. Examples of acyl (and other lipophilic substituents), approaches and specific synthetic methods of attaching such to peptides (with and without the use of spacers) are described in U.S. Patent No. 6,268,343; and U.S. Patent Number 6,458,924.

According to certain preferred embodiments, acyl side chains may be added at position 30 and/or position 33 and/or position 39 and/or position 40 of the peptide back bone.

Pharmaceutically acceptable amides and carbonates of the compounds of Formula (I) may comprise a C₁₋₆ alkyl-, C₅₋₁₀ aryl-, C₅₋₁₀ ar-C₁₋₆ alkyl-, or amino acid-ester or -amide, or - carbamate attached at an appropriate site, for example at an amino group.

Methods for lipidization of sulfhydryl-containing compounds with fatty acid derivatives are disclosed in U.S. Patent No. 5,936,092; U.S. Patent No. 6,093,692; and U.S. Patent No. 6,225,445. Fatty acid derivatives of a compound of the invention comprising a compound of the invention linked to fatty acid via a disulfide linkage may be used for delivery of a compound of the invention to neuronal cells and tissues. Lipidisation markedly increases the absorption of the compounds relative to the rate of absorption of the corresponding unlipidised compounds, as well as prolonging blood and tissue retention of the compounds. Moreover, the disulfide linkage in lipidised derivative is relatively labile in the cells and thus facilitates intracellular release of the molecule from the fatty acid moieties. Suitable lipid-containing moieties are hydrophobic substituents with 4 to 26 carbon atoms, preferably 5 to 19 carbon atoms. Suitable lipid groups include, but are not limited to, the following: palmityl (C₁₅H₃₁,), oleyl (C₁₅H₂₉), stearyl (C₁₇H₃₅), cholate; and deoxycholate.

It will be appreciated by the skilled artisan that particular amino acid residues may be introduced to the oxm sequence in order to facilitate one or more of the modifications described herein.

Cyclization methods include cyclization through the formation of a disulfide bridge and head-to-tail cyclization using a cyclization resin. Cyclized peptides may have enhanced stability, including increased resistance to enzymatic degradation, as a result of their conformational constraints. Cyclization may in particular be expedient where the uncyclized peptide includes an N-terminal cysteine group. Suitable cyclized peptides include monomeric and dimeric head-to-tail cyclized structures. Cyclized peptides may include one or more additional residues, especially an additional cysteine incorporated for the purpose of formation of a disulfide bond or a side chain incorporated for the purpose of resin-based cyclization.

A compound of the invention may be a pegylated structure of Formula (I). Pegylated compounds of the invention may provide additional advantages such as increased solubility, stability and circulating time of the polypeptide, or decreased immunogenicity (see U.S. Patent No. 4,179,337).

Chemical moieties for derivitization of a compound of the invention may also be selected from water soluble polymers such as polyethylene glycol, ethylene glycol/propylene glycol copolymers, carboxymethylcellulose, dextran, polyvinyl alcohol and the like. A polymer moiety for derivatisation of a compound of the invention may be of any molecular weight, and may be branched or unbranched. For ease in handling and manufacturing, the preferred molecular weight of a polyethylene glycol for derivatisation of a compound of the invention is from about 1 kDa to about 100 kDa, the term "about" indicating that in preparations of polyethylene glycol, some molecules will weigh more, some less, than the stated molecular weight. Polymers of other molecular weights may be used, depending on the desired therapeutic profile, for example the duration of sustained release desired, the effects, if any on biological activity, the ease in handling, the degree or lack of antigenicity and other known effects of the polyethylene glycol to a therapeutic protein or analog. For example, the polyethylene glycol may have an average molecular weight of about 200, 500, 1000, 1500, 2000, 2500, 3000, 3500, 4000, 4500, 5000, 5500, 6000, 6500, 7000, 7500, 8000, 8500, 9000, 9500, 10,000, 10,500, 11,000, 11,500, 12,000, 12,500, 13,000, 13,500, 14,000, 14,500, 15,000, 15,500, 16,000, 16,500, 17,000, 17,500, 18,000, 18,500, 19,000, 19,500, 20,000, 25,000, 30,000, 35,000, 40,000, 50,000, 55,000, 60,000, 65,000, 70,000, 75,000, 80,000, 85,000, 90,000, 95,000, or 100,000 kDa.

Salts and solvates of compounds of the invention that are suitable for use in a medicament are those wherein a counterion or associated solvent is pharmaceutically acceptable. However, salts and solvates having non-pharmaceutically acceptable counterions or associated solvents are within the scope of the present invention, for example, for use as intermediates in the preparation of the compounds of Formula (I) and their pharmaceutically acceptable salts or solvates.

Suitable salts according to the invention include those formed with organic or inorganic acids or bases. Pharmaceutically acceptable acid addition salts include those formed with hydrochloric, hydrobromic, sulphuric, nitric, citric, tartaric, acetic, phosphoric, lactic, pyruvic, acetic, trifluoroacetic, succinic, perchloric, fumaric, maleic, glycollic, lactic, salicylic, oxaloacetic, methanesulfonic, ethanesulfonic, p-toluenesulfonic, formic, benzoic, malonic, naphthalene-2-sulfonic, benzenesulfonic, and isethionic acids. Other acids such as oxalic, while not in themselves pharmaceutically acceptable, may be useful as intermediates in obtaining the compounds of the invention and their pharmaceutical acceptable salts. Pharmaceutically acceptable salts with bases include ammonium salts, alkali metal salts, for example potassium and sodium salts, alkaline earth metal salts, for example calcium and magnesium salts, and salts with organic bases, for example dicyclohexylamine and N-methyl-D-glucomine.

Those skilled in the art of organic chemistry will appreciate that many organic compounds can form complexes with solvents in which they are reacted or from which they are precipitated or crystallized. Such complexes are known as "solvates". For example, a complex with water is known as a "hydrate". The present invention provides solvates of compounds of the invention.

Peptides of the invention may be made by any suitable technique for making peptides, including but not limited to conventional methodology, for example, synthesis from individual amino acids, especially step-wise synthesis using an automatic peptide synthesizer; modification of native peptides; or recombinant manufacturing techniques.

### Conditions

The invention also provides a pharmaceutical composition for use in the control of appetite, feeding, food intake, energy expenditure and calorie intake, the composition comprising an effective amount of a compound according to the invention. More particularly, the invention provides a pharmaceutical composition for use in the treatment of obesity, for use in the treatment of eating disorders, or for use in the treatment of diabetes or a symptom of diabetes, or for use in the treatment or prevention of comorbidities associated with obesity or excess weight.

Moreover, the invention provides a method of reducing excess weight, for example cosmetic excess weight, comprising administering to a patient desiring to reduce weight an effective amount of a compound according to the invention.

Furthermore, the invention provides a method of treating a condition selected from obesity, eating disorders and diabetes, comprising administering to a subject in need of treatment for a said condition an effective amount of a compound according to the invention.

The invention also provides use of a compound according to the invention in the manufacture of a medicament for use in the treatment of a condition selected from obesity, eating disorders, diabetes, heart disease, hypertension, lipid disease, and disorders of intestinal and gastric motor activity and other aspects of gut and intestinal function, for example, water absorption and fluid handling, or pancreatic function including the endocrine pancreas, or disorders of hepato-biliary function, or prevention of cancer. Further, the invention provides use of a compound according to the invention in the manufacture of a medicament for use in the control of any one or more of appetite, feeding, food intake, energy expenditure and calorie intake.

The subject to whom the compound is administered may be overweight, for example, obese. Alternatively, or in addition, the subject may be diabetic, for example having insulin resistance or glucose intolerance, or both. The subject may have diabetes mellitus, for example, the subject may have Type II diabetes. The subject may be overweight, for example, obese and have diabetes mellitus, for example, Type II diabetes.

In addition, or alternatively, the subject may have, or may be at risk of having, a disorder in which obesity or being overweight is a risk factor. Such disorders include, but are not limited to, cardiovascular disease, for example hypertension, atherosclerosis, congestive heart failure, and dyslipidemia; stroke; gallbladder disease; osteoarthritis; sleep apnea; reproductive disorders for example, polycystic ovarian syndrome; cancers, for example breast, prostate, colon, endometrial, kidney, and esophagus cancer; varicose veins; acanthosis nigricans; eczema; exercise intolerance; insulin resistance; hypertension; hypercholesterolemia; cholithiasis; osteoarthritis; orthopedic injury; insulin resistance, for example, type 2 diabetes and syndrome X; metabolic syndrome; and thromboembolic disease (see Kopelman (2000), Nature 404:635-43; Rissanen et al., British Med. J. 301, 835, 1990).

Other disorders associated with obesity include depression, anxiety, panic attacks, migraine headaches, PMS, chronic pain states, fibromyalgia, insomnia, impulsivity, obsessive-compulsive disorder, irritable bowel syndrome (IBS), and myoclonus. Furthermore, obesity is a recognized risk factor for increased incidence of complications of general anesthesia. (See e.g., Kopelman, Nature 404:635-43, 2000). In general, obesity reduces life span and carries a serious risk of comorbidities such as those listed above.

Other diseases or disorders associated with obesity are birth defects, maternal obesity being associated with increased incidence of neural tube defects, carpal tunnel syndrome (CTS); chronic venous insufficiency (CVI); daytime sleepiness; deep vein thrombosis (DVT); end stage renal disease (ESRD); gout; heat disorders; impaired immune response; impaired respiratory function; infertility; liver disease; lower back pain; obstetric and gynecologic complications; pancreatititis; as well as abdominal hernias; acanthosis nigricans; endocrine abnormalities; chronic hypoxia and hypercapnia; dermatological effects; elephantitis; gastroesophageal reflux; heel spurs; lower extremity edema; mammegaly which causes considerable problems such as bra strap pain, skin damage, cervical pain, chronic odors and infections in the skin folds under the breasts, etc.; large anterior abdominal wall masses, for example abdominal panniculitis with frequent panniculitis, impeding walking, causing frequent infections, odors, clothing difficulties, lower back pain; musculoskeletal disease; pseudo tumor cerebri (or benign intracranial hypertension), and sliding hiatil hernia.

The present invention further provides a method for increasing energy expenditure in a subject. The method includes, for example, peripherally administering a therapeutically effective amount of a compound of the invention to the subject, thereby altering energy expenditure. Energy is burned in all physiological processes. The body can alter the rate of energy expenditure directly, by modulating the efficiency of those processes, or changing the number and nature of processes that are occurring. For example, during digestion the body expends energy moving food through the bowel, and digesting food, and within cells, the efficiency of cellular metabolism can be altered to produce more or less heat.

In one aspect the method of the invention involves manipulation of the arcuate circuitry that alter food intake coordinately, and reciprocally alter energy expenditure. Energy expenditure is a result of cellular metabolism, protein synthesis, metabolic rate, and calorie utilization. Thus, in this aspect of the invention, administration of a compound of Formula (I) may result in increased energy expenditure, and decreased efficiency of calorie utilization.

The invention also provides a method for improving a lipid profile in a subject. The invention also provides a method for alleviating a condition or disorder that can be alleviated by reducing nutrient availability.

Appetite can be measured by any means known to one of skill in the art. For example, decreased appetite can be assessed by a psychological assessment. For example, administration of a compound of the invention results in a change in perceived hunger, satiety, and/or fullness. Hunger can be assessed by any means known to one of skill in the art. For example, hunger is assessed using psychological assays, such as by an assessment of hunger feelings and sensory perception using a questionnaire, such as, but not limited to, a Visual Analog Score (VAS) questionnaire. In one specific, non-limiting example, hunger is assessed by answering questions relating to desire for food, drink, prospective food consumption, nausea, and perceptions relating to smell or taste.

A compound of the invention may be used for weight control and treatment, for example reduction or prevention of obesity, in particular any one or more of the following: preventing and reducing weight gain; inducing and promoting weight loss; and reducing obesity as measured by the Body Mass Index. A compound of the invention may be used in the control of any one or more of appetite, satiety and hunger, in particular any one or more of the following: reducing, suppressing and inhibiting appetite; inducing, increasing, enhancing and promoting satiety and sensations of satiety; and reducing, inhibiting and suppressing hunger and sensations of hunger. A compound of the invention may be used in maintaining any one or more of a desired body weight, a desired Body Mass Index, a desired appearance and good health.

A subject may be a subject who desires weight loss, for example female and male subjects who desire a change in their appearance. A subject may desire decreased feelings of hunger, for example the subject may be a person involved in a lengthy task that requires a high level of concentration, for example soldiers on active duty, air traffic controllers, or truck drivers on long distance routes, etc.

The present invention may also be used in treating, prevention, ameliorating or alleviating conditions or disorders caused by, complicated by, or aggravated by a relatively high nutrient availability. The term "condition or disorder which can be alleviated by reducing caloric (or nutrient) availability" is used herein to denote any condition or disorder in a subject that is either caused by, complicated by, or aggravated by a relatively high nutrient availability, or that can be alleviated by reducing nutrient availability, for example by decreasing food intake. Subjects who are insulin resistant, glucose intolerant, or have any form of diabetes mellitus, for example, type 1, 2 or gestational diabetes, can also benefit from methods in accordance with the present invention.

Conditions or disorders associated with increased caloric intake include, but are not limited to, insulin resistance, glucose intolerance, obesity, diabetes, including type 2 diabetes, eating disorders, insulin-resistance syndromes, and Alzheimer's disease.

According to the present invention, a compound of Formula (I) is preferably used in the treatment of a human. However, while the compounds of the invention will typically be used to treat human subjects they may also be used to treat similar or identical conditions in other vertebrates for example other primates; farm animals for example swine, cattle and poultry; sport animals for example horses; companion animals for example dogs and cats.

### Compositions

While it is possible for the active ingredient to be administered alone, it is preferable for it to be present in a pharmaceutical formulation or composition. Accordingly, the invention provides a pharmaceutical formulation comprising a compound of Formula (I), or a variant or derivative thereof, or a salt or solvate thereof, as defined above, and a pharmaceutically acceptable excipient. Pharmaceutical compositions of the invention may take the form of a pharmaceutical formulation as described below.

The pharmaceutical formulations according to the invention include those suitable for oral, parenteral (including subcutaneous, intradermal, intramuscular, intravenous, and intraarticular), inhalation (including fine particle dusts or mists which may be generated by means of various types of metered doses, pressurized aerosols, nebulizers or insufflators), rectal and topical (including dermal, transdermal, transmucosal, buccal, sublingual, and intraocular) administration, although the most suitable route may depend upon, for example, the condition and disorder of the recipient.

The formulations may conveniently be presented in unit dosage form and may be prepared by any of the methods well known in the art of pharmacy. All methods include the step of bringing the active ingredient into association with a pharmaceutical carrier which constitutes one or more accessory ingredients. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets, each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste. Various pharmaceutically acceptable carriers and their formulation are described in standard formulation treatises, e.g., Remington's Pharmaceutical Sciences by E. W. Martin. See also Wang, Y. J. and Hanson, M. A., Journal of Parenteral Science and Technology, Technical Report No. 10, Supp. 42:2S, 1988.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein. The present compounds can, for example, be administered in a form suitable for immediate release or extended release. Immediate release or extended release can be achieved by the use of suitable pharmaceutical compositions comprising the present compounds, or, particularly in the case of extended release, by the use of devices such as subcutaneous implants or osmotic pumps. The compounds can be formulated, for administration orally, with delivery agents or carriers that facilitate the transport of therapeutic macromolecules and highly charged compounds across cell membranes, especially in the small intestine. Such delivery agents or carriers may in addition inhibit enzymatic degradation of peptides during passage through the gastrointestinal (GI) tract and/or the formulation may include additional agents that protect against such degradation. The present compounds can also be administered liposomally.

Exemplary compositions for oral administration include suspensions which can contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which can contain, for example, microcrystalline cellulose, dicalcium phosphate, starch, magnesium stearate and/or lactose and/or other excipients, binders, extenders, disintegrants, diluents and lubricants such as those known in the art. The compounds of Formula (I) can also be delivered through the oral cavity by sublingual and/or buccal administration. Moulded tablets, compressed tablets or freeze-dried tablets are exemplary forms which may be used. Exemplary compositions include those formulating the present compound(s) with fast dissolving diluents such as mannitol, lactose, sucrose and/or cyclodextrins. Also included in such formulations may be high molecular weight excipients such as celluloses (avicel) or polyethylene glycols (PEG). Such formulations can also include an excipient to aid mucosal adhesion such as hydroxy propyl cellulose (HPC), hydroxy propyl methyl cellulose (HPMC), sodium carboxy methyl cellulose (SCMC), maleic anhydride copolymer (e.g., Gantrez), and agents to control release such as polyacrylic copolymer (e.g. Carbopol 934). Lubricants, glidants, flavors, coloring agents and stabilizers may also be added for ease of fabrication and use.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example saline or water-for-injection, immediately prior to use. Extemporaneous injection solutions and suspensions may be prepared from sterile powders, granules and tablets of the kind previously described. Exemplary compositions for parenteral administration include injectable solutions or suspensions which can contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid, or Cremaphor. An aqueous carrier may be, for example, an isotonic buffer solution at a pH of from about 3.0 to about 8.0, preferably at a pH of from about 3.5 to about 7.4, for example from 3.5 to 6.0, for example from 3.5 to about 5.0. Useful buffers include sodium citrate-citric acid and sodium phosphate-phosphoric acid, and sodium acetate/acetic acid buffers. The composition preferably does not include oxidizing agents and other compounds that are known to be deleterious to oxm and oxm agonists.

Excipients that can be included are, for instance, other proteins, such as human serum albumin or plasma preparations. If desired, the pharmaceutical composition may also contain minor amounts of non-toxic auxiliary substances, such as wetting or emulsifying agents, preservatives, and pH buffering agents and the like, for example sodium acetate or sorbitan monolaurate.

Exemplary compositions for nasal aerosol or inhalation administration include solutions in saline, which can contain, for example, benzyl alcohol or other suitable preservatives, absorption promoters to enhance bioavailability, and/or other solubilizing or dispersing agents such as those known in the art. Conveniently in compositions for nasal aerosol or inhalation administration the compound of the invention is delivered in the form of an aerosol spray presentation from a pressurized pack or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of e.g., gelatin for use in an inhaler or insufflator can be formulated to contain a powder mix of the compound and a suitable powder base, for example lactose or starch. In one specific, non-limiting example, a compound of the invention is administered as an aerosol from a metered dose valve, through an aerosol adapter also known as an actuator. Optionally, a stabilizer is also included, and/or porous particles for deep lung delivery are included (e.g., see U.S. Patent No. 6,447,743). Intranasal formulations may include delivery agents for reversibly opening the nasal tight junction, thereby increasing drug permeability (e.g., see US Patent Application 10/322,266).

Formulations for rectal administration may be presented as a retention enema or a suppository with the usual carriers such as cocoa butter, synthetic glyceride esters or polyethylene glycol. Such carriers are typically solid at ordinary temperatures, but liquefy and/or dissolve in the rectal cavity to release the drug.

Formulations for topical administration in the mouth, for example buccally or sublingually, include lozenges comprising the active ingredient in a flavoured basis such as sucrose and acacia or tragacanth, and pastilles comprising the active ingredient in a basis such as gelatin and glycerine or sucrose and acacia. Exemplary compositions for topical administration include a topical carrier such as Plastibase (mineral oil gelled with polyethylene).

Preferred unit dosage formulations are those containing an effective dose, as hereinbefore recited, or an appropriate fraction thereof, of the active ingredient.

It should be understood that in addition to the ingredients particularly mentioned above, the formulations of this invention may include other agents conventional in the art having regard to the type of formulation in question, for example those suitable for oral administration may include flavouring agents.

The compounds of the invention are also suitably administered as sustained-release systems. Suitable examples of sustained-release systems of the invention include suitable polymeric materials, for example semi-permeable polymer matrices in the form of shaped articles, e.g., films, or microcapsules; suitable hydrophobic materials, for example as an emulsion in an acceptable oil; or ion exchange resins; and sparingly soluble derivatives of the compound of the invention, for example, a sparingly soluble salt. Sustained-release systems may be administered orally; rectally; parenterally; intracistemally; intravaginally; intraperitoneally; topically, for example as a powder, ointment, gel, drop or transdermal patch; bucally; or as an oral or nasal spray.

Preparations for administration can be suitably formulated to give controlled release of compounds of the invention. For example, the pharmaceutical compositions may be in the form of particles comprising one or more of biodegradable polymers, polysaccharide jellifying and/or bioadhesive polymers, amphiphilic polymers, agents capable of modifying the interface properties of the particles of the compound of Formula (I). These compositions exhibit certain biocompatibility features which allow a controlled release of the active substance. See U.S. Patent No. 5,700,486.

A compound of the invention may be delivered by way of a pump (see Langer, Science 249:1527-1533, 1990; Sefton, CRC Crit. Ref. Biomed. Eng. 14:201, 1987; Buchwald et al., Surgery 88:507, 1980; Saudek et al., N. Engl. J. Med. 321:574, 1989) or by a continuous subcutaneous infusions, for example, using a mini-pump. An intravenous bag solution may also be employed. The key factor in selecting an appropriate dose is the result obtained, as measured by decreases in total body weight or ratio of fat to lean mass, or by other criteria for measuring control or prevention of obesity or prevention of obesity-related conditions, as are deemed appropriate by the practitioner. Other controlled release systems are discussed in the review by Langer, supra. In another aspect of the disclosure, compounds of the invention are delivered by way of an implanted pump, described, for example, in U.S. Patent No. 6,436,091; U.S. Patent No. 5,939,380; U.S. Patent No. 5,993,414.

Implantable drug infusion devices are used to provide patients with a constant and long term dosage or infusion of a drug or any other therapeutic agent. Essentially such device may be categorized as either active or passive. A compound of the present invention may be formulated as a depot preparation. Such a long acting depot formulation can be administered by implantation, for example subcutaneously or intramuscularly; or by intramuscular injection. Thus, for example, the compounds can be formulated with suitable polymeric or hydrophobic materials, for example as an emulsion in an acceptable oil; or ion exchange resins; or as a sparingly soluble derivatives, for example, as a sparingly soluble salt.

A therapeutically effective amount of a compound of the invention may be administered as a single pulse dose, as a bolus dose, or as pulse doses administered over time. Thus, in pulse doses, a bolus administration of a compound of the invention is provided, followed by a time period wherein a compound of the invention is administered to the subject, followed by a second bolus administration. In specific, non-limiting examples, pulse doses of a compound of the invention are administered during the course of a day, during the course of a week, or during the course of a month.

In one embodiment, a therapeutically effective amount of a compound of the invention is administered with a therapeutically effective amount of another agent, for example an additional appetite suppressant, a food-intake-reducing, plasma glucose-lowering or plasma lipid-altering agent. Specific, non-limiting examples of an additional appetite suppressant include amfepramone (diethylpropion), phentermine, mazindol and phenylpropanolamine, fenfluramine, dexfenfluramine, sibutramine, rimonabant, and fluoxetine. The compound of the invention can be administered simultaneously with the additional appetite suppressant, or it may be administered sequentially. Thus, in one embodiment, the compound of the invention is formulated and administered with an appetite suppressant as a single dose.

In another embodiment, a therapeutically effective amount of a compound of the invention is administered in combination with a therapeutically effective amount of another agent, for the treatment of diseases other than obesity, for example diabetes, in which specific non limiting examples of an additional therapeutic agent are GLP-1 or an analogue thereof, exenatide, and pramlintide.

A compound of the invention may be administered whenever the effect, e.g., appetite suppression, decreased food intake, or decreased caloric intake, is desired, or slightly before to whenever the effect is desired, such as, but not limited to about 10 minutes, about 15 minutes, about 30 minutes, about 60 minutes, about 90 minutes, about 120 minutes, about 4 hours, about 8 hours, or about 12 hours, before the time the effect is desired.

A compound of the invention may be administered in combination with whenever the effect, e.g., appetite suppression, decreased food intake, or decreased caloric intake, is desired, or slightly before to whenever the effect is desired, such as, but not limited to about 10 minutes, about 15 minutes, about 30 minutes, about 60 minutes, about 90 minutes, about 120 minutes, about 4 hours, about 8 hours, or about 12 hours, before the time the effect is desired.

### Dosages

The therapeutically effective amount of a compound of the invention will be dependent on the molecule utilized, the subject being treated, the severity and type of the affliction, and the manner and route of administration. For example, a therapeutically effective amount of a compound of the invention may vary from about 0.01 µg per kilogram (kg) body weight to about 1 g per kg body weight, for example about 1 µg to about 5 mg per kg body weight, or about 5 µg to about 1 mg per kg body weight. A compound of the invention may be administered to a subject at 0.5 to 200 picomole (pmol) per kg body weight, or about 20 pmol per kg body weight. In one specific, non-limiting example, a compound of the invention is administered in a dose of about 1 nmol or more, 2 nmol or more, or 5 nmol or more. In this example, the dose of the compound of the invention is generally not more than 100 nmol, for example, the dose is 90 nmols or less, 80 nmols or less, 70 nmols or less, 60 nmols or less, 50 nmols or less, 40 nmols or less, 30 nmols or less, 20 nmols or less, 10 nmols. For example, a dosage range may comprise any combination of any of the specified lower dose limits with any of the specified upper dose limits. Thus, examples of non-limiting dose ranges of compounds of the invention are within the range of from 1 to 100 nmols, from 1 to 90 nmols, from 1 to 80 nmols.

In one specific, non-limiting example, from about 0.5 to about 50 nmol of a compound of the invention is administered, for example about 1 to about 20 nmol, for example, about 2 nmol is administered as a subcutaneous injection. The exact dose is readily determined by one of skill in the art based on the potency of the specific compound utilized, the age, weight, sex and physiological condition of the subject.

In another specific non-limiting example, a compound of the invention is administered to a subject in a dose of about 0.005 mg to about 2 mg per dose, about once, about twice, about three times, or about four times per day.

Suitable doses of compounds of the invention also include those that result in a reduction in calorie intake, food intake, or appetite, or increase in energy expenditure that is equivalent to the reduction in calorie intake, food intake, or appetite, or to increase the energy expenditure, caused by levels of oxm that have been observed in man. Examples of doses include, but are not limited to doses that produce the effect demonstrated when the serum levels of oxm are from about 800 pM to about 1300 pM, or from about 900 pM to about 1000 pM, or about 950 pM.

Suitable doses of compounds of the invention also include those that are equivalent to levels of oxm seen in subjects experiencing conditions associated with reduced appetite, for example jejunoileal bypass (Sarson et al,. Int J Obes, 1981, 5:471-480; Holst et al., Scand J Gastroenterol, 1979, 14:205-207).

In a study of the effects of oxyntomodulin on appetite suppression and food intake reduction in humans (Cohen et al., J. Clin. Endocrinol. Metab., 2003, 88(10), 4696-4701) it was found that an infusion of oxyntomodulin to human volunteers at 3.0 pmol/kg.min for 90 minutes led to a significantly reduced *ad libitum* energy intake (19.3 +/- 5.6%; P<0.01). The total oxyntomodulin infused was 270 pmol/kg body weight. The observed oxyntomodulin-like immunoreactivity in the blood of the subjects rose to around 800 pmol/L during the infusion.

In a study of the effects of oxyntomodulin on weight loss in humans (Wynne et al., Diabetes., 2005, 54(Aug), 2390-2395) it was found that subcutaneous injections of oxyntomodulin to humans volunteers resulted in a significant reduction of body weight. Over the study period of 28 days, body weight of the treatment group was reduced by 2.3 ± 0.4 kg in the treatment group compared with 0.5 ± 0.5 kg in the control group (P<0.0106). 1.8 mg (approximately 400 nmol) of oxyntomodulin was administered three times daily 30 mins before meals. On average, the treatment group had an additional 0.45kg weight loss per week. Energy intake by the treatment group was significantly reduced by 170 ± 37 kcal (25 ± 5%) at the initial study meal within the 28 day study period (P ± 0.0007) and by 250 ± 63 kcal (35 ± 9%) at the final study meal during the 28 day study period (P ± 0.0023), with no change in subjective food palatability. Oxyntomodulin treatment resulted in weight loss and a change in the levels of adipose hormones consistent with a loss of adipose tissue. The anorectic effect was maintained over the 4-week period.

The compounds of the invention have been found to be more active and/or longer-lasting than native oxyntomodulin as used in human studies to date (Cohen *et al.* (2003) and Wynne et al. (2005) Diabetes 54(Aug), 2390-2395). The dosage required for a compound of the invention may be somewhat lower than that required for native oxyntomodulin. As mentioned in the examples section, the potency of the peptides referred to herein as SEQ ID NO: 25, SEQ ID NO: 15, SEQ ID NO: 16, SEQ ID NO: 19 and SEQ ID NO: 29 are, respectively, 2.5 times, 200 times, 400 times and 1-4000 times that of oxyntomodulin and accordingly the dosages of SEQ ID NO: 25, SEQ ID NO: 15, SEQ ID NO: 16, SEQ ID NO: 19 and SEQ ID NO: 29 required to observe an effect in humans can be expected to be a similar order of magnitude lower, for example 2.5 times, 200 times, 400 times, and 1-4000 times lower than the dose of native oxyntomodulin. The magnitude of the potency of the peptides of the invention in comparison to the native oxyntomodulin peptide may also allow the frequency of administration a compound of the invention to be lower than that required for native oxyntomodulin.

The disclosure is illustrated by the following non-limiting Examples.

### EXAMPLES

### Materials and Methods

*Animals:* All animal procedures were approved by the British Home Office Animals (Scientific Procedures) Act 1986 (Project License number 70/5516). Male C57BL/6 mice and male Wistar rats were maintained in individual cages under controlled temperature (21-23 °C) with *ad libitum* access to standard chow (RM1 diet, SDS Ltd, Witham, Essex, UK) and to water. The light cycle was 14 hours light and 10 hours dark with 'dawn' at 07:00, lights full on at 07:30, 'dusk' at 21:00 with lights off at 21:30. These times were fixed in the animal facility.

All animals were handled almost daily for on average nine days prior to the first study. During the acclimatization period, each animal received two saline injections at least two days apart in order to further acclimatize to the procedure on the study days.

*Intra-peritoneal (IP) injections:* IP injections were administered to mice via a 0.5 ml syringe with a 29-gauge needle (maximum injection volume 0.1 ml). Maximum volume administered IP was 0.1 ml.

*Subcutaneous (SC) injections:* SC injections were administered to rats via a 0.5 ml syringe with a 29-gauge needle (maximum injection volume 0.1 ml). Maximum volume administered SC was 0.1 ml.

*Study protocol:* C57BL/6 mice (20-35 g) were injected with the peptide under investigation following a 20-hour fast. Wistar rats were injected following a 24-hour fast. A pre-weighed quantity of chow was presented immediately after injection. The remaining food was measured at regular time intervals (e.g. 1, 2, 3,4, 6, 8, 24 and 48 hours following injection) and food intake calculated.

*Metabolic cage study protocol:* Animals were mainitained at 24°C with a 12:12 hour light-dark cycle (light period 0700-1900). Injections of saline or peptide were carried out in the morning after an overnight fast or in the evening immediately before the onset of the dark phase. Injections were given with a maximum volume of 0.1 ml intraperitoneally (IP) for a mouse and 0.5 ml IP or 0.1 ml subcutaneously (SC) for a rat. All mice or rats were acclimatised to their metabolic cages for one to two days before injections began. Rats/mice were individually housed in special plexiglass cages (Columbus Instruments) with water available *ab libitum.* Rats/mice had *ab libitum* access to ground food (RM1 diet, SDS Ltd., Witham, UK) placed in side-feeders attached to each cage. The side-feeders rested on balances directly linked to a computer measuring individual cage Food Intake (FI). This was set to measure food weight either once every minute or once ever 30 min. Cumulative FI was automatically calculated. The ambulatory activity of each indivdidually housed animal was assessed using the optical beam technique by an Opto M3 technique (Columbus Instruments). Cumulative activity counts along x and z axes were recorded simultaneously ever 30 min and were used to determine horizontal (XAMB) and rearing (ZTOT) movement respectively.

### Peptides Synthesis

### Synthesis of peptides

Peptides were produced by solid phase step-wise synthesis (SPSS) using the Fmoc N-terminal protection strategy. Chain assembly was performed on Applied Biosystems 431, 433 or Pioneer automated synthesisers. Solid phase resins were used with factory pre-loaded C terminal amino acid or with an amide linker as appropriate. The following side-chain protecting groups were used: Asn(Trt), Gln(Trt), Cys(Trt), His(Trt), Asp(tBu), Glu(tBu), Ser(tBu), Thr(tBu), Tyr(tBu), Arg(Pbf), Lys(Boc), Trp(Boc). In some cases, where synthesis difficulty was anticipated, preformed oxazolidine dipeptides were used in place of respective monomers. All chemicals (from various suppliers including Applied Biosystems, Merck Biosciences and AGCT) were synthesis grade. Feedback monitoring to optimise syntheses was employed on all instruments.

### Recovery of peptides

After synthesis, peptides were cleaved from the solid phase resin support and fully side chain deprotected. This was achieved by treatment for 2hours with trifluoroacetic acid (TFA) containing 4% water and 2.5% tri-isopropylsilane to scavenge side-chain protecting groups. Peptide-TFA solutions were filtered from the resins and the peptides precipitated with methyl tertiary butyl ether (MTBE). Peptides were isolated by centrifugation, washed in MTBE and dried under vacuum.

### Analysis and purification of peptides

Peptides were dissolved in de-ionized water, with addition of acetic acid where necessary. Peptide solutions were clarified by centrifugation or filtration (Whatman GD/X syringe filter) prior to analysis and purification.

All peptide products were analysed by reverse phase HPLC on an Applied Biosystems BioCad instrument using an analytical Brownlee Aquapore RP300 C8 or Phenomenex Synergi Hydro C18 column. Purification was performed by reverse phase HPLC using preparative columns of the above types. Acetonitrile-water gradients (with TFA as counter-ion) were used for elution of products. Capillary Zone Electrophoresis (CZE) was performed on crude and purified peptides using a Hewlett Packard 3DCE instrument. Molecular weight determination was performed on a Micromass MALDI -R mass spectrometer.

Purified peptides were freeze-dried in pharmaceutical grade glass vials (Adelphi Vials) and closed under vacuum.

In the following Examples, "xx" or "ex" refers to the insertion of amino acids at the indicated positions to replace the correspondingly numbered residues.

### Derivatised side chains

Peptides having side chains derivatised with an acyl or alkyl group were prepared by standard methods.

### Albumin conjugation.

Analogues to be conjugated to albumin were synthesised as described above, whereupon typically 0.5 µ moles of peptide was dissolved in 50 µl DMSO and 450 µl Phosphate Buffered Saline (PBS, pH 7.4), and 0.25 µ moles of mouse albumin (ie half the number of moles of peptide used) was dissolved in in 500 µl PBS. The albumin and peptide solutions were combined, and 1 ml 2% (v/v) gluteraldehyde solution added in a drop wise manner with stirring for two hours at 4°C. The reaction was stopped by the addition of 200 µl of 10 mg/ml NaBh4, with stirring for 1 h at 4°C. The resulting solution was dialysed against 3 changes of excess PBS (0.02% NaN3) at 4°C for 24 hours, and the peptide-albumin conjugate freeze dried and stored at -20°C.

### Solubility of derivatised peptides

Derivatised polypeptides should be fully dissolved before administration. In order to achieve solubility it may be necessary to dissolve the polypeptides in a small amount of dilute alkali (for example, 50 µl 0.01 NaOH) and then dilute the dissolved peptide in saline.

### Example 1: oxm analogues in which from 4 to 10 amino acids in a generally central region have been replaced by substitute sequences.

The feeding effects of three oxm analogues incorporating 4-, 7- or 10-residue substitutions were investigated. The three compounds correspond to the full length human oxyntomodulin amino acid sequence (SEQ ID NO: 7) with the exception that variable lengths (4-10 amino acids) have been replaced as follows:
oxm(xx15-18): oxm (SEQ ID NO: 7) with residues 15 to 18 replaced by the sequence Glu Glu Glu Ala (SEQ ID NO: 20)
oxm(xx15-21): oxm (SEQ ID NO: 7) with residues 15 to 21 replaced by the sequence Glu Glu Glu Ala Val Arg Leu (SEQ ID NO: 21)
oxm(xx15-24): oxm (SEQ ID NO: 7) with residues 15 to 24 replaced by the sequence Glu Glu Glu Ala Val Arg Leu Phe Ile Glu (SEQ ID NO: 4)

The above-defined sequences fall within the mid-section of the oxm molecule and do not encroach on the C-terminal octapeptide. The complete sequences are as follows:

oxm(xx15-18) SEQ ID NO: 14
His Ser Gln Gly Thr Phe Thr Ser Asp Tyr Ser Lys Tyr Leu Glu Glu Glu Ala Ala Gln Asp Phe Val
Gln Trp Leu Met Asn Thr Lys Arg Asn Arg Asn Asn Ile Ala

oxm(xx15-21) SEQ ID NO: 15
His Ser Gln Gly Thr Phe Thr Ser Asp Tyr Ser Lys Tyr Leu Glu Glu Glu Ala Val Arg Leu Phe Val
Gln Trp Leu Met Asn Thr Lys Arg Asn Arg Asn Asn Ile Ala

oxm(xx15-24) SEQ ID NO: 16
His Ser Gln Gly Thr Phe Thr Ser Asp Tyr Ser Lys Tyr Leu Glu Glu Glu Ala Val Arg Leu Phe Ile
Glu Trp Leu Met Asn Thr Lys Arg Asn Arg Asn Asn Ile Ala

The above peptides were administered by injection to groups of 9 to 10 mice at a dose of 2700 nmol/kg (known as an effective dose for human oxm) as a 'screen' for appetite inhibition effects. Further groups were administered either oxm (for comparison purposes) or saline (control).

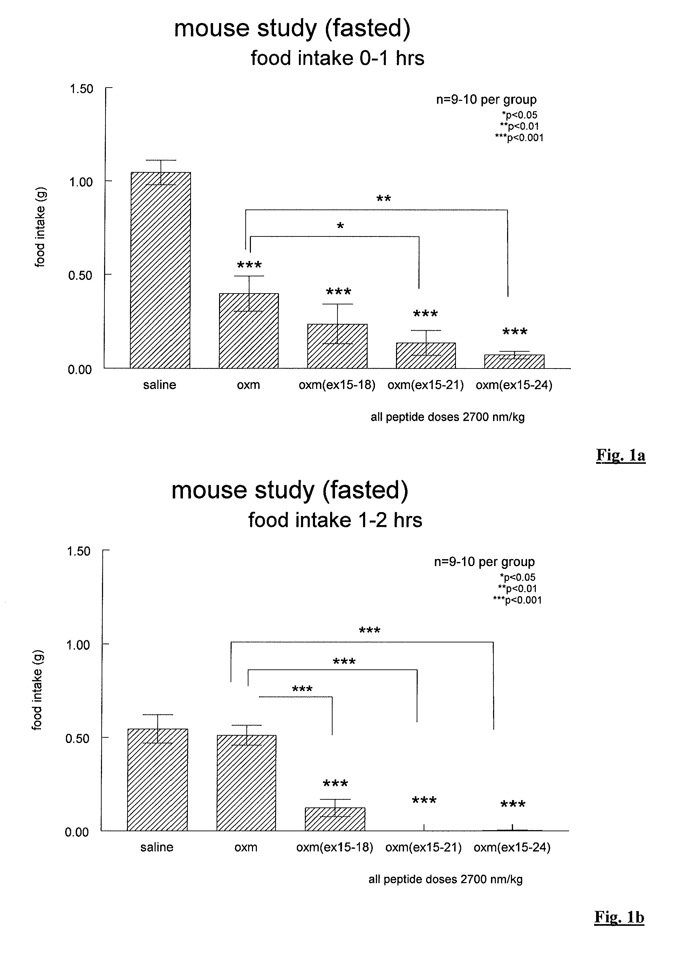

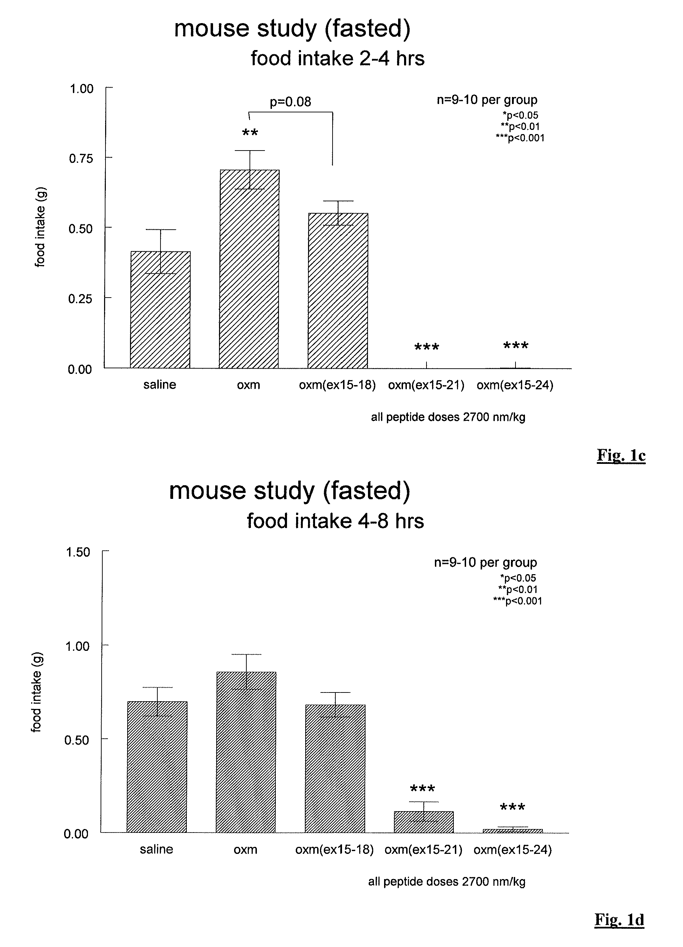

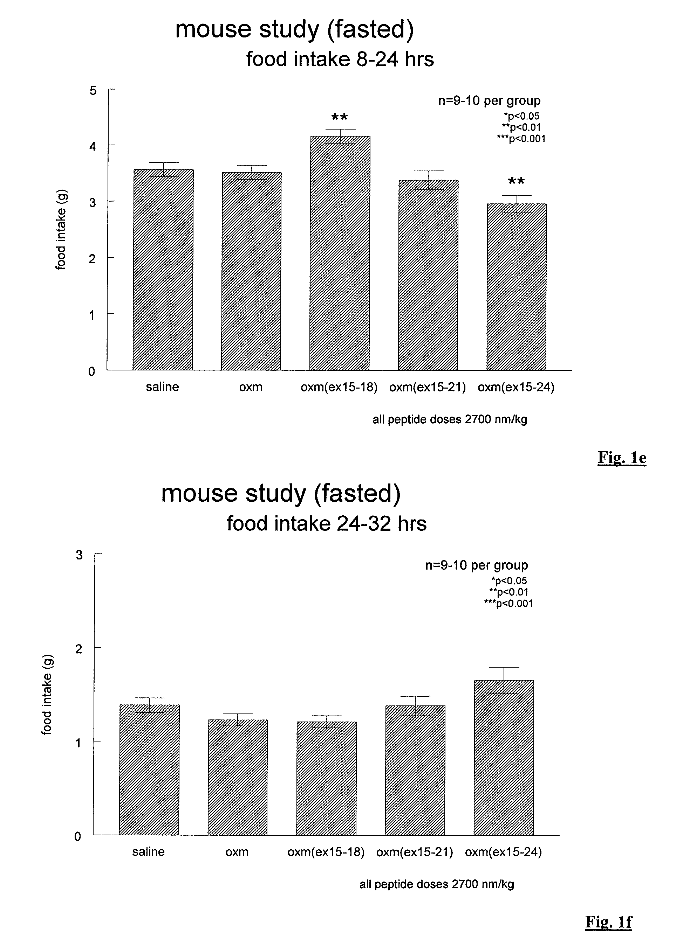

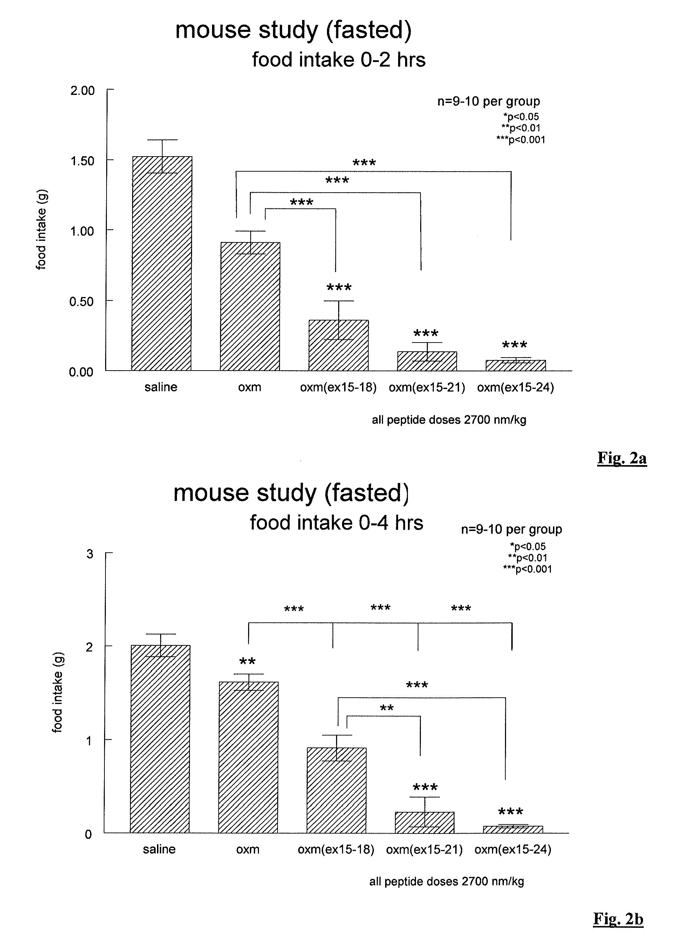

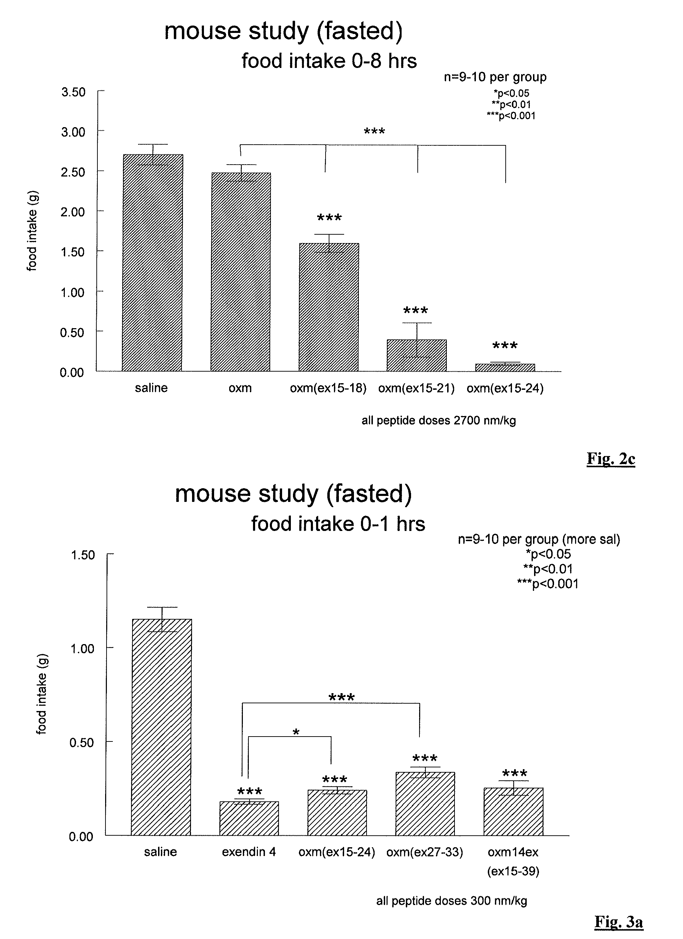

The measured food intake for each group is shown in Figs. 1a to 1f for, respectively, the intervals from 0 to 1 hour, from 1 to 2 hours, from 2 to 4 hours, 4 to 8, 8 to 24, and 24 to 32 hours after injection. In Figs. 2a to 2c are shown the cumulative food intake for each group for 2, 4 and 8 hours after injection. Results suggest that even replacement of 4 amino acids oxm(xx15-18) results in increased and prolonged inhibition of food intake compared to both saline and oxm (see especially Figs. 1d and 2c relating to food intake up to 8 hours). While not statistically significant, the trend is for oxm(xx15-21) and oxm(xx15-24) to give a greater and more prolonged appetite suppressant effect.

### Example 2: Lower dosage studies

Three peptides according to the invention were administered by injection to groups of 9 to 10 mice at a dose of 300 nmol/kg. Further groups were administered either exendin 4 at the same dosage (for comparison purposes) or saline (control).

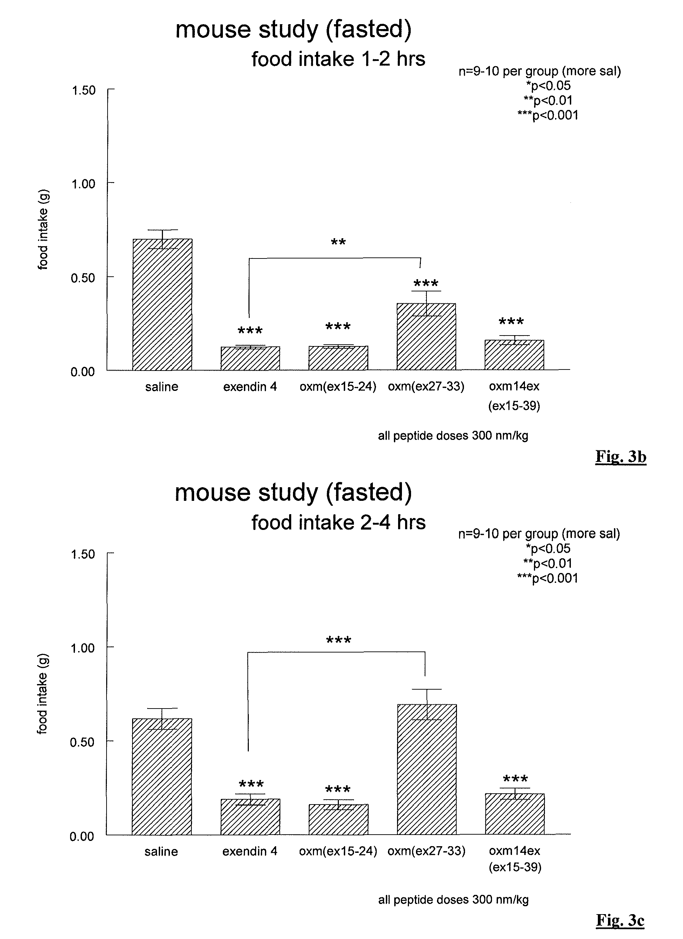

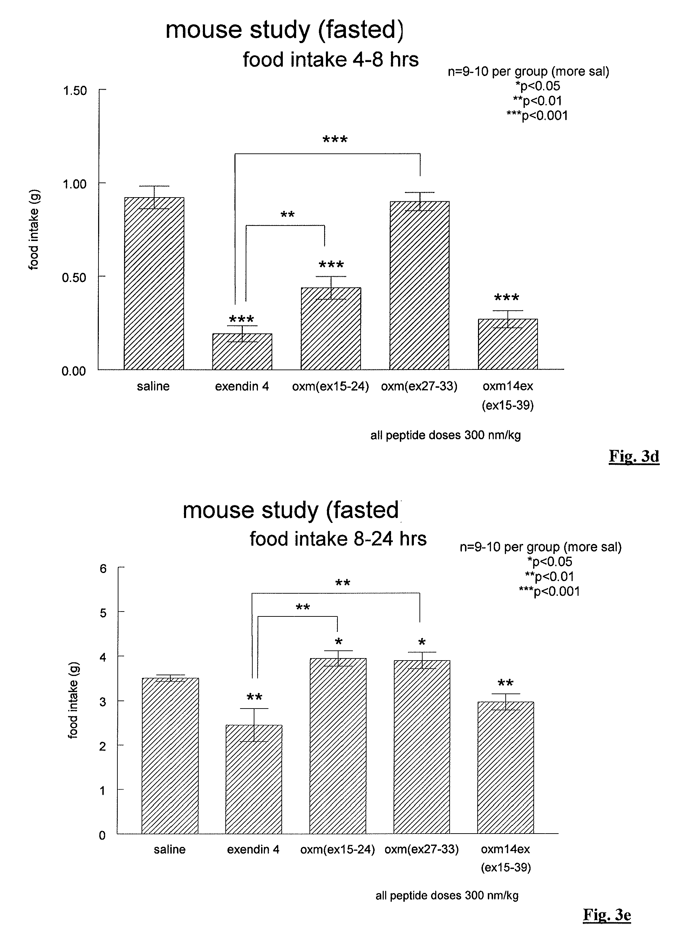

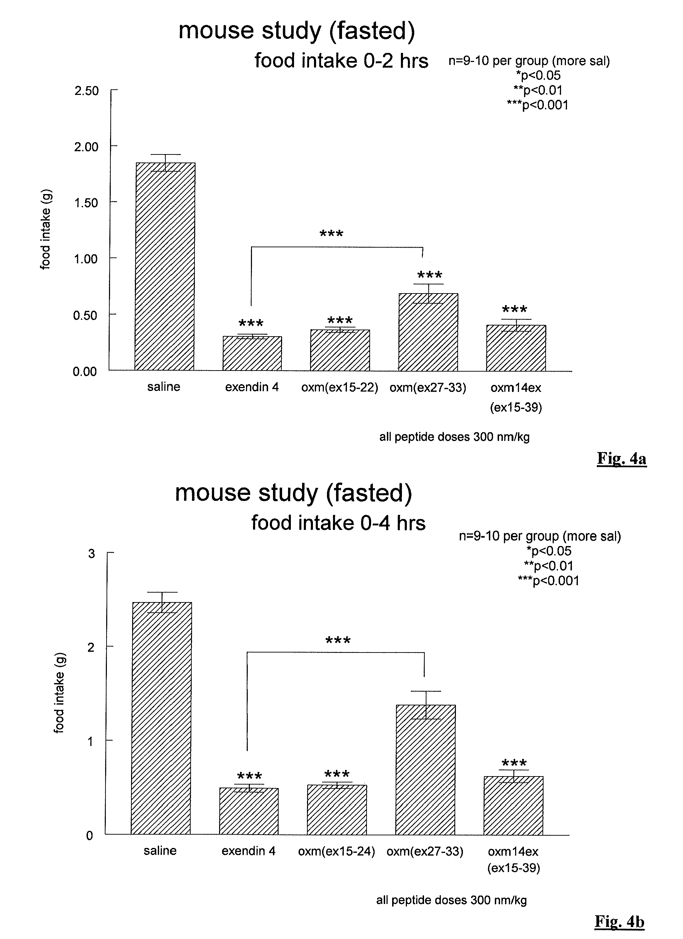

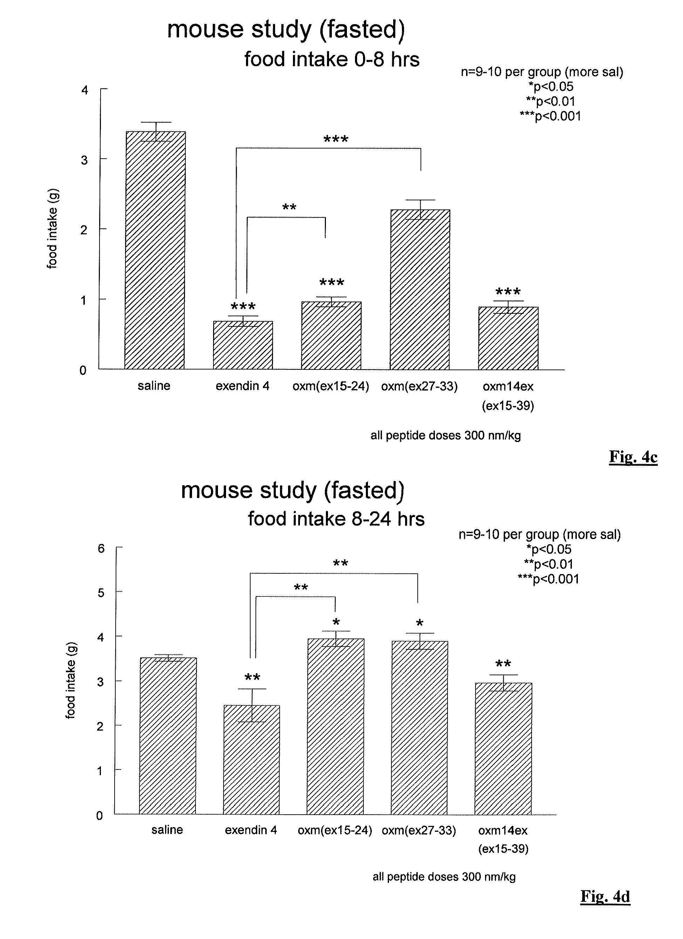

The measured food intake for each group is shown in Figs. 3a to 3e for, respectively, the intervals from 0 to 1 hour, from 1 to 2 hours, from 2 to 4 hours, from 4 to 8 hours and from 8 to 24 hours after injection. In Figs. 4a to 4d are shown the cumulative food intake for each group for 2, 4, 8 and 24 hours after injection.

Thus peptides examined are:
oxm (xx15-24):10 amino acid replacement (SEQ ID NO: 16 - see Example 1)

oxm(xx27-33): His Ser Gln Gly Thr Phe Thr Ser Asp Tyr Ser Lys Tyr Leu Asp Ser Arg Arg Ala
Gln Asp Phe Val Gln Trp Leu Lys Asn Gly Gly Pro Ser Ser Asn Asn Ile Ala
(SEQ ID NO: 19)

Exendin 4 (SEQ ID NO: 22):
His Gly Glu Gly Thr Phe Thr Ser Asp Leu Ser Lys Gln Met Glu Glu Glu Ala Val Arg Leu Phe Ile
Glu Trp Leu Lys Asn Gly Gly Pro Ser Ser Gly Ala Pro Pro Pro Ser

oxm(xx15-39):
His Ser  Gln Gly Thr Phe Thr Ser Asp Tyr Ser Lys Tyr Leu Glu Glu Glu Ala Val Arg Leu Phe Ile
Glu Trp Leu Lys Asn Gly Gly Pro Ser Ser Gly Ala Pro Pro Pro Ser

As shown in Example 1, oxm(xx15-24) has a more potent effect on feeding than oxm.
From Figs. 3a to 3c it may be seen that oxm(xx15-24) and oxm(xx15-39 are of similar potency to exendin up to 4 hours. Exendin appears to have a greater effect on food intake in the 4-8 hour period, but the cumulative data in Fig. 4c shows that oxm(xx15-24) reduces food intake only slightly less than exendin over the first 8 hours post-injection.
From Figs. 3a to 3d it may be seen that oxm(xx27-33) is of similar potency to exendin during an initial period.

### Example 3: oxm analogues in which an amino acid sequence in a non-terminal region has been replaced by substitute sequences.

The feeding effects of two oxm analogues incorporating different 4-residue substitutions were investigated. The three compounds correspond to the oxyntomodulin amino acid sequence (SEQ ID NO: 1) with the exception that four sequential lengths have been replaced as follows:

oxm(xx30-33): human oxm (SEQ ID NO: 7) with residues 30 to 33 replaced by the sequence Gly
Pro Ser Ser (SEQ ID NO: 23)

oxm(xx27-33): oxm (SEQ ID NO: 7) with residues 27 to 33 replaced by the sequence Lys Asn
Gly Gly Pro Ser Ser (SEQ ID NO: 24)

The above-defined sequences fall within the mid-section of the oxm molecule and do not encroach on the C-terminal tetrapeptide. The complete sequences are as follows:

oxm(xx30-33):
His Ser Gln Gly Thr Phe Thr Ser Asp Tyr Ser Lys Tyr Leu Asp Ser Arg Arg Ala Gln Asp Phe
Val Gln Trp Leu Met Asn Thr Gly Pro Ser Ser Asn Asn Ile Ala (SEQ ID NO: 17)

oxm(xx27-33):
His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Ser Ser Asn Asn Ile Ala   (SEQ ID NO: 19)

The above peptides were administered by injection to groups of 9 to 10 mice at a dose of 2700 nmol/kg (known as an effective dose for human oxm) as a 'screen' for appetite inhibition effects. Further groups were administered either oxm at the same dosage (for comparison purposes) or saline (control).

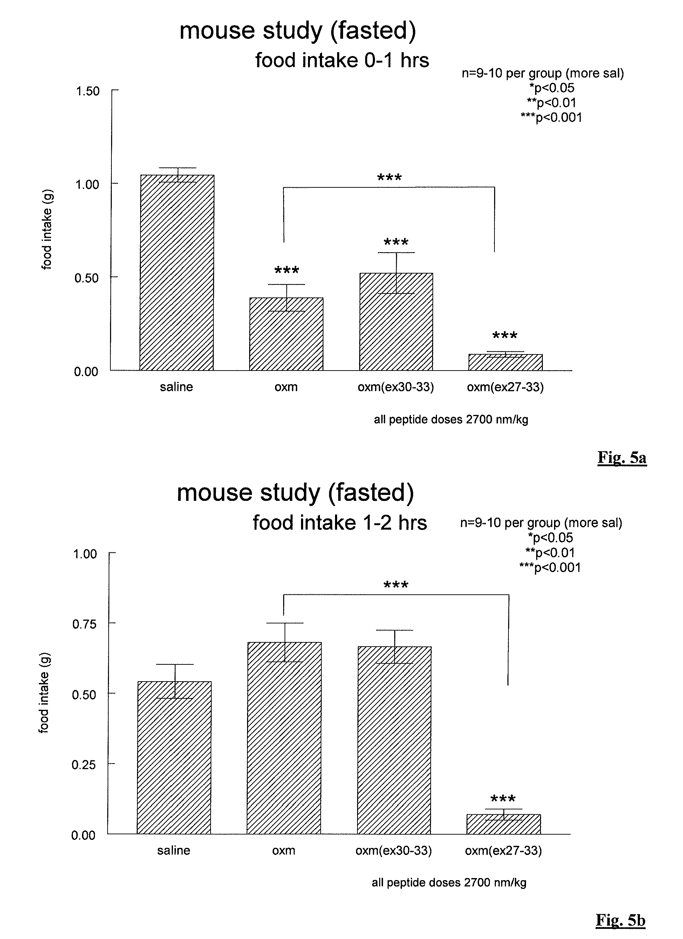

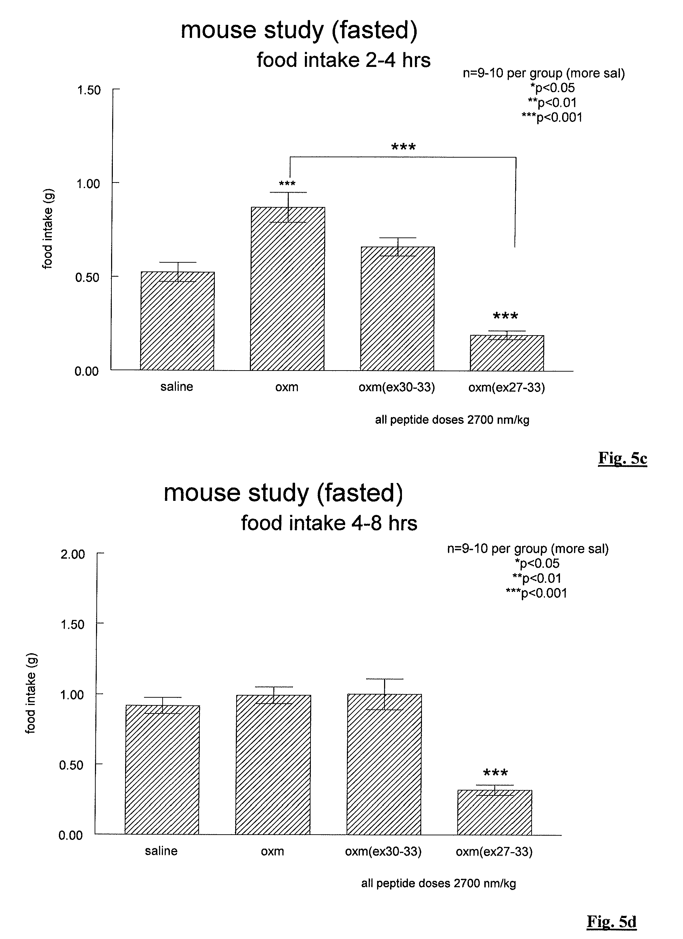

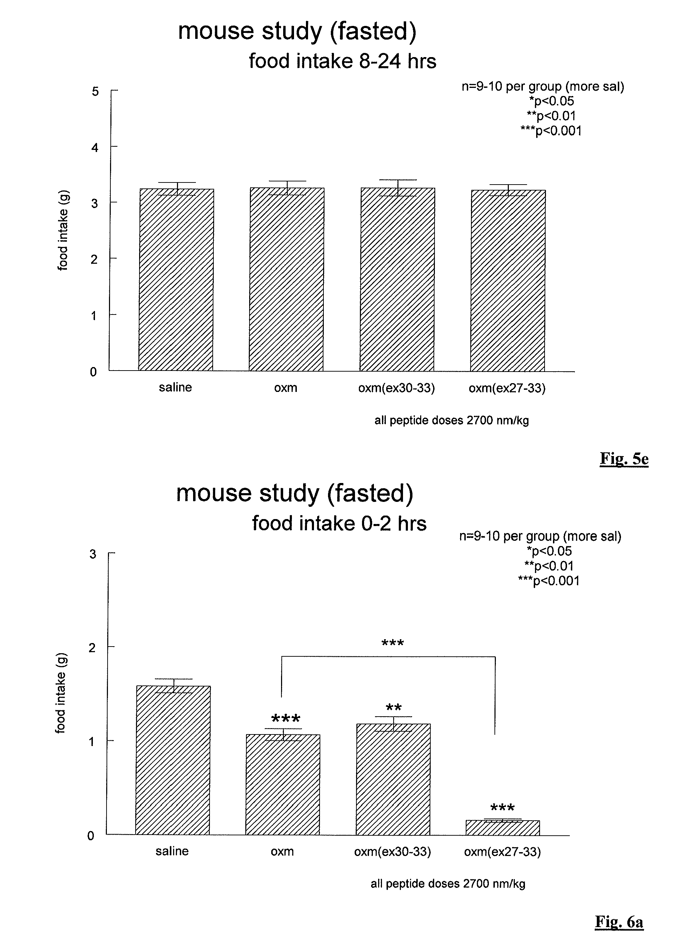

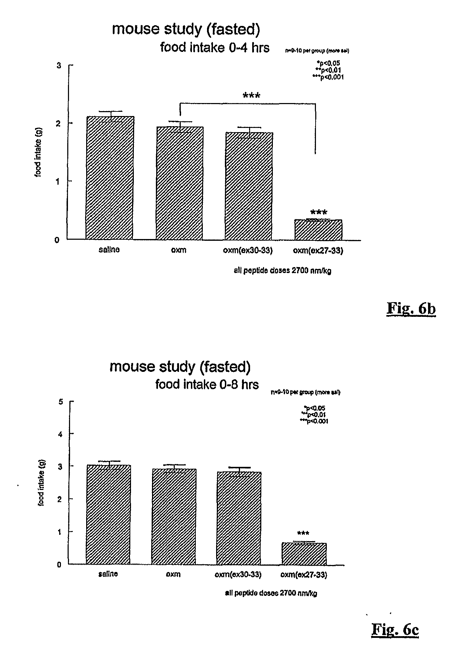

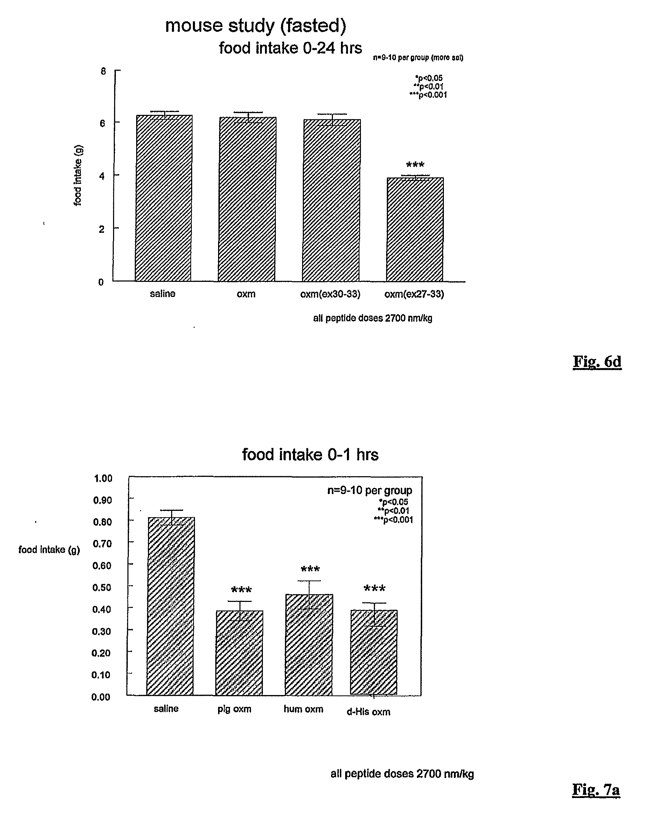

The measured food intake for each group is shown in Figs. 5a to 5e for, respectively, the intervals from 0 to 1 hour, from 1 to 2 hours, from 2 to 4 hours, from 4 to 8 hours, and from 8 to 24 hours after injection. In Figs. 6a to 6d are shown the cumulative food intake for each group for 2, 4, 8 and 24 hours after injection. Results suggest that replacement of 4 amino acids results in increased and prolonged inhibition of food intake compared to both saline and oxm, particularly with oxm(xx27-33).

### Example 4: D-amino-His-oxm

Groups of mice each consisting of from 9 to 10 fasted mice were injected with one of the following:
1-D-amino-His-oxm (full-length human oxm with the histidine at position 1 substituted by D-histidine)
saline (control)
porcine oxm (comparison)
human oxm (comparison)

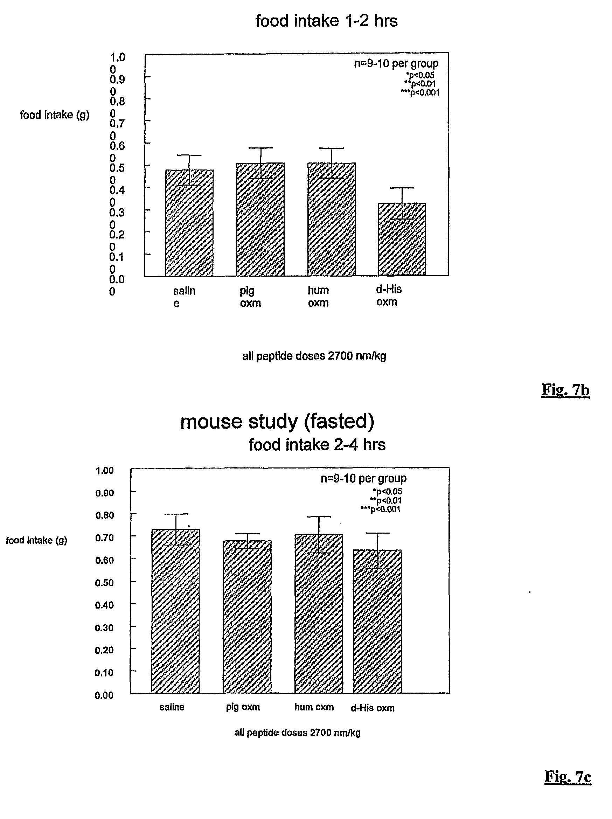

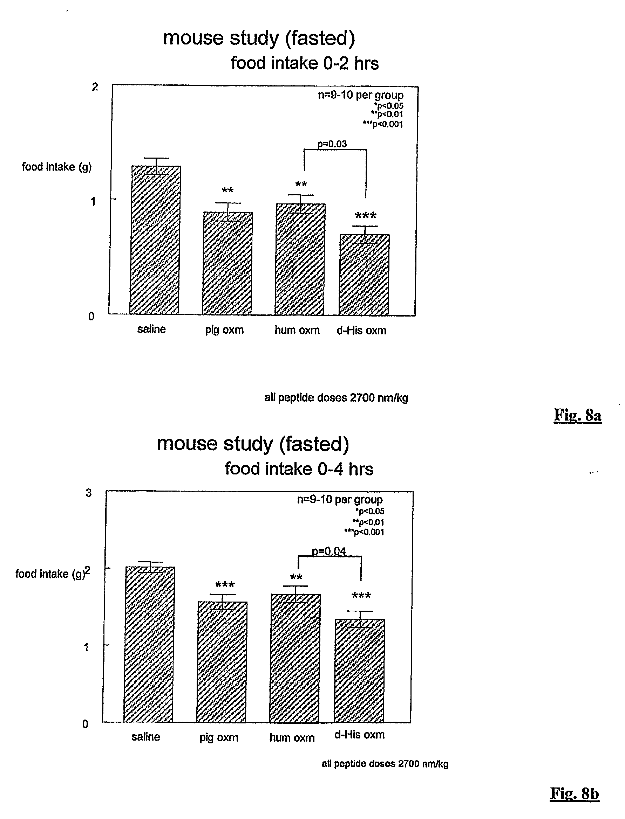

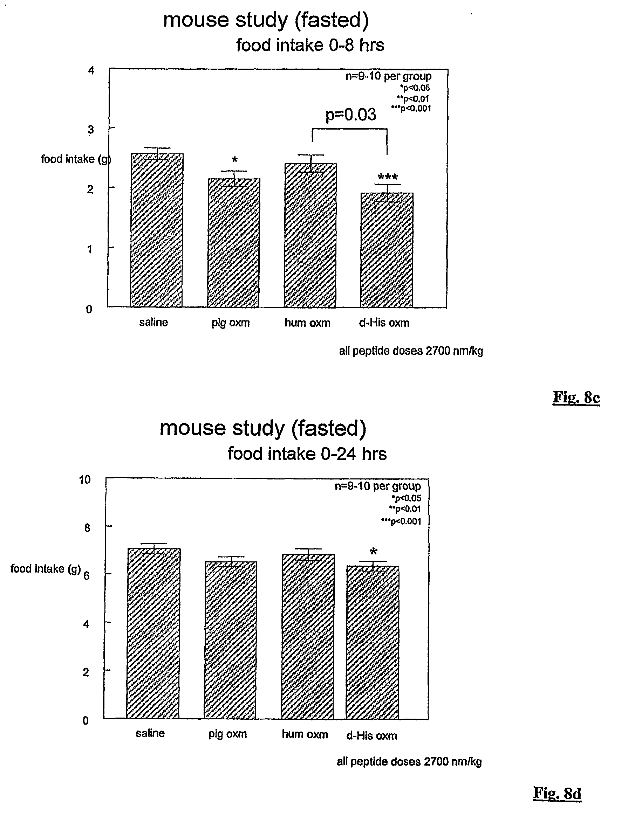

The peptide doses administered were 2700 nmol/kg. The measured food intake for each group is shown in Figs. 7a to 7c for, respectively, the intervals from 0 to 1 hour, from 1 to 2 hours, and from 2 to 4 hours after injection. No significant results were obtained from measurements taken after 4 hours. In Figs. 8a to 8d are shown the cumulative food intake for each group for 2, 4, 8 and 24 hours after injection. From those figures it may be deduced that D-amino-His-oxm is as potent as native human oxm and inhibition of food intake is longer lasting that that seen with unmodified human oxm (p<0.05 for 0-2 and 0-4 hours).

### Example 5: Substitution of residue 2 by Ala, with or without D-amino-His modification at residue 1

Groups of from 9 to 10 fasted mice were each injected with one of the following:
oxm with Ser at residue 2 substituted by Ala ("Ala2-oxm")
oxm with D-His at residue 1 and 2-Ala substitution (D-His-Ala2-oxm")
oxm with 1-D-His at residue 1 and without 2-Ala substitution
oxm (comparison)
saline (control)

The sequence of D-His-Ala 2-oxm is as follows:

D-His Ala Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala
SEQ ID NO:25

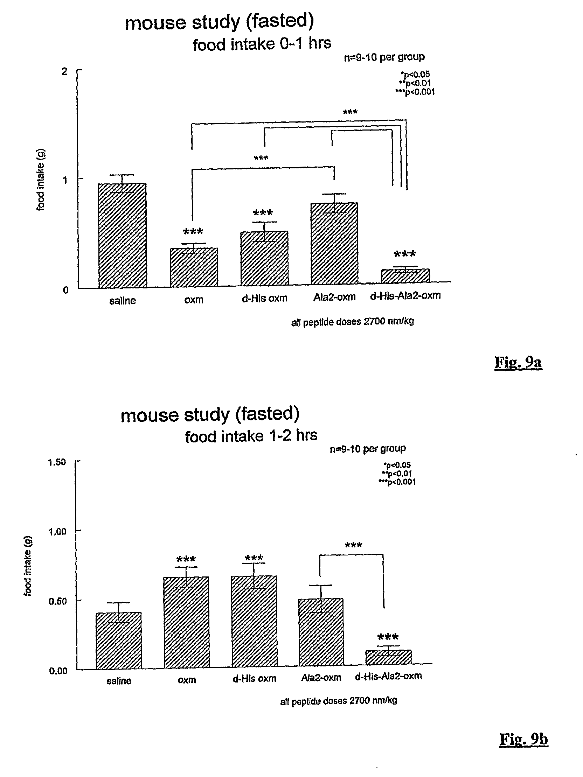

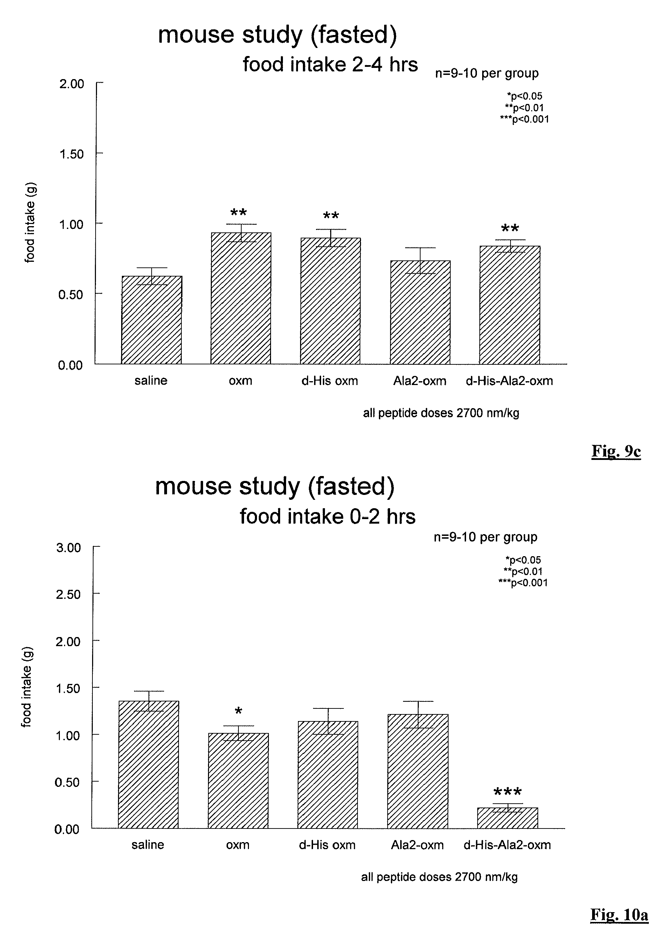

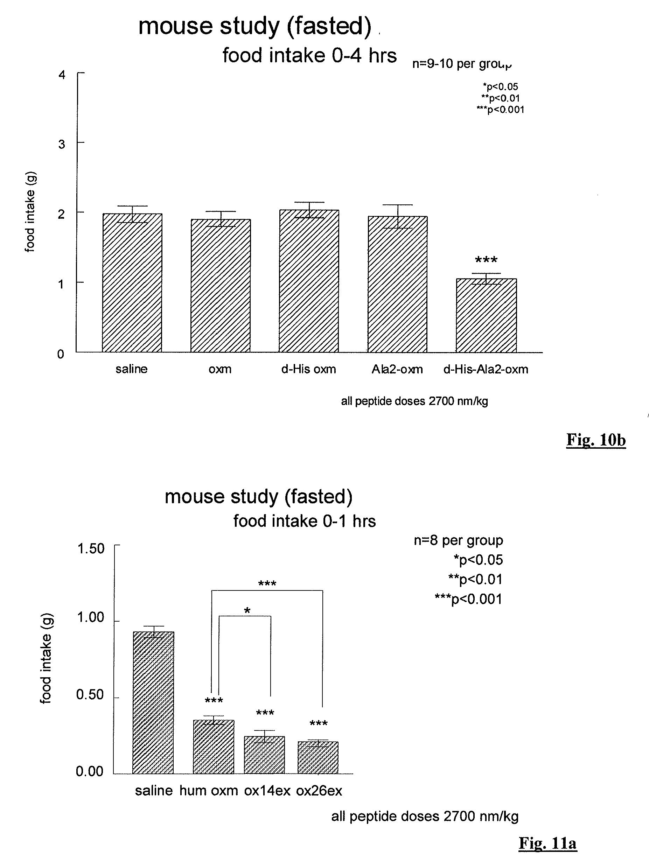

The peptide doses administered were 2700nmol/kg. The measured food intake for each group is shown in Figs. 9a to 9c for, respectively, the intervals from 0 to 1 hour, from 1 to 2 hours, and from 2 to 4 hours after injection. In Figs. 10a and 10b are shown the cumulative food intake for each group for 2 and 4 hours after injection. Oxm reduced food intake as expected. In this study D-His-oxm does reduce food intake, but the results did not confirm any advantage over native oxm.

Ala2-oxm does NOT significantly reduce food intake compared to saline (and its effect is statistically significantly different from that of native oxm). Ala2 modifies the peptide to be more GLP-1-like and it is believed that this makes the peptide more susceptible to dipeptidyl peptidase IV (DPPIV) degradation. D-His-Ala2-oxm is more effective at reducing food intake than Ala2 oxm, which is postulated to be because the D-amino acid in position 1 is likely to provide DPPIV protection.

Surprisingly, D-His-Ala2-oxm is more effective at reducing food intake than native oxm, or, in this Example, than D-His-oxm. It is thought that could be because Ala2 modification aids binding to and activation of the receptor with the postulated DPPIV protection further being afforded by the D-His.

### Example 6: oxm analogues with substitution at oxm27-39 and oxm15-39

The feeding effects of two oxm analogues having the following sequences were investigated:

His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Ser Ser Gly Ala Pro Pro Pro Ser (SEQ ID NO: 26)

His Ser Gln Gly Thr Phe Thr Ser Asp Tyr Ser Lys Tyr Leu Glu Glu Glu Ala Val Arg Leu Phe Ile
Glu Trp Leu Lys Asn Gly Gly Pro Ser Ser Gly Ala Pro Pro Pro Ser (SEQ ID NO: 27)

The above analogue SEQ ID NO: 26 consists of oxm1-26 with a tail corresponding to amino acids 27 to 39 of exendin 4 (that is, amino acids 27 to 39 of SEQ ID NO: 22) attached at the C-terminal end. SEQ ID NO: 27 consists of oxm1-14 with a tail corresponding to amino acids 15 to 39 of exendin 4.

The above peptides were administered by injection to groups of 9 to 10 mice at a dose of 2700 nmol/kg (known as an effective dose for human oxm) as a 'screen' for appetite inhibition effects. Further groups were administered either native human oxm at the same dosage (for comparison purposes) or saline (control).

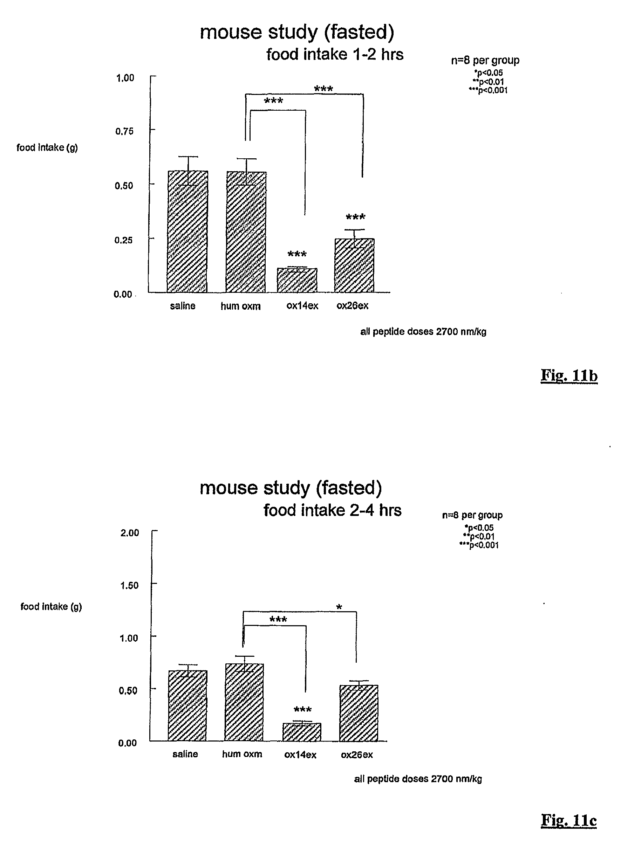

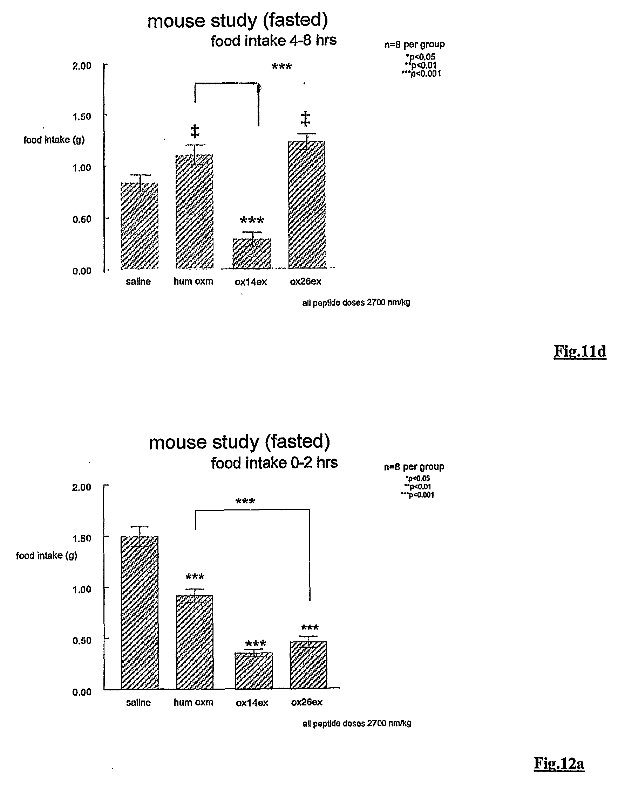

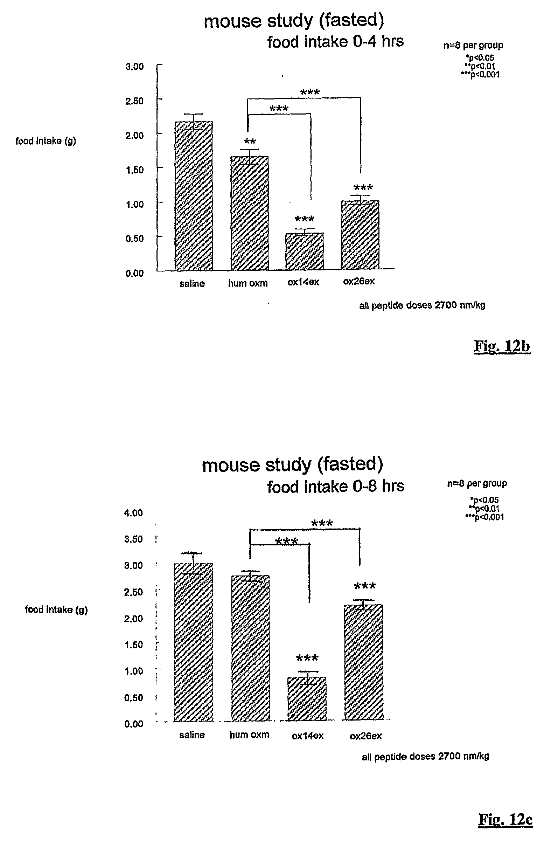

The measured food intake for each group is shown in Figs. **11a to 11d** for, respectively, the intervals from 0 to 1 hour, from 1 to 2 hours, from 2 to 4 hours, and 4 to 8 hours after injection. In Figs. **12a to 12c** are shown the cumulative food intake for each group for 2 & 4 after injection.

The results show that oxm26ex (SEQ ID NO: 26) and oxm14ex (SEQ ID NO: 27) have a potent and long-lasting effect on food intake.

It has previously been thought that exendin is more potent at the GLP1 receptor than GLP1 itself, in large part due to the absence of a residue 2 alanine adjacent to the N-terminal end, it being hypothesized that that is the reason for the resistance of exendin to DPPIV. This Example suggests that, surprisingly, another effect is present, as the exendin portions were from the C-terminal end. It is thought that the strong activities of oxm26ex and oxm14ex may be due to a strong resistance to endopeptidases.

### Example 7: Lower dosage study

oxm26ex (SEQ ID NO: 26) and oxm14ex (SEQ ID NO: 27) were administered by injection to groups of 9 to 10 mice at a dose of 1000 nmol/kg. Further groups were administered native human oxm, GLP-1 or exendin 4 at the same dose (for comparison purposes), or saline (control).

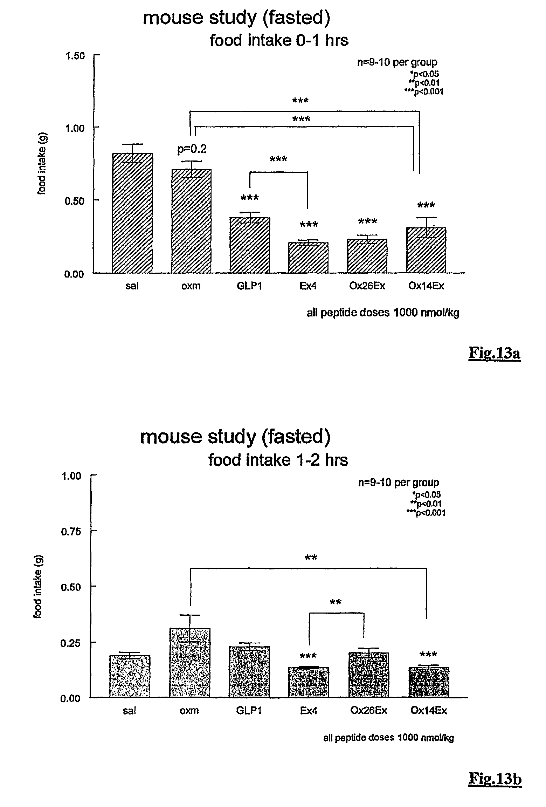

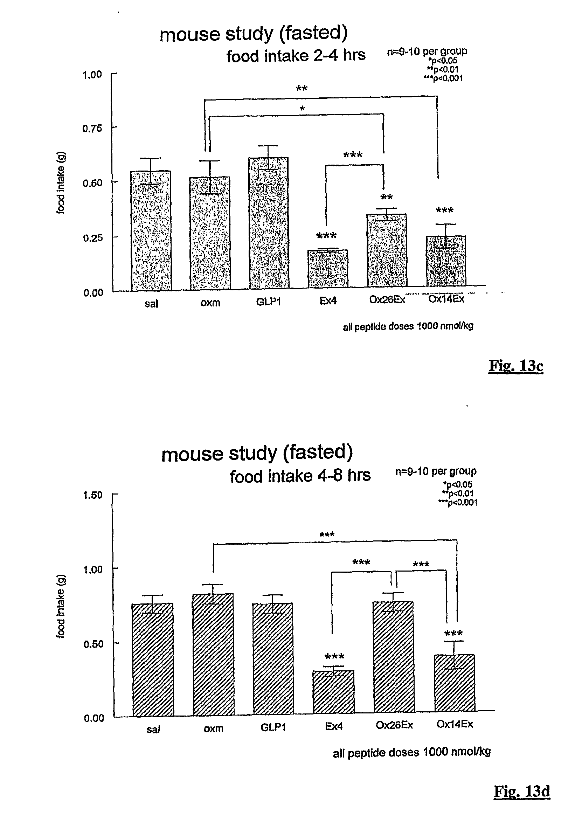

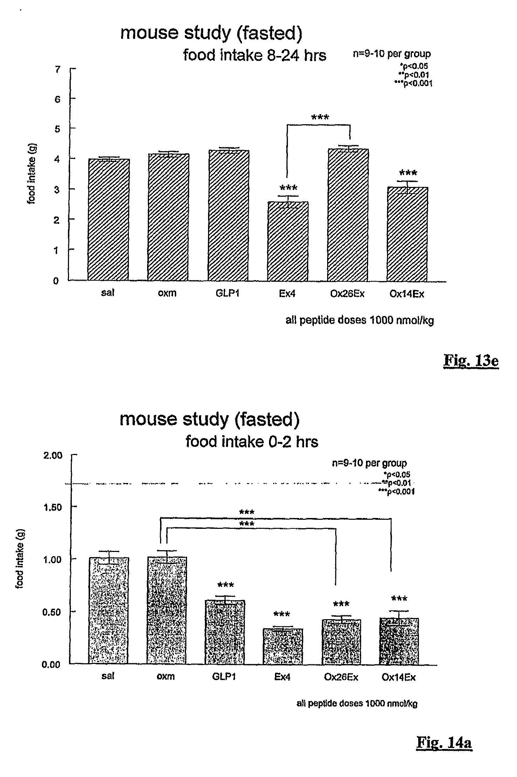

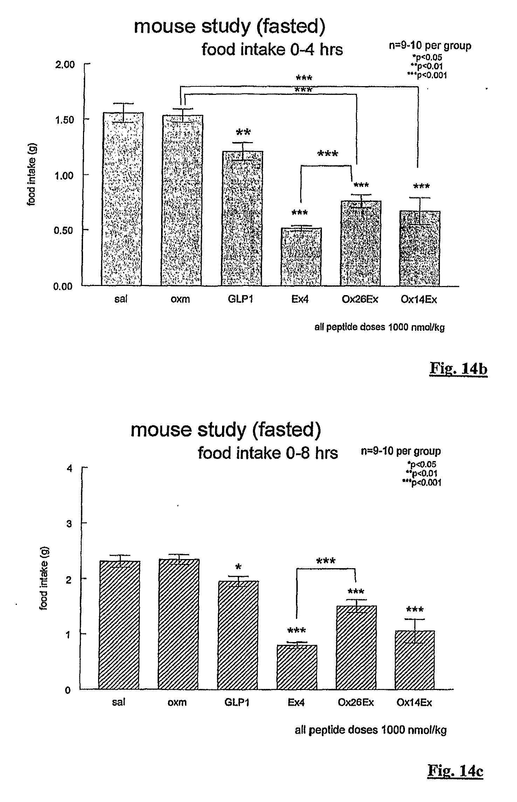

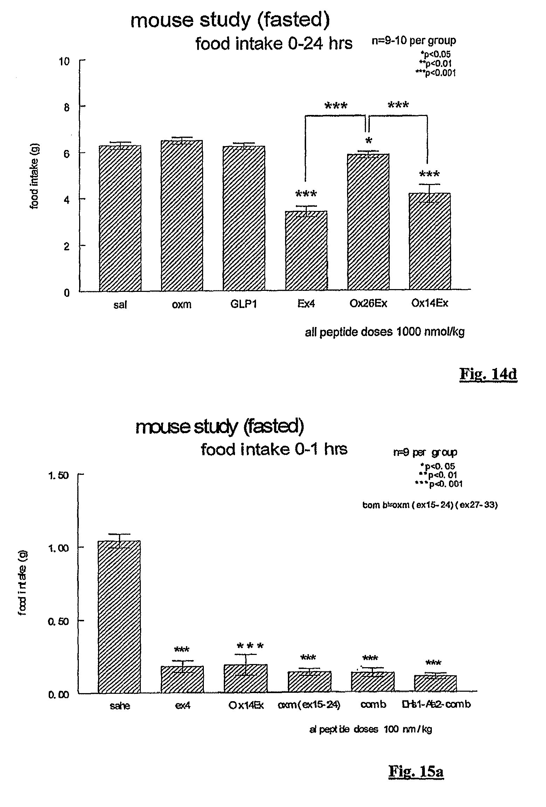

The measured food intake for each group is shown in Figs. 13a to 13e for, respectively, the intervals from 0 to 1 hour, from 1 to 2 hours, from 2 to 4 hours, 4 to 8 hours and 8 to 24 hours after injection. In Figs. 14a to 14d are shown the cumulative food intake for each group for 2, 4, 8 and 24 hours after injection.

In this Example the feeding effect of oxm at 1000 nmol/kg did not quite reach statistical significance. Exendin-4 inhibits food intake. Unsurprisingly, Exendin is more potent than GLP-1 at this dose. oxm26ex and oxm14ex significantly inhibit feeding both compared to saline control and compared to the oxm-treated group, whilst the effects of oxm26ex are not as long-lasting as those of exendin.

### Example 8: oxm analogues with more than one substitute sequence.

The feeding effects of the following oxm analogues were investigated. The three compounds correspond to the oxyntomodulin amino acid sequence (SEQ ID NO: 7) with the exception that sequential lengths have been replaced as follows:
ox14ex (SEQ ID NO: 27- see Example 6)
oxm(xx15-24): See Example 1 SEQ ID NO: 16

oxm(xx15-24)(xx27-33):
His Ser Gln Gly Thr Phe Thr Ser Asp Tyr Ser Lys Tyr Leu Glu Glu Glu Ala Val Arg Leu Phe Ile
Glu Trp Leu Lys Asn Gly Gly Pro Ser Ser Asn Asn Ile Ala (SEQ ID NO: 28)

D-His1-Ala2-oxm(xx15-24)(xx27-33):
D-His Ala Gln Gly Thr Phe Thr Ser Asp Tyr Ser Lys Tyr Leu Glu Glu Glu Ala Val Arg Leu Phe
Ile Glu Trp Leu Lys Asn Gly Gly Pro Ser Ser Asn Asn Ile Ala (SEQ ID NO: 29)

The above test peptides were administered by injection to groups of 9 to 10 mice at a dose of 100 nmol/kg. Further groups were administered either Exendin 4 at the same dosage (for comparison purposes) or saline (control).

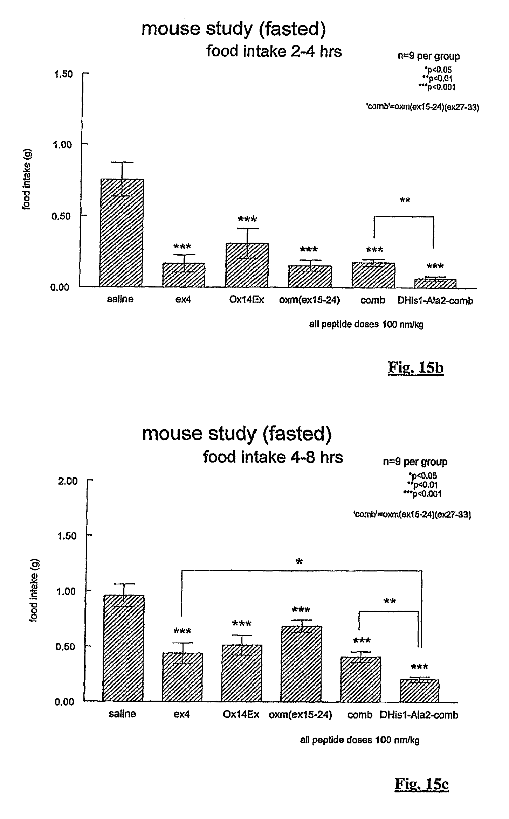

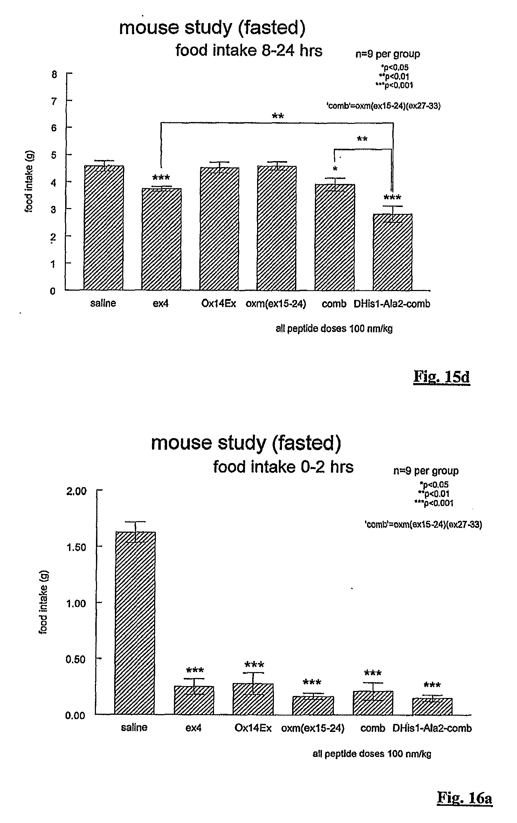

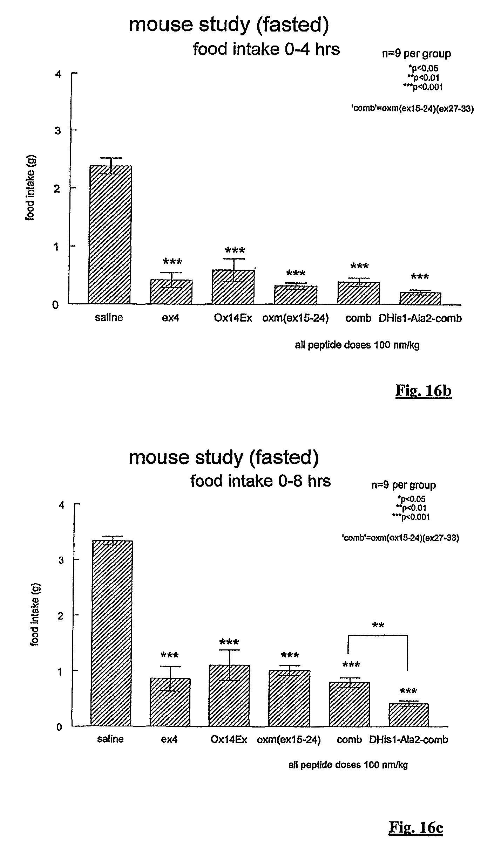

The measured food intake for each group is shown in Figs. 15a to 15d for, respectively, the intervals from 0 to 1 hour, from 2 to 4 hours, from 4 to 8 hours and from 8 to 24 hours after injection. In Figs. 16a to 16c are shown the cumulative food intake for each group for 2, 4, and 8 hours after injection. Results suggest that all the test peptides are effective compared to saline. Figs. 15d and 15e suggest that oxm(xx15-24)(xx27-33) may be more effective than oxm(xx15-24). The D-His1-Ala2-oxm(xx15-24)(xx27-33) analogue is especially long-acting. Indeed, Fig. 16c suggests that that analogue may be even longer-acting than Exendin 4.

### Example 9: Dosage study

The feeding effects of the following oxm analogues were investigated to ascertain suitable dosages:
D-His-Ala2-oxm: See Example 5 SEQ ID NO: 25
oxm(xx15-21): See Example 1 SEQ ID NO: 15
oxm(xx15-24): See Example 1 SEQ ID NO: 16
oxm(xx27-33): See Example 3 SEQ ID NO: 19
D-His1-Ala2-oxm(xx15-24)(xx27-33): See Example 8 - SEQ ID NO: 29.

The above test peptides were administered by injection to groups of 9 to 10 mice at various dosages (all in nmol/kg) as shown in the Table below.

**Table 2**

| SEQ ID NO: | 1^{st} dosage | 2^{nd} dosage | 3^{rd} dosage | 4^{th} dosage |
|---|---|---|---|---|
| 25 | 250 | 500 | 1000 | 2000 |
| 15 | 3 | 10 | 30 | 100 |
| 16 | 1 | 3 | 10 | 30 |
| 19 | 3 | 10 | 30 | 100 |
| 29 | 0.1 | 0.3 | 0.9 | - |

Further groups were administered either oxm at a dosage of 1400 nmol/kg or saline (control).

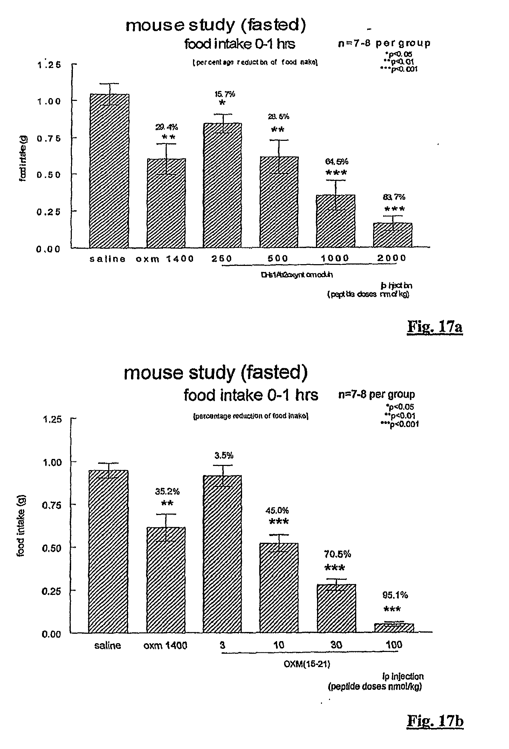

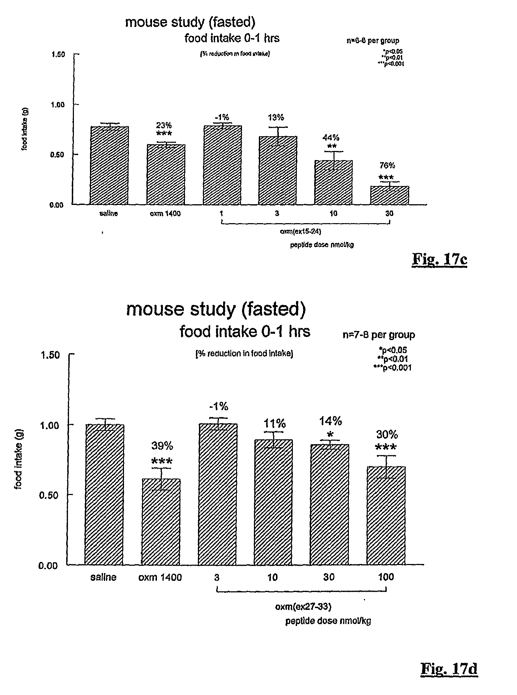

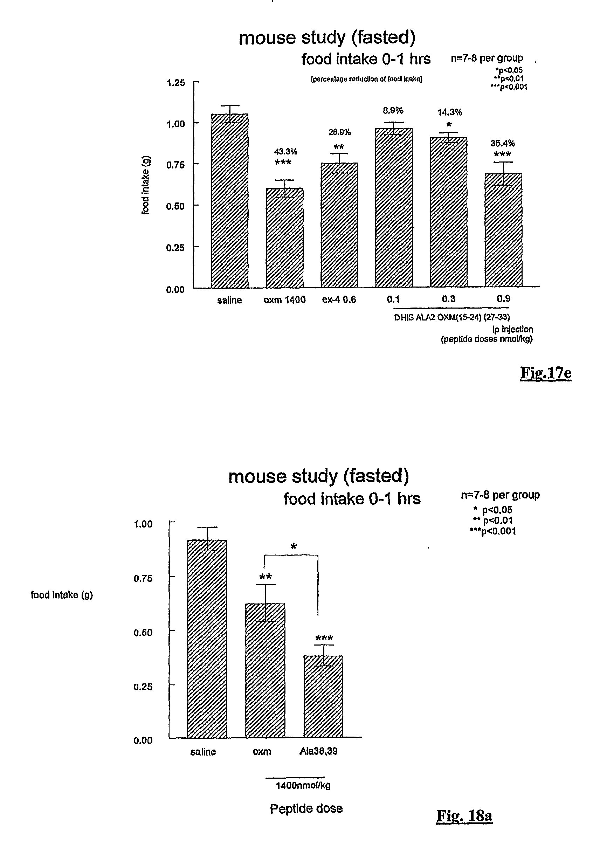

The measured food intake during the first hour after injection is shown for each group in Figs. 17a to 17e. The following table summarises the activity of each of the test peptides relative to oxm.

| Multiples of food inhibition | oxm | Oxm(DHis1 Ala2) | Oxm(xx 15-21) | Oxm(xx15-24) | Oxm(xx27-33) | Oxm(DHisAla2)(xx15-24)(xx27-33) |
|---|---|---|---|---|---|---|
| Factor | 1 | 2.5 | 200 | 400 | 7 | 1000-4000 |

The appetite suppressant Exendin 4 has a potency, calculated according to the same method, of approximately 2000.

### Example 10: Addition of Ala-Ala extension at C-terminal end of oxm

The feeding effects of the following oxm analogue was investigated. The analogue corresponds to the oxyntomodulin amino acid sequence (SEQ ID NO: 1) with the exception that two additional Ala moieties have been included at the C-terminus, that is, at positions 38 and 39:

His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala Ala Ala
SEQ ID NO: 30

The above test peptide was administered by injection to groups of 9 to 10 mice at a dose of 1400 nmol/kg. Further groups were administered either full-length human oxm at the same dosage (for comparison purposes) or saline (control).

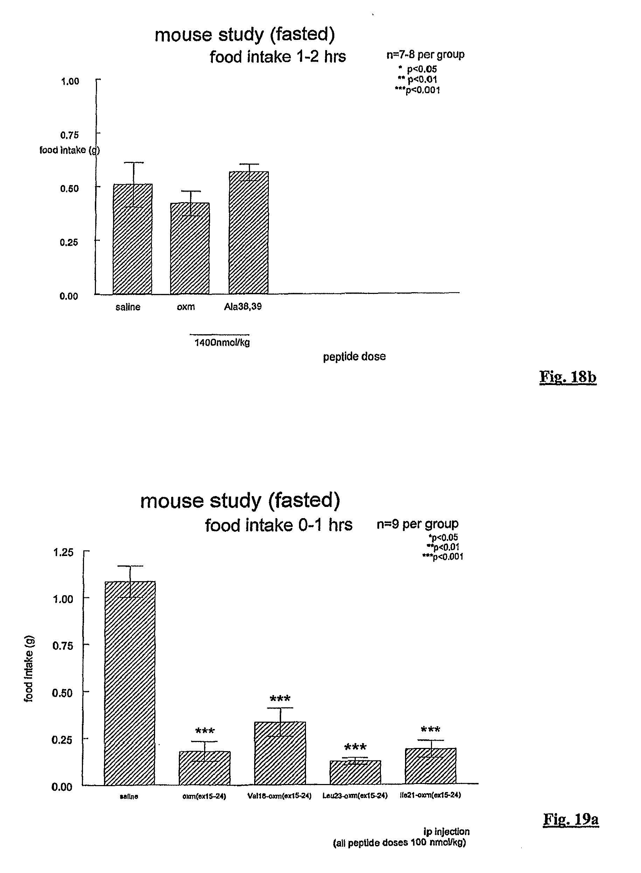

The measured food intake for each group is shown in Figs. 18a and 18b for, respectively, the intervals from 0 to 1 hour, and from 1 to 2 hours after injection. Fig. 18a demonstrates that the addition of the two Ala residues increases the reduction in food intake by comparison to native oxm.

### Example 11: oxm derivatives in which 10 amino acids at positions 15-24 have been replaced by substitute sequences.

The feeding effects of four oxm analogues each incorporating a 10-residue substitution were investigated. The four compounds correspond to SEQ ID NO: 7 with the exception that in each case a single amino acid in the substituted sequence had been replaced as follows:
(i) replacement of Ala by Val at residue 18: 
Val18-oxm(xx15-24): His Ser Gln Gly Thr Phe Thr Ser Asp Tyr Ser Lys Tyr Leu Glu Glu Glu
Val Val Arg Leu Phe Ile Glu Trp Leu Met Asn Thr Lys Arg Asn Arg Asn Asn Ile Ala (SEQ ID
NO: 31)
(ii) replacement of Leu by Ile at residue 21 
Ile21-oxm(xx15-24): His Ser Gln Gly Thr Phe Thr Ser Asp Tyr Ser Lys Tyr Leu Glu Glu Glu Ala
Val Arg Ile Phe Ile Glu Trp Leu Met Asn Thr Lys Arg Asn Arg Asn Asn Ile Ala (SEQ ID NO:
32)
(iii) replacement of Ile by Leu at residue 23

Leu23-oxm(xx15-24): His Ser Gln Gly Thr Phe Thr Ser Asp Tyr Ser Lys Tyr Leu Glu Glu Glu
Ala Val Arg Leu Phe Leu Glu Trp Leu Met Asn Thr Lys Arg Asn Arg Asn Asn Ile Ala (SEQ ID
NO: 33)

The above peptides were administered by injection to groups of 9 to 10 mice at a dose of 100 nmol/kg. Further groups were administered either oxm(xx15-24) or saline (control).

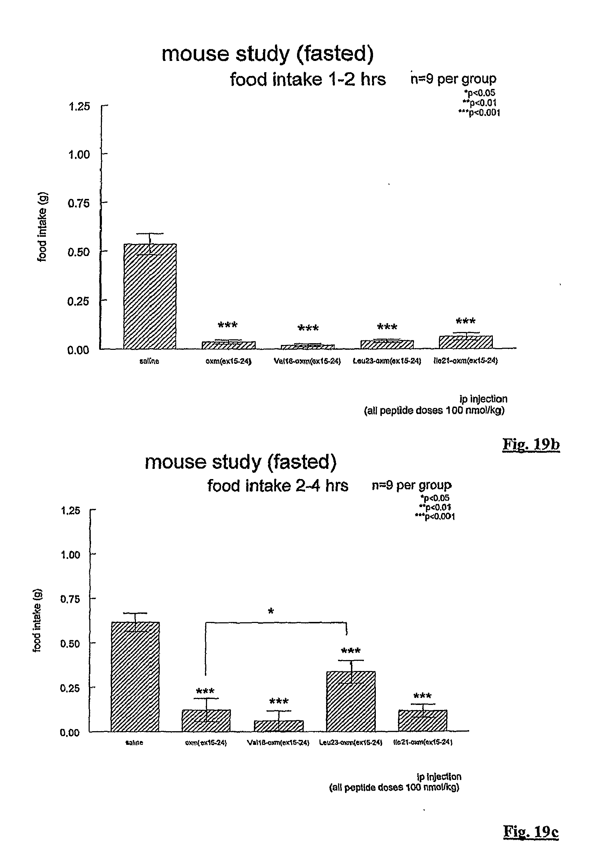

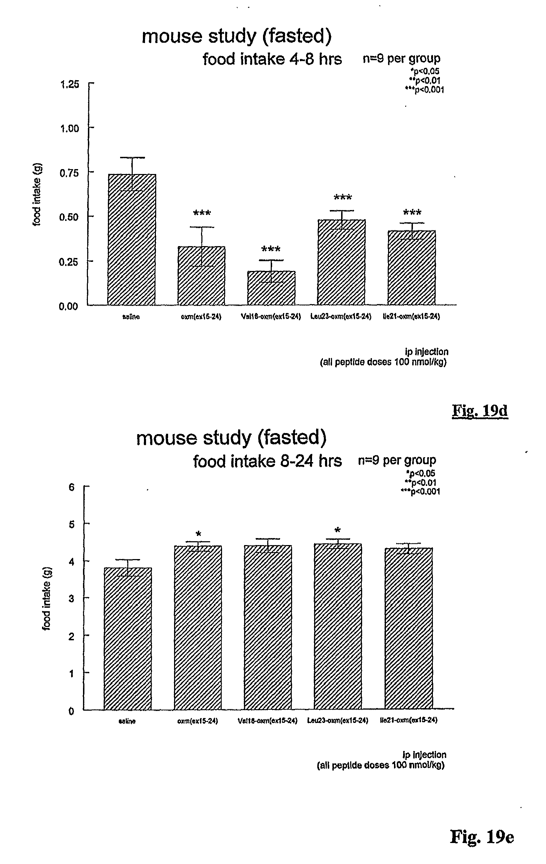

The measured food intake for each group is shown in Figs. 19a to 19e for, respectively, the intervals from 0 to 1 hour, from 1 to 2 hours, from 2 to 4 hours, 4 to 8 hours, and 8 to 24 hours after injection. The results in Figs. 19a to 19e suggest that the variants 18Val-oxm(xx15-24), 21Ile-oxm(xx15-24) and Leu23-oxm(xx15-24) offer similar potency to oxm(xx15-24). Furthermore, in the case of 18Val-oxm(xx15-24), there is a delayed onset of activity as compared with oxm(xx15-24) whilst the activity is more prolonged (see Figs. 19a to 19d with particular reference to Figs. 19a and 19d. The showing of delayed onset offers the prospect of a smoother blood curve, which may permit a relatively large dose to be administered without unacceptable initial nausea, and with the duration of effect post-administration being greater, thereby reducing the likelihood of escape. Tests under analogous conditions using the analogue Ile19-oxm(xx15-24): 
His Ser Gln Gly Thr Phe Thr Ser Asp Tyr Ser Lys Tyr Leu Glu Glu Glu Ala Ile Arg Leu Phe Ile
Glu Trp Leu Met Asn Thr Lys Arg Asn Arg Asn Asn Ile Ala (SEQ ID NO: 34)
 showed that analogue to be equipotent with or slightly more potent than oxm(xx15-24), as well as having a potentially beneficial slightly delayed action as compared with oxm (xx15-24).

### Example 12: Further data concerning substitution of 2-residue by Ala with or without D-amino-His modification

Groups of from 9 to 10 fasted mice were each injected with one of the test compounds

Two oxm analogues were tested with substitutions of the first amino acid (His 1). Compounds tested were
Des-His-oxm
D-His-oxm
Porcine oxm (comparison)
Human oxm (control)

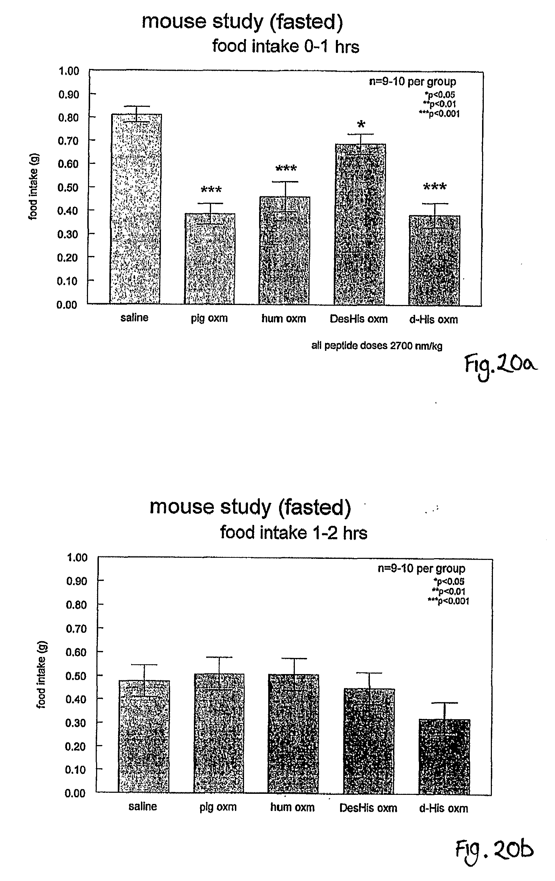

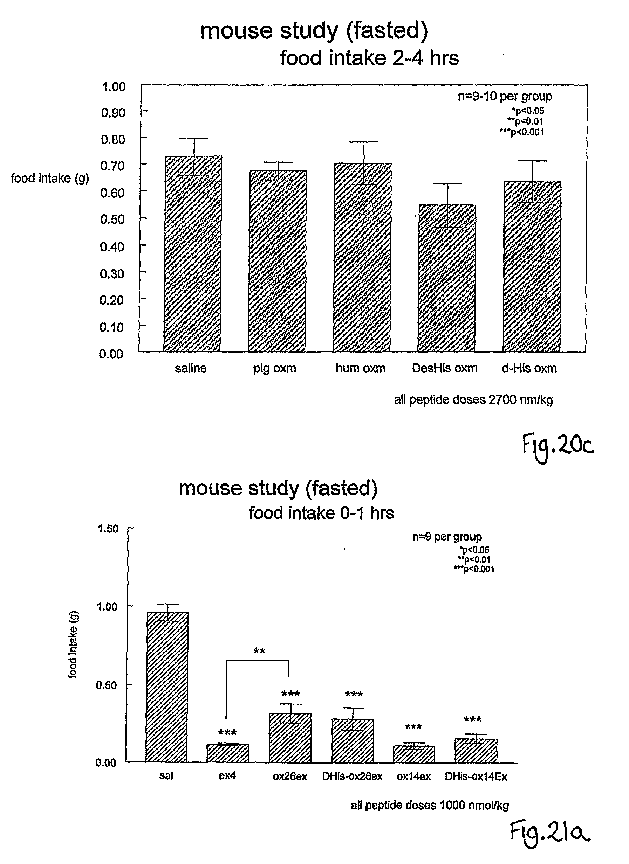

The peptide dose administered was 2700nmol/kg. The measured food intake for each group is shown in Figs. 20a to 20c for, respectively, the intervals from 0 to 1 hour, from 1 to 2 hours, and from 2 to 4 hours after injection. Des-His-oxm and D-his-oxm also reduced food intake. These variant compounds showed a more sustained action than native oxm.

### Example 13: oxm derivatives

Groups of fasted mice were each injected with one of the following:
oxm26ex (SEQ ID NO: 26) 
D-His-oxm26ex
D-His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Ser Ser Gly Ala Pro Pro Pro Ser (SEQ ID NO: 36)
oxm14Ex (SEQ ID NO: 27)

D-His-oxm14ex
D-His Ser Gln Gly Thr Phe Thr Ser Asp Tyr Ser Lys Tyr Leu Glu Glu Glu Ala Val Arg Leu Phe
Ile Glu Trp Leu Lys Asn Gly Gly Pro Ser Ser Gly Ala Pro Pro Pro Ser (SEQ ID NO: 57)

The compounds were administered by injection to groups of 9 to 10 mice at a dose of 1000 nmol/kg . Further groups were administered exendin 4 at the same dose (for comparison purposes), or saline (control).

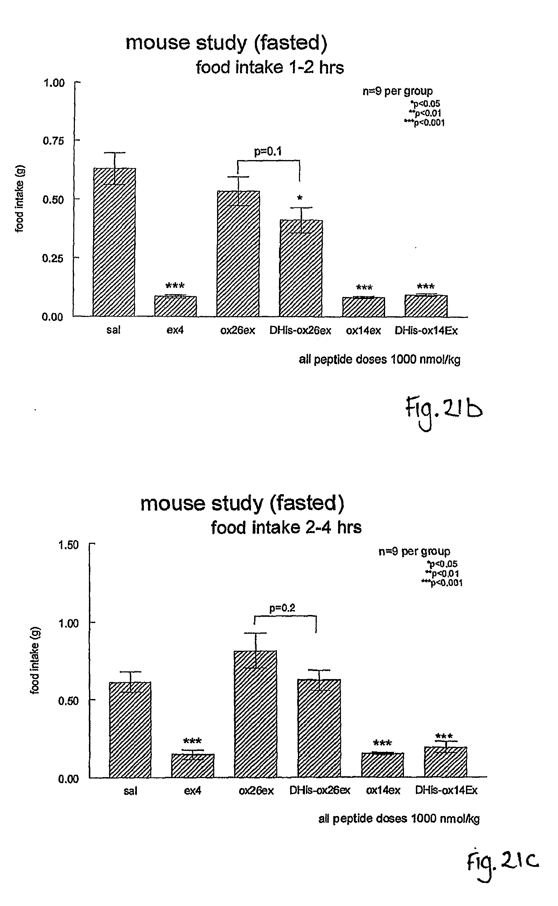

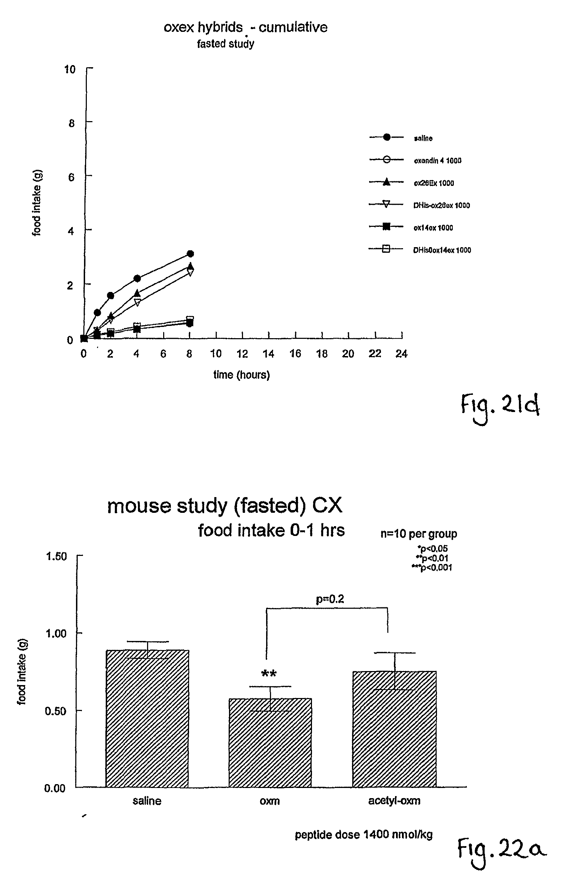

The measured food intake for each group is shown in Figs. 21a to 21c for, respectively, the intervals from 0 to 1 hour, from 1 to 2 hours, and from 2 to 4 hours after injection. Fig 21d shows cumulative data in which D-His-oxm26ex shows a reduction in food intake in comparison to oxm26ex, and all analogues show marked reduction in food intake in comparision to the saline controls.

### Example 14: comparison of oxm and acetyl oxm

Groups of fasted mice were each injected with one of the following:
Oxyntomodulin
Acetyloxyntomodulin 
CH3-CO-NH-CRH-CO-Ser Gln Gly Thr Phe Thr Ser Asp Tyr Ser Lys Tyr Leu Asp Ser Arg Arg
Ala Gln Asp Phe Val Gln Trp Leu Met Asn Thr Lys Arg Asn Arg Asn Asn Ile Ala (where R is
the side group of His).

The compounds were administered by injection to groups of 9 to 10 mice at a dose of 1400 nmol/kg. A further group was administered saline (control).

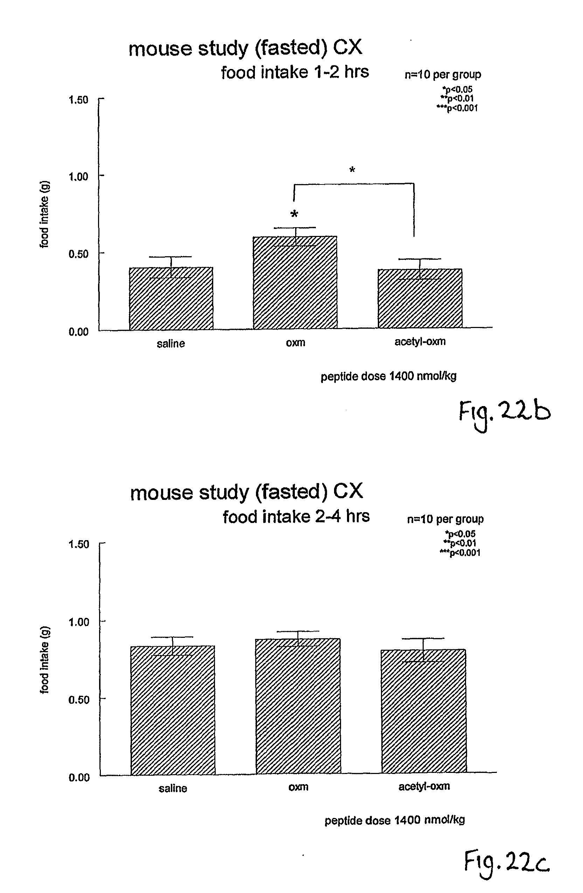

The measured food intake for each group is shown in Figs. 22a to 22c for, respectively, the intervals from 0 to 1 hour, from 1 to 2 hours, and from 2 to 4 hours after injection.

The data shows activity for both compounds. Acetyloxyntomodulin's anorectic effect is later acting than that of oxyntomodulin.

### Example 15: oxm variants

Groups of fasted mice were each injected with one of the following:
Val23-oxm(ex15-24) 
His Ser Gln Gly Thr Phe Thr Ser Asp Tyr Ser Lys Tyr Leu Glu Glu Glu Ala Val Arg Leu Phe Val
Glu Trp Leu Met Asn Thr Lys Arg Asn Arg Asn Asn Ile Ala (SEQ ID NO: 37)
Ala19-Val23-oxm(ex15-24)

His Ser Gln Gly Thr Phe Thr Ser Asp Tyr Ser Lys Tyr Leu Glu Glu Glu Ala Ala Arg Leu Phe Ile
Glu Trp Leu Met Asn Thr Lys Arg Asn Arg Asn Asn Ile Ala (SEQ ID NO: 38)

The compounds were administered by injection to groups of mice at a dose of 100 nmol/kg. A further group was administered saline (control).

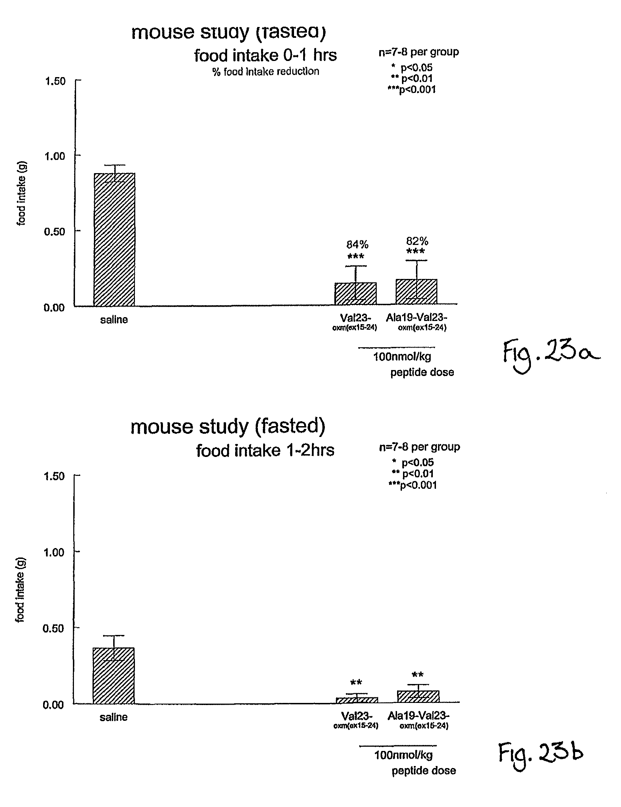

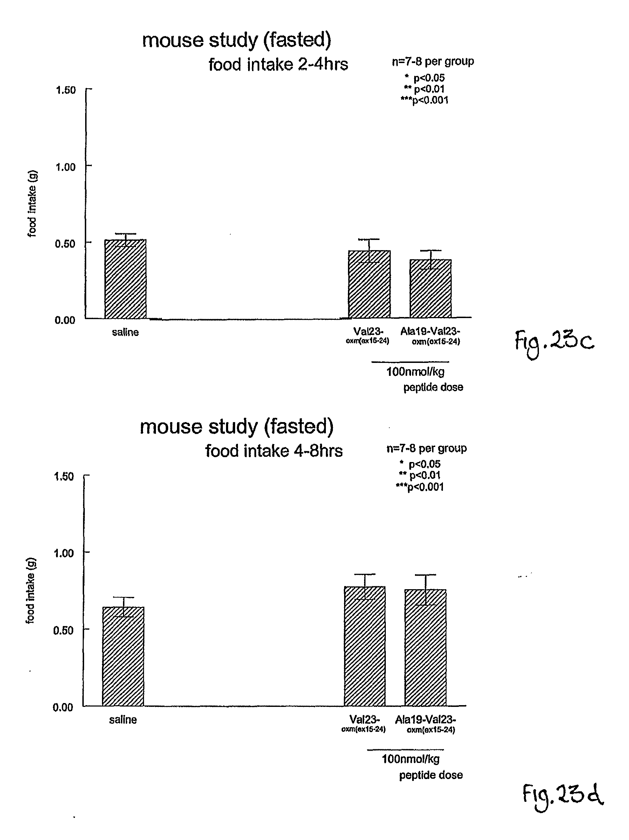

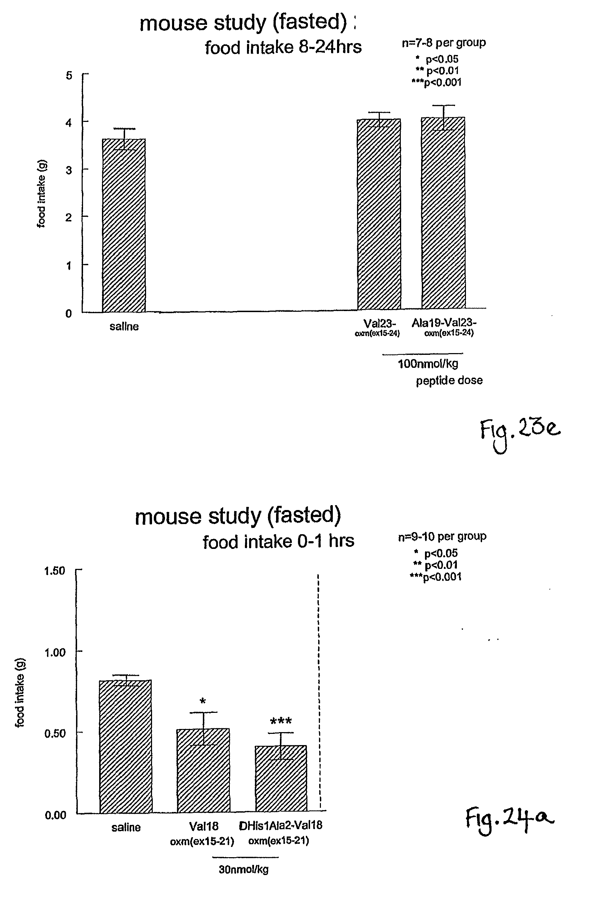

The measured food intake for each group is shown in Figs. 23a to 23e for, respectively, the intervals from 0 to 1 hour, from 1 to 2 hours, from 2 to 4, from 4 to 8 hours and from 8 to 24 hours after injection. A reduction in food intake from 0 to 4 hours is shown.

### Example 16: oxm variants

Groups of fasted mice were each injected with one of the following:
Val18-oxm(ex15-21) 
His Ser Gln Gly Thr Phe Thr Ser Asp Tyr Ser Lys Tyr Leu Glu Glu Glu Val Val Arg Leu Phe Val
Gln Trp Leu Met Asn Thr Lys Arg Asn Arg Asn Asn Ile Ala (SEQ IS NO: 40)
D-His1-Ala2-Val18-oxm(ex15-21)

D-His Ala Gln Gly Thr Phe Thr Ser Asp Tyr Ser Lys Tyr Leu Glu Glu Glu Val Val Arg Leu Phe
Val Gln Trp Leu Met Asn Thr Lys Arg Asn Arg Asn Asn Ile Ala (SEQ ID NO: 41)

The compounds were administered by injection to groups of mice at a dose of 30 nmol/kg. A further group was administered saline (control).

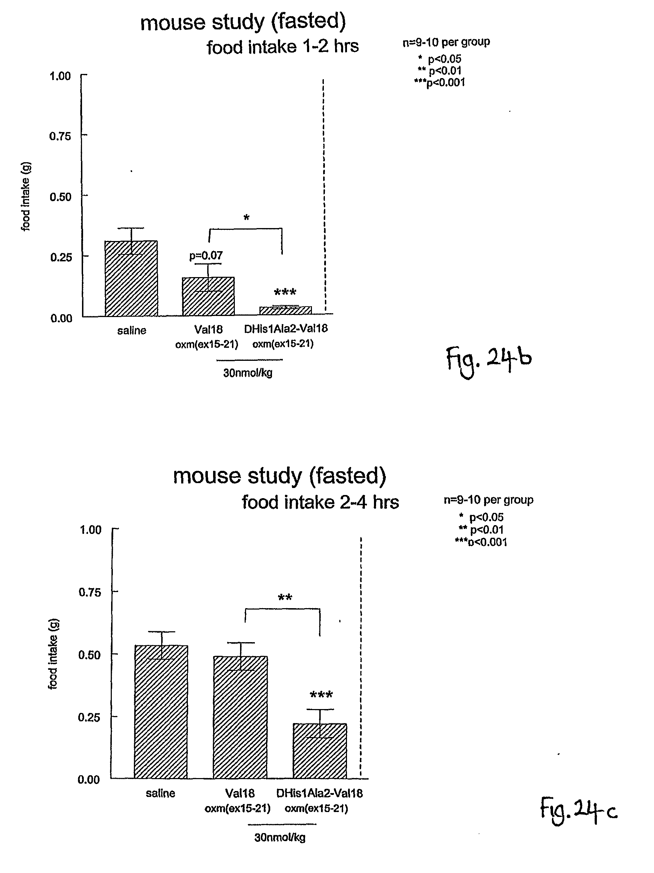

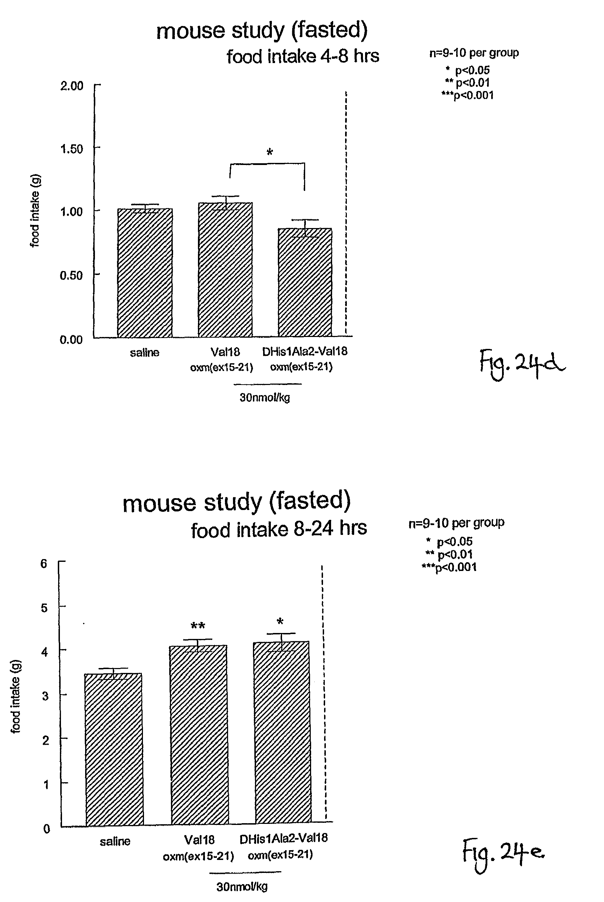

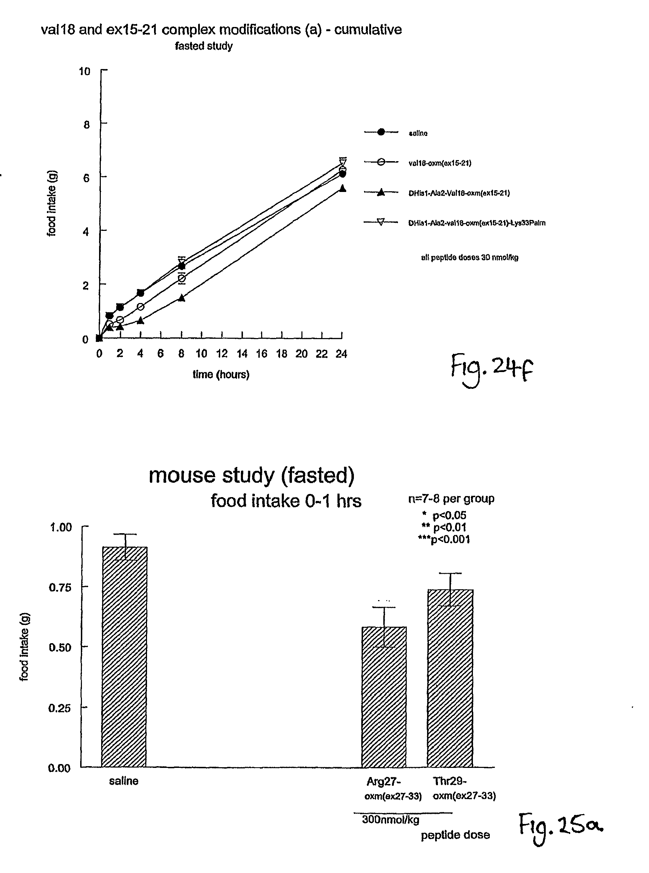

The measured food intake for each group is shown in Figs. 24a to 24e for, respectively, the intervals from 0 to 1 hour, from 1 to 2 hours, from 2 to 4, from 4 to 8 hours and from 8 to 24 hours after injection. Fig.24f shows cumulative data. A reduction in food intake from 0 to 2 hours is shown, with val18-oxm(ex15-21), and from 0 to 8 hours with D-His-Ala2-Val18-oxm(ex15-21).

### Example 17: oxm variants

Groups of fasted mice were each injected with one of the following:
Arg27-oxm(ex27-33) 
His Ser Gln Gly Thr Phe Thr Ser Asp Tyr Ser Lys Tyr Leu Asp Ser Arg Arg Ala Gln Asp Phe
Val Gln Trp Leu Arg Asn Gly Gly Pro Ser Ser Asn Asn Ile Ala (SEQ ID NO: 42)
Thr29-oxm(ex27-33)

His Ser Gln Gly Thr Phe Thr Ser Asp Tyr Ser Lys Tyr Leu Asp Ser Arg Arg Ala Gln Asp Phe
Val Gln Trp Leu Lys Asn Thr Gly Pro Ser Ser Asn Asn Ile Ala (SEQ ID NO: 43)

The compounds were administered by injection to groups of mice at a dose of 300 nmol/kg. A further group was administered saline (control).

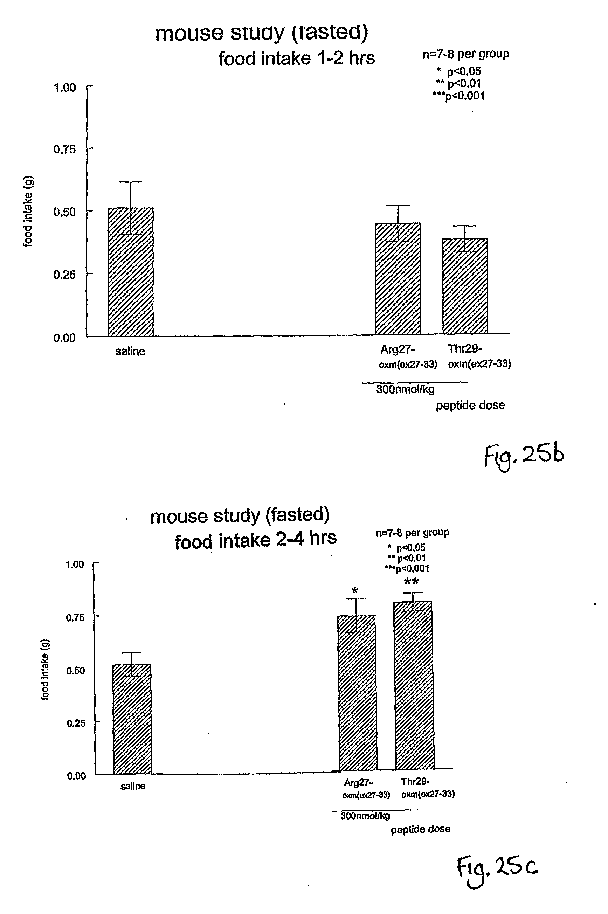

The measured food intake for each group is shown in Figs. 25a to 25c for, respectively, the intervals from 0 to 1 hour, from 1 to 2 hours, and from 2 to 4 hours after injection. A reduction in food intake from 0 to 1 hours is show with Arg27-oxm(ex27-33).

### Example 18: oxm C-terminal extensions

Groups of fasted mice were each injected with one of the following:
oxm-Ala38 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala Ala (SEQ ID NO: 44)
oxm-Ala38,39 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala Ala Ala (SEQ ID NO: 45)
oxm-Ala38-42 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala Ala Ala Ala Ala Ala (SEQ ID
NO: 46)
oxm-Lys33-Ala38

His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Lys Asn Asn Ile Ala Ala (SEQ ID NO: 47)

The compounds were administered by injection to groups of mice at a dose of 1400 nmol/kg. Further groups were administered native humain oxm (comparison) or saline (control).

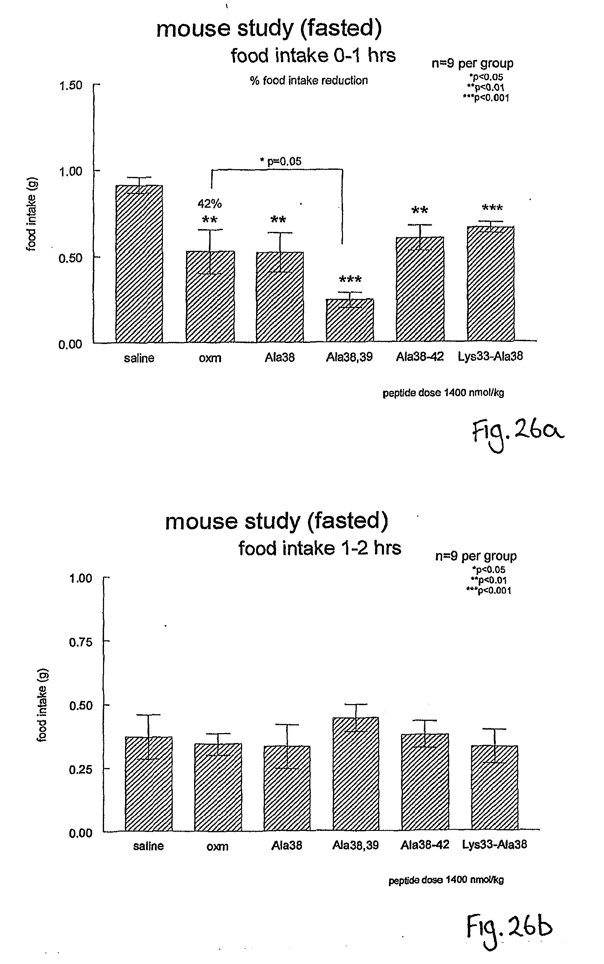

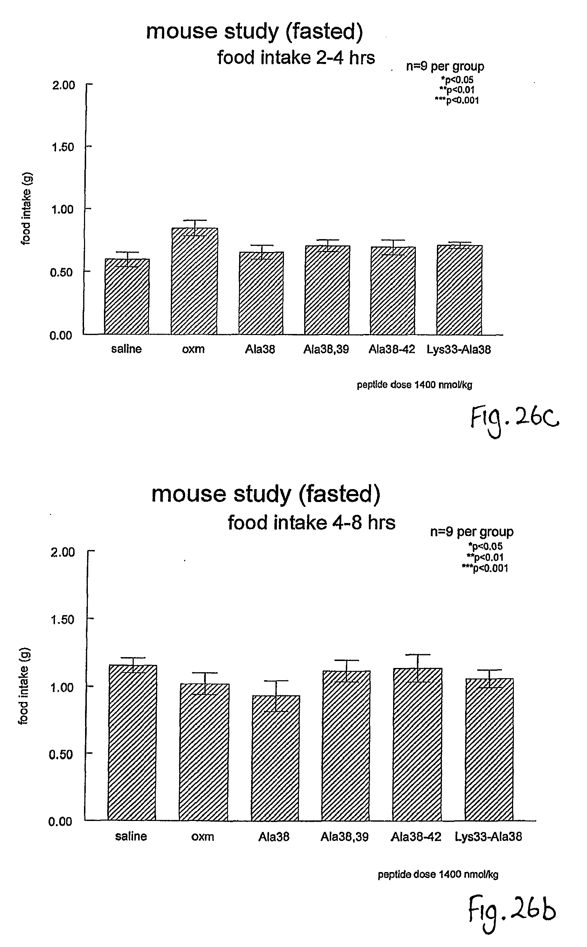

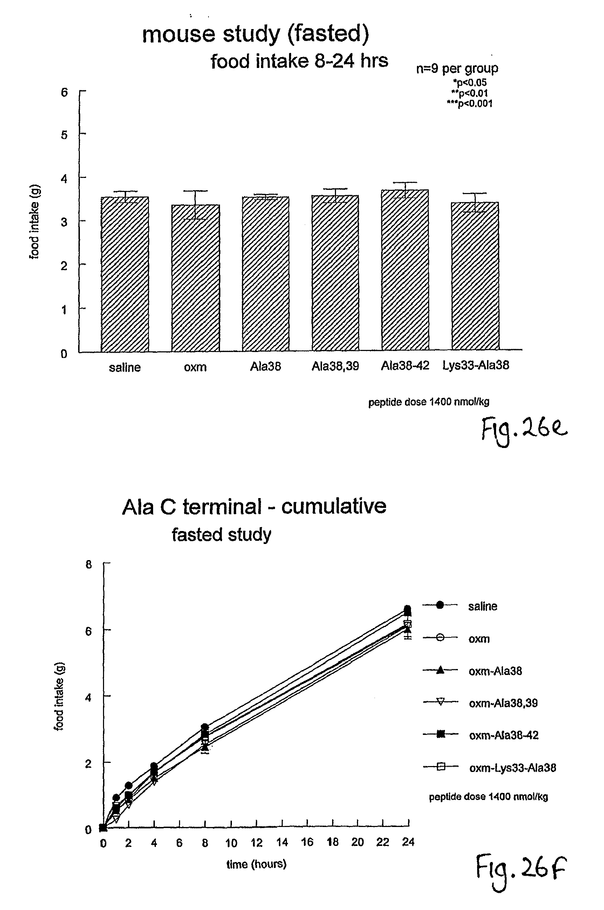

The measured food intake for each group is shown in Figs. 26a to 26e for, respectively, the intervals from 0 to 1 hour, from 1 to 2 hours, from 2 to 4, from 4 to 8, and from 8 to 24 hours after injection. Fig. 26f shows cumulative data. A reduction in food intake from 0 to 1 hours is show with all compounds. The Ala38,39 modification shows greater potency than native oxm.

### Example 19: Further oxm C-terminal extension

Groups of fasted miche were each injected with one of the following:
oxm-Ala38,39 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala Ala Ala (SEQ ID NO: 48)
oxm-Ala38,39,Lys40 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala Ala Ala Lys (SEQ ID NO: 49)
oxm-Ala38,39,Tyr40

His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala Ala Ala Tyr (SEQ ID NO: 50)

The compounds were administered by injection to groups of mice at a dose of 1400 nmol/kg. Further groups were administered native eel oxm (comparison) or saline (control).

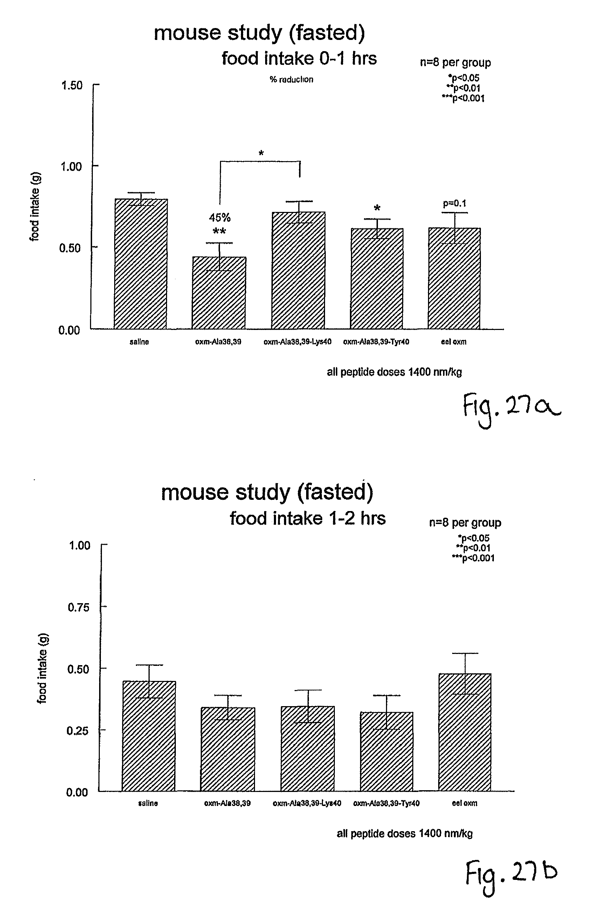

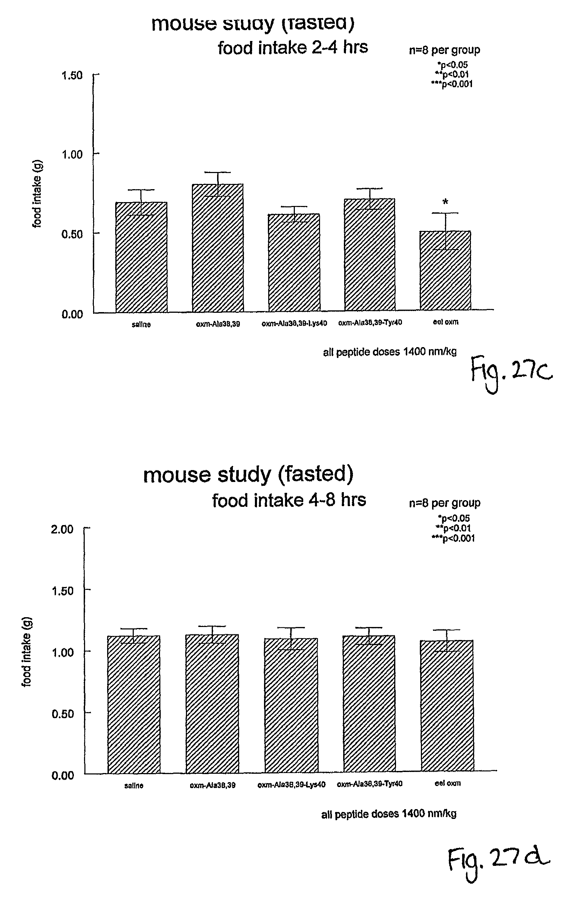

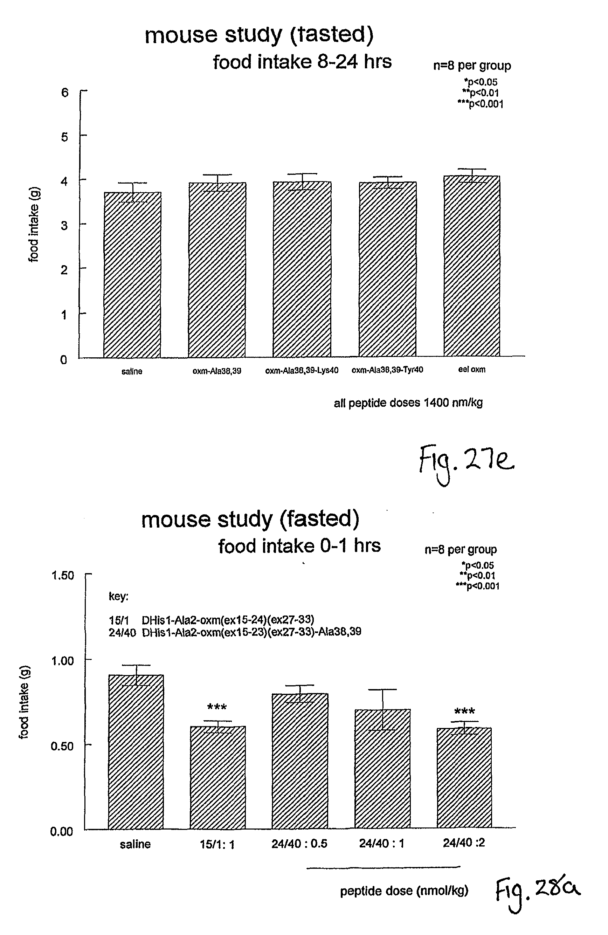

The measured food intake for each group is shown in Figs. 27a to 27e for, respectively, the intervals from 0 to 1 hour, from 1 to 2 hours, from 2 to 4, from 4 to 8, and from 8 to 24 hours after injection. The addition of terminal Lys40 and to a lesser extent Tyr40 appears to diminish the effect of oxm-Ala28,39 on food intake.

### Example 20: effect of Ala38,39 on substituted oxm

Groups of fasted mice were each injected with one of the following:
D-His1-Ala2-oxm(ex15-24)(ex27-33) 
D-His Ala Gln Gly Thr Phe Thr Ser Asp Tyr Ser Lys Tyr Leu Glu Glu Glu Ala Val Arg Leu Phe
Ile Glu Trp Leu Lys Asn Gly Gly Pro Ser Ser Asn Asn Ile Ala (SEQ ID NO :51)
D-His1-Ala2-oxm(ex15-23)(ex27-33)-Ala38,39

D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala (SEQ ID NO : 52)

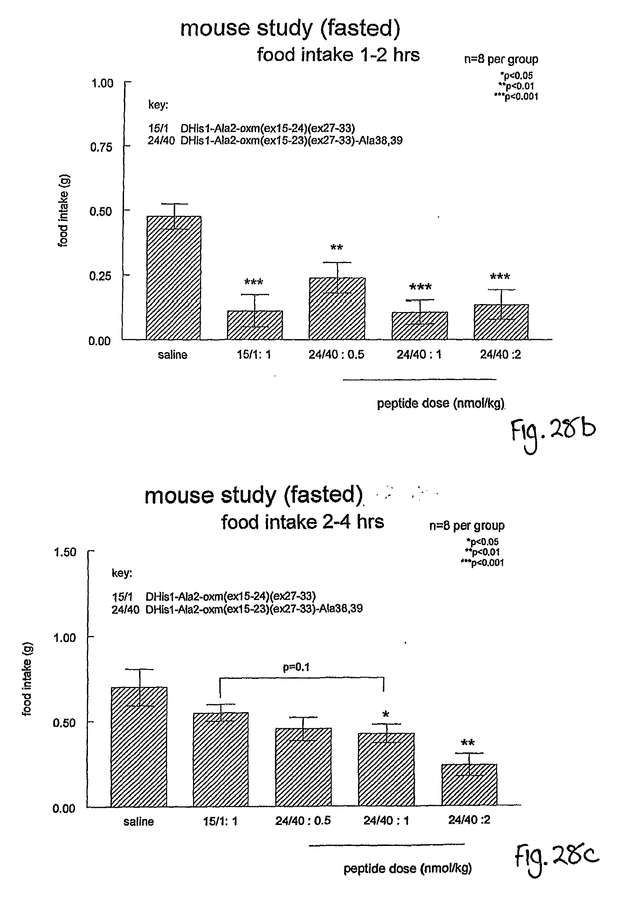

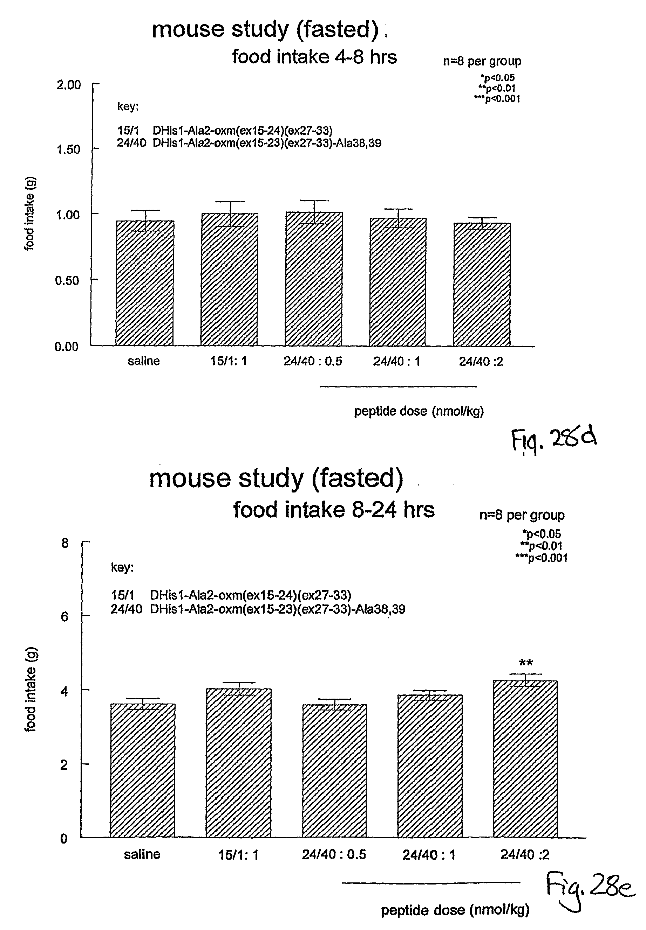

The compounds were administered by injection to groups of mice at doses of 0.5, 1 or 2 nmol/kg. Further groups were administered saline (control). The measured food intake for each group is shown in Figs. 28a to 28e for, respectively, the intervals from 0 to 1 hour, from 1 to 2 hours, from 2 to 4, from 4 to 8, and from 8 to 24 hours after injection.

The C-terminal extension appears to reduce the initial potency, but prolong the effect of the peptide.

### Example 21: Additional carboxyterminal extensions

Groups of fasted mice were each injected with one of the following:
oxm(ex15-23)(ex27-33)-Ala38,39 
His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala (SEQ ID NO: 53)
oxm(ex15-23)(ex27-33)-Ala38,39-Glu40,41-Lys42 
His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala Glu Glu Lys (SEQ ID NO :
54)
oxm(ex15-23)(ex27-33)-Ala38,39-Glu40,41-Lys42-palmitoyl

His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala Glu Glu Lys-palmitoyl (SEQ
ID NO : 55)

The compounds were administered by injection to groups of mice at doses of 100 or 400 nmol/kg. Further groups were administered exendin-4 (comparison) or saline (control).

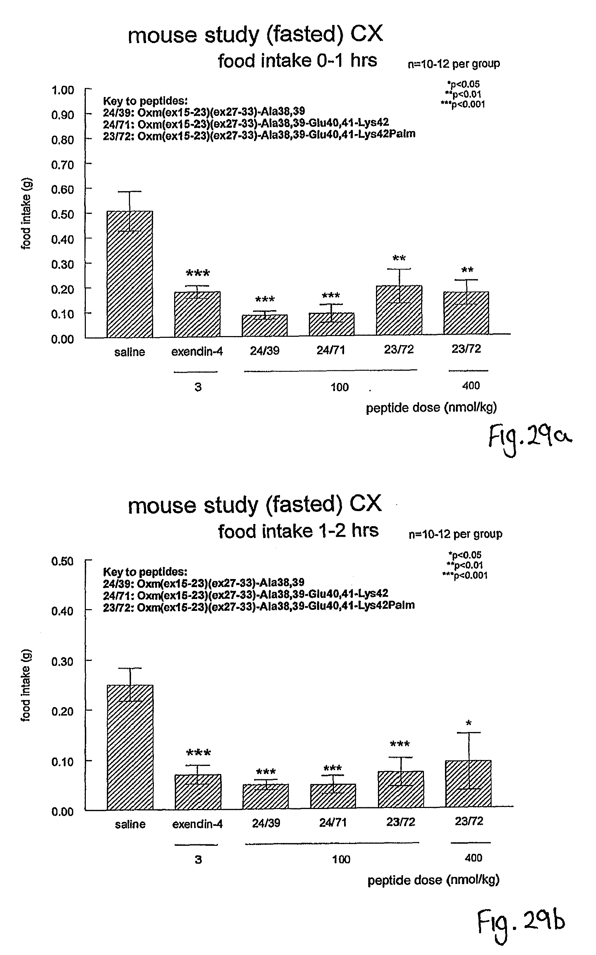

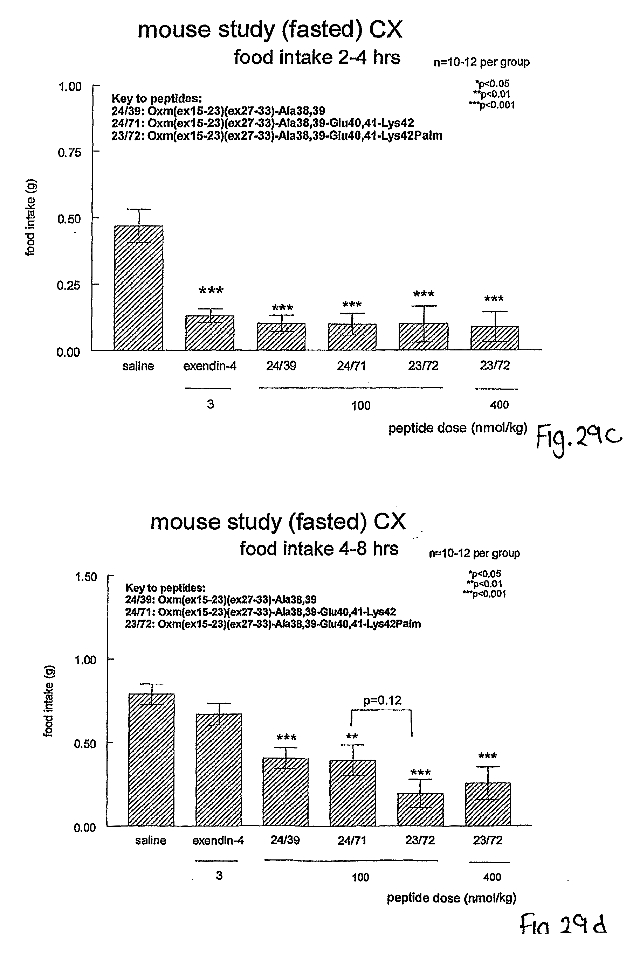

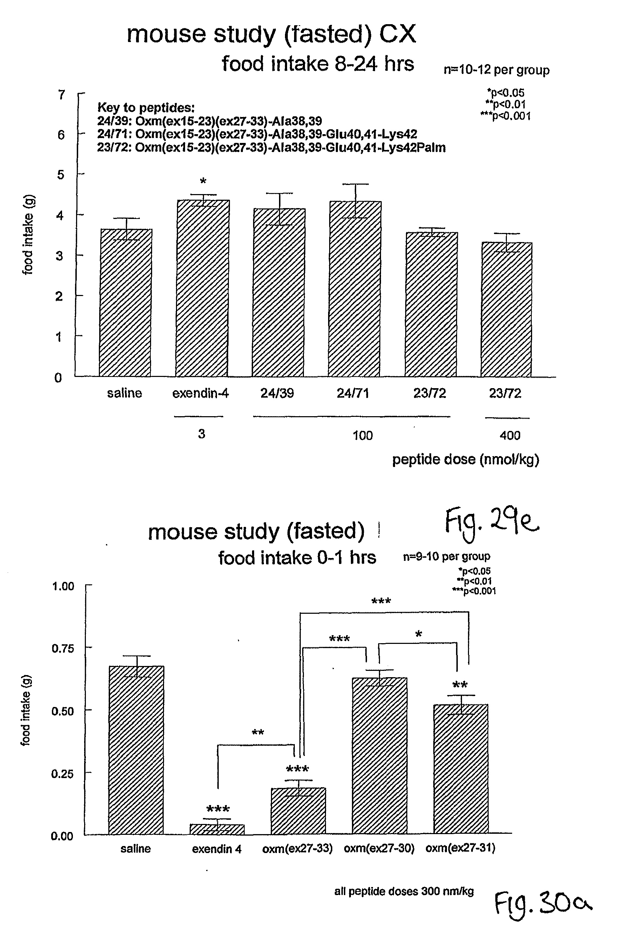

The measured food intake for each group is shown in Figs. 29a to 29e for, respectively, the intervals from 0 to 1 hour, from 1 to 2 hours, from 2 to 4, from 4 to 8, and from 8 to 24 hours after injection. All compounds resulted in reduced food intake over the first 8 hours.

### Example 22: oxm variants

Groups of fasted mice were each injected with one of the following:
oxm(ex27-33) 
His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Ser Ser Asn Asn Ile Ala (SEQ ID NO: 56)
oxm(ex27-30) 
His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Arg Asn Arg Asn Asn Ile Ala (SEQ ID NO: 57)
oxm(ex27-31)

His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Asn Arg Asn Asn Ile Ala (SEQ ID NO: 58)

The compounds were administered by injection to groups of mice at a dose of 300 nmol/kg. Further groups were administered exendin-4 (comparison) or saline (control).

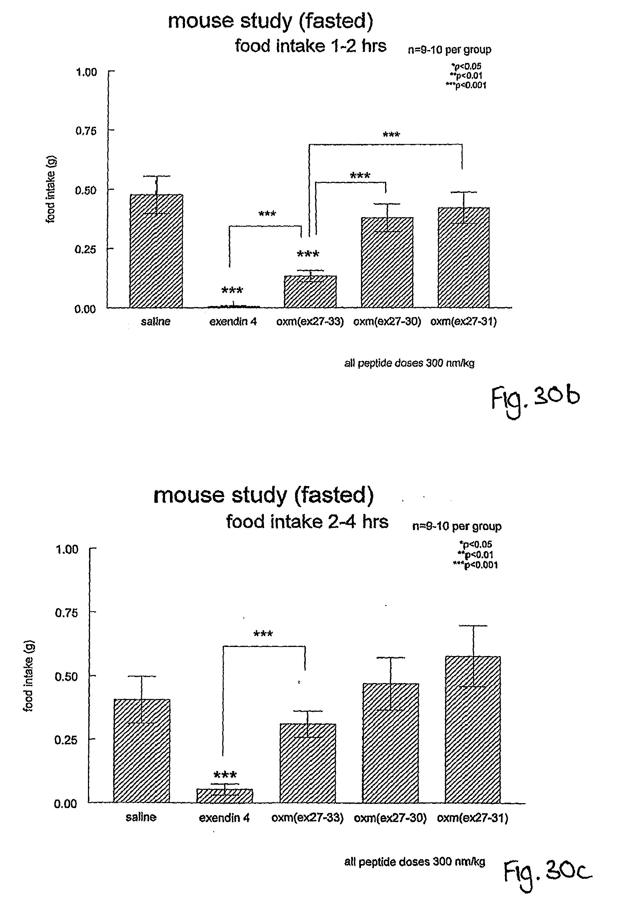

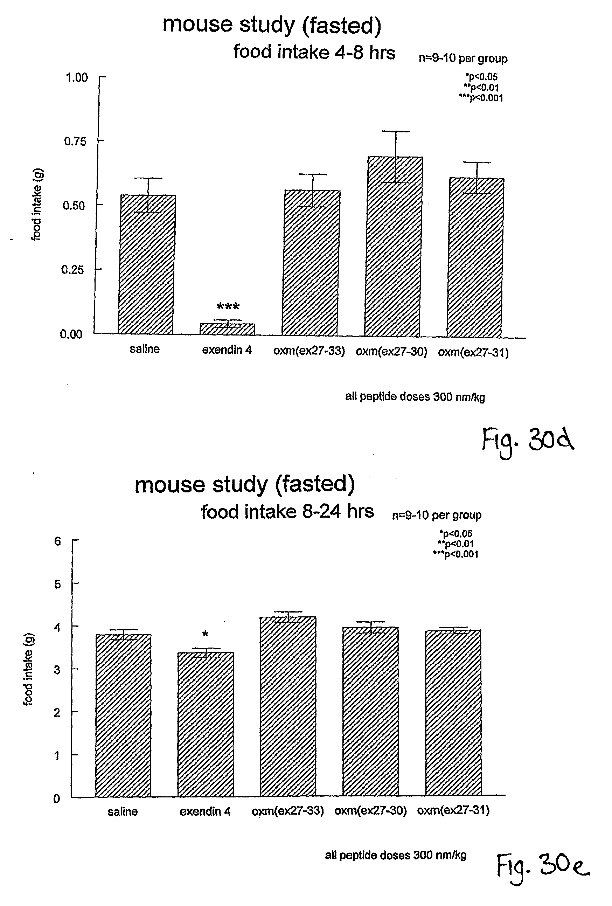

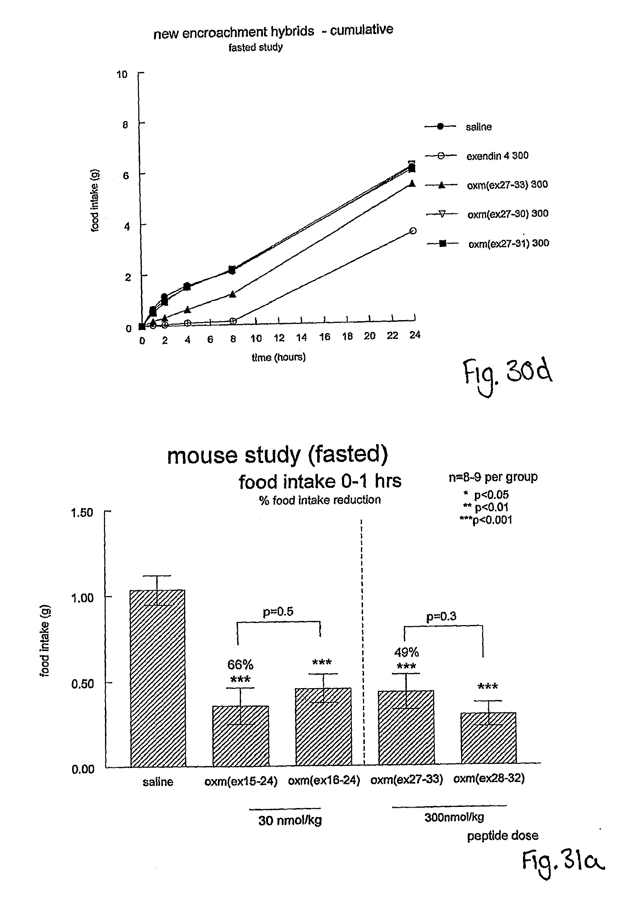

The measured food intake for each group is shown in Figs. 30a to 30e for, respectively, the intervals from 0 to 1 hour, from 1 to 2 hours, from 2 to 4, from 4 to 8, and from 8 to 24 hours after injection. Fig.30f shows cumulative data. All compounds resulted in reduced food intake.

### Example 23: oxm variants

Groups of fasted mice were each injected with one of the following:
oxm(ex15-24) 
His Ser Gln Gly Thr Phe Thr Ser Asp Tyr Ser Lys Tyr Leu Glu Glu Glu Ala Val Arg Leu Phe Ile
Glu Trp Leu Met Asn Thr Lys Arg Asn Arg Asn Asn Ile Ala (SEQ ID NO: 59)
oxm(ex16-24) 
His Ser Gln Gly Thr Phe Thr Ser Asp Tyr Ser Lys Tyr Leu Asp Glu Glu Ala Val Arg Leu Phe Ile
Glu Trp Leu Met Asn Thr Lys Arg Asn Arg Asn Asn Ile Ala (SEQ ID NO: 60)
oxm(ex27-33) 
His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Ser Ser Asn Asn Ile Ala (SEQ ID NO: 61)
oxm(ex28-32)

His Ser Gln Gly Thr Phe Thr Ser Asp Tyr Ser Lys Tyr Leu Asp Ser Arg Arg Ala Gln Asp Phe
Val Gln Trp Leu Met Asn Gly Gly Pro Ser Arg Asn Asn Ile Ala (SEQ ID NO: 62)

The compounds were administered by injection to groups of mice at doses of 30 or 300 nmol/kg. A further group was administered saline (control).

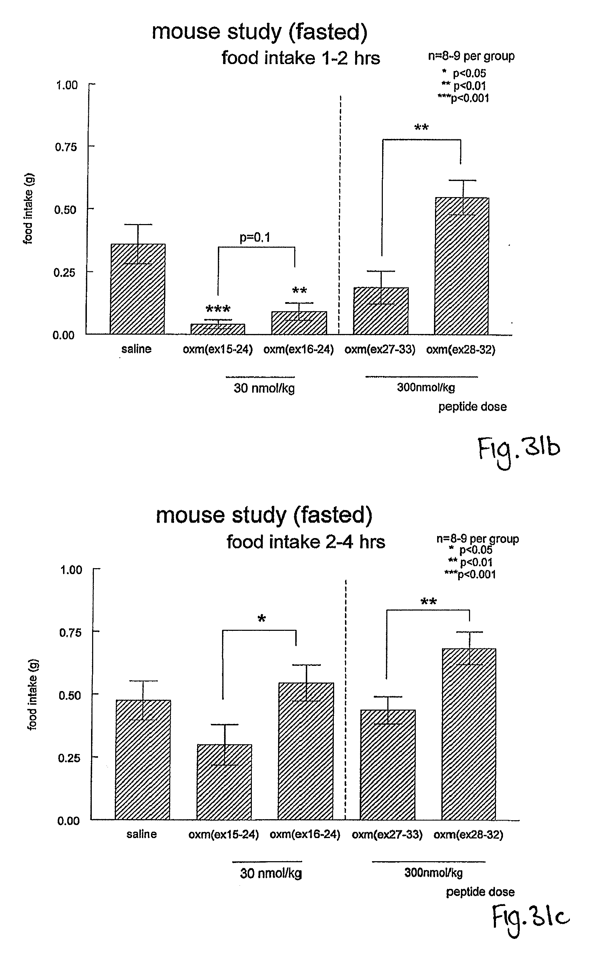

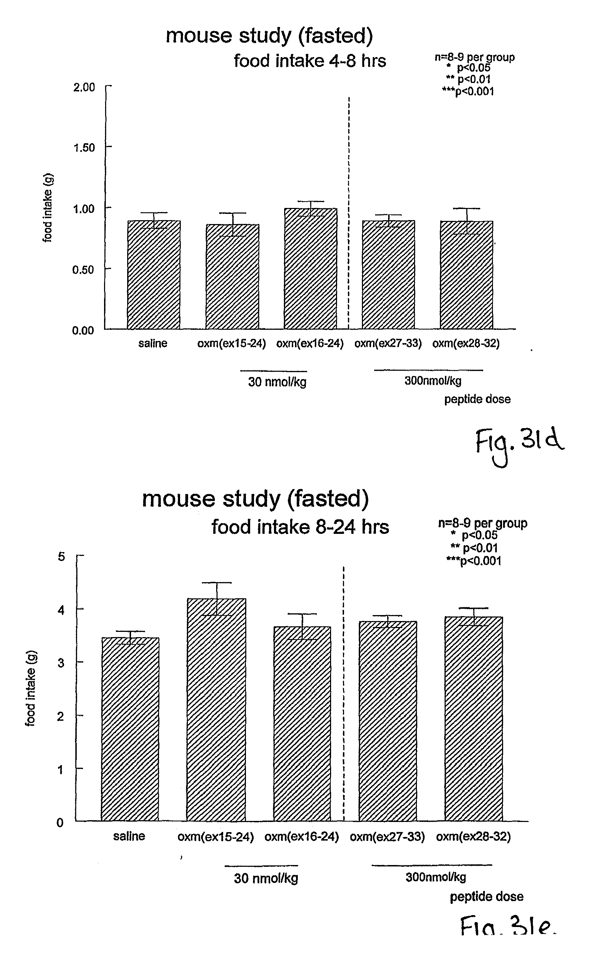

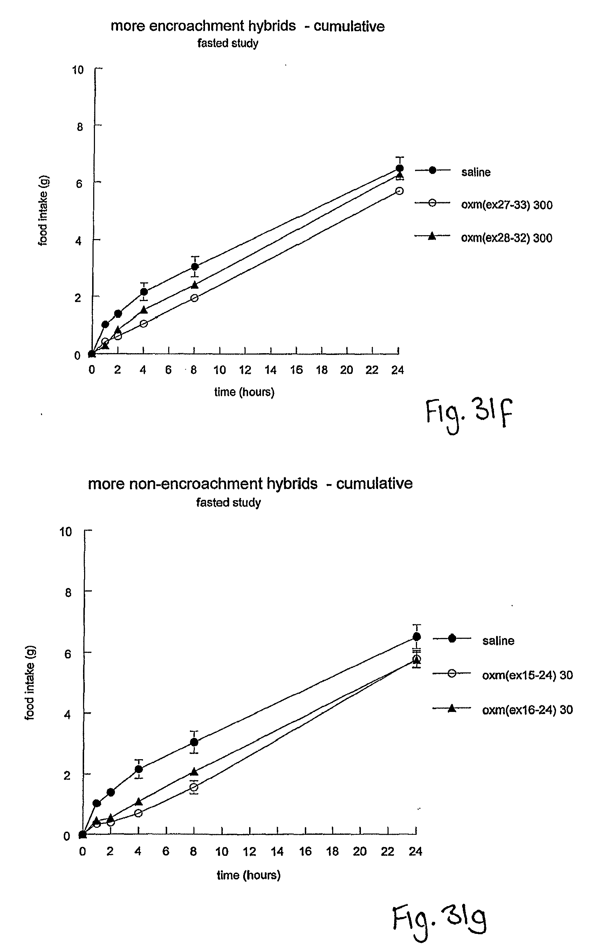

The measured food intake for each group is shown in Figs. 31a to 31e for, respectively, the intervals from 0 to 1 hour, from 1 to 2 hours, from 2 to 4, from 4 to 8, and from 8 to 24 hours after injection. Figs. 31f and 31g show cumulative data. All compounds resulted in reduced cumulative food intake at at least some time points.

### Example 24: oxm variants

Groups of fasted mice were each injected with one of the following:
oxm(ex27-33) 
His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Ser Ser Asn Asn Ile Ala (SEQ ID NO: 61)
oxm(ex29-33)

His Ser Gln Gly Thr Phe Thr Ser Asp Tyr Ser Lys Tyr Leu Asp Ser Arg Arg Ala Gln Asp Phe
Val Gln Trp Leu Met Asn Gly Gly Pro Ser Ser Asn Asn Ile Ala (SEQ ID NO: 62)

The compounds were administered by injection to groups of mice at a dose of 300 nmol/kg. A further group was administered saline (control).

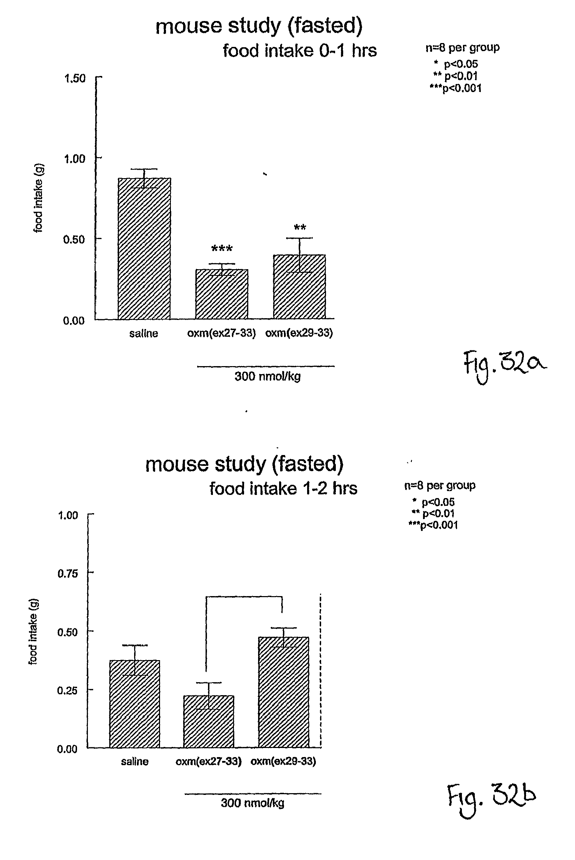

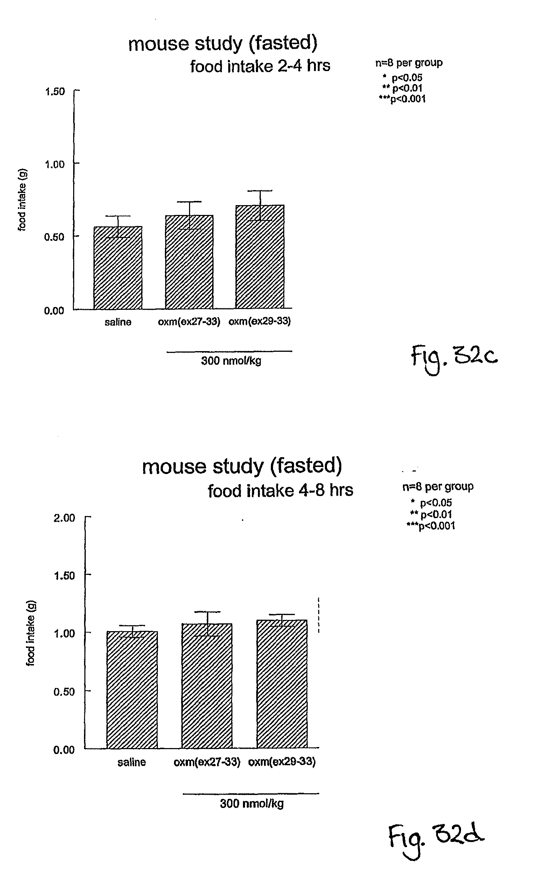

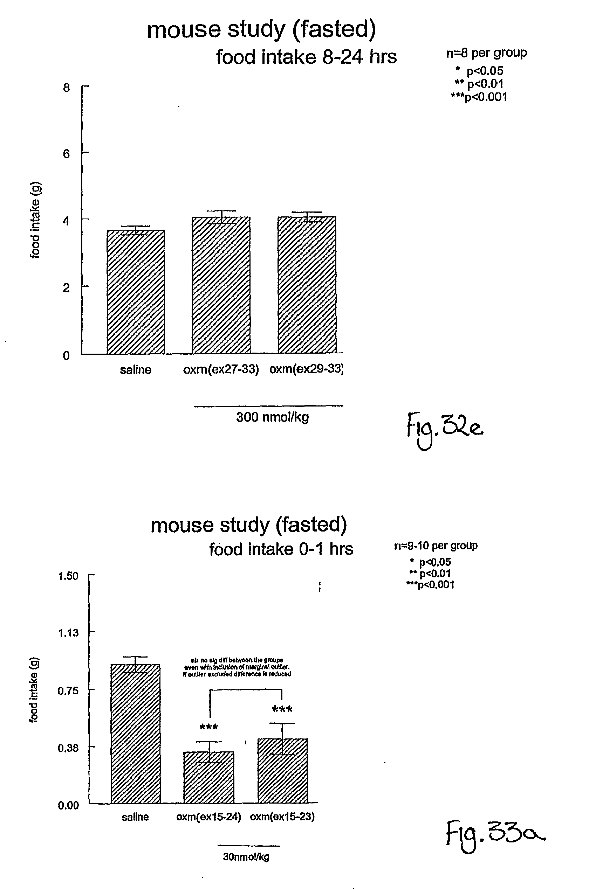

The measured food intake for each group is shown in Figs. 32a to 32e for, respectively, the intervals from 0 to 1 hour, from 1 to 2 hours, from 2 to 4, from 4 to 8, and from 8 to 24 hours after injection. All compounds resulted in reduced food intake at at least some time points.

### Example 25: oxm variants

Groups of fasted mice were each injected with one of the following:
oxm(ex15-23) 
His Ser Gln Gly Thr Phe Thr Ser Asp Tyr Ser Lys Tyr Leu Glu Glu Glu Ala Val Arg Leu Phe Ile
Gln Trp Leu Met Asn Thr Lys Arg Asn Arg Asn Asn Ile Ala (SEQ ID NO: 63)
oxm(ex15-24)

His Ser Gln Gly Thr Phe Thr Ser Asp Tyr Ser Lys Tyr Leu Glu Glu Glu Ala Val Arg Leu Phe Ile
Glu Trp Leu Met Asn Thr Lys Arg Asn Arg Asn Asn Ile Ala (SEQ ID NO: 59)

The compounds were administered by injection to groups of mice at a dose of 30 nmol/kg. A further group was administered saline (control).

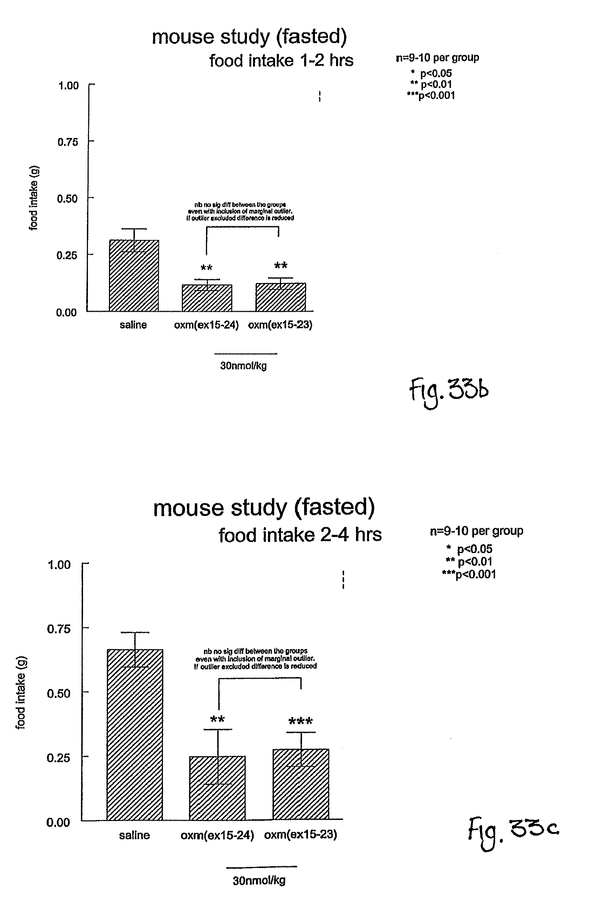

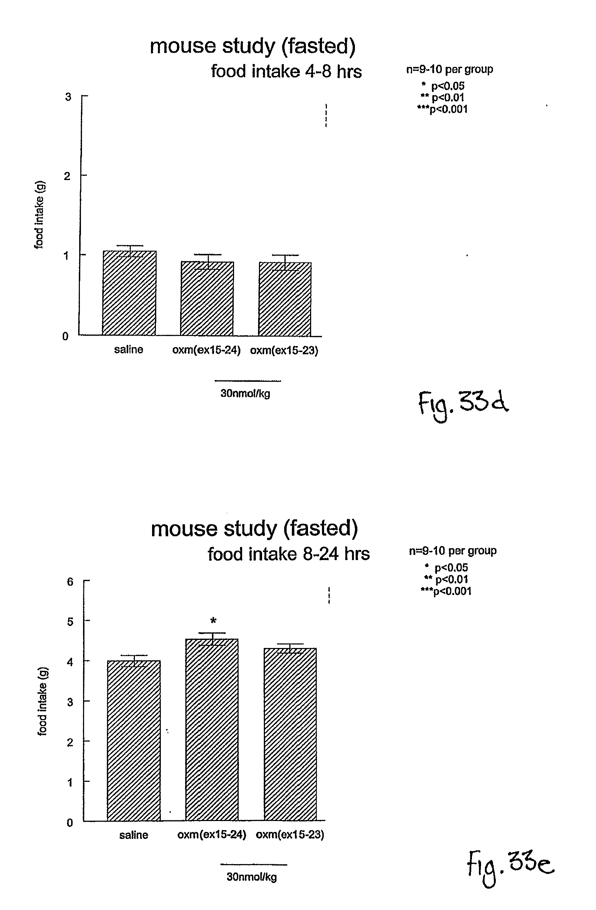

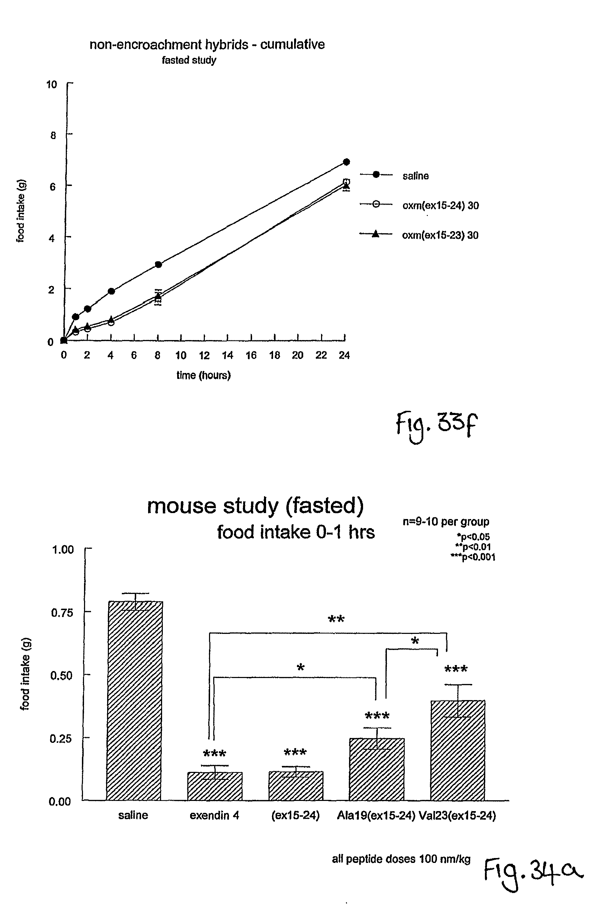

The measured food intake for each group is shown in Figs. 33a to 33e for, respectively, the intervals from 0 to 1 hour, from 1 to 2 hours, from 2 to 4, from 4 to 8, and from 8 to 24 hours after injection. Fig. 33f shows cumulative data. Both compounds reduced food intake for up to 4 hours (and cumulatively) by an approximately equal amount.

### Example 26: oxm(ex15-24) with single amino acid modifications

Groups of fasted mice were each injected with one of the following:
oxm(ex15-24) 
His Ser Gln Gly Thr Phe Thr Ser Asp Tyr Ser Lys Tyr Leu Glu Glu Glu Ala Val Arg Leu Phe Ile
Glu Trp Leu Met Asn Thr Lys Arg Asn Arg Asn Asn Ile Ala (SEQ ID NO: 59)
Ala19-oxm(ex15-24) 
His Ser Gln Gly Thr Phe Thr Ser Asp Tyr Ser Lys Tyr Leu Glu Glu Glu Ala Ala Arg Leu Phe Ile
Glu Trp Leu Met Asn Thr Lys Arg Asn Arg Asn Asn Ile Ala (SEQ ID NO: 64)
Val23-oxm(ex15-24)

His Ser Gln Gly Thr Phe Thr Ser Asp Tyr Ser Lys Tyr Leu Glu Glu Glu Ala Val Arg Leu Phe Val
Glu Trp Leu Met Asn Thr Lys Arg Asn Arg Asn Asn Ile Ala (SEQ ID NO: 68)

The compounds were administered by injection to groups of mice at a dose of 100 nmol/kg. Further groups were administered exendin 4 (comparison) or saline (control). The Alal9 and Val23 modifications result in a sequence that is closer to the oxm sequence prior to modification.

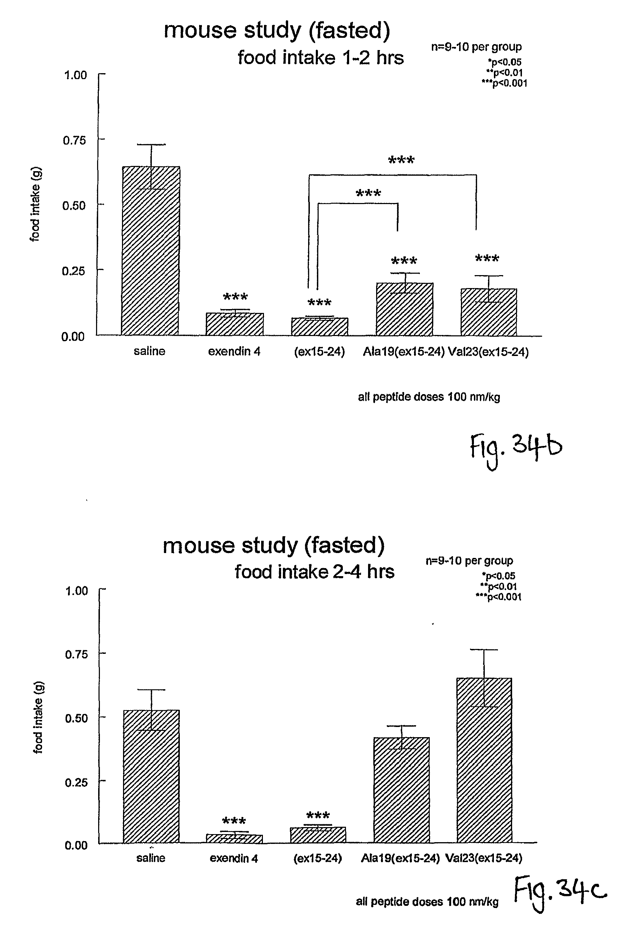

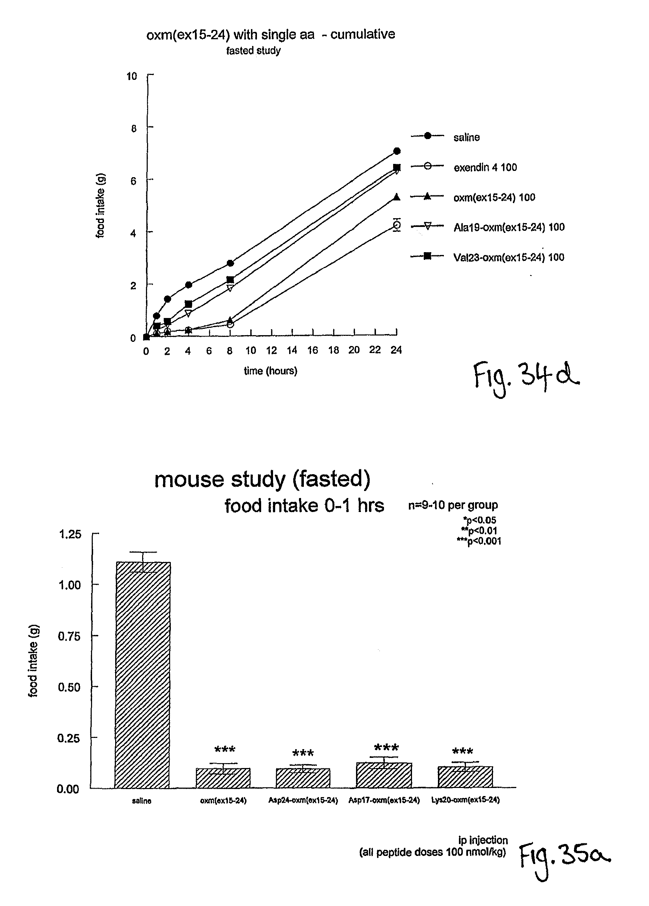

The measured food intake for each group is shown in Figs. 34a to 34c for, respectively, the intervals from 0 to 1 hour, from 1 to 2 hours and from 2 to 4 hours after injection. Fig. 34d shows cumulative data. All compounds reduced food intake at at least some time points. The single amino acid modifications resulted in a reduction in activity.

### Example 27: oxm(ex15-24) with single amino acid modifications

Groups of fasted mice were each injected with one of the following:
oxm(ex15-24) 
His Ser Gln Gly Thr Phe Thr Ser Asp Tyr Ser Lys Tyr Leu Glu Glu Glu Ala Val Arg Leu Phe Ile
Glu Trp Leu Met Asn Thr Lys Arg Asn Arg Asn Asn Ile Ala (SEQ ID NO: 59)
Asp24-oxm(ex15-24) 
His Ser Gln Gly Thr Phe Thr Ser Asp Tyr Ser Lys Tyr Leu Glu Glu Glu Ala Val Arg Leu Phe Ile
Asp Trp Leu Met Asn Thr Lys Arg Asn Arg Asn Asn Ile Ala (SEQ ID NO: 65)
Asp17-oxm(ex15-24) 
His Ser Gln Gly Thr Phe Thr Ser Asp Tyr Ser Lys Tyr Leu Glu Glu Asp Ala Val Arg Leu Phe Ile
Glu Trp Leu Met Asn Thr Lys Arg Asn Arg Asn Asn Ile Ala (SEQ ID NO: 66)
Lys20-oxm(ex15-24)

His Ser Gln Gly Thr Phe Thr Ser Asp Tyr Ser Lys Tyr Leu Glu Glu Glu Ala Val Lys Leu Phe Ile
Glu Trp Leu Met Asn Thr Lys Arg Asn Arg Asn Asn Ile Ala (SEQ ID NO: 67)

The compounds were administered by injection to groups of mice at a dose of 100 nmol/kg. A further group was administered saline (control).

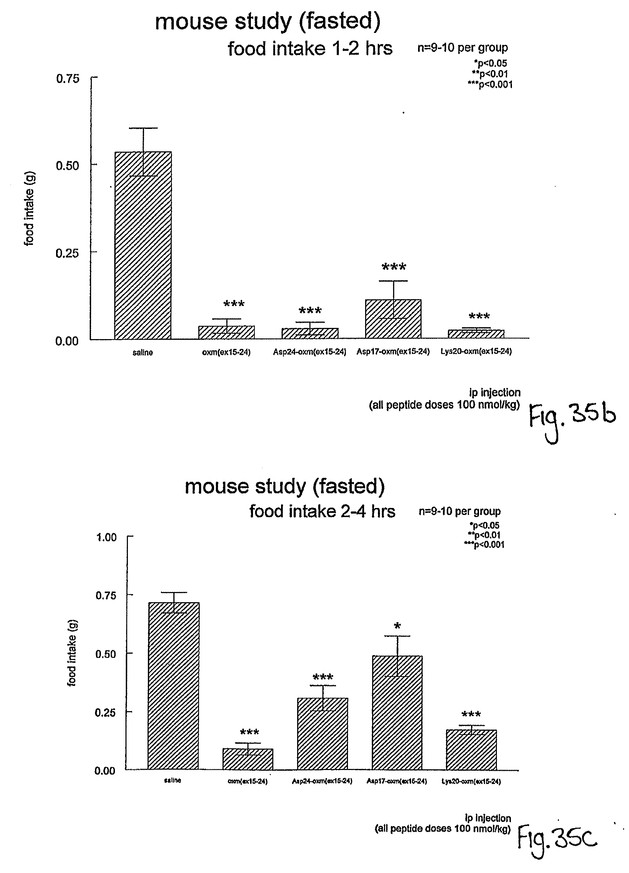

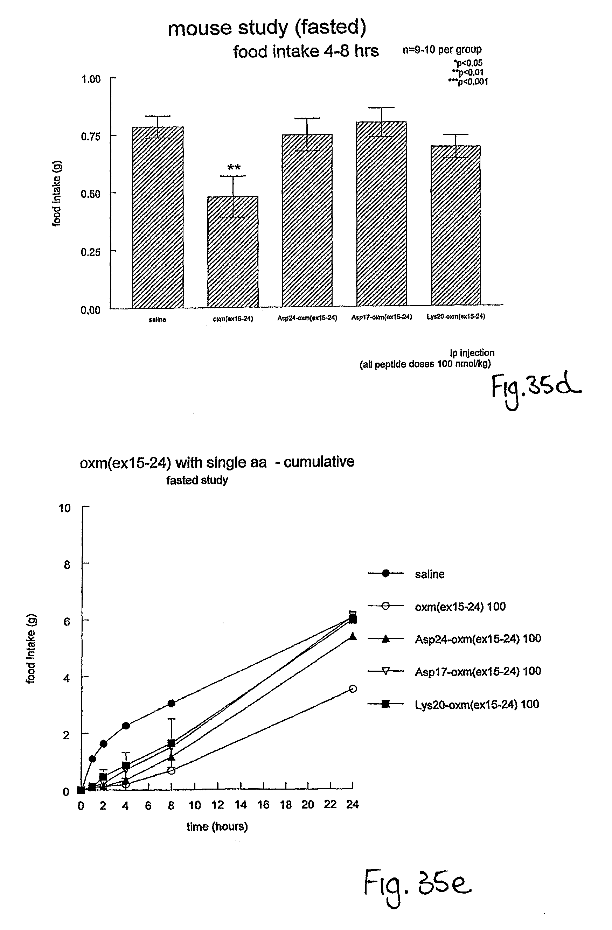

The measured food intake for each group is shown in Figs. 35a to 35d for, respectively, the intervals from 0 to 1 hour, from 1 to 2 hours, from 2 to 4 hours and from 4 to 8 hours after injection. Fig. 35e shows cumulative data. All compounds reduced food intake, but oxm(ex15-24) was the most effective.

### Example 28: oxm(ex15-24) with amino acid modifications

Groups of fasted mice were each injected with one of the following:
Val23-oxm(ex15-24) 
His Ser Gln Gly Thr Phe Thr Ser Asp Tyr Ser Lys Tyr Leu Glu Glu Glu Ala Val Arg Leu Phe Val
Glu Trp Leu Met Asn Thr Lys Arg Asn Arg Asn Asn Ile Ala (SEQ ID NO: 146)
Ala19,Val23-oxm(ex15-24)

His Ser Gln Gly Thr Phe Thr Ser Asp Tyr Ser Lys Tyr Leu Glu Glu Glu Ala Ala Arg Leu Phe Val
Glu Trp Leu Met Asn Thr Lys Arg Asn Arg Asn Asn Ile Ala (SEQ ID NO: 38)

The compounds were administered by injection to groups of mice at a dose of 100 nmol/kg. Further groups were administered native oxm (comparison) or saline (control).

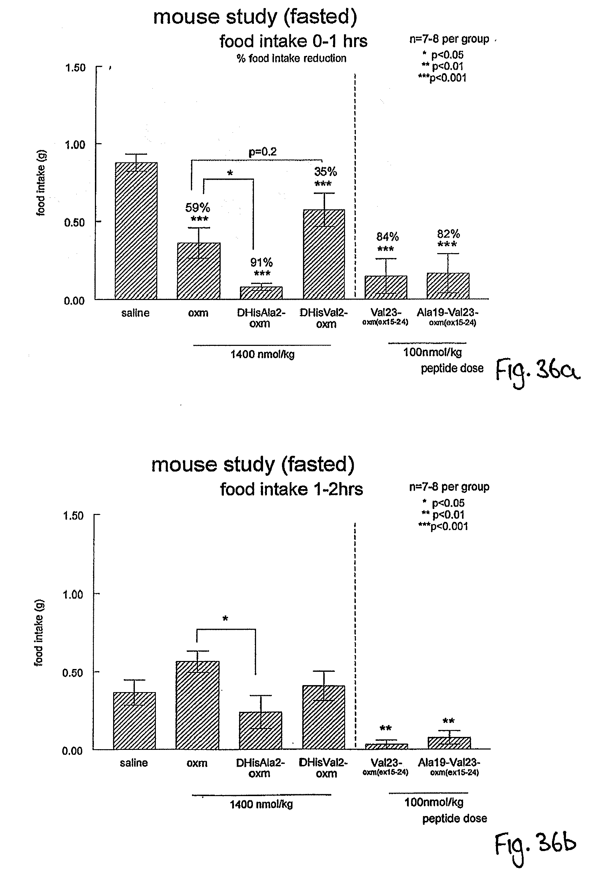

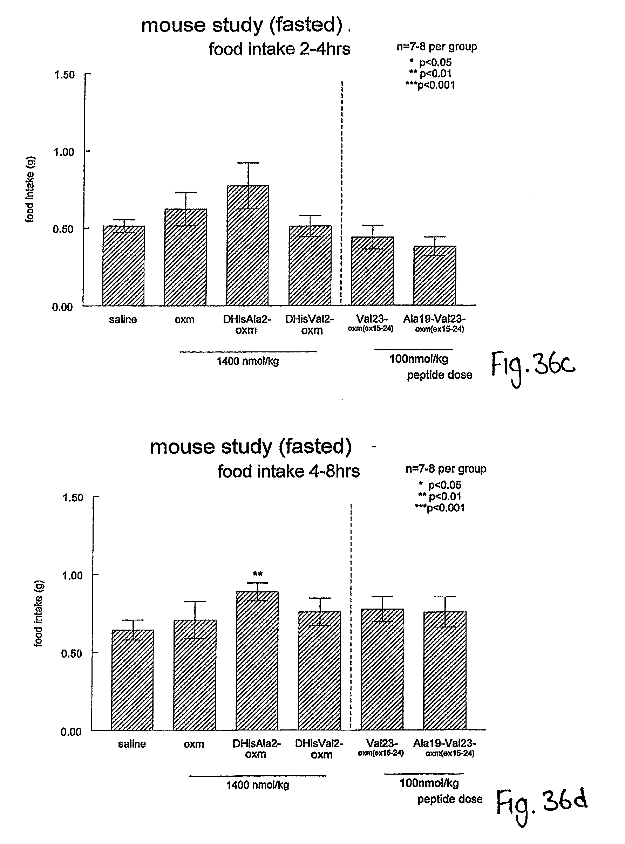

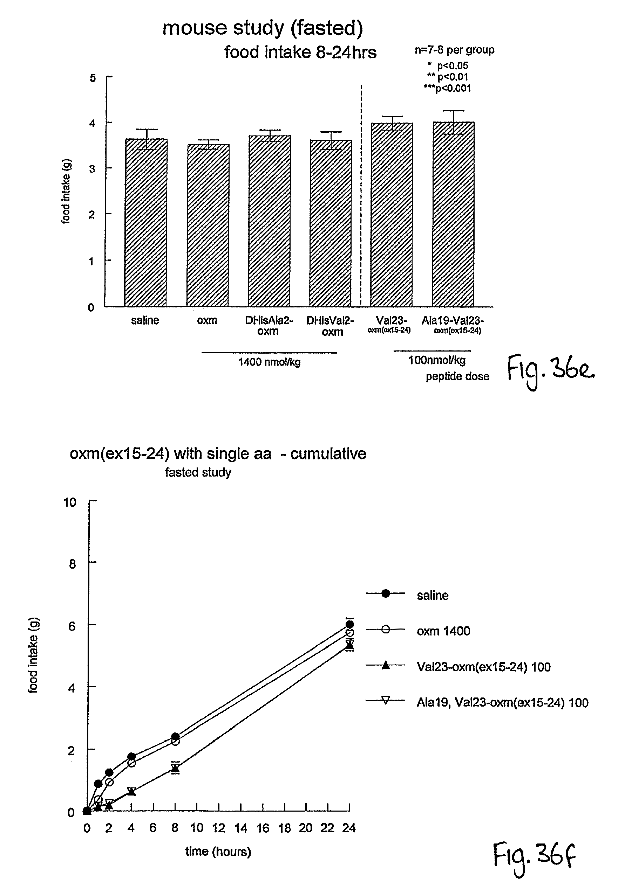

The measured food intake for each group is shown in Figs. 36a to 36e for, respectively, the intervals from 0 to 1 hour, from 1 to 2 hours, from 2 to 4 hours, from 4 to 8 hours, and from 8 to 24 hours after injection. Both compounds reduced cumulative food intake.

### Example 29: oxm(ex15-24) with amino acid modifications

Groups of fasted mice were each injected with one of the following:
oxm(ex15-24) 
His Ser Gln Gly Thr Phe Thr Ser Asp Tyr Ser Lys Tyr Leu Glu Glu Glu Ala Val Arg Leu Phe Ile
Glu Trp Leu Met Asn Thr Lys Arg Asn Arg Asn Asn Ile Ala (SEQ ID NO: 63)
Val18-oxm(ex15-24) 
His Ser Gln Gly Thr Phe Thr Ser Asp Tyr Ser Lys Tyr Leu Glu Glu Glu Val Val Arg Leu Phe Ile
Glu Trp Leu Met Asn Thr Lys Arg Asn Arg Asn Asn Ile Ala (SEQ ID NO: 68)
Gln18-oxm(ex15-24) 
His Ser Gln Gly Thr Phe Thr Ser Asp Tyr Ser Lys Tyr Leu Glu Glu Glu Gln Val Arg Leu Phe Ile
Glu Trp Leu Met Asn Thr Lys Arg Asn Arg Asn Asn Ile Ala (SEQ ID NO: 69)
Ile19-oxm(ex15-24)

His Ser Gln Gly Thr Phe Thr Ser Asp Tyr Ser Lys Tyr Leu Glu Glu Glu Ala Ile Arg Leu Phe Ile
Glu Trp Leu Met Asn Thr Lys Arg Asn Arg Asn Asn Ile Ala (SEQ ID NO: 70)

The compounds were administered by injection to groups of mice at a dose of 30 nmol/kg. A further group was administered saline (control).

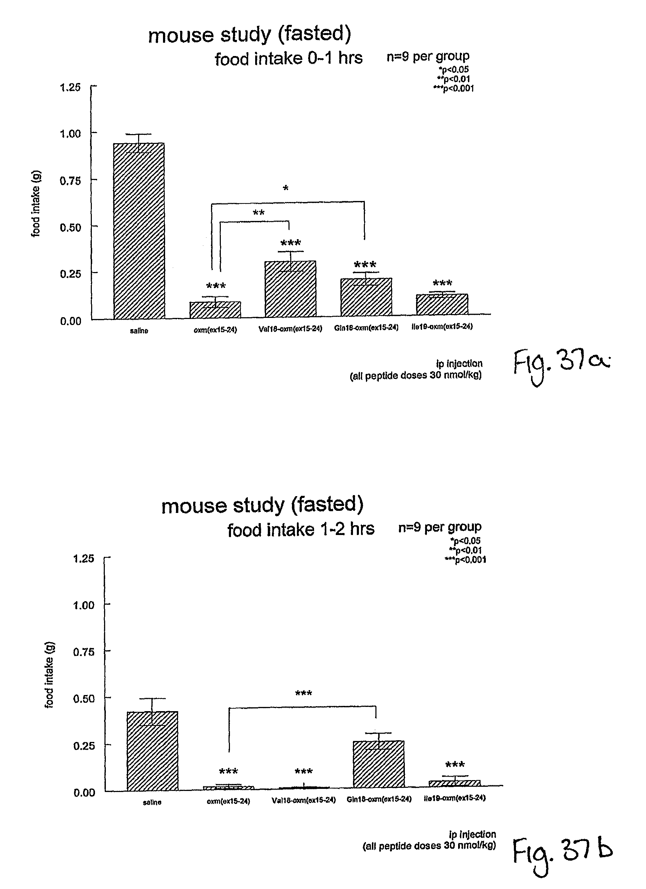

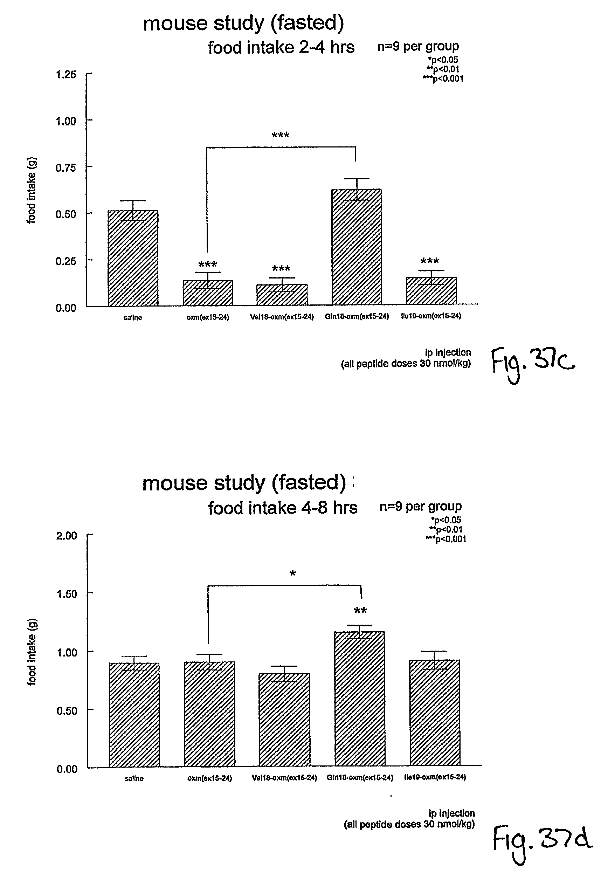

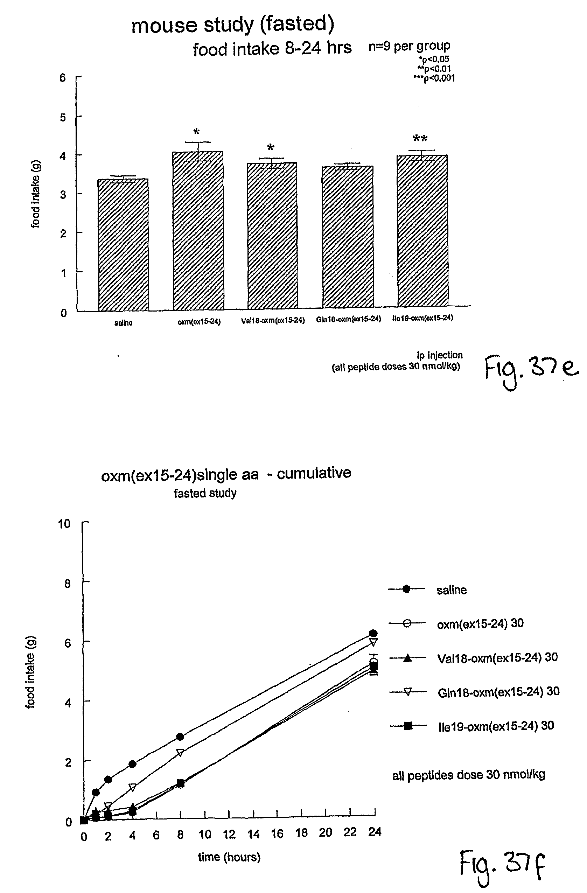

The measured food intake for each group is shown in Figs. 37a to 37e for, respectively, the intervals from 0 to 1 hour, from 1 to 2 hours, from 2 to 4 hours, from 4 to 8 hours, and from 8 to 24 hours after injection. Fig. 37f shows cumulative data. All compounds reduced cumulative food intake.

### Example 30: oxm(ex27-33) with amino acid modifications

Groups of fasted mice were each injected with one of the following:
Oxm 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala (SEQ ID NO: 7)
Ala38-Ala39-oxm 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala Ala Ala (SEQ ID NO: 45)
oxm(ex27-33) 
His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Ser Ser Asn Asn Ile Ala (SEQ ID NO: 56)
Arg27-oxm(ex27-33) 
His Ser Gln Gly Thr Phe Thr Ser Asp Tyr Ser Lys Tyr Leu Asp Ser Arg Arg Ala Gln Asp Phe
Val Gln Trp Leu Arg Asn Gly Gly Pro Ser Ser Asn Asn Ile Ala (SEQ ID NO: 147)
Thr29-oxm(ex27-33)

His Ser Gln Gly Thr Phe Thr Ser Asp Tyr Ser Lys Tyr Leu Asp Ser Arg Arg Ala Gln Asp Phe
Val Gln Trp Leu Lys Asn Thr Gly Pro Ser Ser Asn Asn Ile Ala (SEQ ID NO: 148)

The compounds were administered by injection to groups of mice at doses of 1400 or 300 nmol/kg. A further group was administered saline (control).

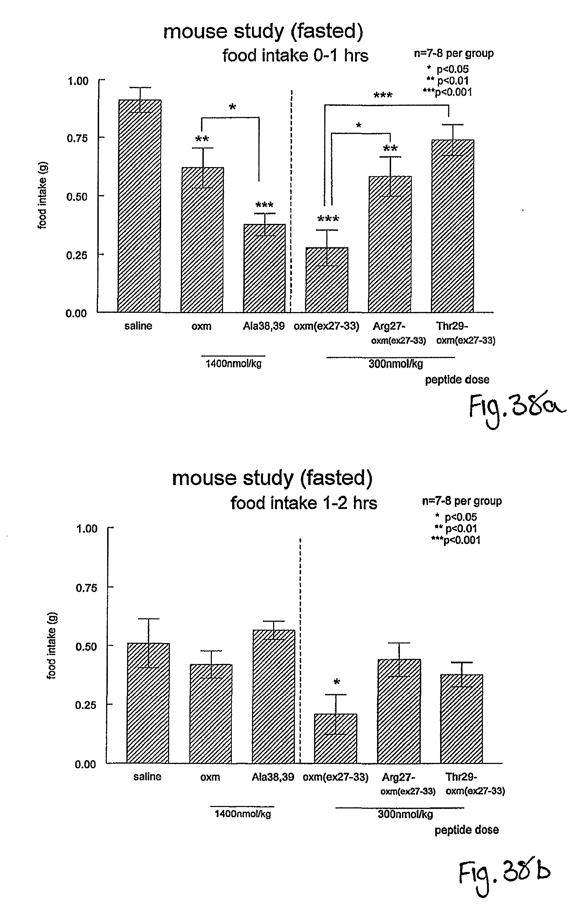

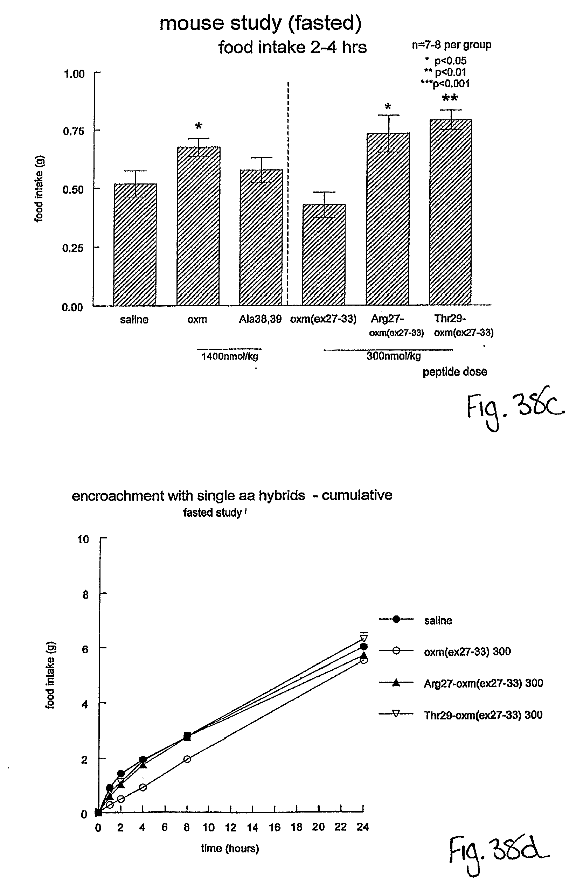

The measured food intake for each group is shown in Figs. 38a to 38c for, respectively, the intervals from 0 to 1 hour, from 1 to 2 hours and from 2 to 4 hours after injection. Fig. 38d shows cumulative data. The Arg27 and thr29 single amino acid changes reduce the effect of oxm(ex27-33) on food intake in a similar way to single amino acid substitutions in oxm(ex15-24)

### Example 31: oxm(ex27-33) with amino acid modifications

Groups of fasted mice were each injected with one of the following:
oxm(ex27-33) 
His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Ser Ser Asn Asn Ile Ala (SEQ ID NO: 56)
Asn32-oxm(ex27-33)

His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Asn Ser Asn Asn Ile Ala (SEQ ID NO: 71)

The compounds were administered by injection to groups of mice at a dose of 300 nmol/kg. A further group was administered saline (control).

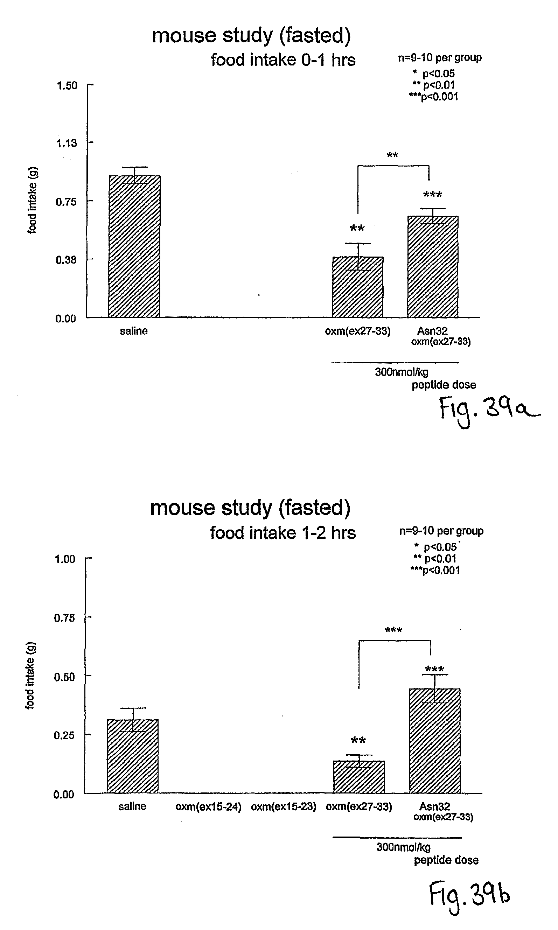

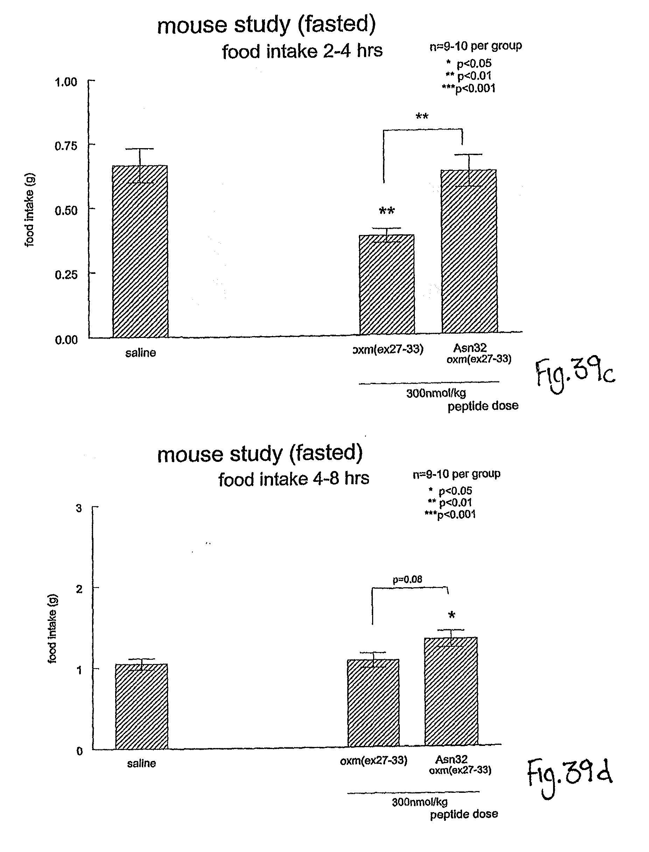

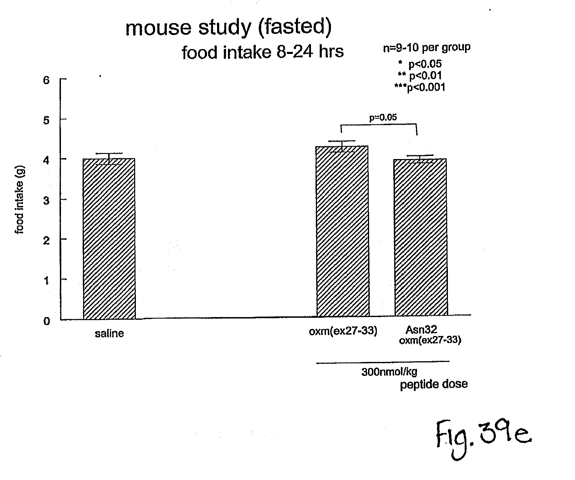

The measured food intake for each group is shown in Figs. 39a to 39e for, respectively, the intervals from 0 to 1 hour, from 1 to 2 hours, from 2 to 4 hours, from 4 to 8 hours and from 8 to 24 hours after injection. The Asn32 single amino acid change reduces the effect of oxm(ex27-33) on food intake.

### Example 32: oxm(ex15-21) with amino acid modifications

Groups of fasted mice were each injected with one of the following:
oxm(ex15-21) 
His Ser Gln Gly Thr Phe Thr Ser Asp Tyr Ser Lys Tyr Leu Glu Glu Glu Ala Val Arg Leu Phe Val
Gln Trp Leu Met Asn Thr Lys Arg Asn Arg Asn Asn Ile Ala (SEQ ID NO: 15)
Gln15-oxm(ex15-21) 
His Ser Gln Gly Thr Phe Thr Ser Asp Tyr Ser Lys Tyr Leu Gln Glu Glu Ala Val Arg Leu Phe Val
Gln Trp Leu Met Asn Thr Lys Arg Asn Arg Asn Asn Ile Ala (SEQ ID NO: 73)
Gln16-oxm(ex15-21) 
His Ser Gln Gly Thr Phe Thr Ser Asp Tyr Ser Lys Tyr Leu Glu Gln Glu Ala Val Arg Leu Phe Val
Gln Trp Leu Met Asn Thr Lys Arg Asn Arg Asn Asn Ile Ala (SEQ ID NO: 74)
Val18-oxm(ex15-21) 
His Ser Gln Gly Thr Phe Thr Ser Asp Tyr Ser Lys Tyr Leu Glu Glu Glu Val Val Arg Leu Phe Val
Gln Trp Leu Met Asn Thr Lys Arg Asn Arg Asn Asn Ile Ala (SEQ ID NO: 40)
Val18,Ile19-oxm(ex15-21)

His Ser Gln Gly Thr Phe Thr Ser Asp Tyr Ser Lys Tyr Leu Glu Glu Glu Val Ile Arg Leu Phe Val
Gln Trp Leu Met Asn Thr Lys Arg Asn Arg Asn Asn Ile Ala (SEQ ID NO: 75)

The compounds were administered by injection to groups of mice at a dose of 30 nmol/kg. A further group was administered saline (control).

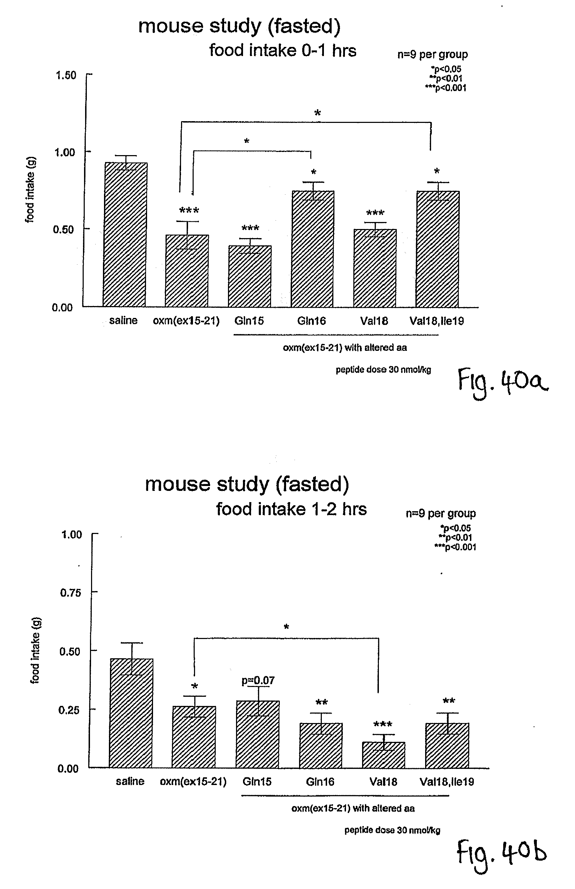

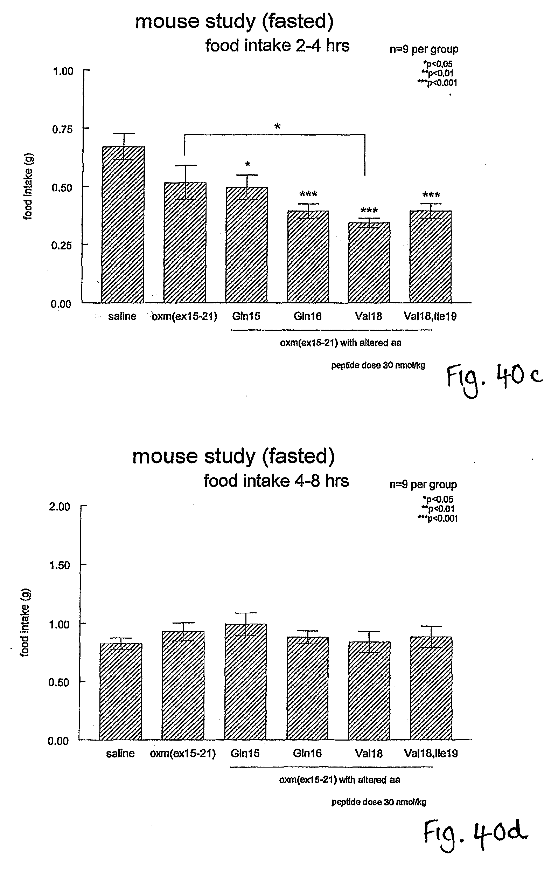

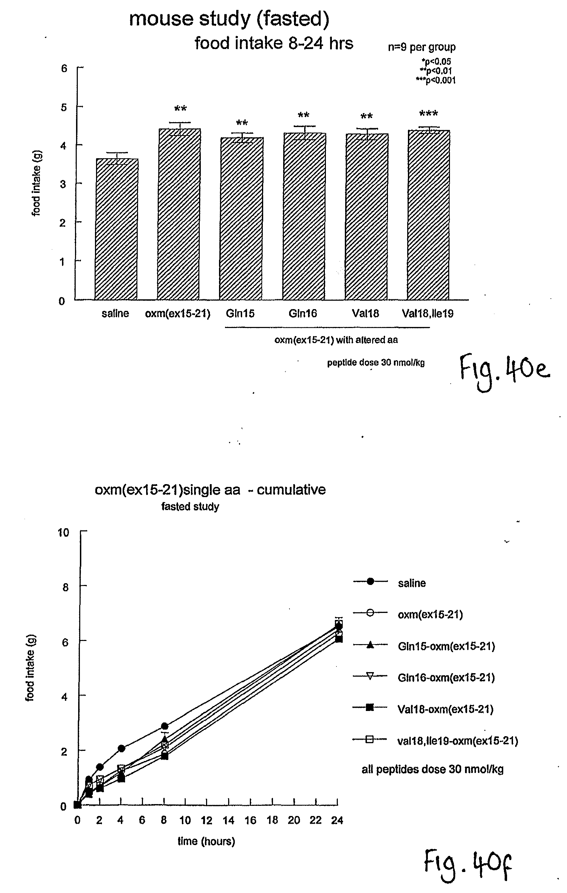

The measured food intake for each group is shown in Figs. 40a to 40e for, respectively, the intervals from 0 to 1 hour, from 1 to 2 hours, from 2 to 4 hours, from 4 to 8 hours and from 8 to 24 hours after injection. Fig.40f shows cumulative data. All compounds reduced cumulative food intake over 8 hours.

### Example 33: oxm(ex15-21) variants

Groups of fasted mice were each injected with one of the following:
oxm(ex15-21) 
His Ser Gln Gly Thr Phe Thr Ser Asp Tyr Ser Lys Tyr Leu Glu Glu Glu Ala Val Arg Leu Phe Val
Gln Trp Leu Met Asn Thr Lys Arg Asn Arg Asn Asn Ile Ala (SEQ ID NO: 15)
D-His1,Ala2-oxm(ex15-21) 
D-His Ala Gln Gly Thr Phe Thr Ser Asp Tyr Ser Lys Tyr Leu Glu Glu Glu Ala Val Arg Leu Phe
Val Gln Trp Leu Met Asn Thr Lys Arg Asn Arg Asn Asn Ile Ala (SEQ ID NO: 76)
D-His1,Ala2-oxm(ex15-21)-Lys33-Ala38,39 
D-His Ala Gln Gly Thr Phe Thr Ser Asp Tyr Ser Lys Tyr Leu Glu Glu Glu Ala Val Arg Leu Phe
Val Gln Trp Leu Met Asn Thr Lys Arg Asn Lys Asn Asn Ile Ala Ala Ala (SEQ ID NO: 77)
Val18-oxm(ex15-21) 
His Ser Gln Gly Thr Phe Thr Ser Asp Tyr Ser Lys Tyr Leu Glu Glu Glu Val Val Arg Leu Phe Val
Gln Trp Leu Met Asn Thr Lys Arg Asn Arg Asn Asn Ile Ala (SEQ ID NO: 40)
Val18,Ala19-oxm(ex15-21)

His Ser Gln Gly Thr Phe Thr Ser Asp Tyr Ser Lys Tyr Leu Glu Glu Glu Val Ala Arg Leu Phe Val
Gln Trp Leu Met Asn Thr Lys Arg Asn Arg Asn Asn Ile Ala (SEQ ID NO: 75)

The compounds were administered by injection to groups of mice at a dose of 30 nmol/kg. A further group was administered saline (control).

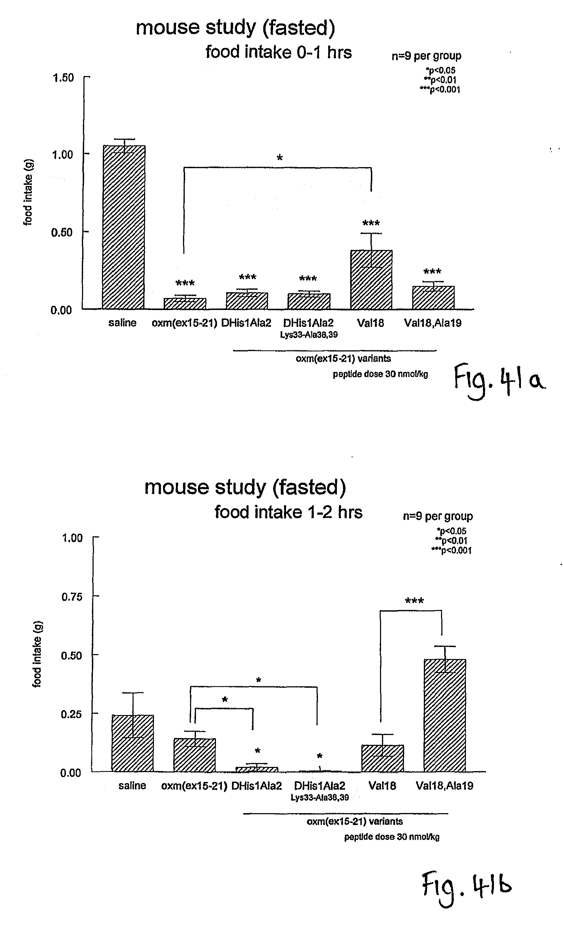

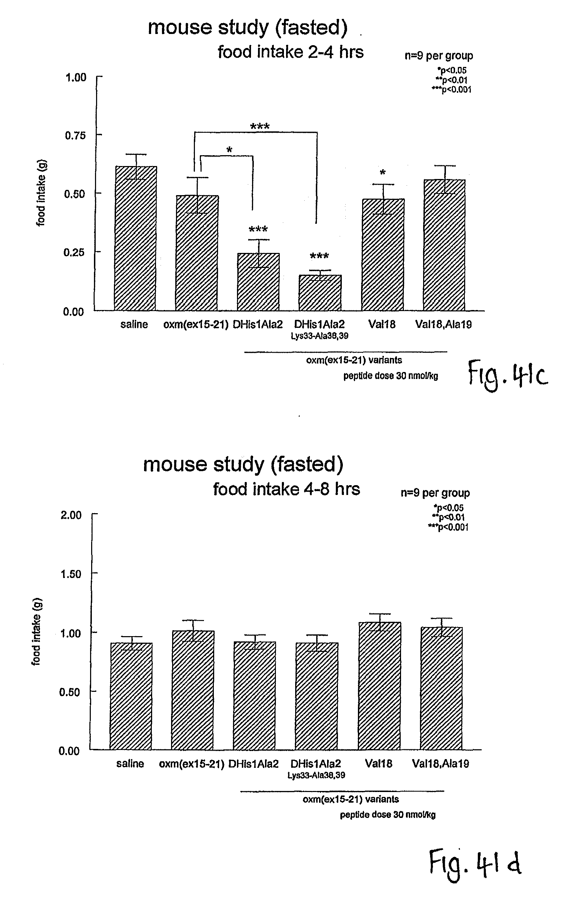

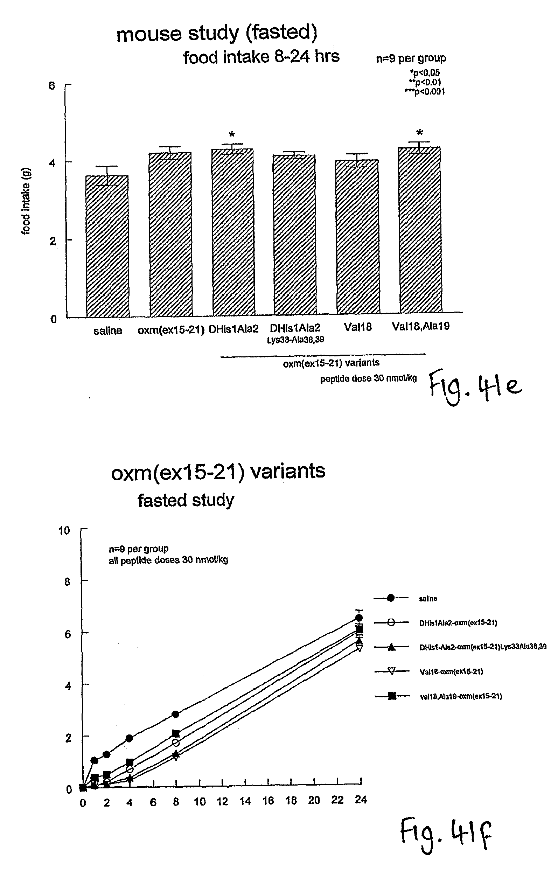

The measured food intake for each group is shown in Figs. 41 a to 41 e for, respectively, the intervals from 0 to 1 hour, from 1 to 2 hours, from 2 to 4 hours, from 4 to 8 hours and from 8 to 24 hours after injection. Fig.41f shows cumulative data. All compounds reduced cumulative food intake. N terminal modification prolonged biological activity.

### Example 34: oxm(ex15-24) variants

Groups of fasted mice were each injected with one of the following:
oxm(ex15-24) 
His Ser Gln Gly Thr Phe Thr Ser Asp Tyr Ser Lys Tyr Leu Glu Glu Glu Ala Val Arg Leu Phe Ile
Glu Trp Leu Met Asn Thr Lys Arg Asn Arg Asn Asn Ile Ala (SEQ ID NO: 63)
Val18-oxm(ex15-24) 
His Ser Gln Gly Thr Phe Thr Ser Asp Tyr Ser Lys Tyr Leu Glu Glu Glu Val Val Arg Leu Phe Ile
Glu Trp Leu Met Asn Thr Lys Arg Asn Arg Asn Asn Ile Ala (SEQ ID NO: 68)
Gln18-oxm(ex15-24) 
His Ser Gln Gly Thr Phe Thr Ser Asp Tyr Ser Lys Tyr Leu Glu Glu Glu Gln Val Arg Leu Phe Ile
Glu Trp Leu Met Asn Thr Lys Arg Asn Arg Asn Asn Ile Ala (SEQ ID NO: 69)
Ile19-oxm(ex15-24)

His Ser Gln Gly Thr Phe Thr Ser Asp Tyr Ser Lys Tyr Leu Glu Glu Glu Ala Ile Arg Leu Phe Ile
Glu Trp Leu Met Asn Thr Lys Arg Asn Arg Asn Asn Ile Ala (SEQ ID NO: 70)

The compounds were administered by injection to groups of mice at a dose of 30 nmol/kg. A further group was administered saline (control).

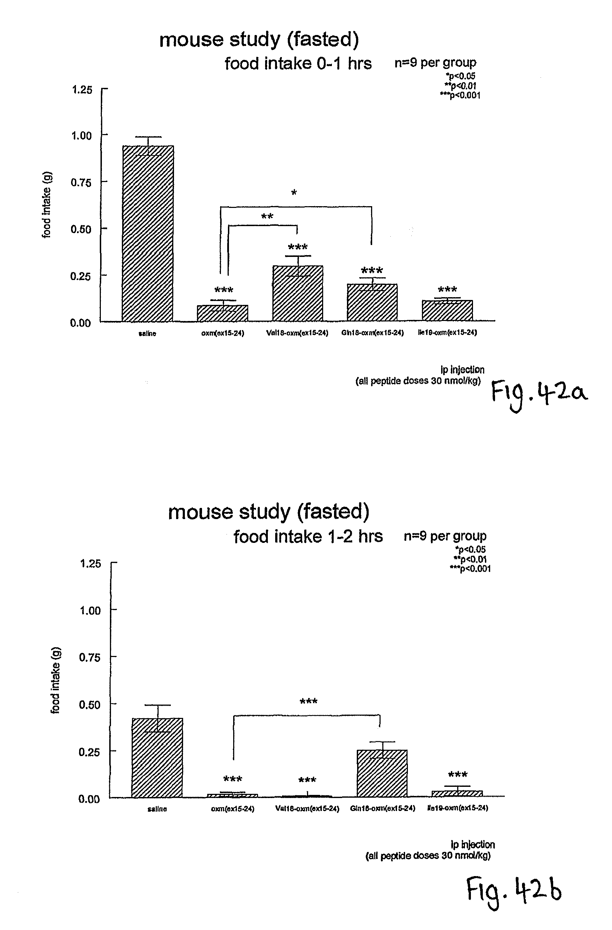

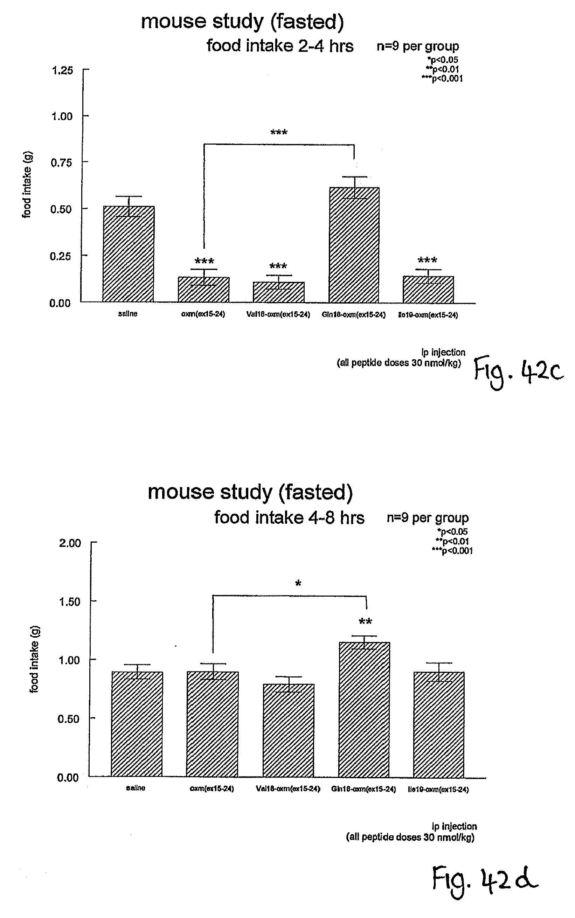

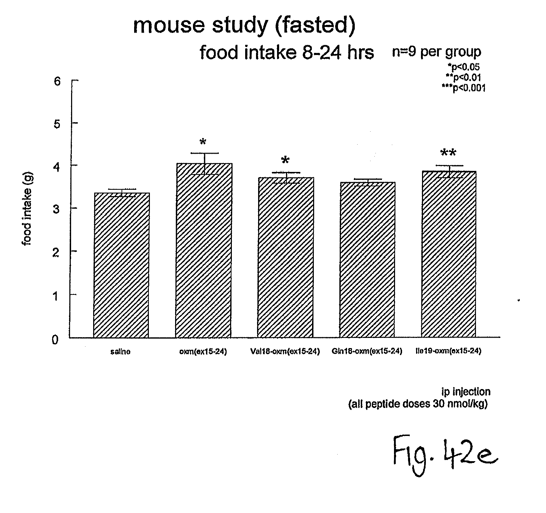

The measured food intake for each group is shown in Figs. 42a to 42e for, respectively, the intervals from 0 to 1 hour, from 1 to 2 hours, from 2 to 4 hours, from 4 to 8 hours and from 8 to 24 hours after injection. All compounds showed bioactivity. In particular, Val18-oxm(ex15-24) and Ile19-oxm(ex15-24) showed a similar reduction in food intake in comparison to oxm(ex15-24) over 4 hours.

### Example 35: multiple substitutions

Groups of fasted mice were each injected with one of the following:
D-His1,Ala2-oxm(ex15-24,27-33) (identified as "combi" in figures) 
D-His Ala Gln Gly Thr Phe Thr Ser Asp Tyr Ser Lys Tyr Leu Glu Glu Glu Ala Val Arg Leu Phe
Ile Glu Trp Leu Lys Asn Gly Gly Pro Ser Ser Asn Asn Ile Ala (SEQ ID NO: 51)
D-His1,Ala2,Gln15,Gln16,Gln17,Val18,Ile19,Arg20,Ile21,Phe22,Ile23,Gln24,Lys33, Ala38,Ala39-oxm (ex15-23,27-33) (identified as "xple subs" in figures)

D-His Ala Gln Gly Thr Phe Thr Ser Asp Tyr Ser Lys Tyr Leu Gln Gln Gln Val Ile Arg Ile Phe Ile
Gln Trp Leu Lys Asn Gly Gly Pro Ser Lys Asn Asn Ile Ala Ala Ala (SEQ ID NO: 79)

The compounds were administered by injection to groups of mice at a dose of 10 nmol/kg. A further group was administered saline (control).

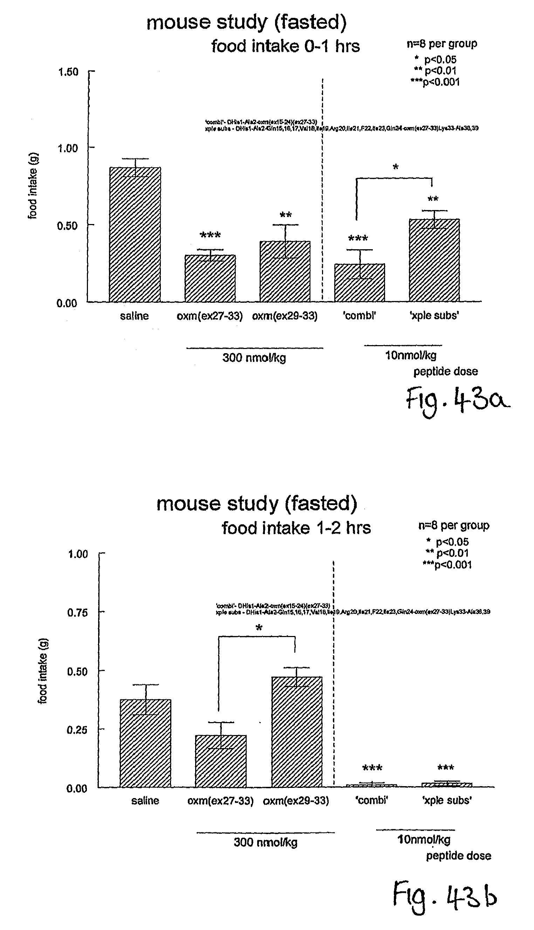

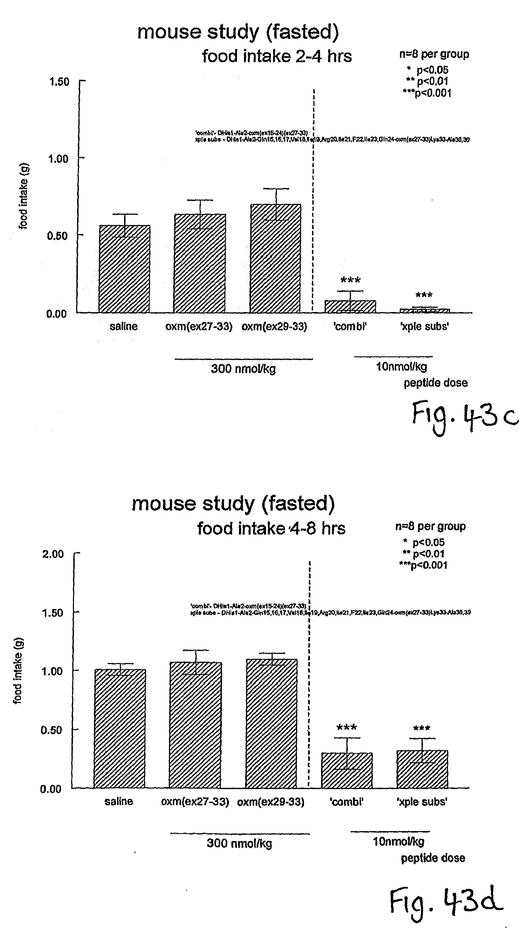

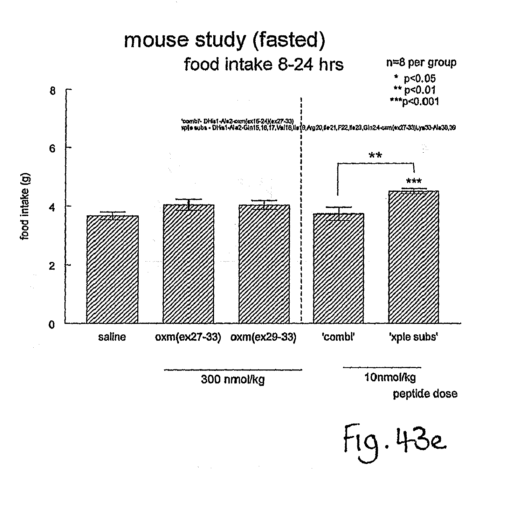

The measured food intake for each group is shown in Figs. 43a to 43e for, respectively, the intervals from 0 to 1 hour, from 1 to 2 hours, from 2 to 4 hours, from 4 to 8 hours and from 8 to 24 hours after injection. Both compounds showed a potent and prolonged reduction in food intake.

### Example 36: multiple substitutions

Groups of fasted mice were each injected with one of the following:
D-His1,Ala2,Lys33,Ala38,Ala39,Lys40-oxm(ex15-23,27-33) 
D-His Ala Gln Gly Thr Phe Thr Ser Asp Tyr Ser Lys Tyr Leu Glu Glu Glu Ala Val Arg Leu Phe
Val Gln Trp Leu Lys Asn Gly Gly Pro Ser Lys Asn Asn Ile Ala Ala Ala Lys (SEQ ID NO: 80)
D-His1,Ala2,Lys33,Ala3 8,Ala39 -oxm(ex15-23,27-33)

D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys Asn Asn Ile Ala Ala Ala (SEQ ID NO: 81)

The compounds were administered by injection to groups of mice at a dose of 3 nmol/kg. Further groups were administered with native oxm (comparison), exendin 4 (comparison) or saline (control).

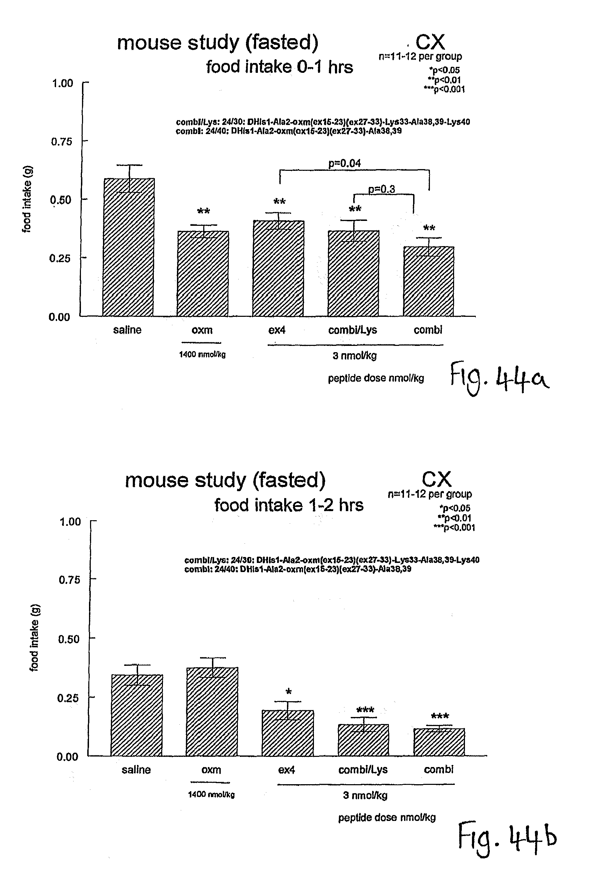

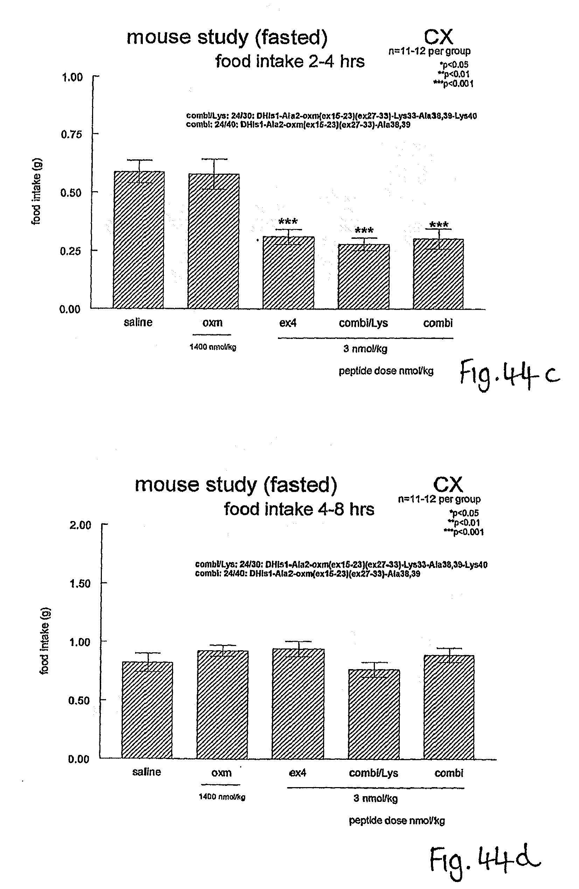

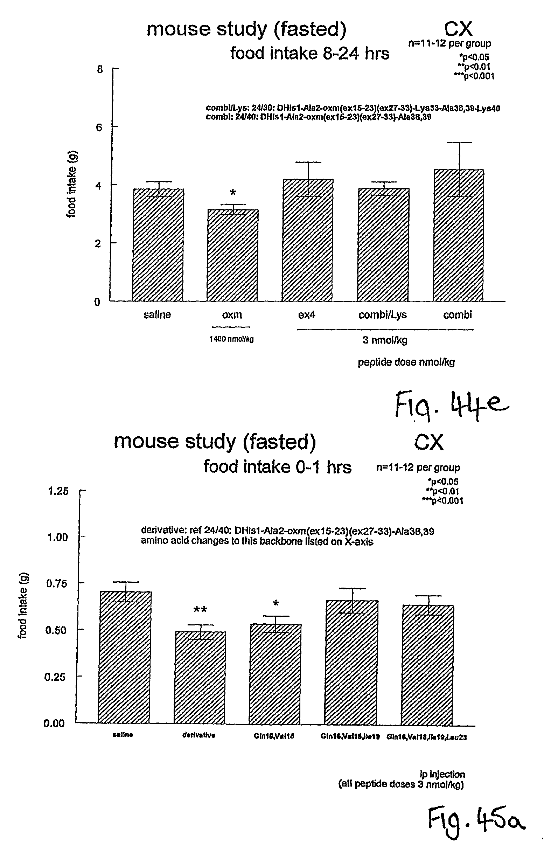

The measured food intake for each group is shown in Figs. 44a to 44e for, respectively, the intervals from 0 to 1 hour, from 1 to 2 hours, from 2 to 4 hours, from 4 to 8 hours and from 8 to 24 hours after injection. Both compounds showed biological activity that was more potent and prolonged than native oxm.

### Example 37: multiple substitutions

Groups of fasted mice were each injected with one of the following:
D-His1,Ala2,Lys33,Ala3 8,A1a39-oxm(ex15-23,27-33) 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys Asn Asn Ile Ala Ala Ala (SEQ ID NO: 81)
D-His1,Ala2,Gln16,Val18,Ala38,Ala39-oxm(ex15-23,27-33) 
D-His Ala Gln Gly Thr Phe Thr Ser Asp Tyr Ser Lys Tyr Leu Glu Gln Glu Val Val Arg Leu Phe
Ile Gln Trp Leu Lys Asn Gly Gly Pro Ser Ser Asn Asn Ile Ala Ala Ala (SEQ ID NO: 82)
D-His1,Ala2,Gln16,Val18,Ile19,Ala38,Ala39-omx(ex15-23,27-33) 
D-His Ala Gln Gly Thr Phe Thr Ser Asp Tyr Ser Lys Tyr Leu Glu Gln Glu Val Ile Arg Leu Phe
Ile Gln Trp Leu Lys Asn Gly Gly Pro Ser Ser Asn Asn Ile Ala Ala Ala (SEQ ID NO : 83)
D-His1,Ala2,Gln16,Val18,Ile19,Leu23,Ala38,Ala39-oxm(ex15-23,27-33)

D-His Ala Gln Gly Thr Phe Thr Ser Asp Tyr Ser Lys Tyr Leu Glu Gln Glu Val Ile Arg Leu Phe
Leu Gln Trp Leu Lys Asn Gly Gly Pro Ser Ser Asn Asn Ile Ala Ala Ala (SEQ ID NO : 84)

The compounds were administered by injection to groups of mice at a dose of 3 nmol/kg or 100 nmol/kg. A further group was administered with saline (control).

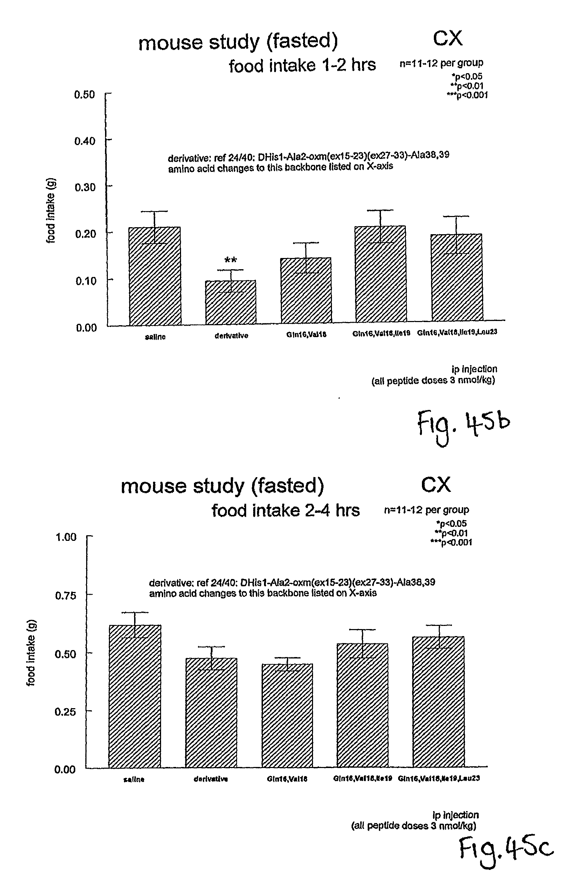

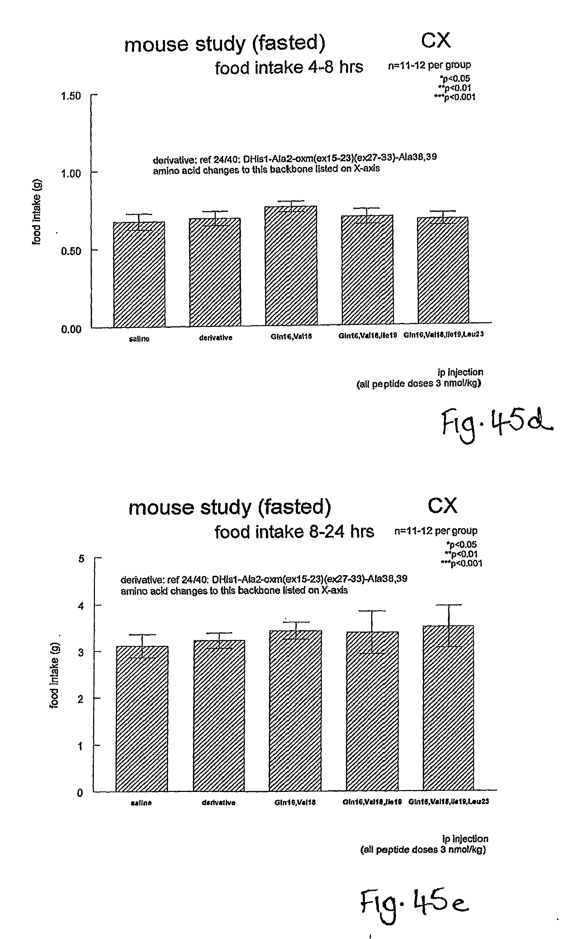

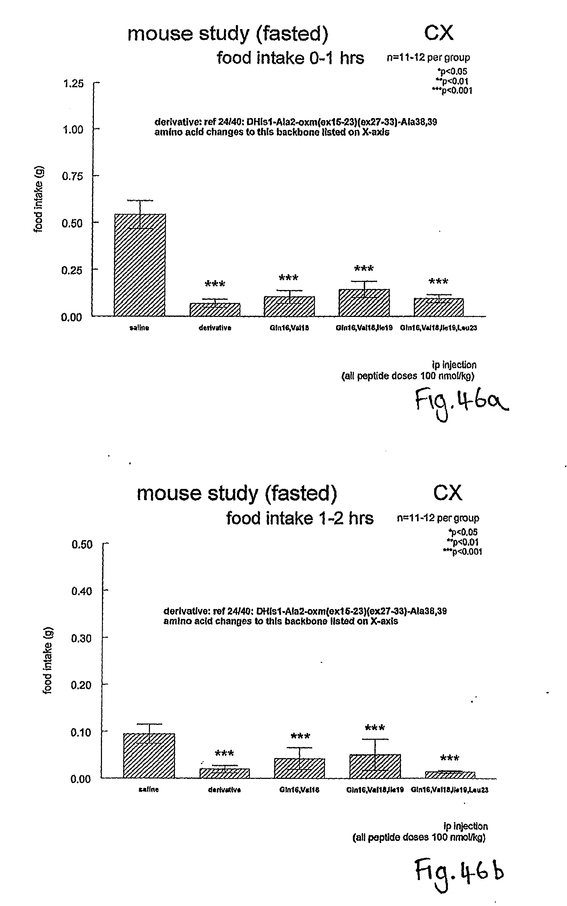

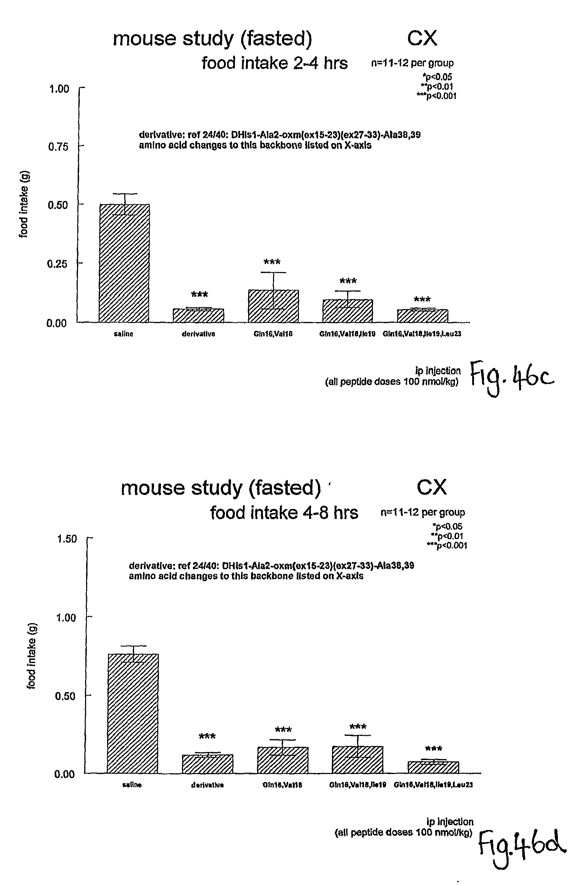

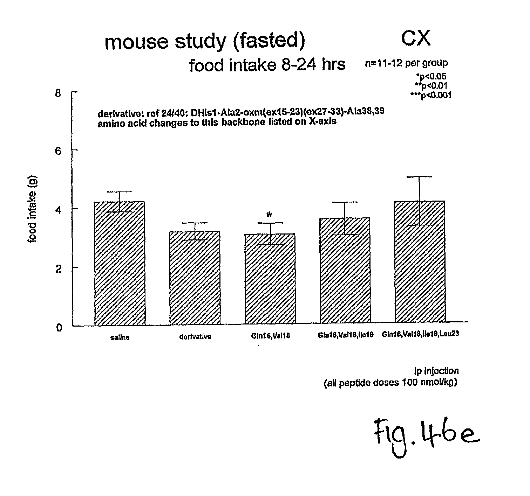

The measured food intake for each group given 3 nmol/kg is shown in Figs. 45 and for each group given 100 nmol/kg is is shown in Fig. 46. Figs. 45a and 46b to Figs. 45e and 46e show, respectively, the intervals from 0 to 1 hour, from 1 to 2 hours, from 2 to 4 hours, from 4 to 8 hours and from 8 to 24 hours after injection. All compounds showed a reduction in food intake over 8 hours.

### Example 38: multiple substitutions - medium dose

Groups of fasted mice were each injected with one of the following:
D-His1,Ala2,Lys33,Ala38,Ala39-oxm(ex15-23,27-33) 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys Asn Asn Ile Ala Ala Ala (SEQ ID NO: 81)
D-His1,Ala2,Gln16,Val18,Ala38,Ala39-oxm(ex15-23,27-33) 
D-His Ala Gln Gly Thr Phe Thr Ser Asp Tyr Ser Lys Tyr Leu Glu Gln Glu Val Val Arg Leu Phe
Ile Gln Trp Leu Lys Asn Gly Gly Pro Ser Ser Asn Asn Ile Ala Ala Ala (SEQ ID NO: 82)
D-His1,Ala2,Gln16,Val18,ne19,Ala38,Ala39-omx(ex15-23,27-33) 
D-His Ala Gln Gly Thr Phe Thr Ser Asp Tyr Ser Lys Tyr Leu Glu Gln Glu Val Ile Arg Leu Phe
Ile Gln Trp Leu Lys Asn Gly Gly Pro Ser Ser Asn Asn Ile Ala Ala Ala (SEQ ID NO : 83)
D-His1,Ala2,Gln16,Val18,ne19,Leu23,Ala38,Ala39-oxm(ex15-23,27-33)

D-His Ala Gln Gly Thr Phe Thr Ser Asp Tyr Ser Lys Tyr Leu Glu Gln Glu Val Ile Arg Leu Phe
Leu Gln Trp Leu Lys Asn Gly Gly Pro Ser Ser Asn Asn Ile Ala Ala Ala (SEQ ID NO : 84)

The compounds were administered by injection to groups of mice at a dose of 50 nmol/kg. A further group was administered with saline (control) and exendin 4 (comparator).

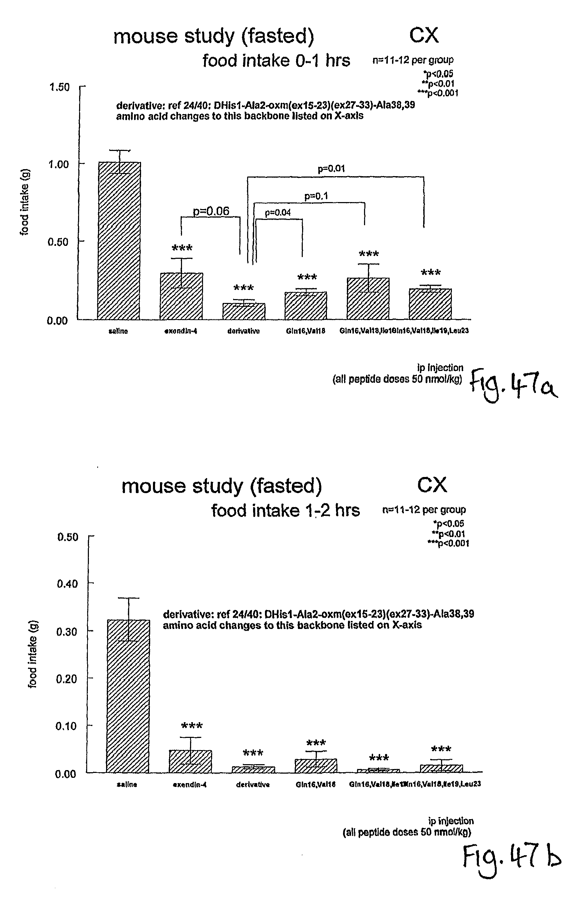

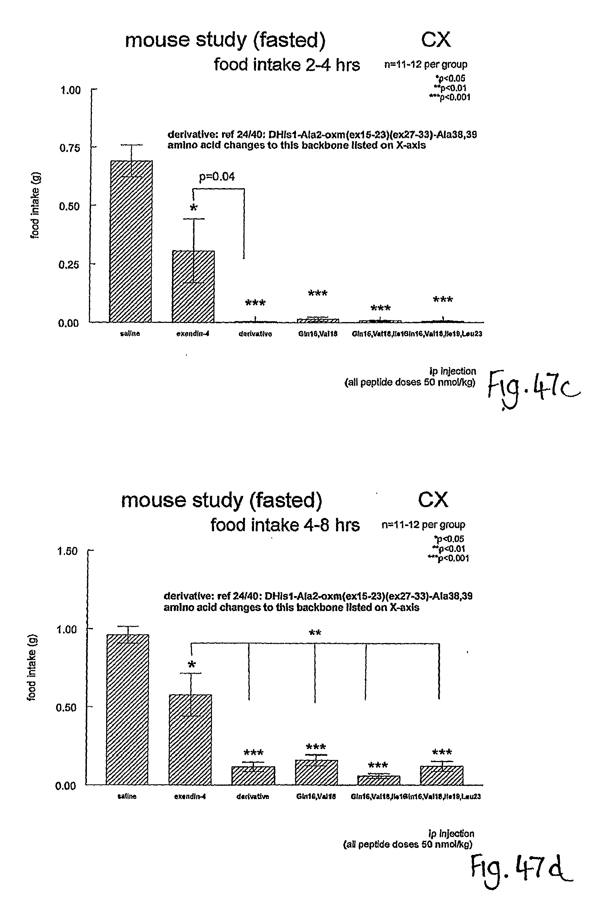

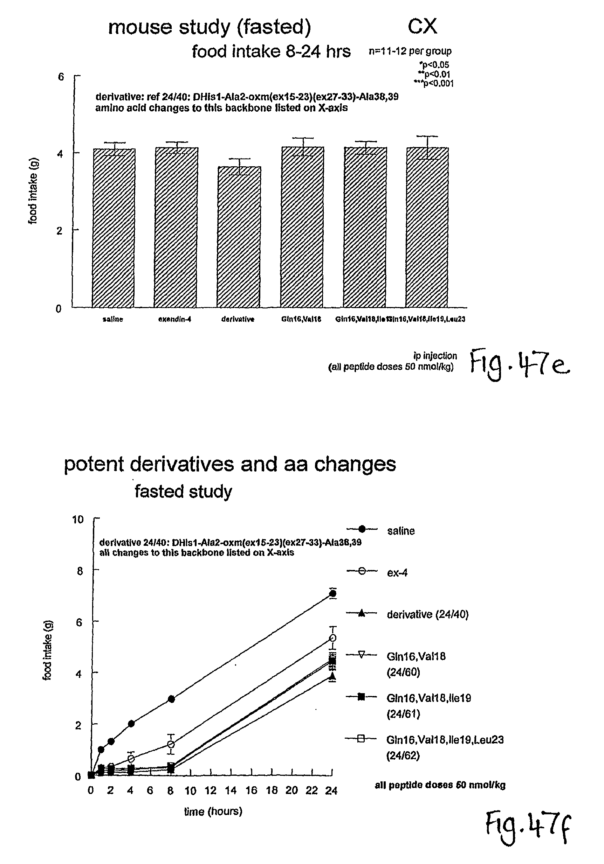

The measured food intake for each group is shown in Figs. 47a to 47e for, respectively, the intervals from 0 to 1 hour, from 1 to 2 hours, from 2 to 4 hours, from 4 to 8 hours and from 8 to 24 hours after injection. Fig. 47f shows cumulative data. All compounds reduced cumulative food intake.

### Example 39: effects of Lys33 and Lys40 on potent oxm analogues

Groups of fasted mice were each injected with one of the following:
D-His1,Ala2,Lys33,Ala38,Ala39,Lys40-oxm(ex15-23,27-33) 
D-His Ala Gln Gly Thr Phe Thr Ser Asp Tyr Ser Lys Tyr Leu Glu Glu Glu Ala Val Arg Leu Phe
Val Gln Trp Leu Lys Asn Gly Gly Pro Ser Lys Asn Asn Ile Ala Ala Ala Lys (SEQ ID NO: 85)
D-His1,Ala2,Ala38,Ala39,Lys40-oxm(ex15-23,27-33) 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala Lys (SEQ ID NO : 86)
D-His1,Ala2,Lys33,Ala38,Ala39-oxm(ex15-23,27-33)

D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys Asn Asn Ile Ala Ala Ala (SEQ ID NO : 81)

The compounds were administered by injection to groups of mice at a dose of 3 nmol/kg. A further group was administered with saline (control).

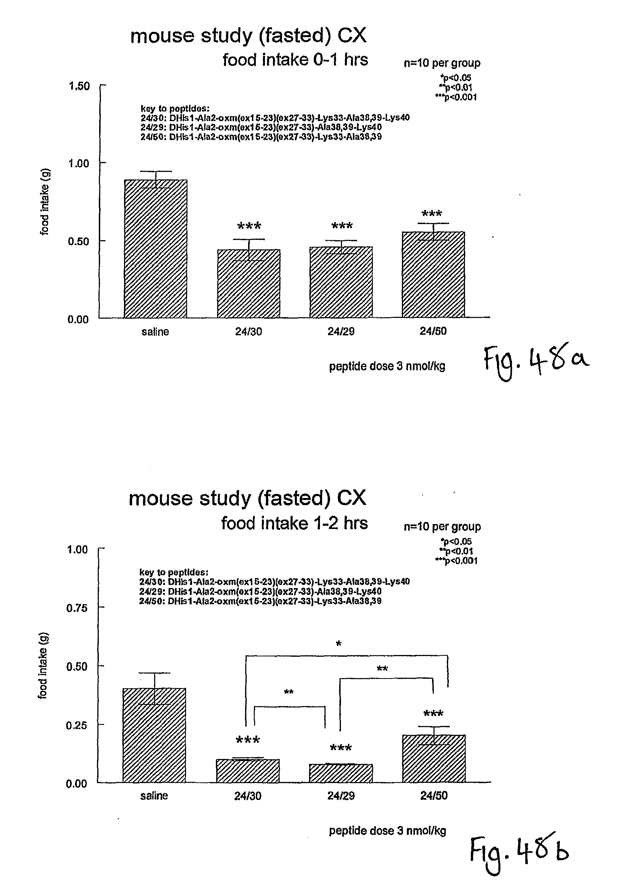

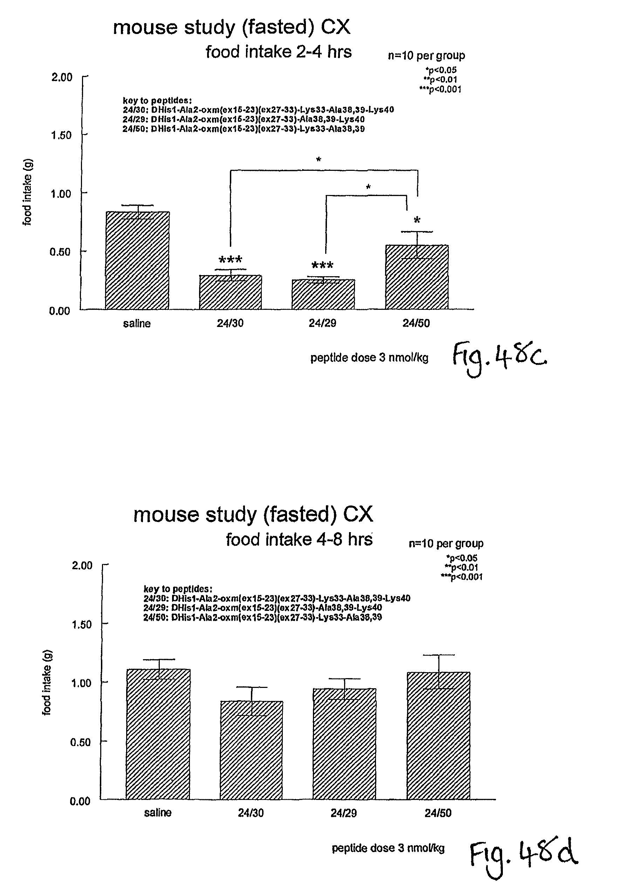

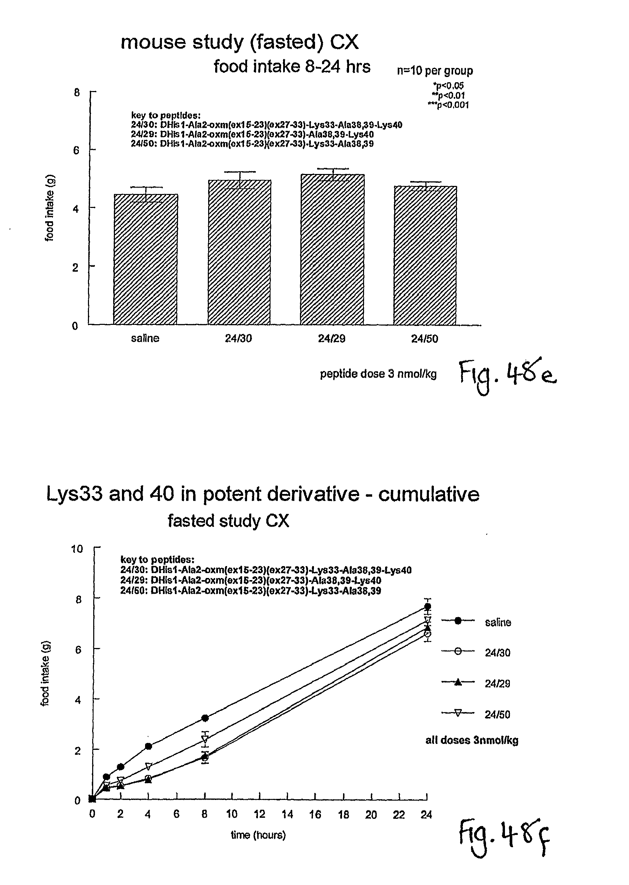

The measured food intake for each group is shown in Figs. 48a to 48e for, respectively, the intervals from 0 to 1 hour, from 1 to 2 hours, from 2 to 4 hours, from 4 to 8 hours and from 8 to 24 hours after injection. Fig. 48f shows cumulative data. All compounds showed reduced cumulative food intake, with the presence of Lys40 in particular showing a beneficial effect on longevity of activity.

### Example 40: multiple substitutions

Groups of fasted mice were each injected with one of the following:
D-His1,Ala2,Ala38,Ala39-oxm(ex15-23,27-33) 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala (SEQ ID NO : 86)
D-His1,Ala2,Glu3,Ala38,Ala39-oxm(ex15-23,27-33) 
D-His Ala Glu Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala (SEQ ID NO : 87)
D-His1,Ser2,Asp3,Ala38,Ala39-oxm(ex15-23,27-33) 
D-His Ser Asp Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala (SEQ ID NO : 88)
Ala2,Asp3,Ala38,Ala39-oxm(ex15-23,27-33) 
His Ala Asp Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala (SEQ ID NO : 89)
Gly2,Glu3,Ala38,Ala38-oxm(ex15-23,27-33)

D-His Gly Glu Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala (SEQ ID NO : 149)

The compounds were administered by injection to groups of mice at a dose of 7 nmol/kg. A further group was administered with saline (control).

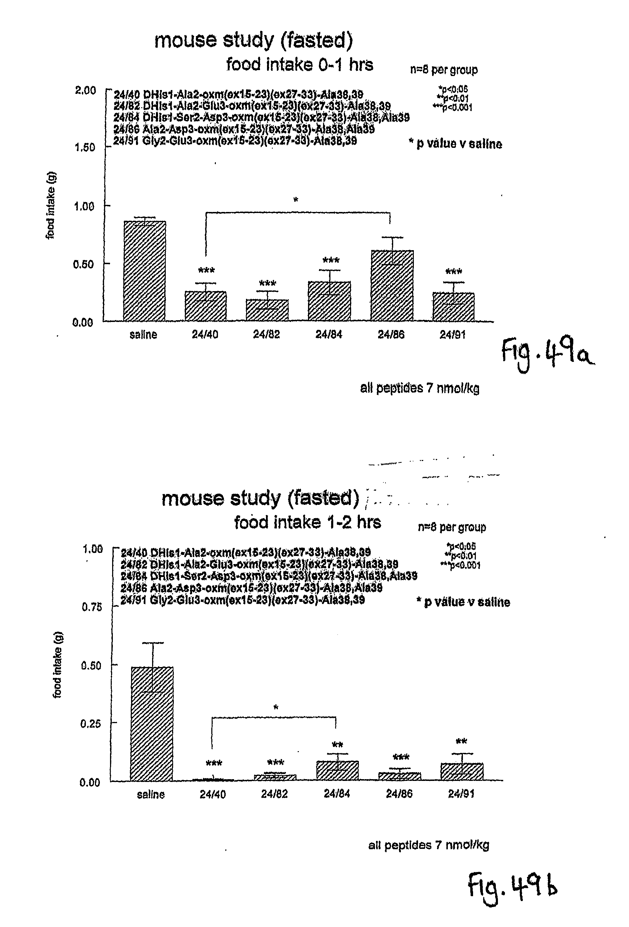

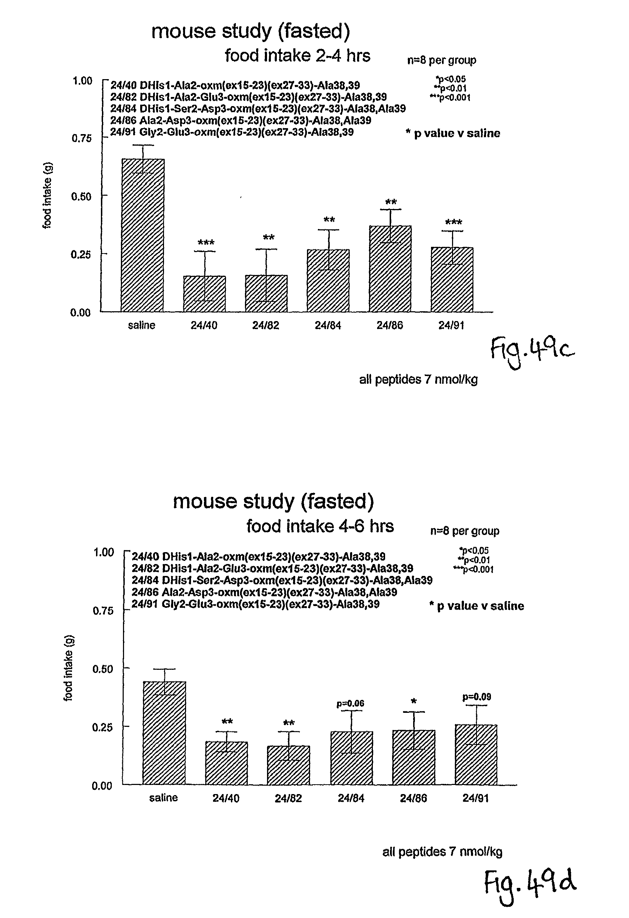

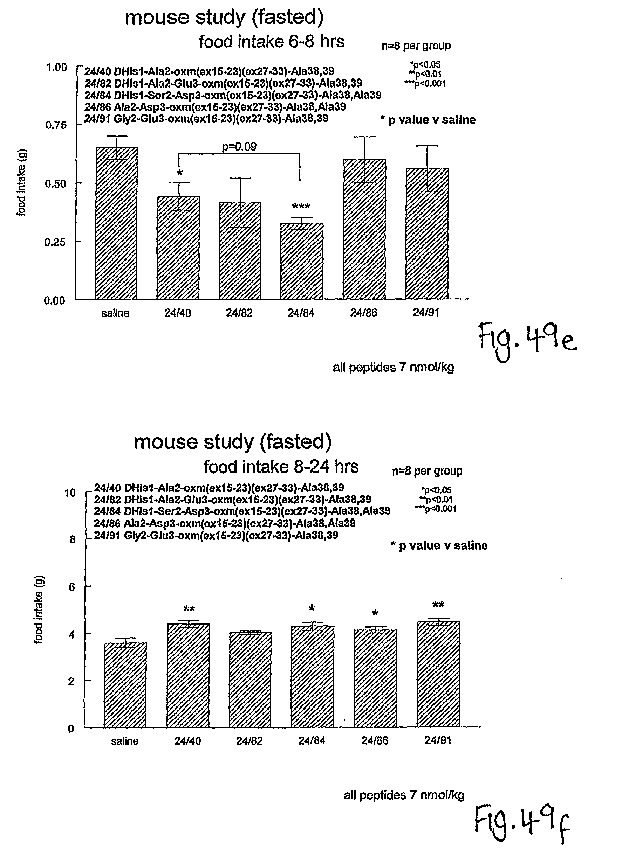

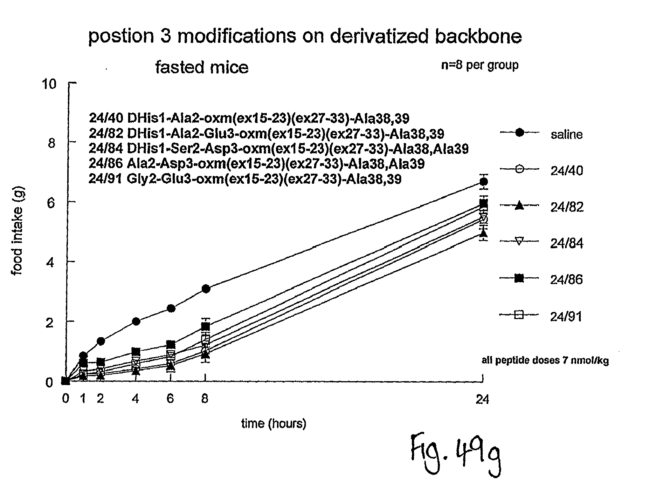

The measured food intake for each group is shown in Figs. 49a to 49f for, respectively, the intervals from 0 to 1 hour, from 1 to 2 hours, from 2 to 4 hours, from 4 to 6 hours, from 6 to 8 hours and from 8 to 24 hours after injection. Fig. 49g shows cumulative data. All compounds showed a reduction in food intake.

### Example 41: residue 1 to 3 substitutions

Groups of fasted mice were each injected with one of the following:
D-His1,Ala2,Ala38,Ala39-oxm(ex15-23,27-33) 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala (SEQ ID NO : 86)
Tyr1,Ala2,Glu3,Ala38,Ala39)oxm(ex15-23,27-33) 
Tyr Ala Glu Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala (SEQ ID NO : 91)
D-His1,Ala2,Gln3,Asn9,Ala38,Ala39-oxm(ex15-23,27-3 3) 
D-His Ala Gln Gly Thr Phe Thr Ser Asn Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala (SEQ ID NO : 92)
D-His1,Ala2,Glu3,Glu24,Ala38,Ala39-oxm(ex15-23,27-33) 
D-His Ala Glu Gly Thr Phe Thr Ser Asp Tyr Ser Lys Tyr Leu Glu Glu Glu Ala Val Arg Leu Phe
Ile Glu Trp Leu Lys Asn Gly Gly Pro Ser Ser Asn Asn Ile Ala Ala Ala (SEQ ID NO : 93)
D-His1,Ala2,Glu3,Glu24,Asp35,Ala38,Ala39-oxm(ex15-23,27-33)

D-His Ala Glu Gly Thr Phe Thr Ser Asp Tyr Ser Lys Tyr Leu Glu Glu Glu Ala Val Arg Leu Phe
Ile Glu Trp Leu Lys Asn Gly Gly Pro Ser Ser Asn Asp Ile Ala Ala Ala (SEQ ID NO : 94)

The compounds were administered by injection to groups of mice at a dose of 7 nmol/kg. A further group was administered with saline (control).

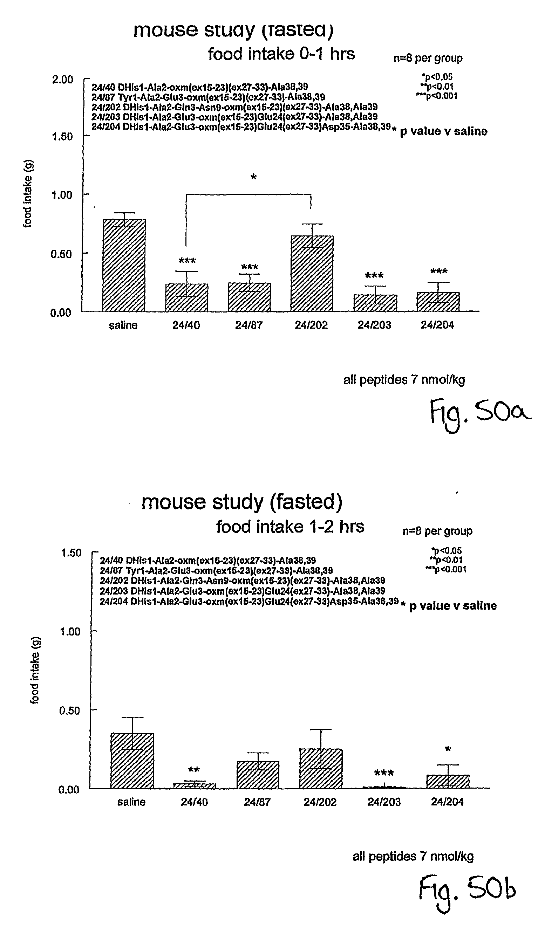

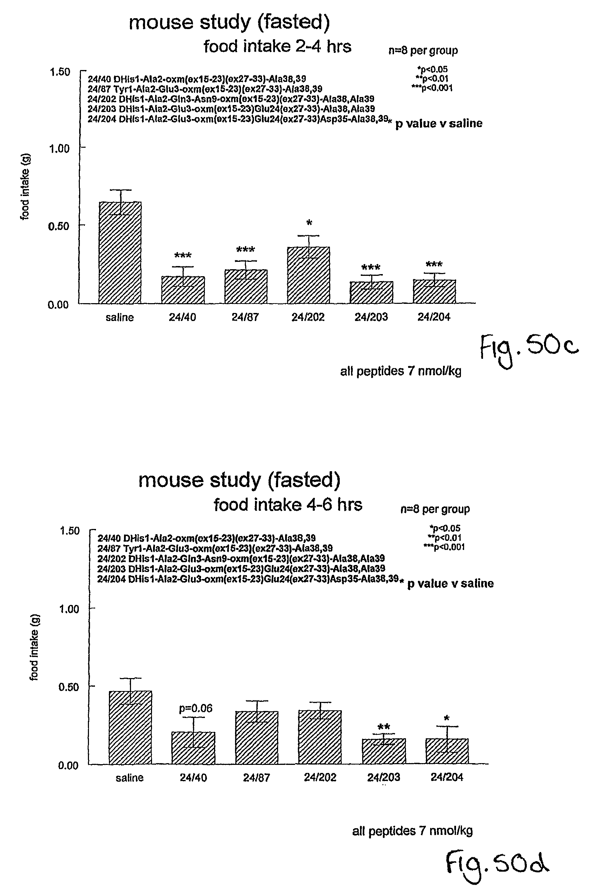

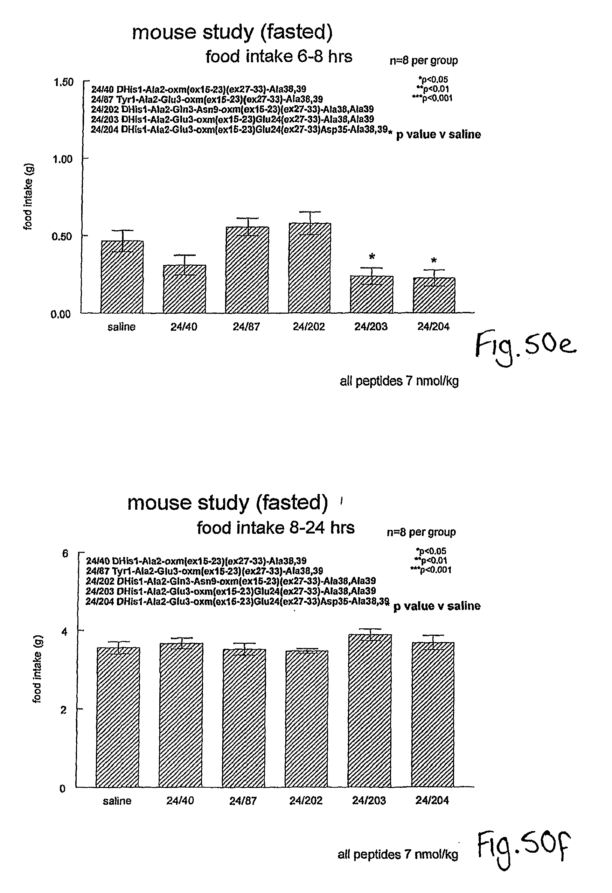

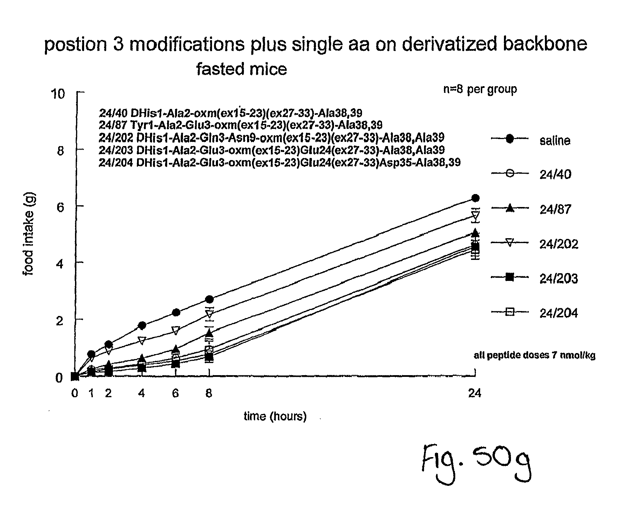

The measured food intake for each group is shown in Figs. 50a to 50f for, respectively, the intervals from 0 to 1 hour, from 1 to 2 hours, from 2 to 4 hours, from 4 to 6 hours, from 6 to 8 hours and from 8 to 24 hours after injection. Fig. 50g shows cumulative data. All compounds showed a reduction in food intake.

### Example 42: further residue 1 to 3 substitutions

Groups of fasted mice were each injected with one of the following:
D-His1,Ala2,Ala38,Ala39-oxm(ex15-23,27-33) 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala (SEQ ID NO : 86)
D-His1,Ala2,Glu3,Ala38,Ala39-oxm(ex15-23,27-33) 
D-His Ala Glu Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala (SEQ ID NO : 87)
D-His1,Ala2,Glu3,Glu24,Ala38,Ala39-oxm(ex15-23,27-33) 
D-His Ala Glu Gly Thr Phe Thr Ser Asp Tyr Ser Lys Tyr Leu Glu Glu Glu Ala Val Arg Leu Phe
Ile Glu Trp Leu Lys Asn Gly Gly Pro Ser Ser Asn Asn Ile Ala Ala Ala (SEQ ID NO : 93)
D-His1,Ser2,Asp3,Ala38,Ala39-oxm(ex15-23,27-33)

D-His Ser Asp Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala (SEQ ID NO : 88)

The compounds were administered by injection to groups of mice at a dose of 5 nmol/kg. Further groups were administered with exendin 4 (comparison) or saline (control).

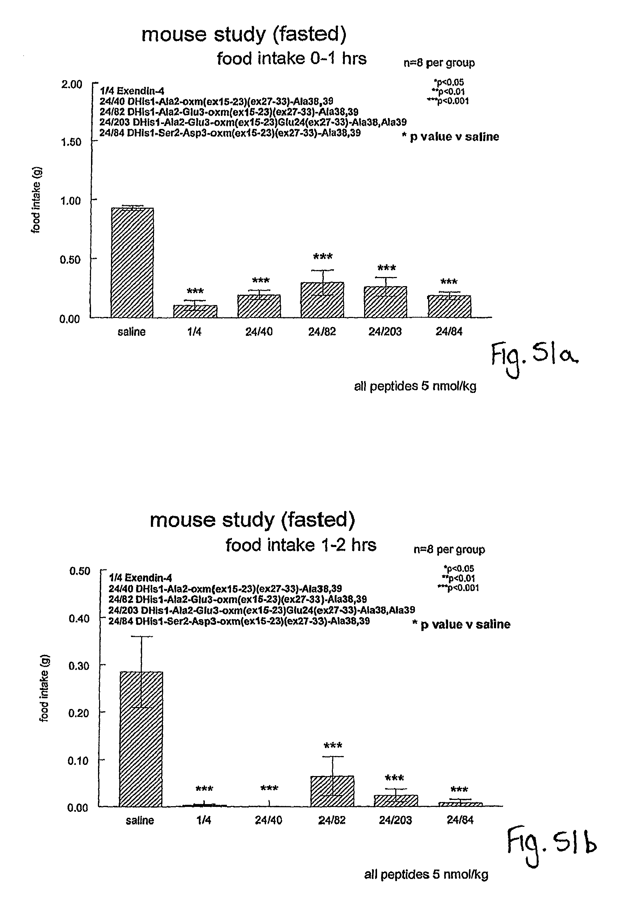

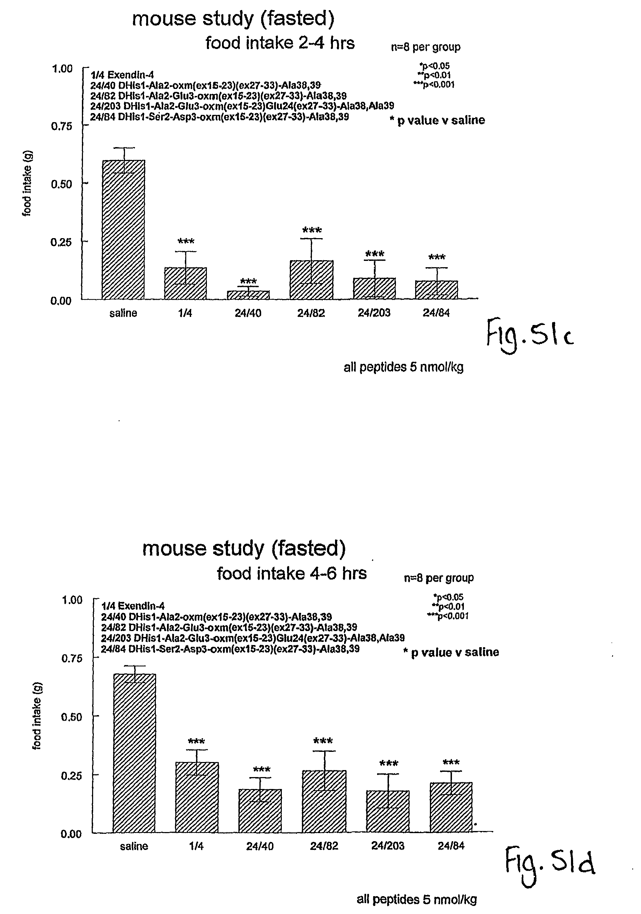

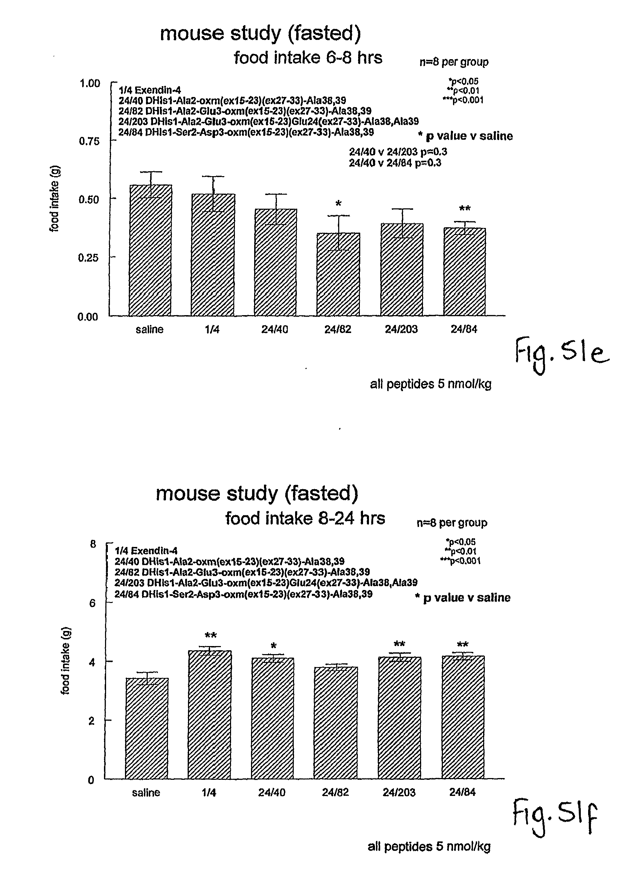

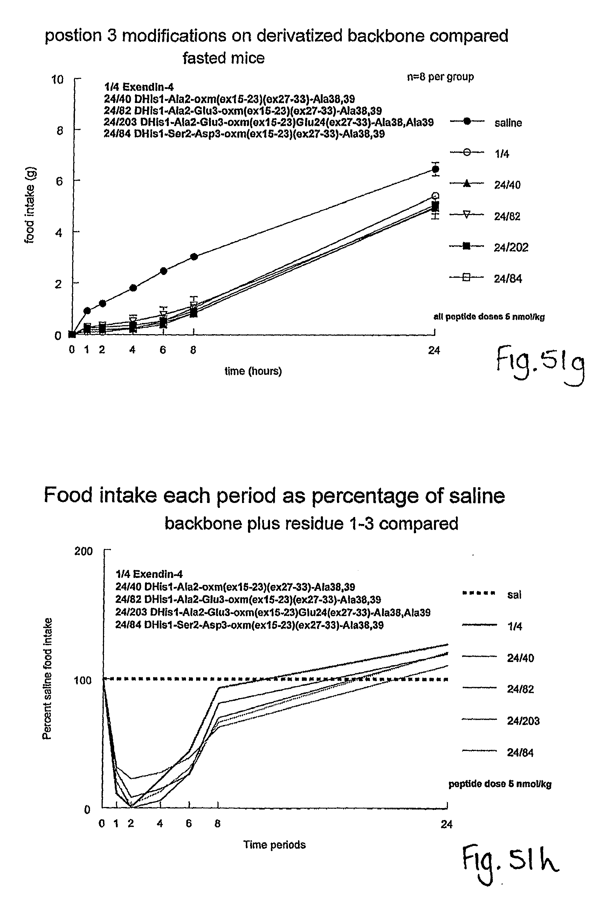

The measured food intake for each group is shown in Figs. 51a to 51f for, respectively, the intervals from 0 to 1 hour, from 1 to 2 hours, from 2 to 4 hours, from 4 to 6 hours, from 6 to 8 hours and from 8 to 24 hours after injection. Figs. 51g and 51h show cumulative data. All compounds showed a reduction in food intake, in some cases this activity is more prolonged than that of exendin 4.

### Example 43: dose responses

Groups of fasted mice were each injected with one of the following:
oxm 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala (SEQ ID NO: 7)
D-His-Ala2-oxm

D-His Ala Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala (SEQ ID NO: 95)

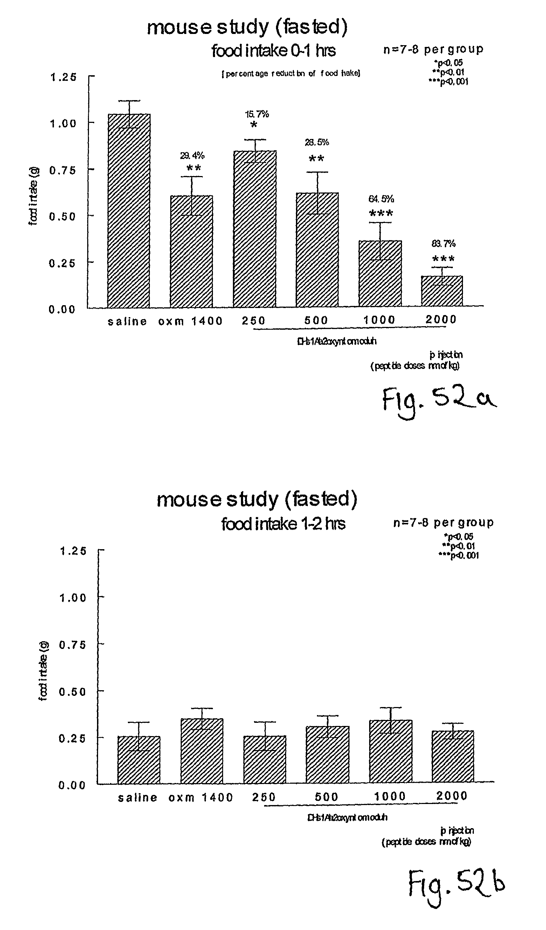

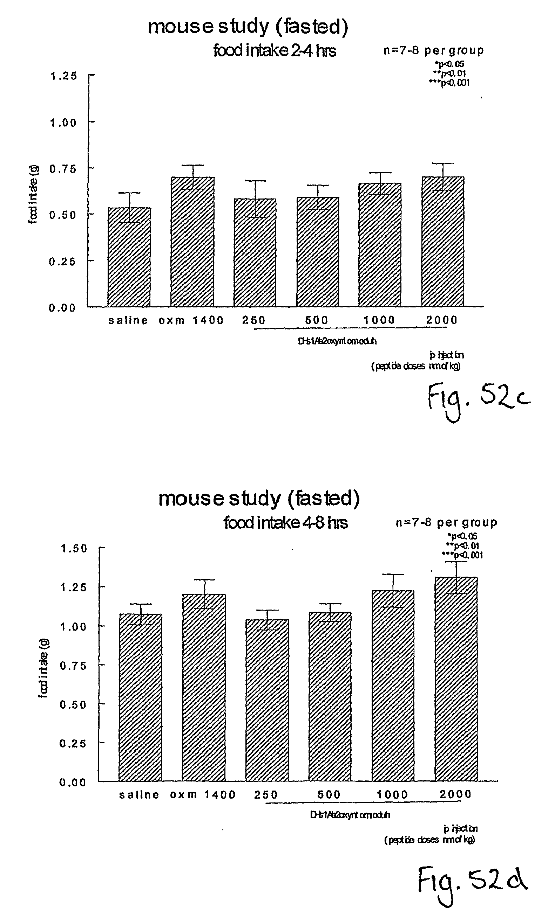

The compounds were administered by injection to groups of mice at various doses (stated in nmol/kg in Fig. 52). A further group was administered with saline (control).

The measured food intake for each group is shown in Figs. 52a to 52d for, respectively, the intervals from 0 to 1 hour, from 1 to 2 hours, from 2 to 4 hours and from 4 to 8 hours after injection.

D-His-Ala2-Oxm was shown to be more effective than oxm. The minimium effective dose of D-His-Ala2-Oxm is approximately 250 nmol/kg, which reduced food intake by 84.3% in the first hour. 500 nmol/kg D-His-Ala2-Oxm provided a similar reduction in food intake as native oxm at the higher dose of 1400 nmol/kg.

### Example 44: added side chains

Groups of fasted mice were each injected with one of the following:
Lys30-hexadecanoate-oxm (identified as "Lys30" in figures) 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys-hexadecanoate Arg Asn Arg Asn Asn Ile Ala (SEQ ID NO:
97)
Lys33-hexadecanoate-oxm (identified as "Lys33" in figures)

His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Lys-hexadecanoate Asn Asn Ile Ala (SEQ ID NO:
98)

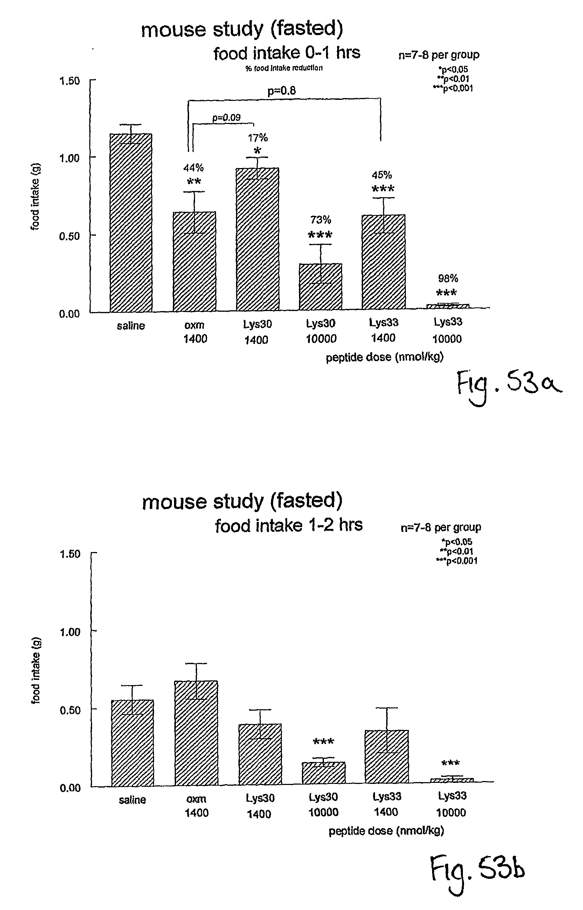

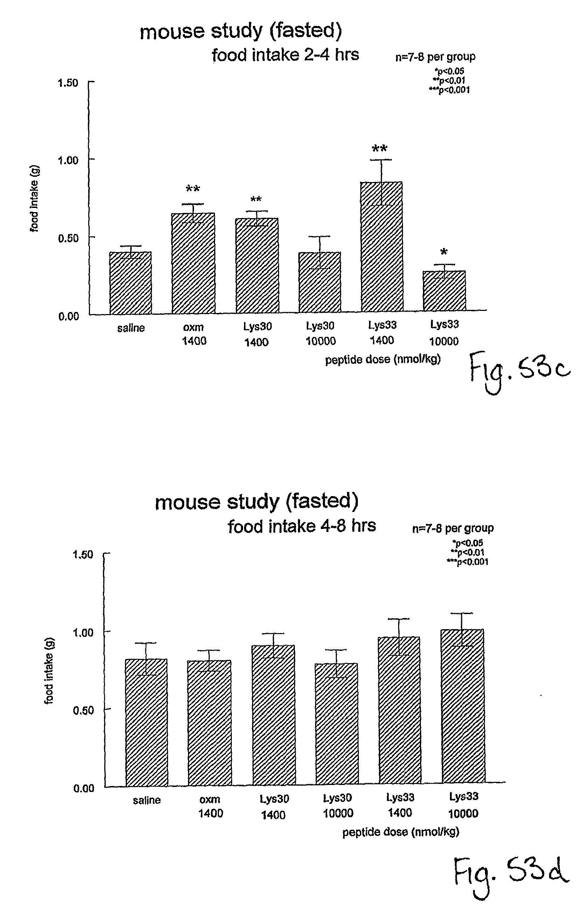

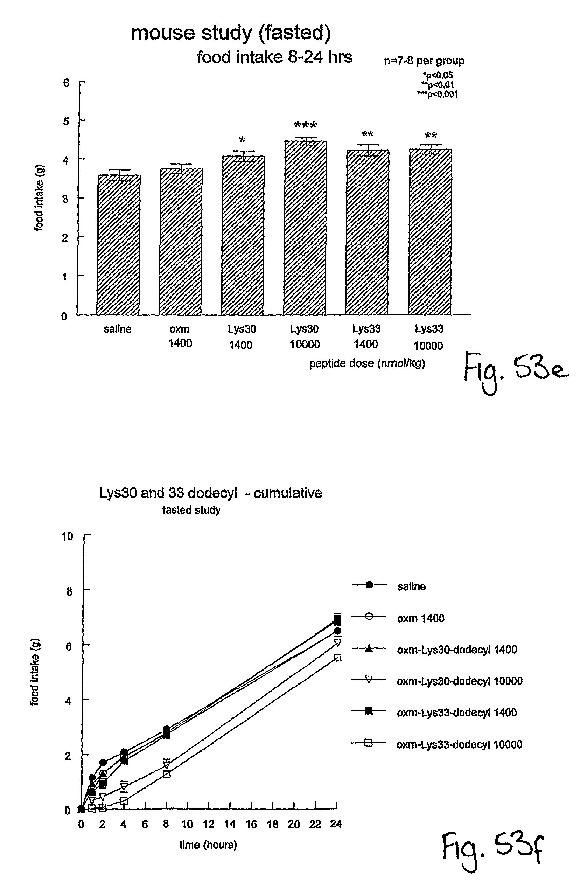

The compounds were administered by injection to groups of mice at various doses (stated in nmol/kg in Fig. 53). Further groups were administered with oxm (comparison) or saline (control).

The measured food intake for each group is shown in Figs. 53a to 53e for, respectively, the intervals from 0 to 1 hour, from 1 to 2 hours, from 2 to 4 hours, from 4 to 8 hours and from 8 to 24 hours after injection. Fig. 53f shows cumulative data.
Side chain addition did not appreciably reduce the compound's bioactivity.

### Example 45: palmitoyl side chain

Groups of fasted mice were each injected with the following:
Lys33-palmitoyl-oxm (identified as "palm33" in figures)

His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Lys-palmitoyl Asn Asn Ile Ala (SEQ ID NO: 99)

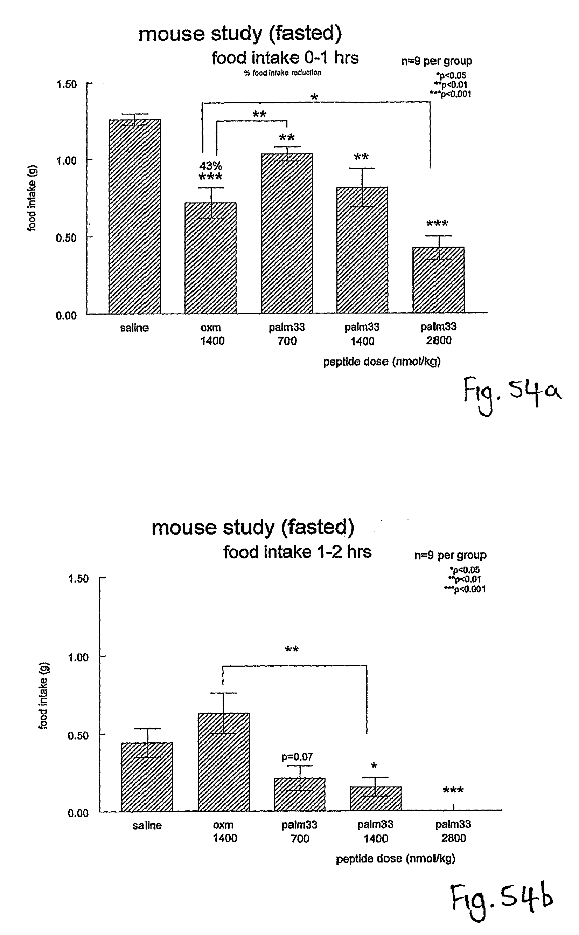

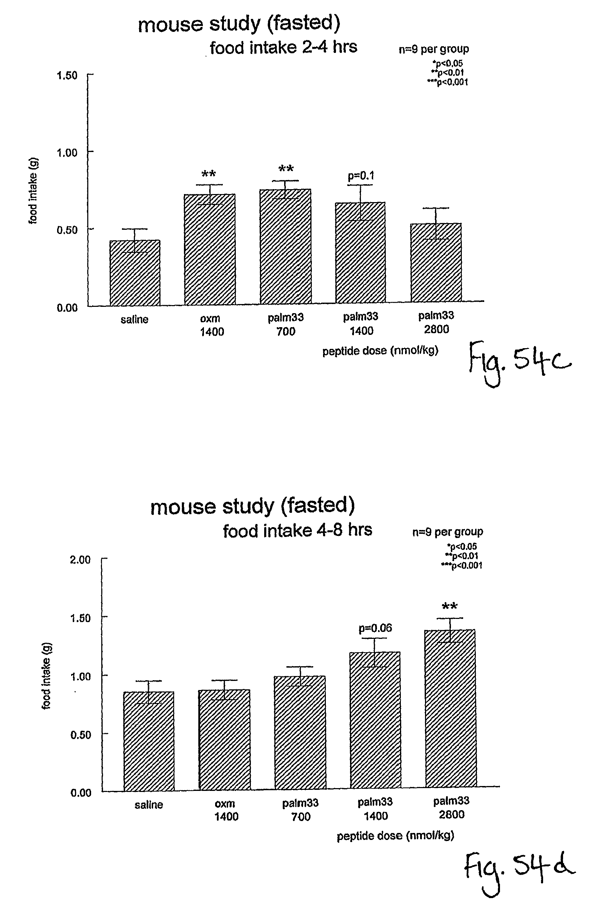

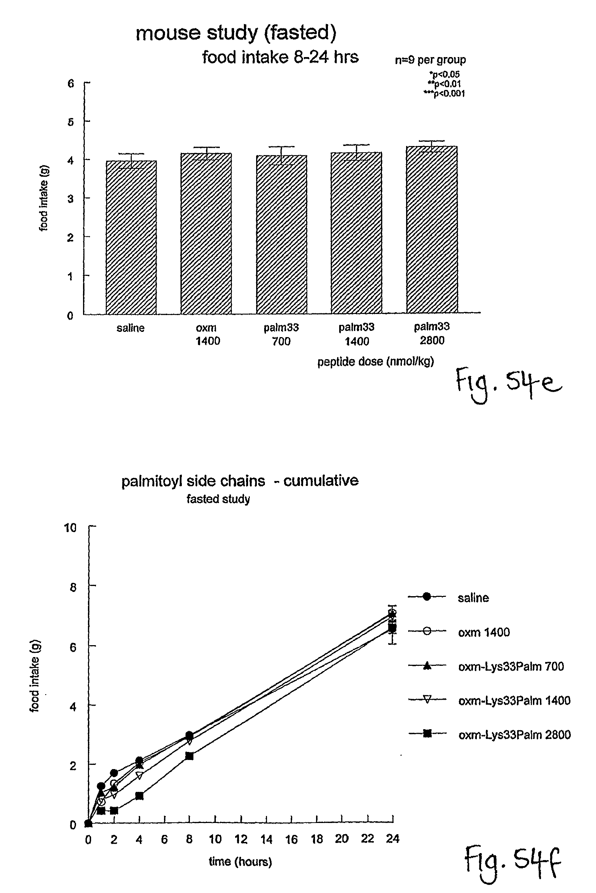

The compound was administered by injection to groups of mice at various doses (stated in nmol/kg in Fig. 54). Further groups were administered with oxm (comparison) or saline (control).

The measured food intake for each group is shown in Figs. 54a to 54e for, respectively, the intervals from 0 to 1 hour, from 1 to 2 hours, from 2 to 4 hours, from 4 to 8 hours and from 8 to 24 hours after injection. Fig. 54f shows cumulative data. The side chain addition prolonged the activity of the analogues into the 1-2 hour period.

### Example 46: palmitoyl side chain

Groups of fasted mice were each injected with one of the following:
D-His1,Ala2,Lys33-palmitoyl-oxm(ex15-21) (identified as "combipalm33" in figures) 
D-His Ala Gln Gly Thr Phe Thr Ser Asp Tyr Ser Lys Tyr Leu Glu Glu Glu Ala Val Arg Leu Asp
Phe Val Gln Trp Leu Met Asn Thr Lys Arg Asn Lys-palmitoyl Asn Asn Ile Ala (SEQ ID NO:
100)
Lys33-palmitoyl-oxm (identified as "palm33" in figures)

His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Lys-palmitoyl Asn Asn Ile Ala (SEQ ID NO: 100)

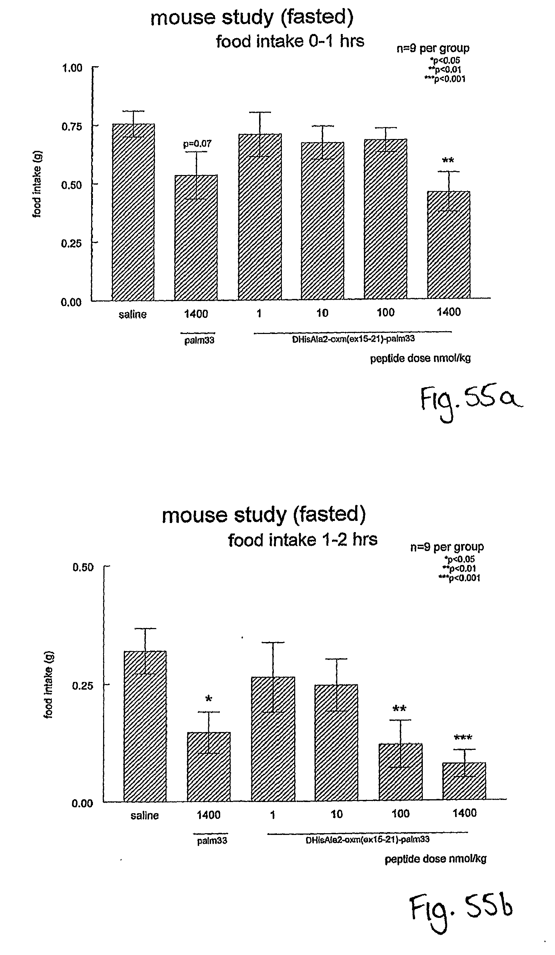

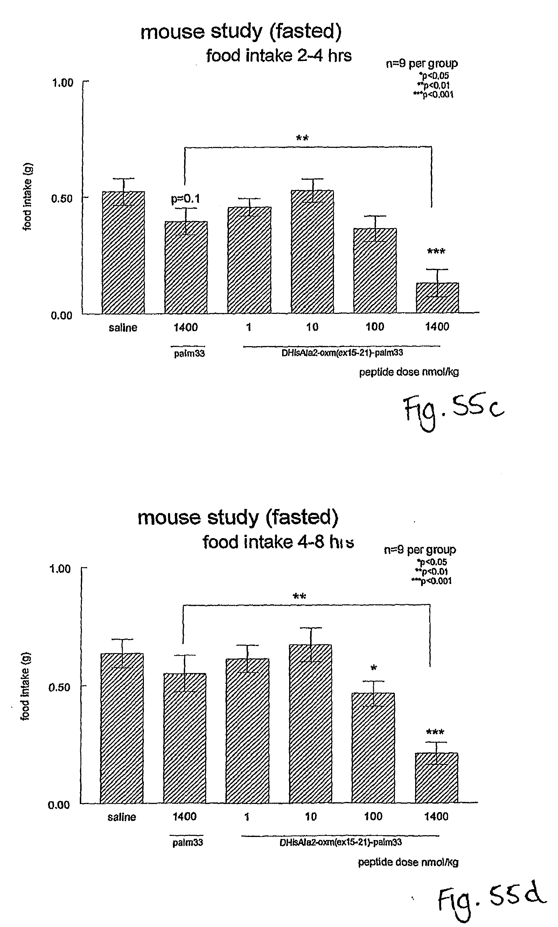

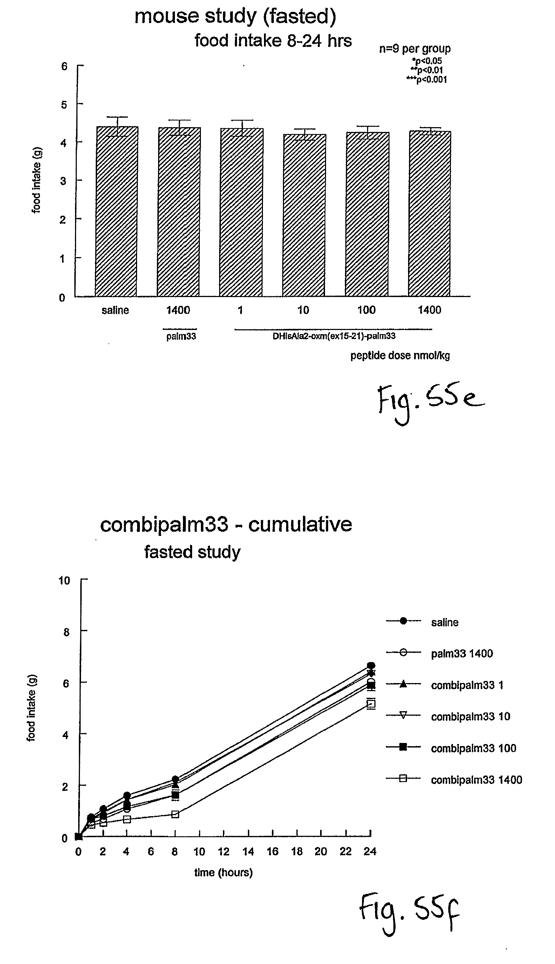

The compounds were administered by injection to groups of mice at various doses (stated in nmol/kg in Fig. 55). A further group was administered with saline (control).

The measured food intake for each group is shown in Figs. 55a to 55e for, respectively, the intervals from 0 to 1 hour, from 1 to 2 hours, from 2 to 4 hours, from 4 to 8 hours and from 8 to 24 hours after injection. Fig. 55f shows cumulative data.

Side chain addition did not appreciably reduce the compound's bioactivity. Side chain addition prolonged the reduction of food intake in the 4 to 8 hour time period.

### Example 47: palmitoyl side chain

Groups of fasted mice were each injected with one of the following:
Lys33-palmitoyl-oxm (identified as "palm33" in figures) 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Lys-palmitoyl Asn Asn Ile Ala (SEQ ID NO: 99)
D-His,Ala2-oxm(ex15-21)Lys33-palmitoyl (identified as "combipalm33" in figures) 
D-His Ala Gln Gly Thr Phe Thr Ser Asp Tyr Ser Lys Tyr Leu Glu Glu Glu Ala Val Arg Leu Phe
Val Gln Trp Leu Met Asn Thr Lys Arg Asn Lys-palmitoyl Asn Asn Ile Ala (SEQ ID NO: 100)
D-His,Ala2,Val18-oxm(ex15-21)Lys33-palmitoyl (identified as "Val18combipalm33" in figures)

D-His Ala Gln Gly Thr Phe Thr Ser Asp Tyr Ser Lys Tyr Leu Glu Glu Glu Val Val Arg Leu Phe
Val Gln Trp Leu Met Asn Thr Lys Arg Asn Lys-palmitoyl Asn Asn Ile Ala (SEQ ID NO: 101)

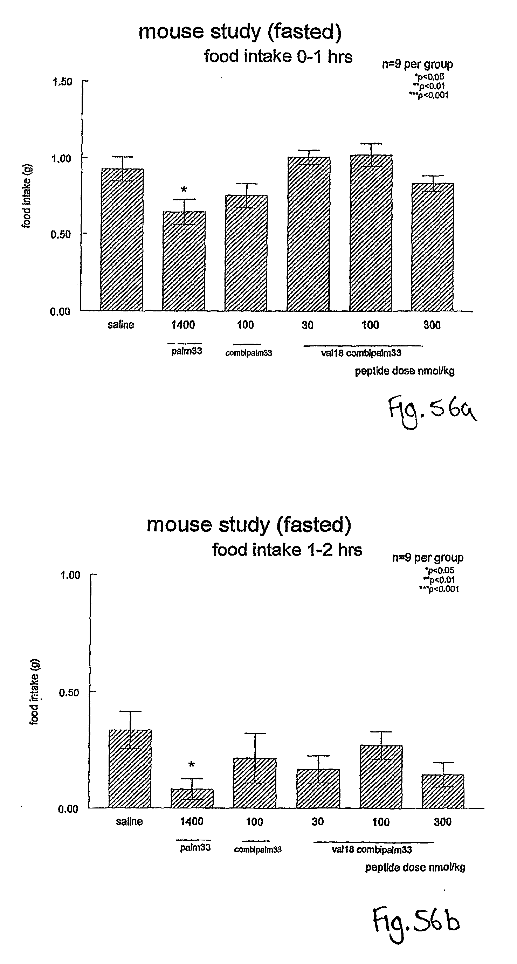

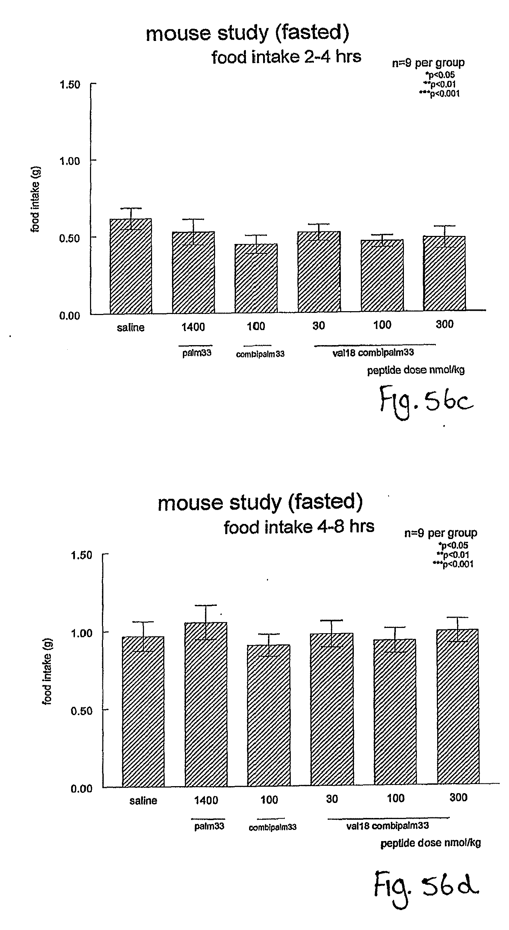

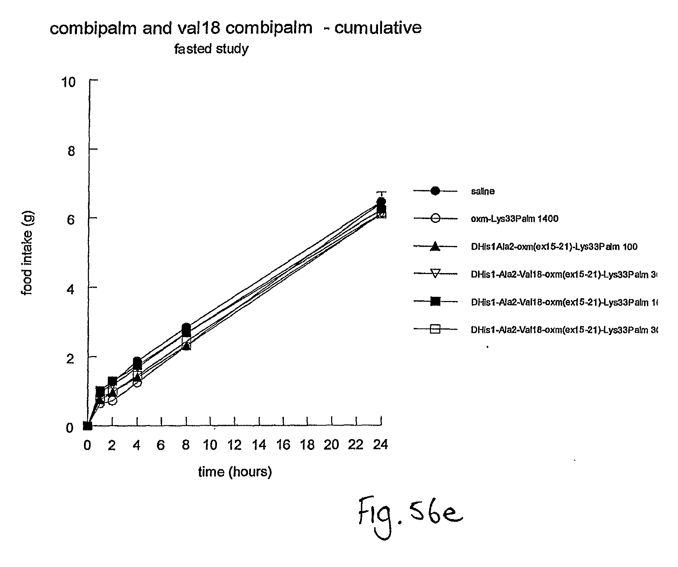

The compounds were administered by injection to groups of mice at various doses (stated in nmol/kg in Fig. 56). A further group was administered with saline (control).

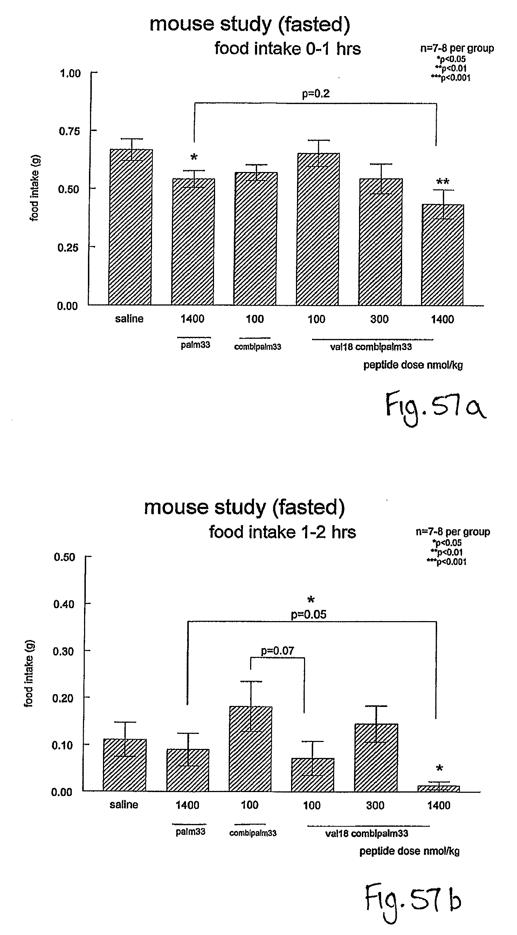

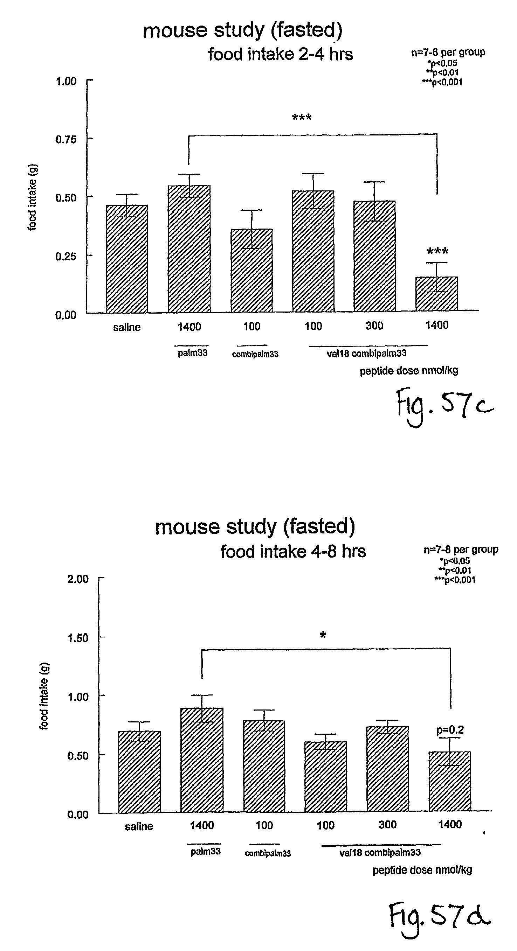

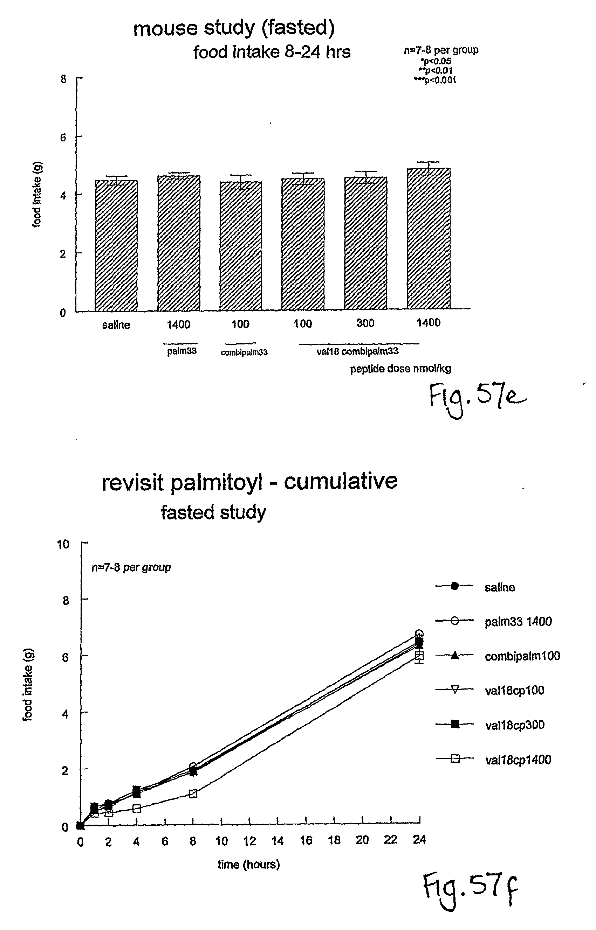

The measured food intake for each group is shown in Figs. 56a to 56d for, respectively, the intervals from 0 to 1 hour, from 1 to 2 hours, from 2 to 4 hours, and 4 to 8 hours after injection. Fig. 56e shows cumulative data. All compounds showed reduced food intake at at least some time points. Figs. 57a and 57f show the results from similar but separate experiments using the same peptides but different dosages. Figs. 57a to 57e show food intake for, respectively, the internals from 0 to 1 hours, from 1 to 2 hours, from 2 to 4 hours, from 4 to 8 hours, and from 8 to 24 hours. Fig. 57f shows cumulative data.

### Example 48: lauroyl side chain on Lys40

Groups of fasted mice were each injected with one of the following:
D-His1,Ala2,Lys33,Ala38,Ala39,Lys40-oxm(ex15-23)(ex27-33) 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys Asn Asn Ile Ala Ala Ala Lys (SEQ ID NO : 85)
D-His1,Ala2,Lys33,Ala38,Ala39,Lys40-lauroyl-oxm(ex15-23)(ex27-33)

D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys Asn Asn Ile Ala Ala Ala Lys-lauroyl (SEQ ID
NO : 105)

Lauroyl derivative was easily solublised using 50 µl 0.01 M NaOH plus an additional 50 µl 0.1M NaOH in a final volume (made up in saline) to 1.56 ml.

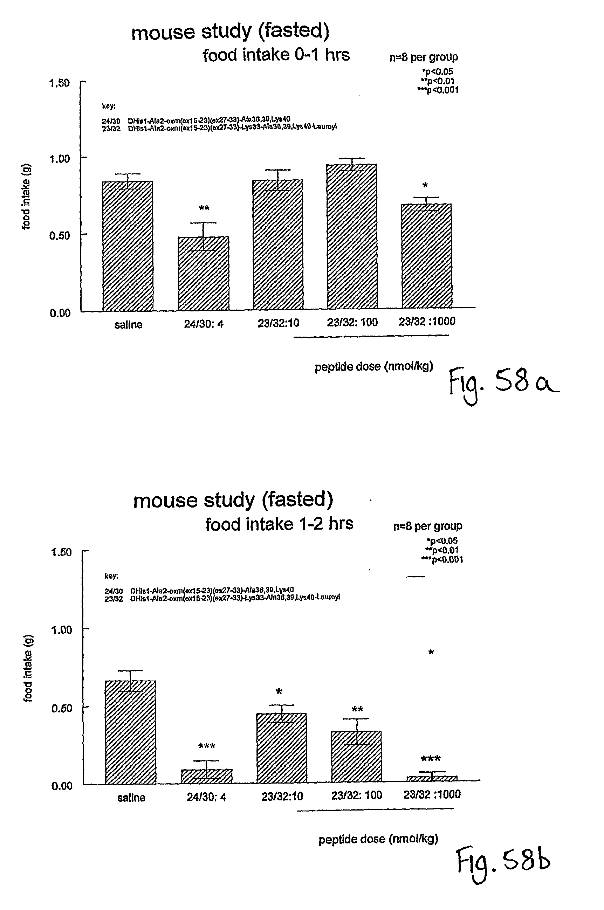

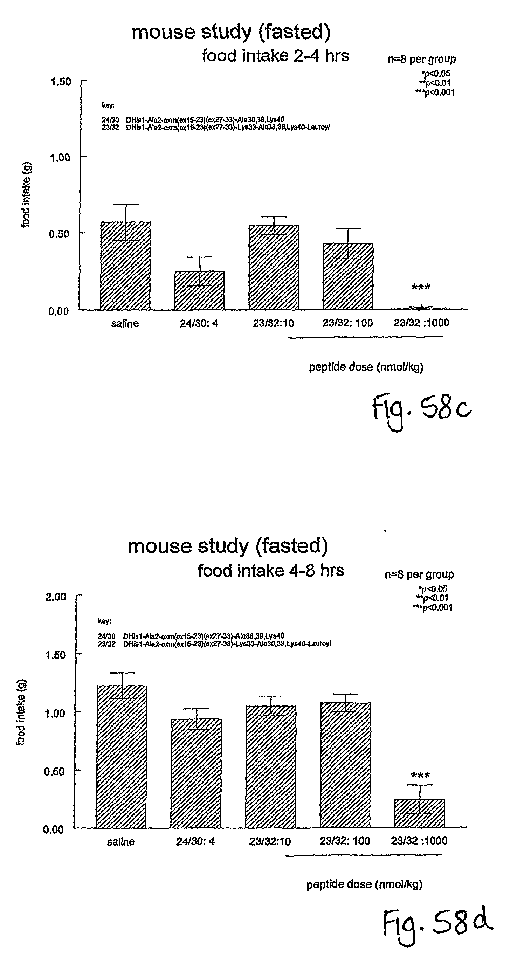

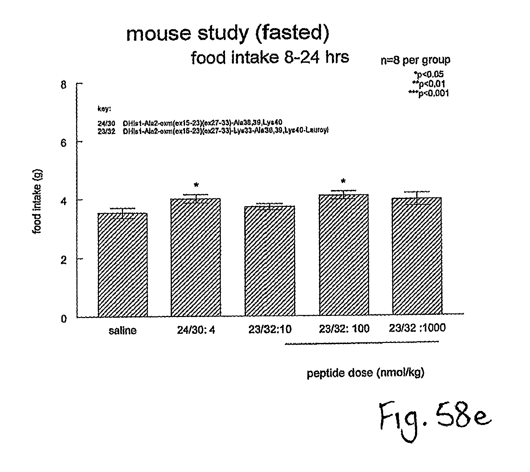

The compounds were administered by injection to groups of mice at various doses (stated in nmol/kg in Fig. 58). A further group was administered with saline (control).

The measured food intake for each group is shown in Figs. 58a to 58e for, respectively, the intervals from 0 to 1 hour, from 1 to 2 hours, from 2 to 4 hours, 4 to 8 hours, and from 8 to 24 hours after injection. All compounds showed reduced food intake.

### Example 49: Palmitoyl side chain on Lys 33 or Lys40

Groups of fasted mice were each injected with one of the following:
oxm-Lys33-palmitoyl 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Lys-palmitoyl Asn Asn Ile Ala (SEQ ID NO: 99)
D-His1Ala2,Lys33-palmitoyl-oxm(ex15-21) 
D-His Ala Gln Gly Thr Phe Thr Ser Asp Tyr Ser Lys Tyr Leu Glu Glu Glu Ala Val Arg Leu Phe
Val Gln Trp Leu Met Asn Thr Lys Arg Asn Lys-palmitoyl Asn Asn Ile Ala (SEQ ID NO : 100)
D-His1,Ala2,Lys33-palmitoyl,Ala38,39-oxm(ex15-23)(ex27-33) 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys-palmitoyl Asn Asn Ile Ala Ala Ala (SEQ ID NO :
103)
D-His1,Ala2,Lys33,Ala38,39,Lys40-palmitoyl-oxm(ex15-23)(ex27-33)

D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys Asn Asn Ile Ala Ala Ala Lys-palmitoyl (SEQ ID
NO : 104)

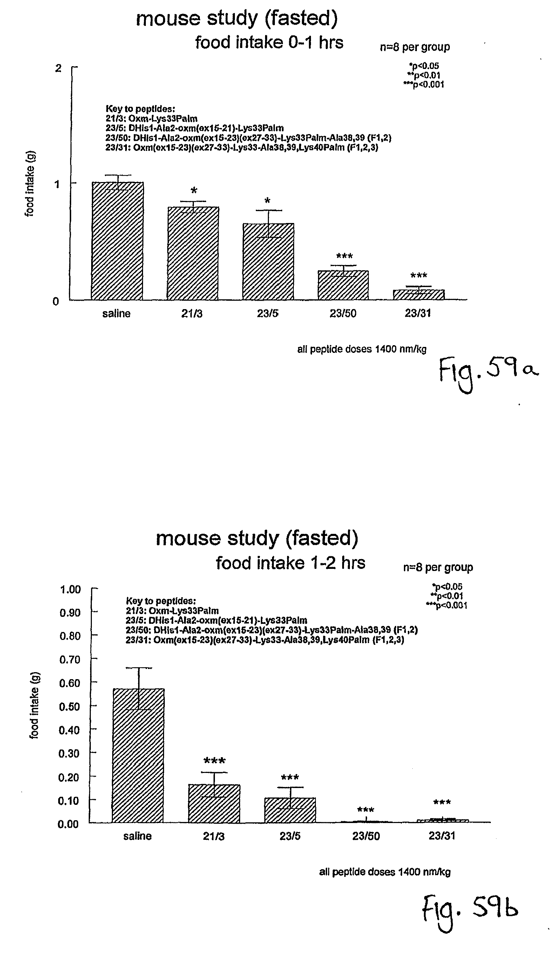

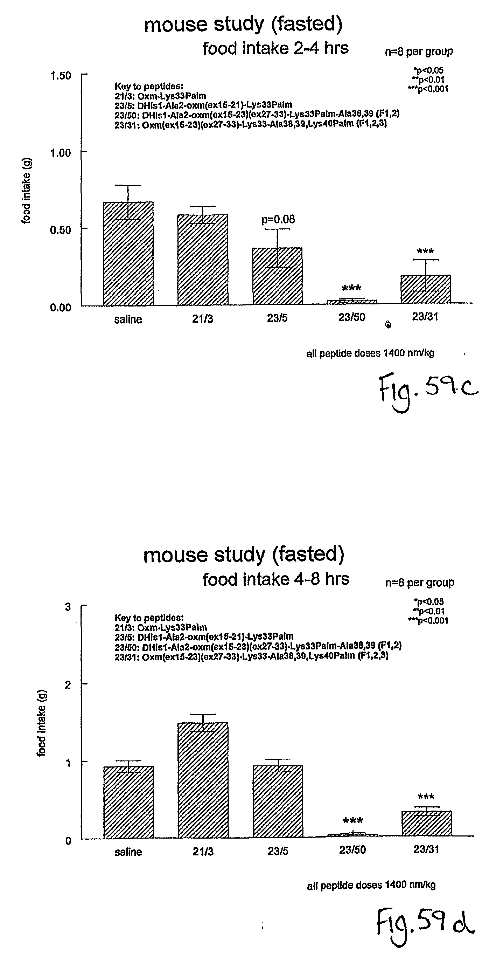

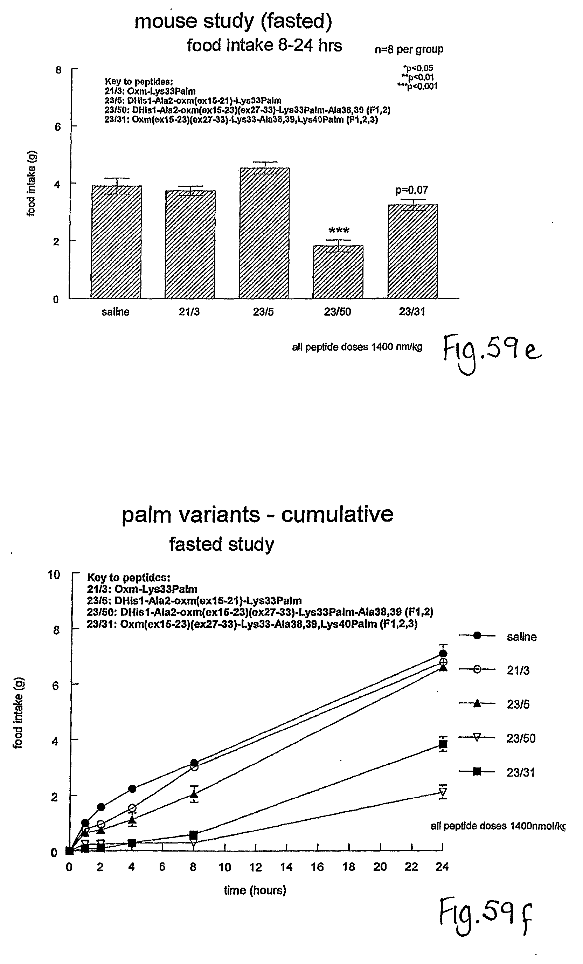

The compounds were administered by injection to groups of mice at various does (stated in nmol/kg in Fig.59). A further group was administered with saline (control).

The measured food intake for each group is shown in Figs. 59a to 59e for, respectively, the intervals from 0 to 1 hour, from 1 to 2 hours, from 2 to 4 hours, 4 to 8 hours, and from 8 to 24 hours after injection. Fig.59f shows cumulative data. All compounds showed reduced cumulative food intake. Palmitoyl derivatives show prolonged activity.

### Example 50: comparison between palmitoyl and lauroyl side chains

Groups of fasted mice were each injected with one of the following:
D-His1,Ala2,Lys33-palmitoyl,Ala38,39-oxm(ex15-23)(ex27-33) 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys-palmitoyl Asn Asn Ile Ala Ala Ala (SEQ ID NO :
103)
D-His1,Ala2,Ala38,39,Lys40-lauroyl-oxm(ex15-23)(ex27-33) 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala Lys-lauroyl (SEQ ID NO :
105)
D-His1,Ala2,Ala38,39,Lys40-palmitoyl-oxm(ex15-23)(ex27-33)

D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala Lys-palmitoyl (SEQ ID
NO : 106)

The compounds were administered by injection to groups of mice at 100nmol/kg. A further group was administered with saline (control).

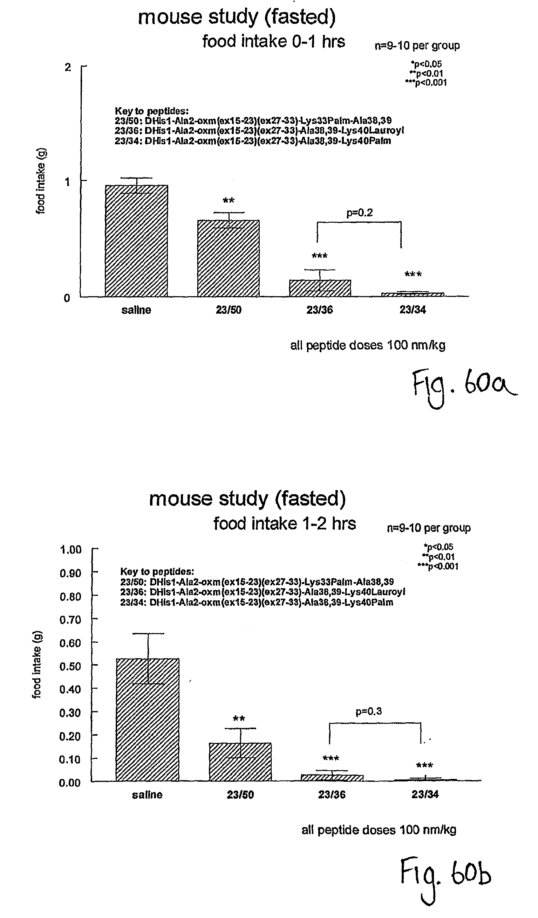

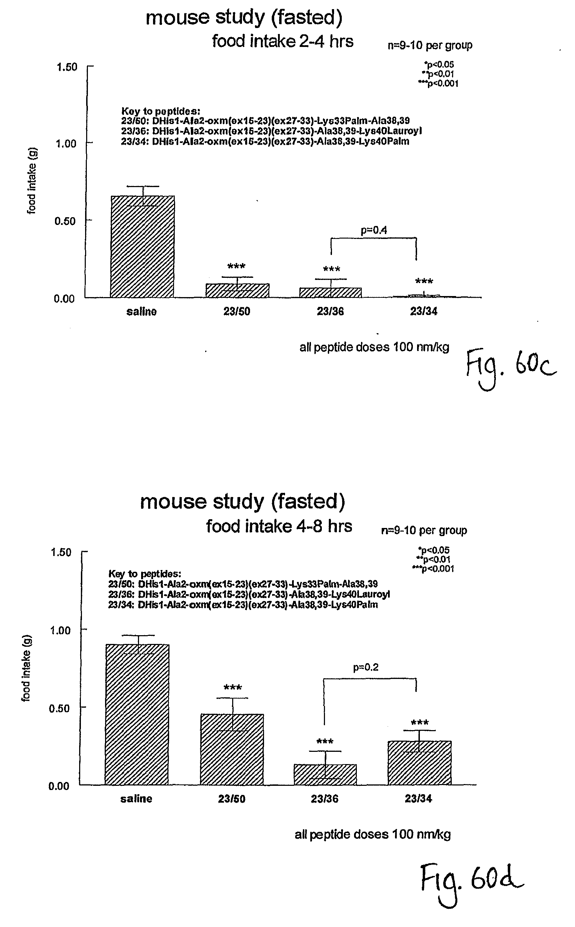

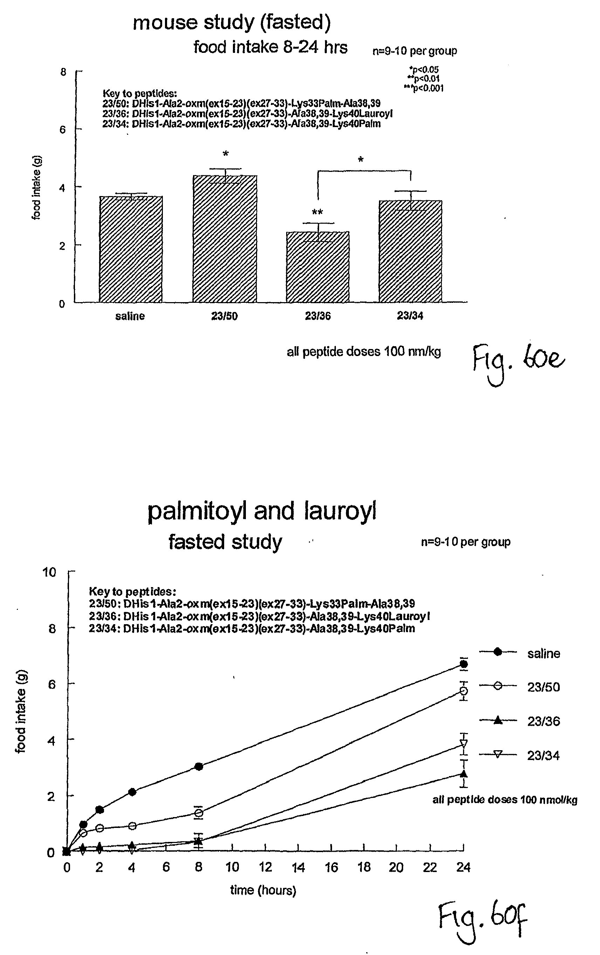

The measured food intake for each group is shown in Figs. 60a to 60e for, respectively, the intervals from 0 to 1 hour, from 1 to 2 hours, from 2 to 4 hours, 4 to 8 hours, and from 8 to 24 hours after injection. Fig.60f shows cumulative data. All compounds showed reduced cumulative food intake. The Lys40 derivatives are more potent than the Lys33 derivatives. Palmitoyl derivatives appear more potent at the earlier time points with lauroyl derivatives showing greater potency at the 4 to 8 hour time period.

### Example 51: complex variants

Groups of fasted mice were each injected with one of the following:
Ala38,Ala39,Glu40,Glu41,Lys42-oxm(ex15-23)(ex27-33) 
His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala Glu Glu Lys (SEQ ID NO :
107)
Ala38,Ala39,Lys40,Lys41,Lys42-oxm(ex15-23)(ex27-33) 
His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala Lys Lys Lys (SEQ ID NO :
108)
Ala38,Ala39,Lys40,Lys41,Lys42-palmitoyl-oxm(ex15-23)(ex27-33) 
His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala Lys Lys Lys-palmitoyl (SEQ
ID NO : 109)
D-His1,Ala2, Ala38,Ala39,Glu40,Glu41,Lys42-palmitoyl-oxm(ex15-23)(ex27-33)

D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala Glu Glu Lys-palmitoyl
(SEQ ID NO : 110)

The compounds were administered by injection to groups of mice at 100 or 400 nmol/kg. A further group was administered with saline (control).

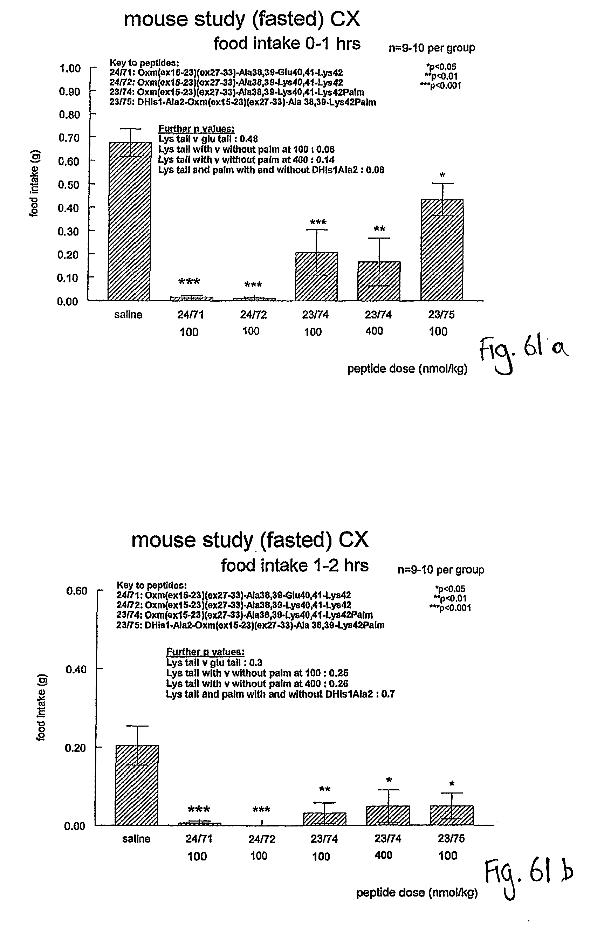

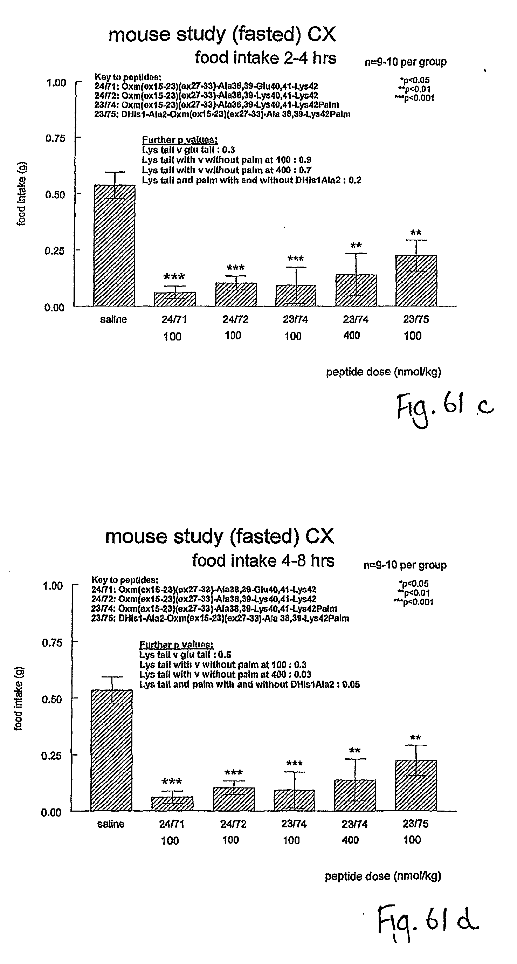

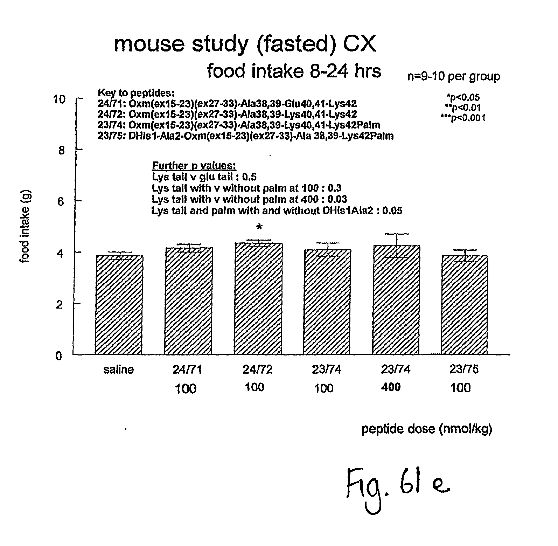

The measured food intake for each group is shown in Figs. 61a to 61e for, respectively, the intervals from 0 to 1 hour, from 1 to 2 hours, from 2 to 4 hours, 4 to 8 hours, and from 8 to 24 hours after injection. All compounds showed a reduction in food intake.

### Example 52: complex variants tested at different doses

Groups of fasted mice were each injected with one of the following:
Ala38,Ala39,Glu40,Glu41,Lys42-oxm(ex15-23)(ex27-33) 
His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala Glu Glu Lys (SEQ ID NO :
107)
Ala38,Ala39,Glu40,Glu41,Lys42-lauroyl-oxm(ex15-23)(ex27-33) 
His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala Glu Glu Lys-lauroyl (SEQ ID
NO : 111)
Ala38,Ala39,Glu40,Glu41,Lys42-palmitoyl-oxm(ex15-23)(ex27-33)

His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala Glu Glu Lys-palmitoyl (SEQ
ID NO : 112)

The compounds were administered by injection to groups of mice at 20 or 50 nmol/kg. A further group was administered with saline (control).

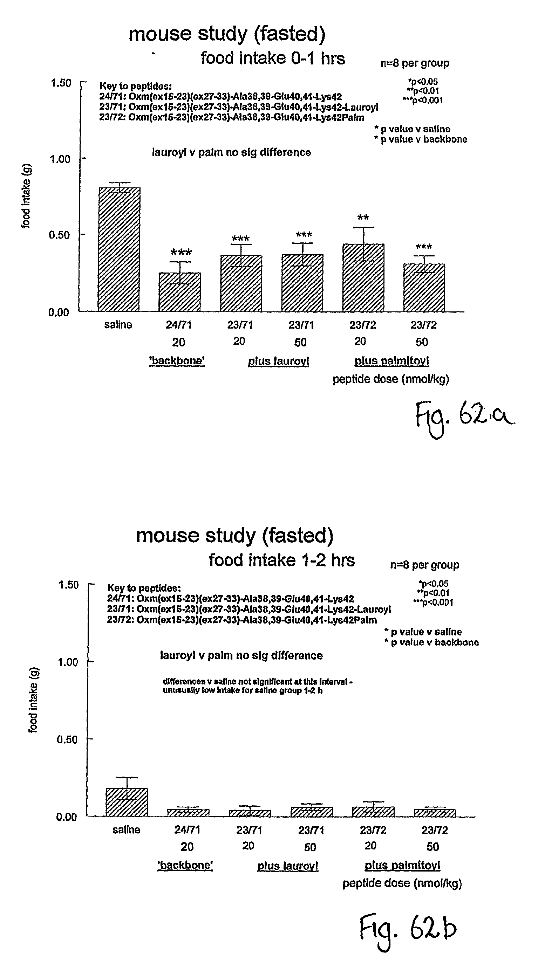

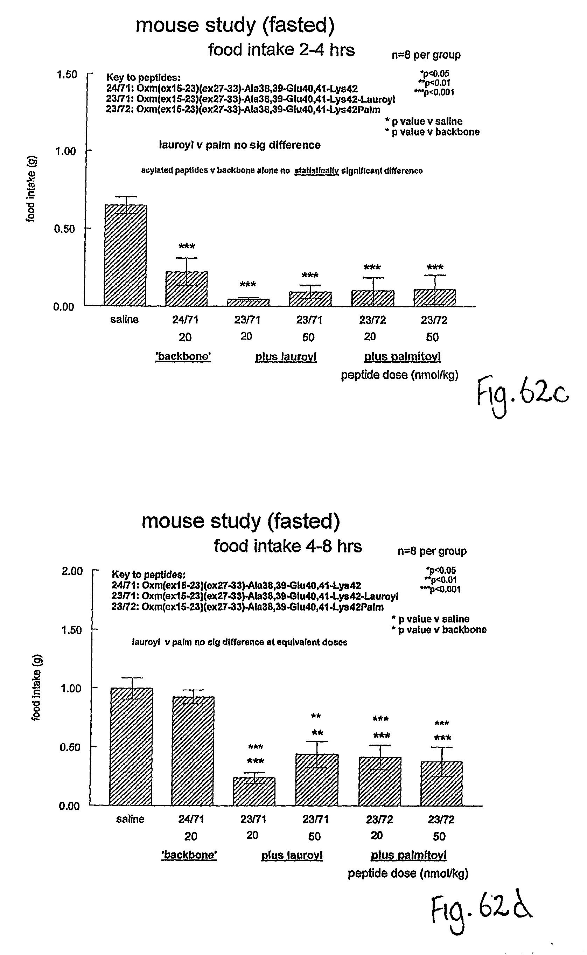

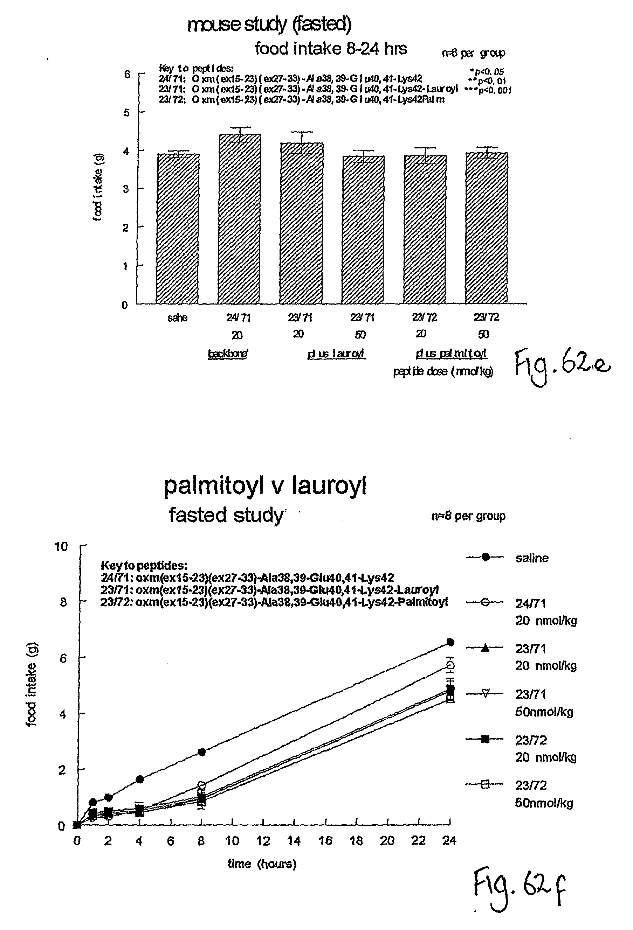

The measured food intake for each group is shown in Figs. 62a to 62e for, respectively, the intervals from 0 to 1 hour, from 1 to 2 hours, from 2 to 4 hours, 4 to 8 hours, and from 8 to 24 hours after injection. All compounds showed a reduction in food intake.

### Example 53: complex variants tested at further doses

Groups of fasted mice were each injected with one of the following:
Ala38,Ala39,Glu40,Glu41,Lys42-oxm(exIS-23)(ex27-33) 
His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala Glu Glu Lys (SEQ ID NO :
107)
Ala38,Ala39,Glu40,Glu41,Lys42-lauroyl-oxm(ex15-23)(ex27-33) 
His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala Glu Glu Lys-lauroyl (SEQ ID
NO : 111)
Ala38,Ala39,Glu40,Glu41,Lys42-palmitoyl-oxm(ex15-23)(ex27-33)

His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala Glu Glu Lys-palmitoyl (SEQ
ID NO : 112)

The compounds were administered by injection to groups of mice at 5 or 10 nmol/kg. A further group was administered with saline (control).

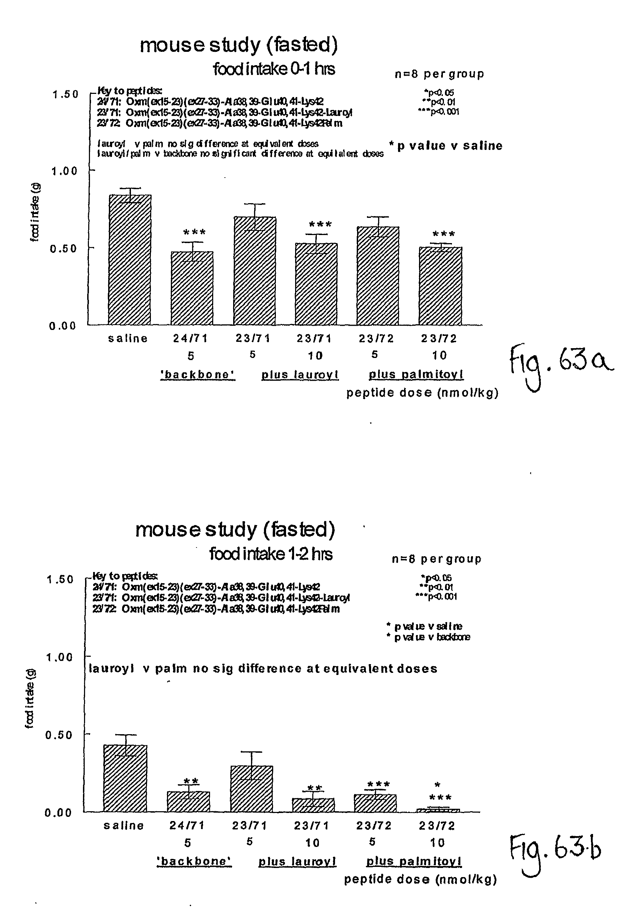

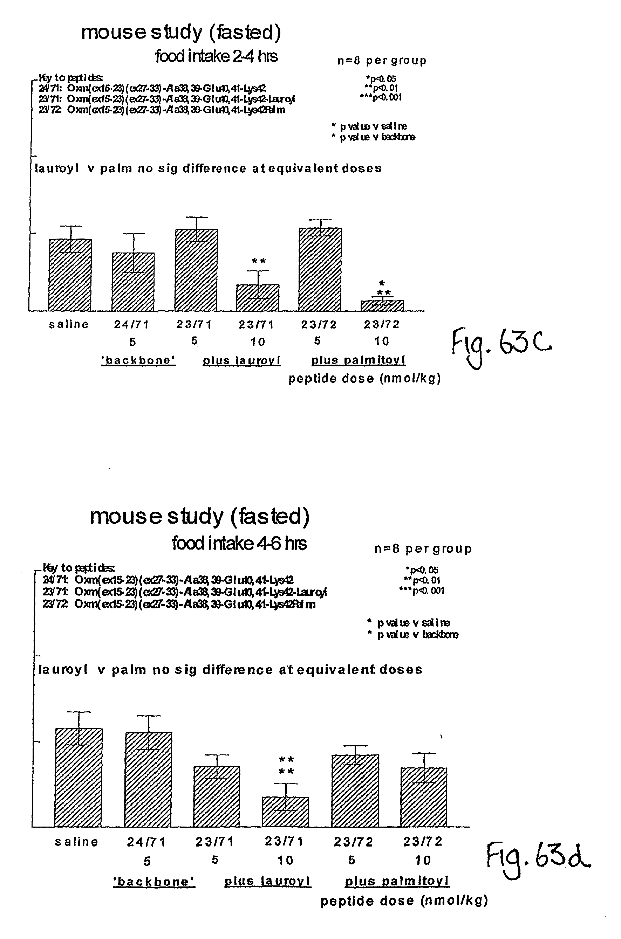

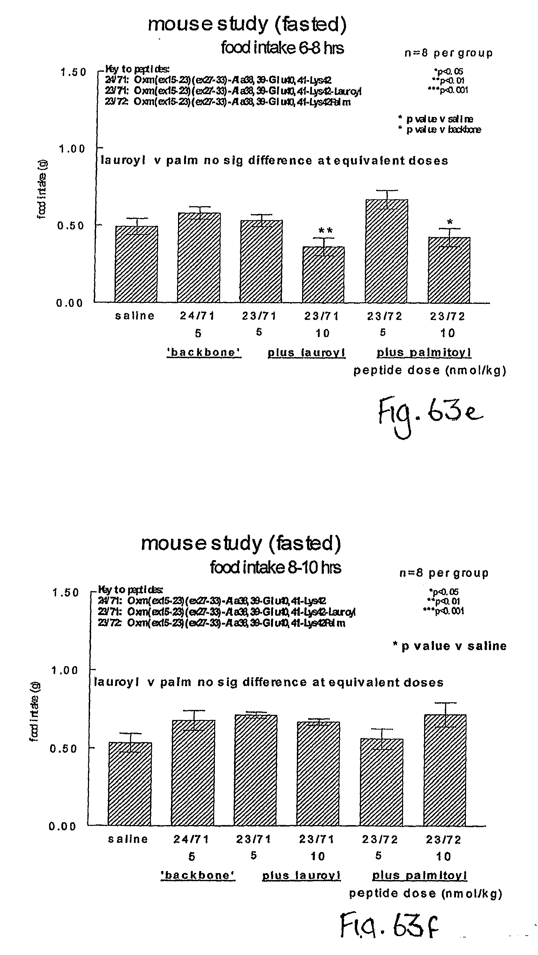

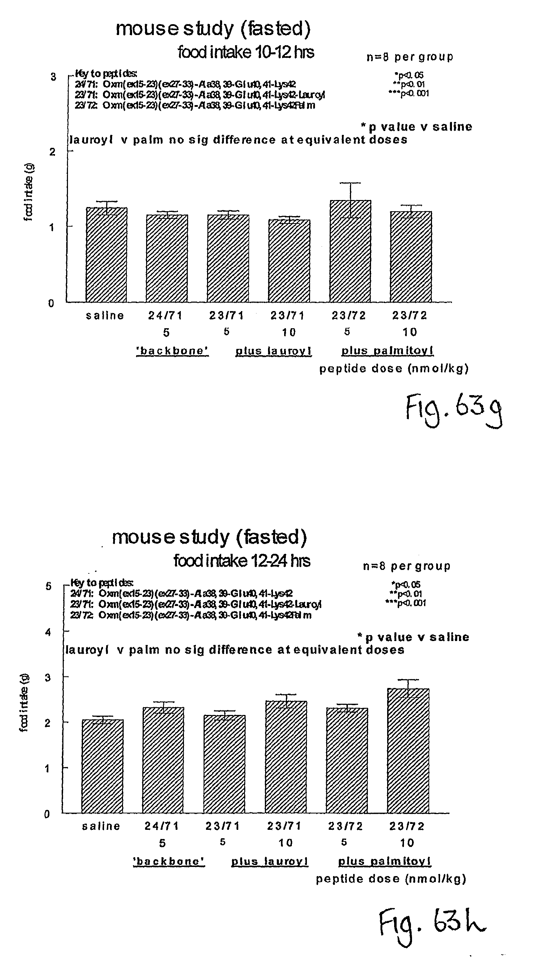

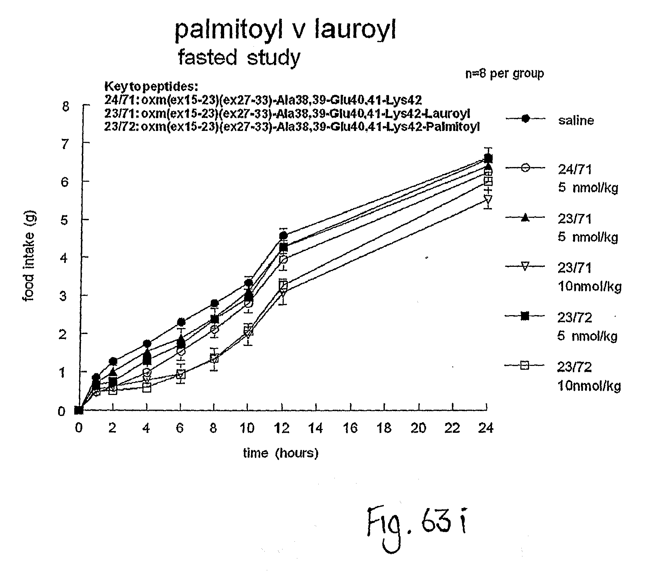

The measured food intake for each group is shown in Figs. 63a to 63h for, respectively, the intervals from 0 to 1 hour, from 1 to 2 hours, from 2 to 4 hours, 4 to 6 hours, 6 to 8 hours, 8 to 10 0 hours, 10 to 12 hours and from 12 to 24 hours after injection. Fig. 63i shows cumulative food intake data. All compounds showed a reduction in food intake.

### Example 54: acyl side chain comparisons

Groups of fasted mice were each injected with one of the following:
D-His1,Ala2,Lys33,Ala38,Ala39,Lys40-oxm (ex15-23)(ex27-33) 
D-His Ala Gln Gly Thr Phe Thr Ser Asp Tyr Ser Lys Tyr Leu Glu Glu Glu Ala Val Arg Leu Phe
Val Gln Trp Leu Lys Asn Gly Gly Pro Ser Lys Asn Asn Ile Ala Ala Ala Lys (SEQ ID NO : 85)
D-His1,Ala2,Lys33,Ala38,Ala39,Lys40-lauroyl-oxm (ex15-23)(ex27-33) 
D-His Ala Gln Gly Thr Phe Thr Ser Asp Tyr Ser Lys Tyr Leu Glu Glu Glu Ala Val Arg Leu Phe
Val Gln Trp Leu Lys Asn Gly Gly Pro Ser Lys Asn Asn Ile Ala Ala Ala Lys-lauroyl (SEQ ID
NO : 104)
D-His1,Ala2,Ala38,Ala39,Lys40-palmitoyl-oxm (ex15-23)(ex27-33) 
D-His Ala Gln Gly Thr Phe Thr Ser Asp Tyr Ser Lys Tyr Leu Glu Glu Glu Ala Val Arg Leu Phe
Val Gln Trp Leu Lys Asn Gly Gly Pro Ser Ser Asn Asn Ile Ala Ala Ala Lys-palmitoyl (SEQ ID
NO : 106)
D-His1,Ala2,Ala38,Ala39,Lys40-lauroyl-oxm (ex15-23)(ex27-33) 
D-His Ala Gln Gly Thr Phe Thr Ser Asp Tyr Ser Lys Tyr Leu Glu Glu Glu Ala Val Arg Leu Phe
Val Gln Trp Leu Lys Asn Gly Gly Pro Ser Ser Asn Asn Ile Ala Ala Ala Lys-lauroyl (SEQ ID
NO : 105)
Ala38,Ala39,Glu40,41,Lys42-lauroyl-oxm (ex15-23)(ex27-33)

His Ser Gln Gly Thr Phe Thr Ser Asp Tyr Ser Lys Tyr Leu Glu Glu Glu Ala Val Arg Leu Phe Val
Gln Trp Leu Lys Asn Gly Gly Pro Ser Ser Asn Asn Ile Ala Ala Ala Glu Glu Lys-lauroyl (SEQ ID
NO : 111)

The compounds were administered by injection to groups of mice at 10 nmol/kg. A further group was administered with saline (control).

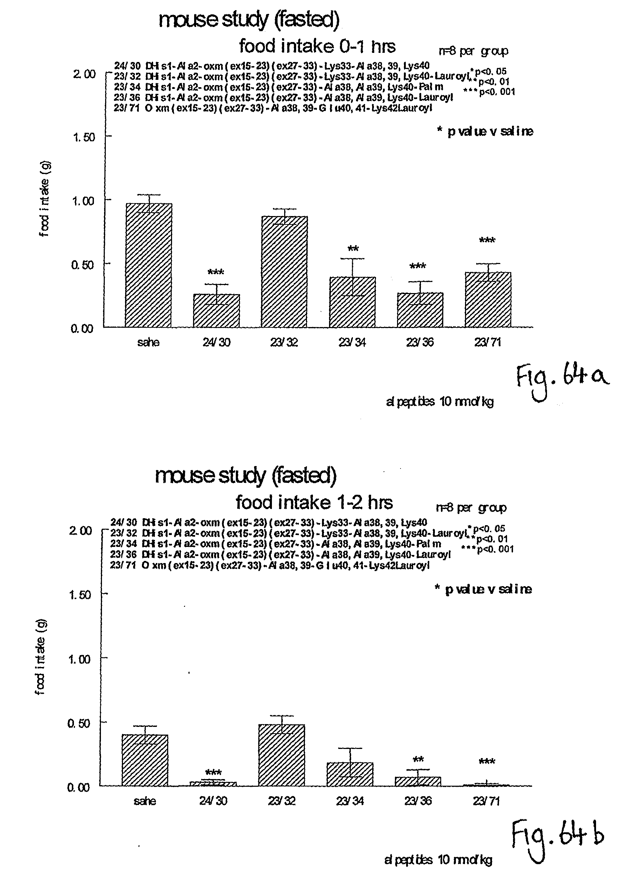

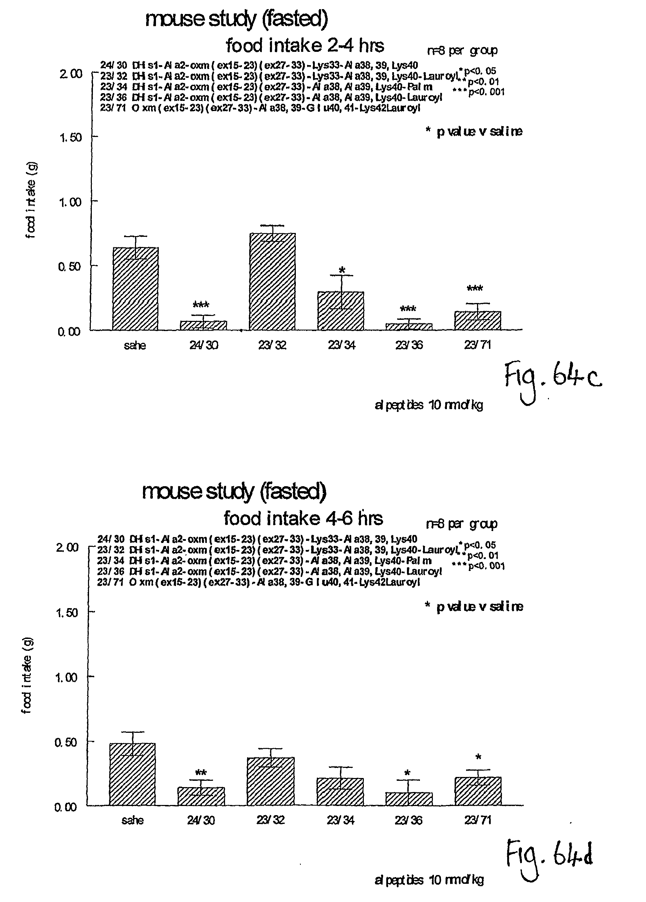

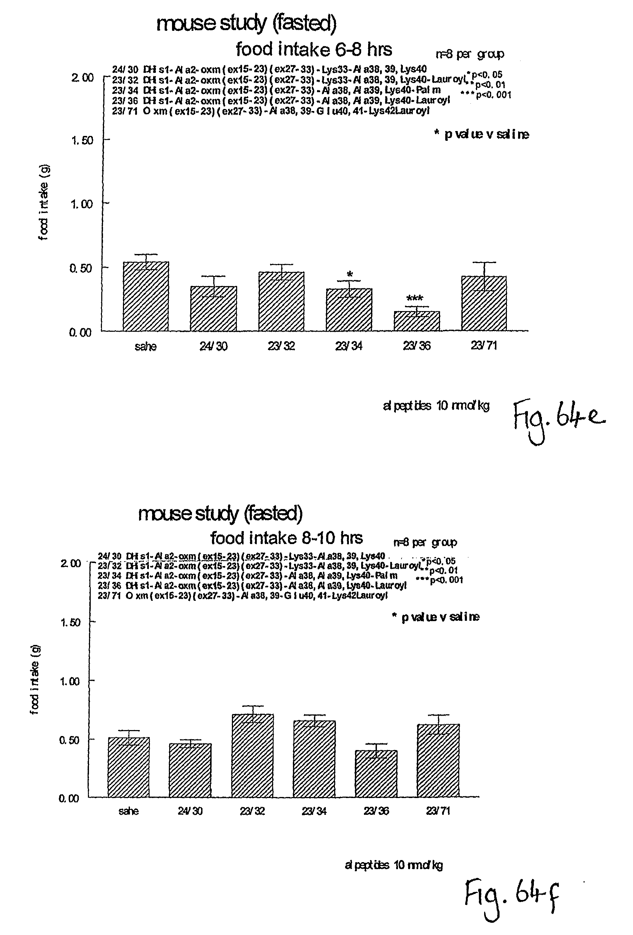

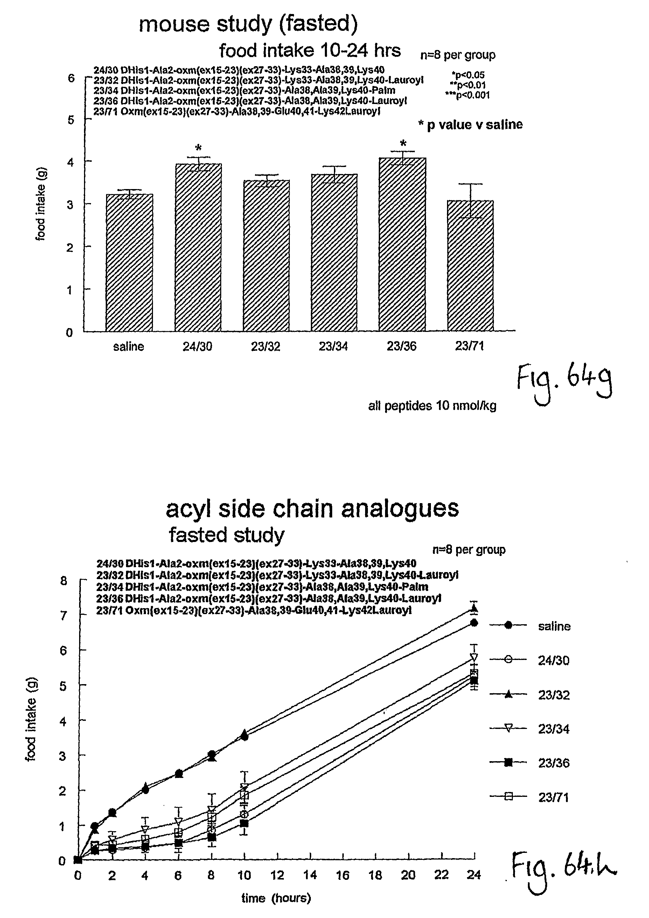

The measured food intake for each group is shown in Figs. 64a to 64g for, respectively, the intervals from 0 to 1 hour, from 1 to 2 hours, from 2 to 4 hours, 4 to 6 hours, 6 to 8 hours, 8 to 10 hours and 10 to 24 hours after injection. Fig. 64h shows cumulative data. All compounds (with the exception of D-His1,Ala2,Lys33,Ala38,Ala39,Lys40-lauroyl-oxm (ex15-21)(ex27-33), which had previously been shown to work)
showed a reduction in food intake.

### Example 55: acyl side chain comparisons

Groups of fasted mice were each injected with one of the following:
D-His1,Ala2,Lys33,Ala38,Ala39,Lys40-oxm (ex15-23)(ex27-33) 
D-His Ala Gln Gly Thr Phe Thr Ser Asp Tyr Ser Lys Tyr Leu Glu Glu Glu Ala Val Arg Leu Phe
Val Gln Trp Leu Lys Asn Gly Gly Pro Ser Lys Asn Asn Ile Ala Ala Ala Lys (SEQ ID NO : 85)
D-His1,Ala2,Lys33,Ala38,Ala39,Lys40-lauroyl-oxm (ex15-23)(ex27-33) 
D-His Ala Gln Gly Thr Phe Thr Ser Asp Tyr Ser Lys Tyr Leu Glu Glu Glu Ala Val Arg Leu Phe
Val Gln Trp Leu Lys Asn Gly Gly Pro Ser Lys Asn Asn Ile Ala Ala Ala Lys-lauroyl (SEQ ID
NO : 104)
D-His1,Ala2,Ala38,Ala39,Lys40-lauroyl-oxm (ex15-23)(ex27-33)

D-His Ala Gln Gly Thr Phe Thr Ser Asp Tyr Ser Lys Tyr Leu Glu Glu Glu Ala Val Arg Leu Phe
Val Gln Trp Leu Lys Asn Gly Gly Pro Ser Ser Asn Asn Ile Ala Ala Ala Lys-lauroyl (SEQ ID
NO : 105)

The compounds were administered by injection to groups of mice at 3, 7 or 10 nmol/kg. A further group was administered with saline (control).

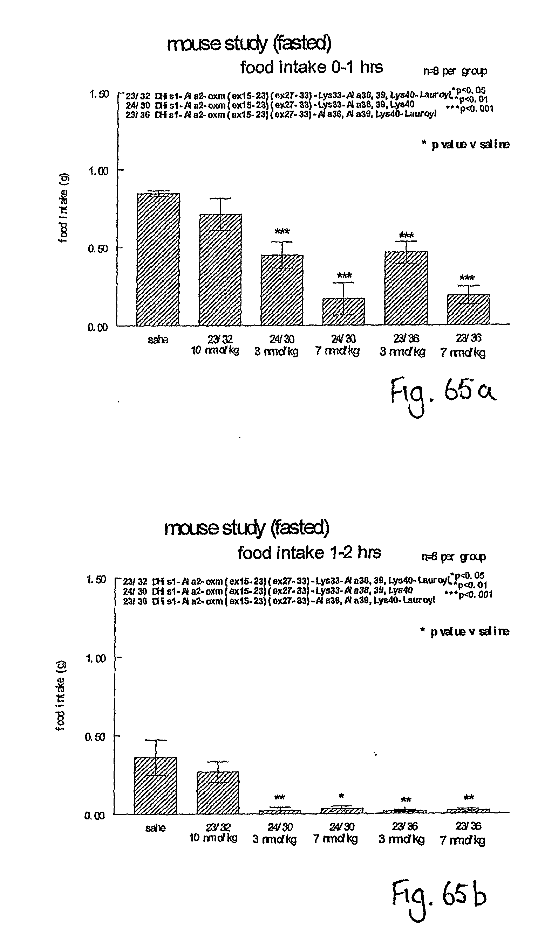

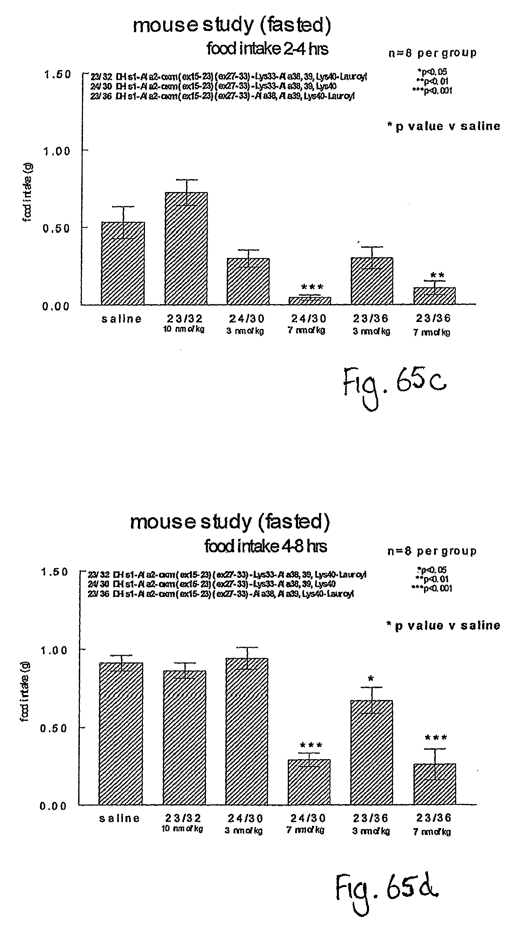

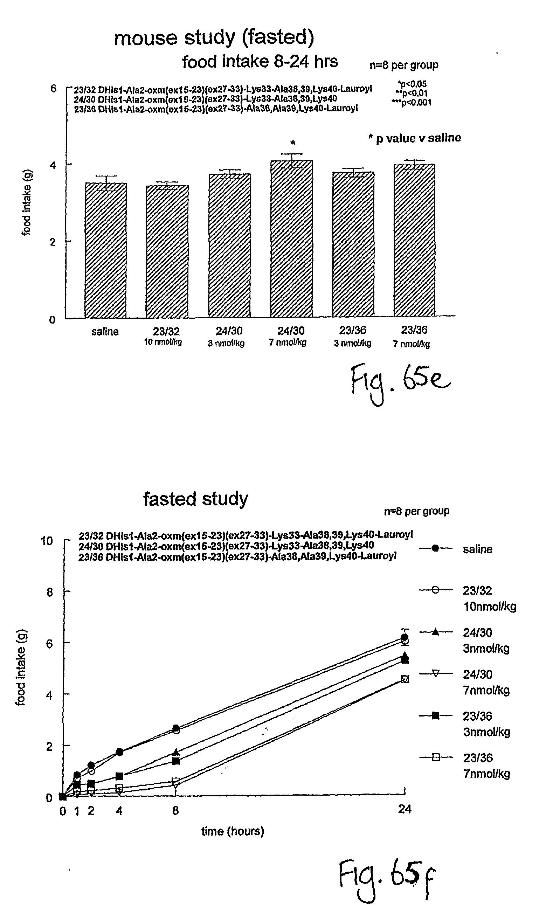

The measured food intake for each group is shown in Figs. 65a to 65e for, respectively, the intervals from 0 to 1 hour, from 1 to 2 hours, from 2 to 4 hours, 4 to 8 hours and 8 to 24 hours after injection. Fig. 65f shows cumulative data. All compounds
showed a reduction in food intake.

**Example 56: acyl and PEG side chain comparisons**

Groups of fasted mice were each injected with one of the following:
D-His1,Ala2-oxm (ex15-23)(ex27-33)-Lys33,Ala38,Ala39,Glu40,Glu41-Lys42 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys Asn Asn Ile Ala Ala Ala Glu Glu Lys (SEQ ID
NO : 113)
D-His1,Ala2,Asp3,Gln16, Val18-oxm(ex15-23)(ex27-33)Lys33,Ala38,Ala39,Glu40, Glu41,Lys42 
D-His Ala Asp Gly Thr Phe Thr Ser Asp Tyr Ser Lys Tyr Leu Glu Gln Glu Val Val Arg Leu Phe
Ile Gln Trp Leu Lys Asn Gly Gly Pro Ser Lys Asn Asn Ile Ala Ala Ala Glu Glu Lys (SEQ ID
NO : 114)
D-His1,Ala2,Asp3,Gln16, Val18-oxm(ex15-23)(ex27-33)Lys33,Ala38,Ala39,Glu40, Glu41,Lys42-Octanoyl 
D-His Ala Asp Gly Thr Phe Thr Ser Asp Tyr Ser Lys Tyr Leu Glu Gln Glu Val Val Arg Leu Phe
Ile Gln Trp Leu Lys Asn Gly Gly Pro Ser Lys Asn Asn Ile Ala Ala Ala Glu Glu Lys-Octanoyl
(SEQ ID NO : 115)
D-His1,Ala2,Asp3,Gln16, Val18-oxm(ex15-23)(ex27-33)Lys33,Ala38,Ala39,Glu40, Glu41,Lys42-PEG

D-His Ala Asp Gly Thr Phe Thr Ser Asp Tyr Ser Lys Tyr Leu Glu Gln Glu Val Val Arg Leu Phe
Ile Gln Trp Leu Lys Asn Gly Gly Pro Ser Lys Asn Asn Ile Ala Ala Ala Glu Glu Lys-PEG (SEQ
ID NO : 116)

The compounds were administered by injection to groups of mice at 6 or 20 nmol/kg. A further group was administered with saline (control).

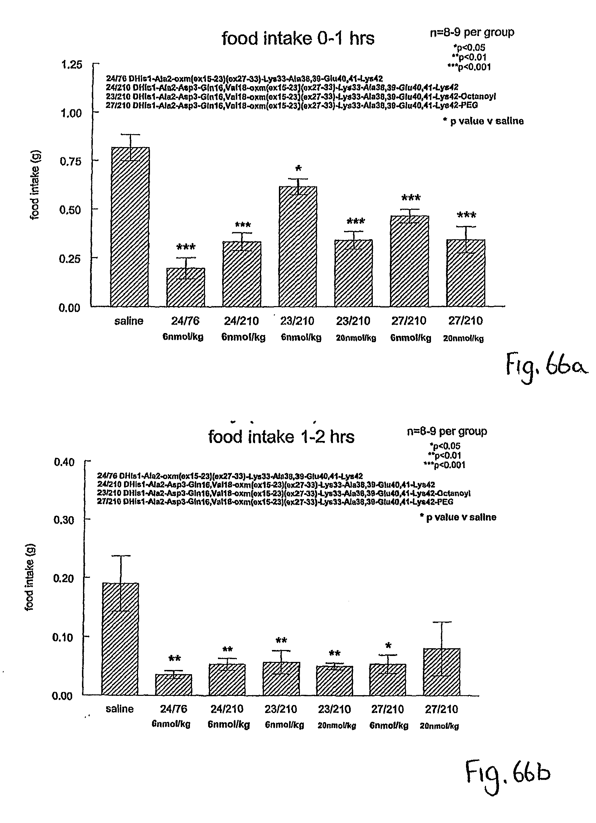

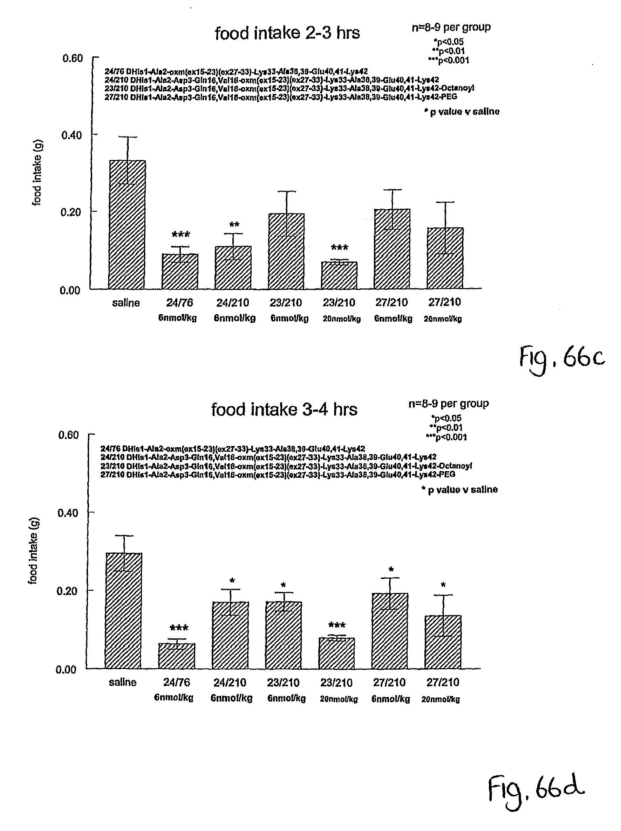

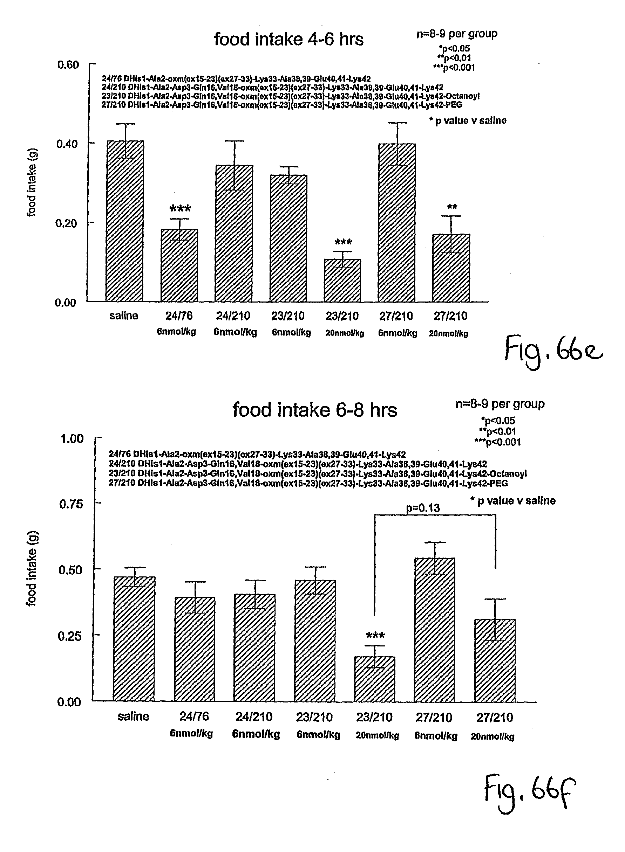

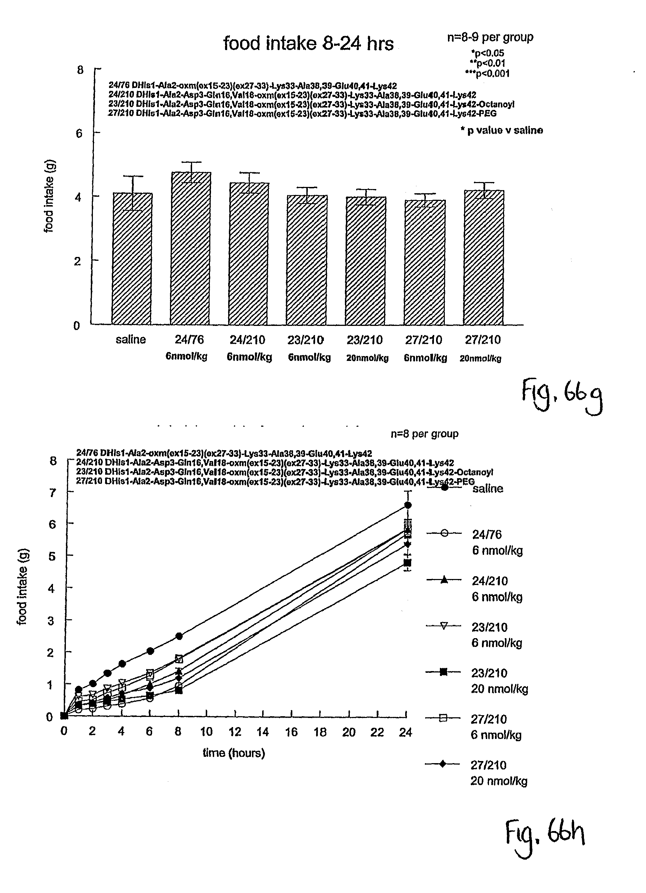

The measured food intake for each group is shown in Figs. 66a to 66g for, respectively, the intervals from 0 to 1 hour, from 1 to 2 hours, from 2 to 3 hours, from 3 to 4 hours, 4 to 6 hours, from 6 to 8 hours and 8 to 24 hours after injection. Fig. 66h shows cumulative data. All compounds caused a reduction in cumulative food intake in comparison to the saline control.

### Example 57: acyl side chain comparisons

Groups of fasted mice were each injected with one of the following:
D-His1,Ser2,Asp3-oxm(ex15-23)(ex27-33)Lys33,Ala38,Ala39,Glu4O, Glu41,Lys42 
D-His Ser Asp Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys Asn Asn Ile Ala Ala Ala Glu Glu Lys (SEQ ID
NO : 117)
D-His1,Ser2,Asp3,Val18-oxm(ex15-23)(ex27-33)Lys33,Ala38,Ala39,Glu4O, Glu41,Lys42 
D-His Ser Asp Gly Thr Phe Thr Ser Asp Tyr Ser Lys Tyr Leu Glu Glu Glu Val Val Arg Leu Phe
Ile Gln Trp Leu Lys Asn Gly Gly Pro Ser Lys Asn Asn Ile Ala Ala Ala Glu Glu Lys (SEQ ID
NO : 118)
D-His1,Ser2,Asp3,Val18-oxm(ex15-23)(ex27-33)Lys33,Ala38,Ala39,Glu4O, Glu41,Lys42-Lauroyl 
D-His Ser Asp Gly Thr Phe Thr Ser Asp Tyr Ser Lys Tyr Leu Glu Glu Glu Val Val Arg Leu Phe
Ile Gln Trp Leu Lys Asn Gly Gly Pro Ser Lys Asn Asn Ile Ala Ala Ala Glu Glu Lys-Lauroyl
(SEQ ID NO : 119)
D-His1,Ser2,Asp3-oxm(ex15-23)(ex27-33)Lys33,Ala38,Ala39,Glu40, Glu41,Lys42-Lauroyl

D-Ser Ala Asp Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys Asn Asn Ile Ala Ala Ala Glu Glu Lys-Lauroyl
(SEQ ID NO : 120)

The compounds were administered by injection to groups of mice at 6 or 20 nmol/kg. A further group was administered with saline (control).

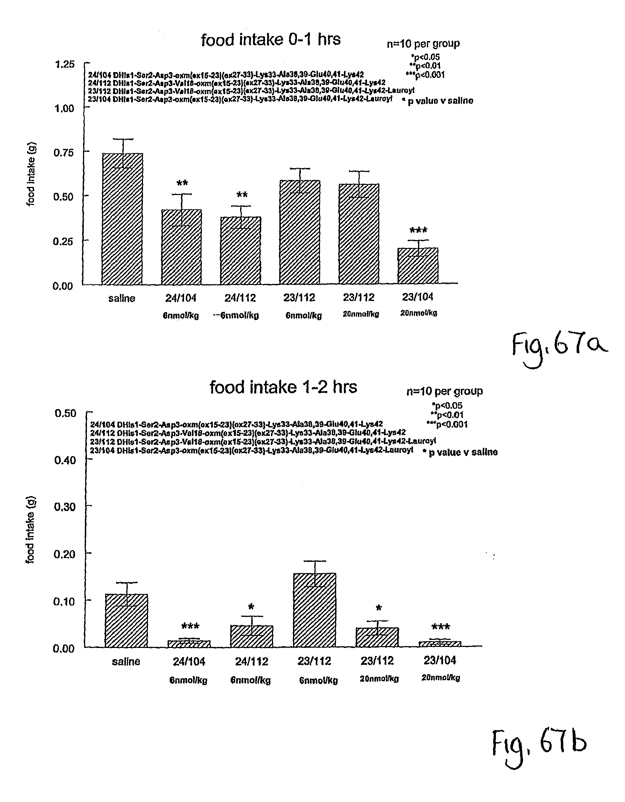

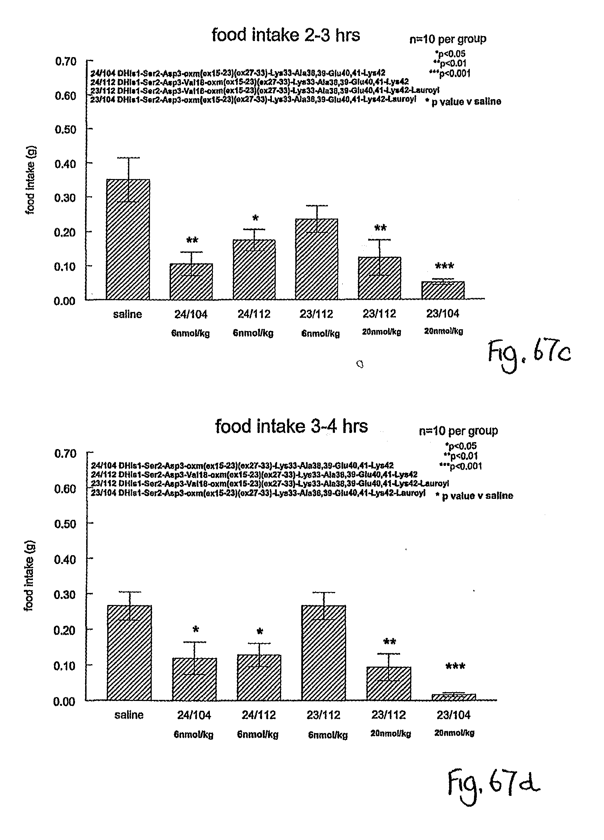

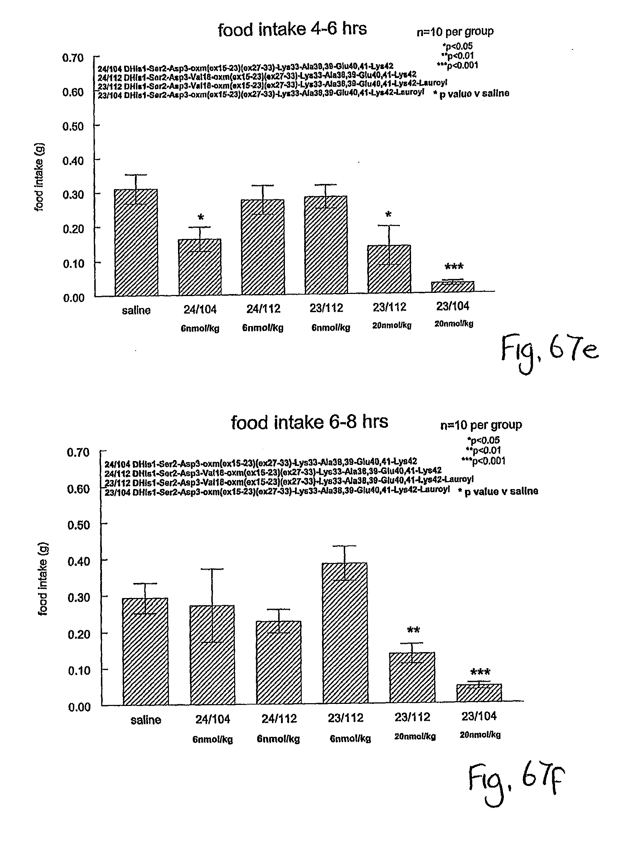

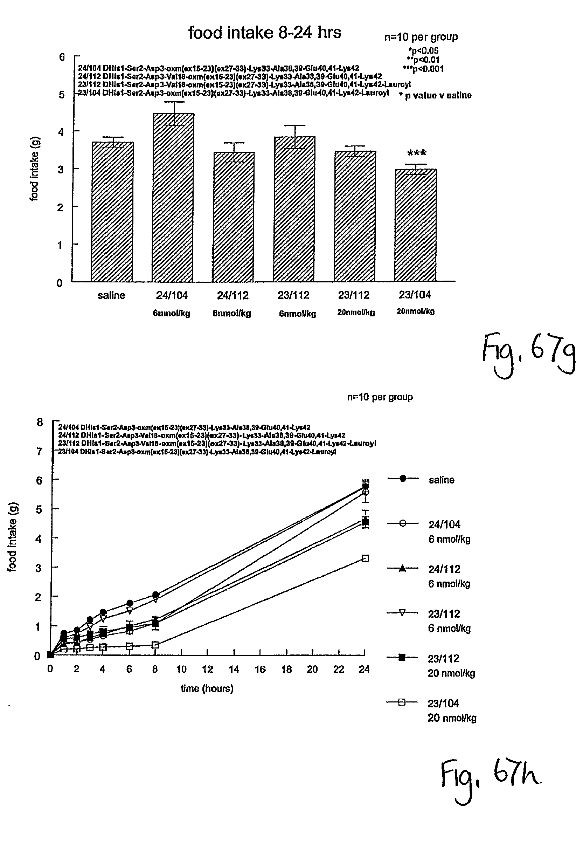

The measured food intake for each group is shown in Figs. 67a to 67g for, respectively, the intervals from 0 to 1 hour, from 1 to 2 hours, from 2 to 3 hours, from 3 to 4 hours, 4 to 6 hours, from 6 to 8 hours and 8 to 24 hours after injection. Fig. 67h shows cumulative data. All compounds caused a reduction in food intake at some of the time points. In particular, the D-His1,Ser2,Asp3,Val18-oxm(ex15-23)(ex27-33)Lys33,Ala38,Ala39, Glu40, Glu41,Lys42-Lauroyl and D-His1,Ser2,Asp3-oxm(ex15-23)(ex27-33)Lys33, Ala38,Ala39,Glu40, Glu41,Lys42-Lauroyl analogues showed a markedly decreased cumulative food intake in comparison to the saline control.

### Example 58

Groups of fasted mice were each injected with one of the following:
D-His1,Ala2-oxm 
D-His Ala Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala (SEQ ID NO: 25)
D-His1,Val2-oxm

D-His Val Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala (SEQ ID NO: 121)

The compounds were administered by injection to groups of mice at 1400 nmol/kg. Further groups were administered with saline and human oxm as negative and positive controls respectively.

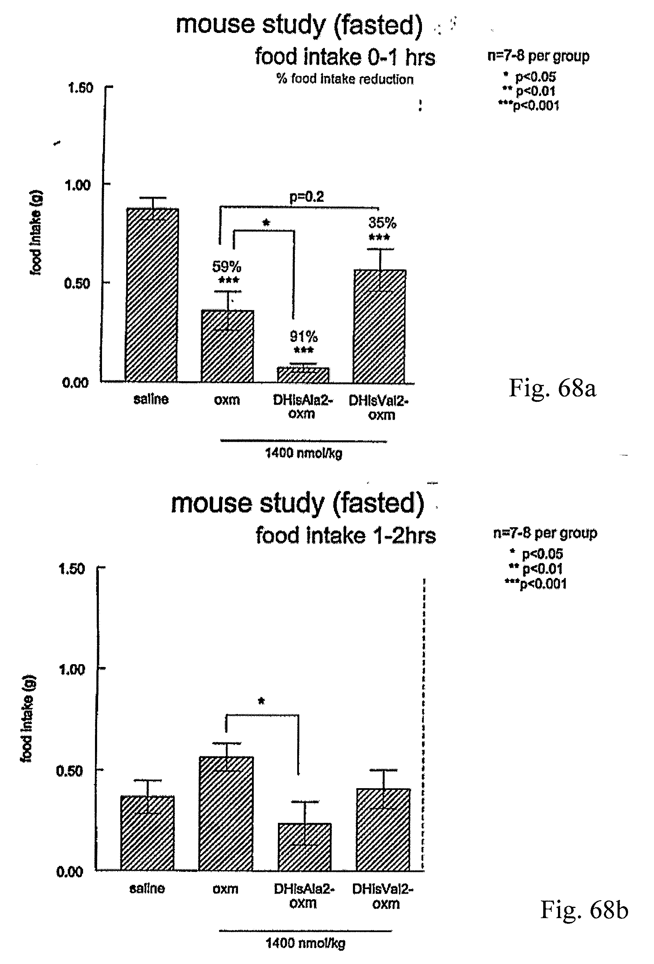

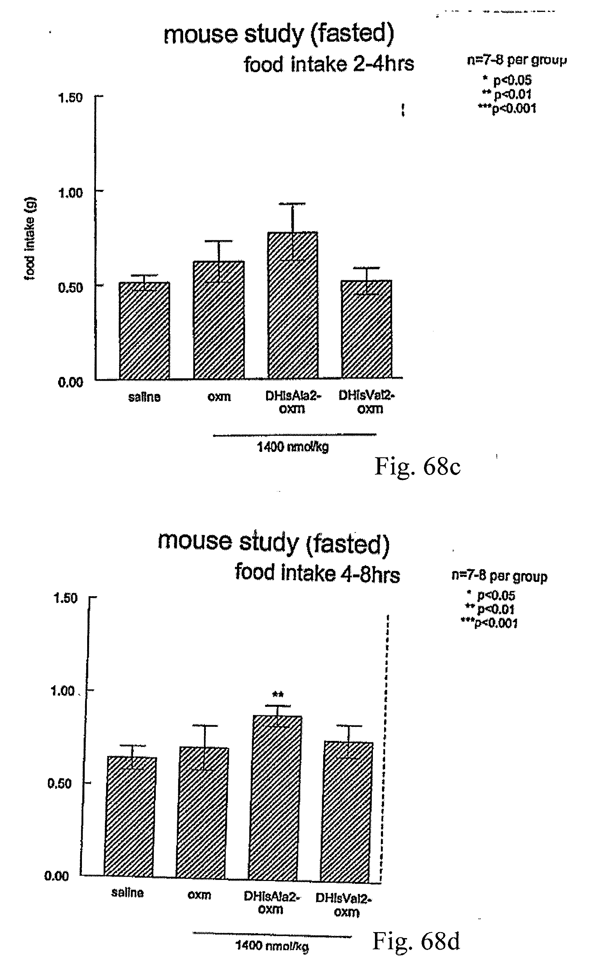

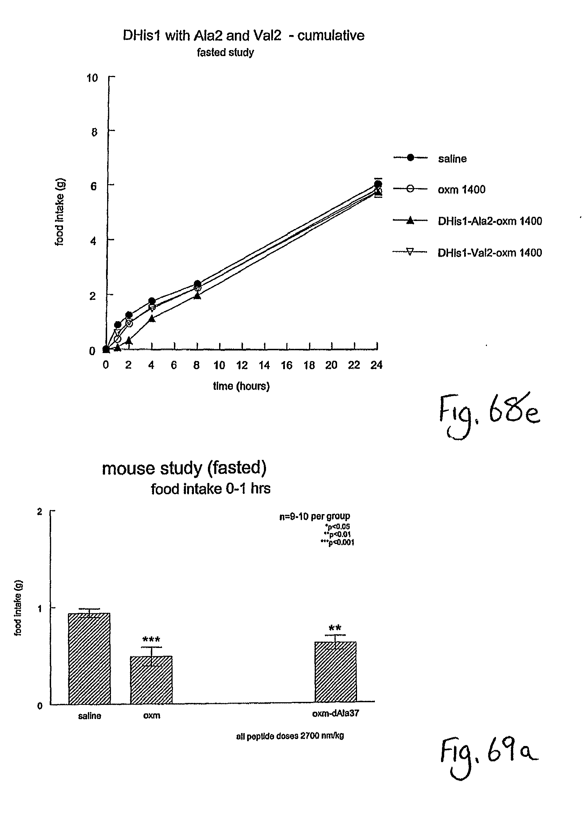

The measured food intake for each group is shown in Figs. 68a to 68d for, respectively, the intervals from 0 to 1 hour, from 1 to 2 hours, from 2 to 4 hours, 4 to 8 hours and 8 to 24 hours after injection. Fig. 68e shows cumulative data. Both compounds caused a reduction in food intake at some of the time points.

### Example 59

Groups of fasted mice were each injected with the following:
D-Ala37-oxm

His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile D-Ala (SEQ ID NO: 122)

The compound was administered by injection to groups of mice at 2700 nmol/kg. Further groups were administered with saline and human oxm as negative and positive controls respectively.

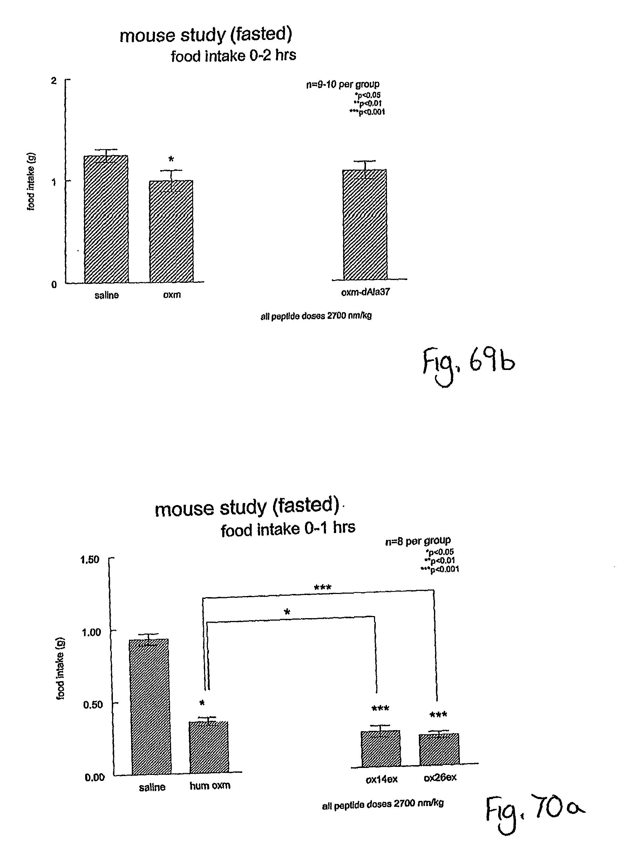

The measured food intake for each group is shown in Figs. 69a and 69b for, respectively, the intervals from 0 to 1 hour and from 0 to 2 hours. The D-Ala37-oxm caused a reduction in food intake in comparison to the saline control.

### Example 60

Groups of fasted mice were each injected with the following:
oxm(xx15-39) "ox14ex": 
His Ser Gln Gly Thr Phe Thr Ser Asp Tyr Ser Lys Tyr Leu Glu Glu Glu Ala Val Glu Leu Phe Ile
Glu Trp Leu Lys Asn Gly Gly Pro Ser Ser Gly Ala Pro Pro Pro Ser (SEQ ID NO: 123)
Glu20-oxm(xx27-39) = "ox26ex":

His Ser Gln Gly Thr Phe Thr Ser Asp Tyr Ser Lys Tyr Leu Asp Ser Arg Arg Ala Glu Asp Phe
Val Gln Trp Leu Lys Asn Gly Gly Pro Ser Ser Gly Ala Pro Pro Pro Ser (SEQ ID NO: 124)

The compounds were administered by injection to groups of mice at a dose of 2700 nmol/kg. Further groups were administered either human oxm at the same dosage (for comparison purposes) or saline (control). Both compounds showed reduced food intake in comparison to both the saline and human oxyntomodulin experiments at some time points.

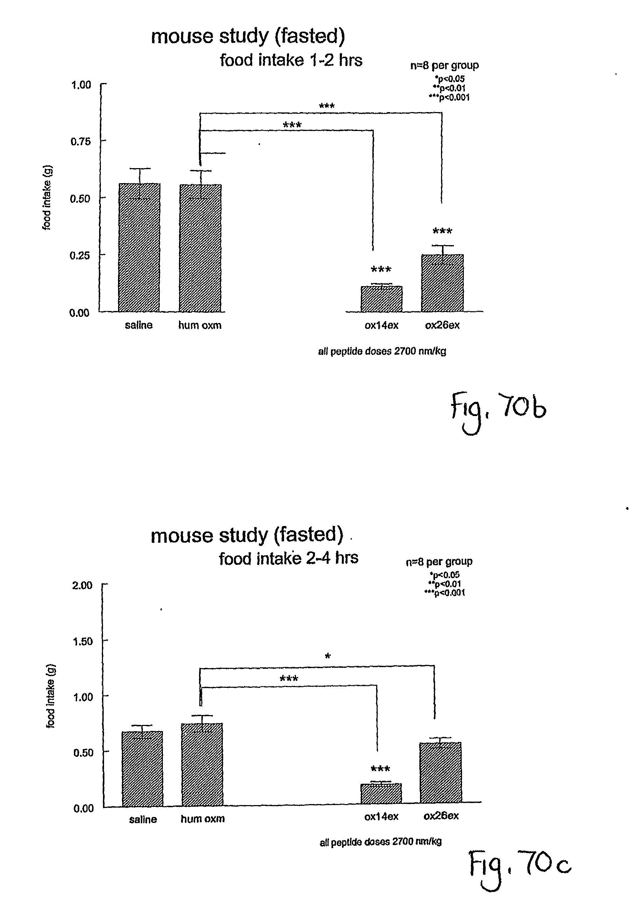

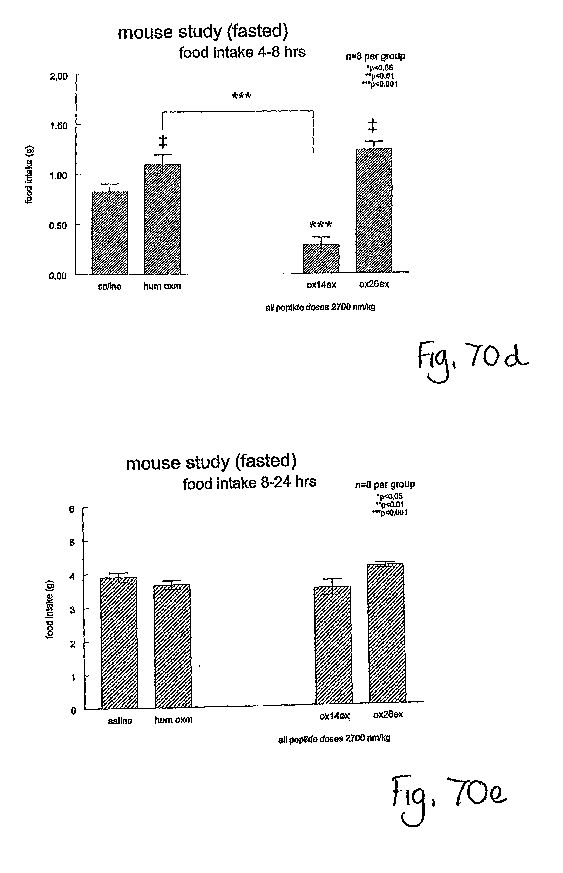

The measured food intake for each group is shown in Figs. 70a to 70e for, respectively, the intervals from 0 to 1 hour, from 1 to 2 hours, from 2 to 4 hours, from 4 to 8 hours and from 8 to 24 hours after injection.

### Example 61

Groups of fasted mice were each injected with the following:
Oxm(xx15-21) 
His Ser Gln Gly Thr Phe Thr Ser Asp Tyr Ser Lys Tyr Leu Glu Glu Glu Ala Val Arg Leu Phe Val
Gln Trp Leu Met Asn Thr Lys Arg Asn Arg Asn Asn Ile Ala (SEQ ID NO: 15)
D-His1,Ala2-oxm(xx15-21) 
D-His Ala Gln Gly Thr Phe Thr Ser Asp Tyr Ser Lys Tyr Leu Glu Glu Glu Ala Val Arg Leu Phe
Val Gln Trp Leu Met Asn Thr Lys Arg Asn Arg Asn Asn Ile Ala (SEQ ID NO: 76)
D-His1,Ala2-oxm(15-21)-Lys33,Ala38,Ala39 
D-His Ala Gln Gly Thr Phe Thr Ser Asp Tyr Ser Lys Tyr Leu Glu Glu Glu Ala Val Arg Leu Phe
Val Gln Trp Leu Met Asn Thr Lys Arg Asn Lys Asn Asn Ile Ala Ala Ala (SEQ ID NO: 77)
Val18-oxm(xx15-21) 
His Ser Gln Gly Thr Phe Thr Ser Asp Tyr Ser Lys Tyr Leu Glu Glu Glu Val Val Arg Leu Phe Val
Gln Trp Leu Met Asn Thr Lys Arg Asn Arg Asn Asn Ile Ala (SEQ ID NO: 40)
Val18,Ala19-oxm(xx15-21)

His Ser Gln Gly Thr Phe Thr Ser Asp Tyr Ser Lys Tyr Leu Glu Glu Glu Val Ala Arg Leu Phe Val
Gln Trp Leu Met Asn Thr Lys Arg Asn Arg Asn Asn Ile Ala (SEQ ID NO: 75)

The compounds were administered by injection to groups of mice at a dose of 30 nmol/kg. A further group were administered saline (control).

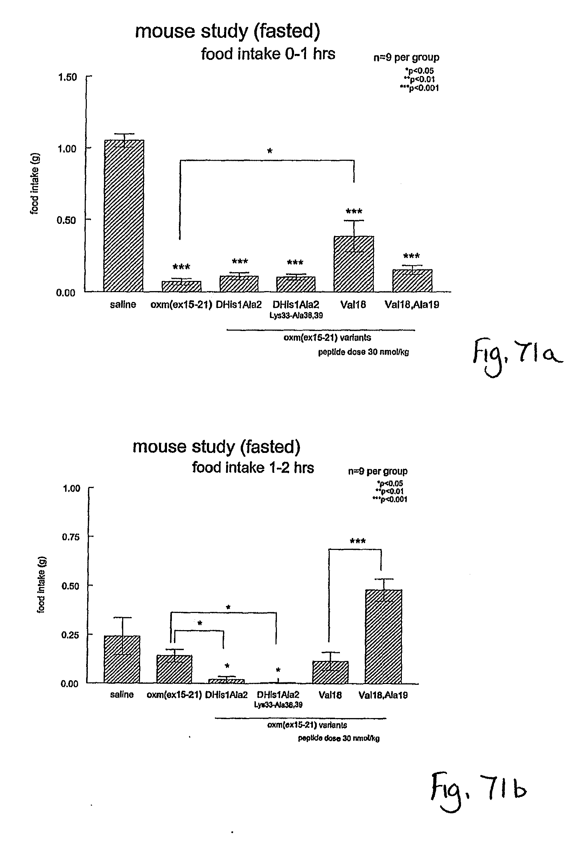

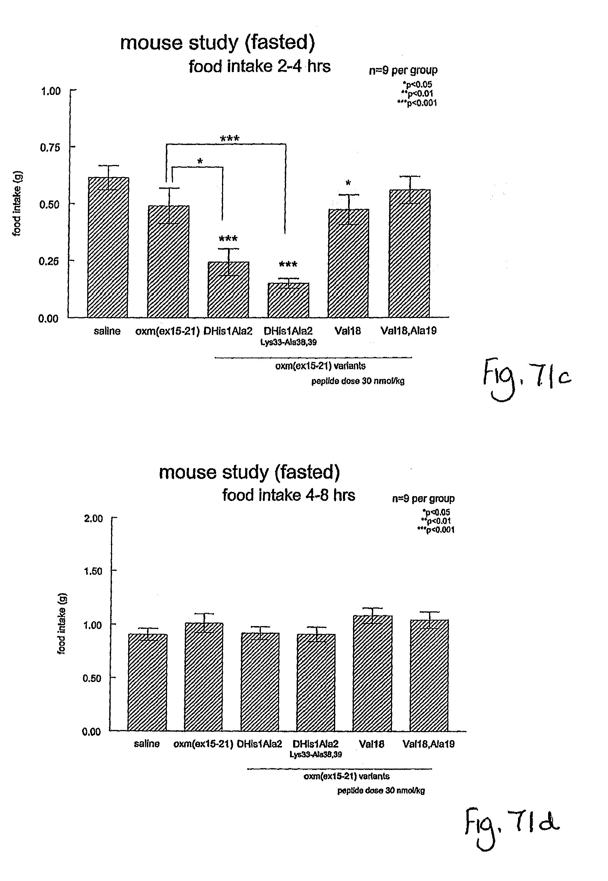

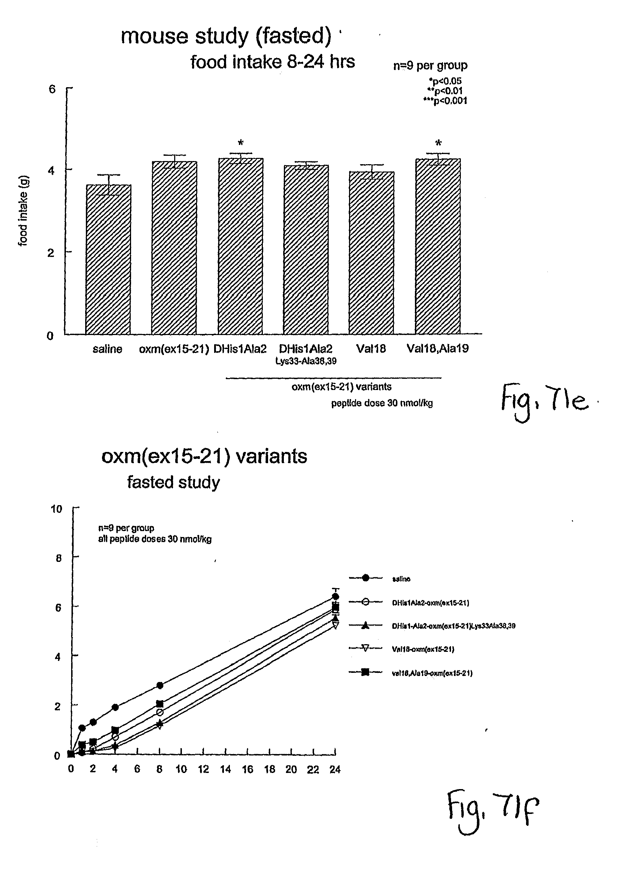

The measured food intake for each group is shown in Figs. 71a to 71e for, respectively, the intervals from 0 to 1 hour, from 1 to 2 hours, from 2 to 4 hours, from 4 to 8 hours and from 8 to 24 hours after injection. Fig. 71f shows cumulative data. All compounds showed reduced food intake in comparison to the saline control at some time points.

### Example 62

Groups of fasted mice were each injected with the following:
1/4 Exendin-4 (SEQ ID NO : 22)
24/40 DHis1-Ala2-oxm(ex15-23)(ex27-33)-Ala38,39 (SEQ ID NO : 86)
24/82 DHis1-Ala2-Glu3-oxm(ex15-23)(ex27-33)-Ala38,39 (SEQ ID NO : 87)
24/203 DHis1-Ala2-Glu3-oxm(ex15-23)-Glu24-(ex27-33)-Ala38,39 (SEQ ID NO : 93)
24/84 DHis1-Ser2-Asp3oxm(ex15-23)(ex27-33)-Ala38,39 (SEQ ID NO : 88)

The compounds were administered by injection to groups of mice at a dose of 5 nmol/kg. A further group were administered saline (control).

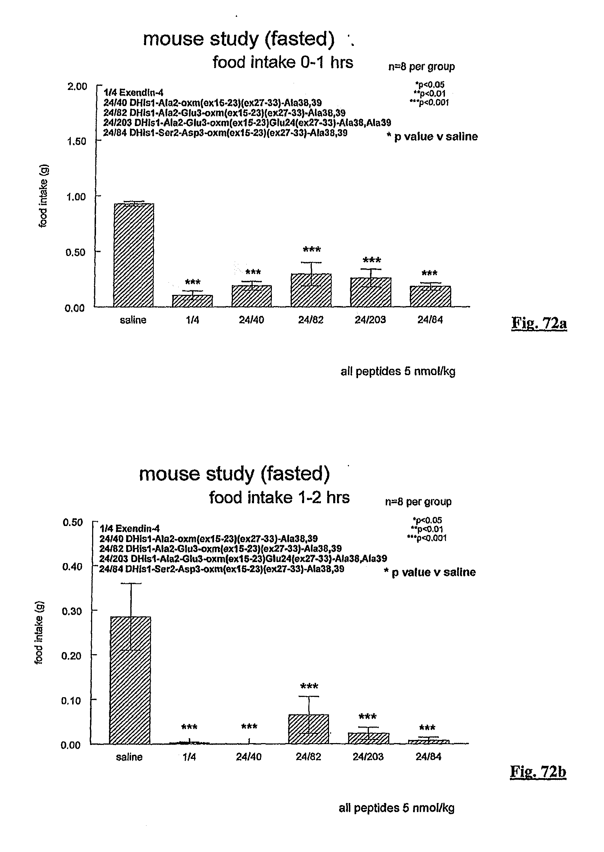

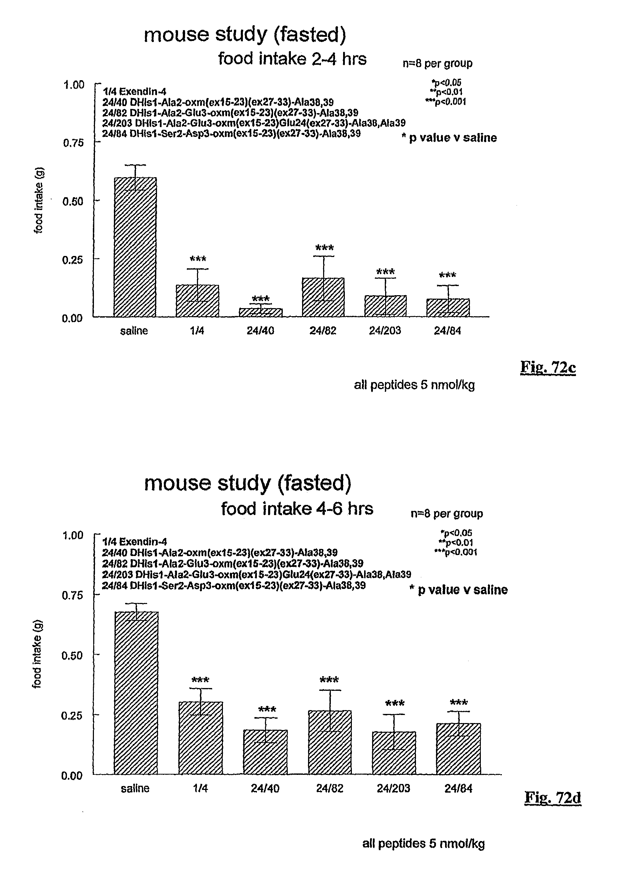

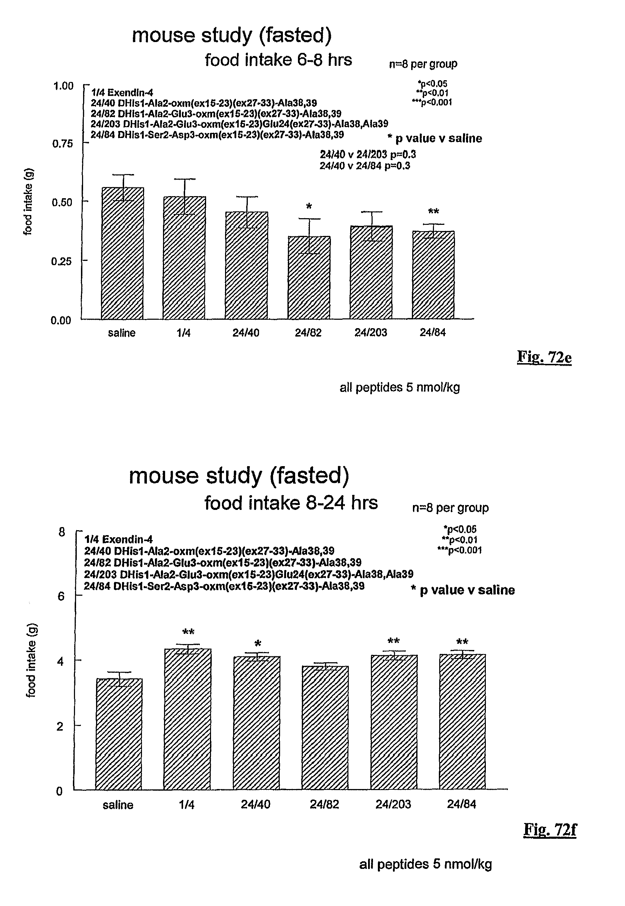

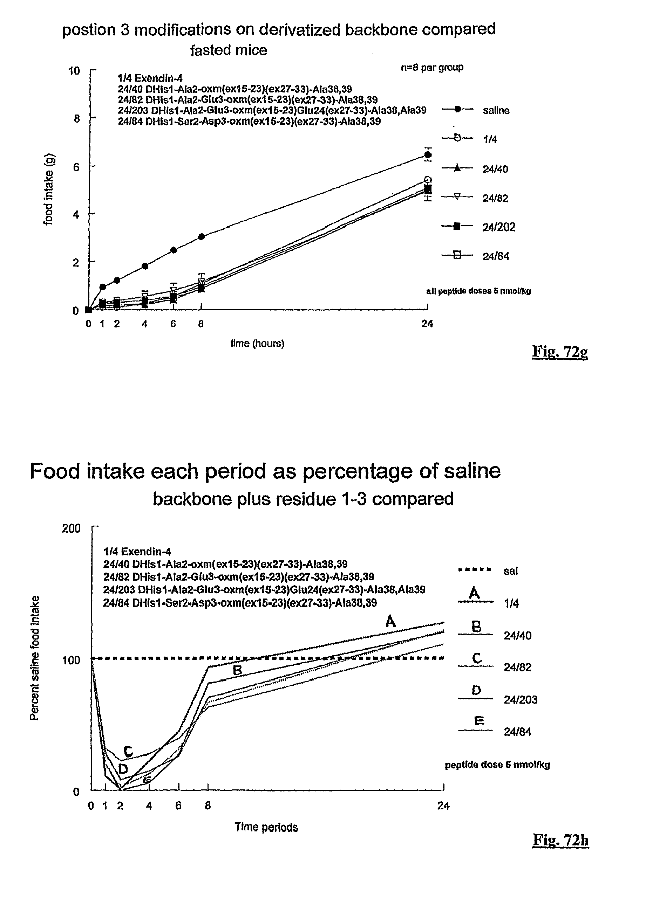

The measured food intake for each group is shown in Figs. 72a to 72f for, respectively, the intervals from 0 to 1 hour, from 1 to 2 hours, from 2 to 4 hours, from 4 to 6 hours, 6 to 8 hours and from 8 to 24 hours after injection. Figs. 72g and h show cumulative data. All compounds showed reduced food intake in comparison to the saline control at some time points. Peptides of the invention showed advantageous effects compared to exendin-4 which was more potent in first hour but which wears off sooner.

### Example 63

Groups of fasted mice were each injected with the following:
24/40 DHis1-Ala2-oxm(ex15-23)(ex27-33)-Ala38,39 (SEQ ID NO : 86)
24/44 DHis1-Ala2-oxm(ex15-23)-Arg27-(ex27-33)-Ala38,39 (SEQ ID NO : 125)
24/41 DHis1-Ala2-Gln17-oxm(ex15-23)(ex27-33)-Ala38,39 (SEQ ID NO : 120)

The compounds were administered by injection to groups of mice at a dose of 6 nmol/kg. A further group were administered saline (control).

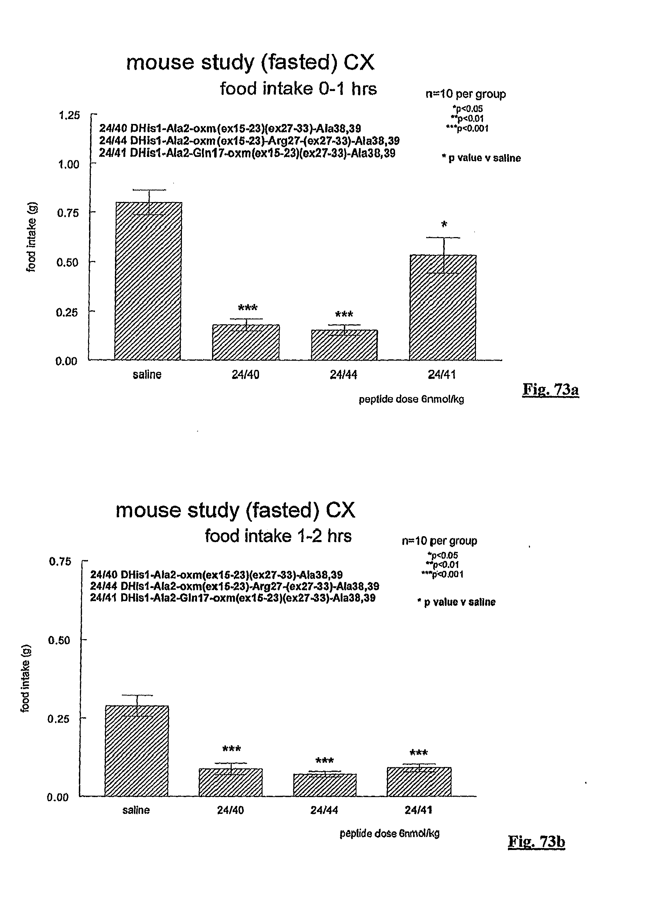

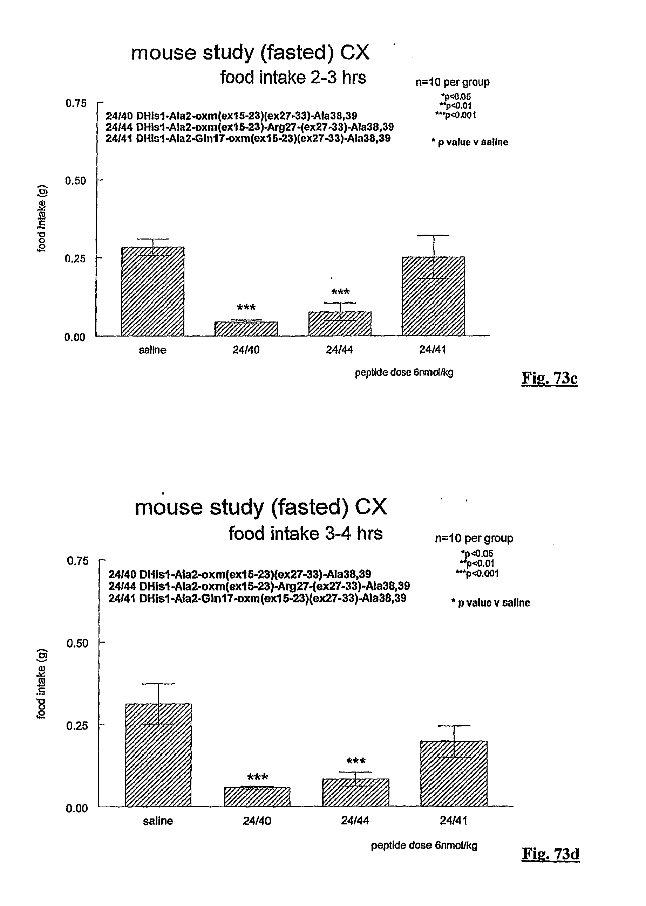

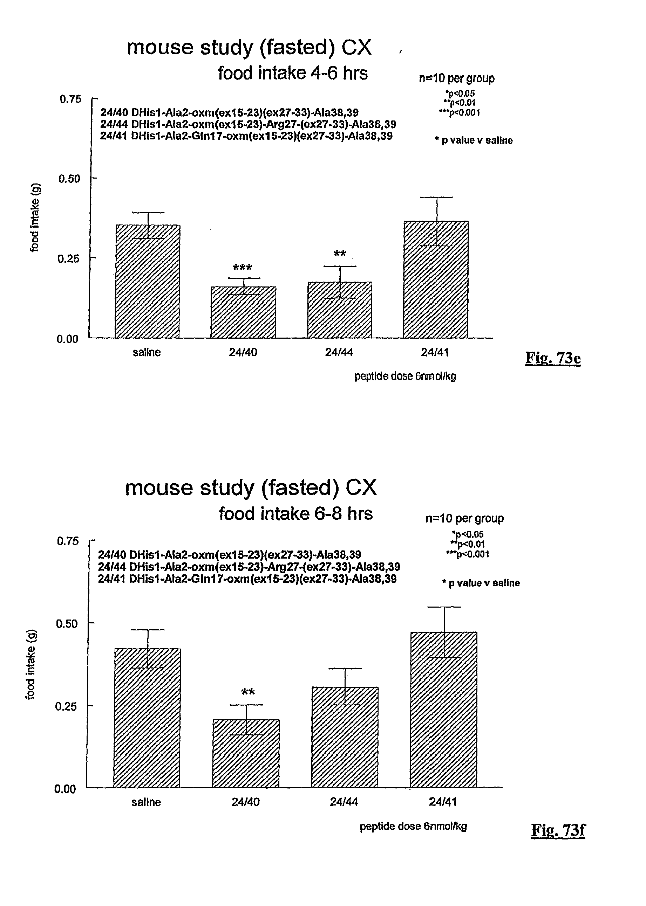

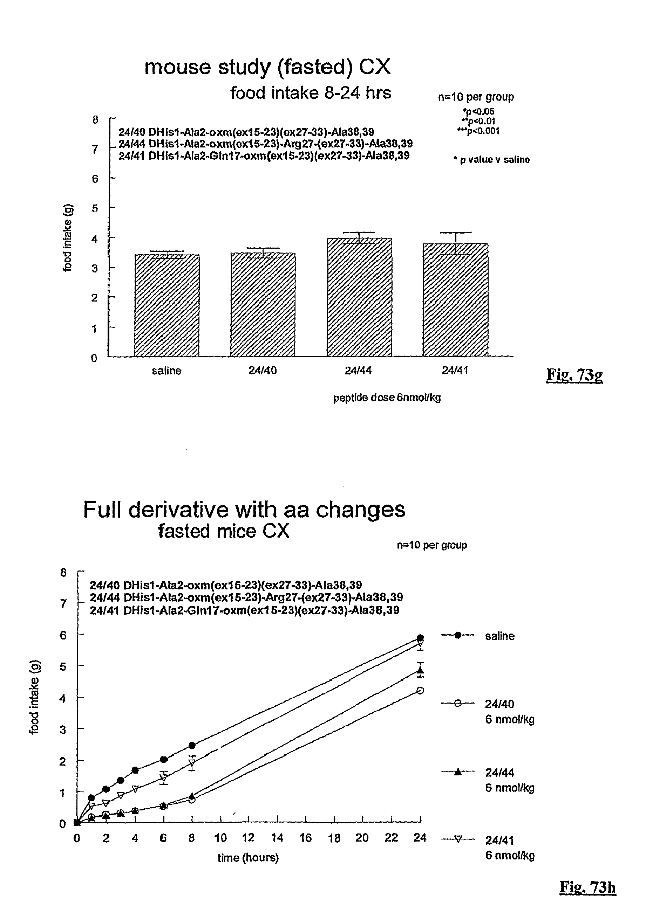

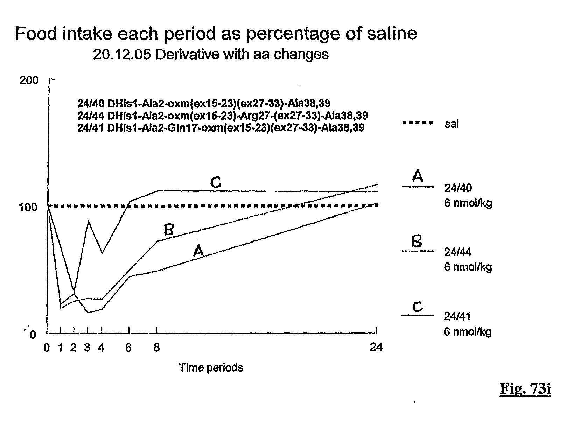

The measured food intake for each group is shown in Fig. 73. All compounds showed reduced food intake in comparison to the saline control at some time points.

### Example 64

Groups of fasted mice were each injected with the following:
24/40 DHis1-Ala2-oxm(ex15-23)(ex27-33)-Ala38,39 (SEQ ID NO : 86)
24/42 DHis1-Ala2-Ser18-oxm(ex15-23)(ex27-33)-Ala38,39 (SEQ ID NO : 127)
24/43 DHis1-Ala2-Leu18-oxm(ex15-23)(ex27-33)-Ala38,39 (SEQ ID NO : 128)

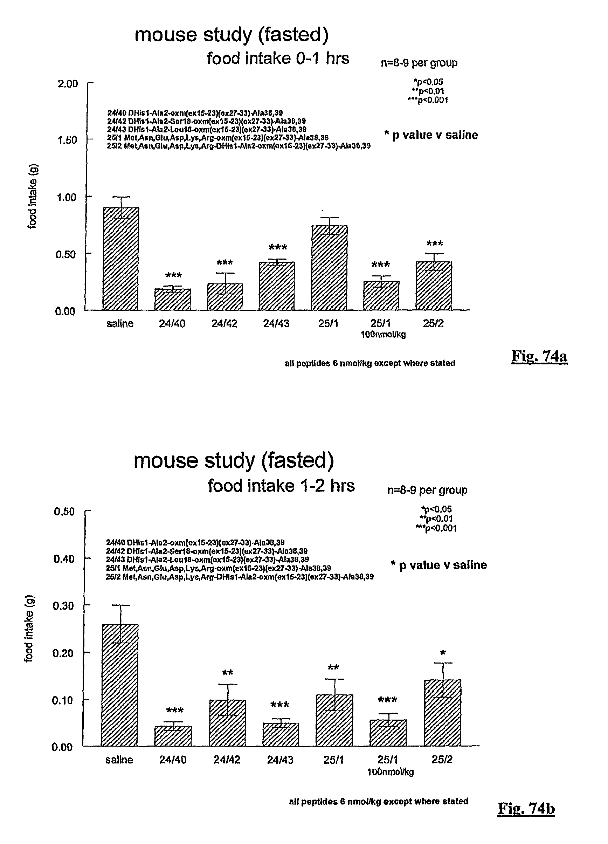

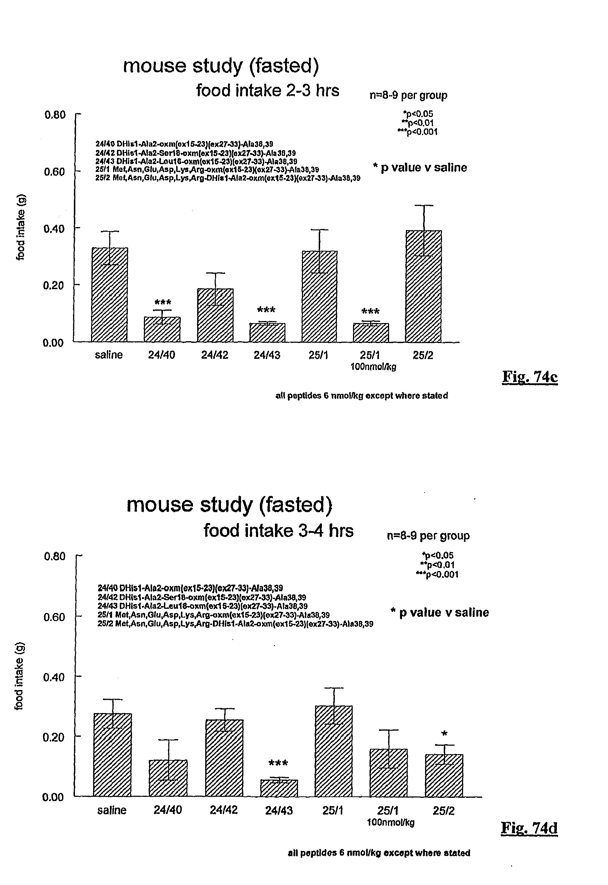

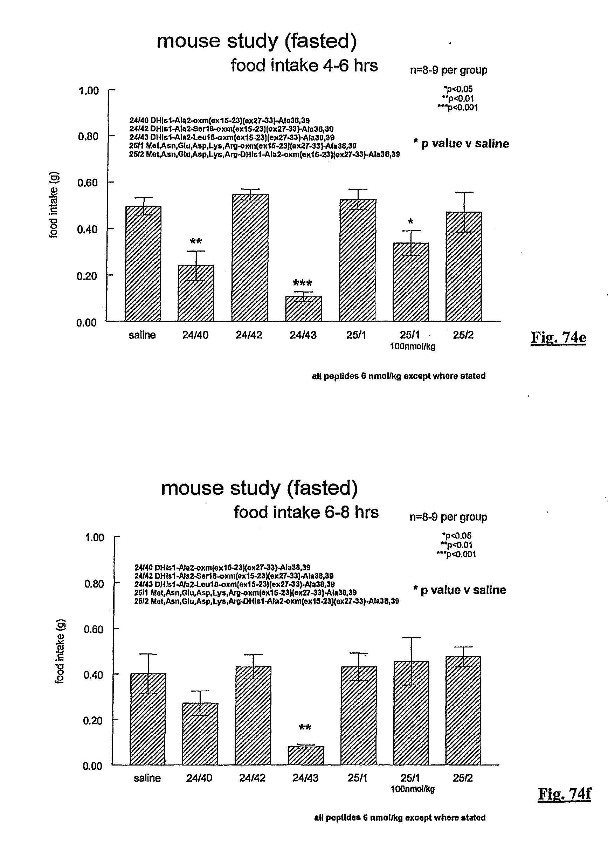

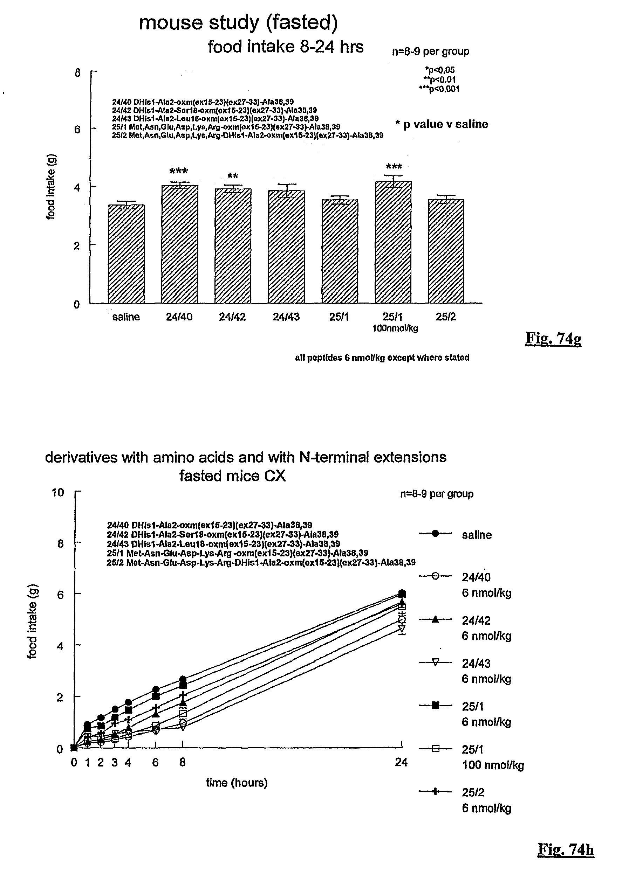

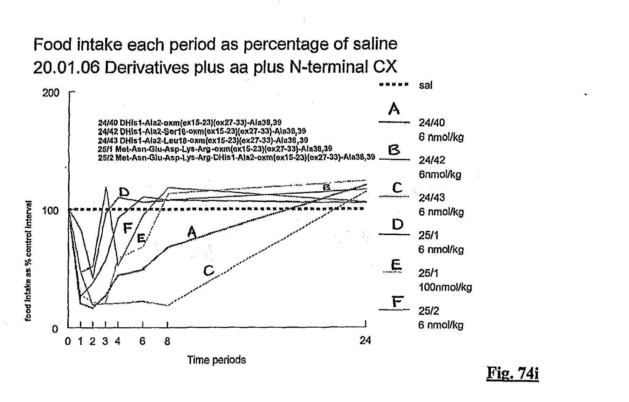

The compounds were administered by injection to groups of mice at a dose of 6 nmol/kg (expect where stated in figure 74). A further group were administered saline (control).

The measured food intake for each group is shown in Fig. 74. All compounds showed reduced food intake in comparison to the saline control at some time points.

### Example 65

Groups of fasted mice were each injected with the following:
24/40 DHis1-Ala2-oxm(ex15-23)(ex27-33)-Ala38,39 (SEQ ID NO : 86)
24/43 DHis1-Ala2-Leu18-oxm(ex15-23)(ex27-33)-Ala38,39 (SEQ ID NO : 128)
24/82 DHis1-Ala2-Glu3-oxm(ex15-23)(ex27-33)-Ala,38,39 (SEQ ID NO : 87)
24/84 DHis1-Ser2-Asp3-oxm(ex15-23)(ex27-33)-Ala, 38, 39 (SEQ ID NO :88)

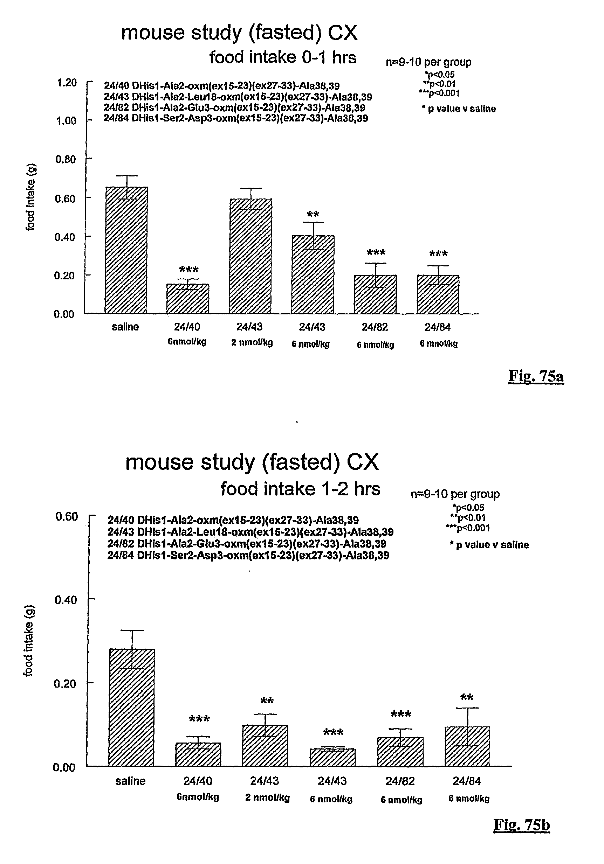

The compounds were administered by injection to groups of mice at the doses shown in Fig. 75A further group were administered saline (control).

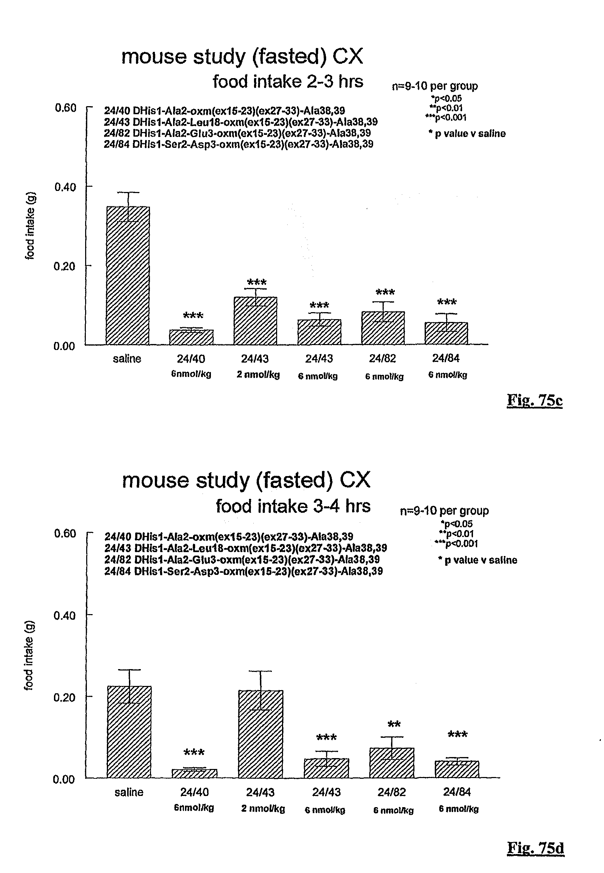

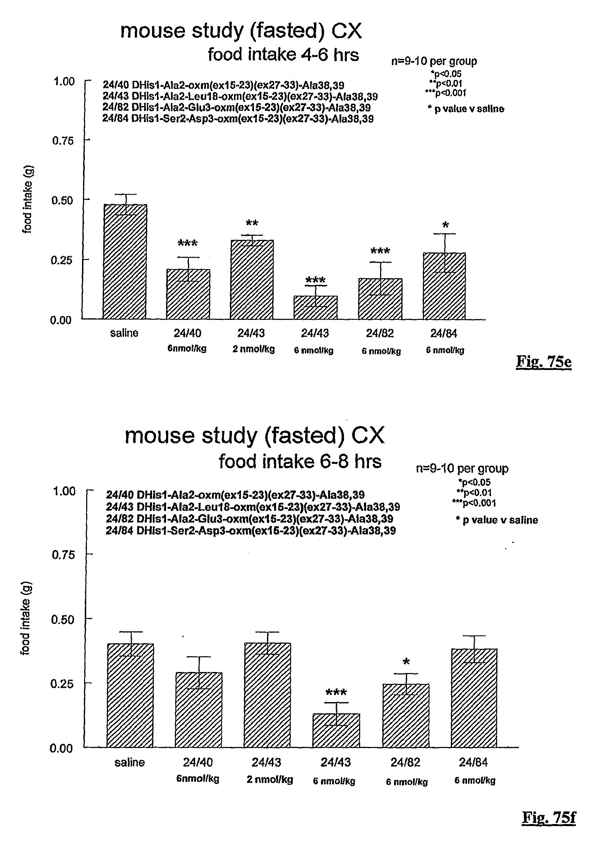

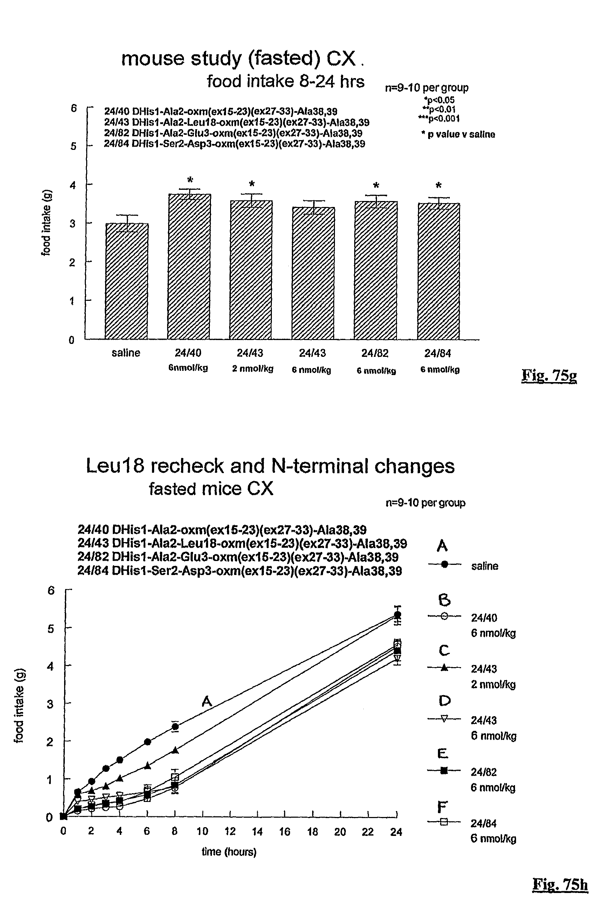

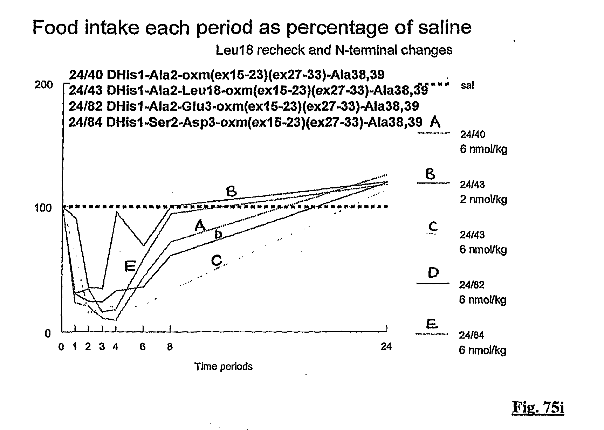

The measured food intake for each group is shown in Fig. 75. Both compounds showed reduced food intake in comparison to the saline control at some time points. The Leu 18 substitution had a favourable effect on feeding profile and is a preferred feature of certain embodiments.

### Example 66

A group of fasted mice were injected with the following:
24/110 DHis-Ser2-Glu3-Val18-oxm(ex15-23)(ex27-33)-Lys33-Ala38,39-Glu40,41-Lys42 (SEQ ID NO: 110)
23/102 DHis1-Ser2-Glu3-oxm(ex15-23)(ex27-33)-Lys33-Ala38,39-Glu40,41-Lys42-Lauroyl (SEQ ID NO: 129)

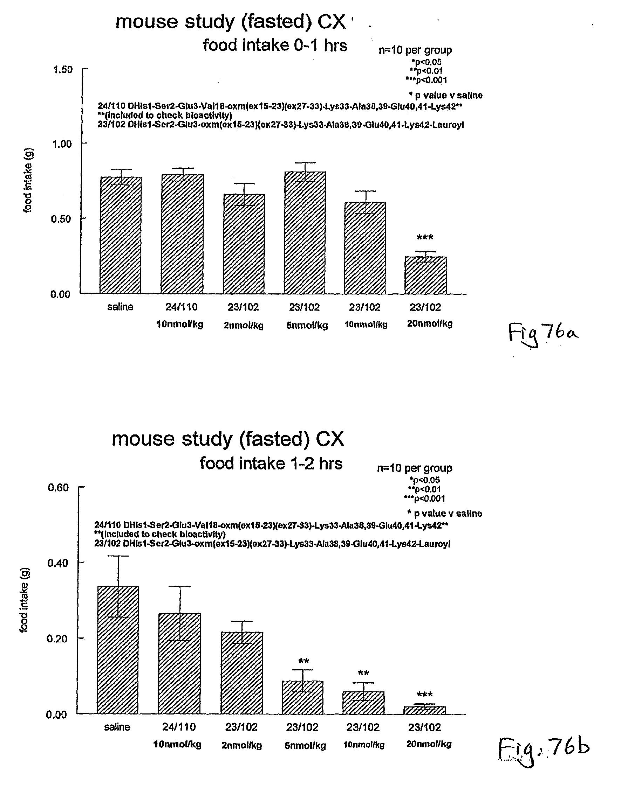

The compound was administered by injection to groups of mice at the doses shown in Fig. 76A further group were administered saline (control).

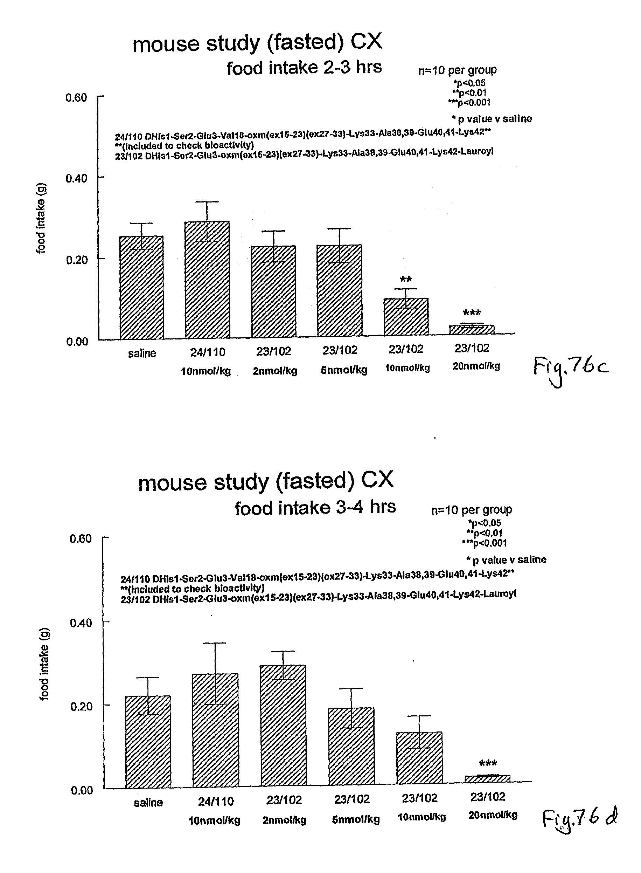

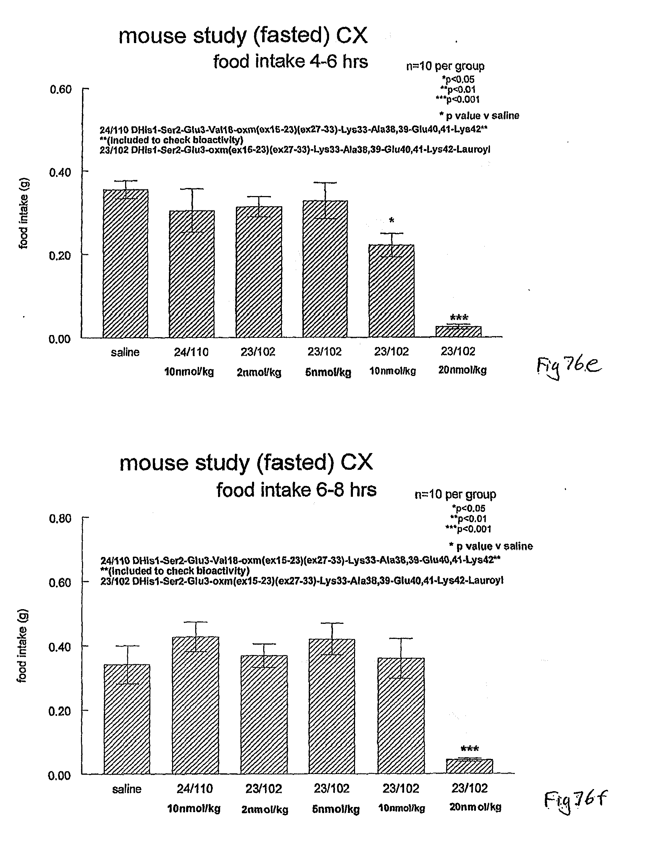

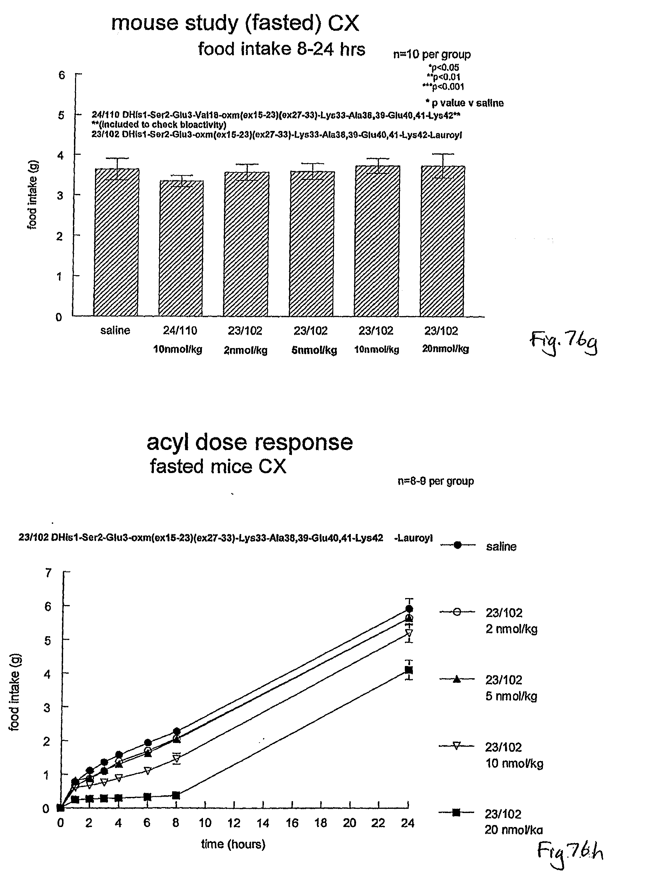

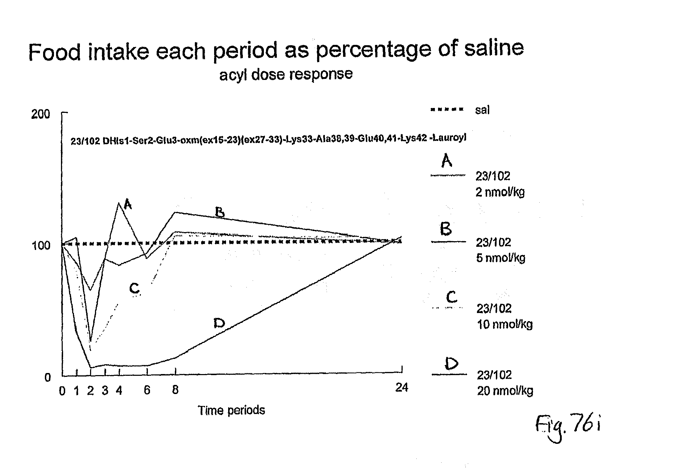

The measured food intake for each group is shown in Fig. 76. The data further shows the dose response for DHis1-Ser2-Glu3-oxm(ex15-23)(ex27-33)-Lys33-Ala38,39-GLu40,41-Lys42-Lauroyl.

### Example 67

Groups of fasted mice were injected with the following:
1/4: Exendin 4 (3 nmol/kg) (SEQ ID NO: 22)
24/76: DHis1-Ala2-oxm(ex15-23)(ex27-33)-Lys33-Ala38,39-Glu40,41-Lys42 (SEQ ID NO: 113)
23/77: DHis1-Ala2-oxm(ex15-23)(ex27-33)-Lys33-Ala38,39-Glu40,41-Lys42-Lauroyl (SEQ ID NO : 132)
23/79: DHis1-Ala2-oxm(ex15-23)(ex27-33)-Lys33-Ala38,39-Glu40,41-Lys42-Palmitoyl (SEQ ID NO : 110)

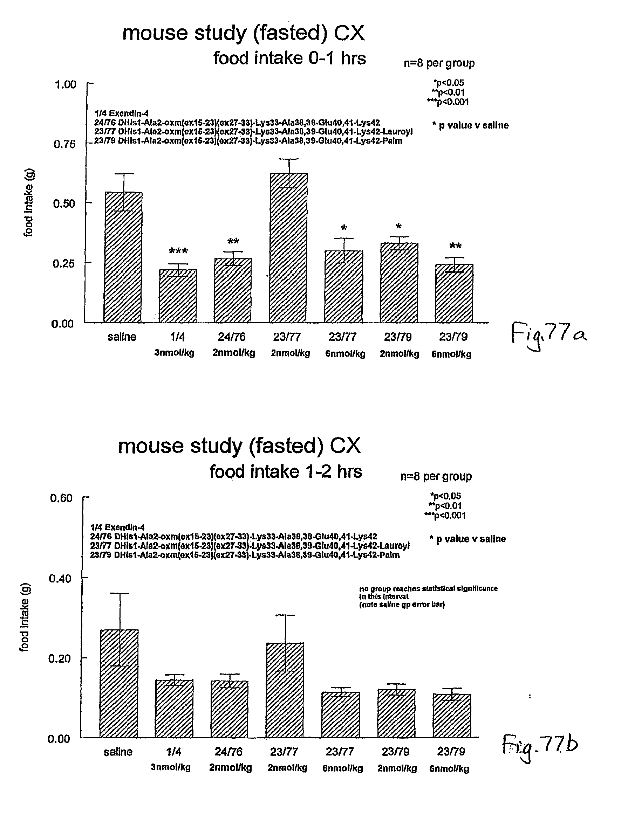

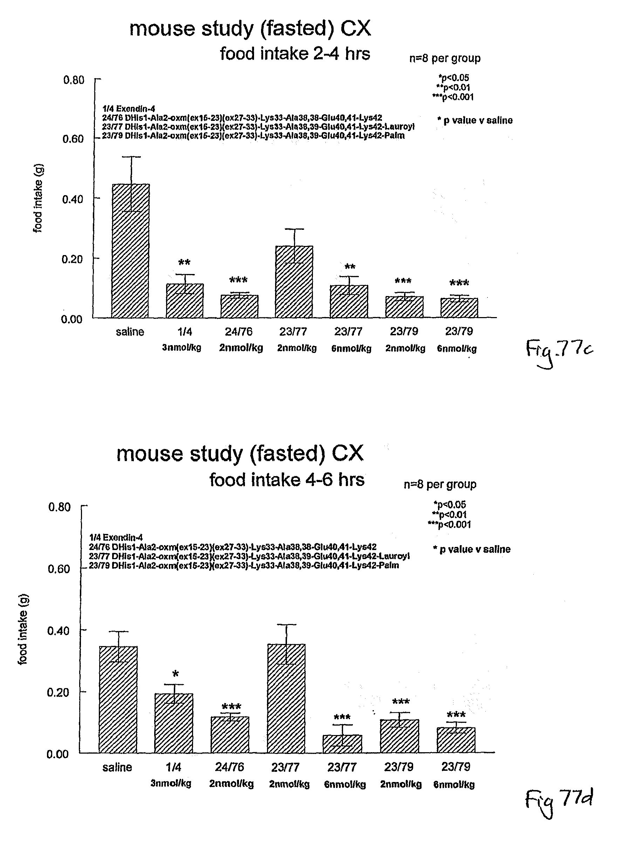

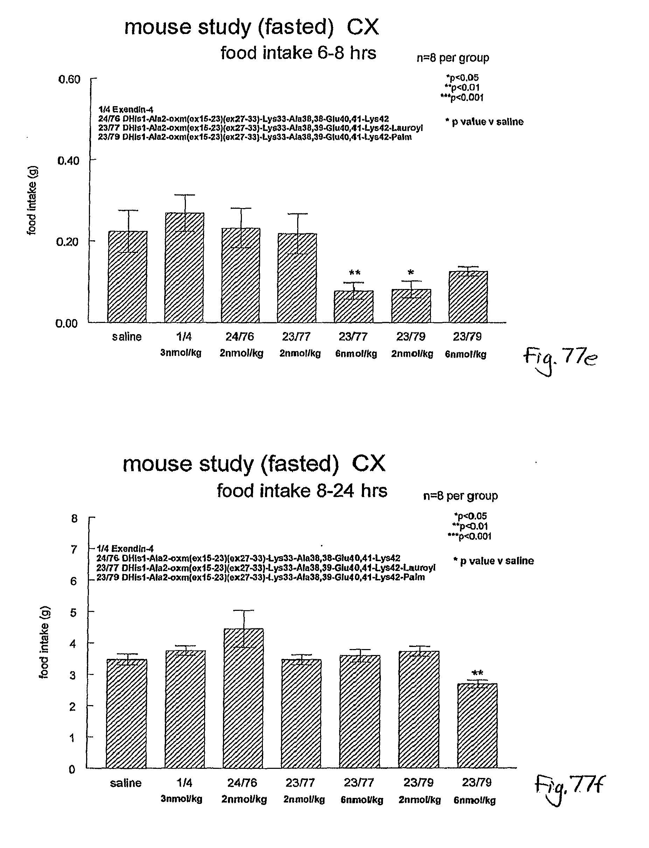

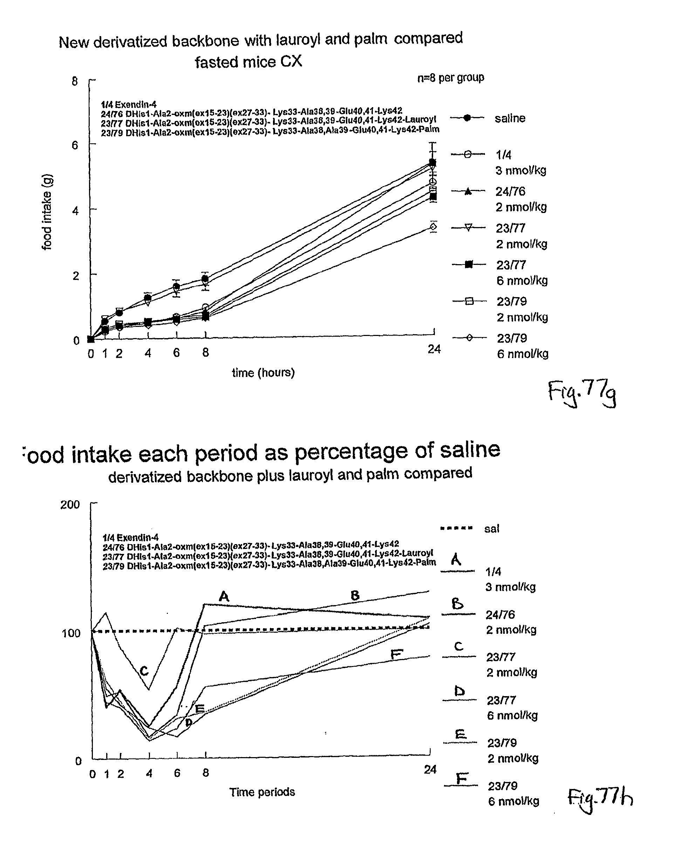

The compounds were administered by injection to groups of mice at the doses shown in Fig. 77. A further group were administered saline (control).

The measured food intake for each group is shown in Fig. 77. Acylation modulates the response profile as previously observed.

### Example 68

Groups of fasted mice were injected with the following:
23/77 DHis1-Ala2-oxm(ex15-23)(ex27-33)-Ala38,39-Glu40,41-Lys42-Lauroyl (SEQ ID NO : 132)
23/79 DHis1-Ala2-oxm(ex15-23)(ex27-33)-Ala38,39-Glu40,41-Lys42-Pahnitoyl (SEQ ID NO : 110)

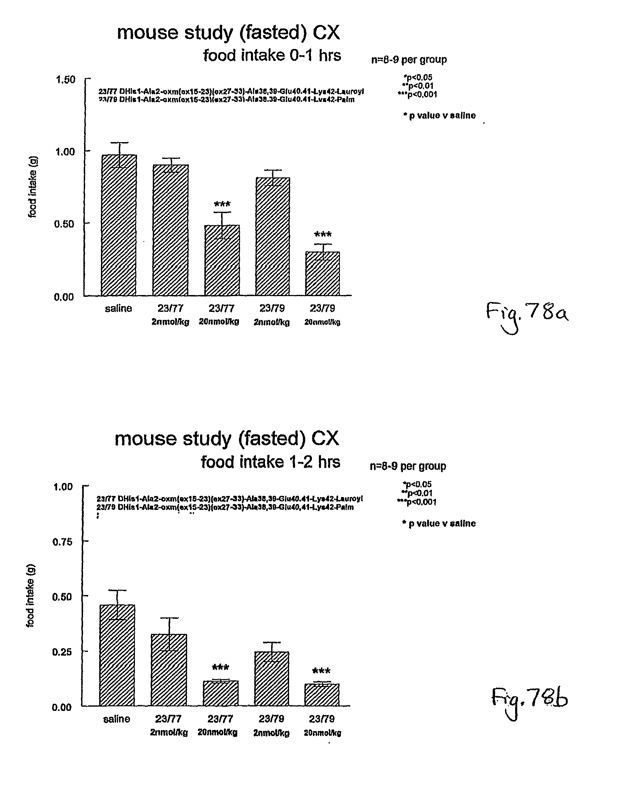

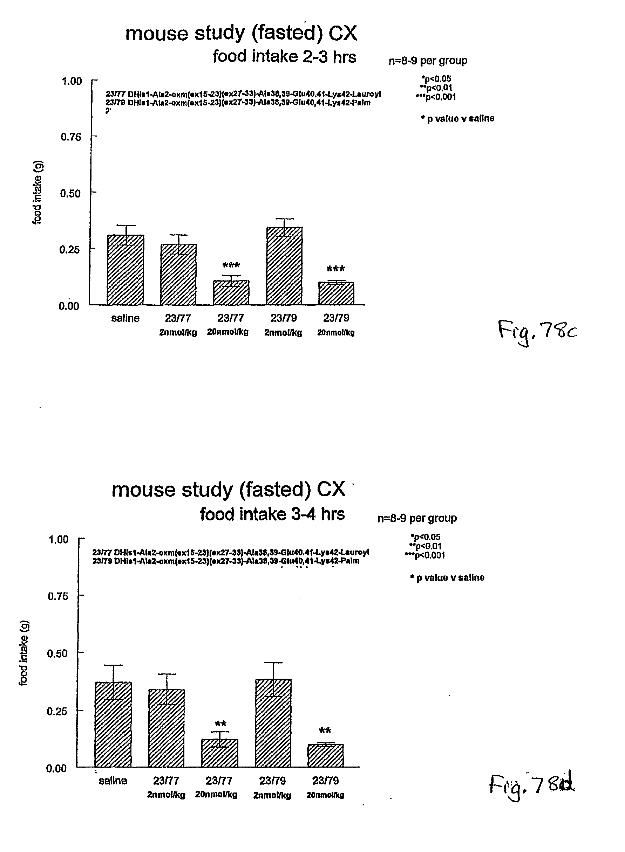

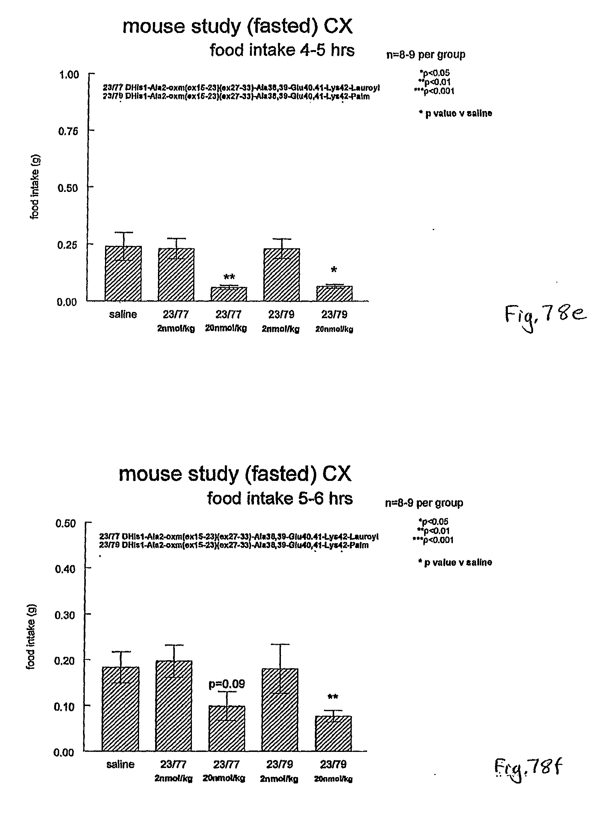

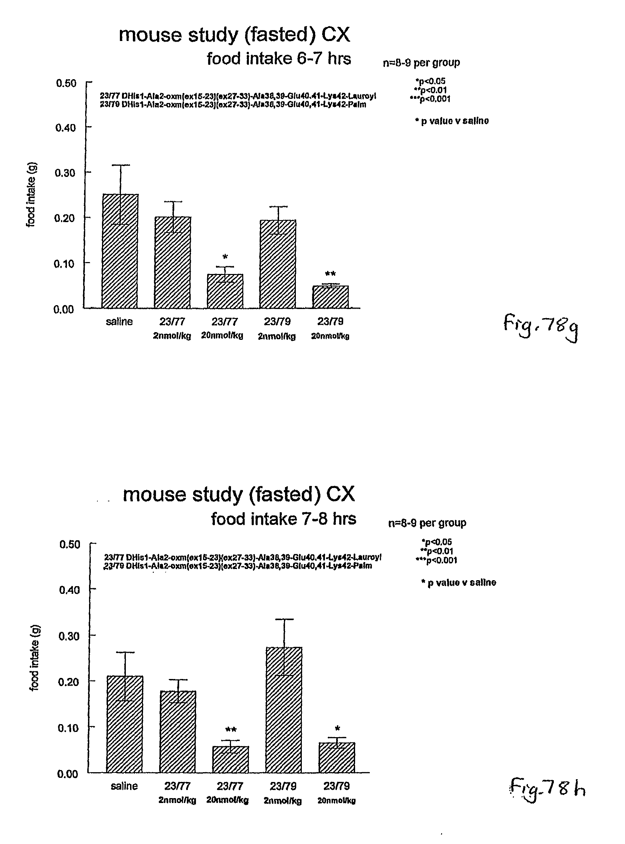

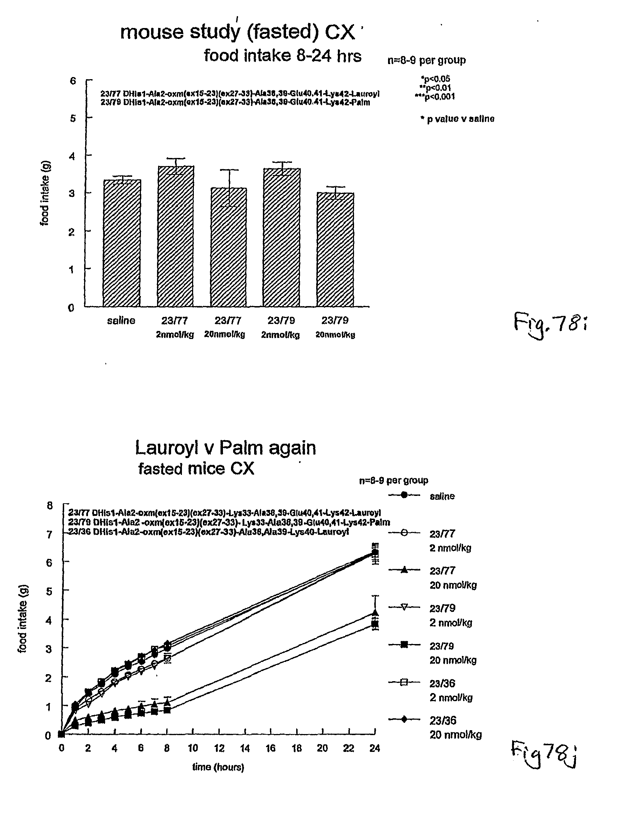

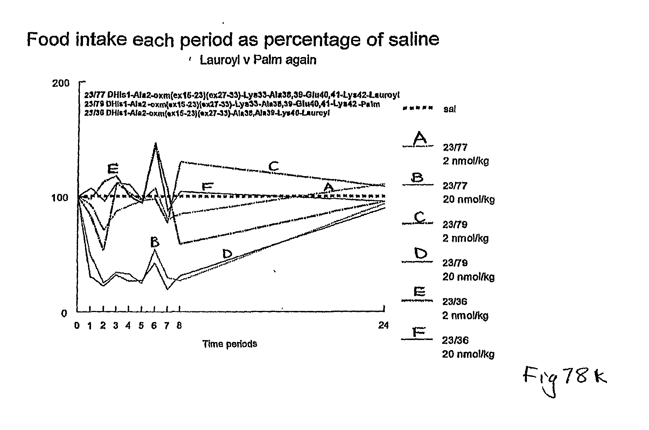

The compounds were administered by injection to groups of mice at the doses shown in Fig. 78. A further group were administered saline (control).

The measured food intake for each group is shown in Fig. 78. No significant difference was demonstrated between lauroyl and palmitoyl modification.

### Example 69

Groups of fasted mice were injected with the following:
24/76 DHis1-Ala2-oxm(ex15-23)(ex27-33)-Lys33-Ala38,39-Glu40,41-Lys42 (3 nmol/kg) (SEQ ID NO : 113)
24/102 DHis1-Ser2-Glu3-oxm(ex15-23)(ex27-33)-Lys33-Ala38,39-Glu40,41-Lys42 (1 and 3 nmol/kg) (SEQ ID NO : 133)
23/102 DHis1-Ser2-Glu3-oxm(ex15-23)(ex27-33)-Lys33-Ala38,39-Glu40,41-Lys42-Lauroyl (3 nmol/kg) (SEQ ID NO : 129)
24/40 DHis1-Ala2-oxm(ex15-23)(ex27-33)-Ala38,39 (as comparator) (3 nmol/kg) (SEQ ID NO : 86)
24/44 DHis1-Ala2-oxm(ex15-23)-Arg27-(ex27-33)-Ala38,39 (3 nmol/kg) (SEQ ID NO : 125)

A further group were administered saline (control).

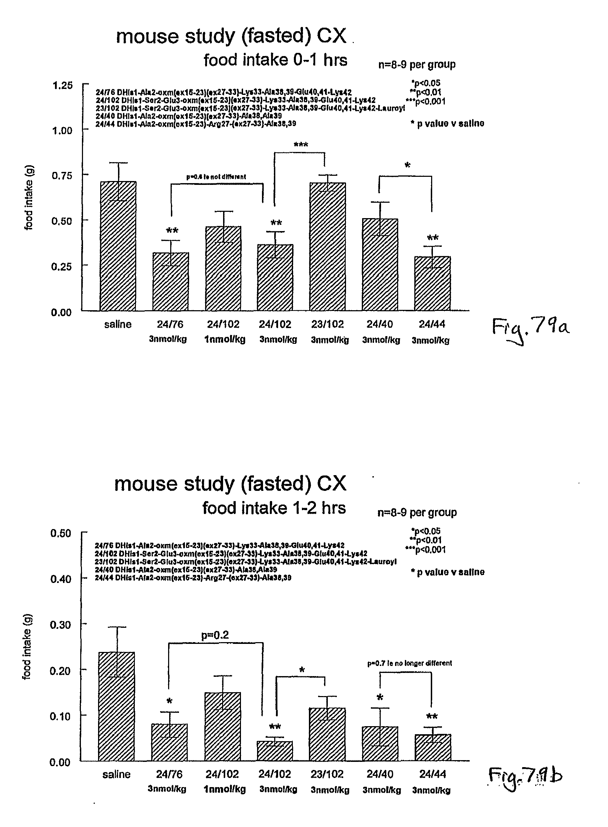

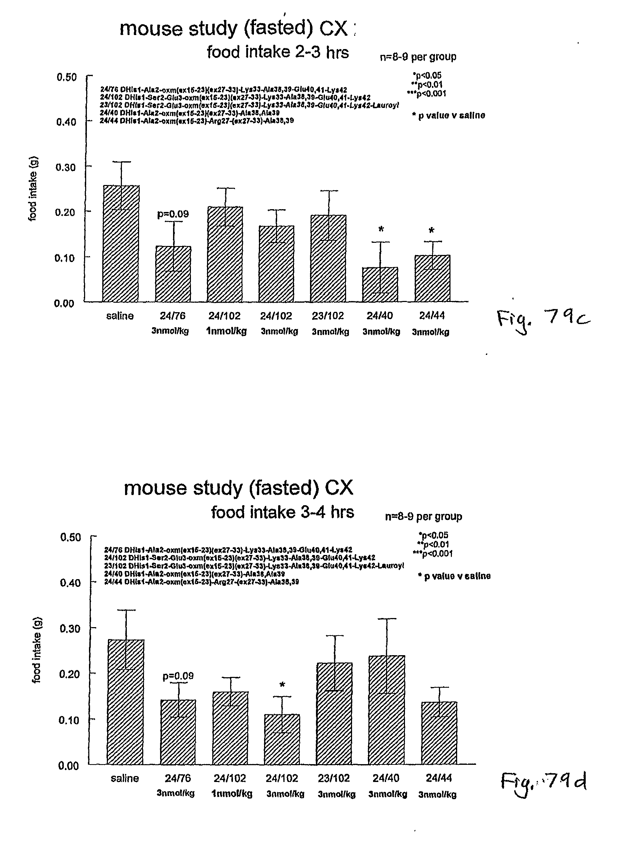

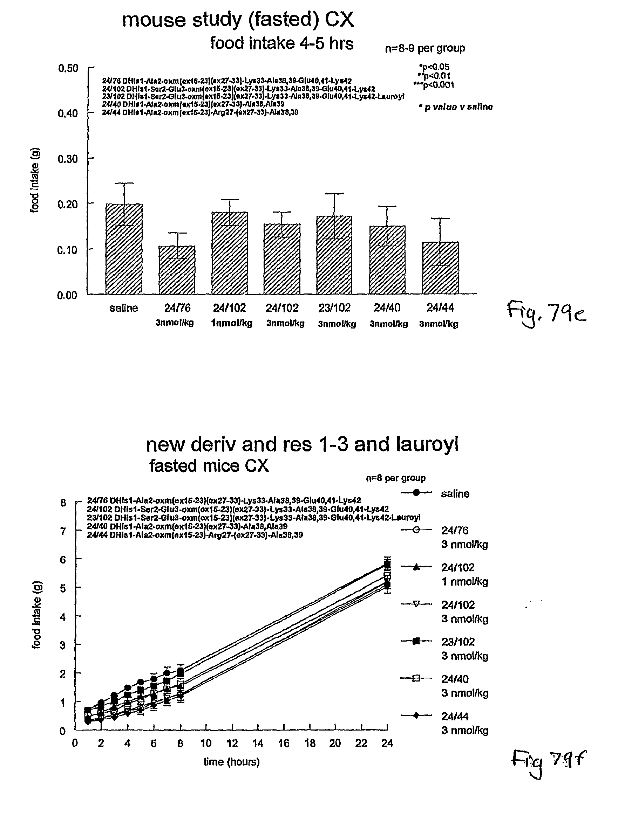

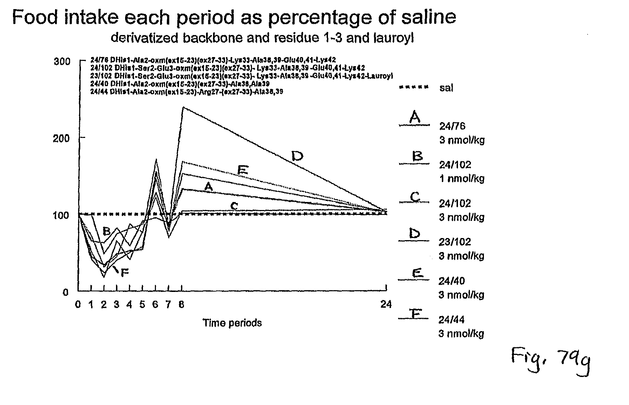

The measured food intake for each group is shown in Fig. 79.

### Example 70

Groups of fasted mice were injected with the following:
24/76 DHis1-Ala2-oxm(ex15-23)(ex27-33)-Lys33-Ala38,39-Glu40,41-Lys42 (SEQ ID NO : 113)
24/102 same backbone as 24/76 but with DHis1-Ser2-Glu3 (SEQ ID NO: 133)
24/104 same backbone as 24/7 but with DHis1-Ser2-Asp3 (SEQ ID NO: 117)
24/110 which is the same as 24/102 (DHis1-Ser2-Glu3) but with additional Val18. (SEQ ID NO: 134)
23/104 which is the same backbone as 24/104 (DHis1-Ser3-Asp3) with additional Lauroyl on Lys42. (SEQ ID NO: 120)

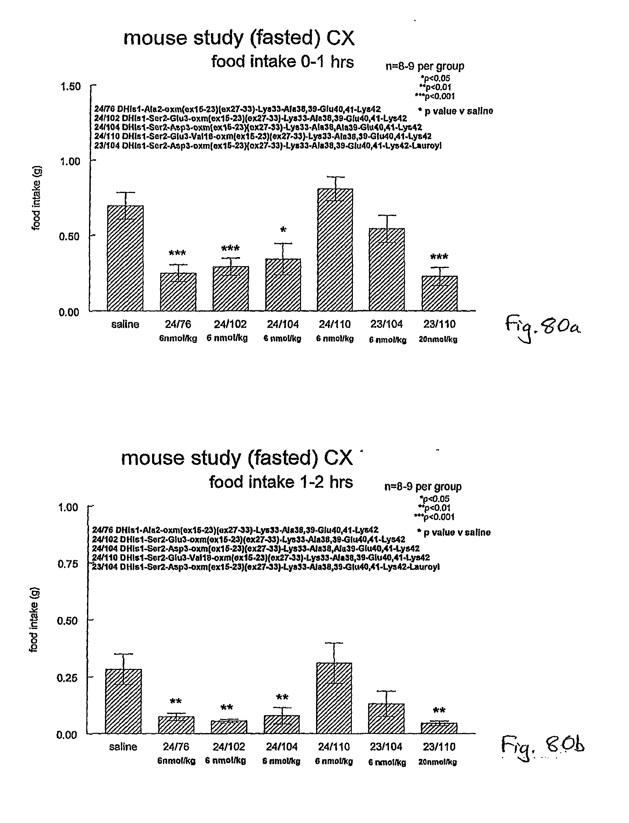

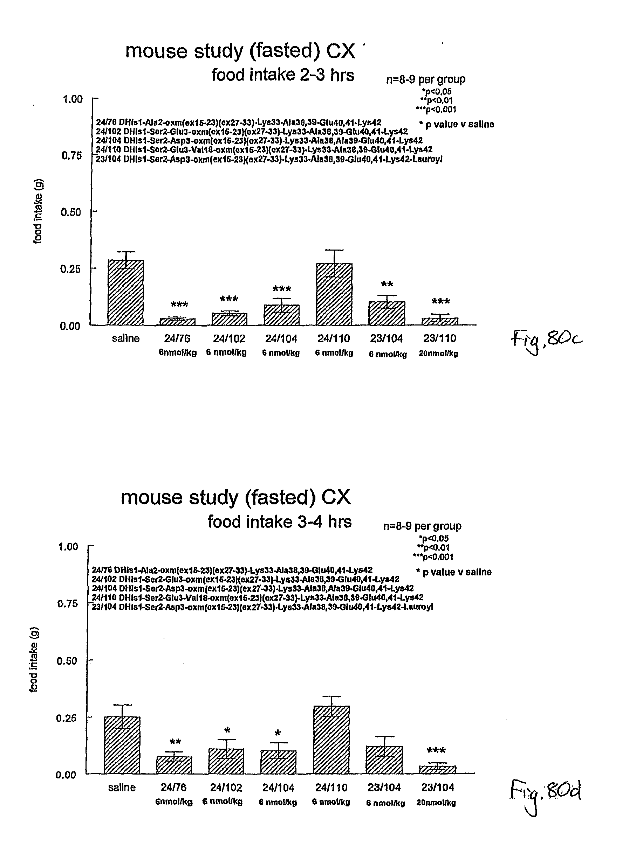

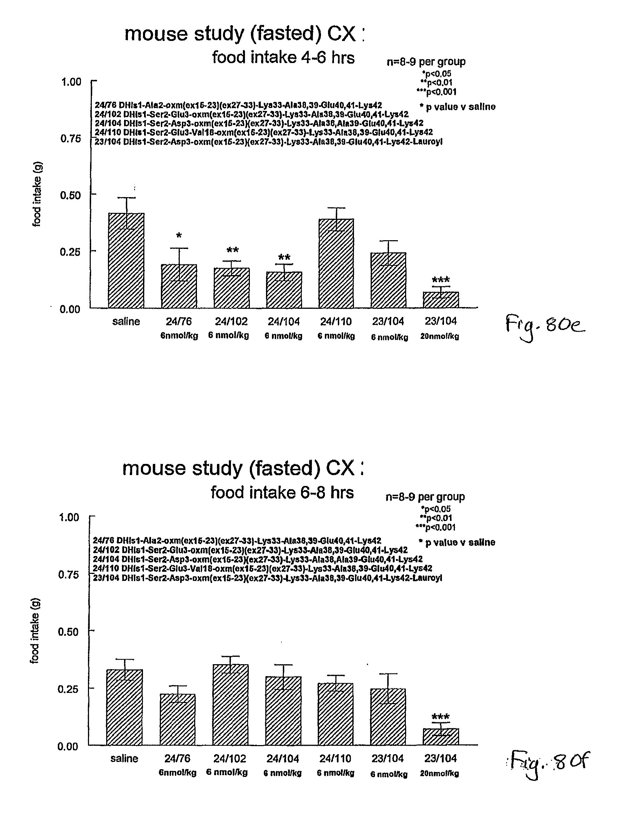

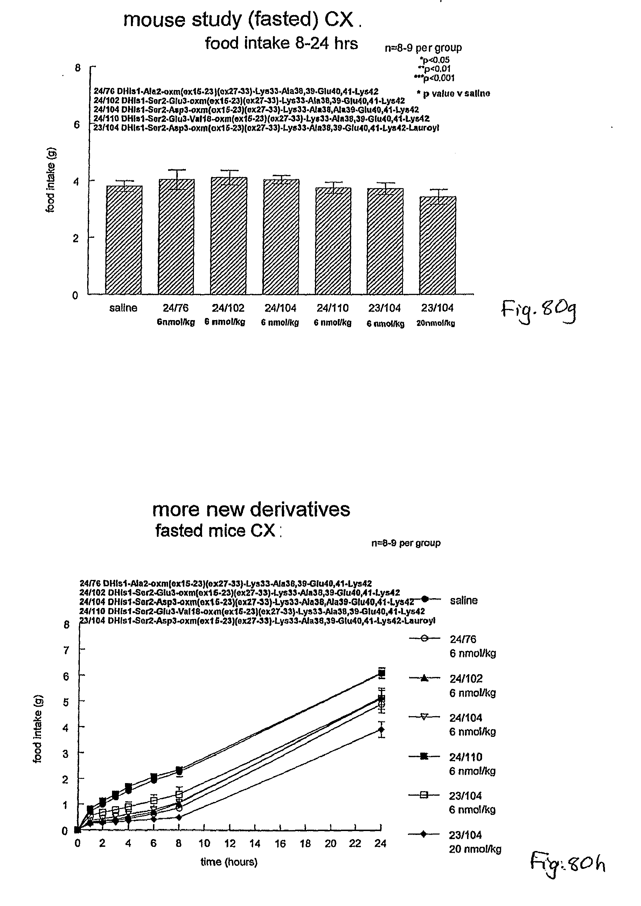

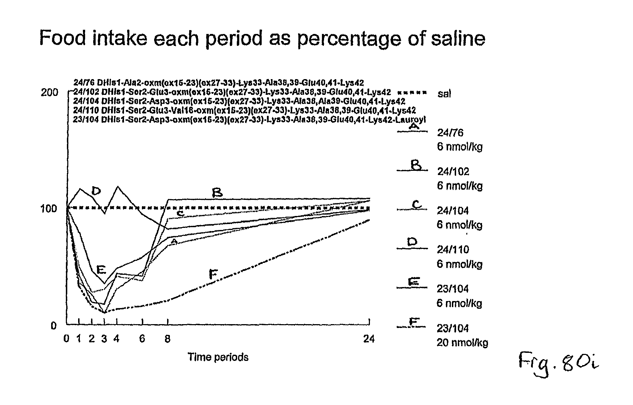

The compounds were administered at the doses shown in Fig. 80.

A further group were administered saline (control).

The measured food intake for each group is shown in Fig. 80.

### Example 71

Groups of fasted mice were injected with the following:
24/40 DHis1-Ala2-oxm(ex15-23)(ex27-33)-Ala38,39 (SEQ ID NO : 86)
24/43 DHis1-Ala2-Leu18-oxm(ex15-23)(ex27-33)-Ala38,39 (SEQ ID NO : 128)
24/82 DHis1-Ala2-Glu3-oxm(ex15-23)(ex27-33)-Ala38,39 (SEQ ID NO : 87)
24/84 DHis1-Ser2-Asp3-oxm(ex15-23)(ex27-33)-Ala38,39 (SEQ ID NO : 88)

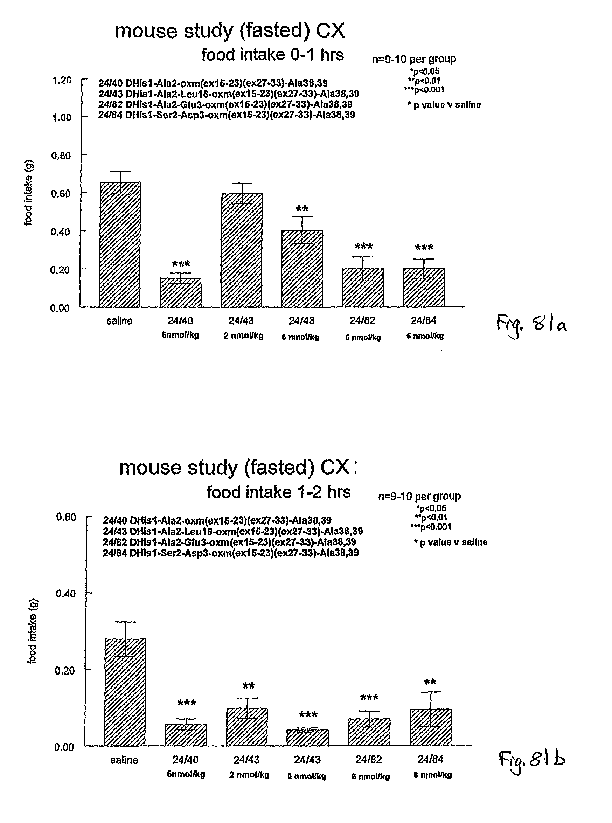

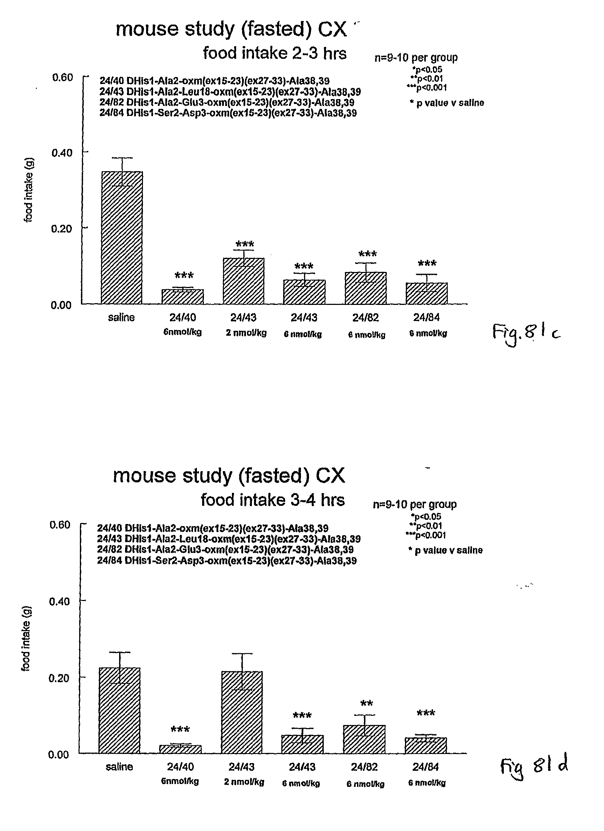

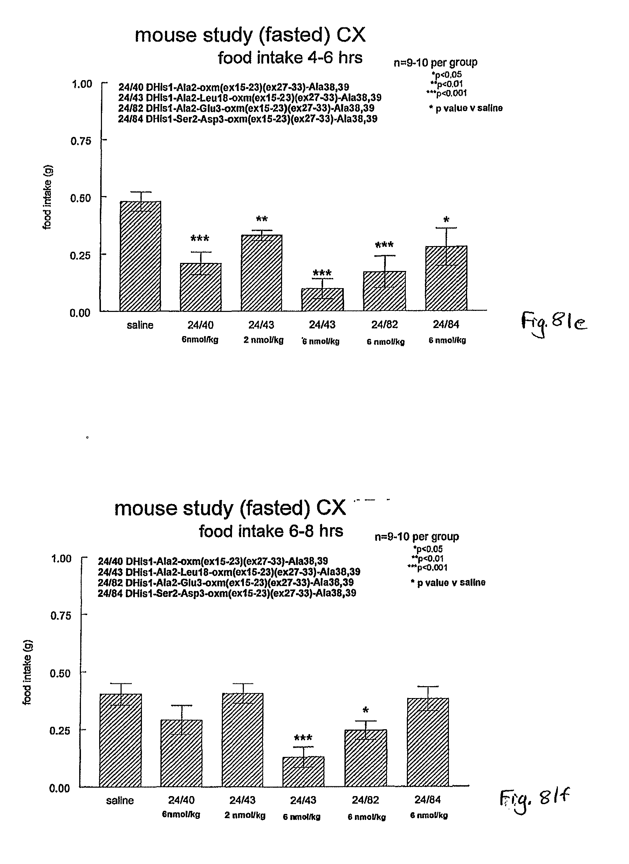

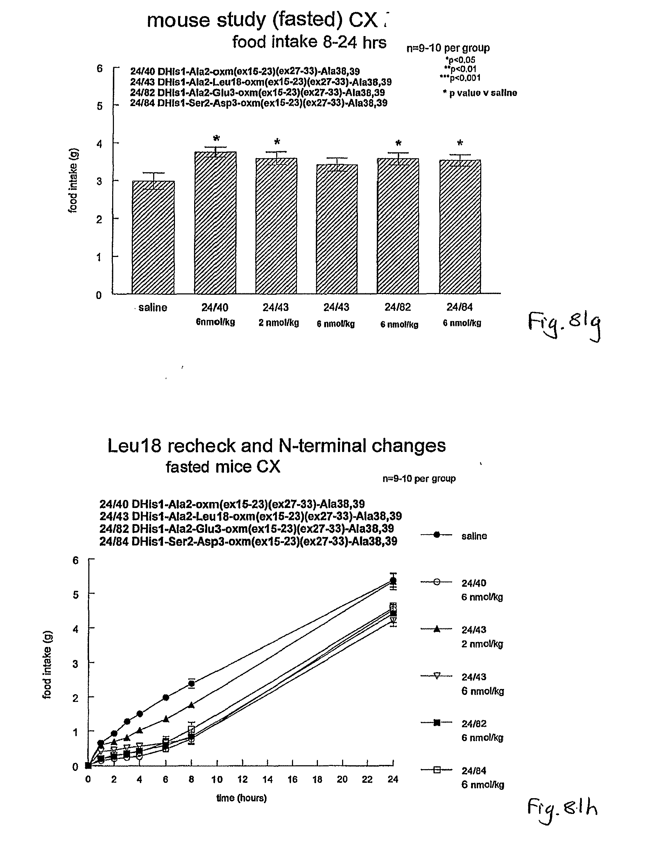

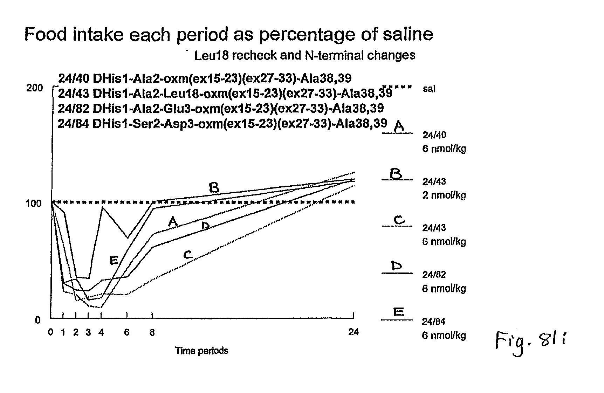

The compounds were administered at the doses shown in Fig. 81.
A further group were administered saline (control).

The measured food intake for each group is shown in Fig. 81.

### Example 72

Groups of fasted mice were injected with the following:
24/104 DHis1-Ser2-Asp3-oxm(ex15-23)(ex27-33)-Lys33-Ala38,39-Glu40,41-Lys42(6 nmol/kg) (SEQ ID NO: 117)
24/112 which is the same backbone as 24/104 but with Val18 8 (6nmol/kg) (SEQ ID NO: 118)
23/112 which is 24/112 with a lauroyl side-chain on Lys42 (6 and 20 nmol/kg) (SEQ ID NO: 119)
23/104 which is 24/104 with Lys42-lauroyl (20nmol/kg) (SEQ ID NO: 120)

A further group were administered saline (control).

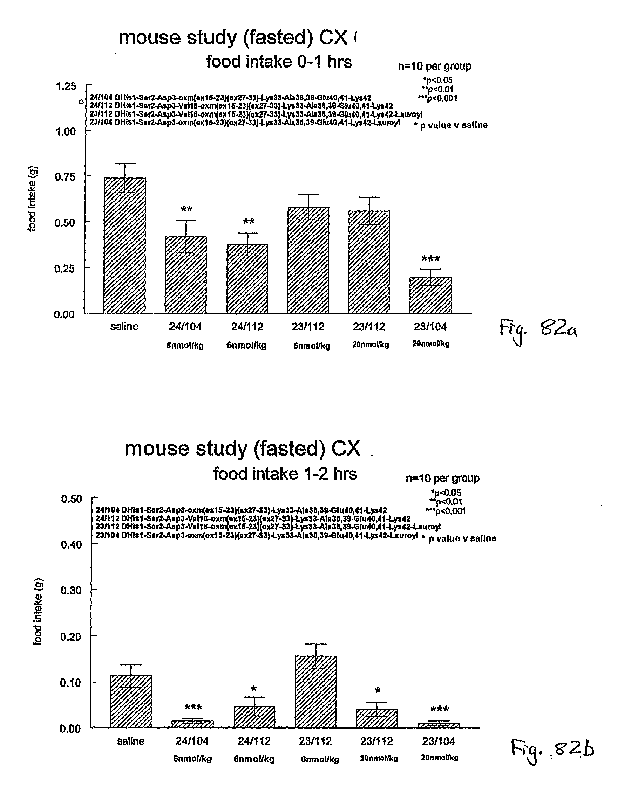

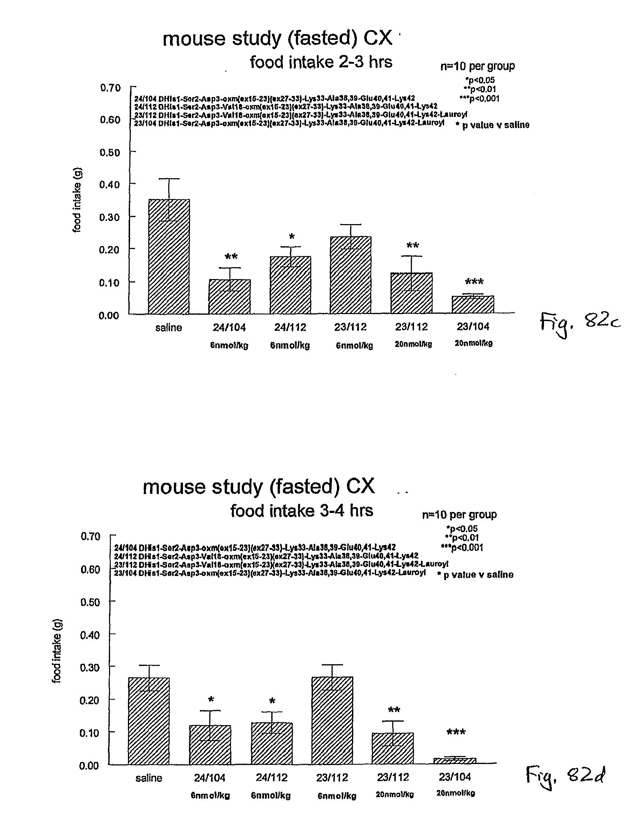

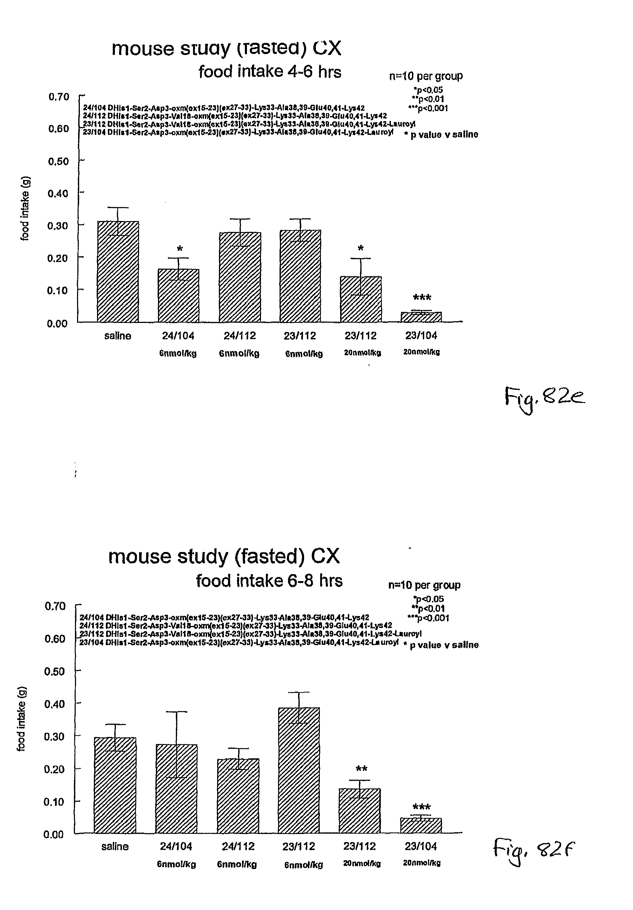

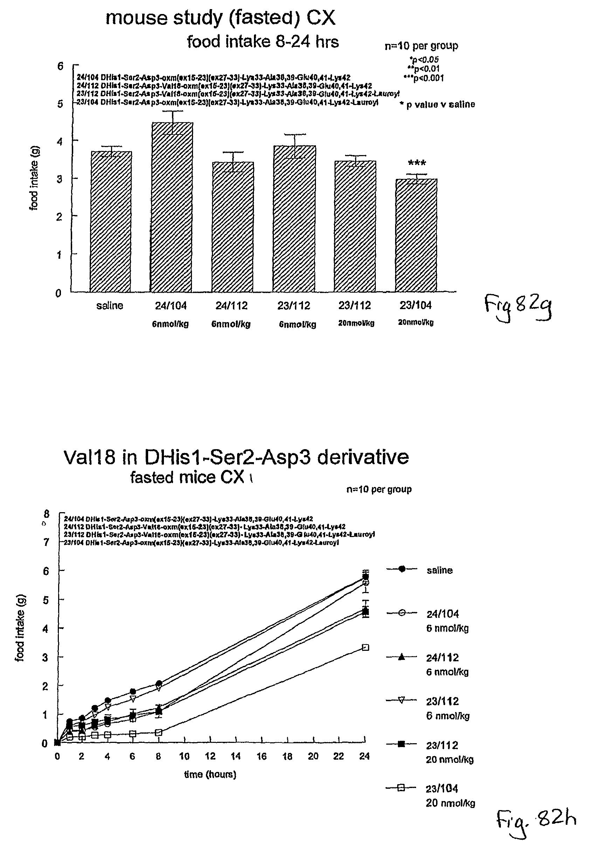

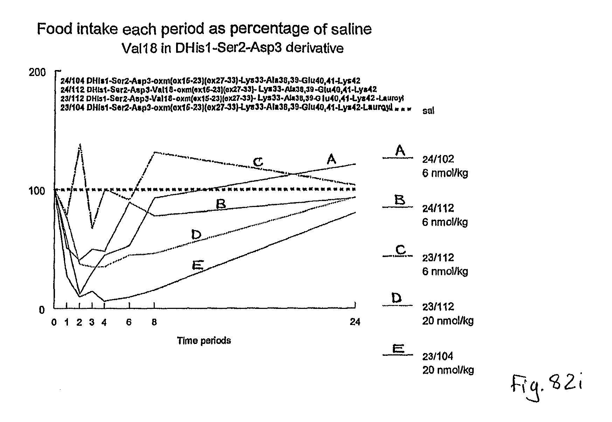

The measured food intake for each group is shown in Fig. 82.

### Example 73

Groups of fasted mice were injected with the following:
24/104 DHis1-Ser2-Asp3-oxm(ex15-23)(ex27-33)-Lys33-Ala38,39-Glu40,41-Lys42 (SEQ ID NO: 117)
24/210 DHis1-Ala2-Asp3-oxm(ex15-23)(ex27-33)-Lys33-Ala38,39-Glu40,41-Lys42 (SEQ ID NO: 114)
24/114 DHis1-Ser2-Asp3-Gln16,Val18-oxm(ex15-23)(ex27-33)-Lys33-Ala38,39-Glu40,41-Lys42 (SEQ ID NO: 135)
A further group were administered saline (control).

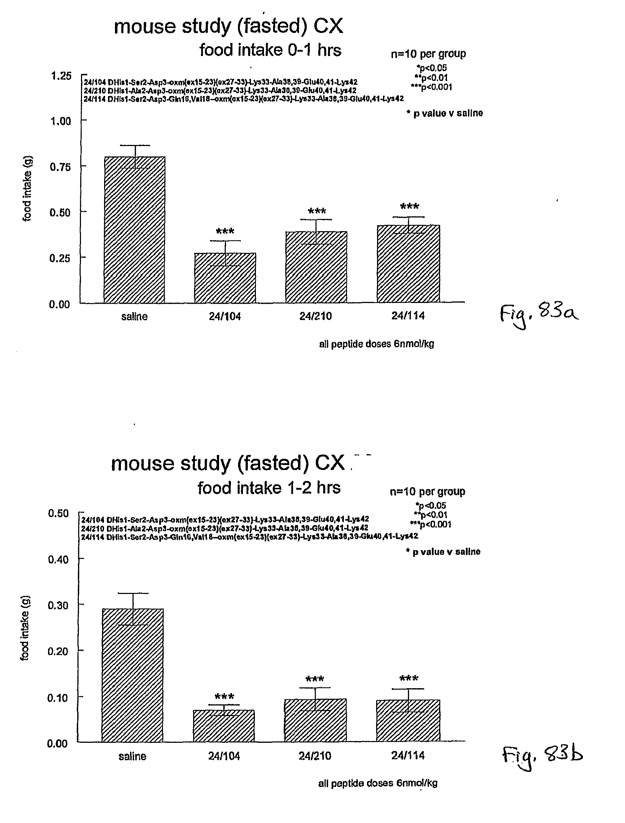

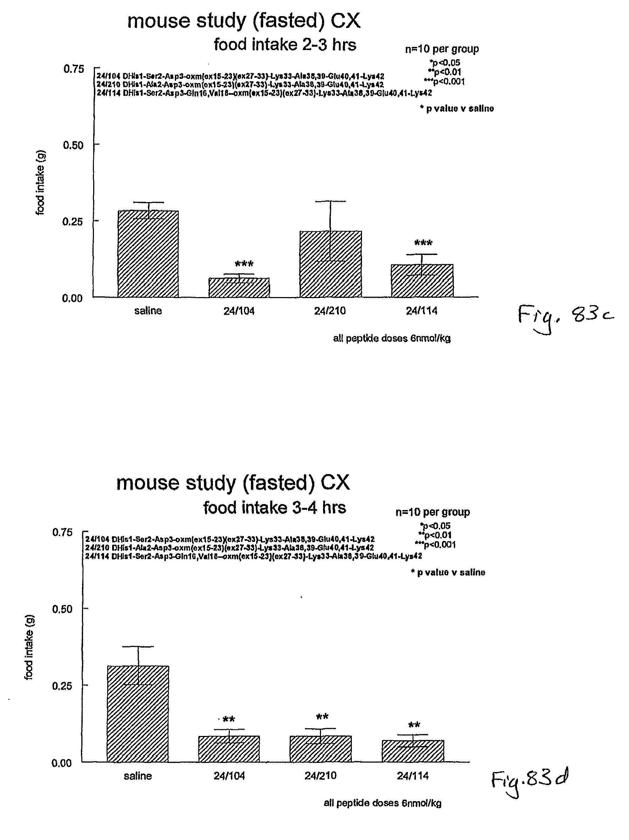

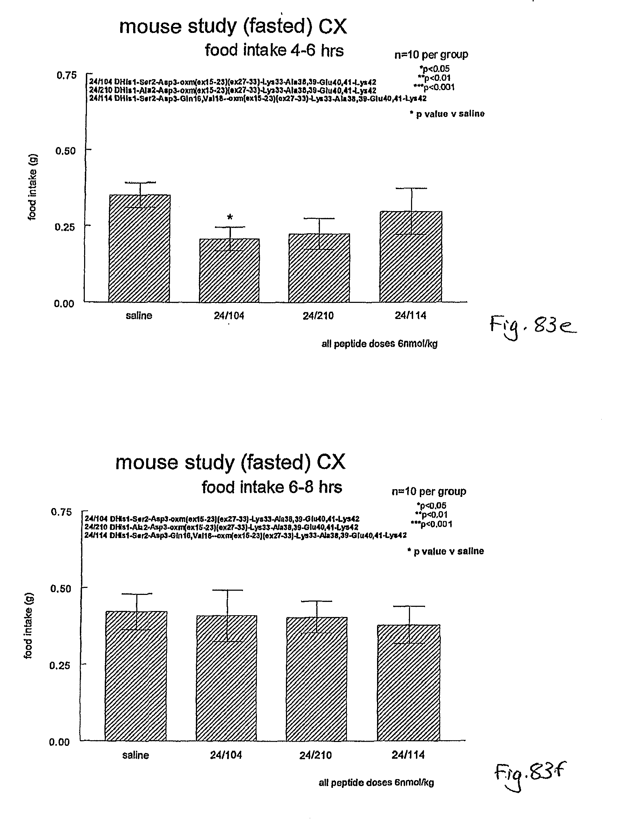

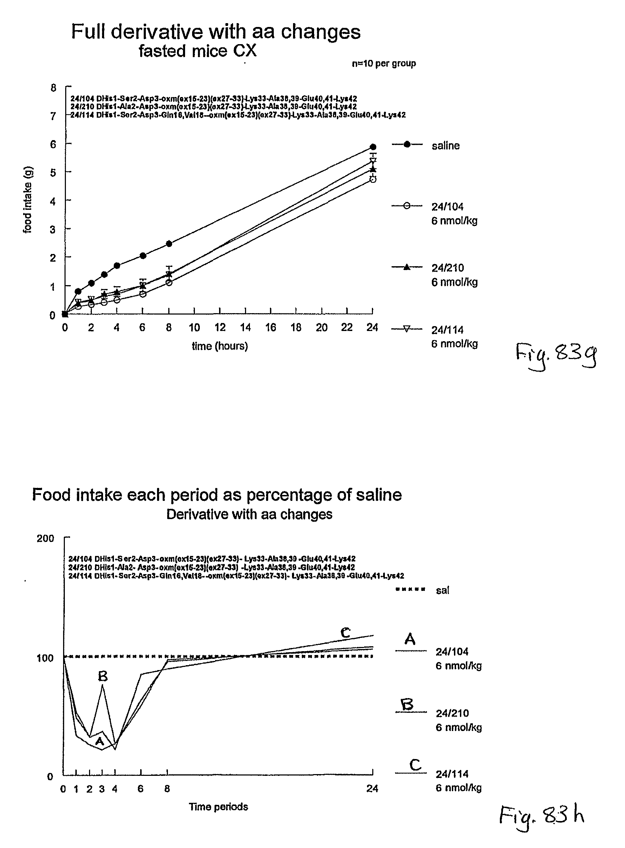

All compounds were administered at a dose of 6nmol/kg
The measured food intake for each group is shown in Fig. 83.

### Example 74

Groups of fasted mice were injected with the following:
15/1 DHis1-Ala2-oxm(ex15-24)(ex27-33) (SEQ ID NO : 51)
24/40 DHis1-Ala2-oxm(ex15-23)(ex27-33)-Ala38,39 (SEQ ID NO : 86)
24/29 DHis1-Ala2-oxm(ex15-23)(ex27-33)-Ala38,39-Lys40 (SEQ ID NO : 85)
24/75 DHis1-Ala2-oxm(ex15-23)(ex27-33)-Ala38,39-Lys40,41-Lys42 (SEQ ID NO : 137)
23/36 DHis1-Ala2-oxm(ex15-23)(ex27-33)-Ala38,39-Lys40-Lauroyl (SEQ ID NO : 105)
23/75 DHis1-Ala2-oxm(ex15-23)(ex27-33)-Ala38,39-Lys40,41-Lys42-Pahnitoyl (SEQ ID NO : 131)
A further group were administered saline (control).

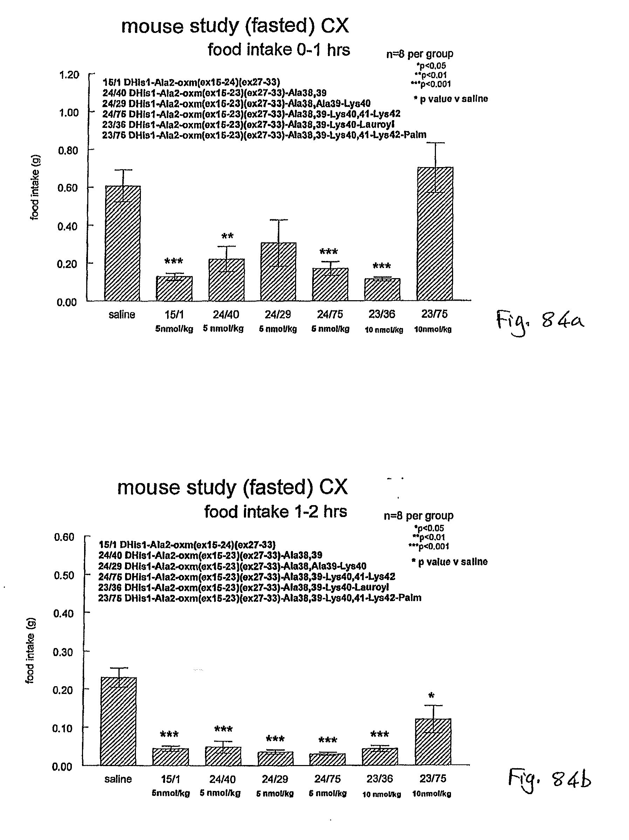

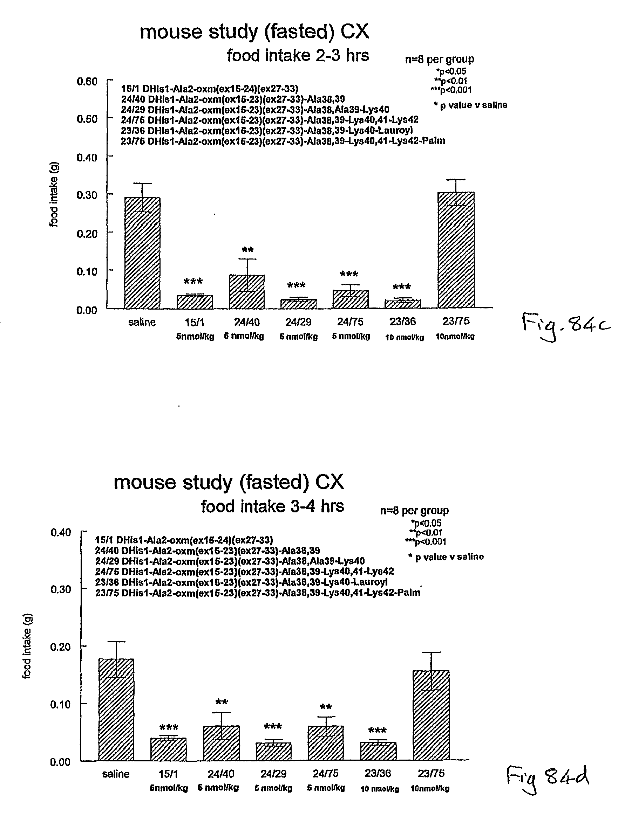

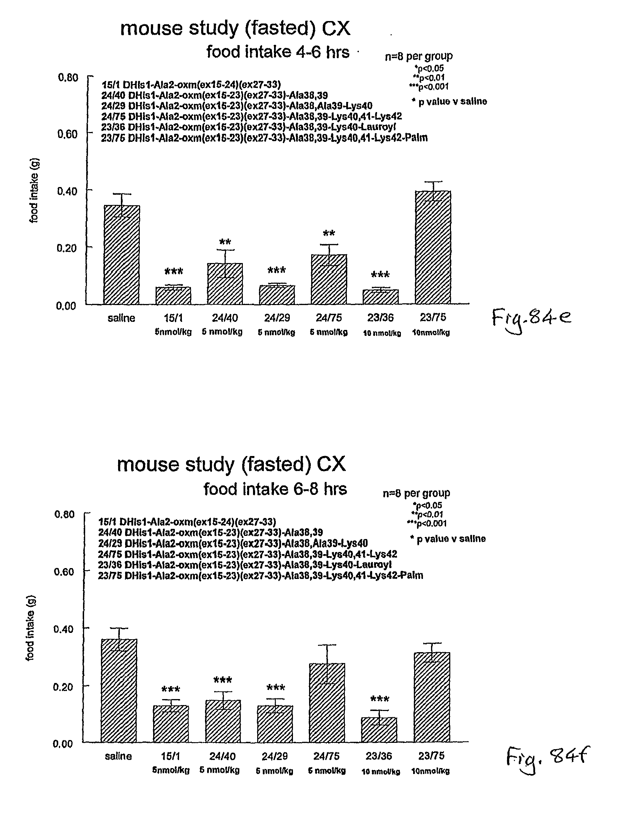

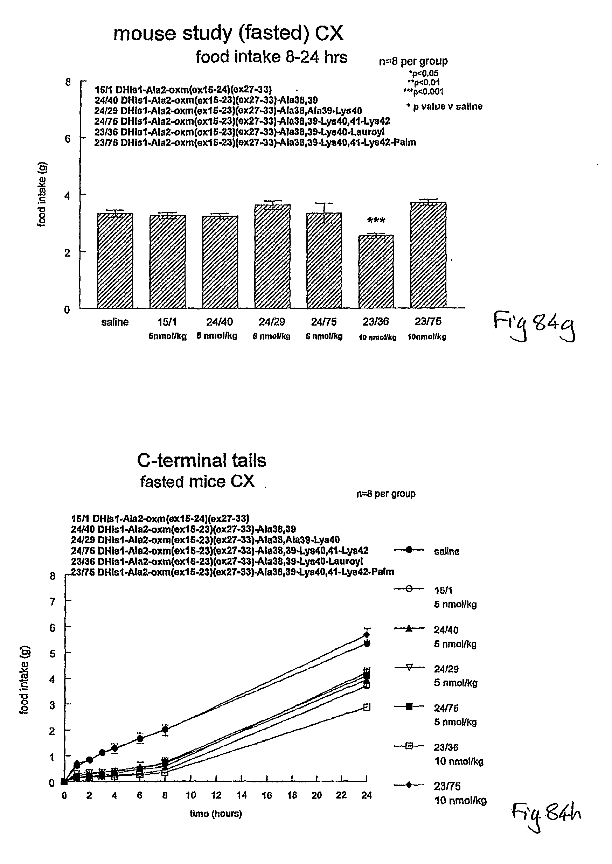

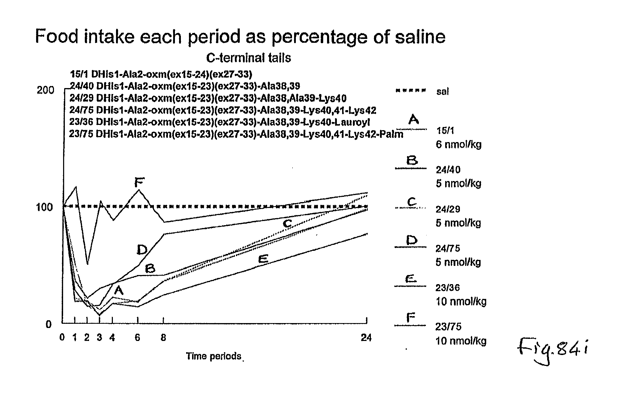

Compounds were injected at the doses stated in Fig. 84.
The measured food intake for each group is shown in Fig. 84.

### Example 75

Groups of fasted mice were injected with the following:
25/1 Met,Asn,Glu,Asp,Lys,Arg-oxm(ex15-23)(ex27-33)-Ala38,39 (SEQ ID NO : 138)
25/2 Met,Asn,Glu,Asp,Lys,Arg-DHis1-Ala2-oxm(ex15-23)(ex27-33)-Ala38,39 (SEQ ID NO : 139)
A further group were administered saline (control).

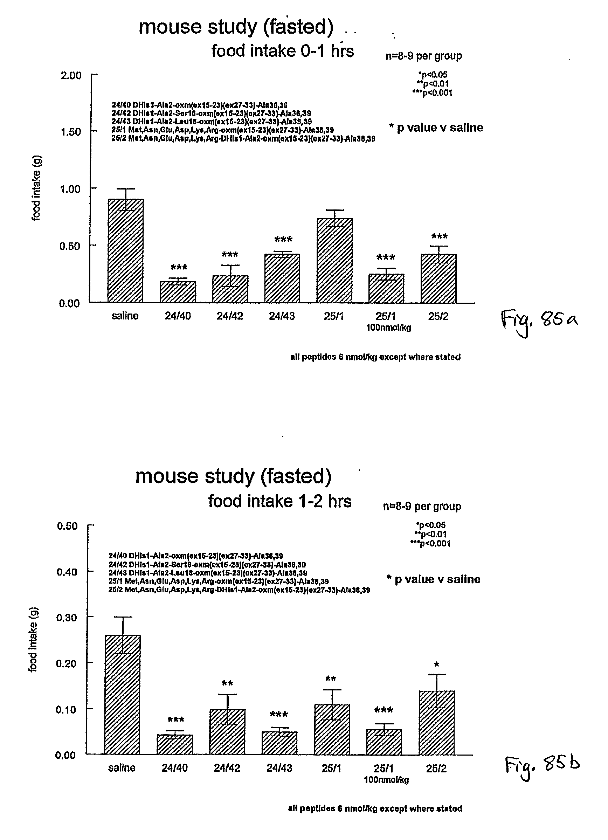

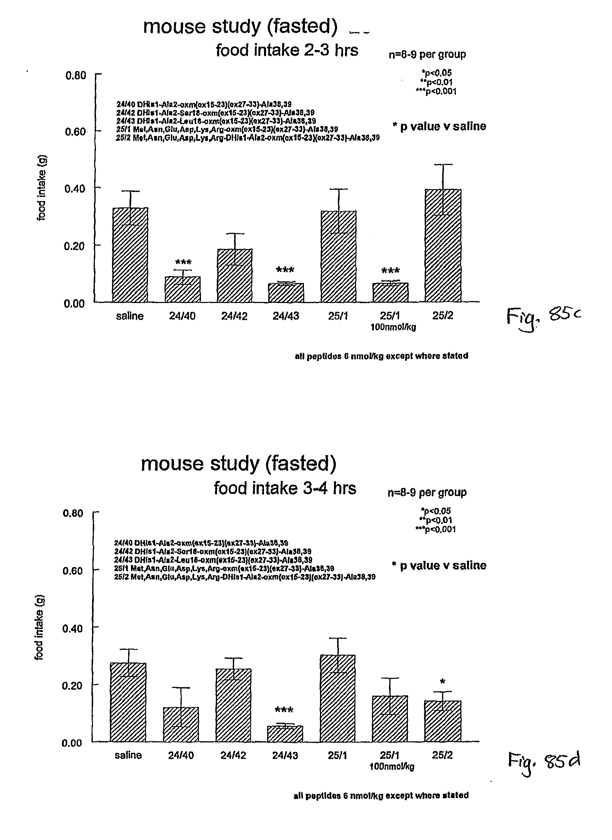

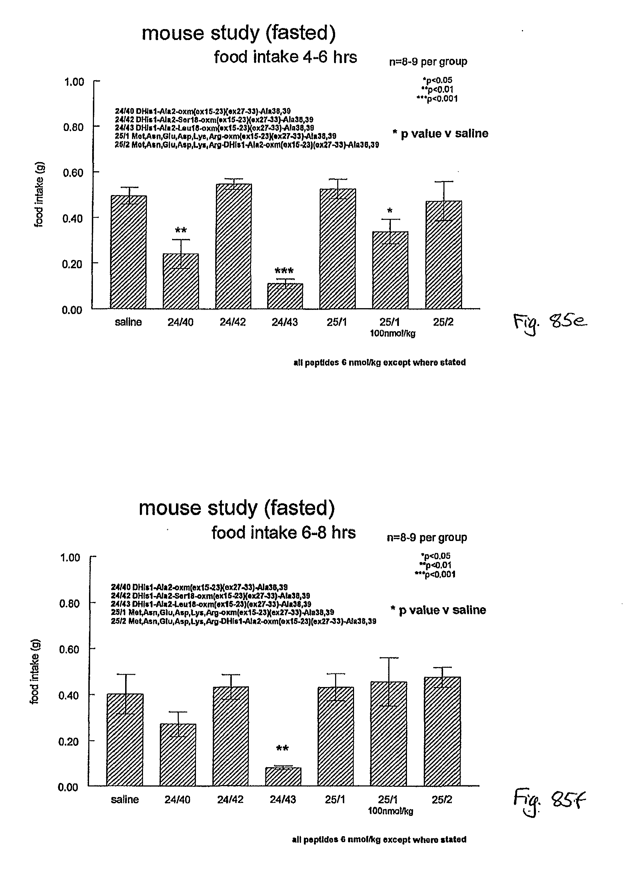

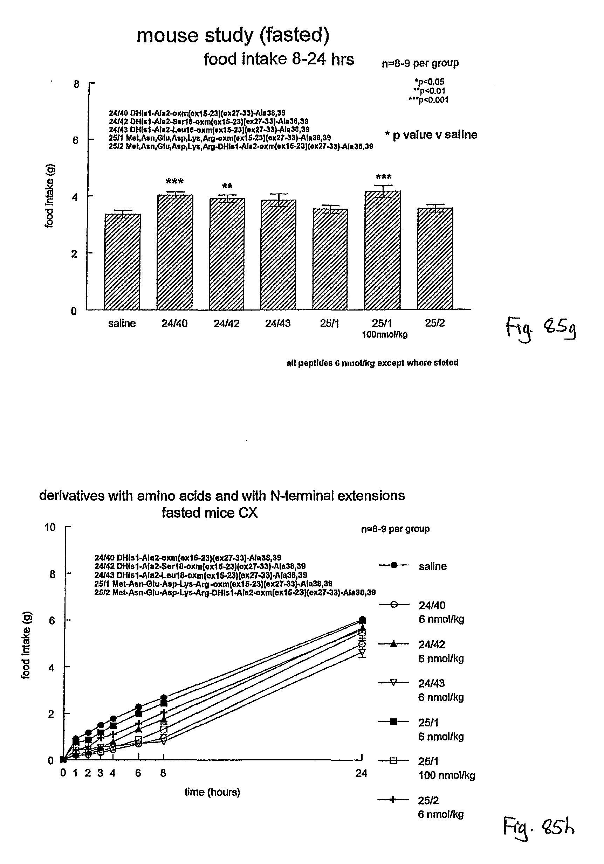

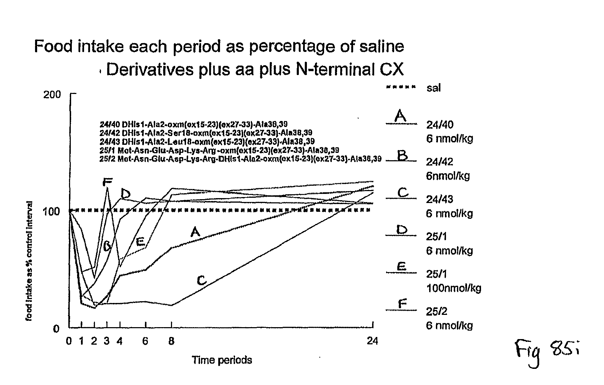

Compounds were injected at the doses stated in Fig. 85
The measured food intake for each group is shown in Fig. 85. (Other data not referred to in this example is also presented in Fig. 85.)

### Example 76

Groups of fasted mice were injected with the following:
23/36 DHis1-Ala2-oxm(ex15-23)(ex27-33)-Ala38,39-Lys40-Lauroyl (SEQ ID NO : 105)
28/75 DHis1-Ala2-oxm(ex15-23)(ex27-33)-Ala38,39-Lys40,41-Lys42-Mouse albumin (albumin-conjugate ratio was 20:1) (SEQ ID NO : 140)
A further group were administered saline (control).

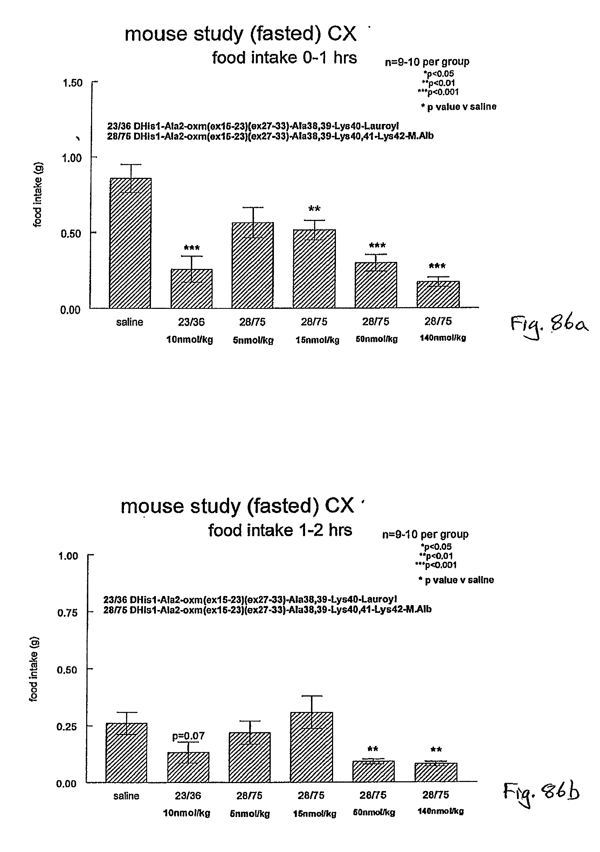

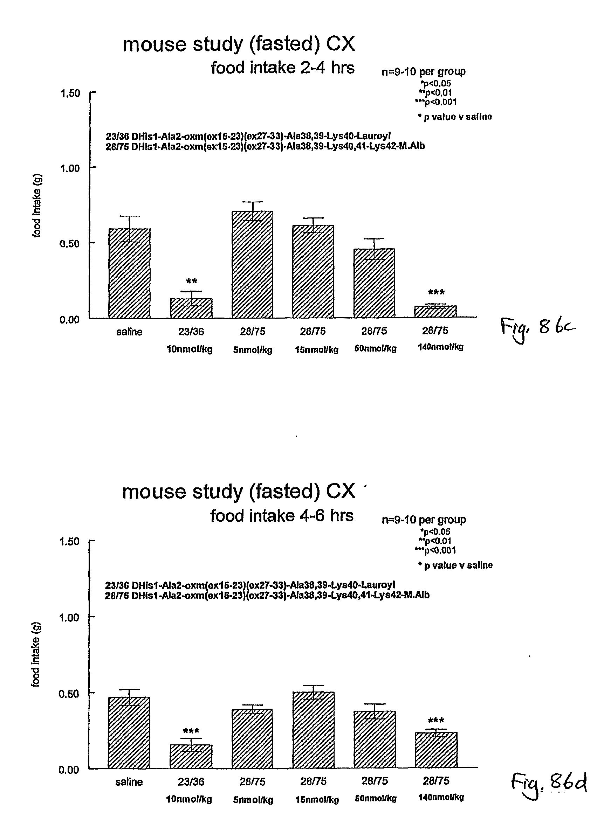

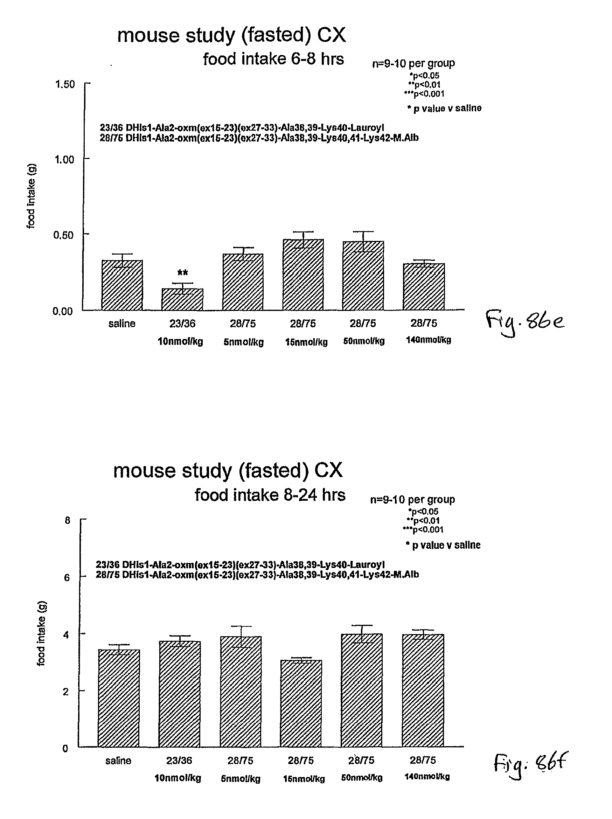

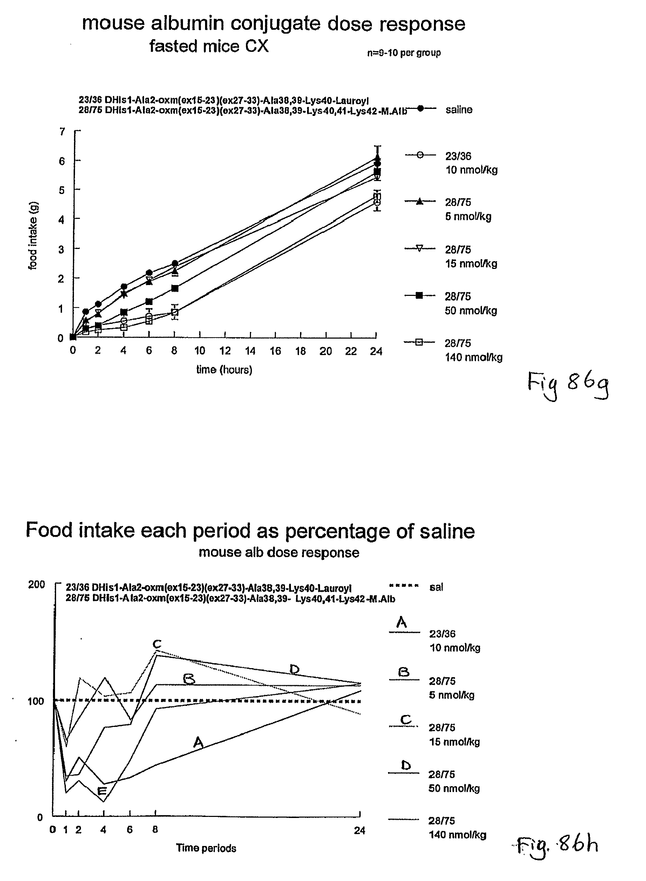

Compounds were injected at the doses stated in Fig. 86.
The measured food intake for each group is shown in Fig. 86.

### Example 77

Groups of fasted mice were injected with the following:
24/76 DHis1-Ala2-oxm(ex15-23)(ex27-33)-Lys33-Ala38,39-Glu40,41-Lys42 (SEQ ID NO : 113)

24/210 DHis1-Ala2-Asp3-Gln16,Val18-oxm(ex15-23)(ex27-33)-Lys33-Ala38,39-Glu40,41-
Lys42 (SEQ ID NO : 114)

23/210 DHis1-Ala2-Asp3-Gln16,Val18-oxm(ex15-23)(ex27-33)-Lys33-Ala38,39-Glu40,41-
Lys42-Octanoyl (SEQ ID NO : 115)

27/210 DHis1-Ala2-Asp3-Gln16,Val18-oxm(ex15-23)(ex27-33)-Lys33-Ala38,39-Glu40,41-
Lys42-PEG (albumin-conjugate ratio was 20:1) (SEQ ID NO : 116)

A further group were administered saline (control).

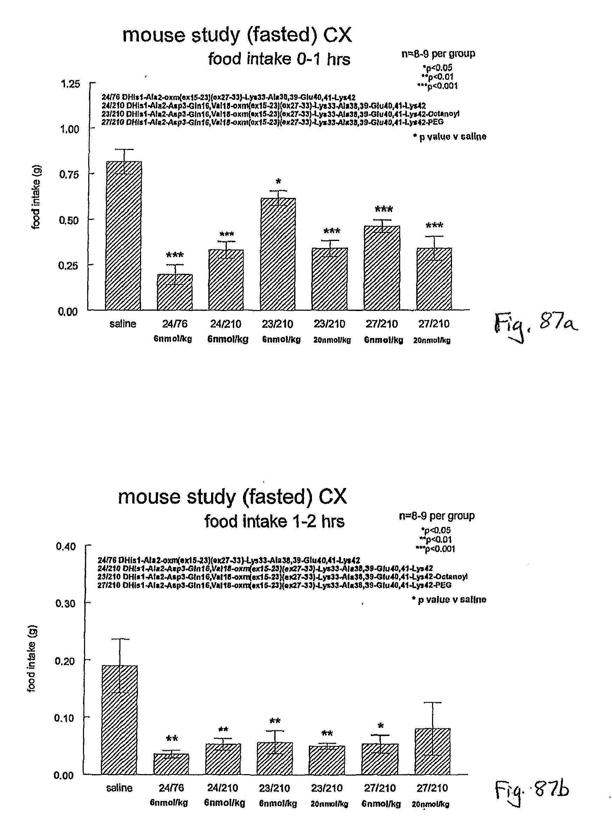

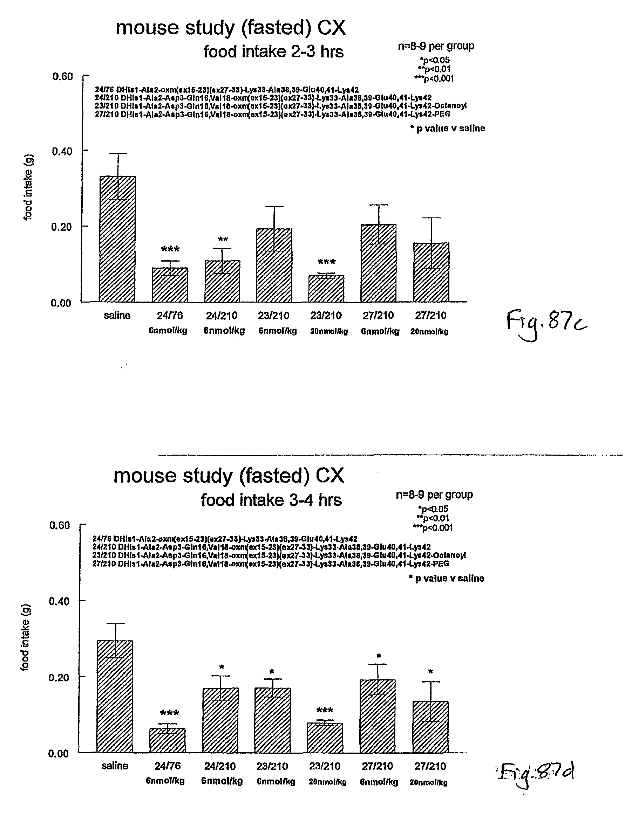

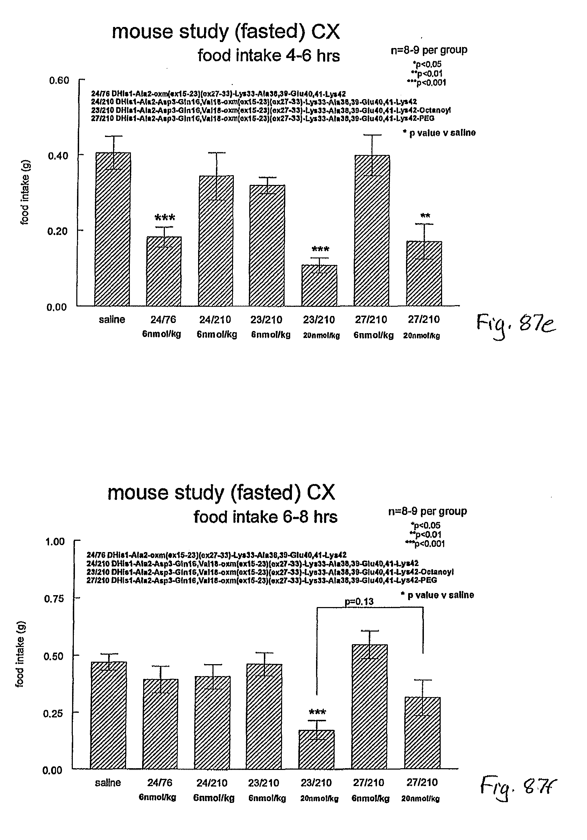

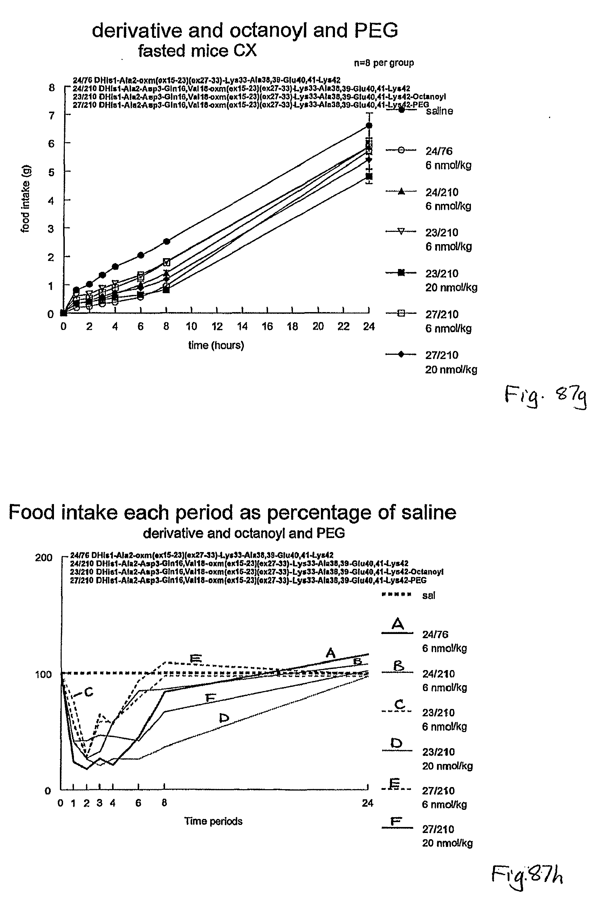

Compounds were injected at the doses stated in Fig. 87.
The measured food intake for each group is shown in Fig. 87.

### Example 78

Groups of fasted mice were injected with the following:
23/36 DHis-Ala2-oxm(ex15-23)(ex27-33)-Ala38,39-Lys40-Lauroyl (SEQ ID NO : 105)
28/76 DHis1-Ala2-oxm(ex15-23)(ex27-33)-Ala38,39-Lys40,41-Lys42-Mouse Albumin (SEQ ID NO : 140) (albumin-conjugate ratio was 2: 1)

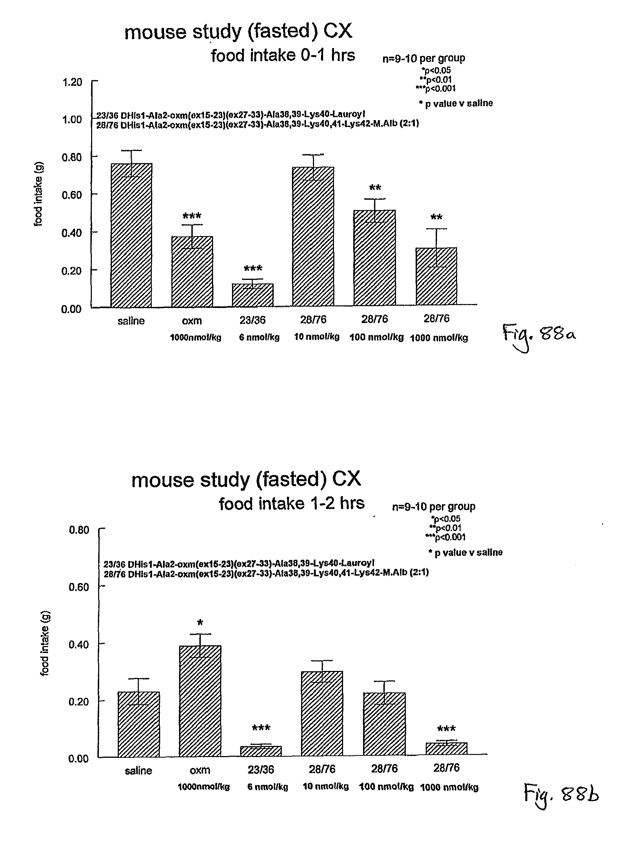

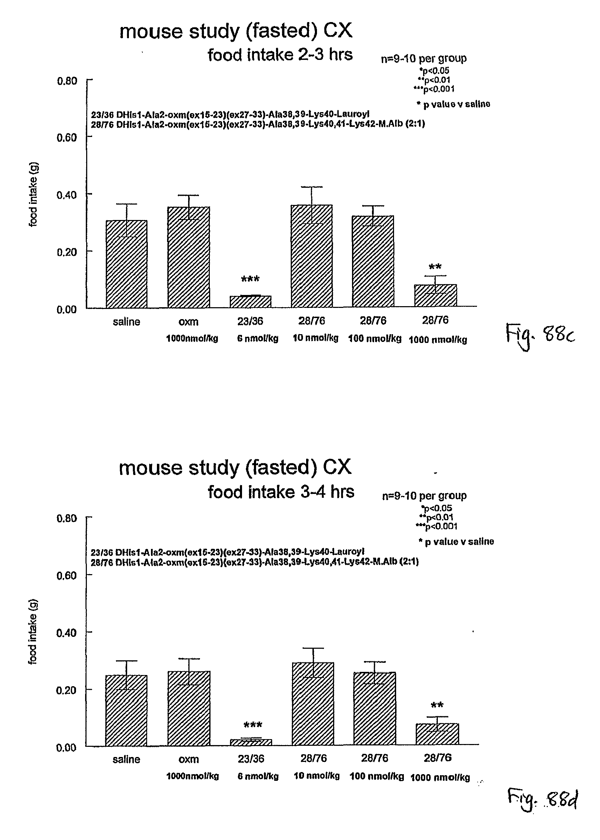

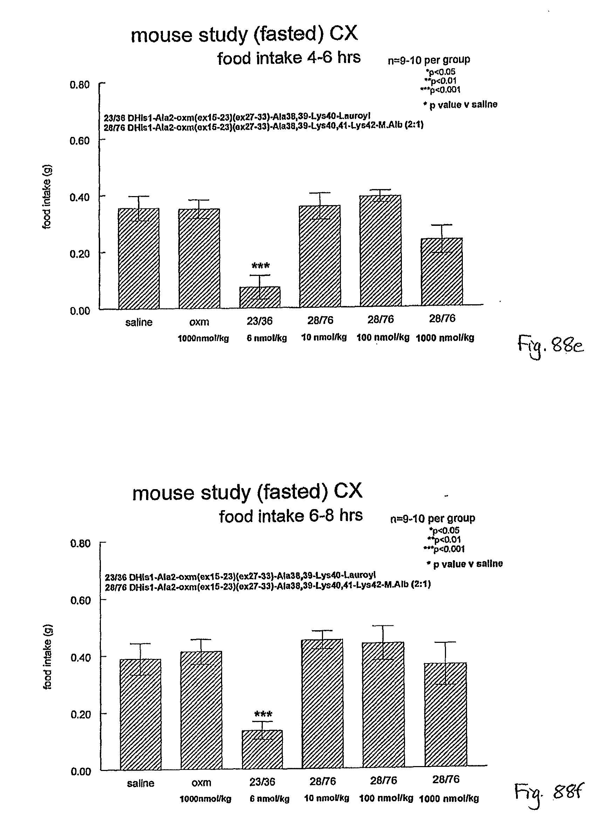

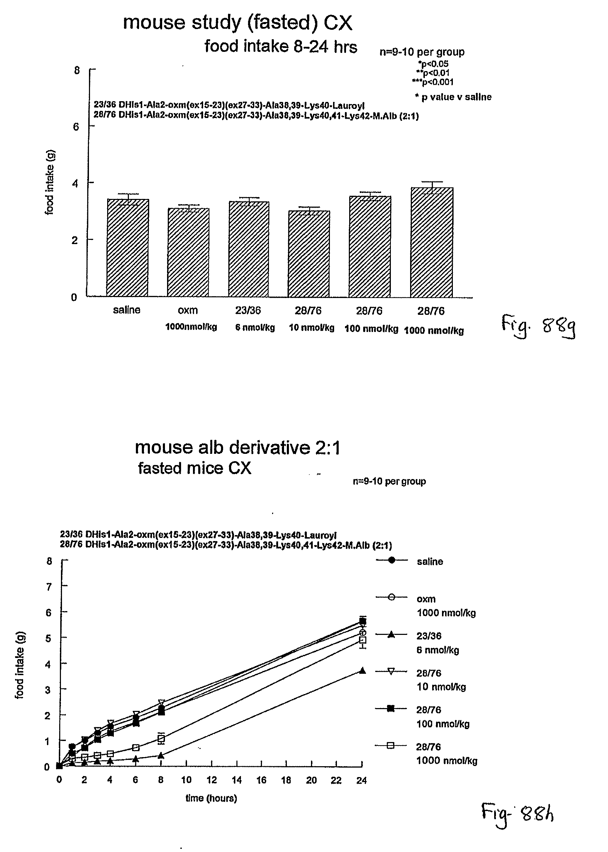

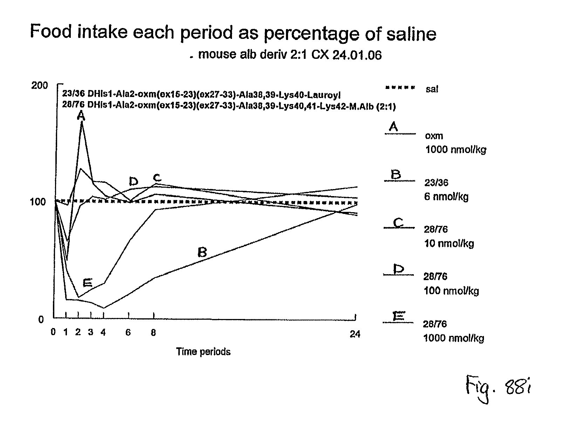

A further group were administered saline (control).
Compounds were injected at the doses stated in Fig. 88
The measured food intake for each group is shown in Fig. 88

### Example 79

Groups of fasted mice were injected with the following:
saline
exendin-4 (SEQ ID NO : 22)
23/36 DHis1-Ala2-oxm(ex15-23)(ex27-33)Ala38,39-Lys40-Lauroyl. (SEQ ID NO : 105)

Injections were once daily for first 7 days and then twice daily until day 10.

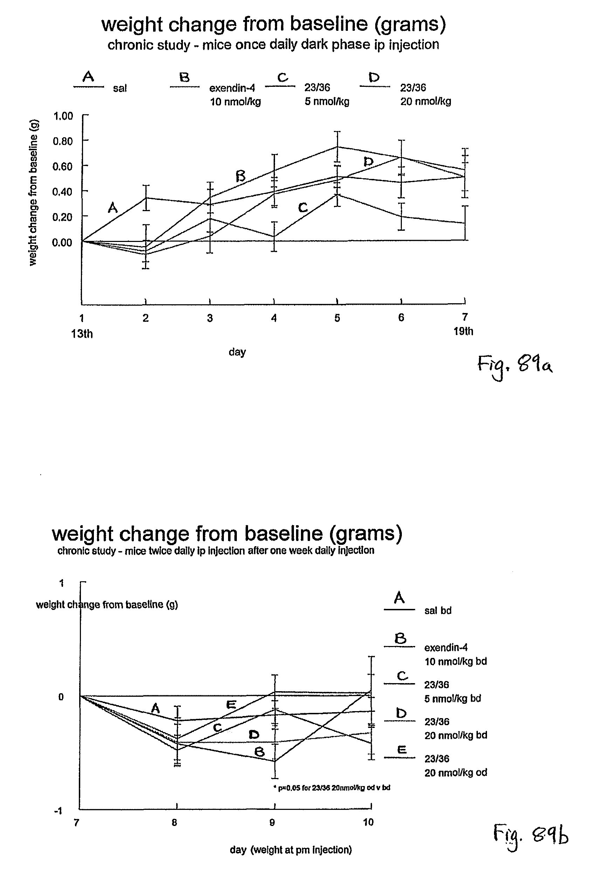

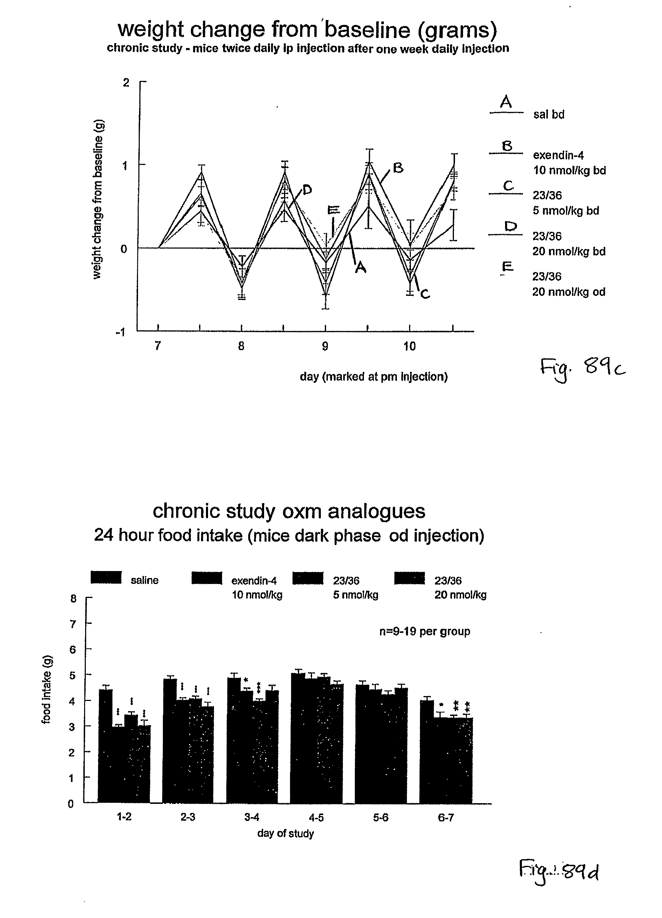

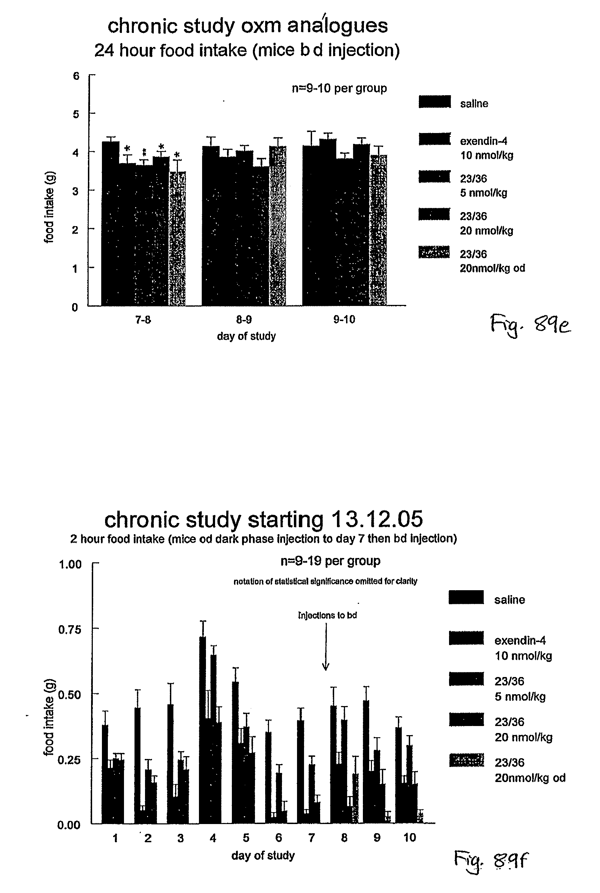

Compounds were injected at the doses stated in Fig. 89
Fig. 89a shows weight change from baseline in mice injected once daily (during dark phase) during the day 1 to day 7 period of study.
Fig. 89b shows weight change from baseline in mice injected twice daily (during dark phase) during the day 7 to day 10 period of study.
Fig. 89c shows weight change from baseline in mice injected twice daily (during dark phase) during the 7 to 10 day period of study.
Fig. 89d shows 24 hour food intake during the day 1 to day 7 period of study.
Fig. 89e shows 24 hour food intake during the day 7 to day 10 period of study.
Fig 89f shows food intake during the 2 hour period following evening injection for the day 1 to day 10 study period.

### Example 80

Groups of fasted rats were injected with the following:
saline
23/36 DHis1-Ala2-oxm(ex15-23)(ex27-33)-Ala38,39-Lys40-Lauroyl (SEQ ID NO : 105)

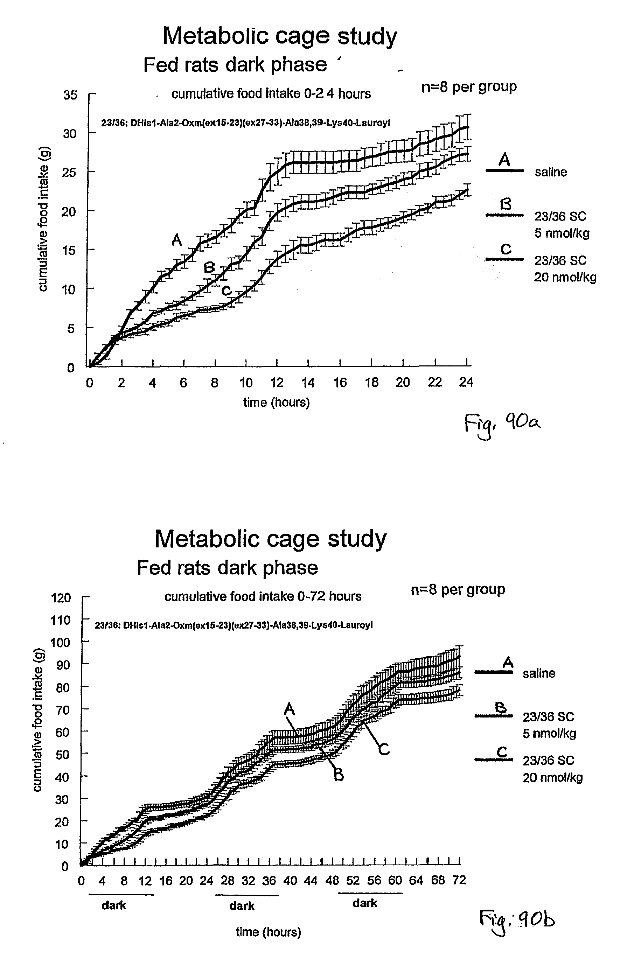

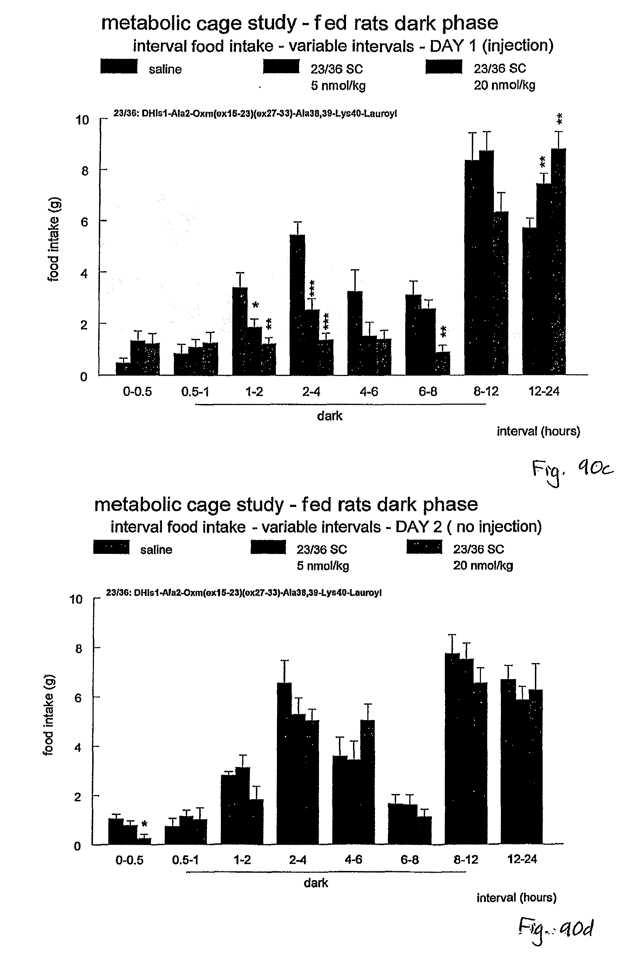

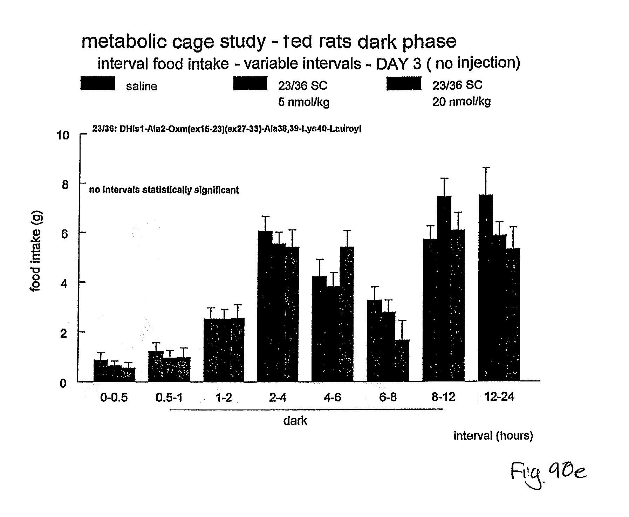

Doses were as stated in Fig. 90. Subcutnaeous injections were once daily in the evening for the first 3 days and food intake followed for 72 hours. Data is presented in Fig. 90.

### Example 81

Groups of fasted large rats (top weight = 420g) were injected ip or sc with the following:
saline
23/36 DHis1-Ala2-oxm(ex15-23)(ex27-33)-Ala38,39-Lys40-Lauroyl (SEQ ID NO : 105)

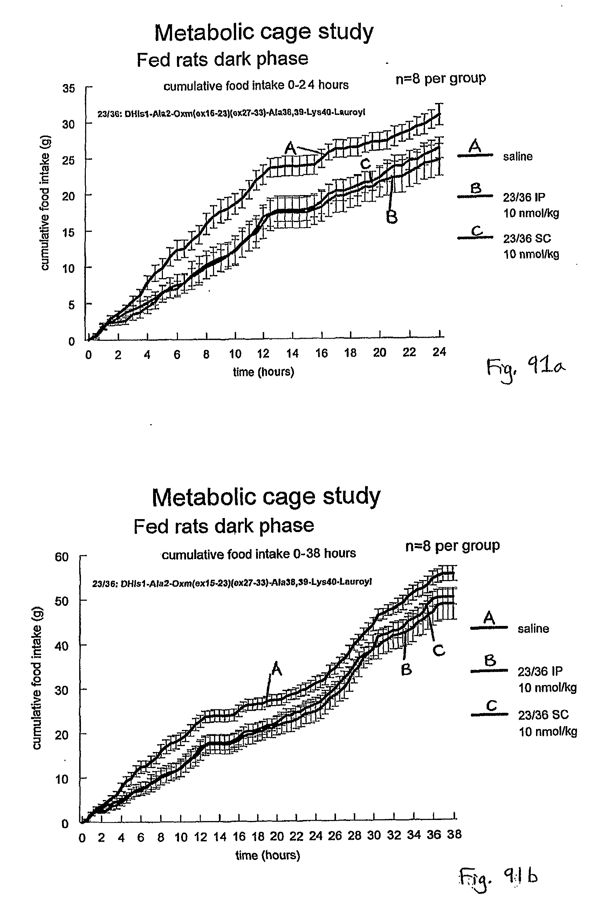

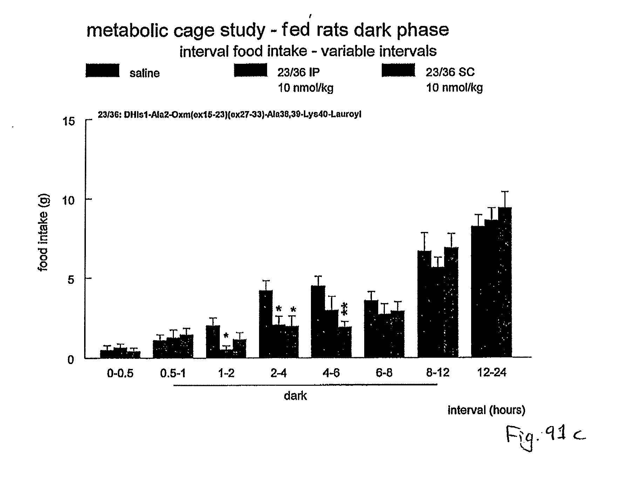

Doses were 10nmol/kg. Data is presented in Fig. 91.

### Example 82

Groups of fasted rats were injected with the following:

saline
23/104 DHis2-Ser2-Asp3-oxm(ex15-23)(ex27-33)-Lys33-Ala38,39-Glu40,41-Lys42-Lauroyl ip
at 10nmol/kg (SEQ ID NO:120)
23/104 DHis2-Ser2-Asp3-oxm(ex15-23)(ex27-33)-Lys33-Ala38,39-Glu40,41-Lys42-Lauroyl sc
at 5, 10 or 30 nmol/kg (SEQ ID NO: 120)

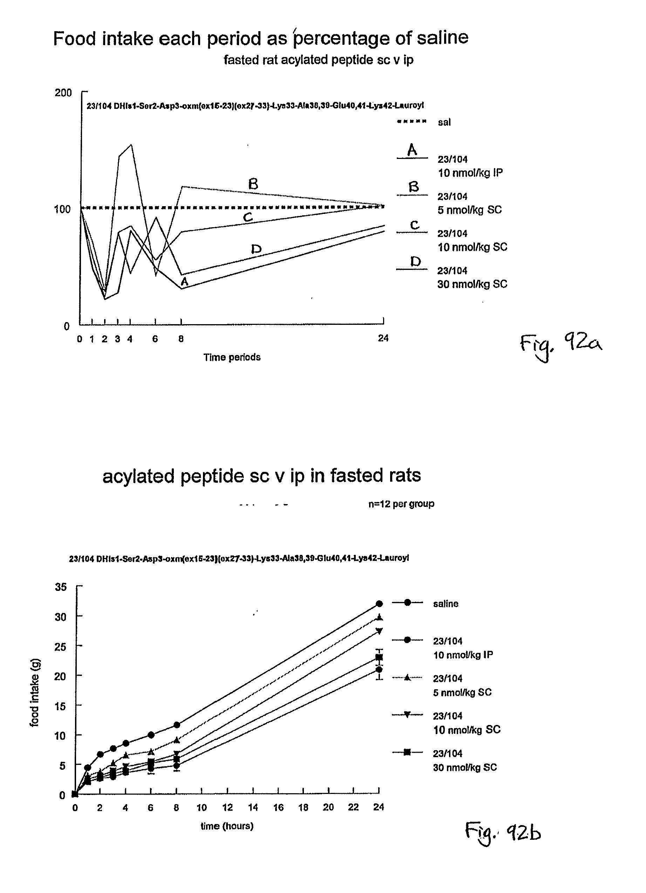

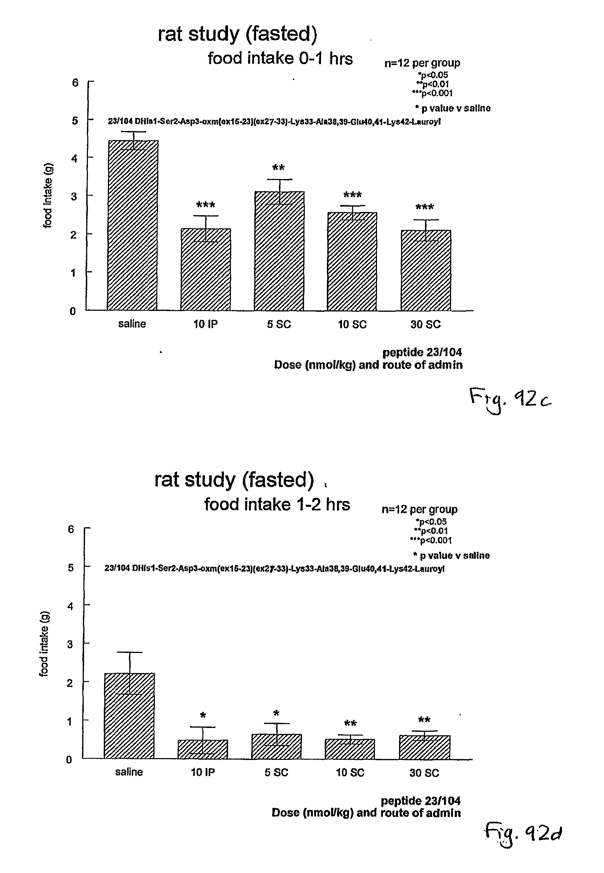

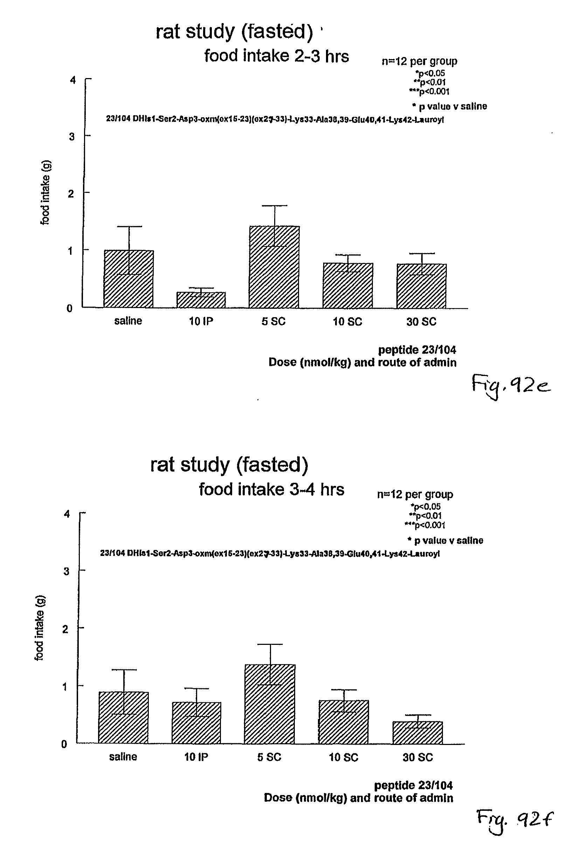

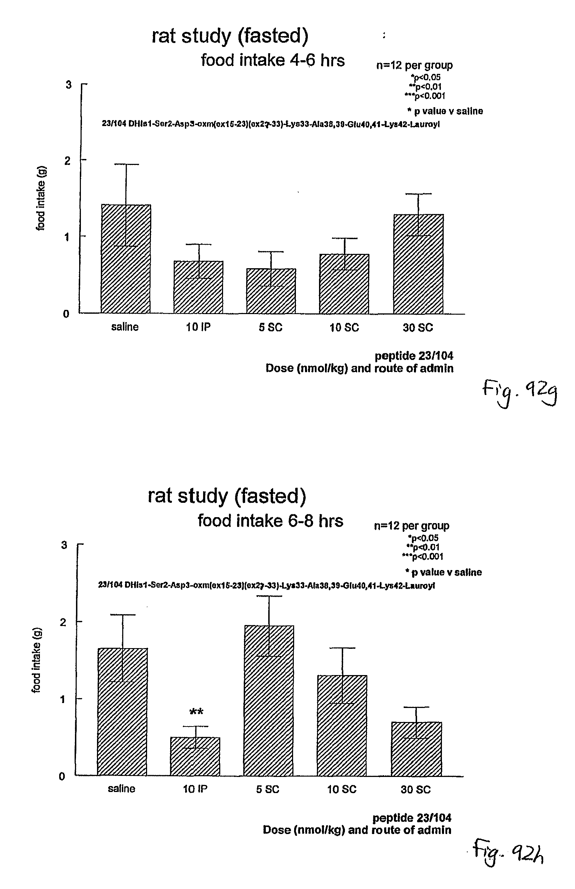

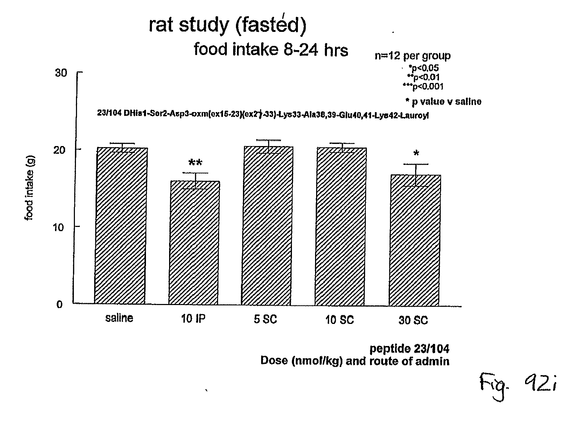

Feed intake data is presented in Fig. 92. It can be seen that peptide delivered by ip injection performs better than that delivered by the sc route.

### Example 83

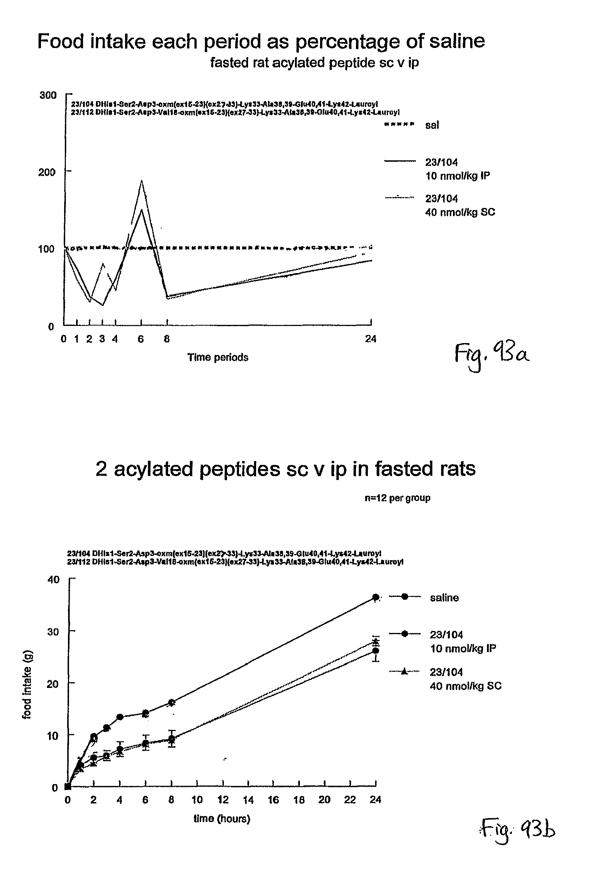

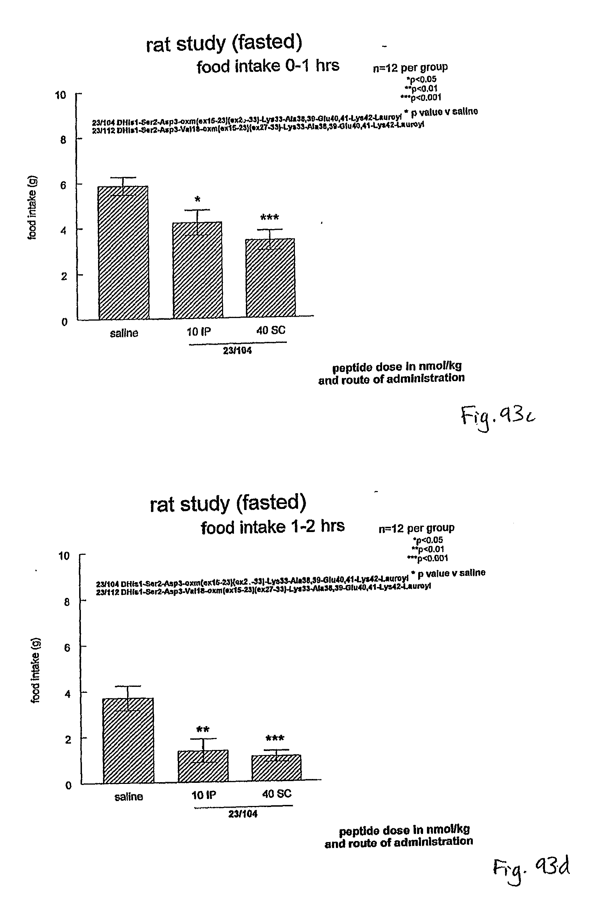

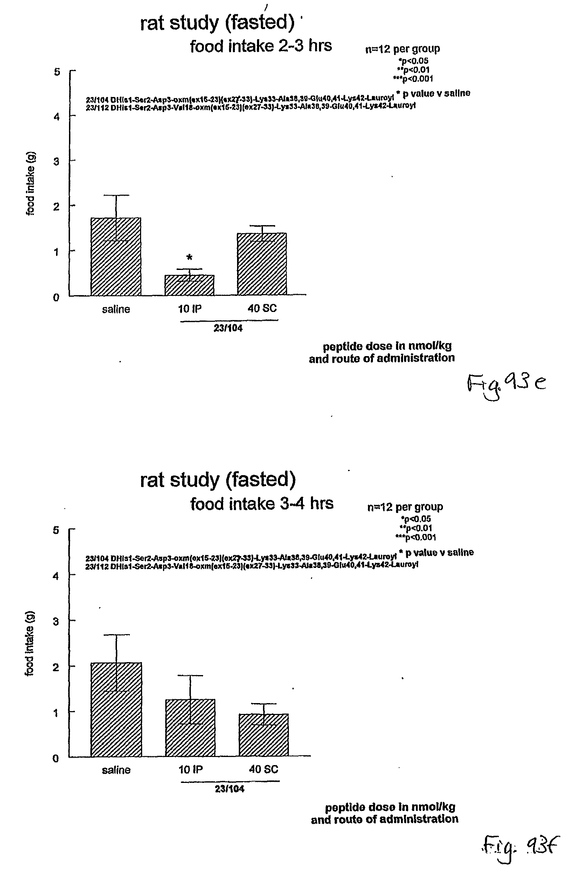

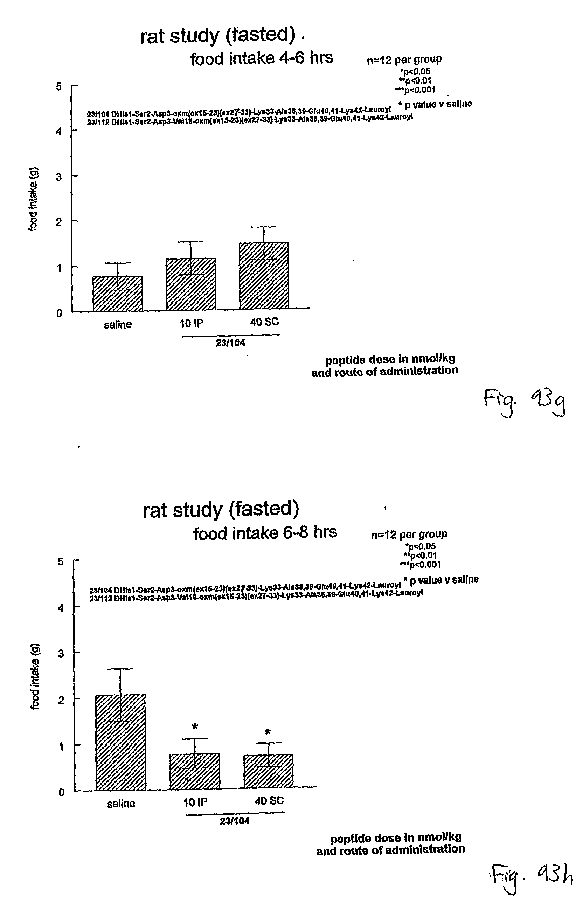

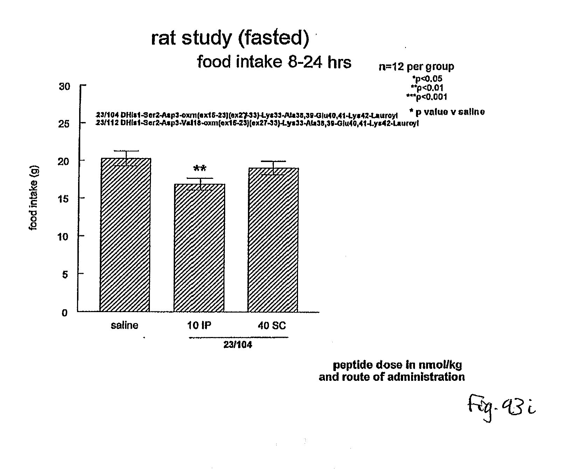

Groups of fasted rats were injected with the following:
saline
23/104 DHis2-Ser2-Asp3-oxm(ex15-23)(ex27-33)-Lys33-Ala38,39-Glu40,41-Lys42-Lauroyl (SEQ ID NO :120)
Doses and route of administration (ip or sc injection) were as stated in Fig. 93. Food intake data is presented in Fig. 93. It can be seen that the peptides showed biological activity when delivered by either route.

### Example 84

Groups of fasted rats were injected with the following:
saline
23/36 DHis1-Ala2-oxm(ex15-23)(ex27-33)-Ala38,39-Lys40Lauroyl (SEQ ID NO : 105)

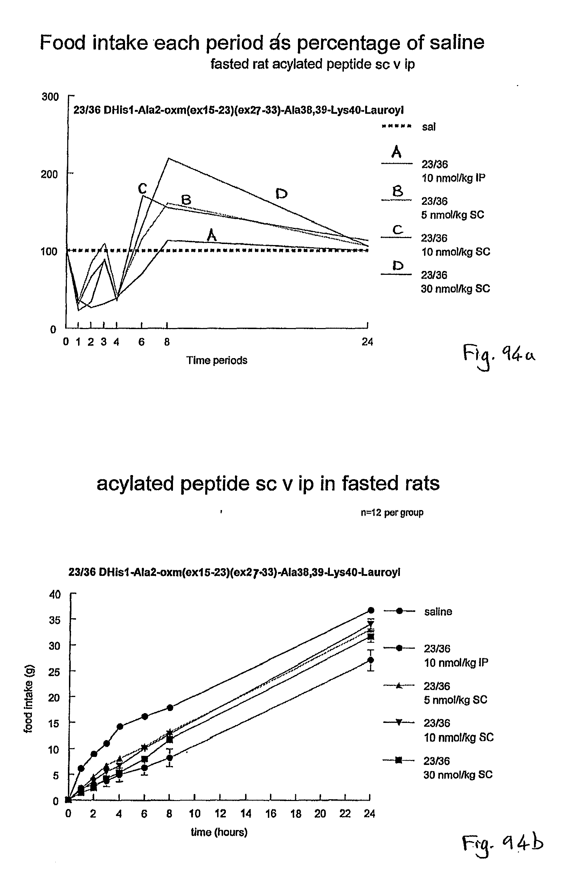

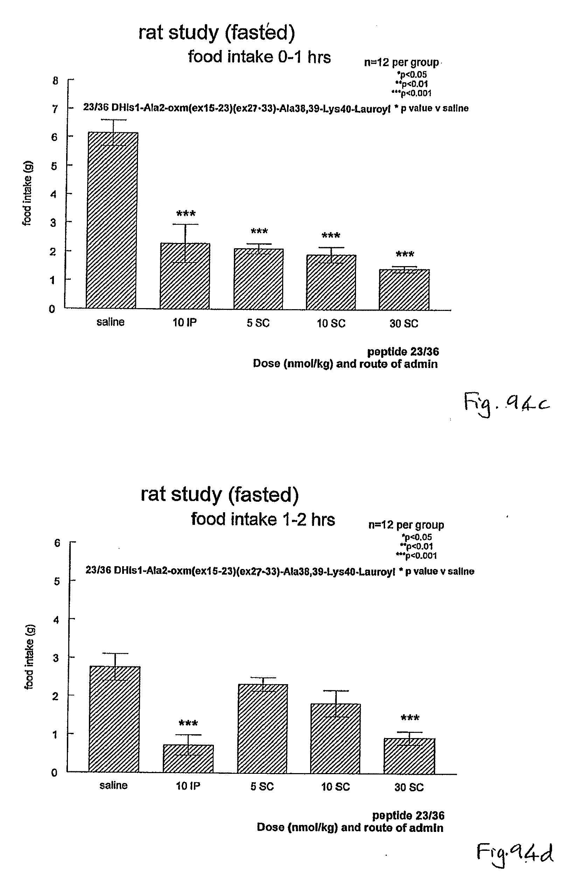

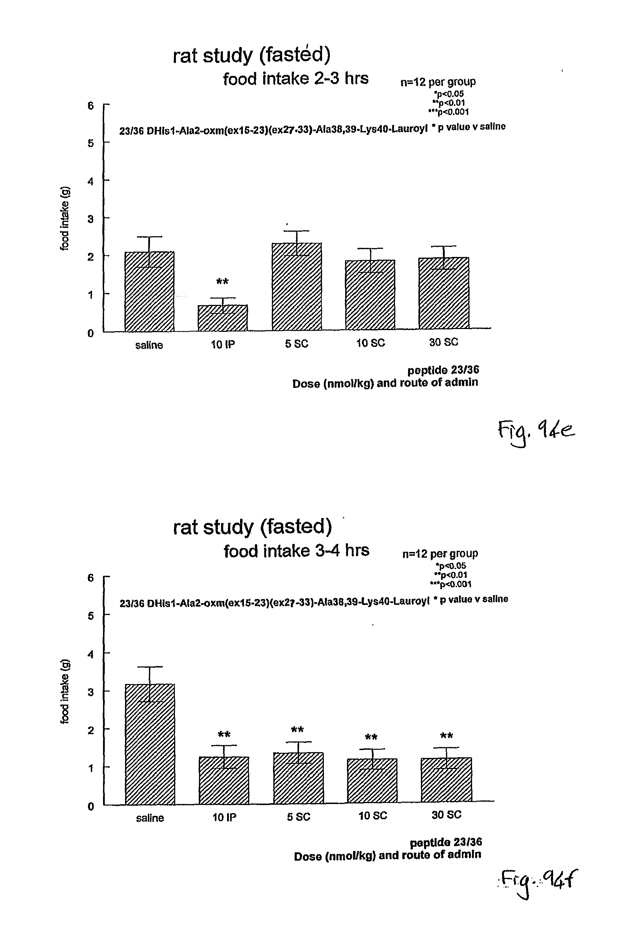

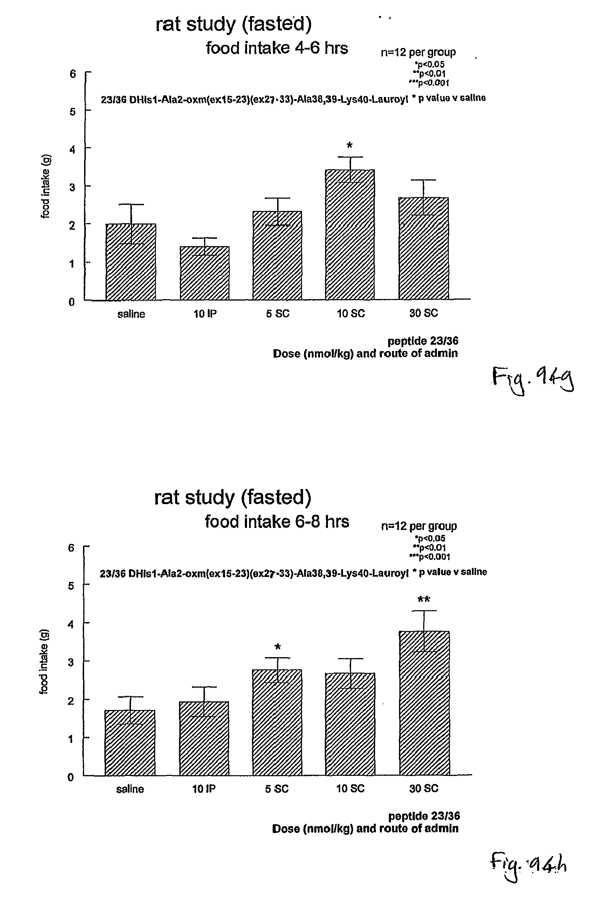

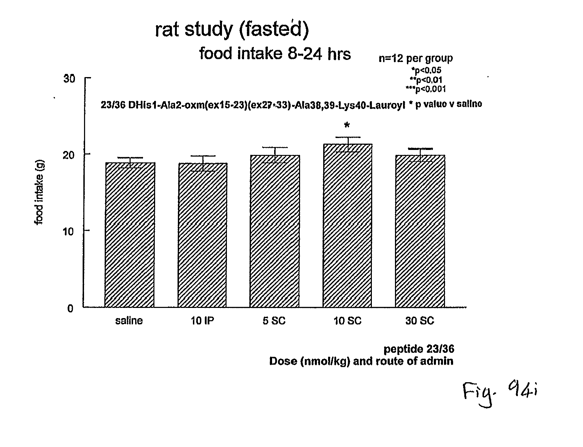

Doses and route of administration (ip or sc injection) were as stated in Fig. 94. Food intake data is presented in Fig. 94. It can be seen that the peptide showed biological activity when delivered by either route.

### Example 85

Groups of fasted rats were injected with the following:
saline
24/40 DHis1-Ala2-oxm(ex15-23)(ex27-33)-Ala38,39 (SEQ ID NO : 86)
24/43 DHis1-Ala2-Leul8-oxm(ex15-23)(ex27-33)-Ala38,39 (SEQ ID NO : 128)

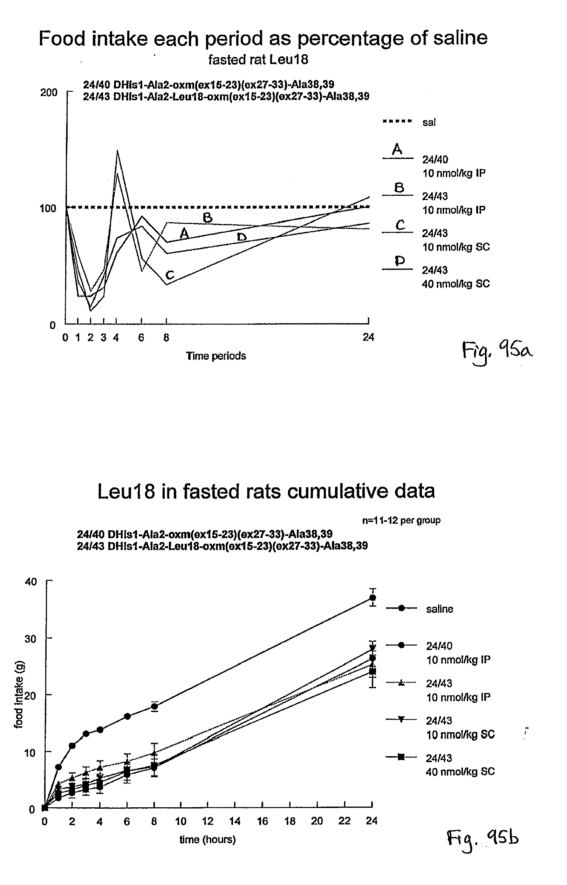

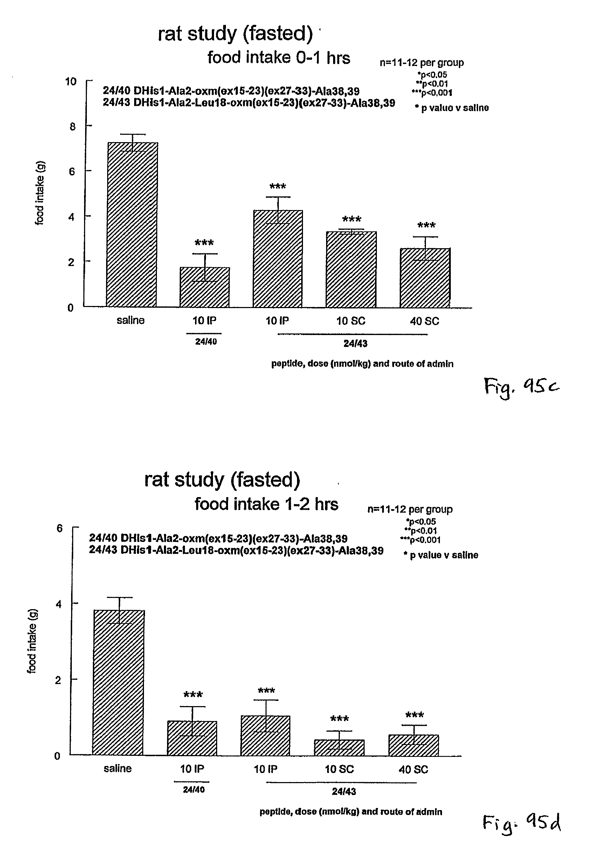

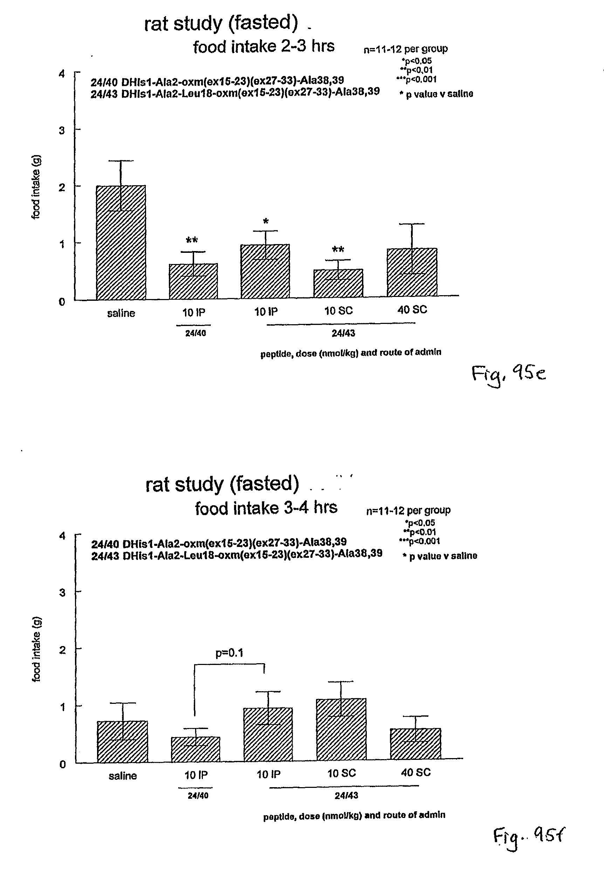

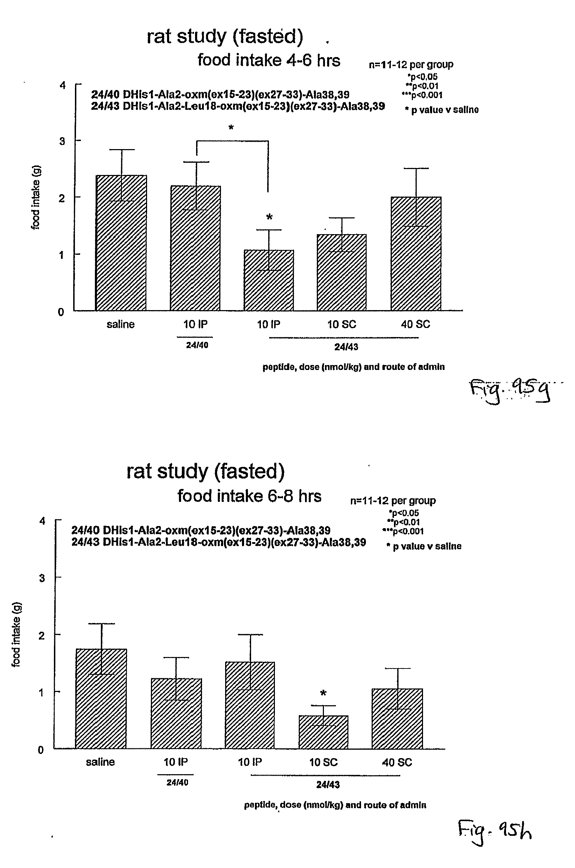

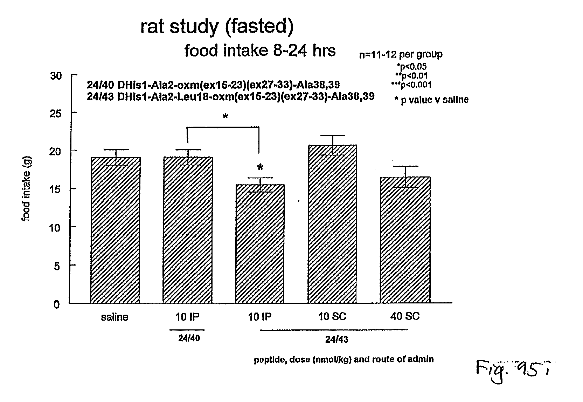

Doses and route of administration (ip or sc injection) were as stated in Fig. 95. Food intake data is presented in Fig. 95. It can be seen that the peptides showed biological activity when delivered by either route.

### Example 86

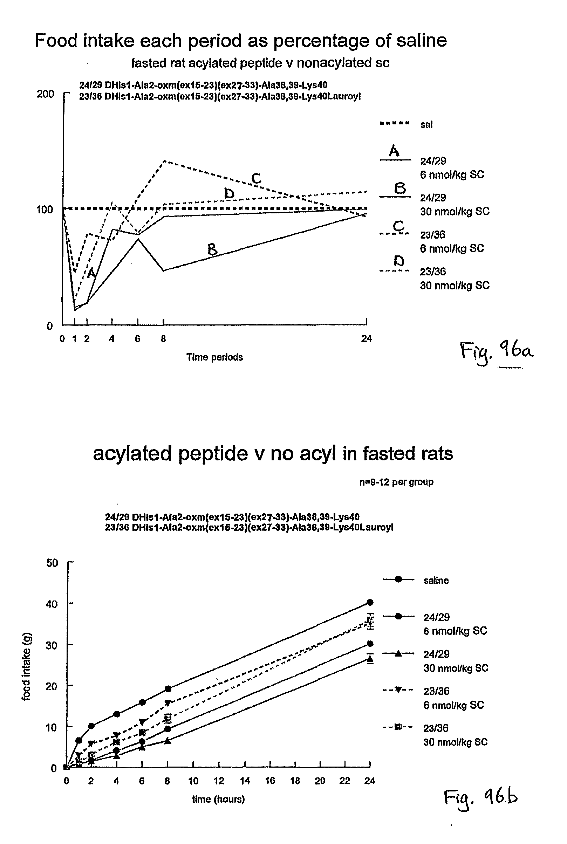

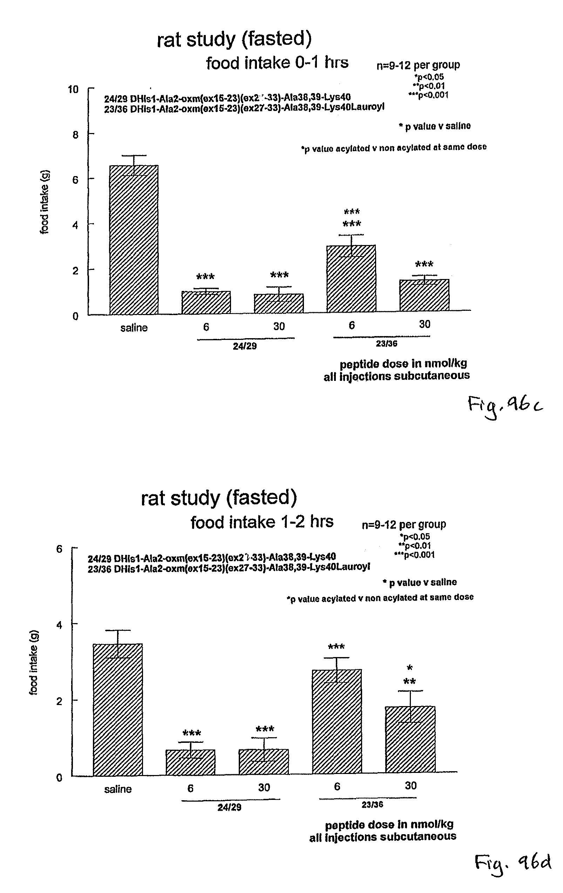

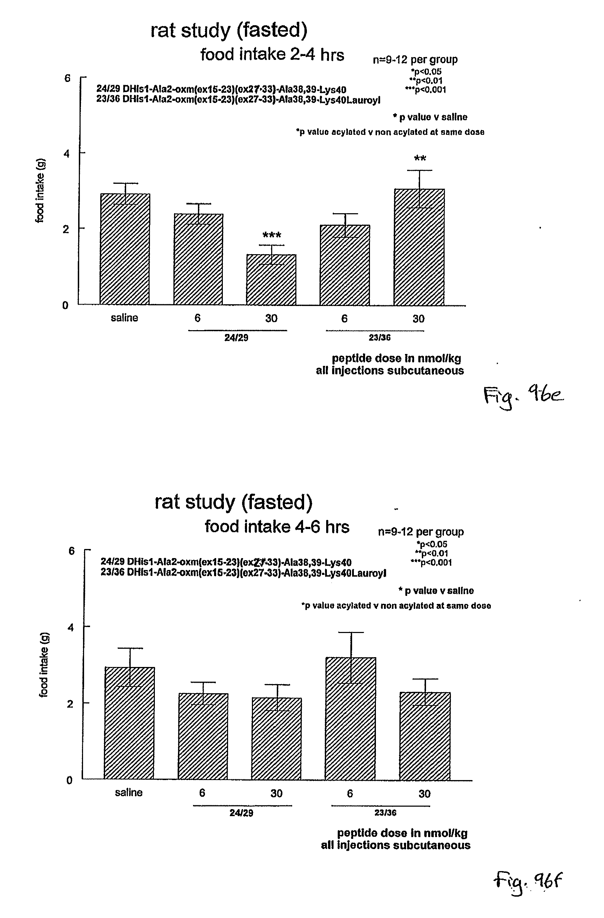

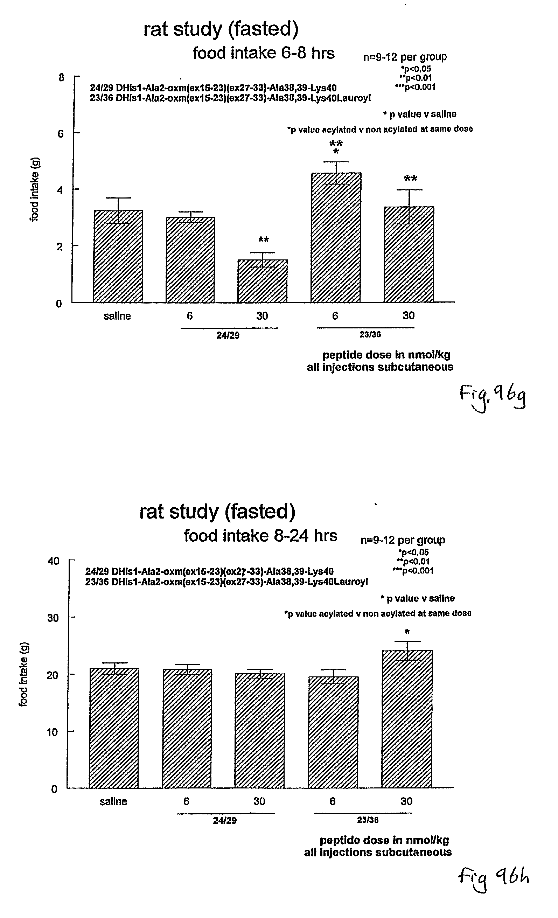

Groups of fasted rats were injected sc with the following:
saline
24/29 DHis1-Ala2-oxm(ex15-23)(ex27-33)-Ala38,39-Lys40 (SEQ ID NO : 85)
23/36 DHis1-Ala2-oxm(ex15-23)(ex27-33)-Ala38,39-Lys40Lauroyl (SEQ ID NO : 105)
Doses were as stated in Fig. 96. Food intake data is presented in Fig. 96.

### Example 87

Groups of fasted rats were injected sc with the following:
saline
24/78 DHis1-Ala2-oxm(ex15-23)(ex27-33)-Lys33-Ala38,39-Glu40,41-Lys42 (SEQ ID NO : 113)
23/79 DHis1-Ala2-oxm(ex15-23)(ex27-33)-Lys33-Ala38,39-Glu40,41-Lys42Palmitoyl (SEQ ID NO: 110)

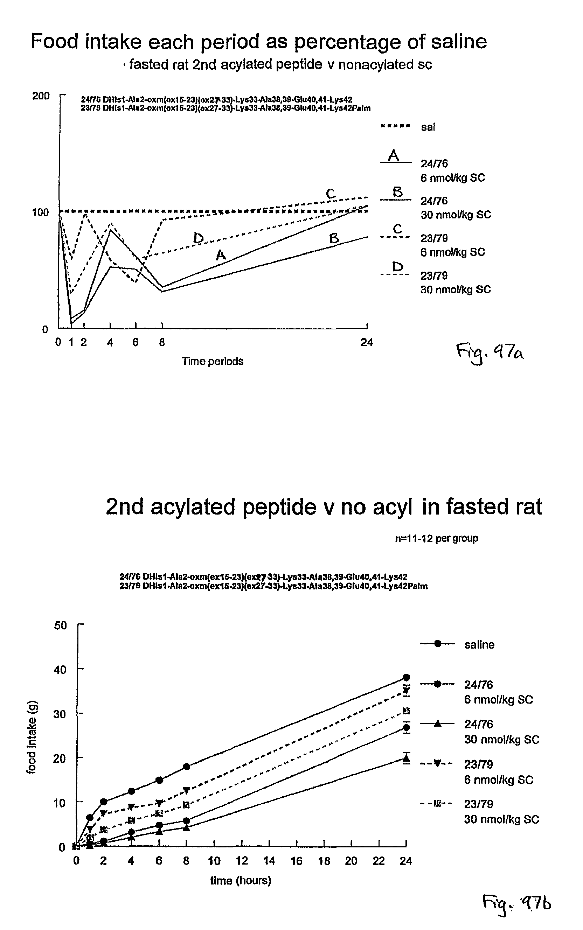

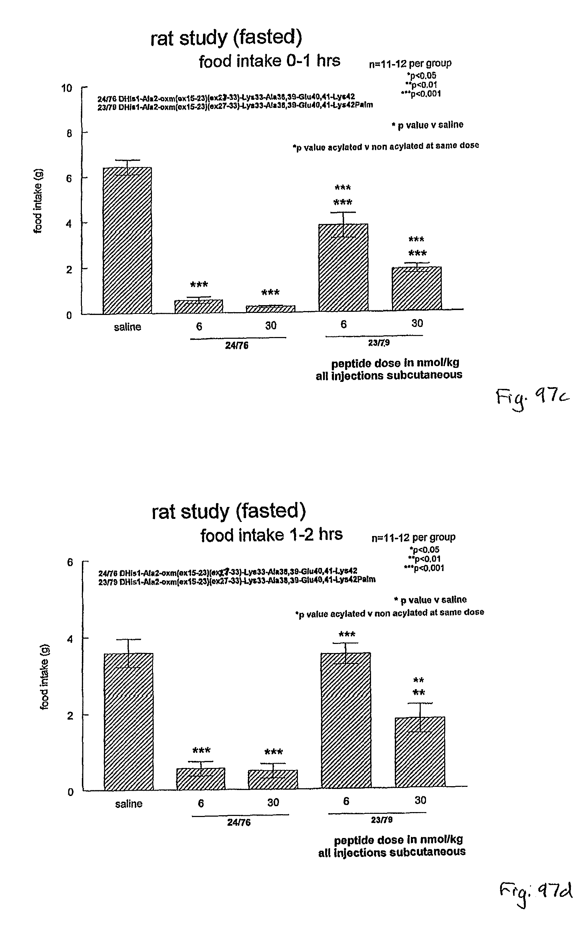

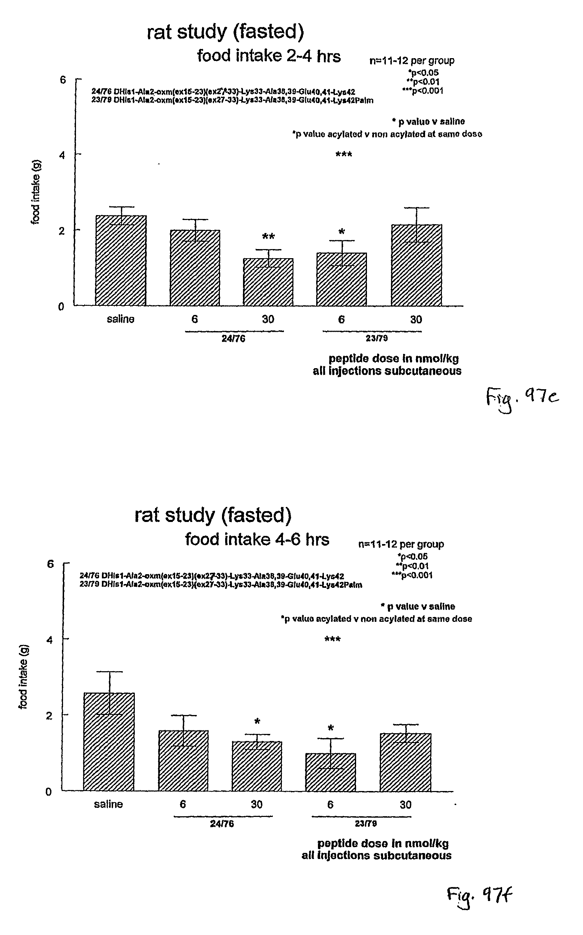

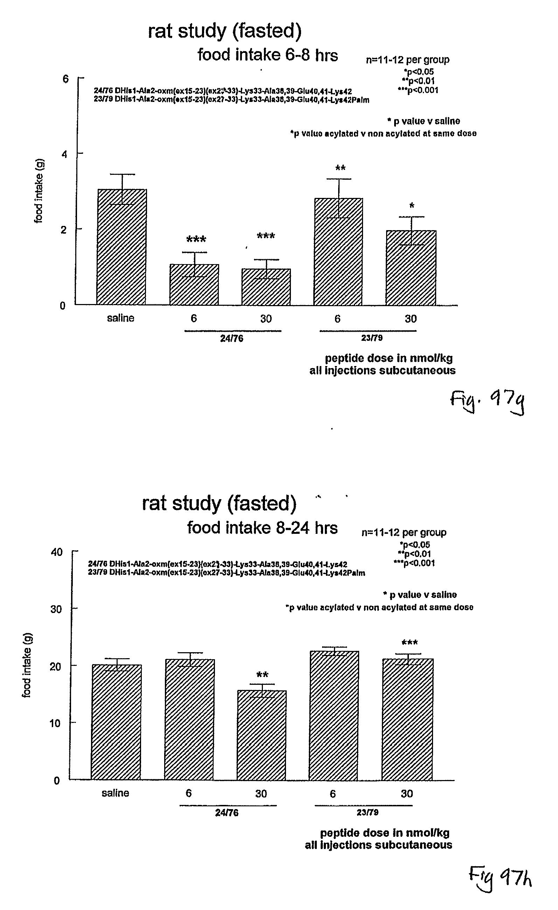

Doses were as stated in Fig. 97. Food intake data is presented in Fig. 97.

### Example 88

Groups of fasted rats were injected sc with the following:
saline
23/36 DHis1-Ala2-oxm(ex15-23)(ex27-33)-Ala38,39-Lys40Lauroyl (SEQ ID NO : 105)
23/79 DHis1-Ala2-oxm(ex15-23)(ex27-33)-Lys33-Ala38,39-Glu40,41-Lys42Palmitoyl (SEQ ID NO: 110)

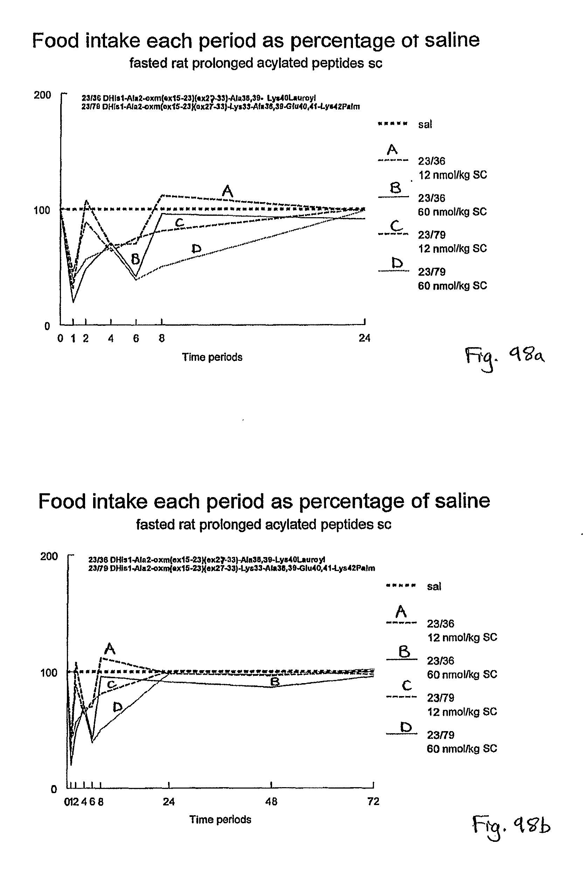

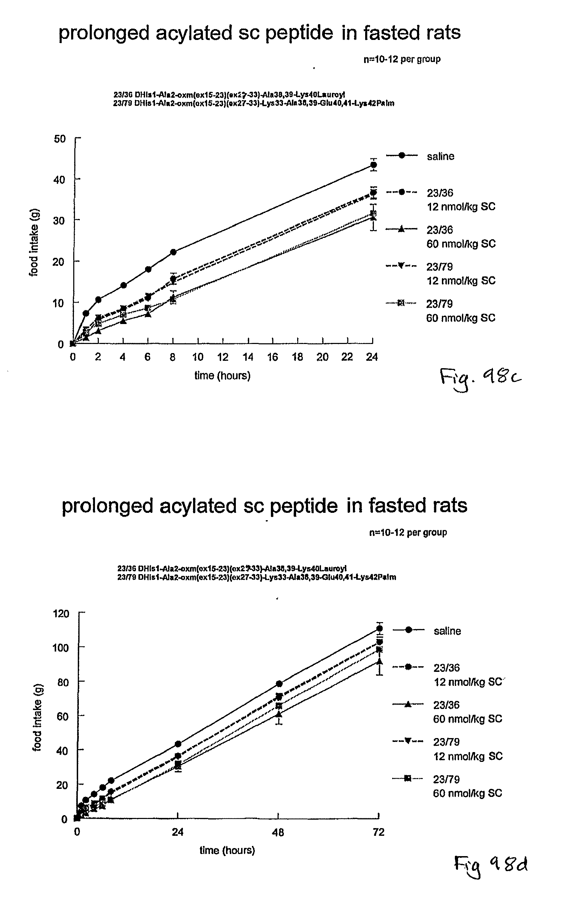

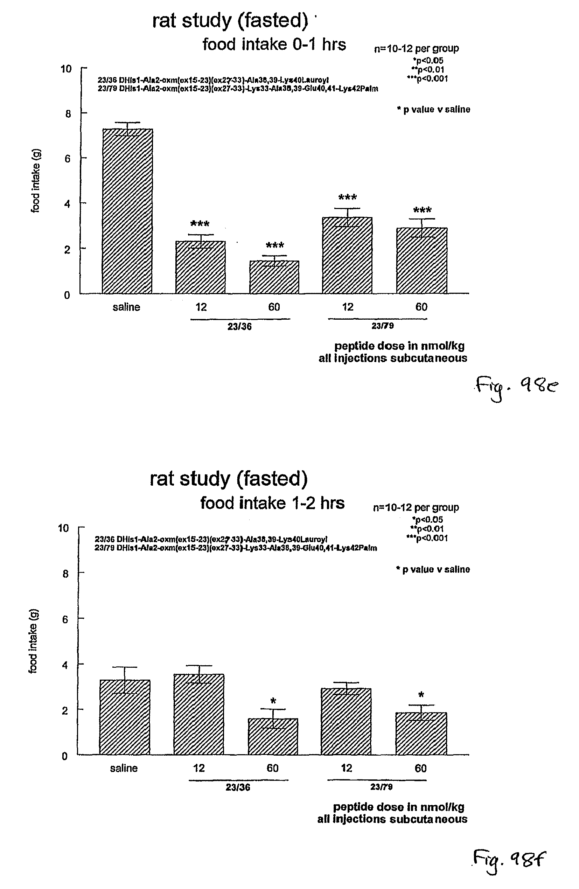

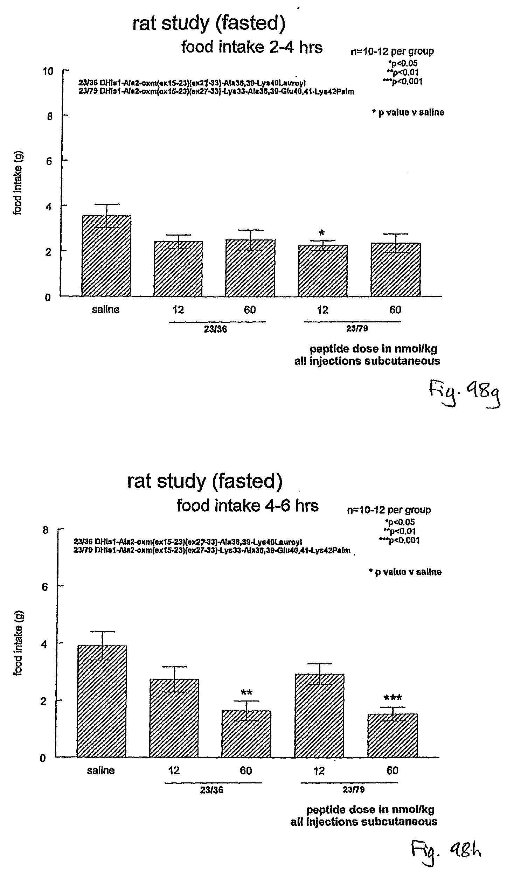

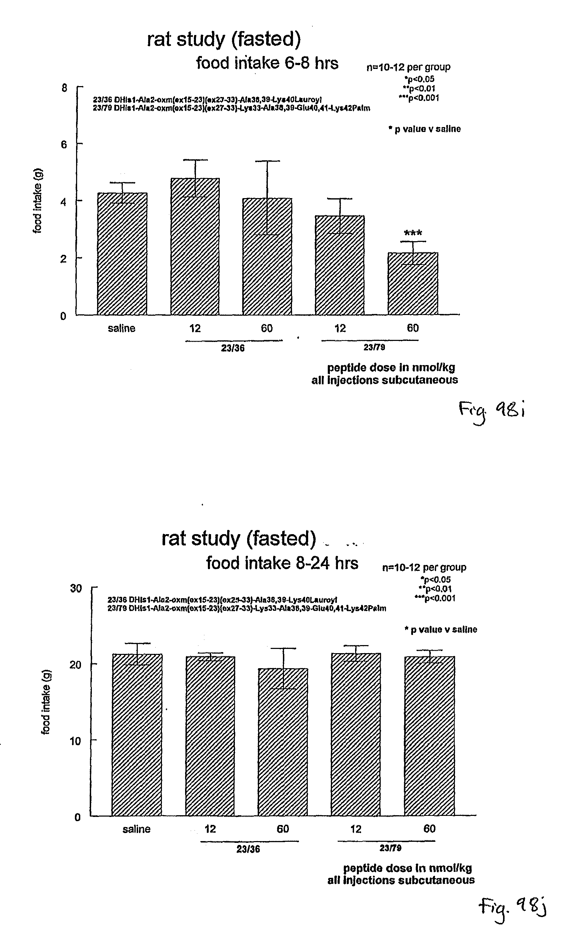

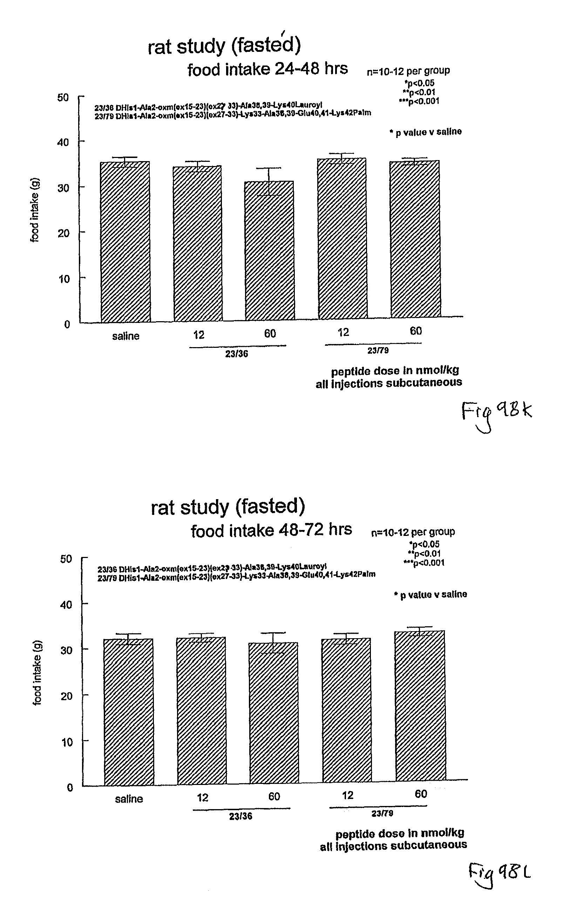

Doses were as stated in Fig. 98. Food intake data is presented in Fig. 98.

### Example 89

Groups of fasted mice were injected with the following:
saline
24/43 DHis-Ala2-Leu18-oxm(ex15-23)(ex27-33)-Ala38,39 (SQE ID NO: 128)
24/314 DHis1-Ser2-Asp3-Ala18-oxm(ex15-23)(ex27-33)-Lys33-Lys38 (SEQ ID NO : 141)
24/316 DHis1-Ser2-Asp3-Leu18-oxm(ex15-23)(ex27-33)-Lys33-Lys38 (SEQ ID NO : 142)
24/318 DHis1-Ser2-Asp3-Ile18-oxm(ex15-23)(ex27-33)-Lys33-Lys38 (SEQ ID NO : 143)
24/322 DHis1-Ser2-Asp3-Val18-Ala19-oxm(ex15-23)(ex27-33)-Lys33-Lys38 (SEQ ID NO: 144)

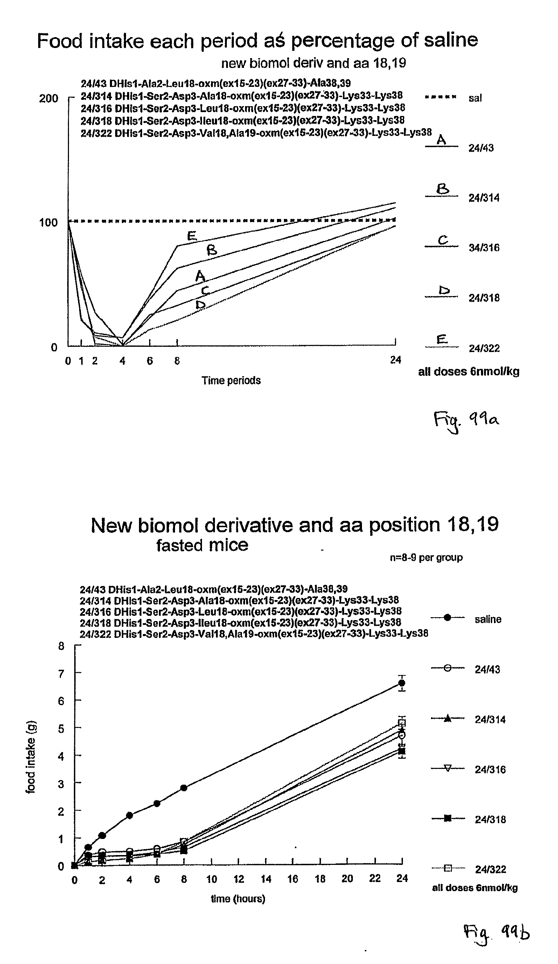

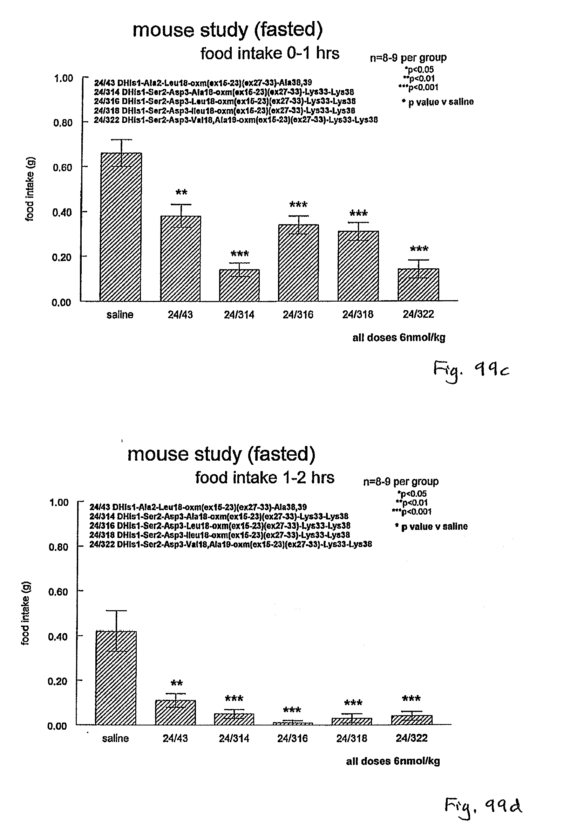

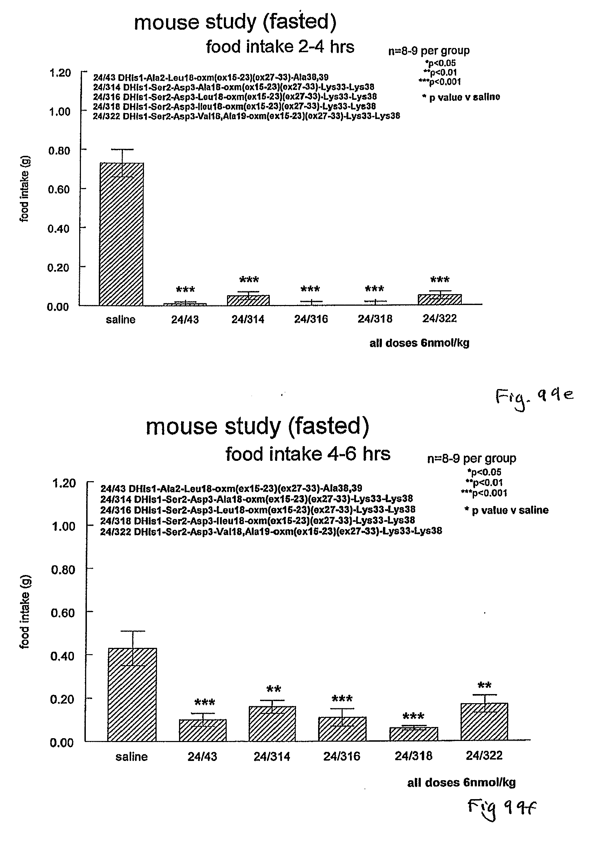

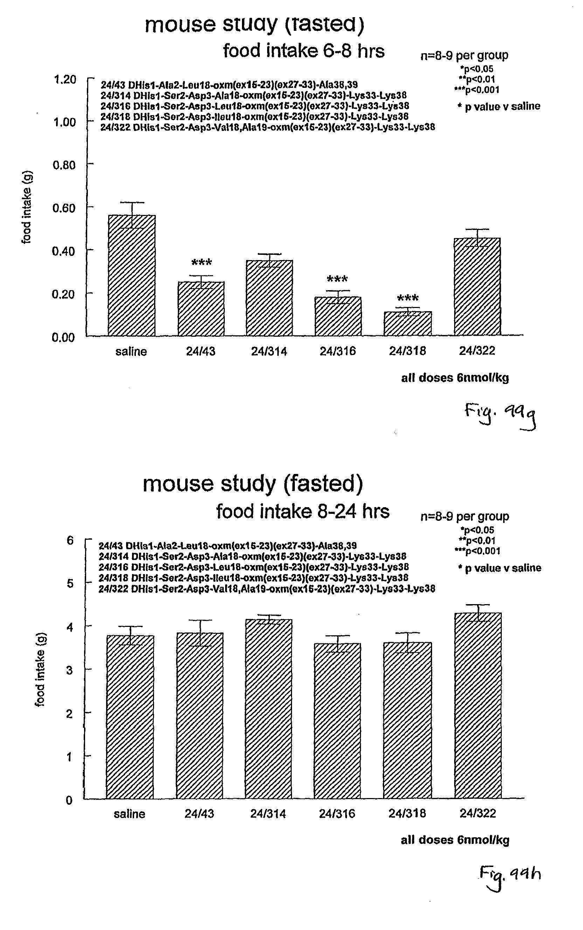

Doses were as stated in Fig. 99. Food intake data is presented in Fig. 99.

### Example 90

Groups of fasted mice were injected with the following:
saline
24/43 DHis-Ala2-Leu18-oxm(ex15-23)(ex27-33)-Ala38,39 (SEQ ID NO: 128)
24/300 DHis1-Ser2-Asp3-Val18-oxm(ex15-23)(ex27-33)-Lys33 (SEQ ID NO : 150)
24/301 DHis1-Ser2-Asp3-Val18-oxm(ex15-23)(ex27-33)-Lys33-Asn34,35-Stop (SEQ ID NO : 151)
24/302 DHis1-Ser2-Asp3-Val18-oxm(ex15-23)(ex27-33)-Lys33-Asn34,35-Lys36-Stop (SEQ ID NO: 152)
24/303 DHis1-Ser2-Asp3-Val18-oxm(ex15-23)(ex27-33)-Lys33-Ala38,39-Glu40,41-Lys42 (SEQ ID NO : 153)

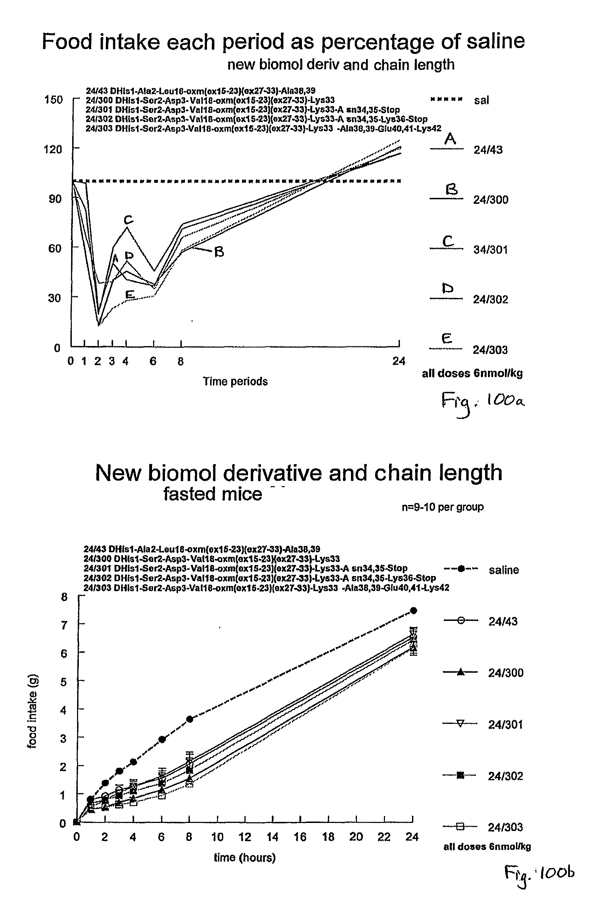

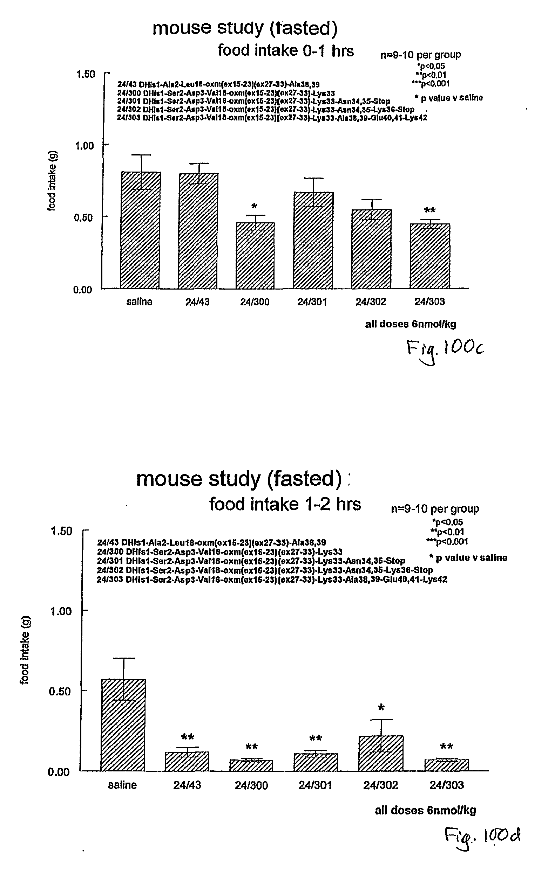

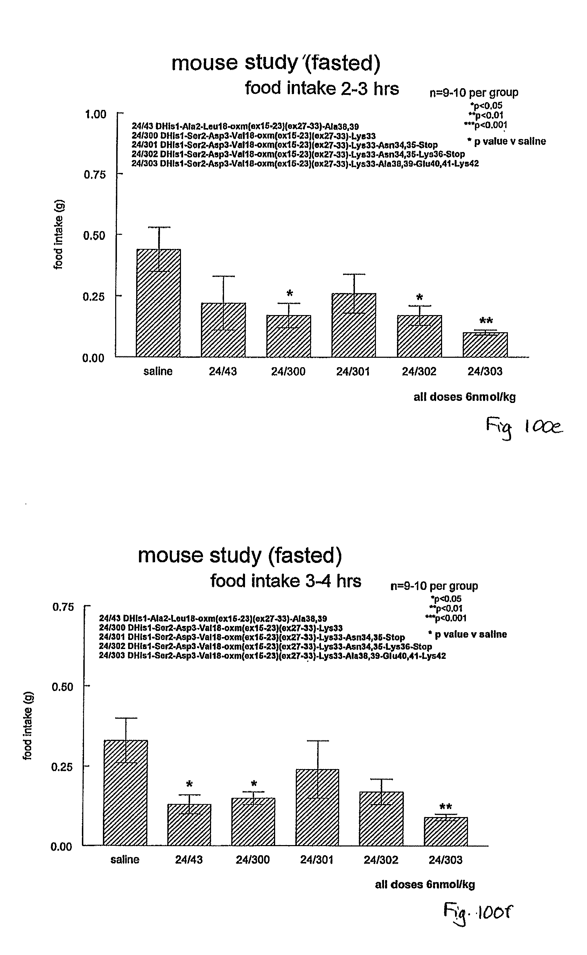

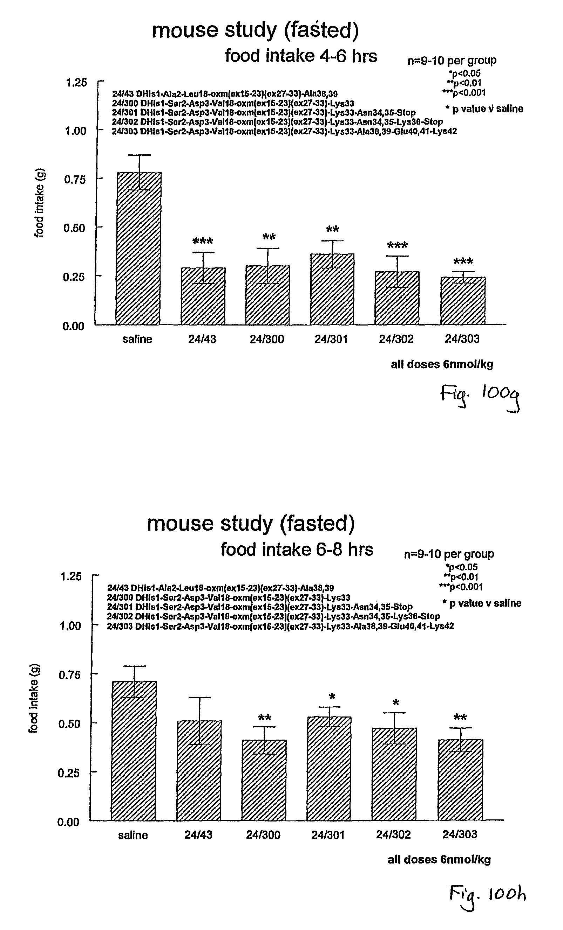

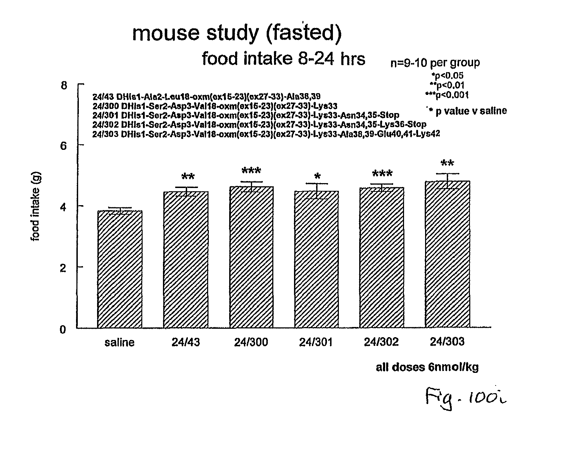

Doses were as stated in Fig. 100. Food intake data is presented in Fig. 100.

### Example 91

Groups of fasted mice were injected with the following:
saline
24/300 DHis1-Ser2-Asp3-Val18-oxm(ex15-23)(ex27-33)-Lys33 (SEQ ID NO : 150)
24/314 DHis1-Ser2-Asp3-Ala18-oxm(ex15-23)(ex27-33)-Lys33-Lys38 (SEQ ID NO : 141)
24/316 DHis1-Ser2-Asp3-Leu18-oxm(ex15-23)(ex27-33)-Lys33-Lys38 (SEQ ID NO : 142)
24/320 DHis1-Ser2-Asp3-Gln16-Leu18-oxm(ex15-23)(ex27-33)-Lys33-Lys38 (SEQ ID NO : 154)
24/324 DHis1-Ser2-Asp3-Ala16-Leu18-oxm(ex15-23)(ex27-33)-Lys33-Lys38 (SEQ ID NO : 155)

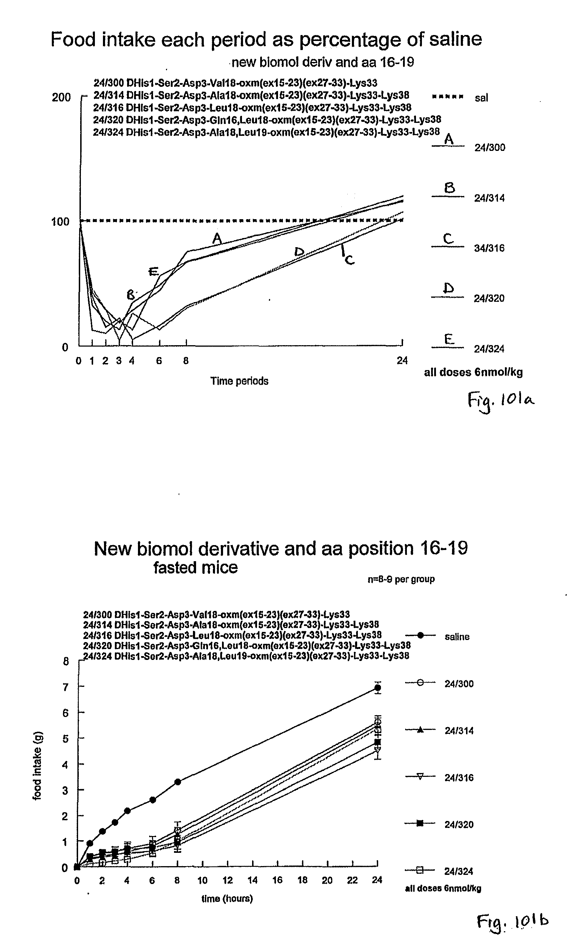

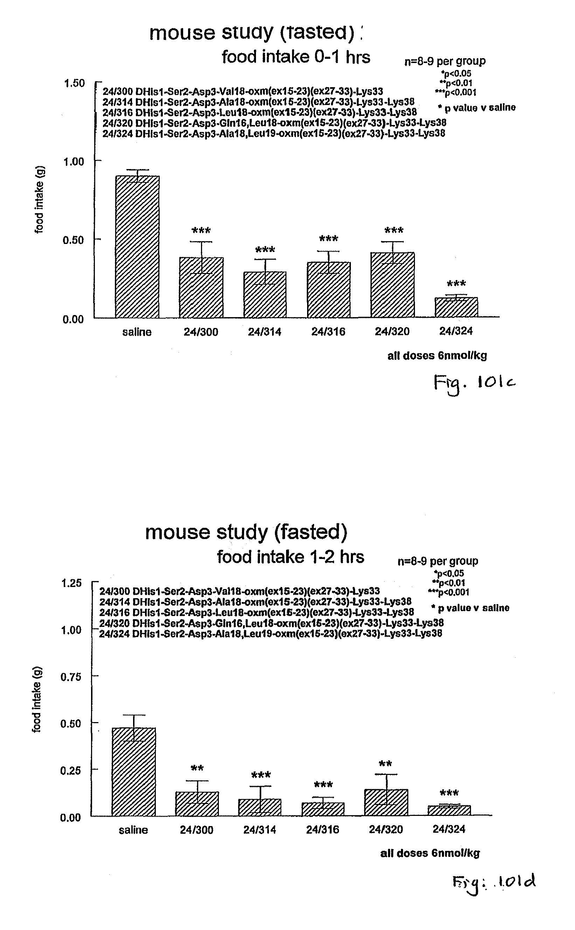

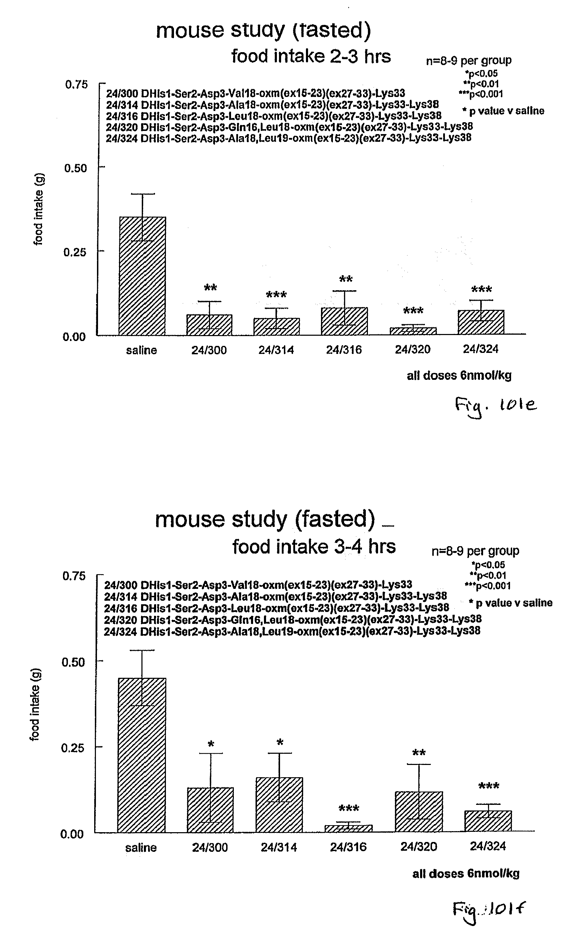

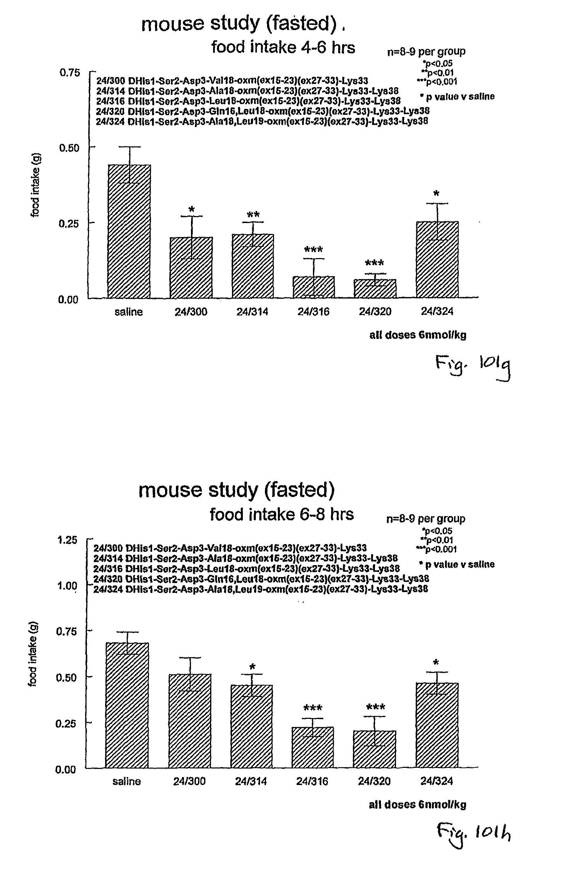

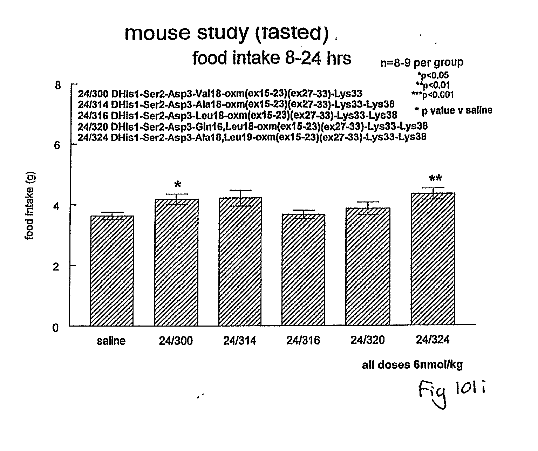

Doses were as stated in Fig. 101. Food intake data is presented in Fig. 101. The data confirms previous observations that peptides containing Leu18 have a favourable and preferred activity profile, and are therefore preferred.

### Example 92

Groups of fasted mice were injected with the following:
saline
24/318 DHis1-Ser2-Asp3-Ile18-oxm(ex15-23)(ex27-33)-Lys33-Lys38 (SEQ ID NO: 143)

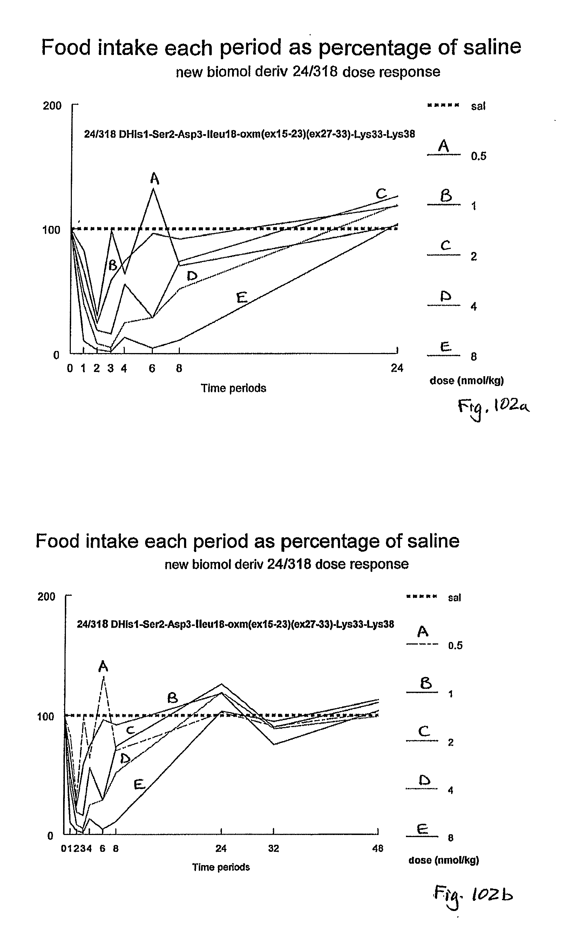

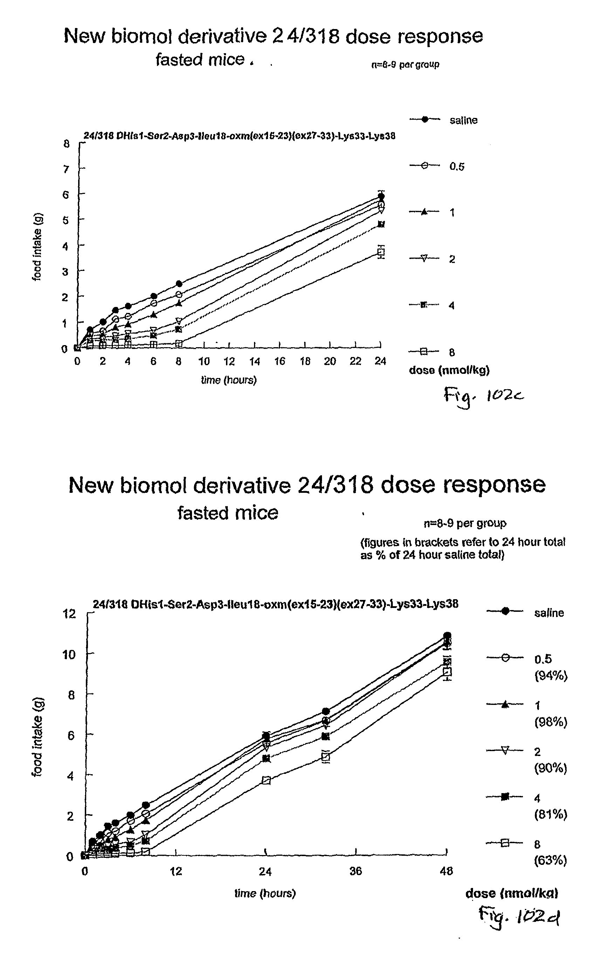

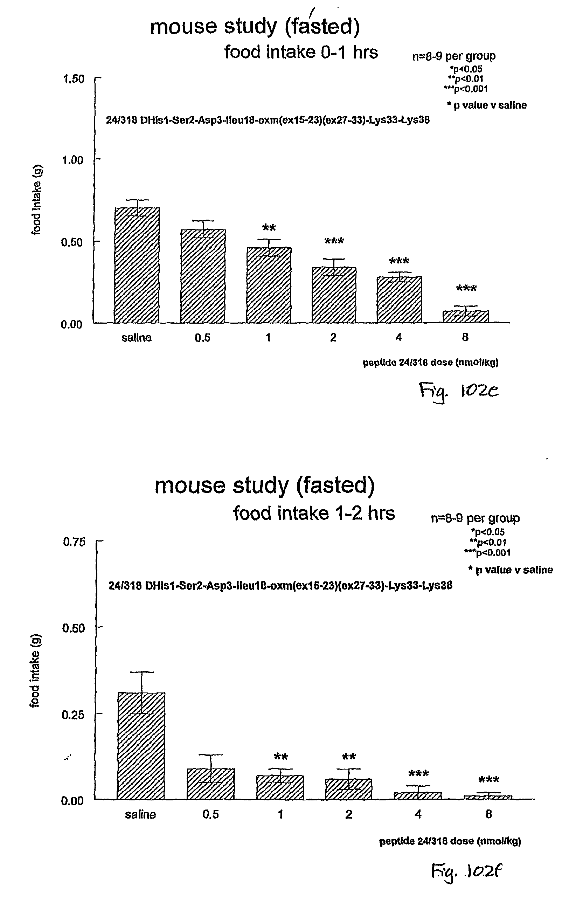

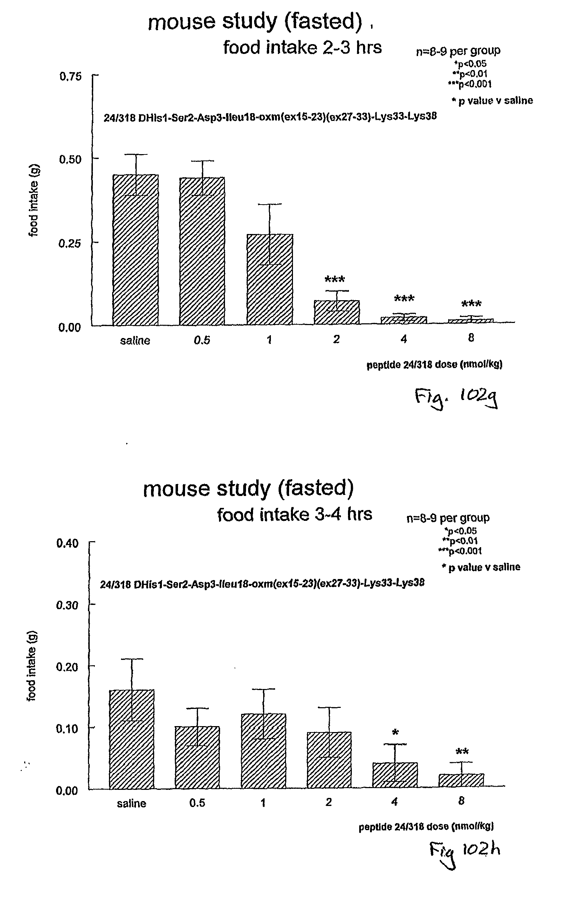

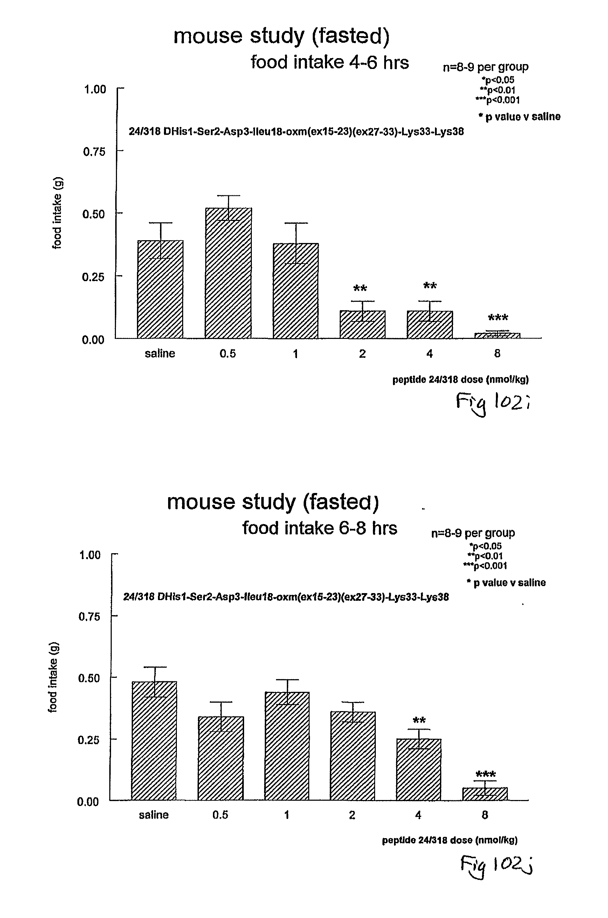

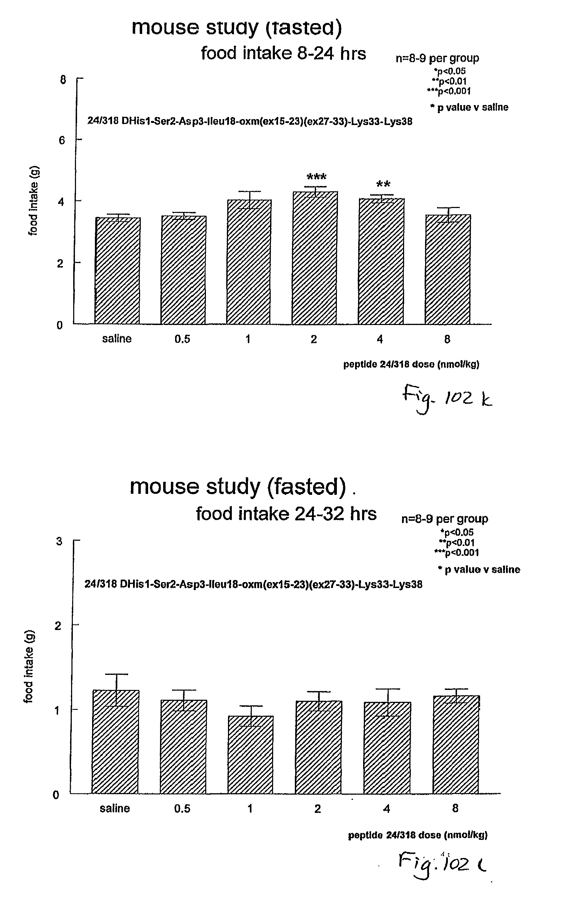

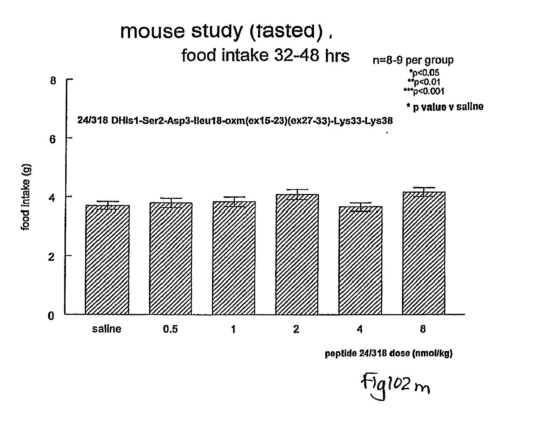

Doses were as stated in Fig. 102. Food intake data is presented in Fig. 102. The data shows the dose response profile for the injected compound.

### Example 93

Groups of fasted mice were injected with the following:
saline
24/316 DHis1-Ser2-Asp3-Leu18-oxm(ex15-23)(ex27-33)-Lys33-Lys38 (SEQ ID NO : 142)
24/330 DHis1-Ala2-Glu3-Leu18-oxm(ex15-24)(ex27-33)-Lys33-Lys38 (SEQ ID NO : 156)
24/331 DHis1-Ala2-Asp3-Leu18-oxm(ex15-24)(ex27-33)-Lys33-Lys38 (SEQ ID NO : 157)
24/337 DHis1-Ala2-Asp3-Leu18-oxm(ex15-24)(ex27-33)-Lys33-Asp35-Lys38 (SEQ ID NO : 158)
24/327 DHis1-Ala2-Asp3-Ile18-Lys20-Tyr21-oxm(ex15-23)(ex27-33)-Lys33- Lys38 (SEQ ID NO: 159)

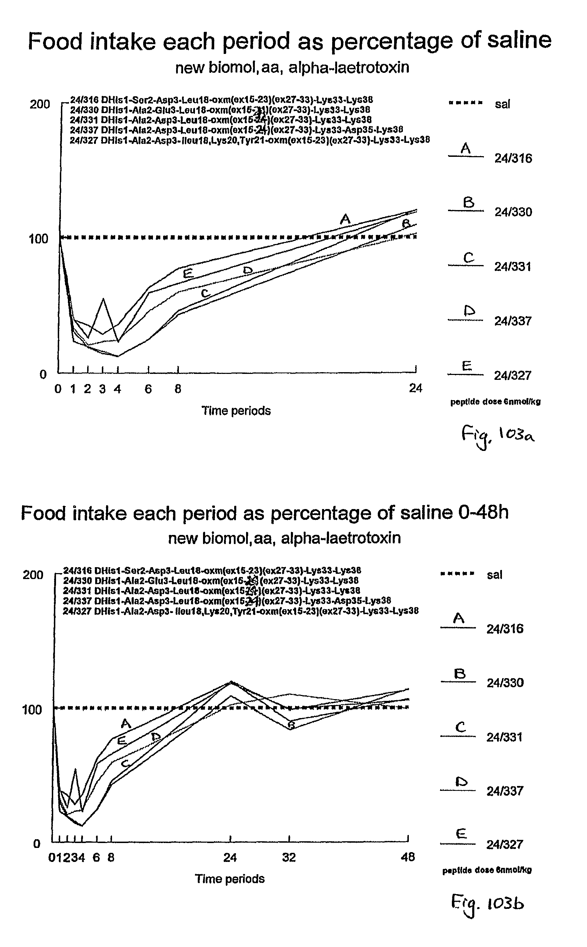

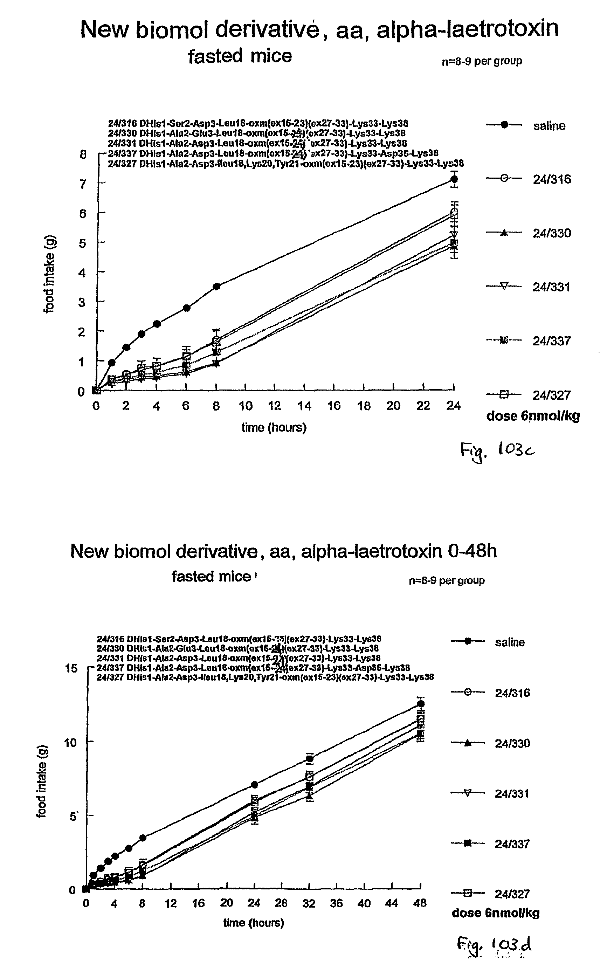

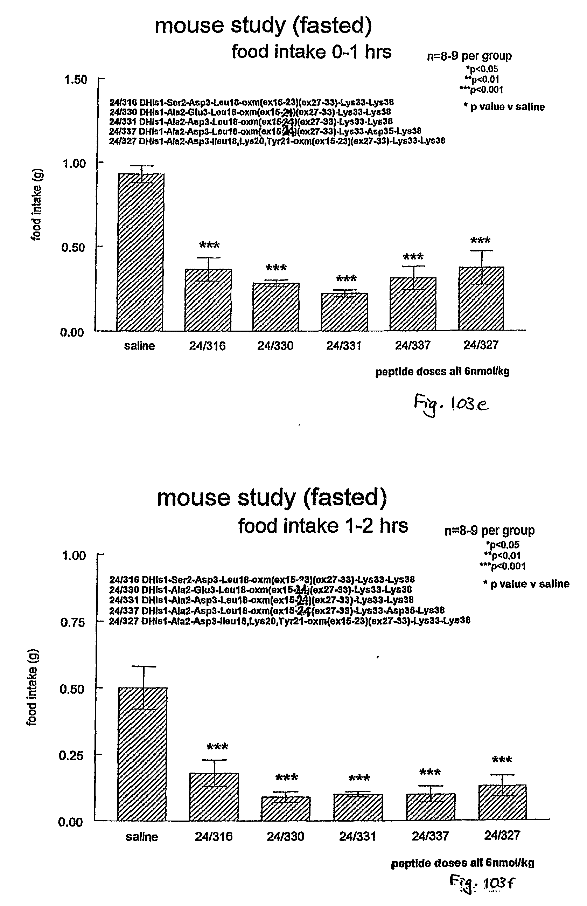

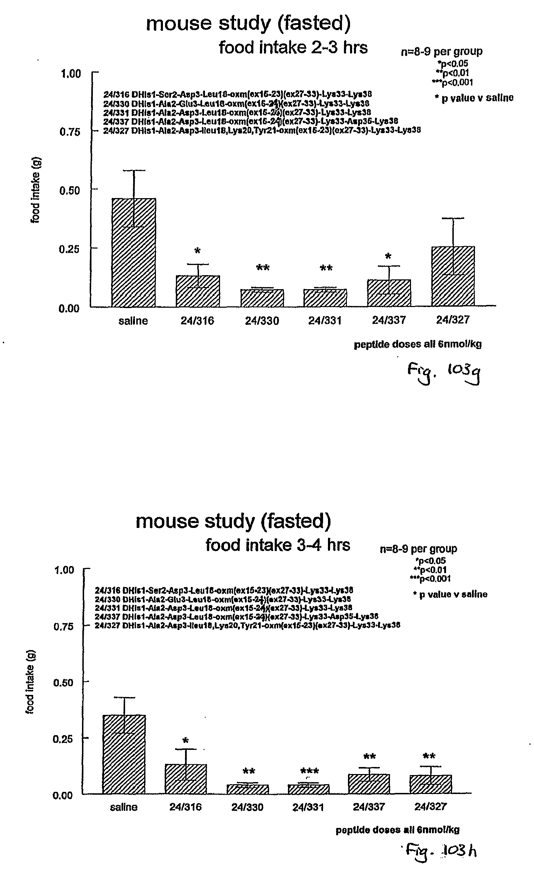

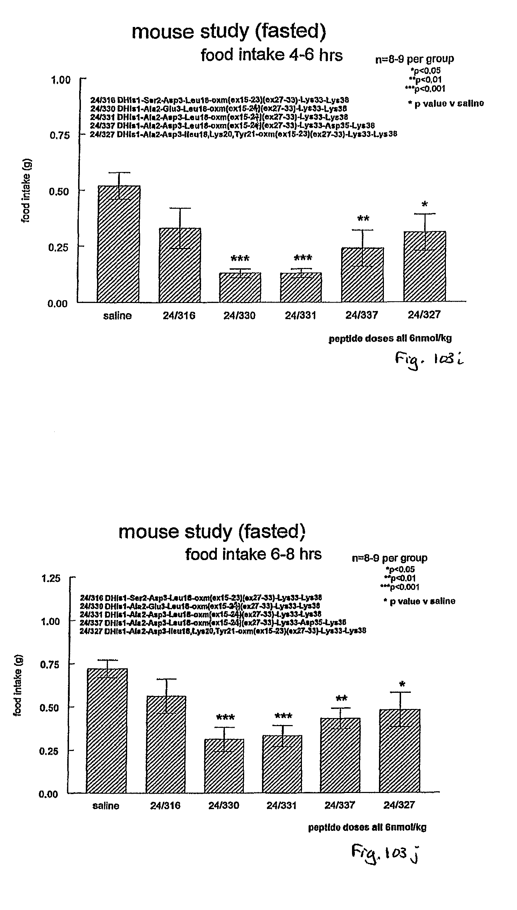

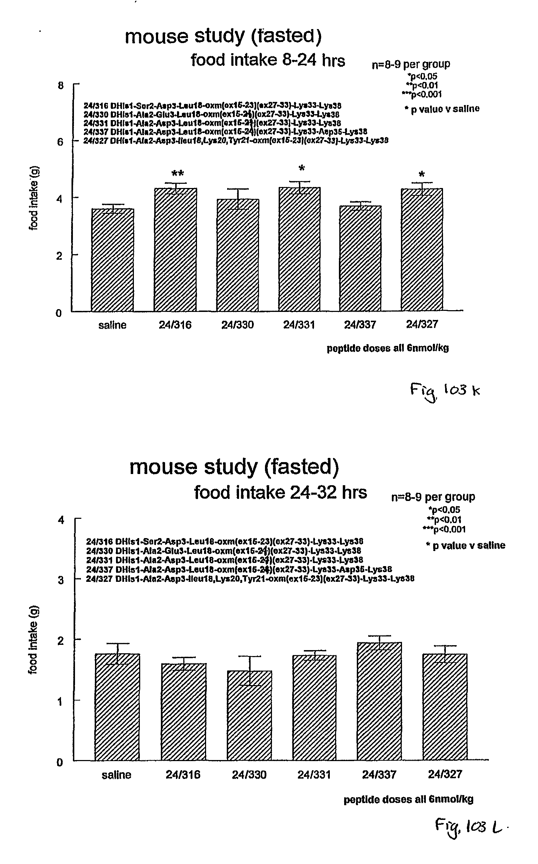

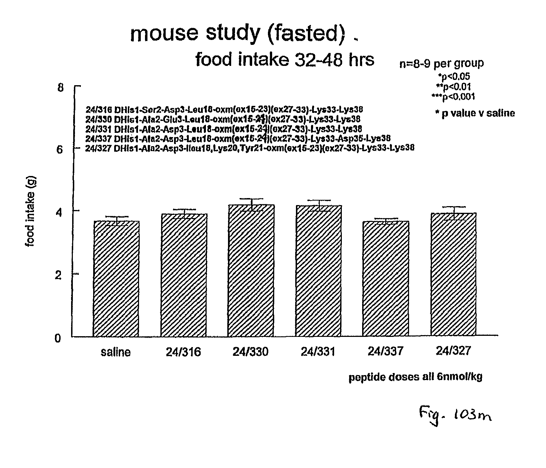

Doses were as stated in Fig. 103. Food intake data is presented in Fig. 103.

### Example 94

Groups of fasted mice were injected with the following:
saline
24/310 DHis1-Ser2-Asp3-Val18-oxm(ex15-23)(ex27-33)-Lys33-Lys38 (SEQ ID NO : 114)
23/310 DHis1- Ser2-Asp3-Val18-oxm(ex15-23)(ex27-33)-Lys33-Lys38-Octanoyl (SEQ ID NO : 160)
23/311 DHis1- Ser2-Asp3-Val18-oxm(ex15-23)(ex27-33)-Lys33-Lys38-Lauroyl (SEQ ID NO : 161)
23/312 DHis1- Ser2-Asp3-Val18-oxm(ex15-23)(ex27-33)-Lys33-Lys38-Palmitoyl (SEQ ID NO : 162)
27/310 DHis1- Ser2-Asp3-Val18-oxm(ex15-23)(ex27-33)-Lys33-Lys38-PEG (SEQ ID NO: 163)

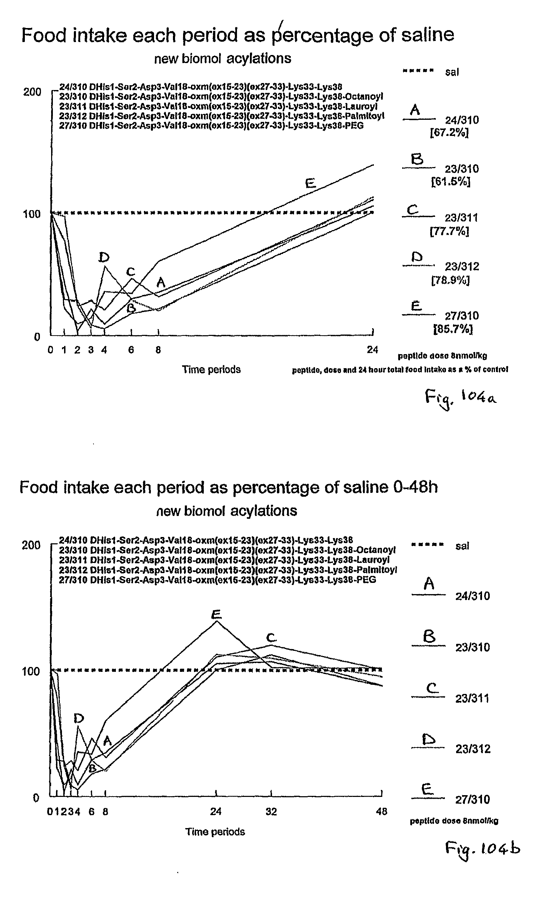

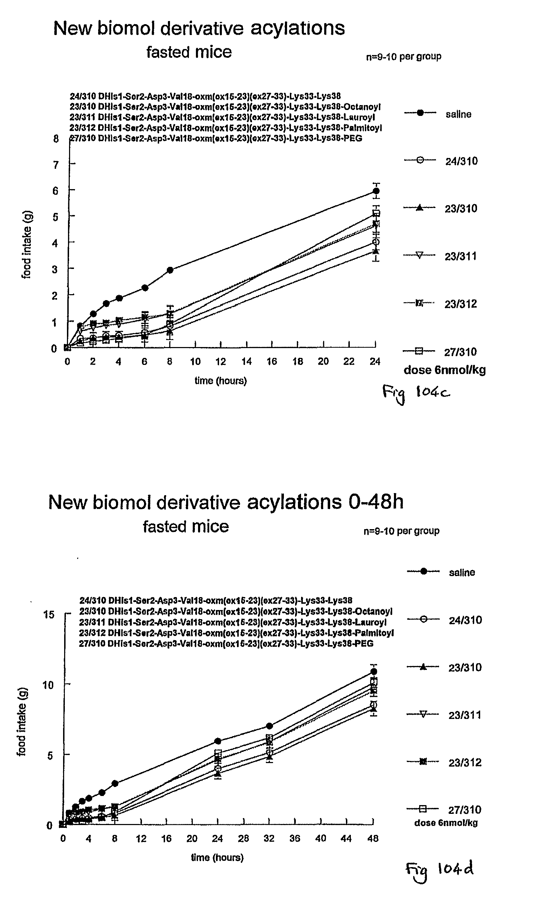

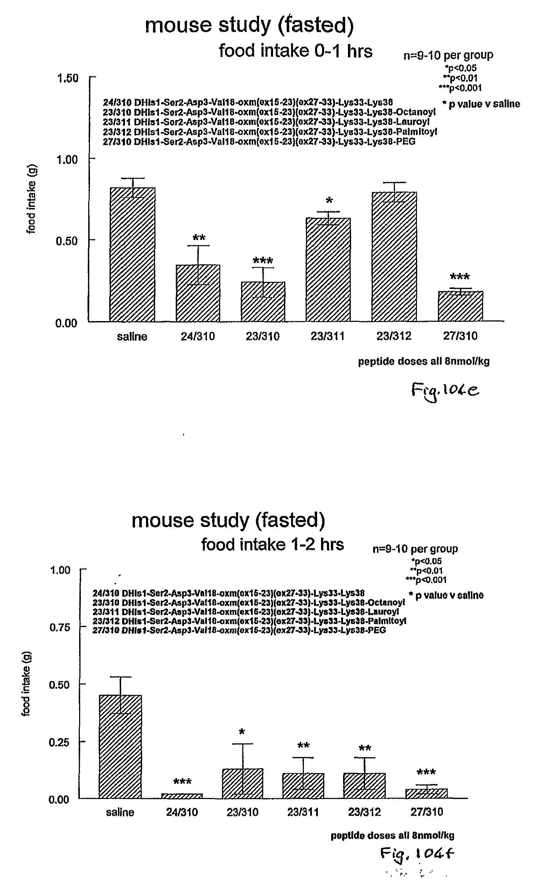

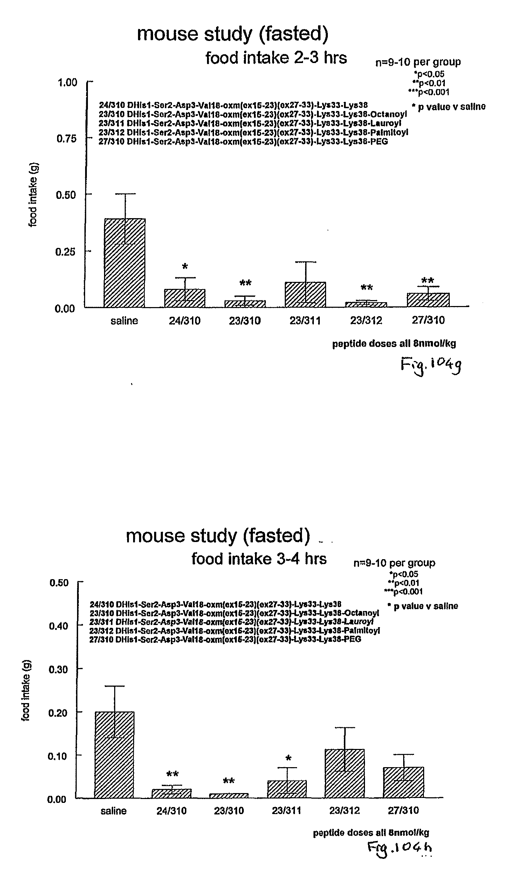

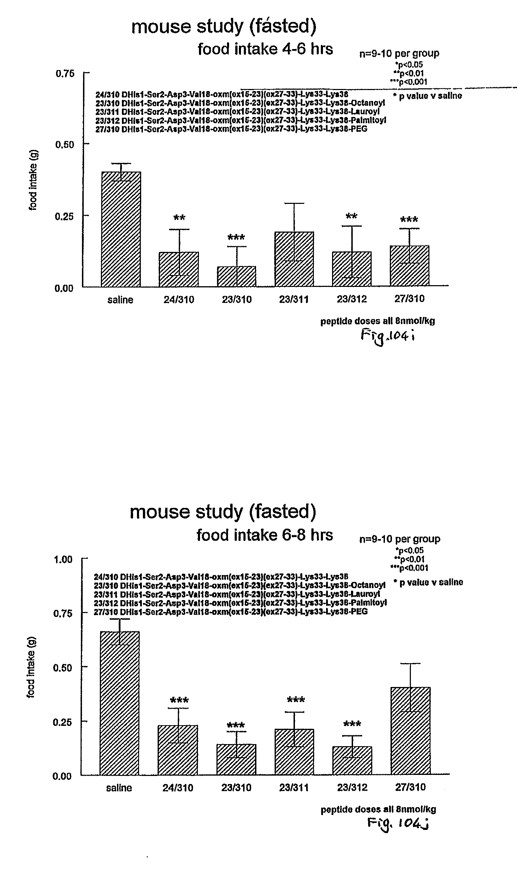

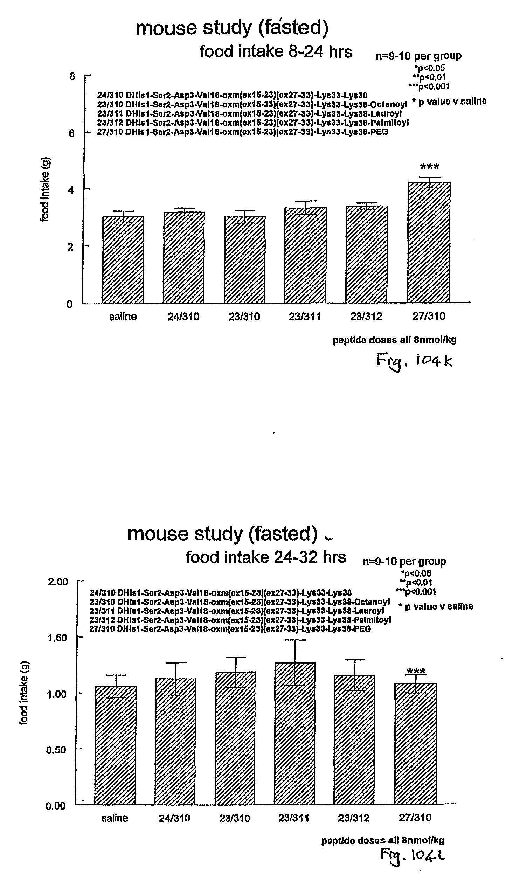

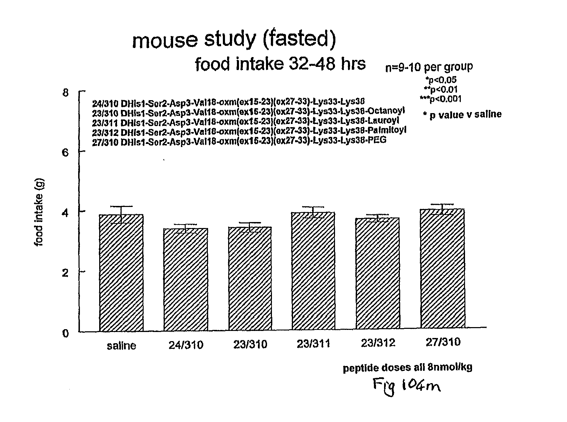

Doses were as stated in Fig. 104. Food intake data is presented in Fig. 104.

### Example 95

Groups of fasted mice were injected with the following:
saline
24/327 DHis1-Ser2-Asp3-Ile18-Lys20,Tyr21-oxm(ex15-23)(ex27-33)-Lys33-Lys38 (SEQ ID NO:164)
24/330 DHis1-Ala2-Glu3-Leu18-oxm(ex15-24)(ex27-33)-Lys33-Lys38 (SEQ ID NO : 156)
24/331 DHis1-Ala2-Asp3-Leu18-oxm(ex15-24)(ex27-33)-Lys33-Lys38 (SEQ ID NO : 157)
24/310 DHis1-Ala2-Asp3-Val18-oxm(ex15-23)(ex27-33)-Lys33-Lys38 (SEQ ID NO : 114)
24/316 DHis1-Ser2-Asp3-Leu18-oxm(ex15-23)(ex27-33)-Lys33-Lys38 (SEQ ID NO: 142)

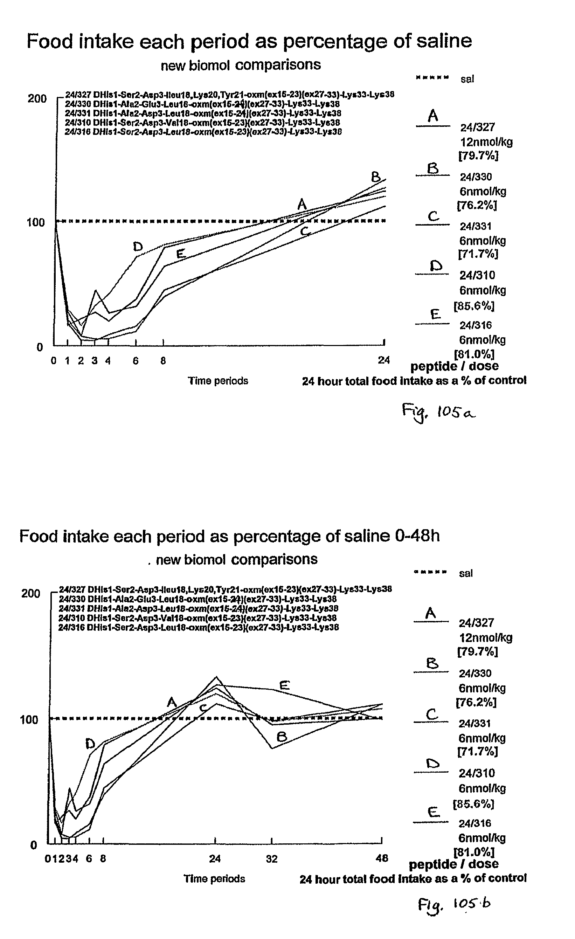

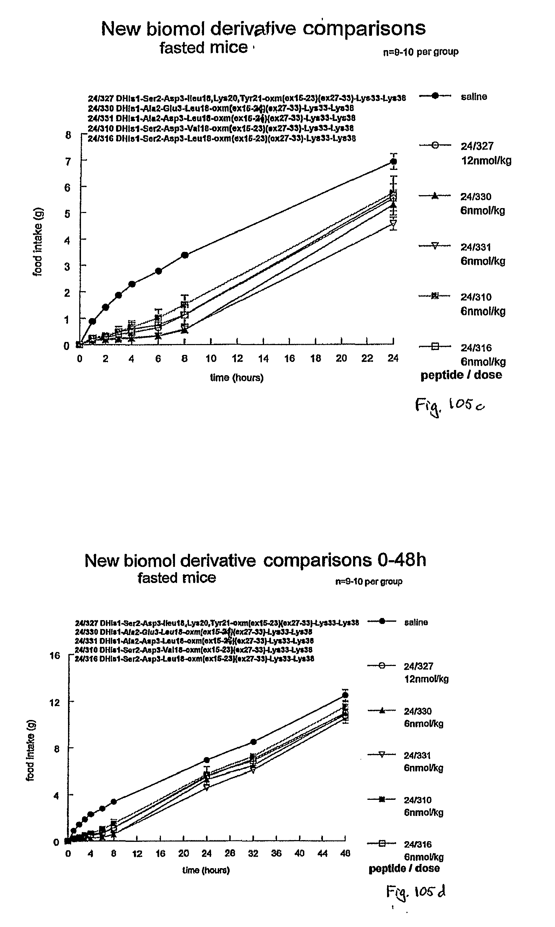

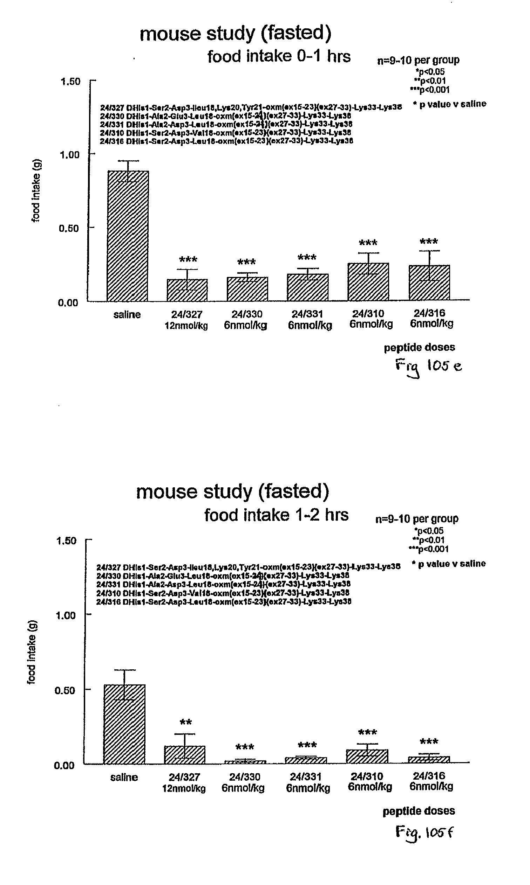

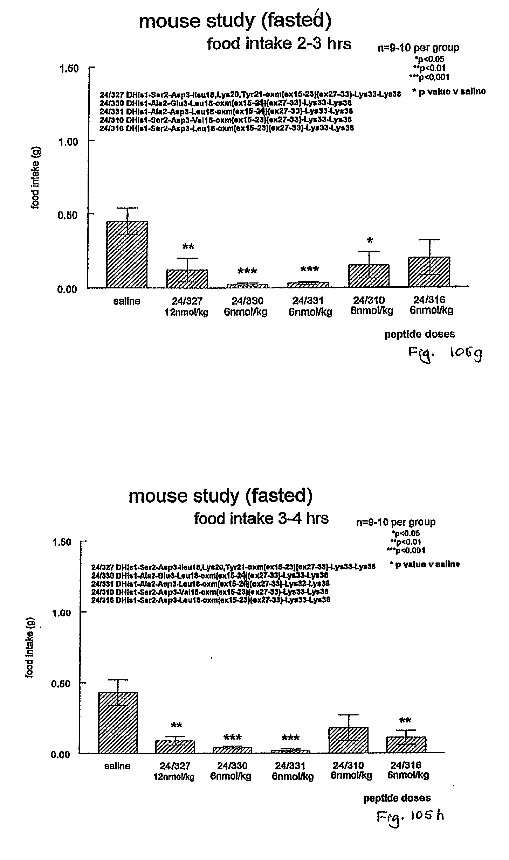

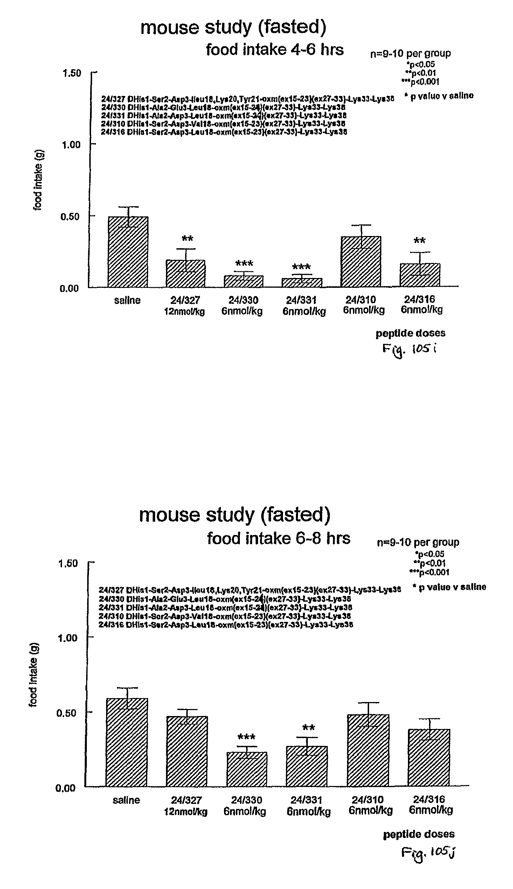

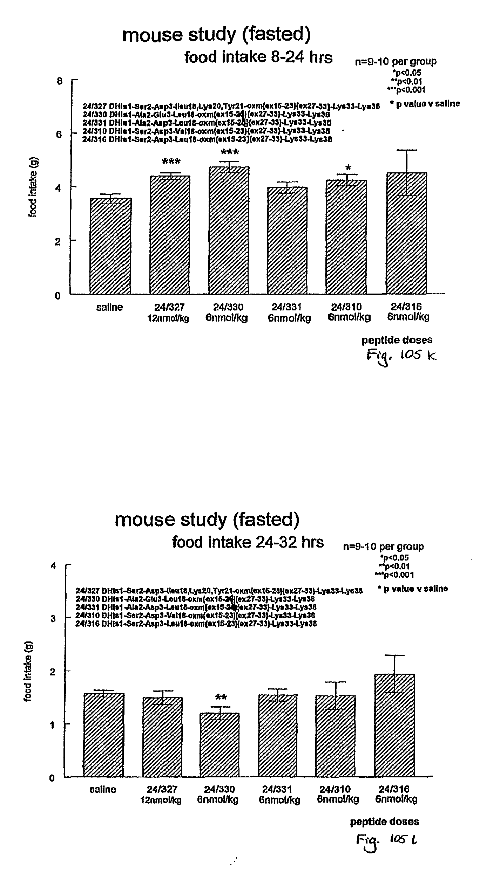

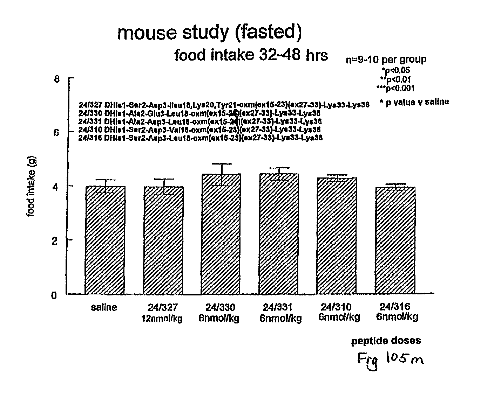

Doses were as stated in Fig. 105. Food intake data is presented in Fig. 105.

### Example 96

Groups of fasted mice were injected with the following:
saline
24/310 DHis1-Ser2-Asp3-Val18-oxm(ex15-23)(ex27-33)Lys33-Lys38 (SEQ ID NO : 114)
23/310 DHis1-Ser2-Asp3-Val18-oxm(ex15-23)(ex27-33)Lys33-Lys38-octanoyl (SEQ ID NO : 160)
23/311 DHis1-Ser2-Asp3-Val18-oxm(ex15-23)(ex27-33)Lys33-Lys38-lauroyl (SEQ ID NO : 161)
23/312 DHis1-Ser2-Asp3-Val18-oxm(ex15-23)(ex27-33)Lys33-Lys38-palmitoyl (SEQ ID NO : 162)
27/310 DHis1-Ser2-Asp3-Val18-oxm(ex15-23)(ex27-33)Lys33-Lys38-PEG (SEQ ID NO: 163)

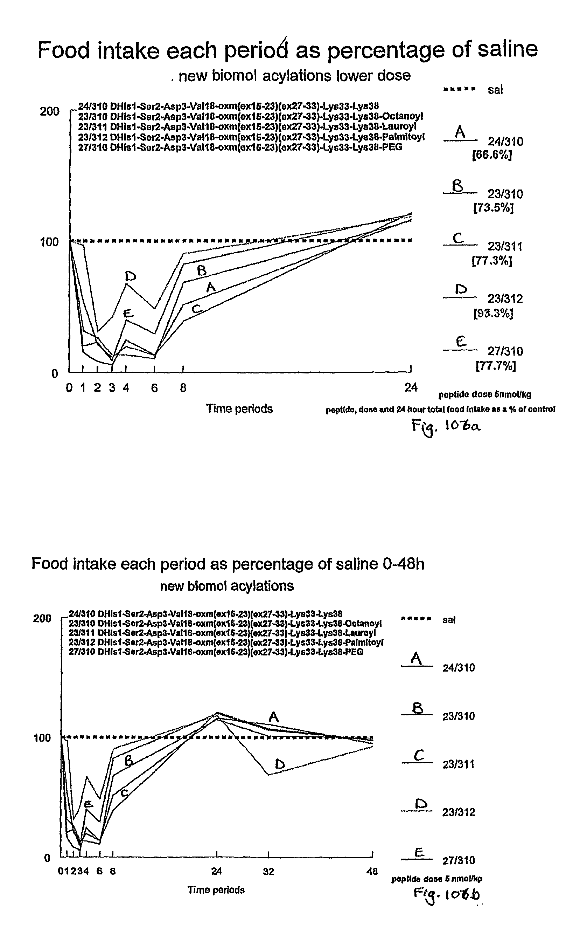

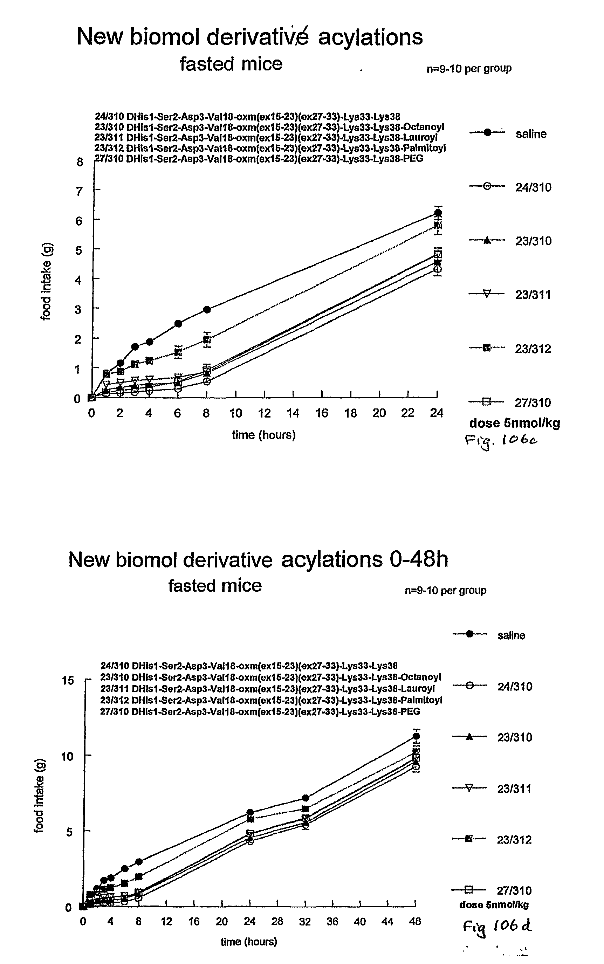

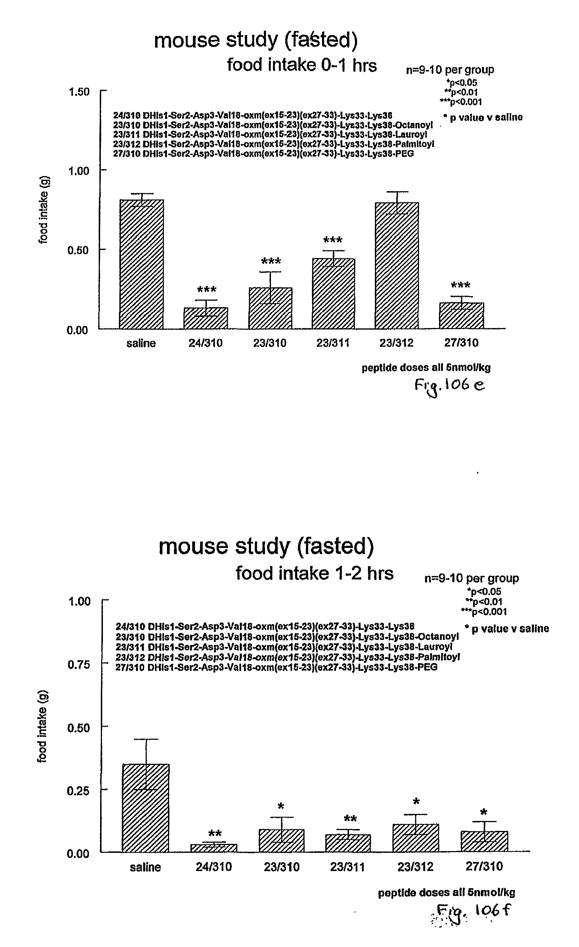

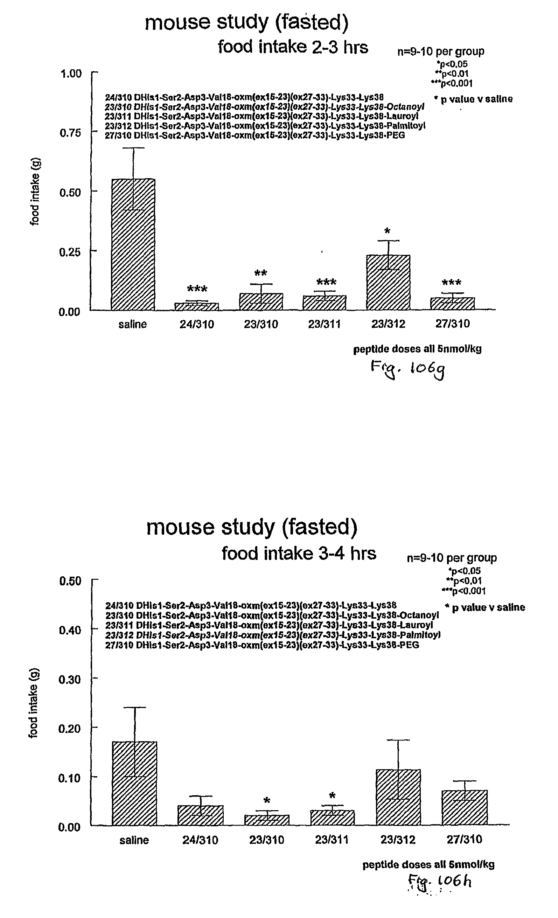

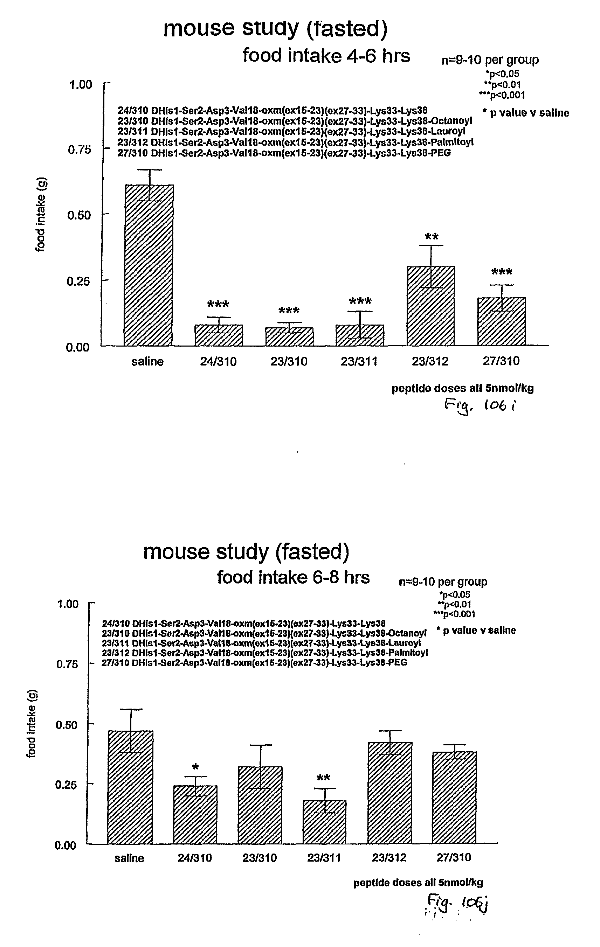

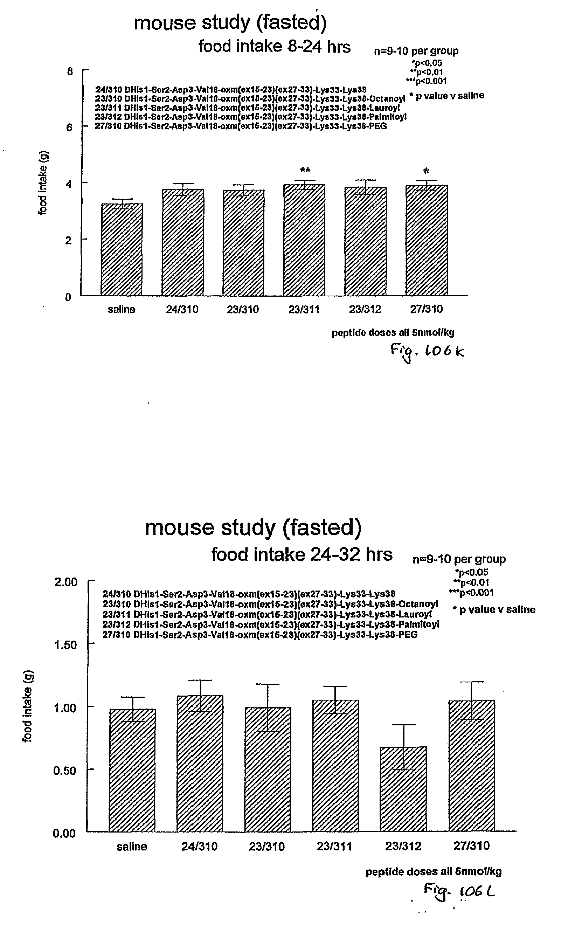

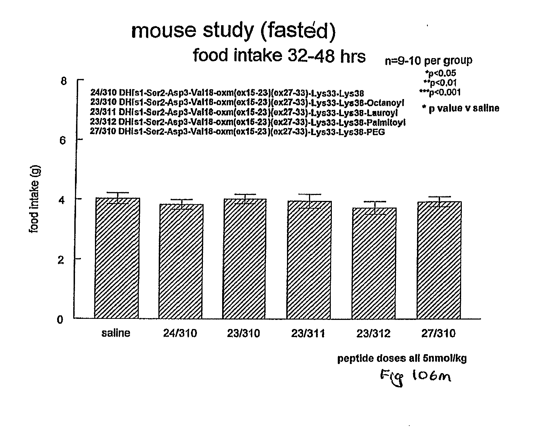

Doses are stated in Fig. 106. Food intake data is presented in Fig. 106.

### Example 97

Groups of fasted rats were injected with the following:
saline
24/310 DHis1-Ser2-Asp3-Val18-oxm(ex15-23)(ex27-33)Lys33-Lys38 (SEQ ID NO : 114)
24/316 DHis1-Ser2-Asp3-Leu18-oxm(ex15-23)(ex27-33)Lys33-Lys38 (SEQ ID NO : 142)

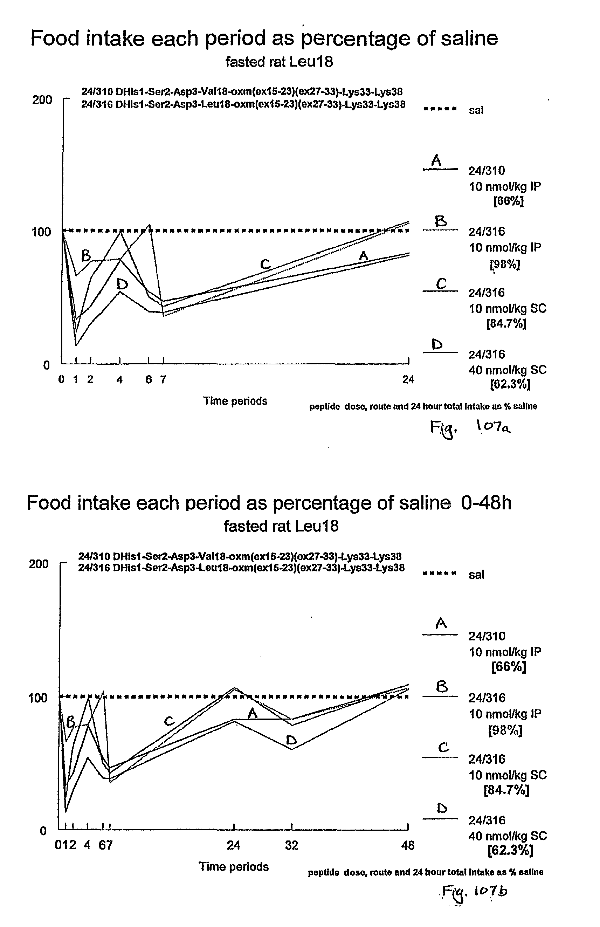

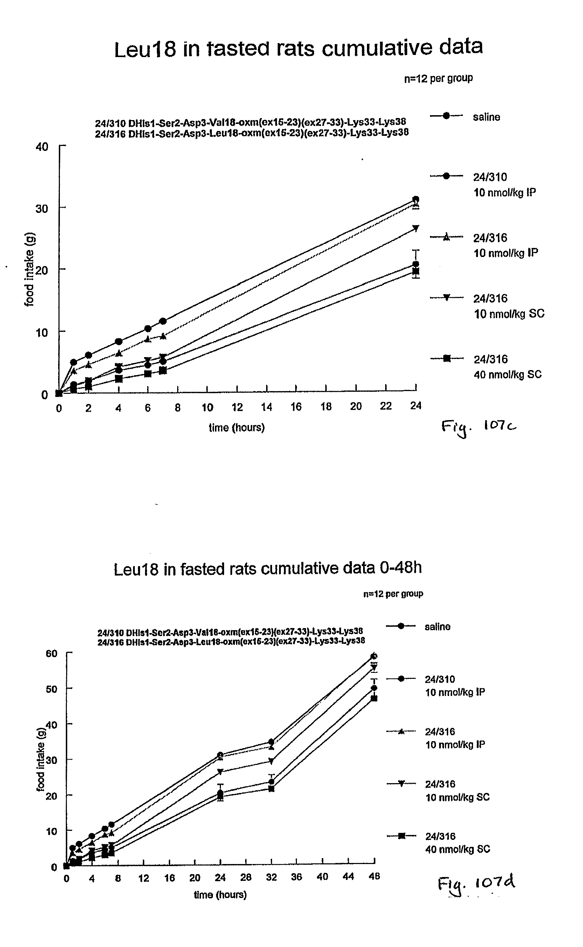

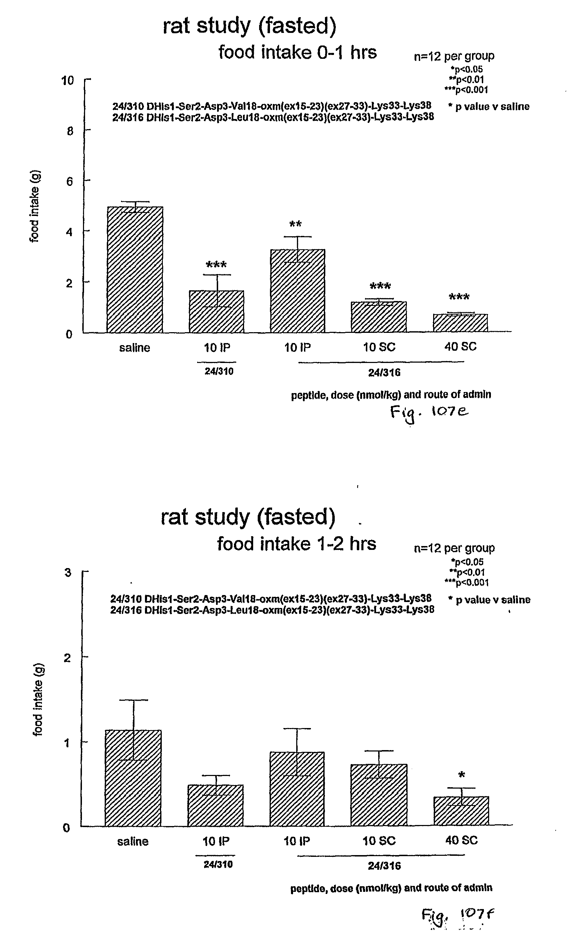

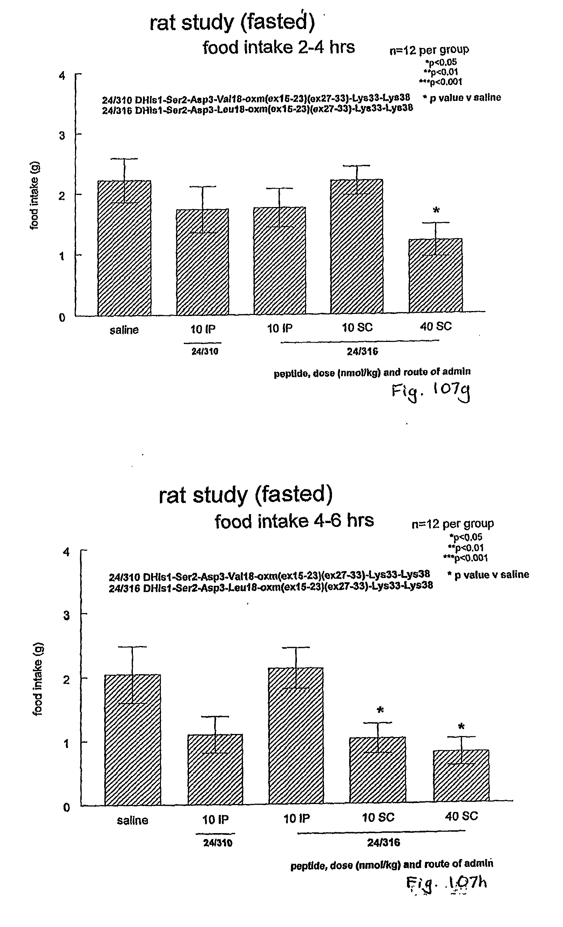

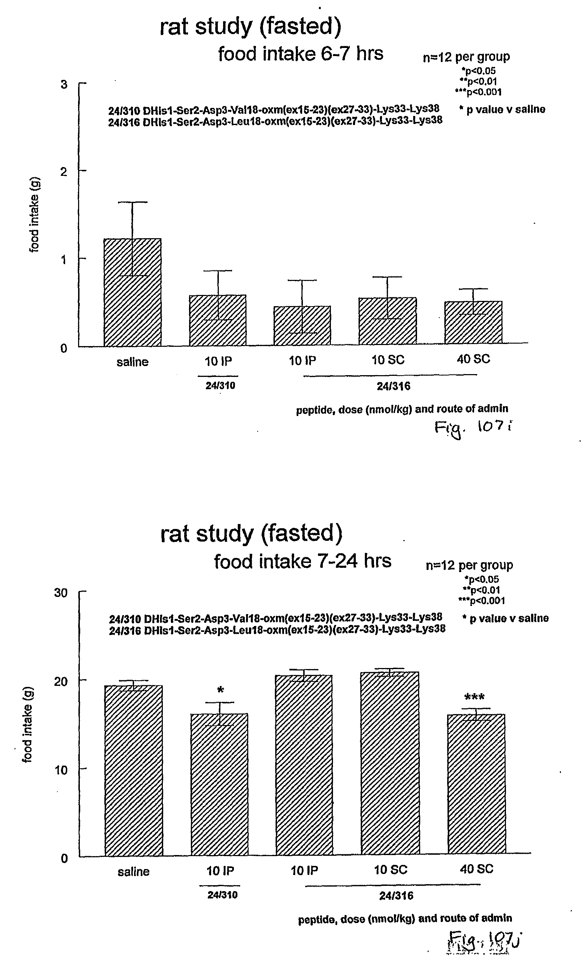

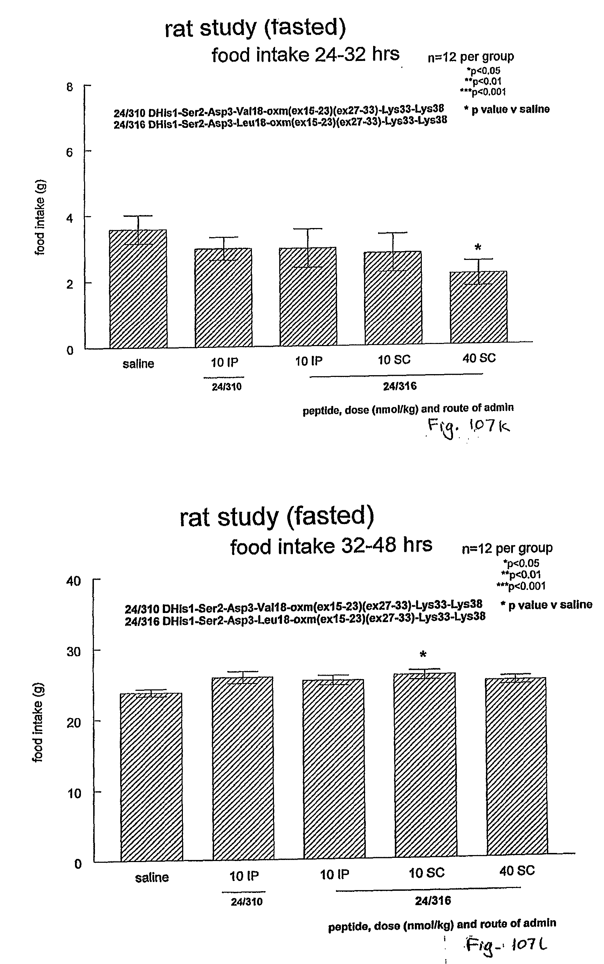

Doses are stated in Fig. 107. Food intake data is presented in Fig. 107.

### Example 98

Groups of fasted rats were injected with the following:
saline
24/314 DHis1-Ser2-Asp3-Ala18-oxm(ex15-23)(ex27-33)Lys33-Lys38 (SEQ ID NO: 141)
24/310 DHis1-Ser2-Asp3-Val18-oxm(ex15-23)(ex27-33)Lys33-Lys38 (SEQ ID NO: 114)
24/316 DHis1-Ser2-Asp3-Leu18-oxm(ex15-23)(ex27-33)Lys33-Lys38 (SEQ ID NO: 142)
24/318 DHis1-Ser2-Asp3-Ile18-oxm(ex15-23)(ex27-33)Lys33-Lys38 (SEQ ID NO: 143)

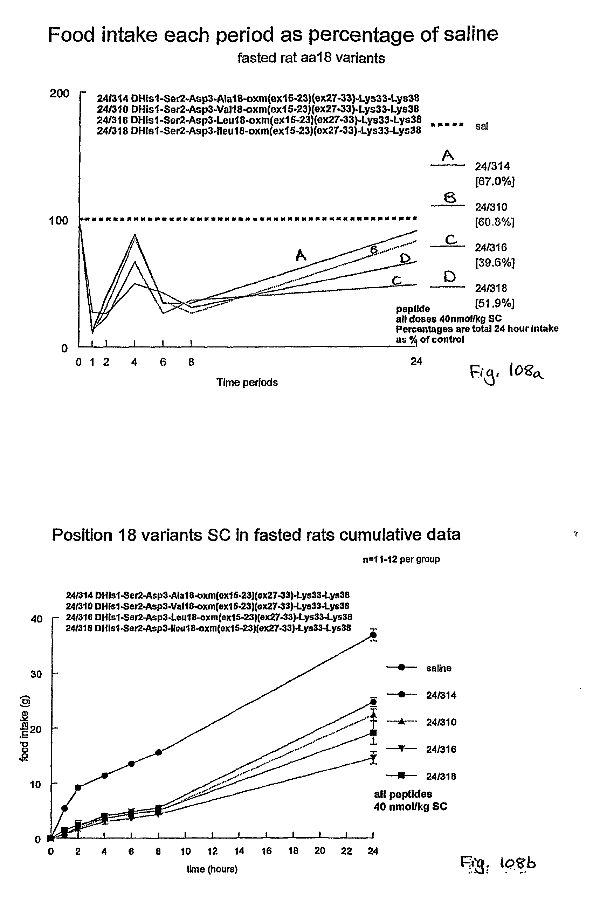

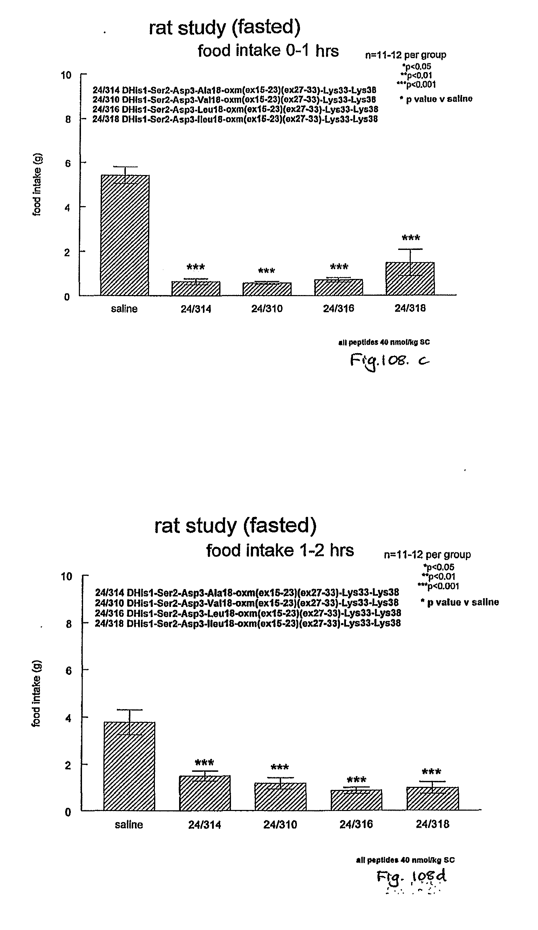

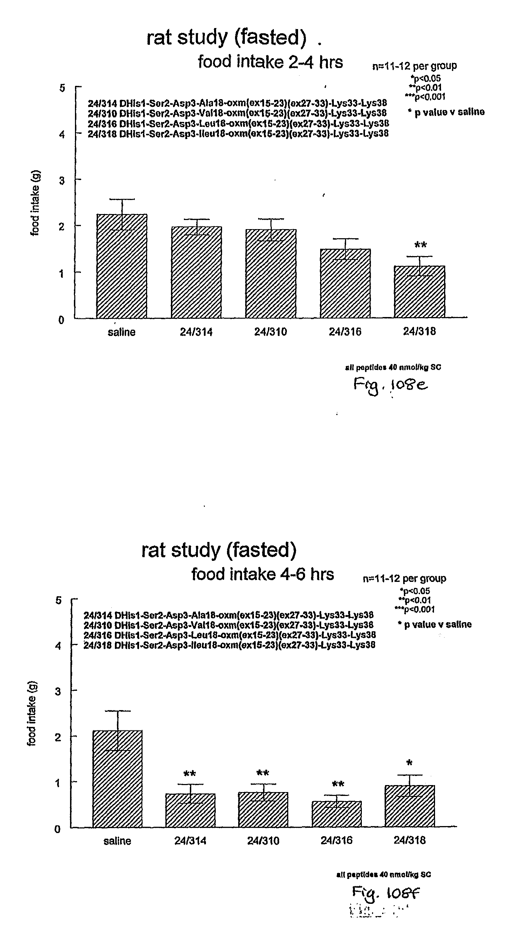

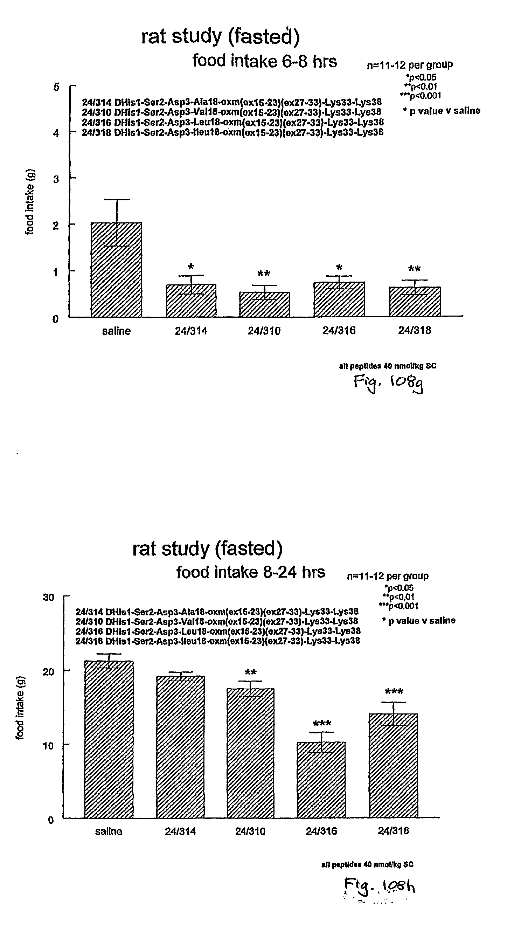

Doses are stated in Fig. 108. Food intake data is presented in Fig. 108.

### Example 99

Groups of fasted rats were injected with the following:
saline
24/310 DHis1-Ser2-Asp3-Val18-oxm(ex15-23)(ex27-33)Lys33-Lys38 (SEQ ID NO : 114)
23/310 DHis1-Ser2-Asp3-Val18-oxm(ex15-23)(ex27-33)Lys33-Lys38-octanoyl (SEQ ID NO : 160)
23/311 DHis1-Ser2-Asp3-Val18-oxm(ex15-23)(ex27-33)Lys33-Lys38-lauroyl (SEQ ID NO : 161)
23/312 DHis1-Ser2-Asp3-Val18-oxm(ex15-23)(ex27-33)Lys33-Lys38-palmitoyl (SEQ ID NO : 162)

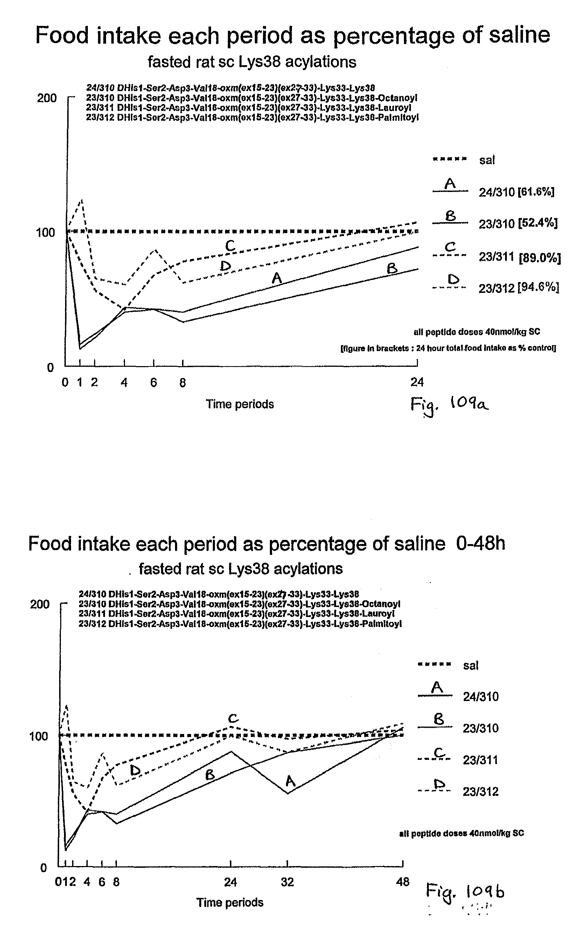

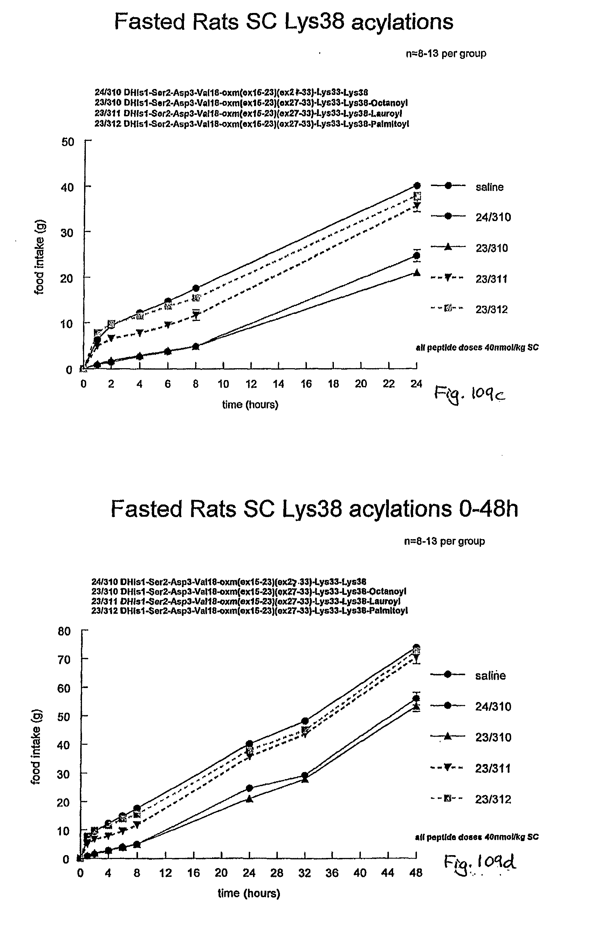

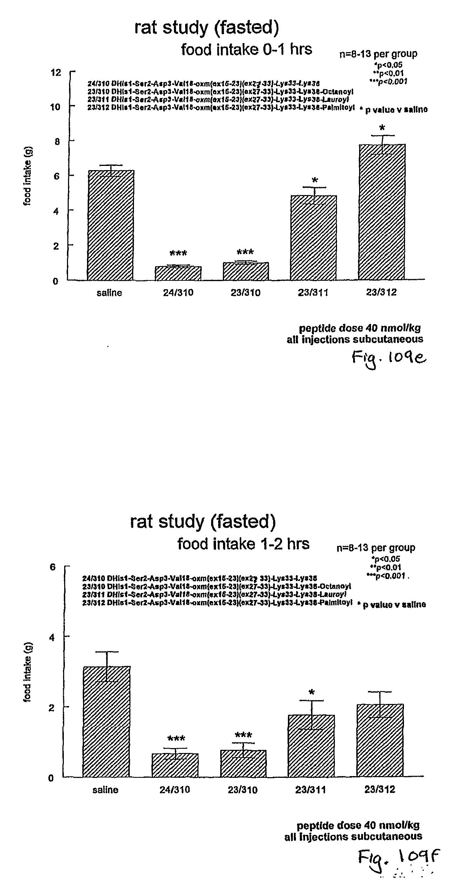

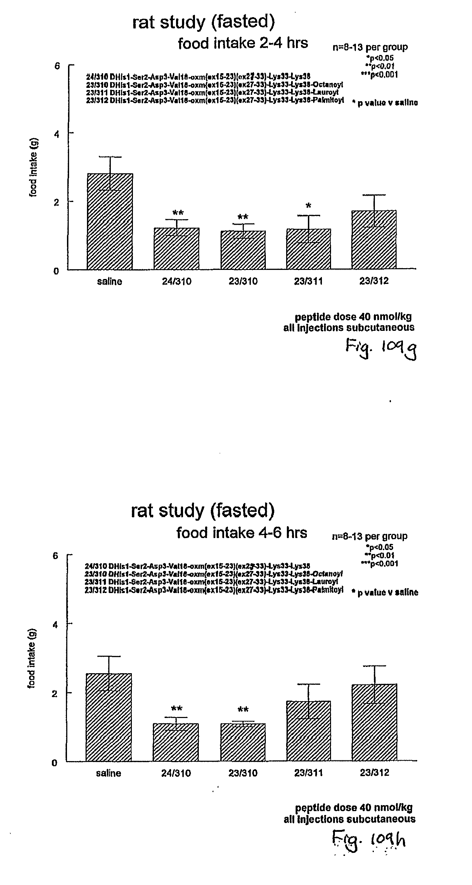

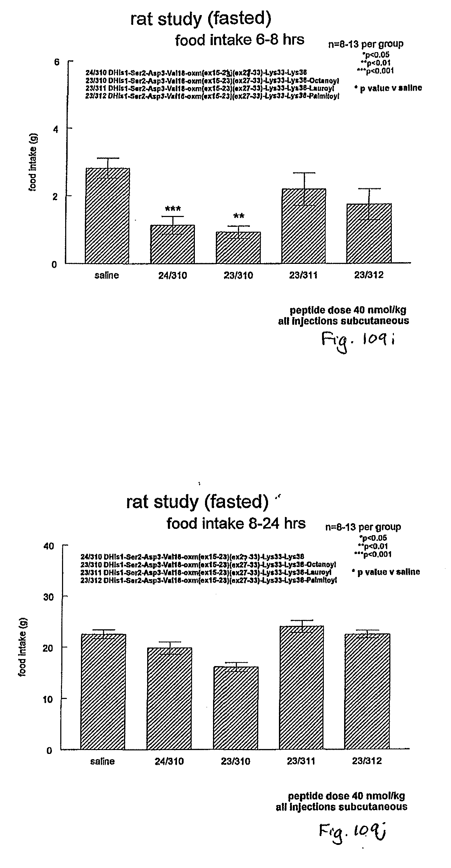

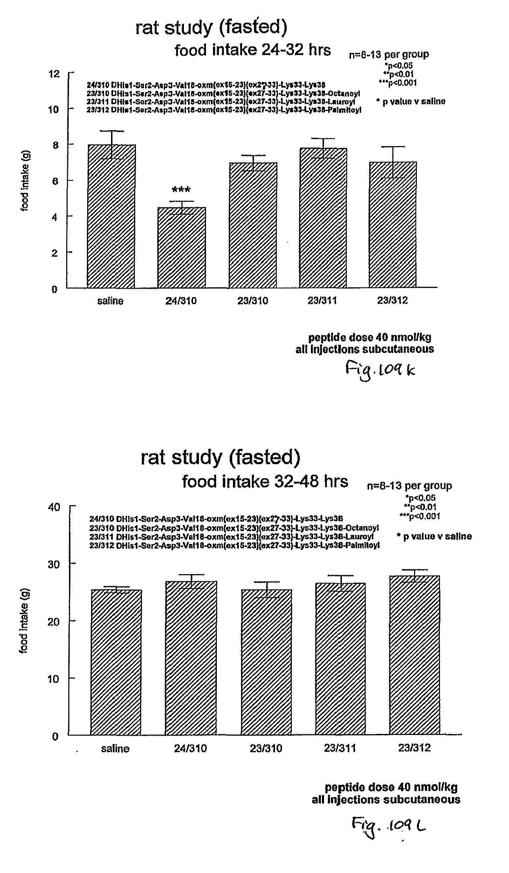

Doses are stated in Fig. 109. Food intake data is presented in Fig. 109.

### Example 100

Groups of fasted rats were injected with the following:
saline
1/4 Exendin-4 (SQE ID NO: 22)
24/310 D-His1-Ser2-Asp3-Val18-OXM(ex15-23)(ex27-33)-Lys 33-Lys 38 (SEQ ID NO: 114)
23/310 D-His1-Ser2-Asp3-Val18-OXM(ex15-23)(ex27-33)-Lys 33-Lys 38-octanoyl (SQE ID NO: 160)

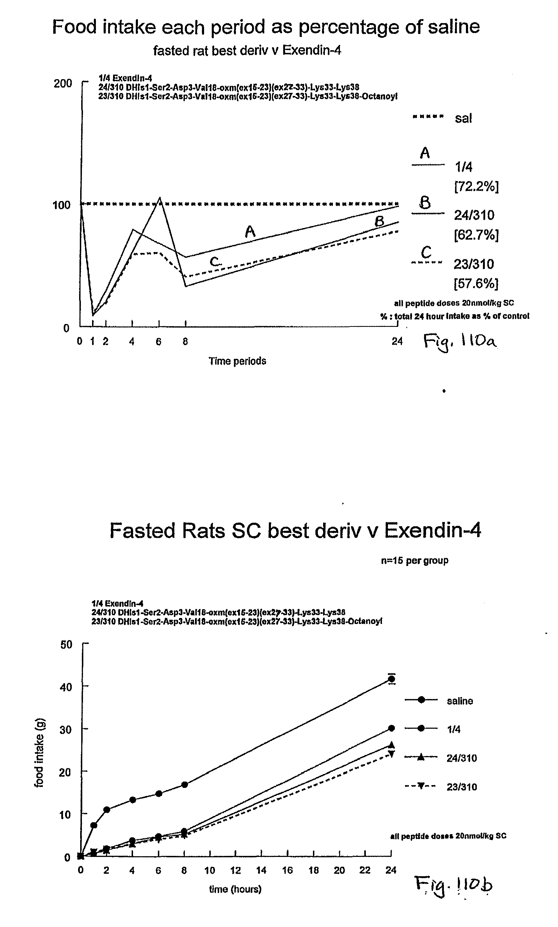

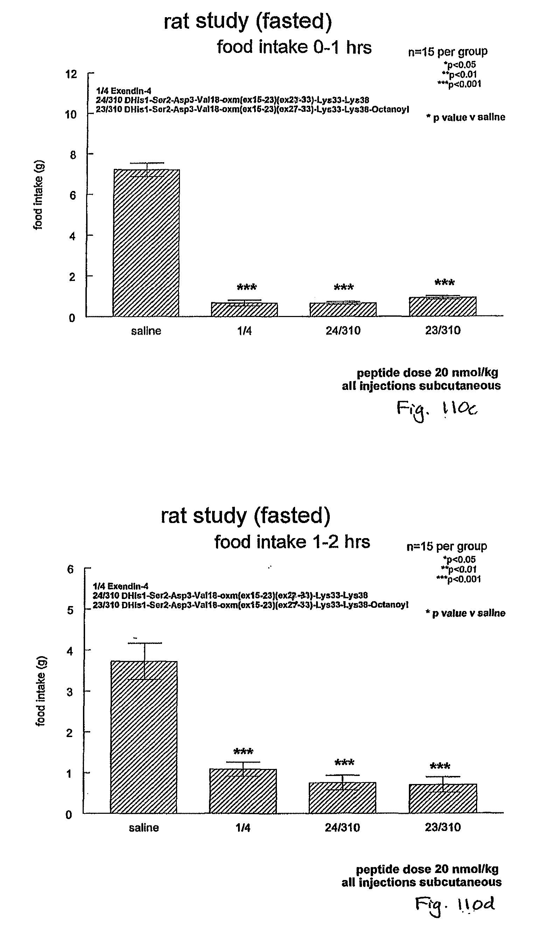

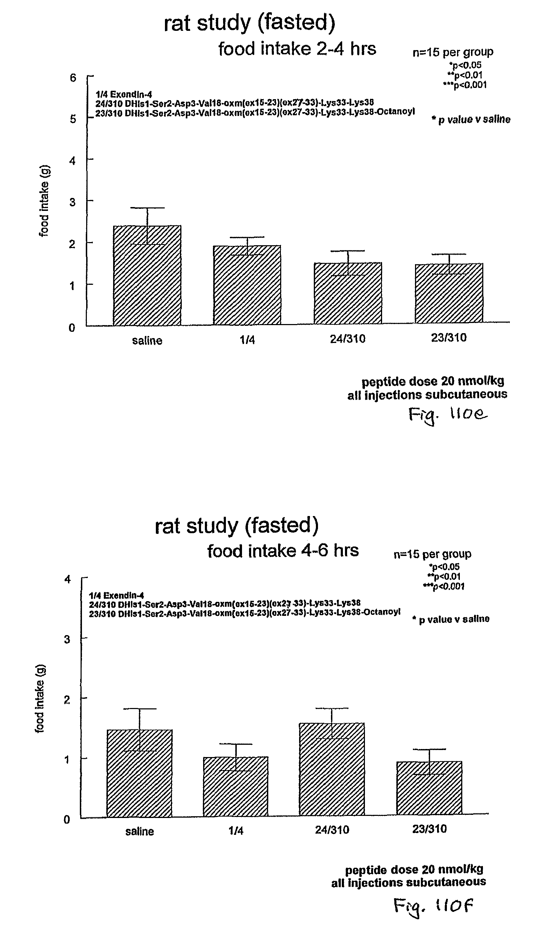

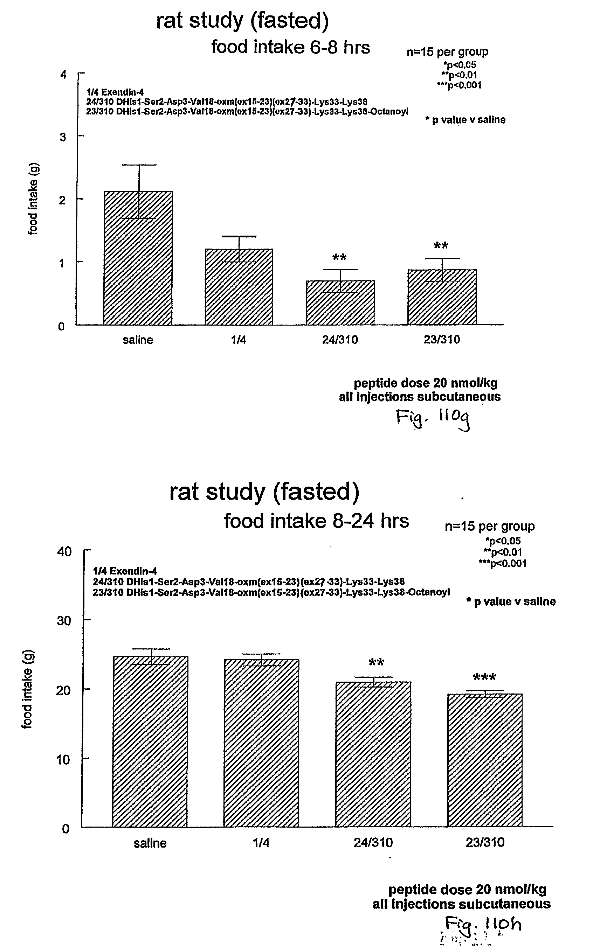

Doses are stated in Fig. 110. Food intake data is presented in Fig. 110.

### Example 101

Groups of fasted rats were injected with the following:
saline
1/4 Exendin-4 (SEQ ID NO : 165)
240/10 D-His1-Leu18-Exendin-4 (SEQ ID NO : 165)
24/340 D-His1-Ser2-Gln16, Leu18-OXM(ex15-23)(ex27-33)Ala37-NH2 (SEQ ID NO : 166)
24/342 D-His1-Ala2-Gln16, Leu18-OXM(ex15-23)(ex27-33)Ala37-NH2 (SEQ ID NO : 167)
24/344 D-His1-Ala2-Gln16, Leu18-OXM(ex15-23)(ex27-33)Lys38-NH2 (SEQ ID NO : 168)

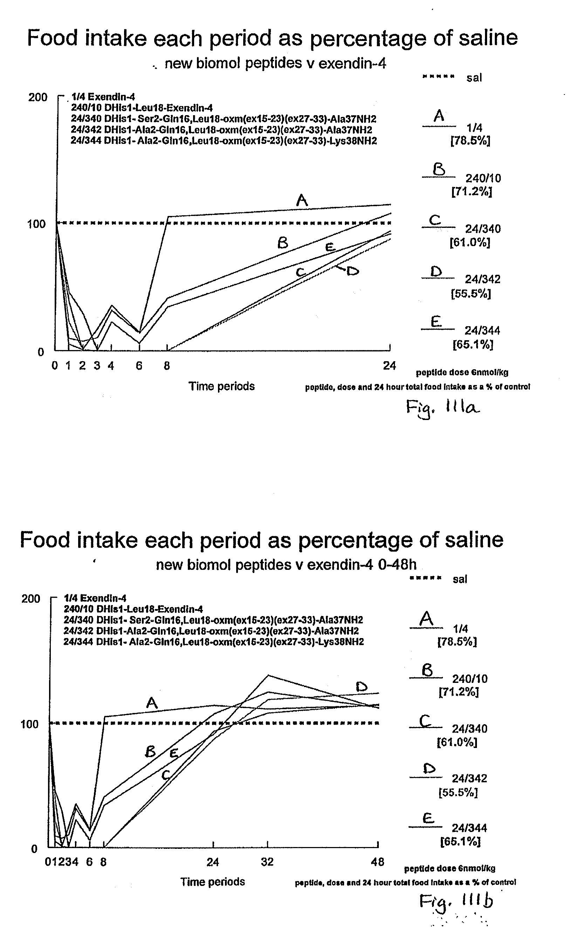

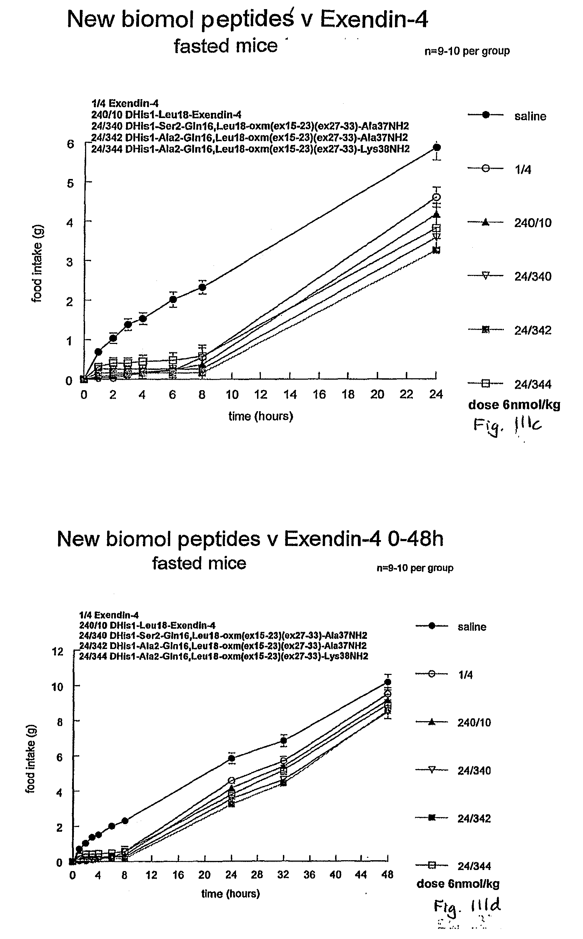

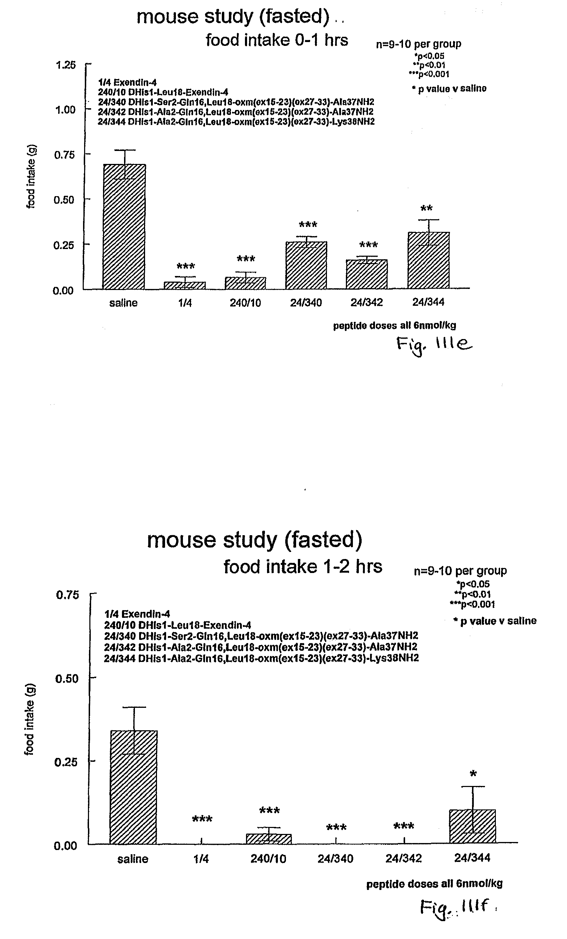

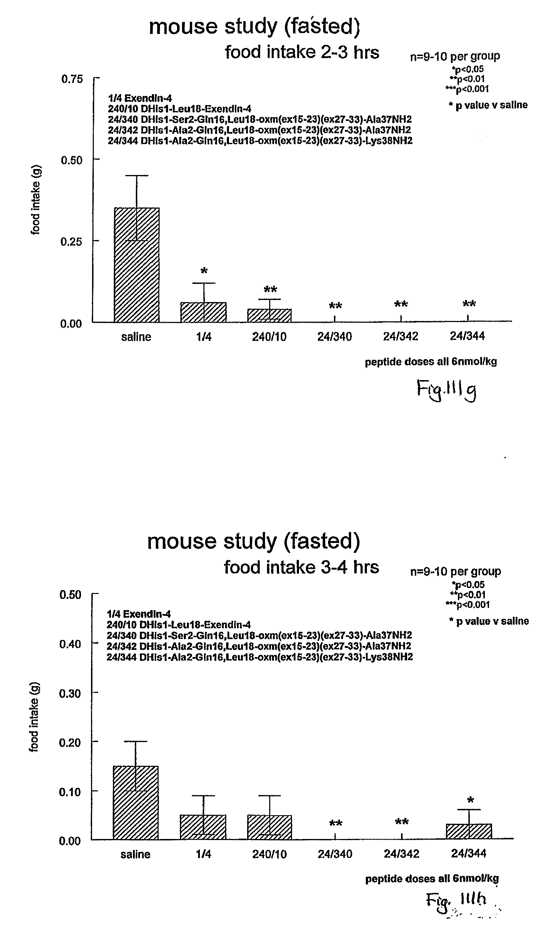

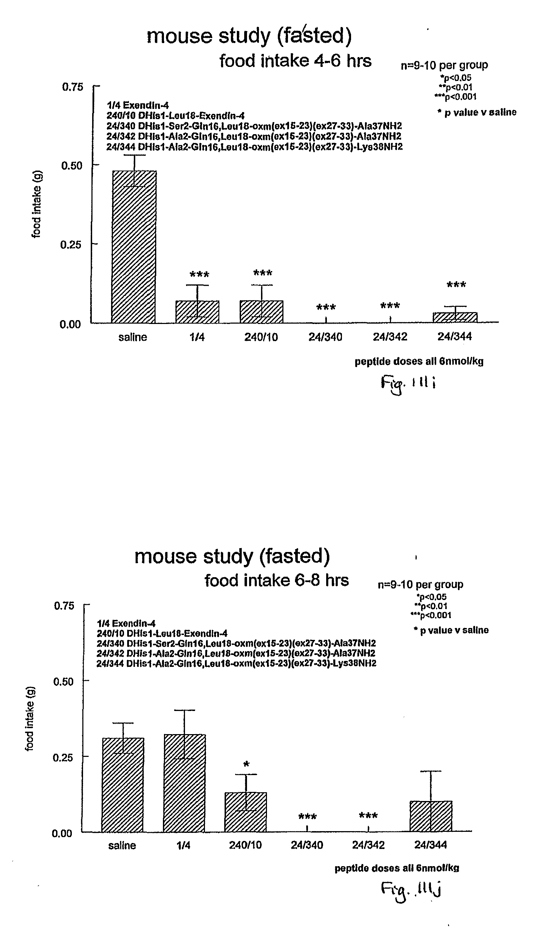

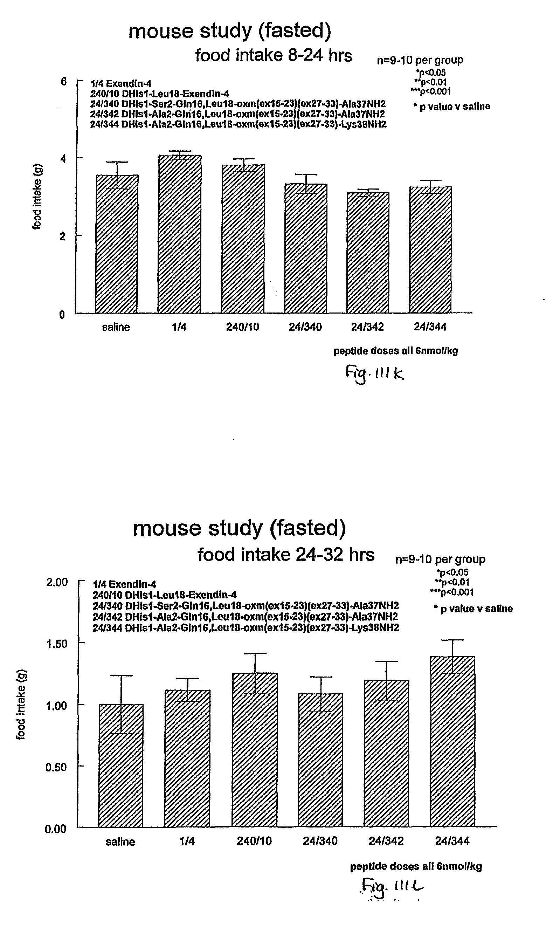

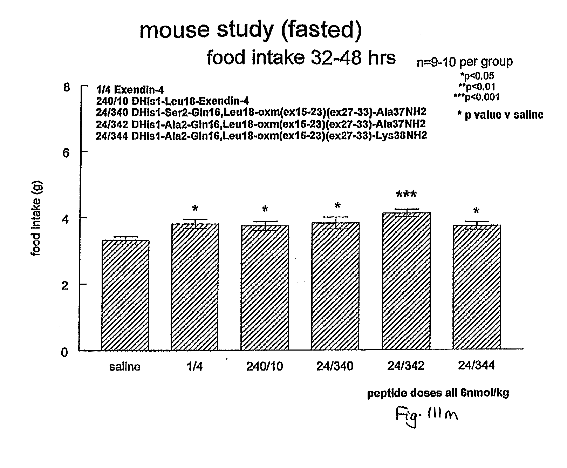

Doses are stated in Fig. 111. Food intake data is presented in Fig. 111.

### Example 102

Groups of fasted mice were injected with the following:
saline
1/4 Exendin-4 (SEQ ID NO : 22)
24/342 D-His1-Ala2-Gln16, Leu18-OXM(ex15-23)(ex27-33)Ala37-NH2 (SEQ ID NO : 167)
24/344 D-His1-Ala2-Gln16, Leu18-OXM(ex15-23)(ex27-33)Lys38-NH2 (SEQ ID NO : 168)
23/344 D-His1-Ala2-Gln16, Leu18-OXM(ex15-23)(ex27-33)Lys38-Lauroyl (SEQ ID NO : 169)

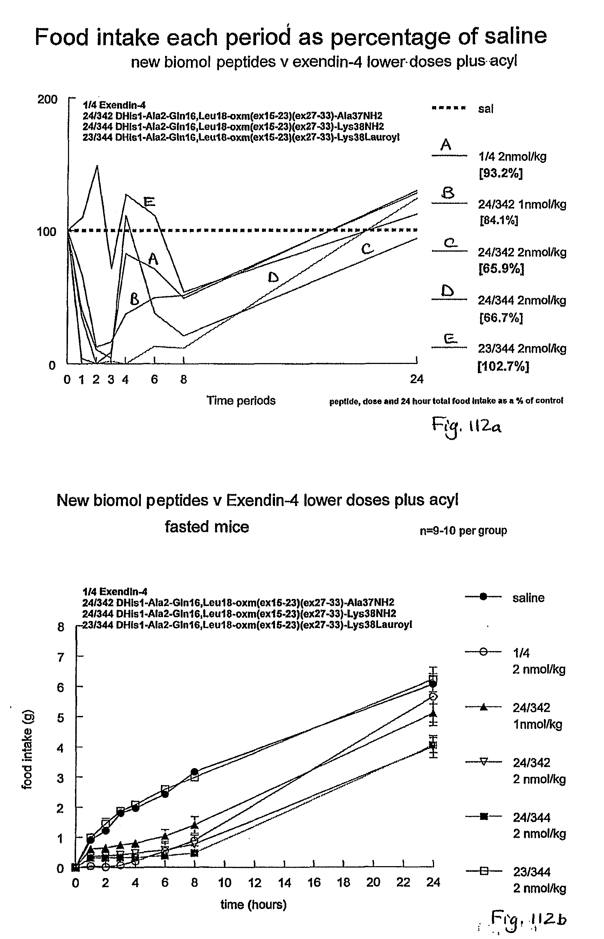

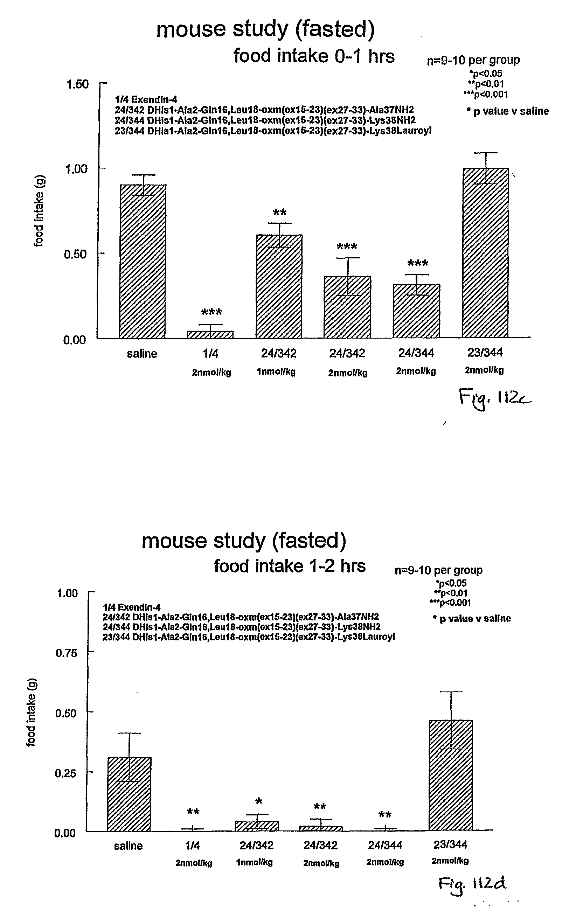

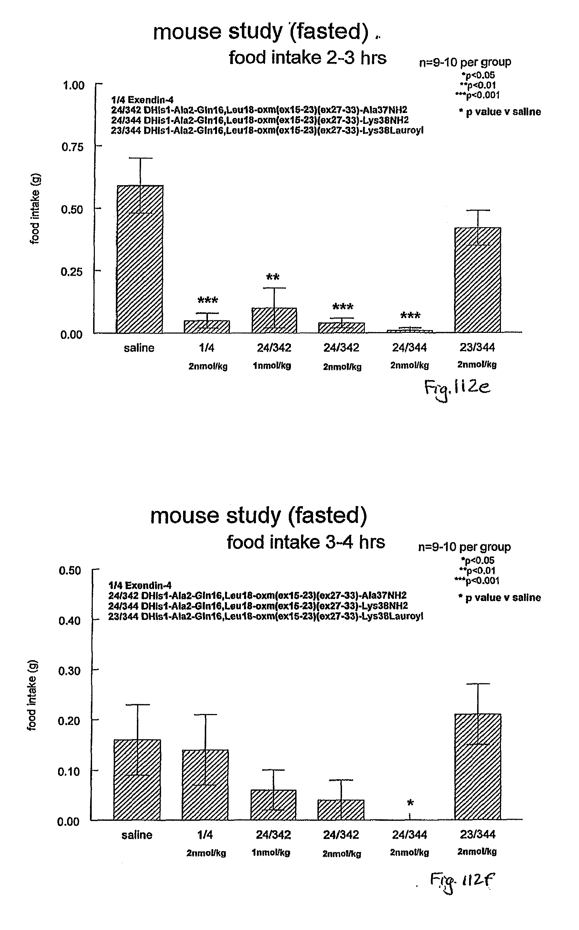

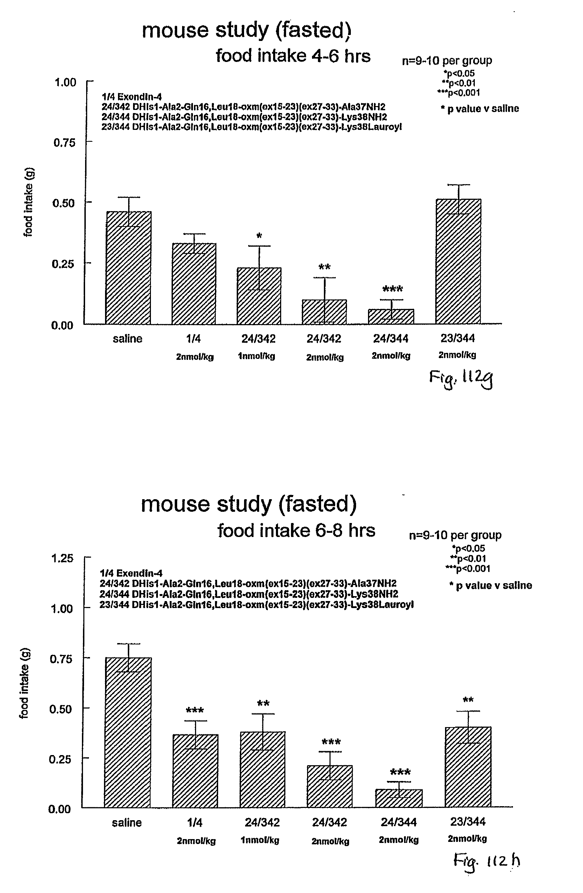

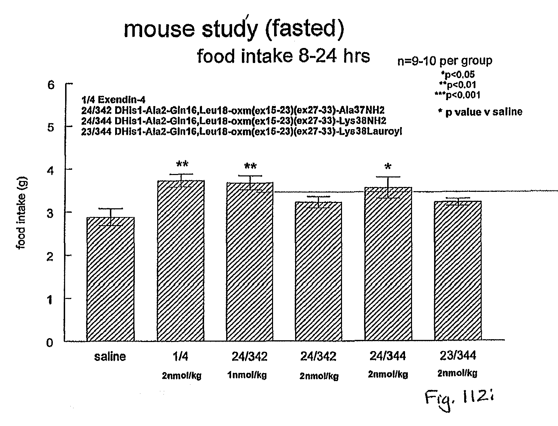

Doses are stated in Fig. 112. Food intake data is presented in Fig. 112.

### Example 103

Groups of fasted mice were injected with the following:
saline
24/344 D-His1-Ala2-Gln3-Gln16, Leu18-OXM(ex15-23)(ex27-33)Lys38-NH2 (SEQ ID NO : 168)
24/331 D-His1-Ala2-Asp3, Leu18-OXM(ex15-24)(ex27-33)Lys33-Lys38 (SEQ ID NO : 157)
23/344 D-His1-Ala2-Gln3-Gln16, Leu18-OXM(ex15-23)(ex27-33)Lys38-Lauroyl (SEQ ID NO : 169)

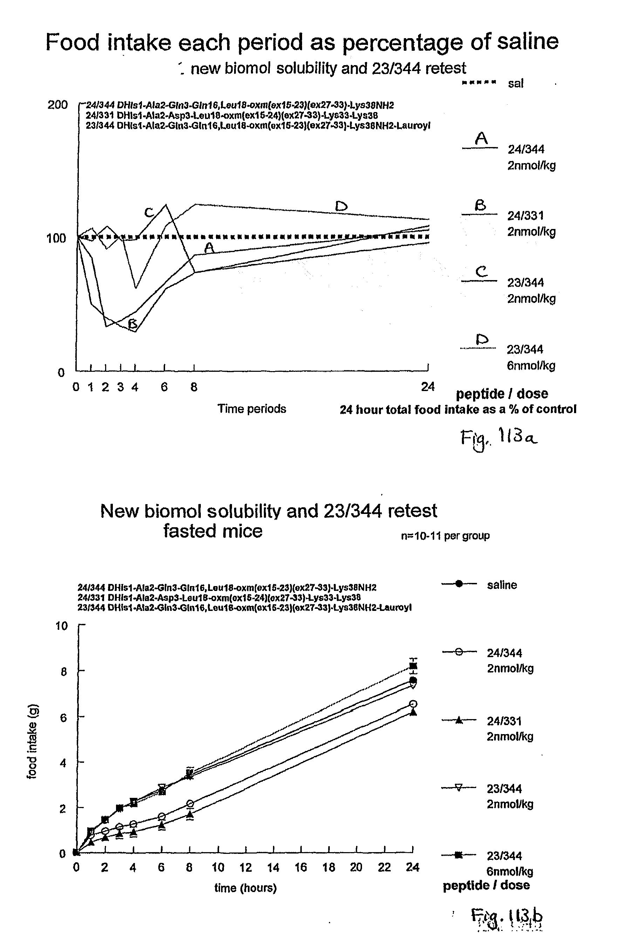

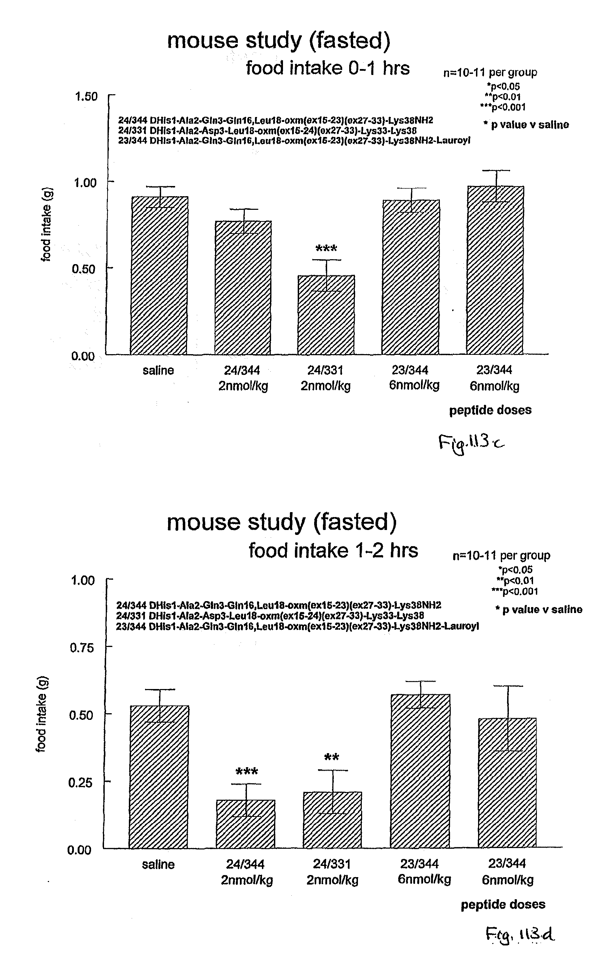

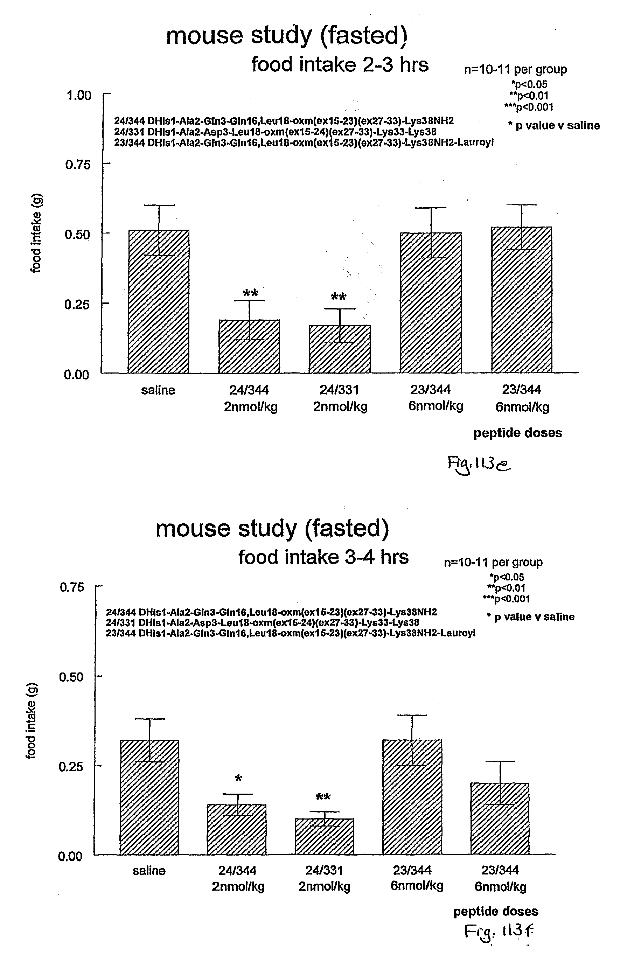

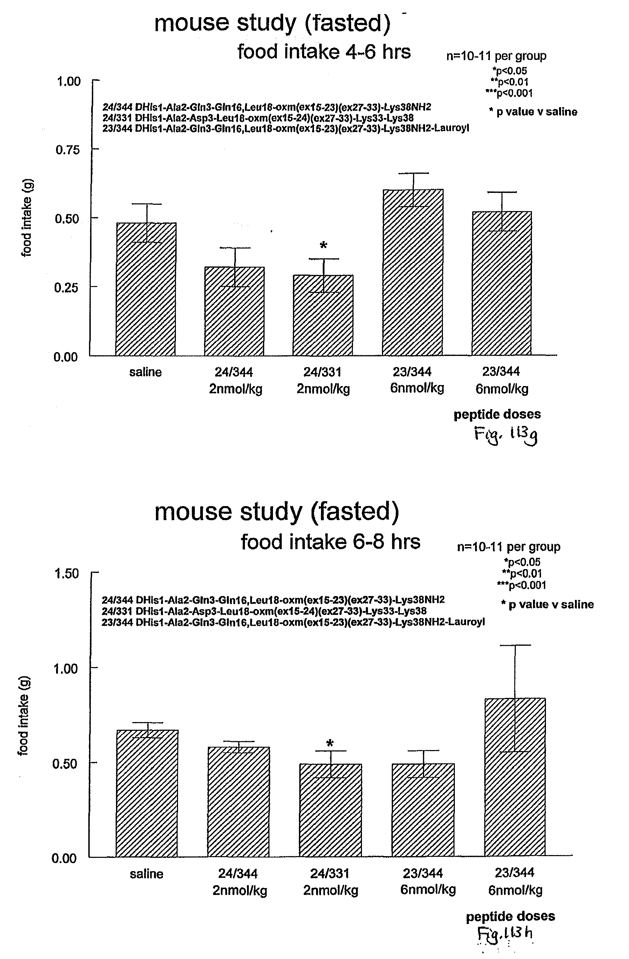

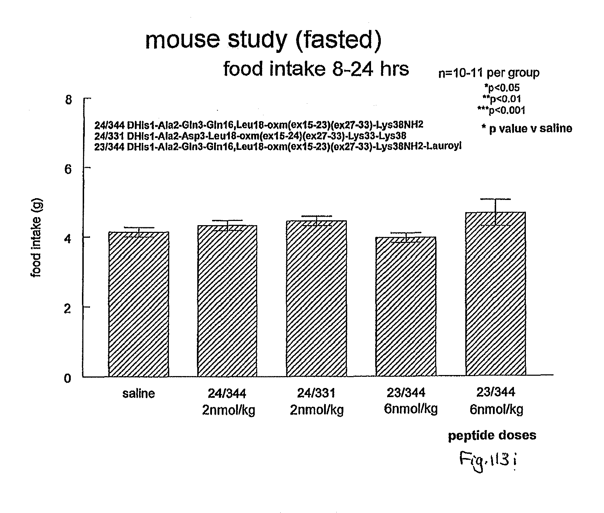

Doses are stated in Fig. 113. Food intake data is presented in Fig. 113.

### Example 104

Groups of fasted mice were injected with the following:
saline
24/343 D-His1-Ala2-Asp3-Ile18-OXM(ex15-23)(ex27-33)-Lys33-Lys3 (SEQ ID NO : 170)
24/347 D-His1-Ala2-Asp3-Gln16-Ile18-Lys20-Tyr21OXM(ex15-23)(ex27-33)-Lys33-Lys38 (SEQ ID NO: 171)
24/348 D-His1-Ala2-Asp3-Ile18-Lys20-Tyr21-Val23-OXM(ex15-23)(ex27-33)-Lys33-Lys38 (SEQ ID NO : 172)

24/349 D-His1-Ala2-Asp3-Arg15-Ile16-Ile18-Lys20-Tyr21-OXM(ex15-23)(ex27-33)-Lys33-
Lys38 (SEQ ID NO : 173)

24/350 D-His1-Ala2-Asp3-Lys15-Ile16-Ile18-Lys20-Tyr21-OXM(ex15-23)(ex27-33)-Lys33-
Lys38 (SEQ ID NO : 174)

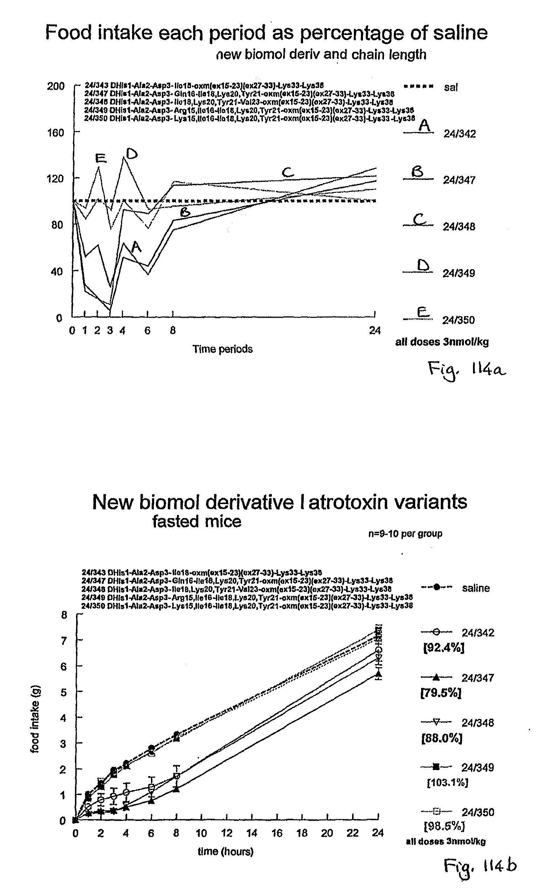

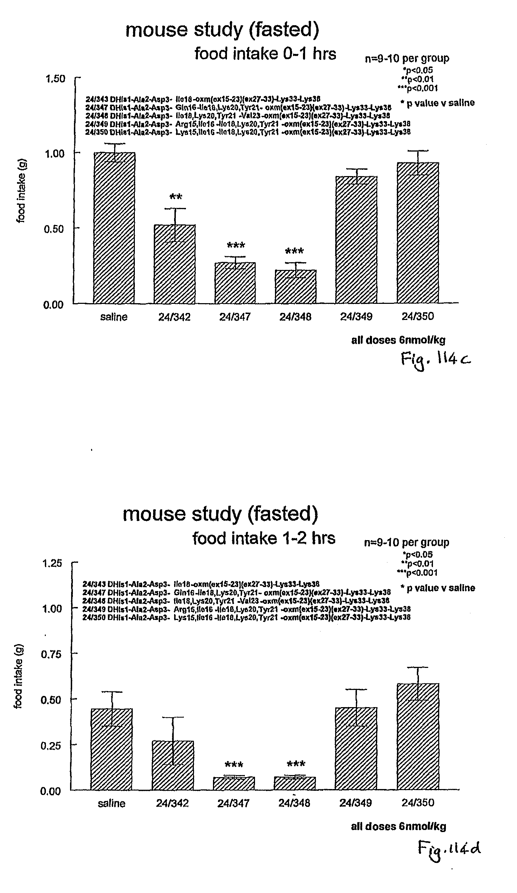

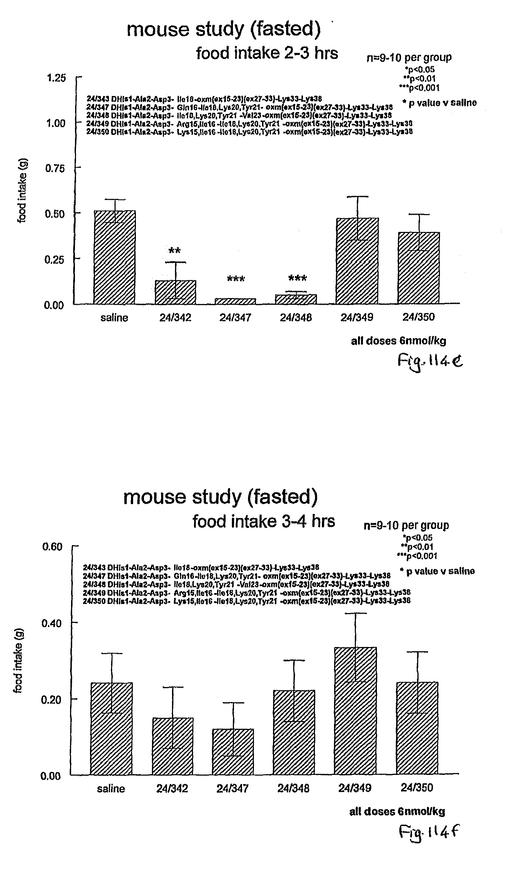

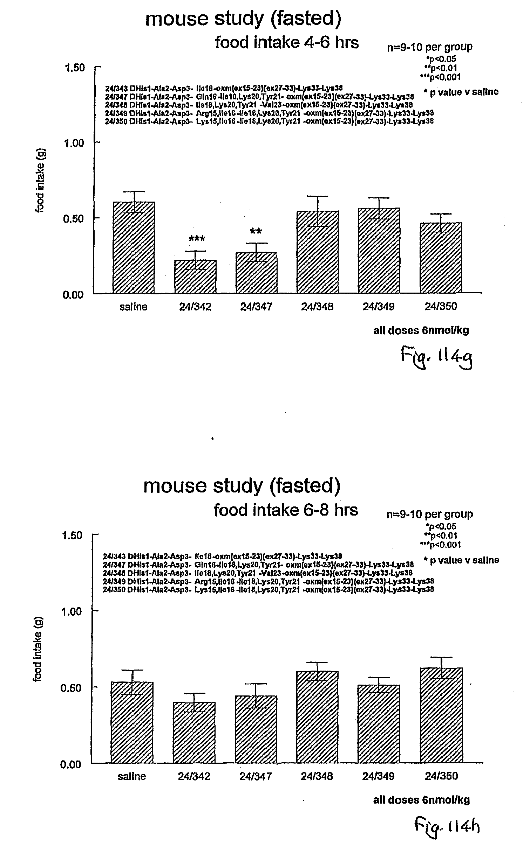

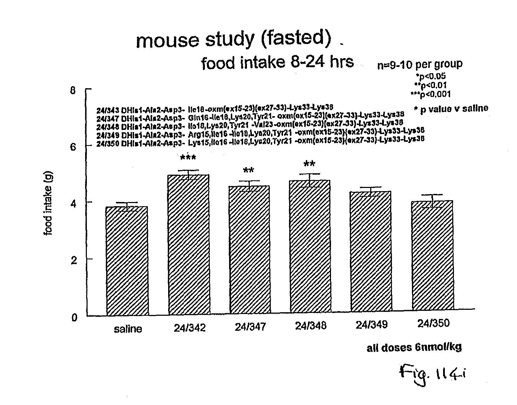

Doses are stated in Fig. 114. Food intake data is presented in Fig. 114.

### Example 105

Groups of fasted mice were injected with the following:
saline
24/331 D-His1-Ala2-Asp3-Leu18-OXM(ex15-24)(ex27-33)Lys33-Lys3 (SEQ ID NO : 157)
24/370 D-His1-Ala2-Asp3-Leu18-OXM(ex15-23)(ex27-33)Lys33-Lys38 (SEQ ID NO : 175)
23/370 D-His1-Ala2-Asp3-Leu18-OXM(ex15-23)(ex27-33)Lys33-Lys38-Octanoyl (SEQ ID NO: 176)
23/371 D-His1-Ala2-Asp3-Leu18-OXM(ex15-23)(ex27-33)Lys33-Lys38-Lauroyl (SEQ ID NO : 177)
23/372 D-His1-Ala2-Asp3-Leu18-OXM(ex15-23)(ex27-33)Lys33-Lys38-Palmitoyl (SEQ ID NO: 178)

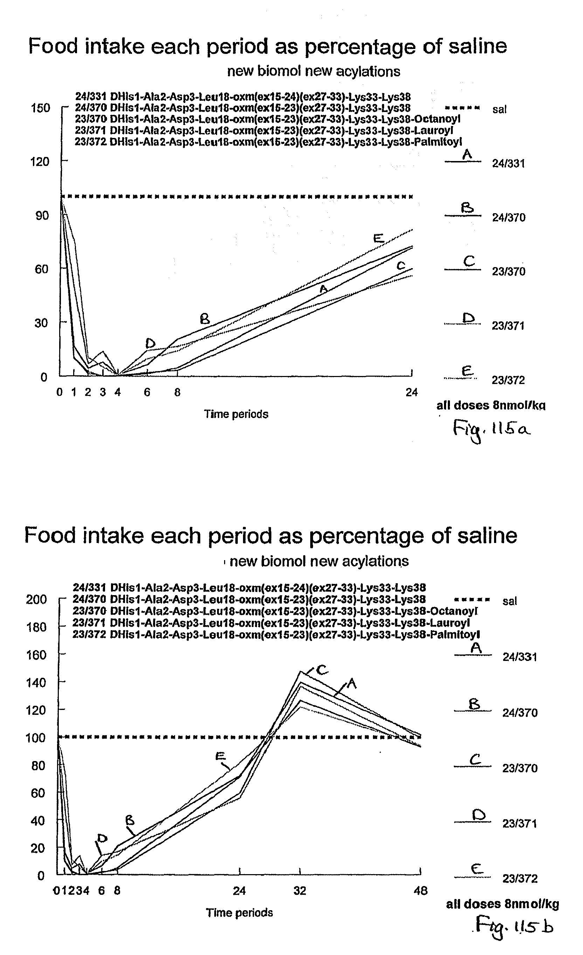

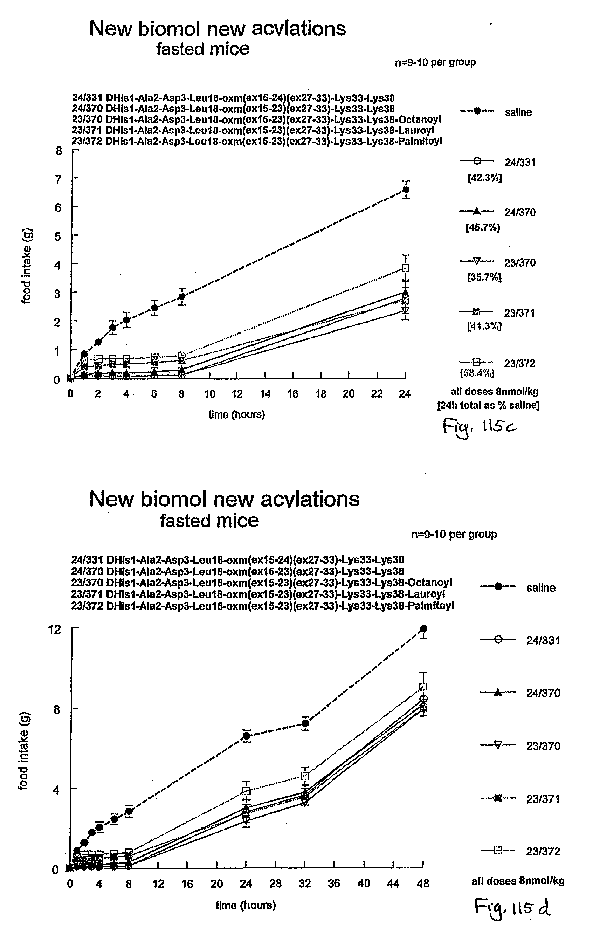

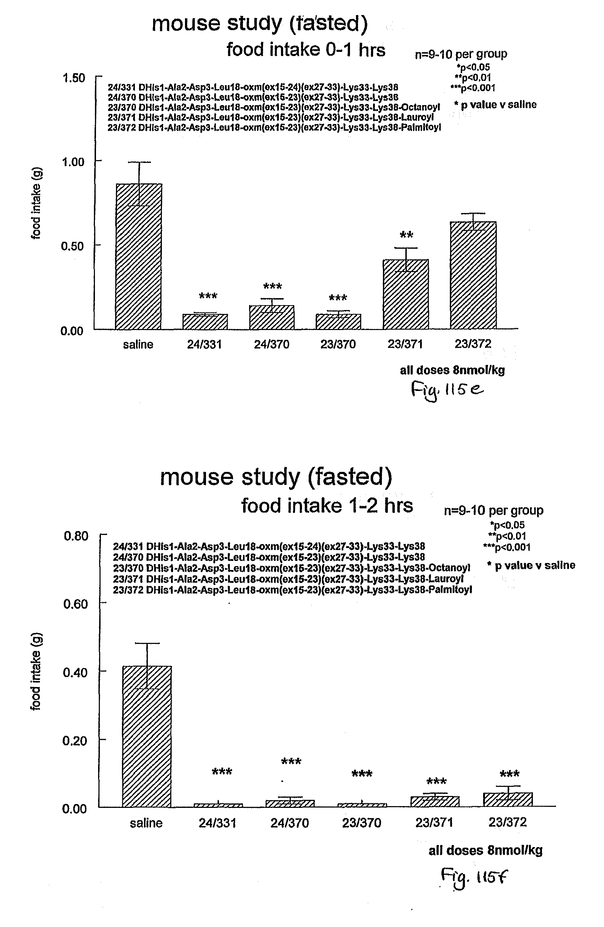

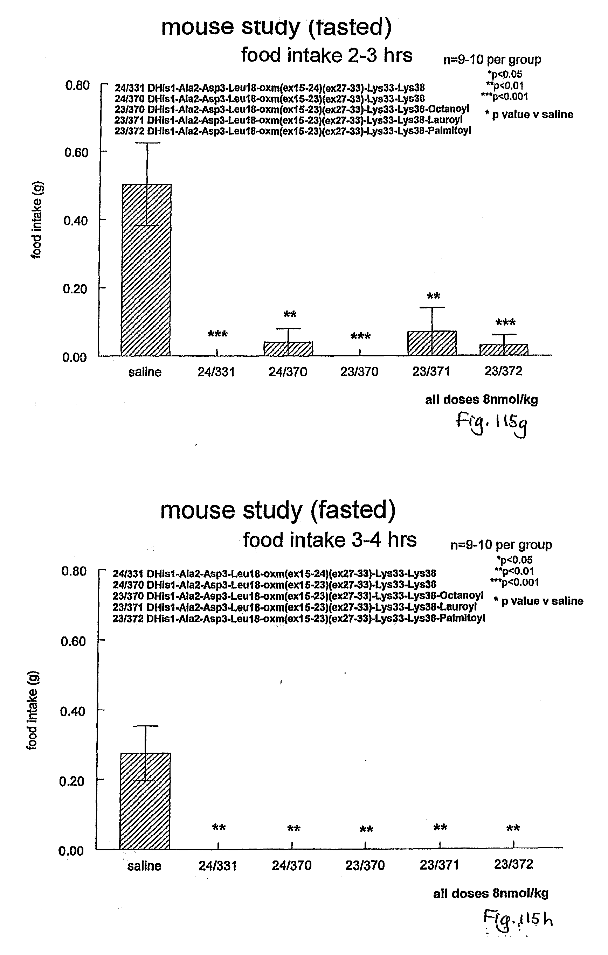

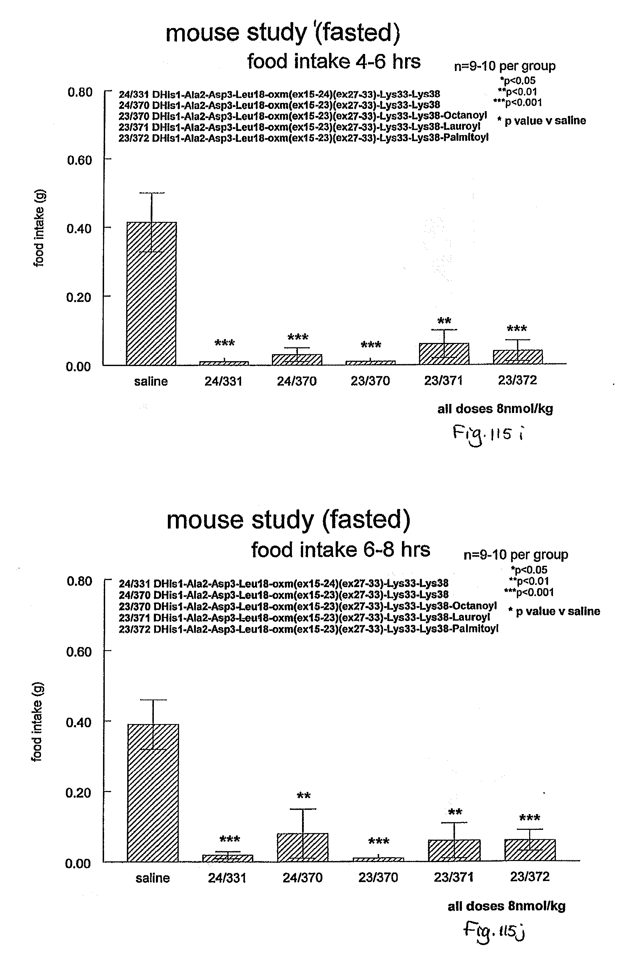

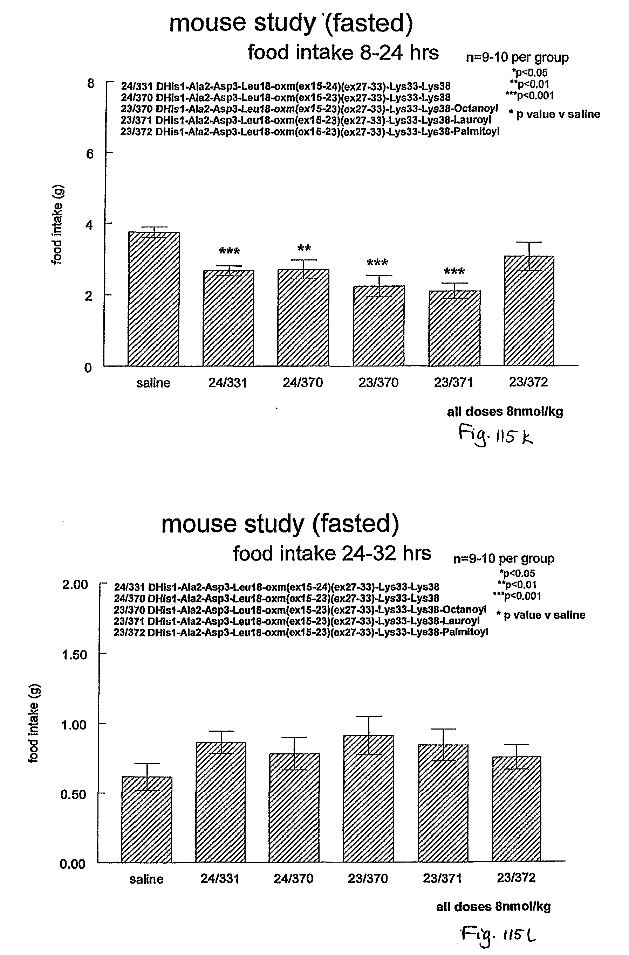

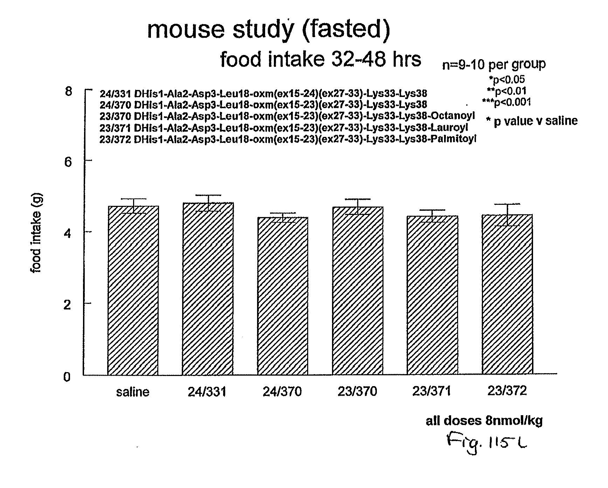

Doses are stated in Fig. 115. Food intake data is presented in Fig. 115.

### Example 106

Groups of fasted rats were injected with the following:
saline
1/4 Exendin-4 (SEQ ID NO : 22)
24/331 D-His1-Ala2-Asp3, Leu18-OXM(ex15-24)(ex27-33)Lys33-Lys38 (SEQ ID NO : 157)

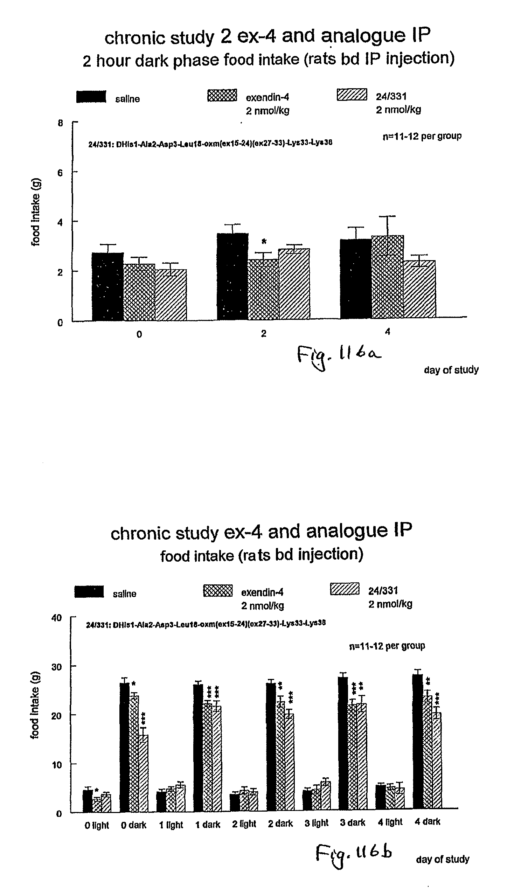

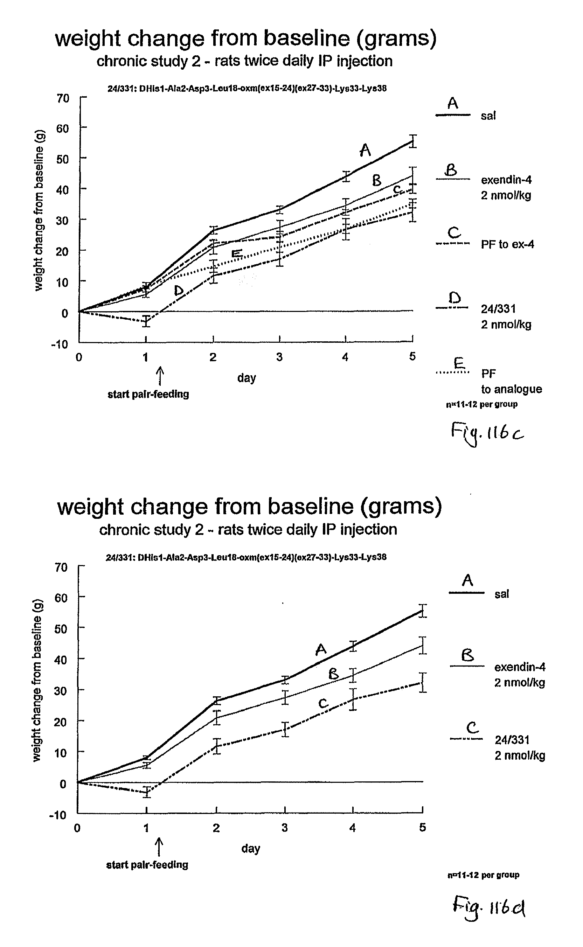

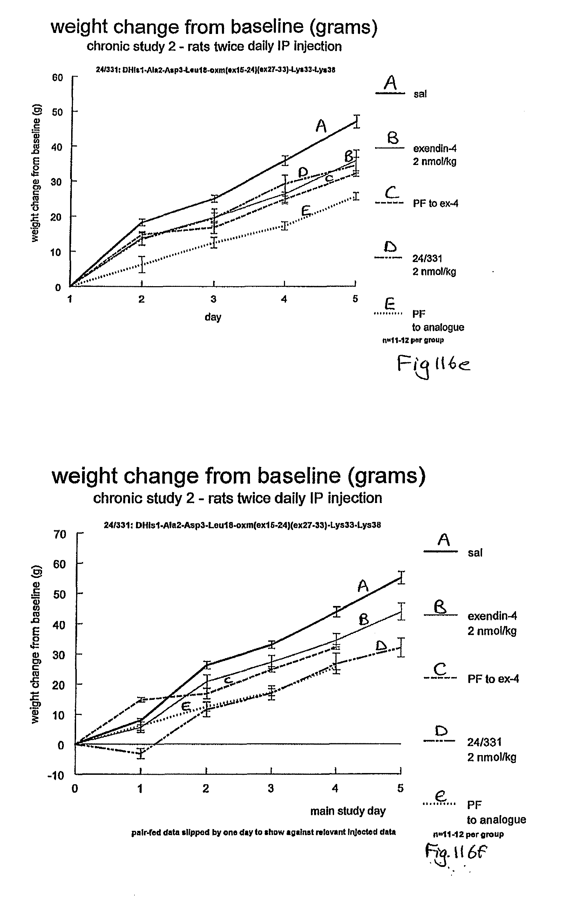

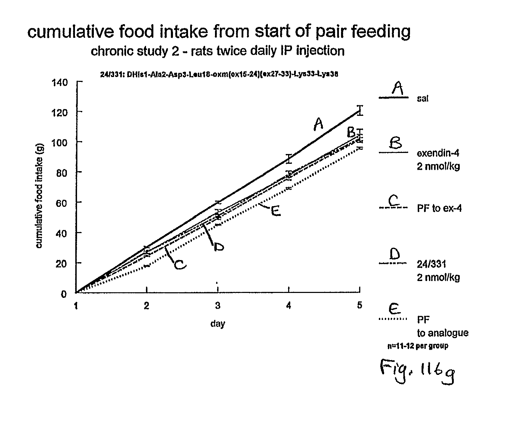

Doses are stated in Fig. 116. Food intake data is presented in Fig. 116.

### Further Example Sequences

Further example of sequences falling within the scope of the invention are given below in single letter amino acid code. It will be understood that the invention encompasses not only the sequences given below but variants and derivatives thereof as described herein. It should be noted that in the sequences given below, the amino acid code "H" at the first position in a sequence represents "D-histidine":
HADGTFTSDYSKYLRIELVKYFVGWLKNGGPSKNNIA
HADGTFTSDYSKYLEIELVKYFVGWLKNGGPSKNNIA
HADGTFTSDYSKYLREELVKYFVGWLKNGGPSKNNIA
HADGTFTSDYSKYLRIELVRYFVGWLKNGGPSKNNIA
HADGTFTSDYSKYLRIELVKLFVGWLKNGGPSKNNIA
HADGTFTSDYSKYLRIELVKYFIGWLKNGGPSKNNIA
HADGTFTSDYSKYLRIELVKYFVEWLKNGGPSKNNIA
HADGTFTSDYSKYLEEELVKYFVGWLKNGGPSKNNIA
HADGTFTSDYSKYLEIELVRYFVGWLKNGGPSKNNIA
HADGTFTSDYSKYLEIELVKLFVGWLKNGGPSKNNIA
HADGTFTSDYSKYLEIELVKLFIGWLKNGGPSKNNIA
HADGTFTSDYSKYLEIELVKYFVEWLKNGGPSKNNIA
HADGTFTSDYSKYLREELVRYFVGWLKNGGPSKNNIA
HADGTFTSDYSKYLREELVKLFVGWLKNGGPSKNNIA
HADGTFTSDYSKYLREELVKYFIGWLKNGGPSKNNIA
HADGTFTSDYSKYLREELVKYFVEWLKNGGPSKNNIA
HADGTFTSDYSKYLRIELVRLFVGWLKNGGPSKNNIA
HADGTFTSDYSKYLRIELVRYFIGWLKNGGPSKNNIA
HADGTFTSDYSKYLRIELVRYFVEWLKNGGPSKNNIA
HADGTFTSDYSKYLRIELVKLFIGWLKNGGPSKNNIA
HADGTFTSDYSKYLRIELVKLFVEWLKNGGPSKNNIA
HADGTFTSDYSKYLRIELVKYFIEWLKNGGPSKNNIA
HADGTFTSDYSKYLEEELVRYFVGWLKNGGPSKNNIA
HADGTFTSDYSKYLEEELVKLFVGWLKNGGPSKNNIA
HADGTFTSDYSKYLEEELVKYFIGWLKNGGPSKNNIA
HADGTFTSDYSKYLEEELVKYFVEWLKNGGPSKNNIA
HADGTFTSDYSKYLEIELVRLFVGWLKNGGPSKNNIA
HADGTFTSDYSKYLEIELVRYFIGWLKNGGPSKNNIA
HADGTFTSDYSKYLEIELVRYFVEWLKNGGPSKNNIA
HADGTFTSDYSKYLEIELVKLFIGWLKNGGPSKNNIA
HADGTFTSDYSKYLEIELVKLFVEWLKNGGPSKNNIA
HADGTFTSDYSKYLEIELVKLFVEWLKNGGPSKNNIA
HADGTFTSDYSKYLEIELVKYFIEWLKNGGPSKNNIA
HADGTFTSDYSKYLREELVRLFVGWLKNGGPSKNNIA
HADGTFTSDYSKYLREELVRYFIGWLKNGGPSKNNIA
HADGTFTSDYSKYLREELVRYFVEWLKNGGPSKNNIA
HADGTFTSDYSKYLREELVKLFIGWLKNGGPSKNNIA
HADGTFTSDYSKYLREELVKLFVEWLKNGGPSKNNIA
HADGTFTSDYSKYLREELVKYFIEWLKNGGPSKNNIA
HADGTFTSDYSKYLRIELVRLFIGWLKNGGPSKNNIA
HADGTFTSDYSKYLRIELVRLFVEWLKNGGPSKNNIA
HADGTFTSDYSKYLRIELVKLFIEWLKNGGPSKNNIA
HADGTFTSDYSKYLEEELVRLFVGWLKNGGPSKNNIA
HADGTFTSDYSKYLEEELVRYFIGWLKNGGPSKNNIA
HADGTFTSDYSKYLEEELVRYFVEWLKNGGPSKNNIA
HADGTFTSDYSKYLEIELVRLFIGWLKNGGPSKNNIA
HADGTFTSDYSKYLEIELVRLFIGWLKNGGPSKNNIA
HADGTFTSDYSKYLEIELVRLFVEWLKNGGPSKNNIA
HADGTFTSDYSKYLREELVRLFIQWLKNGGPSKNNIA
HADGTFTSDYSKYLREELVRLFVEWLKNGGPSKNNIA
HADGTFTSDYSKYLRIELVRLFIEWLKNGGPSKNNIA
HADGTFTSDYSKYLEEELVRLFIGWLKNGGPSKNNIA
HADGTFTSDYSKYLEEELVRLFIGWLKNGGPSKNNIA
HADGTFTSDYSKYLEEELVRLFVEWLKNGGPSKNNIA
HADGTFTSDYSKYLEEELVRYFIEWLKNGGPSKNNIA
HADGTFTSDYSKYLEEELVKLFIEWLKNGGPSKNNIA
HADGTFTSDYSKYLEIELVRLFIEWLKNGGPSKNNIA
HADGTFTSDYSKYLREELVRLFIEWLKNGGPSKNNIA
HADGTFTSDYSKYLEEELVRLFIEWLKNGGPSKNNIA
HADGTFTSDYSKYLRIELVKYFVGWLMNTKRNKNNIA
HADGTFTSDYSKYLEIELVKYFVGWLMNTKRNKNNIA
HADGTFTSDYSKYLREELVKYFVGWLMNTKRNKNNIA
HADGTFTSDYSKYLRIELVRYFVGWLMNTKRNKNNIA
HADGTFTSDYSKYLRIELVKLFVGWLMNTKRNKNNIA
HADGTFTSDYSKYLRIELVKYFIGWLMNTKRNKNNIA
HADGTFTSDYSKYLRIELVKYFVEWLMNTKRNKNNIA
HADGTFTSDYSKYLEEELVKYFVGWLMNTKRNKNNIA
HADGTFTSDYSKYLEIELVRYFVGWLMNTKRNKNNIA
HADGTFTSDYSKYLEIELVKLFVGWLMNTKRNKNNIA
HADGTFTSDYSKYLEIELVKLFIGWLMNTKRNKNNIA
HADGTFTSDYSKYLEIELVKYFVEWLMNTKRNKNNIA
HADGTFTSDYSKYLREELVRYFVGWLMNTKRNKNNIA
HADGTFTSDYSKYLREELVRYFVGWLMNTKRNKNNIA
HADGTFTSDYSKYLREELVKLFVGWLMNTKRNKNNIA
HADGTFTSDYSKYLREELVKYFIGWLMNTKRNKNNIA
HADGTFTSDYSKYLREELVKYFIGWLMNTKRNKNNIA
HADGTFTSDYSKYLREELVKYFIGWLMNTKRNKNNIA
HADGTFTSDYSKYLREELVKYFVEWLMNTKRNKNNIA
HADGTFTSDYSKYLRIELVRLFVGWLMNTKRNKNNIA
HADGTFTSDYSKYLRIELVRYFIGWLMNTKRNKNNIA
HADGTFTSDYSKYLRIELVRYFVEWLMNTKRNKNNIA
HADGTFTSDYSKYLRIELVKLFIGWLMNTKRNKNNIA
HADGTFTSDYSKYLRIELVKLFVEWLMNTKRNKNNIA
HADGTFTSDYSKYLRIELVKYFIEWLMNTKRNKNNIA
HADGTFTSDYSKYLEEELVRYFVGWLMNTKRNKNNIA
HADGTFTSDYSKYLEEELVKLFVGWLMNTKRNKNNIA
HADGTFTSDYSKYLEEELVKYFIGWLMNTKRNKNNIA
HADGTFTSDYSKYLEEELVKYFIGWLMNTKRNKNNIA
HADGTFTSDYSKYLEEELVKYFVEWLMNTKRNKNNIA
HADGTFTSDYSKYLEIELVRLFVGWLMNTKRNKNNIA
HADGTFTSDYSKYLEIELVRYFIGWLMNTKRNKNNIA
HADGTFTSDYSKYLEIELVRYFVEWLMNTKRNKNNIA
HADGTFTSDYSKYLEIELVKLFIGWLMNTKRNKNNIA
HADGTFTSDYSKYLEIELVKLFVEWLMNTKRNKNNIA
HADGTFTSDYSKYLEIELVKYFIEWLMNTKRNKNNIA
HADGTFTSDYSKYLREELVRLFVGWLMNTKRNKNNIA
HADGTFTSDYSKYLREELVRYFIGWLMNTKRNKNNIA
HADGTFTSDYSKYLREELVRYFVEWLMNTKRNKNNIA
HADGTFTSDYSKYLREELVKLFIGWLMNTKRNKNNIA
HADGTFTSDYSKYLREELVKLFVEWLMNTKRNKNNIA
HADGTFTSDYSKYLREELVKYFIEWLMNTKRNKNNIA
HADGTFTSDYSKYLRIELVRLFIGWLMNTKRNKNNIA
HADGTFTSDYSKYLRIELVRLFVEWLMNTKRNKNNIA
HADGTFTSDYSKYLRIELVKLFIEWLMNTKRNKNNIA
HADGTFTSDYSKYLEEELVRLFVGWLMNTKRNKNNIA
HADGTFTSDYSKYLEEELVRYFIGWLMNTKRNKNNIA
HADGTFTSDYSKYLEEELVRYFVEWLMNTKRNKNNIA
HADGTFTSDYSKYLEIELVRLFIGWLMNTKRNKNNIA
HADGTFTSDYSKYLEIELVRLFVEWLMNTKRNKNNIA
HADGTFTSDYSKYLREELVRLFIGWLMNTKRNKNNIA
HADGTFTSDYSKYLREELVRLFVEWLMNTKRNKNNIA
HADGTFTSDYSKYLRIELVRLFIEWLMNTKRNKNNIA
HADGTFTSDYSKYLEEELVRLFIGWLMNTKRNKNNIA
HADGTFTSDYSKYLEEELVRLFVEWLMNTKRNKNNIA
HADGTFTSDYSKYLEEELVRYFIEWLMNTKRNKNNIA
HADGTFTSDYSKYLEEELVKLFIEWLMNTKRNKNNIA
HADGTFTSDYSKYLEIELVRLFIEWLMNTKRNKNNIA
HADGTFTSDYSKYLREELVRLFIEWLMNTKRNKNNIA
HADGTFTSDYSKYLEEELVRLFIEWLMNTKRNKNNIA
HAEGTFTSDYSKYLRIELVKYFVGWLKNGGPSKNNIA
HAEGTFTSDYSKYLEIELVKYFVGWLKNGGPSKNNIA
HAEGTFTSDYSKYLREELVKYFVGWLKNGGPSKNNIA
HAEGTFTSDYSKYLRIELVRYFVGWLKNGGPSKNNIA
HAEGTFTSDYSKYLRIELVKLFVGWLKNGGPSKNNIA
HAEGTFTSDYSKYLRIELVKYFIGWLKNGGPSKNNIA
HAEGTFTSDYSKYLRIELVKYFVEWLKNGGPSKNNIA
HAEGTFTSDYSKYLEEELVKYFVGWLKNGGPSKNNIA
HAEGTFTSDYSKYLEIELVRYFVGWLKNGGPSKNNIA
HAEGTFTSDYSKYLEIELVKLFVGWLKNGGPSKNNIA
HAEGTFTSDYSKYLEIELVKLFIGWLKNGGPSKNNIA
HAEGTFTSDYSKYLEIELVKYFVEWLKNGGPSKNNIA
HAEGTFTSDYSKYLREELVRYFVGWLKNGGPSKNNIA
HAEGTFTSDYSKYLREELVKLFVGWLKNGGPSKNNIA
HAEGTFTSDYSKYLREELVKYFIGWLKNGGPSKNNIA
HAEGTFTSDYSKYLREELVKYFVEWLKNGGPSKNNIA
HAEGTFTSDYSKYLRIELVRLFVGWLKNGGPSKNNIA
HAEGTFTSDYSKYLRIELVRYFIGWLKNGGPSKNNIA
HAEGTFTSDYSKYLRIELVRYFVEWLKNGGPSKNNIA
HAEGTFTSDYSKYLRIELVKLFIGWLKNGGPSKNNIA
HAEGTFTSDYSKYLRIELVKLFVEWLKNGGPSKNNIA
HAEGTFTSDYSKYLRIELVKYFIEWLKNGGPSKNNIA
HAEGTFTSDYSKYLEEELVRYFVGWLKNGGPSKNNIA
HAEGTFTSDYSKYLEEELVKLFVGWLKNGGPSKNNIA
HAEGTFTSDYSKYLEEELVKYFIGWLKNGGPSKNNIA
HAEGTFTSDYSKYLEEELVKYFVEWLKNGGPSKNNIA
HAEGTFTSDYSKYLEIELVRLFVGWLKNGGPSKNNIA
HAEGTFTSDYSKYLEIELVRYFIGWLKNGGPSKNNIA
HAEGTFTSDYSKYLEIELVRYFVEWLKNGGPSKNNIA
HAEGTFTSDYSKYLEIELVKLFIGWLKNGGPSKNNIA
HAEGTFTSDYSKYLEIELVKLFVEWLKNGGPSKNNIA
HAEGTFTSDYSKYLEIELVKYFIEWLKNGGPSKNNIA
HAEGTFTSDYSKYLREELVRLFVGWLKNGGPSKNNIA
HAEGTFTSDYSKYLREELVRYFIGWLKNGGPSKNNIA
HAEGTFTSDYSKYLREELVRYFVEWLKNGGPSKNNIA
HAEGTFTSDYSKYLREELVKLFIGWLKNGGPSKNNIA
HAEGTFTSDYSKYLREELVKLFVEWLKNGGPSKNNIA
HAEGTFTSDYSKYLREELVKYFIEWLKNGGPSKNNIA
HAEGTFTSDYSKYLRIELVRLFIGWLKNGGPSKNNIA
HAEGTFTSDYSKYLRIELVRLFVEWLKNGGPSKNNIA
HAEGTFTSDYSKYLRIELVKLFIEWLKNGGPSKNNIA
HAEGTFTSDYSKYLEEELVRLFVGWLKNGGPSKNNIA
HAEGTFTSDYSKYLEEELVRYFIGWLKNGGPSKNNIA
HAEGTFTSDYSKYLEEELVRYFVEWLKNGGPSKNNIA
HAEGTFTSDYSKYLEIELVRLFIGWLKNGGPSKNNIA
HAEGTFTSDYSKYLEIELVRLFVEWLKNGGPSKNNIA
HAEGTFTSDYSKYLREELVRLFIGWLKNGGPSKNNIA
HAEGTFTSDYSKYLREELVRLFVEWLKNGGPSKNNIA
HAEGTFTSDYSKYLRIELVRLFIEWLKNGGPSKNNIA
HAEGTFTSDYSKYLEEELVRLFIGWLKNGGPSKNNIA
HAEGTFTSDYSKYLEEELVRLFVEWLKNGGPSKNNIA
HAEGTFTSDYSKYLEEELVRYFIEWLKNGGPSKNNIA
HAEGTFTSDYSKYLEEELVKLFIEWLKNGGPSKNNIA
HAEGTFTSDYSKYLEIELVRLFIEWLKNGGPSKNNIA
HAEGTFTSDYSKYLREELVRLFIEWLKNGGPSKNNIA
HAEGTFTSDYSKYLEEELVRLFIEWLKNGGPSKNNIA
HAEGTFTSDYSKYLRIELVKYFVGWLMNTKRNKNNIA
HAEGTFTSDYSKYLEIELVKYFVGWLMNTKRNKNNIA
HAEGTFTSDYSKYLREELVKYFVGWLMNTKRNKNNIA
HAEGTFTSDYSKYLRIELVRYFVGWLMNTKRNKNNIA
HAEGTFTSDYSKYLRIELVKLFVGWLMNTKRNKNNIA
HAEGTFTSDYSKYLRIELVKYFIGWLMNTKRNKNNIA
HAEGTFTSDYSKYLRIELVKYFVEWLMNTKRNKNNIA
HAEGTFTSDYSKYLEEELVKYFVGWLMNTKRNKNNIA
HAEGTFTSDYSKYLEIELVRYFVGWLMNTKRNKNNIA
HAEGTFTSDYSKYLEIELVKLFVGWLMNTKRNKNNIA
HAEGTFTSDYSKYLEIELVKLFIGWLMNTKRNKNNIA
HAEGTFTSDYSKYLEIELVKYFVEWLMNTKRNKNNIA
HAEGTFTSDYSKYLREELVRYFVGWLMNTKRNKNNIA
HAEGTFTSDYSKYLREELVKLFVGWLMNTKRNKNNIA
HAEGTFTSDYSKYLREELVKYFIGWLMNTKRNKNNIA
HAEGTFTSDYSKYLREELVKYFVEWLMNTKRNKNNIA
HAEGTFTSDYSKYLRIELVRLFVGWLMNTKRNKNNIA
HAEGTFTSDYSKYLRIELVRYFIGWLMNTKRNKNNIA
HAEGTFTSDYSKYLRIELVRYFVEWLMNTKRNKNNIA
HAEGTFTSDYSKYLRIELVKLFIGWLMNTKRNKNNIA
HAEGTFTSDYSKYLRIELVKLFVEWLMNTKRNKNNIA
HAEGTFTSDYSKYLRIELVKYFIEWLMNTKRNKNNIA
HAEGTFTSDYSKYLEEELVRYFVGWLMNTKRNKNNIA
HAEGTFTSDYSKYLEEELVKLFVGWLMNTKRNKNNIA
HAEGTFTSDYSKYLEEELVKYFIGWLMNTKRNKNNIA
HAEGTFTSDYSKYLEEELVKYFVEWLMNTKRNKNNIA
HAEGTFTSDYSKYLEIELVRLFVGWLMNTKRNKNNIA
HAEGTFTSDYSKYLEIELVRYFIGWLMNTKRNKNNIA
HAEGTFTSDYSKYLEIELVRYFVEWLMNTKRNKNNIA
HAEGTFTSDYSKYLEIELVKLFIGWLMNTKRNKNNIA
HAEGTFTSDYSKYLEIELVKLFVEWLMNTKRNKNNIA
HAEGTFTSDYSKYLEIELVKYFIEWLMNTKRNKNNIA
HAEGTFTSDYSKYLREELVRLFVGWLMNTKRNKNNIA
HAEGTFTSDYSKYLREELVRYFIGWLMNTKRNKNNIA
HAEGTFTSDYSKYLREELVRYFVEWLMNTKRNKNNIA
HAEGTFTSDYSKYLREELVKLFIGWLMNTKRNKNNIA
HAEGTFTSDYSKYLREELVKLFVEWLMNTKRNKNNIA
HAEGTFTSDYSKYLREELVKYFIEWLMNTKRNKNNIA
HAEGTFTSDYSKYLRIELVRLFIGWLMNTKRNKNNIA
HAEGTFTSDYSKYLRIELVRLFVEWLMNTKRNKNNIA
HAEGTFTSDYSKYLRIELVKLFIEWLMNTKRNKNNIA
HAEGTFTSDYSKYLEEELVRLFVGWLMNTKRNKNNIA
HAEGTFTSDYSKYLEEELVRYFIGWLMNTKRNKNNIA
HAEGTFTSDYSKYLEEELVRYFVEWLMNTKRNKNNIA
HAEGTFTSDYSKYLEIELVRLFIGWLMNTKRNKNNIA
HAEGTFTSDYSKYLEIELVRLFVEWLMNTKRNKNNIA
HAEGTFTSDYSKYLREELVRLFIGWLMNTKRNKNNIA
HAEGTFTSDYSKYLREELVRLFVEWLMNTKRNKNNIA
HAEGTFTSDYSKYLRIELVRLFIEWLMNTKRNKNNIA
HAEGTFTSDYSKYLEEELVRLFIGWLMNTKRNKNNIA
HAEGTFTSDYSKYLEEELVRLFVEWLMNTKRNKNNIA
HAEGTFTSDYSKYLEEELVRYFIEWLMNTKRNKNNIA
HAEGTFTSDYSKYLEEELVKLFIEWLMNTKRNKNNIA
HAEGTFTSDYSKYLEIELVRLFIEWLMNTKRNKNNIA
HAEGTFTSDYSKYLREELVRLFIEWLMNTKRNKNNIA
HAEGTFTSDYSKYLEEELVRLFIEWLMNTKRNKNNIA
HADGTFTSDYSKYLRIELVKYFVQWLKNGGPSKNNIA
HADGTFTSDYSKYLEIELVKYFVQWLKNGGPSKNNIA
HADGTFTSDYSKYLREELVKYFVQWLKNGGPSKNNIA
HADGTFTSDYSKYLRIELVRYFVQWLKNGGPSKNNIA
HADGTFTSDYSKYLRIELVKLFVQWLKNGGPSKNNIA
HADGTFTSDYSKYLRIELVKYFIQWLKNGGPSKNNIA
HADGTFTSDYSKYLRIELVKYFVQWLKNGGPSKNNIA
HADGTFTSDYSKYLEEELVKYFVQWLKNGGPSKNNIA
HADGTFTSDYSKYLEIELVRYFVQWLKNGGPSKNNIA
HADGTFTSDYSKYLEIELVKLFVQWLKNGGPSKNNIA
HADGTFTSDYSKYLEIELVKLFIQWLKNGGPSKNNIA
HADGTFTSDYSKYLEIELVKYFVQWLKNGGPSKNNIA
HADGTFTSDYSKYLREELVRYFVQWLKNGGPSKNNIA
HADGTFTSDYSKYLREELVKLFVQWLKNGGPSKNNIA
HADGTFTSDYSKYLREELVKYFIQWLKNGGPSKNNIA
HADGTFTSDYSKYLREELVKYFVQWLKNGGPSKNNIA
HADGTFTSDYSKYLRIELVRLFVQWLKNGGPSKNNIA
HADGTFTSDYSKYLRIELVRYFIQWLKNGGPSKNNIA
HADGTFTSDYSKYLRIELVRYFVQWLKNGGPSKNNIA
HADGTFTSDYSKYLRIELVKLFIQWLKNGGPSKNNIA
HADGTFTSDYSKYLRIELVKLFVQWLKNGGPSKNNIA
HADGTFTSDYSKYLRIELVKYFIQWLKNGGPSKNNIA
HADGTFTSDYSKYLEEELVRYFVQWLKNGGPSKNNIA
HADGTFTSDYSKYLEEELVKLFVQWLKNGGPSKNNIA
HADGTFTSDYSKYLEEELVKYFIQWLKNGGPSKNNIA
HADGTFTSDYSKYLEEELVKYFVQWLKNGGPSKNNIA
HADGTFTSDYSKYLEIELVRLFVQWLKNGGPSKNNIA
HADGTFTSDYSKYLEIELVRYFIQWLKNGGPSKNNIA
HADGTFTSDYSKYLEIELVRYFVQWLKNGGPSKNNIA
HADGTFTSDYSKYLEIELVKLFIQWLKNGGPSKNNIA
HADGTFTSDYSKYLEIELVKLFVQWLKNGGPSKNNIA
HADGTFTSDYSKYLEIELVKYFIQWLKNGGPSKNNIA
HADGTFTSDYSKYLREELVRLFVQWLKNGGPSKNNIA
HADGTFTSDYSKYLREELVRYFIQWLKNGGPSKNNIA
HADGTFTSDYSKYLREELVRYFVQWLKNGGPSKNNIA
HADGTFTSDYSKYLREELVKLFIQWLKNGGPSKNNIA
HADGTFTSDYSKYLREELVKLFVQWLKNGGPSKNNIA
HADGTFTSDYSKYLREELVKYFIQWLKNGGPSKNNIA
HADGTFTSDYSKYLRIELVRLFIQWLKNGGPSKNNIA
HADGTFTSDYSKYLRIELVRLFVQWLKNGGPSKNNIA
HADGTFTSDYSKYLRIELVKLFIQWLKNGGPSKNNIA
HADGTFTSDYSKYLEEELVRLFVQWLKNGGPSKNNIA
HADGTFTSDYSKYLEEELVRYFIQWLKNGGPSKNNIA
HADGTFTSDYSKYLEEELVRYFVQWLKNGGPSKNNIA
HADGTFTSDYSKYLEIELVRLFIQWLKNGGPSKNNIA
HADGTFTSDYSKYLEIELVRLFVQWLKNGGPSKNNIA
HADGTFTSDYSKYLREELVRLFIQWLKNGGPSKNNIA
HADGTFTSDYSKYLREELVRLFVQWLKNGGPSKNNIA
HADGTFTSDYSKYLRIELVRLFIQWLKNGGPSKNNIA
HADGTFTSDYSKYLEEELVRLFIQWLKNGGPSKNNIA
HADGTFTSDYSKYLEEELVRLFVQWLKNGGPSKNNIA
HADGTFTSDYSKYLEEELVRYFIQWLKNGGPSKNNIA
HADGTFTSDYSKYLEEELVKLFIQWLKNGGPSKNNIA
HADGTFTSDYSKYLEIELVRLFIQWLKNGGPSKNNIA
HADGTFTSDYSKYLREELVRLFIQWLKNGGPSKNNIA
HADGTFTSDYSKYLEEELVRLFIQWLKNGGPSKNNIA
HADGTFTSDYSKYLRIELVKYFVQWLMNTKRNKNNIA
HADGTFTSDYSKYLEIELVKYFVQWLMNTKRNKNNIA
HADGTFTSDYSKYLREELVKYFVQWLMNTKRNKNNIA
HADGTFTSDYSKYLRIELVRYFVQWLMNTKRNKNNIA
HADGTFTSDYSKYLRIELVKLFVQWLMNTKRNKNNIA
HADGTFTSDYSKYLRIELVKYFIQWLMNTKRNKNNIA
HADGTFTSDYSKYLRIELVKYFVQWLMNTKRNKNNIA
HADGTFTSDYSKYLEEELVKYFVQWLMNTKRNKNNIA
HADGTFTSDYSKYLEIELVRYFVQWLMNTKRNKNNIA
HADGTFTSDYSKYLEIELVKLFVQWLMNTKRNKNNIA
HADGTFTSDYSKYLEIELVKLFIQWLMNTKRNKNNIA
HADGTFTSDYSKYLEIELVKYFVQWLMNTKRNKNNIA
HADGTFTSDYSKYLREELVRYFVQWLMNTKRNKNNIA
HADGTFTSDYSKYLREELVKLFVQWLMNTKRNKNNIA
HADGTFTSDYSKYLREELVKYFIQWLMNTKRNKNNIA
HADGTFTSDYSKYLREELVKYFVQWLMNTKRNKNNIA
HADGTFTSDYSKYLRIELVRLFVQWLMNTKRNKNNIA
HADGTFTSDYSKYLRIELVRYFIQWLMNTKRNKNNIA
HADGTFTSDYSKYLRIELVRYFVQWLMNTKRNKNNIA
HADGTFTSDYSKYLRIELVKLFIQWLMNTKRNKNNIA
HADGTFTSDYSKYLRIELVKLFVQWLMNTKRNKNNIA
HADGTFTSDYSKYLRIELVKYFIQWLMNTKRNKNNIA
HADGTFTSDYSKYLEEELVRYFVQWLMNTKRNKNNIA
HADGTFTSDYSKYLEEELVKLFVQWLMNTKRNKNNIA
HADGTFTSDYSKYLEEELVKYFIQWLMNTKRNKNNIA
HADGTFTSDYSKYLEEELVKYFVQWLMNTKRNKNNIA
HADGTFTSDYSKYLEIELVRLFVQWLMNTKRNKNNIA
HADGTFTSDYSKYLEIELVRYFIQWLMNTKRNKNNIA
HADGTFTSDYSKYLEIELVRYFVQWLMNTKRNKNNIA
HADGTFTSDYSKYLEIELVKLFIQWLMNTKRNKNNIA
HADGTFTSDYSKYLEIELVKLFVQWLMNTKRNKNNIA
HADGTFTSDYSKYLEIELVKYFIQWLMNTKRNKNNIA
HADGTFTSDYSKYLREELVRLFVQWLMNTKRNKNNIA
HADGTFTSDYSKYLREELVRYFIQWLMNTKRNKNNIA
HADGTFTSDYSKYLREELVRYFVQWLMNTKRNKNNIA
HADGTFTSDYSKYLREELVKLFIQWLMNTKRNKNNIA
HADGTFTSDYSKYLREELVKLFVQWLMNTKRNKNNIA
HADGTFTSDYSKYLREELVKYFIQWLMNTKRNKNNIA
HADGTFTSDYSKYLRIELVRLFIQWLMNTKRNKNNIA
HADGTFTSDYSKYLRIELVRLFVQWLMNTKRNKNNIA
HADGTFTSDYSKYLRIELVKLFIQWLMNTKRNKNNIA
HADGTFTSDYSKYLEEELVRLFVQWLMNTKRNKNNIA
HADGTFTSDYSKYLEEELVRYFIQWLMNTKRNKNNIA
HADGTFTSDYSKYLEEELVRYFVQWLMNTKRNKNNIA
HADGTFTSDYSKYLEIELVRLFIQWLMNTKRNKNNIA
HADGTFTSDYSKYLEIELVRLFVQWLMNTKRNKNNIA
HADGTFTSDYSKYLREELVRLFIQWLMNTKRNKNNIA
HADGTFTSDYSKYLREELVRLFVQWLMNTKRNKNNIA
HADGTFTSDYSKYLRIELVRLFIQWLMNTKRNKNNIA
HADGTFTSDYSKYLEEELVRLFIQWLMNTKRNKNNIA
HADGTFTSDYSKYLEEELVRLFVQWLMNTKRNKNNIA
HADGTFTSDYSKYLEEELVRYFIQWLMNTKRNKNNIA
HADGTFTSDYSKYLEEELVKLFIQWLMNTKRNKNNIA
HADGTFTSDYSKYLEIELVRLFIQWLMNTKRNKNNIA
HADGTFTSDYSKYLREELVRLFIQWLMNTKRNKNNIA
HADGTFTSDYSKYLEEELVRLFIQWLMNTKRNKNNIA
HAEGTFTSDYSKYLRIELVKYFVQWLKNGGPSKNNIA
HAEGTFTSDYSKYLEIELVKYFVQWLKNGGPSKNNIA
HAEGTFTSDYSKYLREELVKYFVQWLKNGGPSKNNIA
HAEGTFTSDYSKYLRIELVRYFVQWLKNGGPSKNNIA
HAEGTFTSDYSKYLRIELVKLFVQWLKNGGPSKNNIA
HAEGTFTSDYSKYLRIELVKYFIQWLKNGGPSKNNIA
HAEGTFTSDYSKYLRIELVKYFVQWLKNGGPSKNNIA
HAEGTFTSDYSKYLEEELVKYFVQWLKNGGPSKNNIA
HAEGTFTSDYSKYLEIELVRYFVQWLKNGGPSKNNIA
HAEGTFTSDYSKYLEIELVKLFVQWLKNGGPSKNNIA
HAEGTFTSDYSKYLEIELVKLFIQWLKNGGPSKNNIA
HAEGTFTSDYSKYLEIELVKYFVQWLKNGGPSKNNIA
HAEGTFTSDYSKYLREELVRYFVQWLKNGGPSKNNIA
HAEGTFTSDYSKYLREELVKLFVQWLKNGGPSKNNIA
HAEGTFTSDYSKYLREELVKYFIQWLKNGGPSKNNIA
HAEGTFTSDYSKYLREELVKYFVQWLKNGGPSKNNIA
HAEGTFTSDYSKYLRIELVRLFVQWLKNGGPSKNNIA
HAEGTFTSDYSKYLRIELVRYFIQWLKNGGPSKNNIA
HAEGTFTSDYSKYLRIELVRYFVQWLKNGGPSKNNIA
HAEGTFTSDYSKYLRIELVKLFIQWLKNGGPSKNNIA
HAEGTFTSDYSKYLRIELVKLFVQWLKNGGPSKNNIA
HAEGTFTSDYSKYLRIELVKLFVQWLKNGGPSKNNIA
HAEGTFTSDYSKYLRIELVKYFIQWLKNGGPSKNNIA
HAEGTFTSDYSKYLEEELVRYFVQWLKNGGPSKNNIA
HAEGTFTSDYSKYLEEELVKLFVQWLKNGGPSKNNIA
HAEGTFTSDYSKYLEEELVKYFIQWLKNGGPSKNNIA
HAEGTFTSDYSKYLEEELVKYFVQWLKNGGPSKNNIA
HAEGTFTSDYSKYLEIELVRLFVQWLKNGGPSKNNIA
HAEGTFTSDYSKYLEIELVRYFIQWLKNGGPSKNNIA
HAEGTFTSDYSKYLEIELVRYFVQWLKNGGPSKNNIA
HAEGTFTSDYSKYLEIELVKLFIQWLKNGGPSKNNIA
HAEGTFTSDYSKYLEIELVKLFVQWLKNGGPSKNNIA
HAEGTFTSDYSKYLEIELVKYFIQWLKNGGPSKNNIA
HAEGTFTSDYSKYLREELVRLFVQWLKNGGPSKNNIA
HAEGTFTSDYSKYLREELVRYFIQWLKNGGPSKNNIA
HAEGTFTSDYSKYLREELVRYFVQWLKNGGPSKNNIA
HAEGTFTSDYSKYLREELVKLFIQWLKNGGPSKNNIA
HAEGTFTSDYSKYLREELVKLFVQWLKNGGPSKNNIA
HAEGTFTSDYSKYLREELVKYFIQWLKNGGPSKNNIA
HAEGTFTSDYSKYLRIELVRLFIQWLKNGGPSKNNIA
HAEGTFTSDYSKYLRIELVRLFVQWLKNGGPSKNNIA
HAEGTFTSDYSKYLRIELVKLFIQWLKNGGPSKNNIA
HAEGTFTSDYSKYLEEELVRLFVQWLKNGGPSKNNIA
HAEGTFTSDYSKYLEEELVRYFIQWLKNGGPSKNNIA
HAEGTFTSDYSKYLEEELVRYFVQWLKNGGPSKNNIA
HAEGTFTSDYSKYLEIELVRLFIQWLKNGGPSKNNIA
HAEGTFTSDYSKYLEIELVRLFVQWLKNGGPSKNNIA
HAEGTFTSDYSKYLREELVRLFIQWLKNGGPSKNNIA
HAEGTFTSDYSKYLREELVRLFVQWLKNGGPSKNNIA
HAEGTFTSDYSKYLRIELVRLFIQWLKNGGPSKNNIA
HAEGTFTSDYSKYLEEELVRLFIQWLKNGGPSKNNIA
HAEGTFTSDYSKYLEEELVRLFVQWLKNGGPSKNNIA
HAEGTFTSDYSKYLEEELVRYFIQWLKNGGPSKNNIA
HAEGTFTSDYSKYLEEELVKLFIQWLKNGGPSKNNIA
HAEGTFTSDYSKYLEIELVRLFIQWLKNGGPSKNNIA
HAEGTFTSDYSKYLREELVRLFIQWLKNGGPSKNNIA
HAEGTFTSDYSKYLEEELVRLFIQWLKNGGPSKNNIA
HAEGTFTSDYSKYLRIELVKYFVQWLMNTKRNKNNIA
HAEGTFTSDYSKYLEIELVKYFVQWLMNTKRNKNNIA
HAEGTFTSDYSKYLREELVKYFVQWLMNTKRNKNNIA
HAEGTFTSDYSKYLRIELVRYFVQWLMNTKRNKNNIA
HAEGTFTSDYSKYLRIELVKLFVQWLMNTKRNKNNIA
HAEGTFTSDYSKYLRIELVKYFIQWLMNTKRNKNNIA
HAEGTFTSDYSKYLRIELVKYFVQWLMNTKRNKNNIA
HAEGTFTSDYSKYLEEELVKYFVQWLMNTKRNKNNIA
HAEGTFTSDYSKYLEIELVRYFVQWLMNTKRNKNNIA
HAEGTFTSDYSKYLEIELVKLFVQWLMNTKRNKNNIA
HAEGTFTSDYSKYLEIELVKLFIQWLMNTKRNKNNIA
HAEGTFTSDYSKYLEIELVKYFVQWLMNTKRNKNNIA
HAEGTFTSDYSKYLREELVRYFVQWLMNTKRNKNNIA
HAEGTFTSDYSKYLREELVKLFVQWLMNTKRNKNNIA
HAEGTFTSDYSKYLREELVKYFIQWLMNTKRNKNNIA
HAEGTFTSDYSKYLREELVKYFVQWLMNTKRNKNNIA
HAEGTFTSDYSKYLRIELVRLFVQWLMNTKRNKNNIA
HAEGTFTSDYSKYLRIELVRYFIQWLMNTKRNKNNIA
HAEGTFTSDYSKYLRIELVRYFVQWLMNTKRNKNNIA
HAEGTFTSDYSKYLRIELVKLFIQWLMNTKRNKNNIA
HAEGTFTSDYSKYLRIELVKLFVQWLMNTKRNKNNIA
HAEGTFTSDYSKYLRIELVKYFIQWLMNTKRNKNNIA
HAEGTFTSDYSKYLEEELVRYFVQWLMNTKRNKNNIA
HAEGTFTSDYSKYLEEELVKLFVQWLMNTKRNKNNIA
HAEGTFTSDYSKYLEEELVKYFIQWLMNTKRNKNNIA
HAEGTFTSDYSKYLEEELVKYFVQWLMNTKRNKNNIA
HAEGTFTSDYSKYLEIELVRLFVQWLMNTKRNKNNIA
HAEGTFTSDYSKYLEIELVRYFIQWLMNTKRNKNNIA
HAEGTFTSDYSKYLEIELVRYFVQWLMNTKRNKNNIA
HAEGTFTSDYSKYLEIELVKLFIQWLMNTKRNKNNIA
HAEGTFTSDYSKYLEIELVKLFVQWLMNTKRNKNNIA
HAEGTFTSDYSKYLEIELVKYFIQWLMNTKRNKNNIA
HAEGTFTSDYSKYLREELVRLFVQWLMNTKRNKNNIA
HAEGTFTSDYSKYLREELVRYFIQWLMNTKRNKNNIA
HAEGTFTSDYSKYLREELVRYFVQWLMNTKRNKNNIA
HAEGTFTSDYSKYLREELVKLFVQWLMNTKRNKNNIA
HAEGTFTSDYSKYLREELVKYFIQWLMNTKRNKNNIA
HAEGTFTSDYSKYLRIELVRLFIQWLMNTKRNKNNIA
HAEGTFTSDYSKYLRIELVRLFVQWLMNTKRNKNNIA
HAEGTFTSDYSKYLRIELVKLFIQWLMNTKRNKNNIA
HAEGTFTSDYSKYLEEELVRLFVQWLMNTKRNKNNIA
HAEGTFTSDYSKYLEEELVRYFIQWLMNTKRNKNNIA
HAEGTFTSDYSKYLEEELVRYFVQWLMNTKRNKNNIA
HAEGTFTSDYSKYLEIELVRLFIQWLMNTKRNKNNIA
HAEGTFTSDYSKYLEIELVRLFVQWLMNTKRNKNNIA
HAEGTFTSDYSKYLREELVRLFIQWLMNTKRNKNNIA
HAEGTFTSDYSKYLREELVRLFVQWLMNTKRNKNNIA
HAEGTFTSDYSKYLRIELVRLFIQWLMNTKRNKNNIA
HAEGTFTSDYSKYLEEELVRLFIQWLMNTKRNKNNIA
HAEGTFTSDYSKYLEEELVRLFVQWLMNTKRNKNNIA
HAEGTFTSDYSKYLEEELVRYFIQWLMNTKRNKNNIA
HAEGTFTSDYSKYLEEELVKLFIQWLMNTKRNKNNIA
HAEGTFTSDYSKYLEIELVRLFIQWLMNTKRNKNNIA
HAEGTFTSDYSKYLREELVRLFIQWLMNTKRNKNNIA
HAEGTFTSDYSKYLEEELVRLFIQWLMNTKRNKNNIA

In view of the foregoing, it will be appreciated that the invention described herein *inter alia* relates to the following items:
1. A compound having the general Formula (I):

Z-X-S1 (I)

in which:
X is oxm 4-14; and
Z is an amino acid sequence of three amino acid residues;
wherein S1 is an amino acid sequence corresponding to line A (SEQ ID NO:1) or an amino acid sequence corresponding to line A in which m amino acids of said line A are substituted by m corresponding amino acids from residues 15 to 37 in line B (SEQ ID NO: 2), and t further amino acid residues of line A are substituted by t corresponding amino acid residues from residues 15 to 24 of line R, in which line A, line B and line R (SEQ ID NO: 145) are as follows:

| | | | | | | | | | | |
|---|---|---|---|---|---|---|---|---|---|---|
| Line A | Asp | Ser | Arg | Arg | Ala | Gln | Asp | Phe | Val | Gln |
| | 15 | 16 | 17 | 18 | 19 | 20 | 21 | 22 | 23 | 24 |
| Line B | Glu | Glu | Glu | Ala | Val | Arg | Leu | Phe | Ile | Glu |
| Line R | Arg | Ile | Glu | Ile | Val | Lys | Tyr | Phe | Ile | Gly |
| | | | | | | | | | | |
| Line A | Trp | Leu | Met | Asn | Thr | Lys | Arg | Asn | Arg | Asn |
| | 25 | 26 | 27 | 28 | 29 | 30 | 31 | 32 | 33 | 34 |
| Line B | Trp | Leu | Lys | Asn | Gly | Gly | Pro | Ser | Ser | Gly |
| | | | | | | | | | | |
| Line A | Asn | Ile | Ala | | | | | | | |
| | 35 | 36 | 37 | | | | | | | |
| Line B | Ala | Pro | Pro | | | | | | | |

the compound optionally further including an extension moiety attached to the amino acid at position 37, the optional extension moiety comprising one or more amino acids,
m being an integer not less than 1;
t being 0, 1, 2, 3, 4, 5, 6, 7, 8, 9 or 10
a variant or derivative thereof;
or a salt or solvate thereof
with the proviso that, if S1 is identical to line A, Z is not His Ser Gln.
2. A compound according to item 1, in which t=0.
3. A compound according to item 1, in which t=5 or more.
4. A compound according to item 1, 2 or 3, in which Z is an amino acid sequence of two amino acid residues followed by Gln, Asp or Glu.
5. A compound according to any preceding item, in which the amino acid replacements from line B comprises at least one sequential group of at least three amino acids.
6. A compound according to any preceeding item, in which the replaced amino acids comprises a sequential group of not more than twelve amino acids.
7. A compound according to any one of items 1 to 6, having a Formula selected from the following group: 
His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Ser Ser Gly Ala Pro Pro Pro Ser (SEQ ID NO: 26).
 
His Ser Gln Gly Thr Phe Thr Ser Asp Tyr Ser Lys Tyr Leu Glu Glu Glu Ala Val Arg Leu Phe Ile
Glu Trp Leu Lys Asn Gly Gly Pro Ser Ser Gly Ala Pro Pro Pro Ser; and
 
D-His Ala Asp Gly Thr Phe Thr Ser Asp Tyr Ser Lys Tyr Ile Arg Glu Glu Ile Val Arg Leu Phe
Val Gln Trp Leu Met Asn Thr Lys Arg Asn Lys Asn Asn Ile Ala
 
D-His Ser Gln Gly Thr Phe Thr Ser Asp Tyr Ser Lys Tyr Leu Glu Glu Glu Ala Val Arg Leu Phe
Ile Glu Trp Leu Lys Asn Gly Gly Pro Ser Ser Gly Ala Pro Pro Pro Ser.
 8. A compound according to item 1, in which the compound is of general Formula (II):

Z - X - S2 - Y (II)

in which
X and Z are as defined in item 1 or item 4;
Y is oxm25-37; and
wherein S2 is an amino acid sequence corresponding to line C (SEQ ID NO: 3) in which n amino acids of said line C are substituted by n corresponding amino acids from line D (SEQ ID NO: 4) and u further amino acids of line C are substituated by u corresponding amino acids from line S (SEQ ID NO: 145), in which line C, line D and Line S are as follows:

| | | | | | | | | | | |
|---|---|---|---|---|---|---|---|---|---|---|
| Line C | Asp | Ser | Arg | Arg | Ala | Gln | Asp | Phe | Val | Gln |
| | 15 | 16 | 17 | 18 | 19 | 20 | 21 | 22 | 23 | 24 |
| Line D | Glu | Glu | Glu | Ala | Val | Arg | Leu | Phe | Ile | Glu |
| Line S | Arg | Ile | Glu | Ile | Val | Lys | Tyr | Phe | Ile | Gly |

and n is an integer not less than 1;
and u is 0, 1, 2, 3, 4, 5, 6, 7, 8 or 9;
a variant or derivative thereof;
or a salt or solvate thereof.
9. A compound according to item 8 in which u=0.
10. A compound according to item 8 in which u=5 or more.
11. A compound according to item 8, 9 or 10, in which the amino acid substitution from line D comprises at least three amino acids.
12. A compound according to item 8, 9 or 10, in which the amino acid substitution from line D comprises at least one sequential group of two or more amino acids.
13. A compound according to item 8, 9 or 10, in which the amino acid substitution from line D comprises a sequential group of at least four amino acids.
14. A compound according to item 8, 9 or 10, in which the amino acid substitution from line D comprises a sequential group of not more than ten amino acids.
15. A compound according to item 13 or item 14, in which the sequential group has from 5 to 10 amino acids.
16. A compound according to any one of items 13 to 15, in which the sequential group has from 6 to 10 amino acids.
17. A compound according to any one of items 13 to 16, in which the sequential group has from 7 to 10 amino acids.
18. A compound according to item 8, 9 or 10 in which the amino acid substitution from line S comprises at least three amino acids.
19. A compound according to item 8, 9 or 10, in which the amino acid substitution from line S comprises at least one sequential group of two or more amino acids.
20. A compound according to item 8, 9 or 10, in which the amino acid substitution from line S comprises a sequential group of at least four amino acids.
21. A compound according to item 8, 9 or 10, in which the amino acid substitution from line S comprises a sequential group of not more than ten amino acids.
22. A compound according to item 20 or item 21, in which the sequential group has from 5 to 10 amino acids.
23. A compound according to any one of items 20 to 22, in which the sequential group has from 6 to 10 amino acids.
24. A compound according to any one of items 20 to 23, in which the sequential group has from 7 to 10 amino acids.
25. A compound according to item 8, which is a compound of sequence: 
His Ser Gln Gly Thr Phe Thr Ser Asp Tyr Ser Lys Tyr Leu Glu Glu Glu Ala Ala Gln Asp Phe Val
Gln Trp Leu Met Asn Thr Lys Arg Asn Arg Asn Asn Ile Ala (SEQ ID NO: 14),
 
His Ser Gln Gly Thr Phe Thr Ser Asp Tyr Ser Lys Tyr Leu Glu Glu Glu Ala Val Arg Leu Phe Val
Gln Trp Leu Met Asn Thr Lys Arg Asn Arg Asn Asn Ile Ala (SEQ ID NO: 15),
 
His Ser Gln Gly Thr Phe Thr Ser Asp Tyr Ser Lys Tyr Leu Glu Glu Glu Ala Val Arg Leu Phe Ile
Glu Trp Leu Met Asn Thr Lys Arg Asn Arg Asn Asn Ile Ala (SEQ ID NO: 16).
 
His Ser Gln Gly Thr Phe Thr Ser Asp Tyr Ser Lys Tyr Leu Asp Glu Glu Ala Val Arg Leu Phe
Ile Glu Trp Leu Met Asn Thr Lys Arg Asn Arg Asn Asn Ile Ala,
 
His Ser Gln Gly Thr Phe Thr Ser Asp Tyr Ser Lys Tyr Leu Glu Glu Glu Ala Val Arg Leu Phe Ile
Gln Trp Leu Met Asn Thr Lys Arg Asn Arg Asn Asn Ile Ala,
 
D-His Ala Gln Gly Thr Phe Thr Ser Asp Tyr Ser Lys Tyr Leu Glu Glu Glu Ala Val Arg Leu Phe
Val Gln Trp Leu Met Asn Thr Lys Arg Asn Arg Asn Asn Ile Ala,
 
His Ser Gln Gly Thr Phe Thr Ser Asp Tyr Ser Lys Tyr Leu Glu Glu Glu Ala Ala Arg Leu Phe Ile
Glu Trp Leu Met Asn Thr Lys Arg Asn Arg Asn Asn Ile Ala,
 
D-His Ala Asp Gly Thr Phe Thr Ser Asp Tyr Ser Lys Tyr Ile Arg Glu Glu Leu Val Lys Tyr Phe
Val Gly Trp Leu Met Asn Thr Lys Arg Asn Arg Asn Asn Ile Ala
 
His Ser Gln Gly Thr Phe Thr Ser Asp Tyr Ser Lys Tyr Leu Glu Glu Glu Ala Val Arg Leu Phe Val
Glu Trp Leu Met Asn Thr Lys Arg Asn Arg Asn Asn Ile Ala,
 
His Ser Gln Gly Thr Phe Thr Ser Asp Tyr Ser Lys Tyr Leu Glu Glu Glu Ala Val Arg Leu Phe Ile
Glu Trp Leu Met Asn Thr Lys Arg Asn Arg Asn Asn Ile Ala,
 
D-His Ala Asp Gly Thr Phe Thr Ser Asp Tyr Ser Lys Tyr Ile Arg Ile Glu Leu Val Lys Leu Phe
Val Gly Trp Leu Met Asn Thr Lys Arg Asn Lys Asn Asn Ile Ala
 His Ser Gln Gly Thr Phe Thr Ser Asp Tyr Ser Lys Tyr Leu Glu Glu Glu Ala Val Arg Leu Phe Val Gln Trp Leu Met Asn Thr Lys Arg Asn Lys Asn Asn Ile Ala Ala, 
His Ser Gln Gly Thr Phe Thr Ser Asp Tyr Ser Lys Tyr Leu Glu Glu Glu Ala Val Lys Leu Phe Ile
Glu Trp Leu Met Asn Thr Lys Arg Asn Arg Asn Asn Ile Ala,
 
His Ser Gln Gly Thr Phe Thr Ser Asp Tyr Ser Lys Tyr Leu Glu Glu Asp Ala Val Arg Leu Phe Ile
Glu Trp Leu Met Asn Thr Lys Arg Asn Arg Asn Asn Ile Ala,
 
His Ser Gln Gly Thr Phe Thr Ser Asp Tyr Ser Lys Tyr Leu Glu Glu Glu Ala Val Arg Leu Phe Ile
Asp Trp Leu Met Asn Thr Lys Arg Asn Arg Asn Asn Ile Ala,
 
His Ser Gln Gly Thr Phe Thr Ser Asp Tyr Ser Lys Tyr Leu Glu Glu Glu Gln Val Arg Leu Phe Ile
Glu Trp Leu Met Asn Thr Lys Arg Asn Arg Asn Asn Ile Ala,
 
His Ser Gln Gly Thr Phe Thr Ser Asp Tyr Ser Lys Tyr Leu Glu Glu Glu Ala Ile Arg Leu Phe Ile
Glu Trp Leu Met Asn Thr Lys Arg Asn Arg Asn Asn Ile Ala,
 
His Ser Gln Gly Thr Phe Thr Ser Asp Tyr Ser Lys Tyr Leu Glu Glu Glu Ala Val Arg Leu Phe
Lys Gln Trp Leu Met Asn Thr Lys Arg Asn Arg Asn Asn Ile Ala,
 
His Ser Gln Gly Thr Phe Thr Ser Asp Tyr Ser Lys Tyr Leu Gln Glu Glu Ala Val Arg Leu Phe Val
Gln Trp Leu Met Asn Thr Lys Arg Asn Arg Asn Asn Ile Ala,
 
His Ser Gln Gly Thr Phe Thr Ser Asp Tyr Ser Lys Tyr Leu Glu Gln Glu Ala Val Arg Leu Phe Val
Gln Trp Leu Met Asn Thr Lys Arg Asn Arg Asn Asn Ile Ala,
 
His Ser Gln Gly Thr Phe Thr Ser Asp Tyr Ser Lys Tyr Leu Glu Glu Glu Val Val Arg Leu Phe Val
Gln Trp Leu Met Asn Thr Lys Arg Asn Arg Asn Asn Ile Ala,
 
His Ser Gln Gly Thr Phe Thr Ser Asp Tyr Ser Lys Tyr Leu Glu Glu Glu Val Ile Arg Leu Phe Val
Gln Trp Leu Met Asn Thr Lys Arg Asn Arg Asn Asn Ile Ala,
 
His Ser Gln Gly Thr Phe Thr Ser Asp Tyr Ser Lys Tyr Leu Glu Glu Glu Val Ile Arg Leu Phe Val
Gln Trp Leu Met Asn Thr Lys Arg Asn Arg Asn Asn Ile Ala,
 
D-His Ala Gln Gly Thr Phe Thr Ser Asp Tyr Ser Lys Tyr Leu Glu Glu Glu Val Ile Arg Ile Phe
Val Gln Trp Leu Met Asn Thr Lys Arg Asn Arg Asn Asn Ile Ala,
 
D-His Ala Gln Gly Thr Phe Thr Ser Asp Tyr Ser Lys Tyr Leu Glu Glu Glu Val Val Arg Leu Phe
Val Gln Trp Leu Met Asn Thr Lys Arg Asn Arg Asn Asn Ile Ala,
 
D-His Ala Gln Gly Thr Phe Thr Ser Asp Tyr Ser Lys Tyr Leu Glu Glu Glu Ala Val Arg Leu Phe
Ile Gln Trp Leu Met Asn Thr Lys Arg Asn Lys Asn Asn Ile Ala, or
 
D-His Ala Gln Gly Thr Phe Thr Ser Asp Tyr Ser Lys Tyr Leu Glu Glu Glu Ala Val Arg Leu Phe
Val Gln Trp Leu Met Asn Thr Lys Arg Asn Lys Asn Asn Ile Ala Ala Ala.
 26. A compound according to item 1, in which the compound is of general Formula (III):

Z-X'-S3-Y' (III)

in which
X' is oxm4-26;
Z is as defined in item 1 or item 4;
Y' is oxm34-37; and
wherein S3 is an amino acid sequence corresponding to line E (SEQ ID NO: 5) in which p amino acids of said line E are substituted by p corresponding amino acids from line F (SEQ ID NO: 6), in which line E and line F are as follows:

| | | | | | | | |
|---|---|---|---|---|---|---|---|
| Line E | Met | Asn | Thr | Lys | Arg | Asn | Arg |
| | 27 | 28 | 29 | 30 | 31 | 32 | 33 |
| Line F | Lys | Asn | Gly | Gly | Pro | Ser | Ser |

and p is an integer not less than 1;
a variant or derivative thereof;
or a salt or solvate thereof.
27. A compound according to item 26, in which the substituted amino acids comprise at least three amino acids.
28. A compound according to item 26 or item 27, in which the substituted amino acids comprise at least one sequential group of two or more amino acids.
29. A compound according to item 28, in which the substituted amino acids comprise a sequential group of at least three amino acids.
30. A compound according to any one of items 26 to 29, in which the substituted amino acids comprise a sequential group of not more than seven amino acids.
31. A compound according to item 30, in which the sequential group has from 3 to 7 amino acids.
32. A compound according to item 31, in which the sequential group has four amino acids.
33. A compound according to any one of items 28 to 30, in which the sequential group has from 4 to 7 amino acids.
34. A compound according to item 26 which is a compound of sequence: 
His Ser Gln Gly Thr Phe Thr Ser Asp Tyr Ser Lys Tyr Leu Asp Ser Arg Arg Ala Gln Asp Phe
Val Gln Trp Leu Met Asn Thr Gly Pro Ser Ser Asn Asn Ile Ala (SEQ ID NO: 17),
 
His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Arg Asn Arg Asn Asn Ile Ala (SEQ ID NO: 18),
 
His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Ser Ser Asn Asn Ile Ala (SEQ ID NO: 19),
 
His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Asn Ser Asn Asn Ile Ala,
 
His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Arg Asn Arg Asn Asn Ile Ala,
 
His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Asn Arg Asn Asn Ile Ala,
 
His Ser Gln Gly Thr Phe Thr Ser Asp Tyr Ser Lys Tyr Leu Asp Ser Arg Arg Ala Gln Asp Phe
Val Gln Trp Leu Met Asn Gly Gly Pro Ser Arg Asn Asn Ile Ala,
 
His Ser Gln Gly Thr Phe Thr Ser Asp Tyr Ser Lys Tyr Leu Asp Ser Arg Arg Ala Gln Asp Phe
Val Gln Trp Leu Met Asn Gly Gly Pro Ser Ser Asn Asn Ile Ala,
 
His Ser Gln Gly Thr Phe Thr Ser Asp Tyr Ser Lys Tyr Leu Asp Ser Arg Arg Ala Gln Asp Phe
Val Gln Trp Leu Arg Asn Gly Gly Pro Ser Ser Asn Asn Ile Ala, or
 
His Ser Gln Gly Thr Phe Thr Ser Asp Tyr Ser Lys Tyr Leu Asp Ser Arg Arg Ala Gln Asp Phe
Val Gln Trp Leu Lys Asn Thr Gly Pro Ser Ser Asn Asn Ile Ala.
 35. A compound of the general formula (IV):

Z-X-S2-Trp-Leu-S3-Y' (IV)

in which:
Z, X are as defined in item 1 or item 4,
S2 is as defined in any one of item 8 to 24; and
S3 and Y' are as defined in any one of items 26 to 33.
36. A compound according to item 35, which is a compound of a sequence selected from the following group: 
D-His Ala Gln Gly Thr Phe Thr Ser Asp Tyr Ser Lys Tyr Leu Glu Gln Glu Val Val Arg Leu Phe
Ile Gln Trp Leu Lys Asn Gly Gly Pro Ser Ser Asn Asn Ile Ala Ala Ala,
 
D-His Ala Gln Gly Thr Phe Thr Ser Asp Tyr Ser Lys Tyr Leu Glu Gln Glu Val Ile Arg Leu Phe
Ile Gln Trp Leu Lys Asn Gly Gly Pro Ser Ser Asn Asn Ile Ala Ala Ala,
 
D-His Ala Gln Gly Thr Phe Thr Ser Asp Tyr Ser Lys Tyr Leu Glu Gln Glu Val Ile Arg Leu Phe
Leu Gln Trp Leu Lys Asn Gly Gly Pro Ser Ser Asn Asn Ile Ala Ala Ala,
 
His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala Glu Glu Lys,
 
D-His Ala Gln Gly Thr Phe Thr Ser Asp Tyr Ser Lys Tyr Leu Gln Gln Gln Val Ile Arg Ile Phe Ile
Gln Trp Leu Lys Asn Gly Gly Pro Ser Lys Asn Asn Ile Ala Ala Ala,
 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala Lys,
 
D-His Ala Gln Gly Thr Phe Thr Ser Asp Tyr Ser Lys Tyr Leu Glu Glu Glu Ala Val Arg Leu Phe
Val Gln Trp Leu Lys Asn Gly Gly Pro Ser Lys Asn Asn Ile Ala Ala Ala Lys,
 
D-His Ala Asp Gly Thr Phe Thr Ser Asp Tyr Ser Lys Tyr Leu Glu Glu Glu Ile Val Arg Tyr Phe
Val Gly Trp Leu Lys Asn Gly Gly Pro Ser Lys Asn Asn Ile Ala
 
His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Lys Asn Asn Ile Ala Ala Ala Lys,
 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys Asn Asn Ile Ala Ala Ala; and
 
D-His Ala Asp Gly Thr Phe Thr Ser Asp Tyr Ser Lys Tyr Leu Arg Glu Glu Ile Val Lys Leu Phe
Ile Gly Trp Leu Lys Asn Gly Gly Pro Ser Lys Asn Asn Ile Ala
 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys Asn Asn Ile Ala Ala Ala.
 37. A compound of the general Formula (V):

Z-X-S4-S5-E (V)

in which:
X is oxm 4-14,
Z is as defined in item 1 or item 4,
S4 represents a ten amino acid sequence consisting of from 0 to 10 correspondingly positioned amino acids from the sequence Asp Ser Arg Arg Ala Gln Asp Phe Val Gln (SEQ ID NO: 35), from 1 to 10 correspondingly positioned amino acids from the sequence Glu Glu Glu Ala Val Arg Leu Phe Ile Glu (SEQ ID NO: 4), from 0 to 10 correspondingly positioned amino acids from the sequence Arg Ile Glu Ile Val Lys Tyr Phe Ile Gly (SEQ ID NO: 145),), optionally from 0 to 9 correspondingly positioned amino acids from the sequence Arg Ile Glu Ile Val Lys Tyr Phe Ile Gly (SE ID NO: 145) and from 0 to 5 amino acids that differ in identity from the correspondingly positioned amino acids in SEQ ID NO: 4, SEQ ID NO: 145, and SEQ ID NO: 35,
S5 represents oxm25-37 or represents oxm25-37 in which at least one residue at positions 27 to 33 has been substituted by one or more correspondingly numbered residues from the sequence Lys(27) Asn(28) Gly(29) Gly(30) Pro(31) Ser(32) Ser(33) (SEQ ID NO: 24); and
E represents an optional extension moiety comprising one or more amino acid residues;
a variant or derivative thereof;
or a salt or solvate thereof.
38. A compound according to item 37, in which S4 contains at least three correspondingly positioned amino acids from either SEQ ID NO: 4 or SEQ ID NO: 145.
39. A compound according to item 37, in which S4 includes at least one amino acid differing in identity to the correspondingly positioned amino acids in SEQ ID NO: 4, SEQ ID NO: 145 and SEQ ID NO: 35.
40. A compound according to item 37, in which S4 contains at least six correspondingly positioned amino acids from SEQ ID NO: 4 or SEQ ID NO: 145, and at least one amino acid differing in identity to the correspondingly positioned amino acids in SEQ ID NO: 4, SEQ ID NO: 145 and SEQ ID NO: 35.
41. A compound according to item 37, in which S4 is made up of nine correspondingly positioned amino acids from SEQ ID NO: 4 or SEQ ID NO: 145, and at least one amino acid differing in identity to the correspondingly positioned amino acids in SEQ ID NO: 4, SEQ ID NO: 145, and SEQ ID NO: 35.
42. A compound according to any one of items 37 to 41, in which S5 represents:

-Trp-Leu-S3-Y'

in which Y' is as defined in item 26 and S3 is as defined in any one of items 26 to 33.
43. A compound according to item 42, which has one of the following sequences: 
His Ser Gln Gly Thr Phe Thr Ser Asp Tyr Ser Lys Tyr Leu Glu Glu Glu Val Val Arg Leu Phe Ile
Glu Trp Leu Met Asn Thr Lys Arg Asn Arg Asn Asn Ile Ala (SEQ ID NO: 31),
 
His Ser Gln Gly Thr Phe Thr Ser Asp Tyr Ser Lys Tyr Leu Glu Glu Glu Ala Val Arg Ile Phe Ile
Glu Trp Leu Met Asn Thr Lys Arg Asn Arg Asn Asn Ile Ala (SEQ ID NO: 32),
 
His Ala Glu Gly Thr Phe Thr Ser Asp Tyr Ser Lys Tyr Leu Glu Ile Glu Leu Val Arg Tyr Phe Val
Glu Trp Leu Lys Asn Gly Gly Pro Ser Lys Asn Asn Ile Ala Lys
 
His Ser Gln Gly Thr Phe Thr Ser Asp Tyr Ser Lys Tyr Leu Glu Glu Glu Ala Val Arg Leu Phe
Leu Glu Trp Leu Met Asn Thr Lys Arg Asn Arg Asn Asn Ile Ala (SEQ ID NO: 33),
 
His Ser Gln Gly Thr Phe Thr Ser Asp Tyr Ser Lys Tyr Leu Glu Glu Glu Ala Ile Arg Leu Phe Ile
Glu Trp Leu Met Asn Thr Lys Arg Asn Arg Asn Asn Ile Ala (SEQ ID NO: 34);
 
D-His Ala Glu Gly Thr Phe Thr Ser Asp Tyr Ser Lys Tyr Leu Glu Glu Glu Leu Val Lys Leu Phe
Ile Glu Trp Leu Lys Asn Gly Gly Pro Ser Lys Asn Asn Ile Ala
 
D-His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Ser Ser Gly Ala Pro Pro Pro Ser, or
 
His Ser Gln Gly Thr Phe Thr Ser Asp Tyr Ser Lys Tyr Leu Asp Ser Arg Arg Ala Gln Asp Phe
Val Gln Trp Leu Lys Asn Gly Gly Pro Ser Ser Gly Ala Pro Pro Pro Ser.
 44. A compound according to any one of the preceding items, in which Z represents His-Ser-Gln.
45. A compound according to any one of the preceding items, in which Z represents a moiety A-B-C in which A is an amino acid other than L-histidine, B is L-alanine or L-serine and C is L-aspartate, L-glutamate or L-glutamine.
46. A compound according to item 45, in which Z represents D-His-Ala-Gln; D-His-Ala-Glu; D-His-Ala-Asp; D-His-Ser-Gln; D-His-Ser-Glu; or D-His-Ser-Asp.
47. A compound according to item 1, in which:
X is as defined in item 1;
S1 corresponds to at least a sequence of line A as defined in item 1 and having at least six amino acids;
And Z is A¹- A²-A³-; wherein
A¹ is an amino acid other than L-histidine;
A² is Ala or Ser;
A³ is Gln, Glu or Asp.
48. A compound according to item 47, in which A¹ is a D-amino acid.
49. A compound according to item 48, in which the D-amino acid is D-histidine.
50. A compound according to item 47, in which A¹ is D-histidine, A² is L-alanine and A³ is L-glutamate or L-aspartate.
51. A compound according to any one of items 47 to 50 in which S1 includes the entirety of line A.
52. A compound according to any one of the preceding items, in which the terminal residue is an amide group.
53. A compound according to any one of items 1 to 51, having an extension moiety consisting of at least two amino acids.
54. A compound according to item 53, having one of the following extension moieties - Ala(38)-Ala(39); -Lys(38); -Ala(38)-Ala(39)-Lys(40); -Ala(38)-Ala(39)-Lys(40)-Lys(41); - Ala(38)-Ala(39)-Glu(40)-Glu(41)-Lys(42).
55. A compound according to item 53, having the extension moiety -Pro(38)-Ser(39).
56. A compound having the formula oxm-Ala(38) Ala(39)
57. A compound as in any one of the preceding items, wherein at least one of the amino acid residues has an alkyl or acyl side chain.
58. A compound as in item 57, wherein said alkyl or acyl side chain or side chains are selected from the group comprising hexadecyl, dodecyl, palmitoyl and lauroyl.
59. A compound as in item 57 or item 58 having an acyl side chain attached to the amino acid residue at one or more of positions 30, 33, 38, 40 and 42.
60. A compound as in item 57 having a formula selected from the following group: 
D-His Ala Gln Gly Thr Phe Thr Ser Asp Tyr Ser Lys Tyr Leu Glu Glu Glu Ala Val Arg Leu Phe
Ile Gln Trp Leu Met Asn Thr Lys Arg Asn Lys-lauroyl Asn Asn Ile Ala,
 
D-His Ala Gln Gly Thr Phe Thr Ser Asp Tyr Ser Lys Tyr Leu Glu Glu Glu Ala Val Arg Leu Phe
Ile Gln Trp Leu Met Asn Thr Lys Arg Asn Lys-palmitoyl Asn Asn Ile Ala,
 
D-His Ala Gln Gly Thr Phe Thr Ser Asp Tyr Ser Lys Tyr Leu Glu Glu Glu Ala Val Arg Leu Phe
Val Gln Trp Leu Met Asn Thr Lys Arg Asn Lys-palmitoyl Asn Asn Ile Ala,
 
D-His Ala Glu Gly Thr Phe Thr Ser Asp Tyr Ser Lys Tyr Leu Glu Glu Glu Leu Val Lys Leu Phe
Ile Glu Trp Leu Lys Asn Gly Gly Pro Ser Lys Asn Asn Ile Ala Lys-palmitoyl
 
D-His Ala Gln Gly Thr Phe Thr Ser Asp Tyr Ser Lys Tyr Leu Glu Glu Glu Val Val-palmitoyl
Arg Leu Phe Val Gln Trp Leu Met Asn Thr Lys Arg Asn Arg Asn Asn Ile Ala,
 
D-His Ala Gln Gly Thr Phe Thr Ser Asp Tyr Ser Lys Tyr Leu Glu Glu Glu Ala Val Arg Leu Phe
Val Gln Trp Leu Lys Asn Gly Gly Pro Ser Lys Asn Asn Ile Ala Ala Ala Lys-palmitoyl,
 
His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Lys Asn Asn Ile Ala Ala Ala Lys-palmitoyl,
 
D-His Ala Gln Gly Thr Phe Thr Ser Asp Tyr Ser Lys Tyr Leu Glu Glu Glu Ala Val Arg Leu Phe
Val Gln Trp Leu Lys Asn Gly Gly Pro Ser Lys Asn Asn Ile Ala Ala Ala Lys-lauroyl,
 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala Lys-palmitoyl,
 
D-His Ala Asp Gly Thr Phe Thr Ser Asp Tyr Ser Lys Tyr Leu Arg Ile Glu Leu Val Arg Tyr Phe
Val Glu Trp Leu Lys Asn Gly Gly Pro Ser Lys-lauroyl Asn Asn Ile Ala
 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Ser Asn Asn Ile Ala Ala Ala Lys-lauroyl,
 
D-His Ala Gln Gly Thr Phe Thr Ser Asp Tyr Ser Lys Tyr Leu Glu Glu Glu Ala Val Arg Leu Phe
Ile Gln Trp Leu Lys Asn Gly Gly Pro Ser Lys-palmitoyl Asn Asn Ile Ala Ala Ala; and
 
His Ser Gln Gly Thr Phe Thr Ser Asp Tyr Ser Lys Tyr Leu Glu Glu Glu Ala Val Arg Leu Phe Ile
Gln Trp Leu Lys Asn Gly Gly Pro Ser Ser Asn Asn Ile Ala Ala Ala Glu Glu Lys-palmitoyl.
 61. A compound according to any one of the preceding items, wherein independently of the sequence substitutions defined by reference to lines A, B, C, D, E, F, R, and S above a further amino acid from positions 4 to 37 is substituted by an alternative amino acid.
62. A compound according to any one of the preceding items, wherein independently of the sequence substitutions defined by reference to lines A, B, C, D, E, F, R, and S above a further two amino acids from positions 4 to 37 are substituted by alternative amino acids.
63. A compound according to any one of the preceding items, wherein independently of the sequence substitutions defined by reference to lines A, B, C, D, E, F, R, and S above a further three amino acids from positions 4 to 37 are substituted by alternative amino acids.
64. A compound according to any one of the preceding items, wherein independently of the sequence substitutions defined by reference to lines A, B, C, D, E, F, R, and S above a further four amino acids from positions 4 to 37 are substituted by alternative amino acids.
65. A compound according to any one of the preceding items, wherein independently of the sequence substitutions defined by reference to lines A, B, C, D, E, F, R, and S above a further five amino acids from positions 4 to 37 are substituted by alternative amino acids.
66. A compound according to any one of the preceding items, wherein independently of the sequence substitutions defined by reference to lines A, B, C, D, E, F, R, and S above a further six amino acids from positions 4 to 37 are substituted by alternative amino acids.
67. A compound according to any one of the preceding items, wherein independently of the sequence substitutions defined by reference to lines A, B, C, D, E, F, R, and S above a further seven amino acids from positions 4 to 37 are substituted by alternative amino acids.
68. A compound according to any one of the preceding items, wherein independently of the sequence substitutions defined by reference to lines A, B, C, D, E, F, R, and S above a further eight amino acids from positions 4 to 37 are substituted by alternative amino acids.
69. A compound according to any one of the preceding items, wherein independently of the sequence substitutions defined by reference to lines A, B, C, D, E, F, R, and S above a further nine amino acids from positions 4 to 37 are substituted by alternative amino acids.
70. A compound according to any one of the preceding items, wherein independently of the sequence substitutions defined by reference to lines A, B, C, D, E, F, R, and S above a further ten or more amino acids from positions 4 to 37 are substituted by alternative amino acids.
71. A variant compound according to any one of items 61 to 70, wherein said variant compound retains at least some of the activity of the corresponding non-variant compound.
72. A variant compound according to any one of items 61 to 70, wherein said variant compound exhibits either enhanced appetite suppression activity, a greater potency, a more therapeutically useful profile, or a lower clearance rate, relative to the corresponding non-variant compound.
73. A variant compound according to any one of items 61 to 70, wherein at least one of said further amino acid substitutions is a conservative amino acid substitution according to the following table:

| Original Residue | Conservative Substitutions |
|---|---|
| Ala | Ser |
| Arg | Lys |
| Asn | Gln, His |
| Asp | Glu |
| Cys | Ser |
| Gln | Asn |
| Glu | Asp |
| His | Asn; Gln |
| Ile | Leu, Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

74. A variant compound according to item 73, wherein all of said further amino acid substitutions are conservative amino acid substitutions according to the table given in item 66.
75. A compound having the general Formula (VI): 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala-X (VI)
 wherein X is an amine group or at least one amino acid, a variant or derivative thereof; or a salt or solvate thereof.
76. A compound as in item 75, wherein X is one amino acid.
77. A compound as in item 75, wherein X is two amino acids.
78. A compound as in item 75, wherein X is three amino acids.
79. A compound as in item 75, wherein X is four amino acids.
80. A compound as in item 75, wherein X is five amino acids.
81. A compound as in item 75, wherein X is Ala-Y wherein Y is any one or more amino acids or is absent.
82. A compound as in any one of items 75 to 81 which has a sequence selected from the following group: 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala Ala,
 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala Ala Ala Lys,
 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala Ala Ala Ala Ala Ala,
 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala Ala Ala Tyr,
 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys-dodecyl Arg Asn Arg Asn Asn Ile Ala,
 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Lys-dodecyl Asn Asn Ile Ala;
 and 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Lys-palmitoyl Asn Asn Ile Ala.
 83. A compound having the general Formula (VII): 
His Ser Gln Gly Thr Phe Thr Ser Asp Tyr Ser Lys Tyr Leu Asp Ser Arg Arg Ala Gln Asp Phe
Val Gln Trp Leu Met Asn Thr Lys Arg Asn Arg Asn Asn Ile Ala (VII)
 or a compound of general formula (I) to (VI) where amino acid residue 30 is Lys
wherein
i) Lys(30) is acyl or PEG derivatised; or
ii) The compound is a variant compound wherein any amino acid from position 1 to 37 is substituted with Lys to which an acyl or PEG side chain is derivatised.
84. A pharmaceutical composition comprising a compound according to any one of the preceding items and one or more pharmaceutically acceptable carriers.
85. A method of treating or preventing obesity or diabetes or other co-morbidities of obesity in a subject in need thereof comprising administering to the subject a compound of any one of items 1 to 83.
86. A method of reducing appetite in a subject, reducing food intake in a subject, or reducing calorie intake in a subject, reducing the body weight in a subject, reducing body weight gain in a subject, or increasing anergy expenditure in a subject comprising administering to the subject a compound of any one of items 1 to 83.
87. The method of item 85 or item 86, wherein the subject is overweight.
88. The method of item 85 or item 86, wherein the subject is obese.
89. The method of item 85 or item 86, wherein the subject is diabetic.
90. The method of any one of items 85 to 89, wherein the compound is administered peripherally.
91. The method of any one of items 85 to 89 wherein the compound is administered subcutaneously, intravenously, intramuscularly, intranasally, transdermally, transmucosally, orally or sublingually.
92. Use of a compound as in any one of items 1 to 83 for the manufacture of a medicament for the treatment of obesity or diabetes.
93. Use of a compound as in any one of items 1 to 83 for the manufacture of a medicament for the reduction of appetite in a subject, for the reduction of food intake in a subject, for the reduction of calorie intake in a subject, reducing the body weight of a subject, reducing body weight gain in a subject, or increasing energy expenditure in a subject.
94. A compound as in any one of items 1 to 83 for use as a medicament.
95. A compound as in any one of items 1 to 83 for use as a medicament for the treatment or prevention of obesity or diabetes or other co-morbidities of obesity.
96. A compound as in any one of items 1 to 83 for use as a medicament for the reduction of appetite in a subject, or for the reduction of food intake in a subject, or for the reduction of calorie intake in a subject, the reduction of body weight in a subject, the reduction of body weight gain in a subject, or the increase of anergy expenditure in a subject.

## Claims

1. A compound having the general Formula (IV):
Z-X-S2-Trp-Leu-S3-Y' (IV)
in which:
X is oxm4-14;
Z represents D-His-Ala-Gln; D-His-Ala-Glu; D-His-Ala-Asp; D-His-Ser-Gln; D-His-Ser-Glu; or D-His-Ser-Asp;
S2 is an amino acid sequence corresponding to line C (SEQ ID NO:3), in which n amino acids of said line C are substituted by n corresponding amino acids from line D (SEQ ID NO:4), and u further amino acids of line C are substituted by u corresponding amino acids from line S (SEQ ID NO:145), in which line C, line D, and line S are as follows:
| | | | | | | | | | | |
|---|---|---|---|---|---|---|---|---|---|---|
| Line C | Asp | Ser | Arg | Arg | Ala | Gln | Asp | Phe | Val | Gln |
| | 15 | 16 | 17 | 18 | 19 | 20 | 21 | 22 | 23 | 24 |
| Line D | Glu | Glu | Glu | Ala | Val | Arg | Leu | Phe | Ile | Glu |
| Line S | Arg | Ile | Glu | Ile | Val | Lys | Tyr | Phe | Val | Gly |
n being an integer not less than 1; and
u is 0, 1, 2, 3, 4, 5, 6, 7, 8, or 9;
S3 is an amino acid sequence corresponding to line E (SEQ ID NO:5), in which p amino acids of said line E are substituted by p corresponding amino acids from line F (SEQ ID NO:6), in which line E and line F are as follows:
| | | | | | | | |
|---|---|---|---|---|---|---|---|
| Line E | Met | Asn | Thr | Lys | Arg | Asn | Arg |
| | 27 | 28 | 29 | 30 | 31 | 32 | 33 |
| Line F | Lys | Asn | Gly | Gly | Pro | Ser | Ser |
p is an integer not less than 1; and
Y' is oxm34-37;
the compound optionally further including an extension moiety attached to the amino acid at position 37, the optional extension moiety comprising one or more amino acids,
or a salt or solvate thereof,
wherein optionally the compound has been modified by a process selected from amidation, glycosylation, carbamylation, alkylation, acylation, for example acetylation, sulfation, phosphorylation, cyclization, lipidization, protein (for example albumin) conjugation and pegylation; and
wherein independently of the sequence substitutions defined above a further one, two, three or four amino acids from positions 4 to 37 are substituted by an alternative amino acid.

2. A compound according to claim 1, in which u=0.

3. A compound according to claim 1, in which u=5 or more.

4. A compound according to any preceding claim, in which the amino acid replacements from lines D or F comprise at least one sequential group of at least three amino acids.

5. A compound according to any preceding claim, in which the replaced amino acids in S2 comprise a sequential group of not more than ten amino acids and the replaced amino acids in S3 comprise a sequential group of not more than seven amino acids.

6. A compound having the general Formula (V):
Z-X-S4-S5-E (V)
in which:
X is oxm 4-14;
Z represents D-His-Ala-Gln; D-His-Ala-Glu; D-His-Ala-Asp; D-His-Ser-Gln; D-His-Ser-Glu; or D-His-Ser-Asp;
S4 represents a ten amino acid sequence consisting of from 0 to 10 correspondingly positioned amino acids from the sequence Asp Ser Arg Arg Ala Gln Asp Phe Val Gln (SEQ ID NO:35), from 1 to 10 correspondingly positioned amino acids from the sequence Glu Glu Glu Ala Val Arg Leu Phe Ile Glu (SEQ ID NO: 4), optionally from 0 to 9 correspondingly positioned amino acids from the sequence Arg Ile Glu Ile Val Lys Tyr Phe Val Gly (SEQ ID NO:145) and from 0 to 5 amino acids that differ in identity from the correspondingly positioned amino acids in SEQ ID NO: 4, and SEQ ID NO:35;
S5 represents oxm25-37 or represents oxm25-37 in which at least one residue at positions 27 to 33 has been substituted by one or more correspondingly numbered residues from the sequence Lys(27) Asn(28) Gly(29) Gly(30) Pro(31) Ser(32) Ser(33) (SEQ ID NO: 24); and
E represents an optional extension moiety comprising one or more amino acid residues;
or a salt or solvate thereof;
wherein independently of the sequence substitutions defined above a further one, two, three or four amino acids from positions 4 to 37 are substituted by an alternative amino acid.

7. A compound according to any one of the preceding claims, in which the terminal residue is amidated.

8. A compound according to any one of the preceding claims, having an extension moiety consisting of at least two amino acids.

9. A compound according to claim 8, having one of the following extension moieties: -Ala(38)-Ala(39); -Lys(38); -Ala(38)-Ala(39)-Lys(40); -Ala(38)-Ala(39)-Lys(40)-Lys(41); -Ala(38)-Ala(39)-Glu(40)-Glu(41)-Lys(42); or -Pro(38)-Ser(39).

10. A compound according to any one of the preceding claims, wherein at least one of the amino acid residues has an alkyl or acyl side chain.

11. A compound according to claim 10, wherein said alkyl or acyl side chain or side chains are selected from the group consisting of hexadecyl, dodecyl, palmitoyl and lauroyl.

12. A compound according to any one of the preceding claims, wherein at least one of the amino acid residues is modified by amidation, glycosylation, carbamylation, sulfation, phosphorylation, cyclization, lipidization, protein-conjugation, or pegylation.

13. A compound according to claim 1, having a formula selected from the following group:

D-His Ala Asp Gly Thr Phe Thr Ser Asp Tyr Ser Lys Tyr Ile Arg Glu Glu Ile Val Arg
Leu Phe Val Gln Trp Leu Met Asn Thr Lys Arg Asn Lys Asn Asn Ile Ala;

D-His Ala Gln Gly Thr Phe Thr Ser Asp Tyr Ser Lys Tyr Leu Glu Glu Glu Ala Val
Arg Leu Phe Val Gln Trp Leu Met Asn Thr Lys Arg Asn Arg Asn Asn Ile Ala;

D-His Ala Asp Gly Thr Phe Thr Ser Asp Tyr Ser Lys Tyr Ile Arg Glu Glu Leu Val
Lys Tyr Phe Val Gly Trp Leu Met Asn Thr Lys Arg Asn Arg Asn Asn Ile Ala;

D-His Ala Asp Gly Thr Phe Thr Ser Asp Tyr Ser Lys Tyr Ile Arg Ile Glu Leu Val Lys
Leu Phe Val Gly Trp Leu Met Asn Thr Lys Arg Asn Lys Asn Asn Ile Ala;

D-His Ala Gln Gly Thr Phe Thr Ser Asp Tyr Ser Lys Tyr Leu Glu Glu Glu Val Ile
Arg Ile Phe Val Gln Trp Leu Met Asn Thr Lys Arg Asn Arg Asn Asn Ile Ala;

D-His Ala Gln Gly Thr Phe Thr Ser Asp Tyr Ser Lys Tyr Leu Glu Glu Glu Val Val
Arg Leu Phe Val Gln Trp Leu Met Asn Thr Lys Arg Asn Arg Asn Asn Ile Ala;

D-His Ala Gln Gly Thr Phe Thr Ser Asp Tyr Ser Lys Tyr Leu Glu Glu Glu Ala Val
Arg Leu Phe Ile Gln Trp Leu Met Asn Thr Lys Arg Asn Lys Asn Asn Ile Ala;

D-His Ala Gln Gly Thr Phe Thr Ser Asp Tyr Ser Lys Tyr Leu Glu Glu Glu Ala Val
Arg Leu Phe Val Gln Trp Leu Met Asn Thr Lys Arg Asn Lys Asn Asn Ile Ala Ala
Ala;

D-His Ala Gln Gly Thr Phe Thr Ser Asp Tyr Ser Lys Tyr Leu Glu Gln Glu Val Val
Arg Leu Phe Ile Gln Trp Leu Lys Asn Gly Gly Pro Ser Ser Asn Asn Ile Ala Ala Ala;

D-His Ala Gln Gly Thr Phe Thr Ser Asp Tyr Ser Lys Tyr Leu Glu Gln Glu Val Ile
Arg Leu Phe Ile Gln Trp Leu Lys Asn Gly Gly Pro Ser Ser Asn Asn Ile Ala Ala Ala;

D-His Ala Gln Gly Thr Phe Thr Ser Asp Tyr Ser Lys Tyr Leu Glu Gln Glu Val Ile
Arg Leu Phe Leu Gln Trp Leu Lys Asn Gly Gly Pro Ser Ser Asn Asn Ile Ala Ala Ala;

D-His Ala Gln Gly Thr Phe Thr Ser Asp Tyr Ser Lys Tyr Leu Gln Gln Gln Val Ile
Arg Ile Phe Ile Gln Trp Leu Lys Asn Gly Gly Pro Ser Lys Asn Asn Ile Ala Ala Ala;

D-His Ala Gln Gly Thr Phe Thr Ser Asp Tyr Ser Lys Tyr Leu Glu Glu Glu Ala Val
Arg Leu Phe Ile Gln Trp Leu Lys Asn Gly Gly Pro Ser Ser Asn Asn Ile Ala Ala Ala
Lys;

D-His Ala Gln Gly Thr Phe Thr Ser Asp Tyr Ser Lys Tyr Leu Glu Glu Glu Ala Val
Arg Leu Phe Val Gln Trp Leu Lys Asn Gly Gly Pro Ser Lys Asn Asn Ile Ala Ala Ala
Lys;

D-His Ala Asp Gly Thr Phe Thr Ser Asp Tyr Ser Lys Tyr Leu Glu Glu Glu Ile Val
Arg Tyr Phe Val Gly Trp Leu Lys Asn Gly Gly Pro Ser Lys Asn Asn Ile Ala;

D-His Ala Gln Gly Thr Phe Thr Ser Asp Tyr Ser Lys Tyr Leu Glu Glu Glu Ala Val
Arg Leu Phe Ile Gln Trp Leu Lys Asn Gly Gly Pro Ser Lys Asn Asn Ile Ala Ala Ala;

D-His Ala Asp Gly Thr Phe Thr Ser Asp Tyr Ser Lys Tyr Leu Arg Glu Glu Ile Val
Lys Leu Phe Ile Gly Trp Leu Lys Asn Gly Gly Pro Ser Lys Asn Asn Ile Ala;

D-His Ala Gln Gly Thr Phe Thr Ser Asp Tyr Ser Lys Tyr Leu Glu Glu Glu Ala Val
Arg Leu Phe Ile Gln Trp Leu Lys Asn Gly Gly Pro Ser Lys Asn Asn Ile Ala Ala Ala;

D-His Ala Glu Gly Thr Phe Thr Ser Asp Tyr Ser Lys Tyr Leu Glu Glu Glu Leu Val
Lys Leu Phe Ile Glu Trp Leu Lys Asn Gly Gly Pro Ser Lys Asn Asn Ile Ala;

D-His Ala Gln Gly Thr Phe Thr Ser Asp Tyr Ser Lys Tyr Leu Glu Glu Glu Ala Val
Arg Leu Phe Ile Gln Trp Leu Met Asn Thr Lys Arg Asn Lys-lauroyl Asn Asn Ile Ala;

D-His Ala Gln Gly Thr Phe Thr Ser Asp Tyr Ser Lys Tyr Leu Glu Glu Glu Ala Val
Arg Leu Phe Ile Gln Trp Leu Met Asn Thr Lys Arg Asn Lys-palmitoyl Asn Asn Ile
Ala;

D-His Ala Gln Gly Thr Phe Thr Ser Asp Tyr Ser Lys Tyr Leu Glu Glu Glu Ala Val
Arg Leu Phe Val Gln Trp Leu Met Asn Thr Lys Arg Asn Lys-palmitoyl Asn Asn Ile
Ala;

D-His Ala Glu Gly Thr Phe Thr Ser Asp Tyr Ser Lys Tyr Leu Glu Glu Glu Leu Val
Lys Leu Phe Ile Glu Trp Leu Lys Asn Gly Gly Pro Ser Lys Asn Asn Ile Ala Lys-
palmitoyl;

D-His Ala Gln Gly Thr Phe Thr Ser Asp Tyr Ser Lys Tyr Leu Glu Glu Glu Val Val-
palmitoyl Arg Leu Phe Val Gln Trp Leu Met Asn Thr Lys Arg Asn Arg Asn Asn Ile
Ala;

D-His Ala Gln Gly Thr Phe Thr Ser Asp Tyr Ser Lys Tyr Leu Glu Glu Glu Ala Val
Arg Leu Phe Val Gln Trp Leu Lys Asn Gly Gly Pro Ser Lys Asn Asn Ile Ala Ala Ala
Lys-palmitoyl;

D-His Ala Gln Gly Thr Phe Thr Ser Asp Tyr Ser Lys Tyr Leu Glu Glu Glu Ala Val
Arg Leu Phe Val Gln Trp Leu Lys Asn Gly Gly Pro Ser Lys Asn Asn Ile Ala Ala Ala
Lys-lauroyl;

D-His Ala Gln Gly Thr Phe Thr Ser Asp Tyr Ser Lys Tyr Leu Glu Glu Glu Ala Val
Arg Leu Phe Ile Gln Trp Leu Lys Asn Gly Gly Pro Ser Ser Asn Asn Ile Ala Ala Ala
Lys-palmitoyl;

D-His Ala Asp Gly Thr Phe Thr Ser Asp Tyr Ser Lys Tyr Leu Arg Ile Glu Leu Val
Arg Tyr Phe Val Glu Trp Leu Lys Asn Gly Gly Pro Ser Lys-lauroyl Asn Asn Ile Ala;

D-His Ala Gln Gly Thr Phe Thr Ser Asp Tyr Ser Lys Tyr Leu Glu Glu Glu Ala Val
Arg Leu Phe Ile Gln Trp Leu Lys Asn Gly Gly Pro Ser Ser Asn Asn Ile Ala Ala Ala
Lys-lauroyl; or

D-His Ala Gln Gly Thr Phe Thr Ser Asp Tyr Ser Lys Tyr Leu Glu Glu Glu Ala Val
Arg Leu Phe Ile Gln Trp Leu Lys Asn Gly Gly Pro Ser Lys-palmitoyl Asn Asn Ile
Ala Ala Ala.

14. A pharmaceutical composition comprising a compound according to any one of the preceding claims and one or more pharmaceutically acceptable carriers.

15. A compound according to any one of claims 1 to 13 for use as a medicament.

16. A compound according to any one of claims 1 to 13 for use in the treatment or prevention of obesity, eating disorders, diabetes, heart disease, hypertension, lipid disease, and disorders of intestinal and gastric motor activity.

17. A compound according to claim 16 for use in the treatment or prevention of diabetes.

18. A compound according to any of the preceding claims, wherein the compound is administered in combination with GLP-1 or an analogue thereof, exenatide or pramlintide.

19. A compound according to any one of claims 1 to 13, further comprising a fusion partner.

20. A compound according to claim 19, wherein the fusion partner is an antibody or an antibody fragment.

21. A compound according to any one of claims 1 to 13 for use in the reduction of appetite in a subject, or for the reduction of food intake in a subject, or for the reduction of calorie intake in a subject, the reduction of body weight in a subject, the reduction of body weight gain in a subject, or the increase of energy expenditure in a subj ect.
